# EUROPEAN PATENT SPECIFICATION

(11) **EP 4 511 366 B1**
(45) Date of publication and mention of the grant of the patent: **28.10.2026**
(21) Application number: 23724975.0
(22) Date of filing: 19.04.2023
(51) Int. Cl.: C07D 401/12, C07D 401/14, C07D 407/14, C07D 413/14, A61P 35/00, A61K 31/495

(54) **KIT INHIBITORS**
KIT INHIBITOREN
INHIBITEURS DE KIT

(30) Priority: 19.04.2022 US 202263332387 P
(43) Date of publication of application: 26.02.2025
(73) Proprietor: Blueprint Medicines Corporation, Cambridge, MA 02139 (US)
(72) Inventor: AHMAD, Omar, Cambridge, Massachusetts 02139 (US); BARVIAN, Kevin, Cambridge, Massachusetts 02139 (US); DINEEN, Thomas A., Cambridge, Massachusetts 02139 (US); GRASSIAN, Alexandra, Cambridge, Massachusetts 02139 (US); KIM, Joseph L., Cambridge, Massachusetts 02139 (US); MILLER, Stephen, Cambridge, Massachusetts 02139 (US); MOINE, Ludivine, Cambridge, Massachusetts 02139 (US); PEROLA, Emanuele, Cambridge, Massachusetts 02139 (US)
(74) Representative: Regimbeau
(86) International application number: PCT/US2023/019090
(87) International publication number: WO 2023/205226

(56) References cited:
- WO-A1-2014/074660
- WO-A1-2015/057873
- WO-A1-2018/183923
- WO-A1-2021/204626
- KOZINOVA M T ET AL: "Molecular-Genetic Basis of Gastrointestinal Stromal Tumor Personalized Therapy by Receptor Tyrosine Kinase Inhibitors (A Review)", PHARMACEUTICAL CHEMISTRY JOURNAL, SPRINGER NEW YORK LLC, US, vol. 55, no. 4, 1 July 2021 (2021-07-01), pages 315 - 322, XP037533323, ISSN: 0091-150X, [retrieved on 20210723], DOI: 10.1007/S11094-021-02419-8

## Description

### CROSS-REFERENCE TO RELATED APPLICATIONS

This application claims the benefit of and priority to U.S. Provisional Application No. 63/332,387 filed April 19, 2022.

### FIELD

This application is directed to KIT inhibitors and methods for their use, such as to control the activity of mutated forms of the enzyme KIT (also referred to as "CD117") in a subject.

### BACKGROUND

This invention relates to novel N-(pyridin-2-yl)pyrimidine-4-amine compounds and their use as selective inhibitors of mutant KIT protein kinases for use in pharmaceutical compositions to treat gastrointestinal stromal tumor (GIST).

The enzyme KIT (also called CD117) is a receptor tyrosine kinase expressed on a wide variety of cell types. The KIT molecule contains a long extracellular domain, a transmembrane segment, and an intracellular portion. The ligand for KIT is stem cell factor (SCF), whose binding to the extracellular domain of KIT induces receptor dimerization, kinase domain activation and activation of downstream signaling pathways. Mutations in KIT are found in over 80% of primary GIST patients, most commonly KIT mutations are found in exon 11 and less commonly in exon 9; these mutations make the KIT enzyme function independent of activation by its ligand, SCF, leading to a high cell division rate and possibly genomic instability (C. R. Antonescu, The GIST paradigm: Lessons for other kinase-driven cancers. J. Pathol. 223, 251-261 (2011)). Constitutive activation of KIT plays a central role in the oncogenic behavior of GIST.

GIST patients with primary mutations in exon 9 or 11 of KIT respond well to frontline therapy of imatinib, with an overall response rate of 60%; but after patients progress (or fail after imatinib), the subsequent therapies, including sunitinib, regorafenib and ripretinib, show less robust efficacy and the overall response rate remains below 10% for each of these therapies (Ther. Adv. Med. Oncol. 2021; 13: 1758835920986498).

It has been observed that patients who develop resistance to imatinib often display on-target mutations in KIT, most frequently in exon 17 (at several different amino acids including D816) or in exon 13 (including V654A and N655K). These resistance mutations occur in addition to the primary exon 9 or 11 mutation (Debiec-Rychter M, et al. Gastroenterology.2005;128:270-9.) (Roberts KG, et al. Mol Cancer Ther. 2007;6:1159-66.) (Tamborini E, et al. Oncogene. 2006; 25:6140-6). In clinical trials with small molecules designed to target the exon 17 resistant mutations in KIT, it has been observed that the patients with KIT exon 13, specifically KIT exon 13 V654A, mutations are unlikely to respond to treatment (George, S., Abstract #11511, American Society of Clinical Oncology, June 2018, Chicago, Illinois; Heinrich, M., Abstract #3027631, Connective Tissue Oncology Society, November 2018, Rome, Italy). For transformative therapy for GIST patients, the resistant mutations such as exon 13 mutations within the KIT enzyme must be inhibited.

Current KIT inhibitors are inadequate for treatment of GIST that has become resistant to inhibitors of the primary activating KIT mutations. For example, the current KIT inhibitors fail to sufficiently inhibit the mutation exon 13, *e.g.,* V654A and N655K. As such, there is a need for a small molecule treatment targeting resistant KIT mutations, e.g., exon 13 V654A and/or exon 13 N655K resistant mutations.

### SUMMARY

Provided herein are compounds, or pharmaceutically acceptable salts thereof, and compositions which are useful for inhibiting mutant KIT proteins and for treating gastrointestinal stromal tumors (GISTs). For example, the IC₅₀ values for inhibition of autophosphorylation of KIT in Table 1 demonstrate that these compounds are potent inhibitors of mutant KIT.

The invention is defined in the appended claims and any other aspects or embodiments set forth herein are for information only. In the following, the references to the methods of treatment by therapy are to be interpreted as references to compounds and compositions of the present invention for use in those methods.

A first embodiment of the invention is a compound represented by Formula (I): 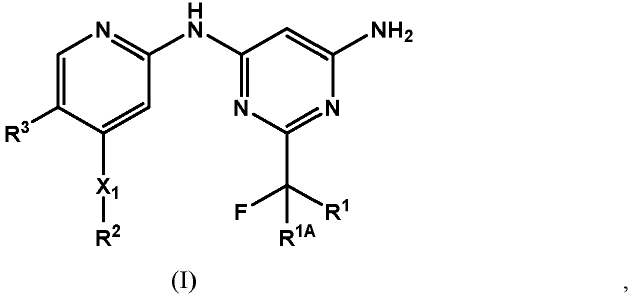 or a pharmaceutically acceptable salt thereof, wherein:
R¹ and R^{1A} are each independently selected from H, halogen, and CH₃, or R¹ and R^{1A} taken together with the carbon to which they are attached form cyclopropyl;
R² is selected from C₁₋₅alkyl, CD₃, C₃₋₆cycloalkyl, bicyclo[1.1.1]pentane, and 4- to 6-membered heterocycle containing O, wherein said alkyl, cycloalkyl or heterocycle is optionally substituted with 1-3 R⁴;
each R⁴ is independently selected from halogen, CH₃, C₂₋₃alkenyl, OH, CH₂OH, C₄₋₆cycloalkyl, 4- to 6-membered heterocycle containing O, and phenyl, wherein said alkyl, cycloalkyl, heterocycle or phenyl is optionally substituted with OH or NH₂, C₁₋₂alkyl, CH₂NH₂, or halogen;
R³ is C(O)NHR⁵, 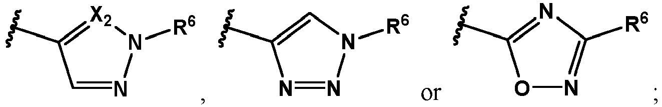
X₁ is NH or O;
X₂ is N or CH;
X₃ is N or CH;
R⁵ is selected from H, C₁₋₃alkyl, CD₃, C₃₋₄cycloalkyl and bicyclo[1.1.1] pentane, wherein said alkyl, cycloalkyl or bicyclo[1.1.1.]pentane is optionally substituted with 1-2 R⁷;
each R⁷ is independently selected from CN, NH₂, OH, CH₂OH, cyclopropyl, pyridinyl, and oxazolyl, or taken together two R⁷ attached to the same carbon atom form 4-membered heterocycle containing N;
R⁶ is independently selected from C₁₋₅alkyl, CHF₂, CF₃, 4- or 5-membered heterocycle containing N or O, and C₃₋₄cycloalkyl, wherein said alkyl or heterocycle is optionally substituted with one R⁸;
R⁸ is independently selected from OH, NR⁹R⁹, OCH₃, CH₃ and 4-membered heterocycle containing N or O, wherein said alkyl or heterocycle is optionally substituted with one R¹⁰;
each R⁹ is independently selected from H, CH₃ and CH₂CF₃; and
R¹⁰ is selected from CH₃ and CF₃.

Another embodiment of the invention is a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing).

Another embodiment of the invention is a method of treating a patient suffering from gastrointestinal stromal tumor (GIST) with an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or with an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing).

The present disclosure describes a method of treating a patient suffering from a malignant disease, comprising administering to the patient an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering the patient an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing).

Another embodiment of the invention is a method of treating a patient suffering from a malignant disease characterized by an exon 9 KIT mutation, an exon 11 KIT mutation, an exon 13 KIT mutation or an exon 17 KIT mutation, or a combination thereof, comprising administering to the patient an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering the patient an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing).

The present disclosure further describes a method of treating a patient suffering from gastrointestinal stromal tumor (GIST), comprising administering to the patient an effective combination comprising (i) an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), and (ii) a second agent.

Another embodiment of the invention is a method of treating a patient suffering from gastrointestinal stromal tumor (GIST), comprising administering to the patient an effective combination comprising a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), and at least one additional agent.

Another embodiment of the invention is a method of treating a patient suffering from a malignant disease characterized by a tumor with an exon 9 KIT mutation, an exon 11 KIT mutation or an exon 17 KIT mutation, or a combination thereof, comprising (a) obtaining a biological sample from the patient; (b) detecting the presence or absence of an exon 13 KIT mutation or exon 14 KIT mutation; and (c) administering an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), to the patient, if the mutation is detected.

The present disclosure further describes a method of treating a patient suffering from a malignant disease characterized by a tumor with an exon 9 KIT mutation, an exon 11 KIT mutation, or an exon 17 KIT mutation, or a combination thereof, comprising (a) obtaining a biological sample from the patient; (b) detecting the presence or absence of an exon 13 KIT mutation or exon 14 KIT mutation; and (c) administering an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), to the patient, if the mutation is present.

The present disclosure further describes a method of treating a patient suffering from a malignant disease characterized by a tumor with an exon 9 KIT mutation, an exon 11 KIT mutation, or an exon 17 KIT mutation, or a combination thereof, comprising administering an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), to the patient, if an exon 13 KIT mutation or exon 14 KIT mutation is detected.

The present disclosure further describes a method of treating a patient suffering from a malignant disease characterized by a tumor with an exon 9 KIT mutation, an exon 11 KIT mutation or an exon 17 KIT mutation, or a combination thereof, comprising administering an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), to the patient, if an exon 13 KIT mutation or exon 14 KIT mutation is present.

The present disclosure further describes a method of treating a patient suffering from gastrointestinal stromal tumor (GIST), comprising administering to the patient an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), wherein the GIST is mutation resistant to a KIT inhibitor administered to treat GIST with a primary activating mutation in exon 9 or exon 11.

The present disclosure further describes a method of treating a patient suffering from gastrointestinal stromal tumor (GIST) characterized by a primary activating mutation in exon 9 KIT or exon 11 KIT, comprising administering to the patient: (i) a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or a pharmaceutical composition comprising a pharmaceutically acceptable carrier or excipient and a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), and (ii) a KIT inhibitor effective against GIST characterized by a primary activating mutation in exon 9 KIT or exon 11 KIT.

### DETAILED DESCRIPTION

The disclosed compounds or pharmaceutically acceptable salts thereof are inhibitors of KIT enzymes and are useful for treating a KIT-dependent disorder or disease.

### Definitions

The following abbreviations and terms have the indicated means throughout:
The term "alkyl" used alone or as part of a larger moiety, such as "alkoxy", "hydroxyalkyl" and the like, means a saturated aliphatic straight-chain or branched monovalent hydrocarbon radical. Unless otherwise specified, an alkyl group typically has 1 to 6 carbon atoms (C₁-₆ alkyl), *(i.e.,* 1, 2, 3, 4, 5 or 6) alternatively, 1 to 3 carbon atoms (C₁-₃ alkyl) *(i.e.,* 1, 2 or 3). Examples include methyl, ethyl, propyl, isopropyl, butyl, isobutyl, tert-butyl, and the like.

The term "alkenyl" means an aliphatic straight-chain or branched monovalent hydrocarbon radical with one double bond. Unless otherwise specified, an alkenyl group typically has 1 to 6 carbon atoms (C₁-₃ alkenyl), alternatively, 1 to 3 carbon atoms (C₁-₃ alkenyl).

"Cycloalkyl" means a saturated aliphatic cyclic hydrocarbon ring radical. Unless otherwise specified, a cycloalkyl has 3 to 8 ring carbon atoms (C₃-₈ cycloalkyl), alternatively, 3 to 6 ring carbon atoms (C₃-₆ cycloalkyl), alternatively, 3 to 5 carbon atoms (C₃₋₅ cycloalkyl). Examples of cycloalkyl include cyclopropyl, cyclobutyl, cyclopentyl, cyclohexyl, and the like.

The term "halogen" or "halo" means fluorine or fluoro (F), chlorine or chloro (Cl), bromine or bromo (Br), or iodine or iodo (I).

The term "heterocycle" refers to a monocyclic non-aromatic ring radical containing unless otherwise specified, 3 to 8 ring atoms *(i.e.,* "3, 4, 5, 6, 7, or 8 membered") selected from carbon atoms and 1 or 2 heteroatoms. Each heteroatom is independently selected from nitrogen, quaternary nitrogen, oxidized nitrogen *(e.g.,* NO); oxygen; and sulfur, including sulfoxide and sulfone. Representative heterocycles include azetidinyl, morpholinyl, thiomorpholinyl, pyrrolidinonyl, pyrrolidinyl, piperidinyl, piperazinyl, hydantoinyl, valerolactamyl, oxiranyl, oxetanyl, tetrahydrofuranyl, tetrahydropyranyl, tetrahydropyrindinyl, tetrahydropyrimidinyl, tetrahydrothiophenyl, tetrahydrothiopyranyl, and the like.

The term "hydroxyl" or "hydroxy" refers to the group OH.

The term "substituted", whether preceded by the term "optionally" or not, refers to the replacement of a hydrogen substituent in a given structure with a non-hydrogen substituent. Thus, for example, a substituted alkyl is an alkyl wherein at least one non-hydrogen substituent is in the place of a hydrogen substituent on the alkyl group. To illustrate, monofluoroalkyl is an alkyl substituted with a fluoro substituent, and difluoroalkyl is an alkyl substituted with two fluoro substituents. It should be recognized that if there is more than one substitution on a substituent, each non-hydrogen substituent can be identical or different (unless otherwise stated).

If a group is described as "optionally substituted", the group can be either (1) not substituted or (2) substituted. If a group is described as optionally substituted with up to a particular number of non-hydrogen substituents, that group can be either (1) not substituted; or (2) substituted by up to that particular number of non-hydrogen substituents or by up to the maximum number of substitutable positions on the substituent, whichever is less. Thus, for example, if a group is described as a cycloalkyl optionally substituted with up to 3 non-hydrogen substituents, then any cycloalkyl with less than 3 substitutable positions would be optionally substituted by up to only as many non-hydrogen substituents as the cycloalkyl has substitutable positions.

Compounds having one or more chiral centers can exist in various stereoisomeric forms, *i.e.,* each chiral center can have an R or S configuration or can be a mixture of both. Stereoisomers are compounds that differ only in their spatial arrangement. Stereoisomers include all diastereomeric and enantiomeric forms of a compound. Enantiomers are stereoisomers that are non-superimposable mirror images of each other. Diastereomers are stereoisomers having two or more chiral centers that are not identical and are not mirror images of each other.

When the stereochemical configuration at a chiral center in a compound having one or more chiral centers is depicted by its chemical name (e.g., where the configuration is indicated in the chemical name by "R" or *"S")* or structure *(e.g.,* the configuration is indicated by "wedge" bonds), the enrichment of the indicated configuration relative to the opposite configuration is greater than 50%, 60%, 70%, 80%, 90%, 99% or 99.9%.

"Enrichment of the indicated configuration relative to the opposite configuration" is a mole percent and is determined by dividing the number of compounds with the indicated stereochemical configuration at the chiral center(s) by the total number of all of the compounds with the same or opposite stereochemical configuration in a mixture.

When two or more stereoisomers are depicted by their chemical names or structures, and the names or structures are connected by an "or", one or the other of the two or more stereoisomers is intended, but not both. The enrichment of one stereoisomer relative to the other is as indicated above.

When a disclosed compound having a chiral center is depicted by a structure without showing a configuration at that chiral center, the structure is meant to encompass the compound with the S configuration at that chiral center, the compound with the R configuration at that chiral center, or the compound with a mixture of the R and S configuration at that chiral center. When a disclosed compound having a chiral center is depicted by its chemical name without indicating a configuration at that chiral center with *"S"* or *"R",* the name is meant to encompass the compound with the S configuration at that chiral center, the compound with the R configuration at that chiral center or the compound with a mixture of the R and S configuration at that chiral center.

A racemic mixture means a mixture of 50% of one enantiomer and 50% of its corresponding enantiomer. The present teachings encompass all enantiomerically-pure, enantiomerically-enriched, diastereomerically pure, diastereomerically enriched, and racemic mixtures, and diastereomeric mixtures of the compounds described herein.

Enantiomeric and diastereomeric mixtures can be resolved into their component enantiomers or stereoisomers by well known methods, such as chiral-phase gas chromatography, chiral-phase high performance liquid chromatography, crystallizing the compound as a chiral salt complex, or crystallizing the compound in a chiral solvent. Enantiomers and diastereomers can also be obtained from diastereomerically or enantiomerically pure intermediates, reagents, and catalysts by known asymmetric synthetic methods.

"Peak 1" or "first eluting isomer" in the Experimental section refers to an intended reaction product compound obtained from a chromatography separation/purification that elutes earlier than a second intended reaction product compound from the same preceding reaction. The second intended product compound is referred to as "peak 2" or "second eluting isomer".

When a compound is designated by a name or structure that indicates a single enantiomer, unless indicated otherwise, the compound is at least 60%, 70%, 80%, 90%, 99% or 99.9% optically pure (also referred to as "enantiomerically pure"). Optical purity is the weight in the mixture of the named or depicted enantiomer divided by the total weight in the mixture of both enantiomers.

When the stereochemistry of a disclosed compound is named or depicted by structure, and the named or depicted structure encompasses more than one stereoisomer (e.g., as in a diastereomeric pair), it is to be understood that, unless otherwise indicated, one of the encompassed stereoisomers or any mixture of the encompassed stereoisomers are included. It is to be further understood that the stereoisomeric purity of the named or depicted stereoisomers at least 60%, 70%, 80%, 90%, 99% or 99.9% by weight. The stereoisomeric purity in this case is determined by dividing the total weight in the mixture of the stereoisomers encompassed by the name or structure by the total weight in the mixture of all of the stereoisomers.

As used herein, the term "pharmaceutically acceptable salt" refers to pharmaceutical salts that are, within the scope of sound medical judgment, suitable for use in contact with the tissues of humans and lower animals without undue toxicity, irritation, and allergic response, and are commensurate with a reasonable benefit/risk ratio.

Pharmaceutically acceptable salts are known in the art. For example, S. M. Berge et al. describes pharmacologically acceptable salts in J. Pharm. Sci. (1977) 66:1-19. Compounds of this invention with basic groups can form pharmaceutically acceptable salts with pharmaceutically acceptable acid(s). Suitable pharmaceutically acceptable acid addition salts of the compounds described herein include salts of inorganic acids (such as hydrochloric acid, hydrobromic, phosphoric, nitric, and sulfuric acids) and of organic acids (such as acetic acid, benzenesulfonic, benzoic, methanesulfonic, and p-toluenesulfonic acids). Compounds of this invention with acidic groups can form pharmaceutically acceptable salts with pharmaceutically acceptable base(s). Suitable pharmaceutically acceptable basic salts include ammonium salts, alkali metal salts (such as sodium and potassium salts) and alkaline earth metal salts (such as magnesium and calcium salts).

A "subject" or "patient" is a mammal in need of medical treatment, preferably a human, but can also be an animal in need of veterinary treatment, e.g., companion animals (e.g., dogs, cats, and the like), farm animals (e.g., cows, sheep, pigs, horses, and the like) and laboratory animals (e.g., rats, mice, guinea pigs, and the like). In one aspect, the patient is a human. In one aspect, the patient is an adult human.

The term "effective amount" means an amount when administered to the subject or patient which results in beneficial or desired results, including clinical results, e.g., inhibits, suppresses or reduces the symptoms of the condition being treated in the subject as compared to a control. For example, a therapeutically effective amount can be given in unit dosage form (e.g., 0.1 mg to about 50 g per day, alternatively from 1 mg to about 5 grams per day. The precise amount of compound or pharmaceutically acceptable salt thereof administered to provide an "effective amount" to the subject will depend on the mode of administration, the type, and severity of the disease or condition, and on the characteristics of the subject, such as general health, age, sex, body weight, and tolerance to drugs. The skilled artisan will be able to determine appropriate dosages depending on these and other factors. When administered in combination with other therapeutic agents, e.g., when administered in combination with an anti-cancer or antiviral agent, an "effective amount" of any additional therapeutic agent(s) will depend on the type of drug used. Suitable dosages are known for approved therapeutic agents and can be adjusted by the skilled artisan according to the condition of the subject, the type of condition(s) being treated and the amount of a compound of the invention or a pharmaceutically acceptable salt thereof being used by following, for example, dosages reported in the literature and recommended in the Physician's Desk Reference (57th ed., 2003).

As used herein, a "malignant disease" refers to a disease in which abnormal cells divide without control and can invade nearby tissues. Malignant cells can also spread to other parts of the body through the blood or lymph system. Examples of malignant diseases are carcinoma, sarcoma, leukemia, and lymphoma. Cancer is a malignant disease. Systemic mastocytosis is a malignant disease. Indolent systemic mastocytosis is a malignant disease.

Examples of cancer include, but are not limited to, gastrointestinal stomal tumor (GIST), AML (acute myeloid leukemia), melanoma, lung cancer, uterine cancer, astrocytoma, liver cancer, seminoma, renal cell carcinoma, intercranial germ cell tumors, pancreatic cancer and mediastinal B-cell lymphoma.

As used herein, an "inhibitor" refers to a compound or a pharmaceutically acceptable salt thereof that inhibits a protein e.g., an enzyme such that a reduction in activity of the protein can be observed e.g., by biochemical assay. In certain embodiments, an inhibitor has an IC50 of less than 1mM, less than 500 nM, less than 250 nM, less than 100 nM, less than 50 nM, less than 20 nM, less than 10 nM, less than 5 nM, and less than 1 nM.

The term "KIT" refers to a human tyrosine kinase that may be referred to as mast/stem cell growth factor receptor (SCFR), proto-oncogene c-KIT, tyrosine-protein kinase Kit or CD117.

The term "KIT mutation", as used herein, refers to a KIT gene, cDNA, mRNA, or protein whose sequence differs from the KIT gene sequence of human reference genome hg19, or the corresponding cDNA, mRNA, or protein. In some embodiments, when discussing KIT mutations in a nucleotide sequence that encodes a KIT polypeptide, mutations are described in terms of the change that is produced in the sequence of the polypeptide that is encoded by the nucleotide. In some embodiments the KIT mutation is V654A, N655K or K642E in exon 13. As used herein, the term "an exon 9 KIT mutation" refers to a mutation in exon 9 of KIT. As used herein, the term "an exon 11 KIT mutation" refers to a mutation in exon 11 of KIT. As used herein, the term "an exon 13 KIT mutation" refers to a mutation in exon 13 of KIT. As used herein, the term "an exon 17 KIT mutation" refers to a mutation in exon 17 of KIT. As used herein, the term "an exon 18 KIT mutation" refers to a mutation in exon 18 of KIT. A829P is a mutation at the very start of exon 18 KIT but, in some embodiments, A829P is referred to as an "exon 17" KIT mutation. In some embodiments, the KIT mutation is N822K, D816V, D816E, D816F, D816H, D816I, D816Y, D820E, D820Y and D823D in exon 17. In some embodiments, the KIT mutation is A829P in exon 18 or in exon 17.

As used herein, a "selective KIT inhibitor" refers to a compound or a pharmaceutically acceptable salt thereof that selectively inhibits KIT protein kinase over another protein kinase and exhibits at least a 2-fold selectivity for a KIT protein kinase over another kinase. For example, a selective KIT inhibitor inhibitor exhibits at least a 10-fold selectivity; at least a 15-fold selectivity; at least a 20-fold selectivity; at least a 30-fold selectivity; at least a 40-fold selectivity; at least a 50-fold selectivity; at least a 60-fold selectivity; at least a 70-fold selectivity; at least a 80-fold selectivity; at least a 90-fold selectivity; at least 100-fold, at least 125-fold, at least 150-fold, at least 175-fold, or at least 200-fold selectivity for a KIT kinase over another kinase. In some embodiments, a selective KIT inhibitor exhibits at least 150-fold selectivity over another kinase, e.g., VEGFR2 (vascular endothelial growth factor receptor 2), SRC (Non-receptor protein tyrosine kinase), and FLT3 (Fms-Like Tyrosine kinase 3). See for example, Evans et al. (2017). In some embodiments, selectivity for a KIT kinase over another kinase is measured in a cellular assay (e.g., a cellular assay as provided herein). In other embodiments, selectivity for a KIT kinase or over another kinase is measured in a biochemical assay (e.g., a biochemical assay provided in Evans, et al. (2017)). In some embodiments, a KIT inhibitor is a selective KIT inhibitor. In some embodiments, the selective KIT inhibitor is a compound disclosed herein, including a compound of Formula (I), (II), (III), (IV), (V), (VI), (VII), (VIII), (IX) or (X), or a compound of Table 1, or a pharmaceutically acceptable salt of any of the foregoing. In some embodiments, the selective KIT inhibitor is avapritinib. In some embodiments, the selective KIT inhibitor is bezuclastinib. In some embodiments, the selective KIT inhibitor is BLU-263. In some embodiments, a KIT inhibitor is a pan-KIT inhibitor. In one embodiment, a pan-KIT inhibitor is AZD3229.

### Compound Embodiments

Example embodiments include:
First embodiment: a compound represented by Formula (I): 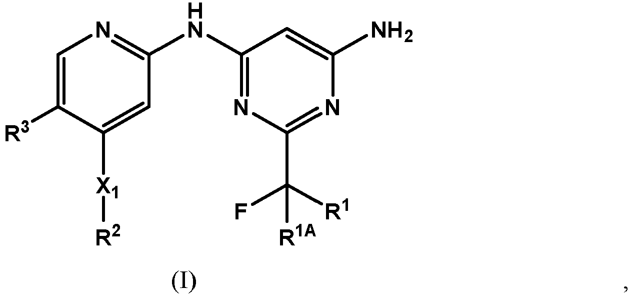 or a pharmaceutically acceptable salt thereof. The variables in Formula (I) are described in the summary above.

Second embodiment: a compound represented by Formula (I), or a pharmaceutically acceptable salt thereof, wherein R¹ and R^{1A} are each independently selected from H, halogen and CH₃; R² is selected from C₁₋₅alkyl, CD₃, C₃₋₆cycloalkyl and 4- to 6-membered heterocycle containing O, wherein said alkyl, cycloalkyl or heterocycle is optionally substituted with 1-3 R⁴; each R⁴ is independently selected from halogen, CH₃, OH, NH₂, C₄₋₆cycloalkyl, 4- to 6-membered heterocycle containing O, and phenyl, wherein said cycloalkyl or phenyl is optionally substituted with OH or NH₂; each R⁷ is independently selected from CN, OH, CH₂OH, cyclopropyl, pyridinyl, and oxazolyl, or taken together two R⁷ attached to the same carbon atom form 4-membered heterocycle containing N; and R¹⁰ is CH₃. The remainder of the variables in Formula (I) are described above in the first embodiment. In some second embodiments, each R⁴ is independently selected from halogen, CH₃, OH, C₄₋₆cycloalkyl, 4- to 6-membered heterocycle containing O, and phenyl, wherein said cycloalkyl or phenyl is optionally substituted with OH or NH₂.

Third embodiment: a compound represented by Formula (II): 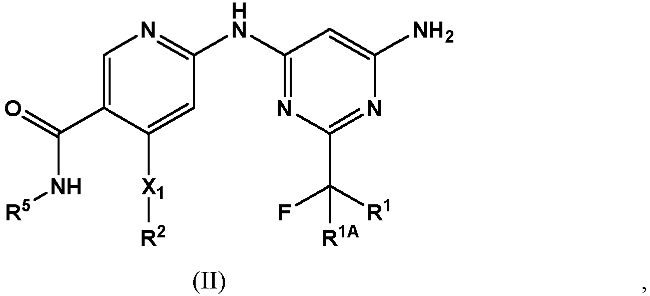 or a pharmaceutically acceptable salt thereof. The variables in Formula (II) are described in the first and/or second embodiment.

Fourth embodiment: a compound represented by Formula (III): 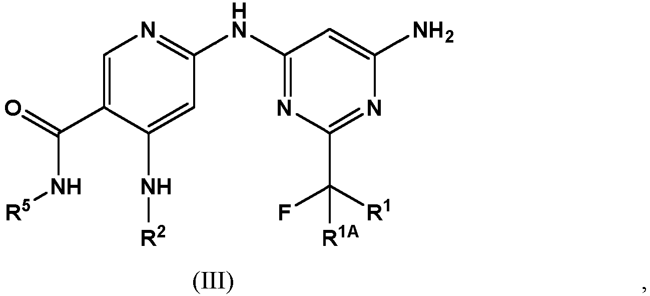 or a pharmaceutically acceptable salt thereof. The variables in Formula (III) are described in the first and/or second embodiment.

Fifth embodiment: a compound represented by Formula (I), (II) or (III), or a pharmaceutically acceptable salt thereof, wherein R¹ and R^{1A} are each independently selected from H, halogen and CH₃; R² is selected from C₁₋₅alkyl, CD₃, C₃₋₄cycloalkyl and 4- to 6-membered heterocycle containing O, wherein said alkyl, cycloalkyl or heterocycle is optionally substituted with 1-3 R⁴; each R⁴ is independently selected from halogen, CH₃ and phenyl; R⁵ is selected from H, C₁₋₃alkyl, CD₃, C₃₋₄cycloalkyl and bicyclo[1.1.1] pentane, wherein said alkyl, cycloalkyl or bicyclo[1.1.1.]pentane is optionally substituted with 1-2 R⁷; and each R⁷ is independently selected from CN, OH, CH₂OH, cyclopropyl, pyridinyl and oxazolyl, or taken together two R⁷ attached to the same carbon atom form 4-membered heterocycle containing N. The remainder of the variables in Formula (I) are described above in the first or second embodiment.

Sixth embodiment: a compound represented by Formula (I), (II) or (III), or a pharmaceutically acceptable salt thereof, wherein R² is selected from CH₃, CD₃, CH₂CH₃, CH₂CH₂CH₃, CH(CH₃)₂, CH₂CH(CH₃)₂, CH(CH₃)CH₂CH₃, cyclobutyl, cyclopropyl, oxetanyl, tetrahydrofuranyl, tetrahydrofuranyl and tetrahydropyranyl, each of which is optionally substituted with 1-3 R⁴. The remainder of the variables in Formula (I), (II) and (III) are described above in the first, second and/or fifth embodiment.

Seventh embodiment: a compound represented by Formula (I), (II) or (III), or a pharmaceutically acceptable salt thereof, wherein R² is selected from: 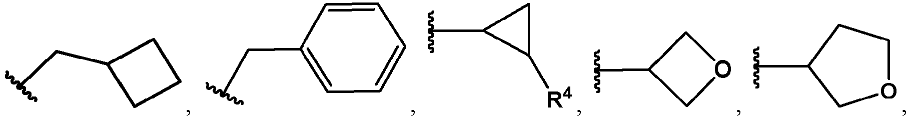 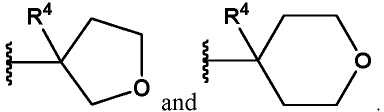 The remainder of the variables in Formula (I), (II) and (III) are described above in the first, second and/or fifth embodiment.

Eighth embodiment: a compound represented by Formula (I), (II) or (III), or a pharmaceutically acceptable salt thereof, wherein each R⁴ is independently selected from F, CH₃, cyclobutyl and phenyl. The remainder of the variables in Formula (I), (II) and (III) are described above in the first, second, fifth, sixth and/or seventh embodiment.

Ninth embodiment: a compound represented by Formula (I), (II) or (III), or a pharmaceutically acceptable salt thereof, wherein R⁵ is selected from H, CH₃, CD₃, CH₂CH₃, CH₂CH₂CH₃, CH(CH₃)₂, cyclobutyl, cyclopropyl and bicylo[1.1.1]pentanyl, wherein said CH₃, CH₂CH₃, CH₂CH₂CH₃, CH(CH₃)₂, cyclobutyl, cyclopropyl or bicylo[1.1.1]pentanyl are optionally substituted with 1-2 R⁷. The remainder of the variables in Formula (I), (II) and (III) are described above in the first, second, fifth, sixth, seventh and/or eighth embodiment.

Tenth embodiment: a compound represented by Formula (I), (II) or (III), or a pharmaceutically acceptable salt thereof, wherein R⁵ is selected from: 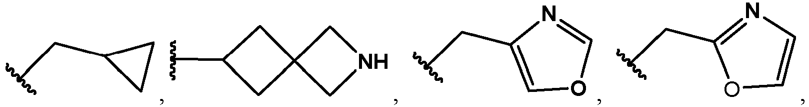 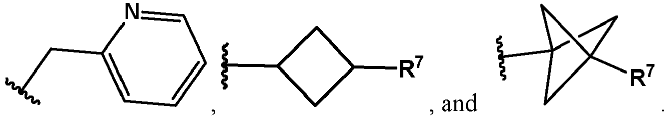 The remainder of the variables in Formula (I), (II) and (III) are described above in the first, second, fifth, sixth, seventh and/or eighth embodiment.

Eleventh embodiment: a compound represented by Formula (I), (II) or (III), or a pharmaceutically acceptable salt thereof, wherein each R⁷ is independently selected from CN, CH₂OH, cyclopropyl, 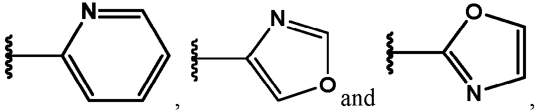 or taken together two R⁷ attached to the same carbon atom form 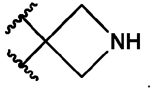 The remainder of the variables in Formula (I), (II) and (II) are described above in the first, second, fifth, sixth, seventh, eighth, ninth and/or tenth embodiment.

Twelfth embodiment: a compound represented by Formula (I), (II) or (III), or a pharmaceutically acceptable salt thereof, wherein R¹ and R^{1A} are each independently selected from H, F and CH₃. The remainder of the variables in Formula (I), (II) and (II) are described above in the first, second, fifth, sixth, seventh, eighth, ninth, tenth and/or eleventh embodiment.

Thirteenth embodiment: a compound represented by Formula (IV), (V) or (VI): 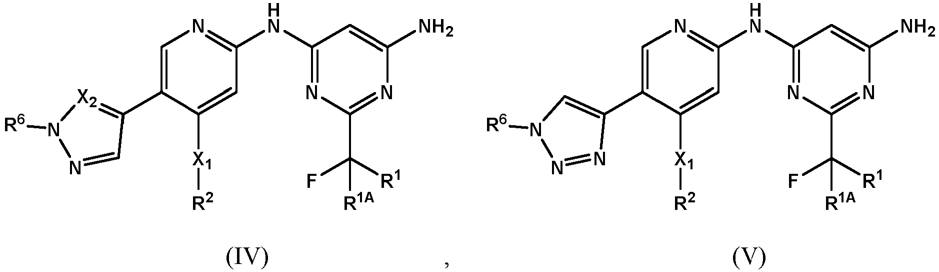 or 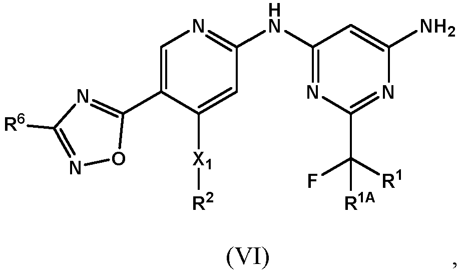 or a pharmaceutically acceptable salt thereof. The variables in Formula (IV), (V) and (VI) are described above in the first and/or second embodiment.

Fourteenth embodiment: a compound represented by Formula (VII), (VII), (IX) or (X): 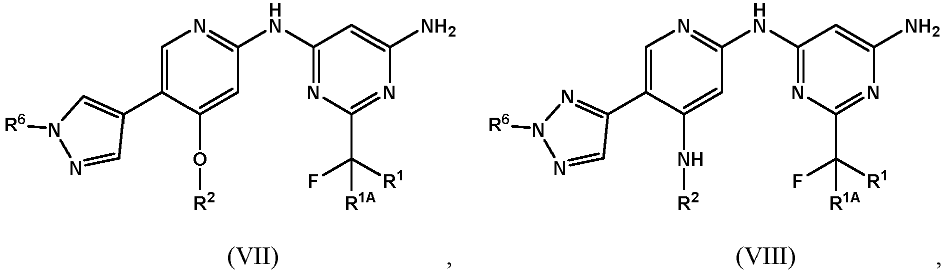 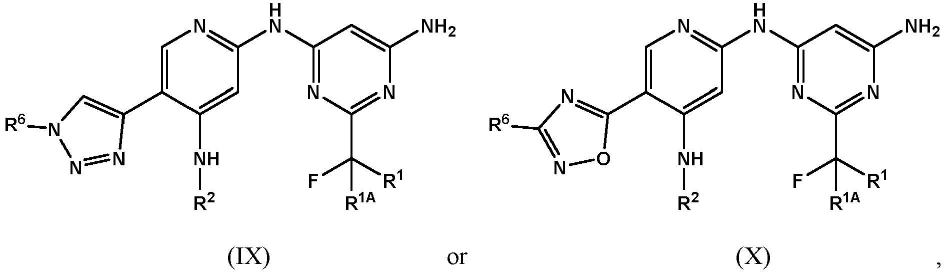 or a pharmaceutically acceptable salt thereof. The variables in Formula (VII), (VIII), (IX) and (X) are described above in the first and/or second embodiment.

Fifteenth embodiment: a compound represented by Formula (I), (IV), (V), (VI), (VII), (VIII), (IX) or (X), or a pharmaceutically acceptable salt thereof, wherein R¹ and R^{1A} are each independently selected from H, halogen, and CH₃; R² is selected from C₁₋₃alkyl, CD₃, C₃₋₄cycloalkyl and 4- to 6-membered heterocycle containing one O, wherein said alkyl, cycloalkyl, or heterocycle is optionally substituted with 1-3 R⁴; each R⁴ is independently selected from halogen, OH, cyclopropyl, 4- to 6-membered heterocycle containing one O, and phenyl, wherein said cyclopropyl or phenyl is optionally substituted with OH or NH₂; R⁶ is selected from C₁₋₄alkyl, CHF₂, CF₃, 4- or 5-membered heterocycle containing N or O, and C₃₋₄cycloalkyl, wherein said alkyl, heterocycle or cycloalkyl is optionally substituted with R⁸; R⁸ is selected from OH, NR⁹R⁹, OCH₃, CH₃ and 4-membered heterocycle containing N or O, wherein said heterocycle is optionally substituted with CH₃; and each R⁹ is independently selected from H, CH₃, and CH₂CF₃. The remainder of the variables in Formula (I), (IV), (V), (VI), (VII), (VIII), (IX) or (X) are described above in the first and/or second embodiment.

Sixteenth embodiment: a compound represented by Formula (I), (IV), (V), (VI), (VII), (VIII), (IX) or (X), or a pharmaceutically acceptable salt thereof, wherein R² is selected from CH₃, CD₃, CH₂CH₃, CH₂CH₂CH₃, CH(CH₃)₂, cyclobutyl, cyclopropyl, oxetanyl, tetrahydrofuranyl, tetrahydrofuranyl and tetrahydropyranyl, each of which is optionally substituted with 1-3 R⁴. The remainder of the variables in Formula (I), (IV), (V), (VI), (VII), (VIII), (IX) or (X) are described above in the first, second and/or fifteenth embodiment.

Seventeenth embodiment: a compound represented by Formula (I), (IV), (V), (VI), (VII), (VIII), (IX) or (X), or a pharmaceutically acceptable salt thereof, wherein R² is selected from: 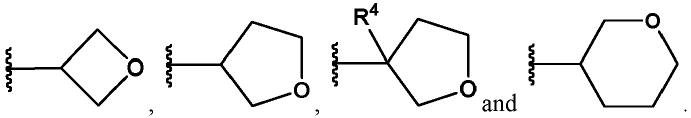 The remainder of the variables in Formula (I), (IV), (V), (VI), (VII), (VIII), (IX) or (X) are described above in the first, second and/or fifteenth embodiment.

Eighteenth embodiment: a compound represented by Formula (I), (IV), (V), (VI), (VII), (VIII), (IX) or (X), or a pharmaceutically acceptable salt thereof, wherein each R⁴ is independently selected from F, OH, cyclobutyl, oxetanyl, phenyl, tetrahydrofuranyl and tetrahydropyranyl, wherein said cyclobutyl, oxetanyl, oxetanyl, phenyl, tetrahydrofuranyl or tetrahydropyranyl is optionally substituted with OH or NH₂. The remainder of the variables in Formula (I), (IV), (V), (VI), (VII), (VIII), (IX) or (X) are described above in the first, second, fifteenth, sixteenth and/or seventeenth embodiment.

Nineteenth embodiment: a compound represented by Formula (I), (IV), (V), (VI), (VII), (VIII), (IX) or (X), or a pharmaceutically acceptable salt thereof, wherein each R⁴ is independently selected from F, OH, 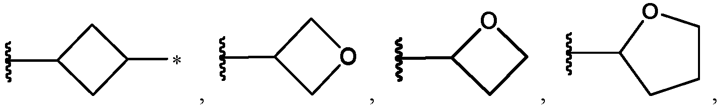 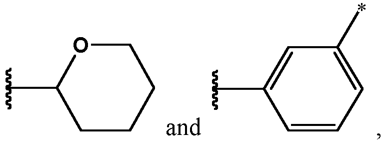 wherein *- represents OH or NH₂. The remainder of the variables in Formula (I), (IV), (V), (VI), (VII), (VIII), (IX) or (X) are described above in the first, second, fifteenth, sixteenth and/or seventeenth embodiment.

Twentieth embodiment: a compound represented by Formula (I), (IV), (V), (VI), (VII), (VIII), (IX) or (X), or a pharmaceutically acceptable salt thereof, wherein R⁶ is selected from CH₃, CH₂CH₃, CH₂CH₂CH₃, CH(CH₃)₂, CH₂CH(CH₃)₂, C(CH₃)₃, CH₂CH₂CH(CH₃)₂, CHF₂, CF₃, cyclopropyl, cyclobutyl, azetidinyl, oxetanyl, tetrahydrofuranyl and pyrrolidinyl, each of which is optionally substituted with R⁸. The remainder of the variables in Formula (I), (IV), (V), (VI), (VII), (VIII), (IX) or (X) are described above in the first, second, fifteenth, sixteenth, seventeenth, eighteenth and/or nineteenth embodiment.

Twenty-first embodiment: a compound represented by Formula (I), (IV), (V), (VI), (VII), (VIII), (IX) or (X), or a pharmaceutically acceptable salt thereof, wherein R⁶ is selected from: 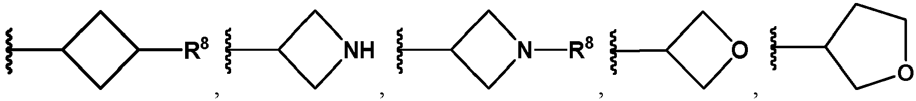 and 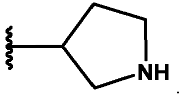 The remainder of the variables in Formula (I), (IV), (V), (VI), (VII), (VIII), (IX) or (X) are described above in the first, second, fifteenth, sixteenth, seventeenth, eighteenth and/or nineteenth embodiment.

Twenty-second embodiment: a compound represented by Formula (I), (IV), (V), (VI), (VII), (VIII), (IX) or (X), or a pharmaceutically acceptable salt thereof, wherein R⁸ is selected from OH, OCH₃, CH₃, NH₂, NHCH₃, N(CH₃)₂, NHCH₂CF₃, N(CH₃)CH₂CF₃, azetidinyl, azetidinyl and oxetanyl. The remainder of the variables in Formula (I), (IV), (V), (VI), (VII), (VIII), (IX) or (X) are described above in the first, second, fifteenth, sixteenth, seventeenth, eighteenth, nineteenth, twentieth and/or twenty-first embodiment.

Twenty-third embodiment: a compound represented by Formula (I), (IV), (V), (VI), (VII), (VIII), (IX) or (X), or a pharmaceutically acceptable salt thereof, wherein R⁸ is selected from: 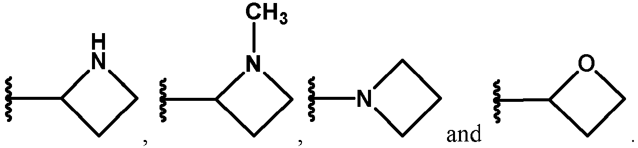 The remainder of the variables in Formula (I), (IV), (V), (VI), (VII), (VIII), (IX) or (X) are described above in the first, second, fifteenth, sixteenth, seventeenth, eighteenth, nineteenth, twentieth and/or twenty-first embodiment.

Twenty-fourth embodiment: a compound represented by Formula (I), (IV), (V), (VI), (VII), (VIII), (IX) or (X), or a pharmaceutically acceptable salt thereof, wherein R¹ and R^{1A} are each independently selected from H, F and CH₃. The remainder of the variables in Formula (I), (IV), (V), (VI), (VII), (VIII), (IX) or (X) are described above in the first, second, fifteenth, sixteenth, seventeenth, eighteenth, nineteenth, twentieth, twenty-first, twenty-second and/or twenty-third embodiment.

Twenty-fifth embodiment: a compound represented by Formula (III) or (VII): 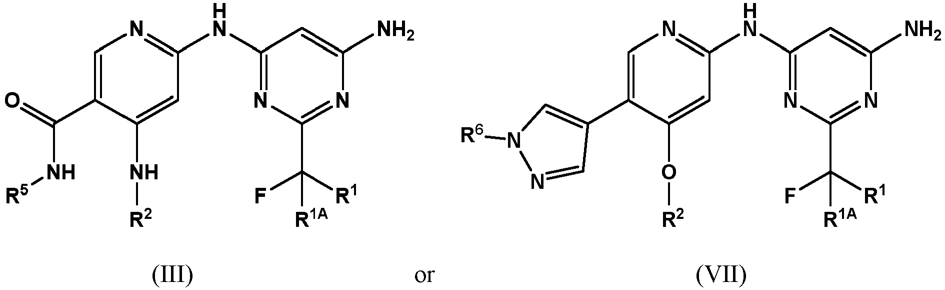 or a pharmaceutically acceptable salt thereof, wherein R¹ and R^{1A} are each independently selected from H, F and CH₃; R² is selected from C₁₋₃alkyl, CD₃, C₃₋₄cycloalkyl and 4-membered heterocycle containing one O, wherein said alkyl is optionally substituted with 1-3 halo; R⁵ is selected from CH₃, CH₂CH₃ and cyclopropyl; R⁶ is selected from C₁₋₅alkyl, CHF₂, CF₃ and 4- to 5-membered heterocycle containing O or N, wherein said alkyl or heterocycle is optionally substituted with R⁸; R⁸ is selected from OH, NR⁹R⁹, OCH₃, CH₃ and 4-membered heterocycle containing O or N; and each R⁹ is independently selected from H and CH₃.

Twenty-sixth embodiment: a compound represented by Formula (III) or (VII), or a pharmaceutically acceptable salt thereof, wherein R² is selected from CH₃, CHF₂, CF₃, CD₃, CH₂CH₃, CH₂CF₃, CH₂CH₂CH₃, CH(CH₃)₂, CH(CH₃)CH₂F, CH(CH₃)CHF₂, cyclobutyl, cyclopropyl and 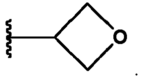 The remainder of the variables in Formula (III) or (VII) are described above in the twenty-fifth embodiment.

Twenty-seventh embodiment: a compound represented by Formula (III) or (VII), or a pharmaceutically acceptable salt thereof, wherein R⁶ is selected from CH₃, CH₂CH₃, CH₂CH₂CH₃, CH₂CH(CH₃)₂, CH₂CH₂CH(CH₃)₂, CHF₂, azetidinyl, oxetanyl, tetrahydrofuranyl and pyrrolidinyl, each of which is optionally substituted with one R⁸. The remainder of the variables in Formula (III) or (VII) are described above in the twenty-fifth and/or twenty-sixth embodiment.

Twenty-eighth embodiment: a compound represented by Formula (III) or (VII), or a pharmaceutically acceptable salt thereof, wherein R⁶ is selected from: 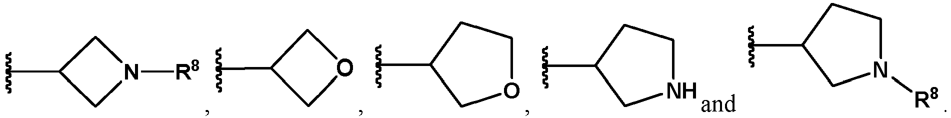 The remainder of the variables in Formula (III) or (VII) are described above in the twenty-fifth and/or twenty-sixth embodiment.

Twenty-ninth embodiment: a compound represented by Formula (III) or (VII), or a pharmaceutically acceptable salt thereof, wherein R⁸ is selected from OH, OCH₃, NH₂, NHCH₃, N(CH₃)₂, CH₃, 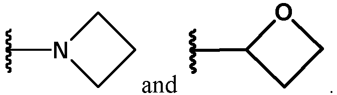 The remainder of the variables in Formula (III) or (VII) are described above in the twenty-fifth, twenty-sixth, twenty-seventh and/or twenty-eighth embodiment.

Thirtieth embodiment: a compound represented by Formula (III) or (VII), or a pharmaceutically acceptable salt thereof, wherein R⁸ is selected from OH, OCH₃, NH₂, NHCH₃, N(CH₃)₂, and CH₃. The remainder of the variables in Formula (III) or (VII) are described above in the twenty-fifth, twenty-sixth, twenty-seventh, twenty-eighth and/or twenty-ninth embodiment.

Thirty-first embodiment: a compound represented by Formula (III) or (VII), or a pharmaceutically acceptable salt thereof, wherein R⁶ is selected from CH₃, CH₂CH₃, CH₂CH₂CH₃, CH₂CH(CH₃)₂, CH₂CH₂CH(CH₃)₂ and CHF₂, each of which is optionally substituted with one R⁸. The remainder of the variables in Formula (III) or (VII) are described above in the twenty-fifth, twenty-sixth, twenty-seventh, twenty-eighth, twenty-ninth and/or thirtieth embodiment. In some thirty-first embodiments, R⁶ is selected from CH₃, CH₂CH₃, CH₂CH₂CH₃, CH₂CH(CH₃)₂, CH₂CH₂CH(CH₃)₂ and CHF₂, each of which is optionally substituted with one R⁸. In some thirty-first embodiments, R⁶ is selected from CH₃, CH₂CH₃, CH₂CH₂CH₃, CH₂CH(CH₃)₂, and CH₂CH₂CH(CH₃)₂, each of which is substituted with one R⁸.

Thirty-second embodiment: a compound represented by Formula (III) or (VII), or a pharmaceutically acceptable salt thereof, wherein R⁶ is selected from CH₃, CH₂CH₂OH, CH₂CH₂N(CH₃)₂, CH₂CH₂NHCH₃, CH₂CH₂NH₂, CH₂CH(N(CH₃)₂)CH₃, CH₂CH(NHCH₃)CH₃, CH₂C(NHCH₃)(CH₃)₂, CH₂C(NH₂)(CH₃)₂, CH₂CH₂OCH₃, CH₂CH(OH)(CH₃), CH₂C(OH)(CH₃)₂, CH₂CH₂C(OH)(CH₃)₂, and C(CH₃)₂CH₂OH. The remainder of the variables in Formula (III) or (VII) are described above in the twenty-fifth, twenty-sixth, twenty-seventh, twenty-eighth, twenty-ninth, thirtieth and/or thirty-first embodiment.

Thirty-third embodiment: a compound represented by Formula (III), or a pharmaceutically acceptable salt thereof. The variables in Formula (III) are described above in the twenty-fifth, twenty-sixth, twenty-seventh, twenty-eighth, twenty-ninth, thirtieth, thirty-first and/or thirty-second embodiment.

Thirty-fourth embodiment: a compound represented by Formula (VII), or a pharmaceutically acceptable salt thereof. The variables in Formula (VII) are described above in the twenty-fifth, twenty-sixth, twenty-seventh, twenty-eighth, twenty-ninth, thirtieth, thirty-first and/or thirty-second embodiment.

The invention also includes the compounds depicted in Table 1 and prepared in the Exemplification, both the neutral form and pharmaceutically acceptable salts thereof.

In one embodiment, the Compounds 204-211 in Table 2, and pharmaceutically acceptable salts thereof, are excluded from the invention.

The invention also includes pharmaceutical compositions containing a compound of Formula (I), (II), (III), (IV), (V), (VI), (VII), (VIII), (IX) or (X), or a compound depicted in Table 1, or pharmaceutically acceptable salts thereof, and a pharmaceutically acceptable carrier or excipient.

Another embodiment of the invention is a compound disclosed herein, including a compound of Formulae (I), (II), (III), (IV), (V), (VI), (VII), (VIII), (IX) or (X), or a compound in Table 1, or a pharmaceutically acceptable salt of any of the foregoing, in which one or more hydrogen atoms is replaced with deuterium. The deuterium enrichment at any one of the sites where hydrogen has been replaced by deuterium is at least 50%, 75%, 85%, 90%, 95%, 98% or 99%. Deuterium enrichment is a mole percent and is obtained by dividing the number of compounds with deuterium enrichment at the site of enrichment with the number of compounds having hydrogen or deuterium at the site of enrichment.

### Use Embodiments

Compounds of the invention (e.g., compounds of Formula (I), (II), (III), (IV), (V), (VI), (VII), (VIII), (IX) or (X), or compounds in Table 1, or pharmaceutically acceptable salts of any of the foregoing) are useful as KIT inhibitors. For example, KIT inhibitors of this invention inhibit mutant KIT protein kinases where the mutation is in exon 13, such as K642E, V654A or N655K KIT mutation, or in exon 14, such as T680K KIT mutation. K642E is an exon 13 KIT mutation that can be a primary activating mutation. It is less common than the exon 9 and exon 11 primary activating KIT mutations. The KIT inhibitors of the invention can therefore treat conditions associated with aberrant KIT activity in humans or non-human patients. KIT inhibitors of the invention (also referred to as compounds of the invention) include compounds of Formula (I), (II), (III), (IV), (V), (VI), (VII), (VIII), (IX) and/or (X), and pharmaceutically acceptable satls thereof, and compounds depicted in Table 1. The compounds of the invention are selective for exon 13 KIT over KIT and PDGFR wild type and therefore are expected to show minimal side effects resulting from wild type inhibition (such as anemia, thrombocytopenia and edema). The compounds of the invention also exhibit decreased brain penetration. Decreased or lack of brain penetration is anticipated to provide safety benefits and minimize unwanted cognitive effects. The method comprises administering to the patient an effective amount of a compound of the invention, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering to the patient an effective amount of a pharmaceutical composition comprising a compound of the invention, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), and a pharmaceutically acceptable carrier or excipient.

In one aspect, the invention is directed to methods of treating a patient suffering from a malignant disease (or cancer) characterized by a KIT mutation, e.g., an exon 9 KIT mutation, an exon 11 KIT mutation, an exon 13 KIT mutation or an exon 17 KIT mutation, or a combination thereof. Examples of malignant diseases or cancers treatable by compounds of the invention include gastrointestinal stromal tumor (GIST), AML (acute myeloid leukemia), melanoma, lung cancer, uterine cancer, astrocytoma, liver cancer, seminoma, renal cell carcinoma, intercranial germ cell tumors, pancreatic cancer and mediastinal B-cell lymphoma. In one aspect, the malignant disease (or cancer) is selected from gastrointestinal stromal tumor (GIST), melanoma, lung cancer, uterine cancer, astrocytoma, liver cancer, seminoma, renal cell carcinoma, and pancreatic cancer. In one aspect, the malignant disease (or cancer) is selected from AML (acute myeloid leukemia), intercranial germ cell tumors, and mediastinal B-cell lymphoma. In one aspect, the cancer is gastrointestinal stromal tumor (GIST); in another aspect, the cancer is AML (acute myeloid leukemia); in another aspect, the cancer is melanoma; in yet another aspect, the cancer is lung cancer; in yet another aspect, the cancer is uterine cancer; in yet another aspect, the cancer is astrocytoma; in yet another aspect, the cancer is liver cancer; in yet another aspect, the cancer is seminoma; in yet another aspect, the cancer is renal cell carcinoma (RCC); in one aspect the RCC is pancreatic neuroendocring tumor (pNET); in yet another aspect, the cancer is intercranial germ cell tumor; in yet another aspect, the cancer is pancreatic cancer; and in yet another aspect, the cancer is mediastinal B-cell lymphoma. In one aspect, the cancer treatable by compounds of the invention (e.g., a compound of Formula (I), (II), (III), (IV), (V), (VI), (VII), (VIII), (IX) or (X), or a compound of Table 1, or a pharmaceutically acceptable salt of any of the foregoing) is advanced. In another aspect, the cancer is adjuvant.

In an aspect, the invention provides a method of treating a patient suffering from a malignant disease (or cancer) characterized by an exon 9 KIT mutation, an exon 11 KIT mutation, an exon 13 KIT mutation or an exon 17 KIT mutation, or a combination thereof, comprising administering to the patient an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing). In some embodiments, the malignant disease (or cancer) is selected from gastrointestinal stromal tumor (GIST), AML (acute myeloid leukemia), melanoma, lung cancer, uterine cancer, astrocytoma, liver cancer, seminoma, renal cell carcinoma, intercranial germ cell tumor, pancreatic cancer and mediastinal B-cell lymphoma. In some embodiments, the malignant disease (or cancer) is gastrointestinal stromal tumor (GIST). In some embodiments, the method further comprises administering to the patient an effective amount of one or more agents *(e.g., 1,* 2, 3, or 4 additional agents). In some embodiments, the patient received one or more prior treatments (e.g., treatment prior to administering a compound of the invention) for the malignant disease (or cancer). In some embodiments, the malignant disease (or cancer) progressed after the prior treatment. In some embodiments, the prior treatment comprises administering one or more agents *(e.g.,* 1, 2, 3, or 4 additional agents). In some embodiments, the agent is a KIT inhibitor. In some embodiments, the malignant disease (or cancer) has a mutation resistant to the agent (*e.g.,* the KIT inhibitor). In some embodiments, the agent is selected from imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus and larotrectinib, and pharmaceutically acceptable salts thereof. In some embodiments, the one or more agents comprises avapritinib or a pharmaceutically acceptable salt thereof. In some embodiments, the prior treatment comprises administering one or more agents, each independently selected from: imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus and larotrectinib, and pharmaceutically acceptable salts thereof. In some embodiments, the prior treatment comprises administering imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus or larotrectinib, or a pharmaceutically acceptable salt thereof, or a combination thereof. In some embodiments, the prior treatment comprises administering imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus or larotrectinib, or a pharmaceutically acceptable salt thereof. In some embodiments, the prior treatment comprises administering imatinib or a pharmaceutically acceptable salt thereof. In some embodiments, the malignant disease (or cancer) has an imatinib-resistant mutation. In some embodiments, the prior treatment comprises administering sunitinib or a pharmaceutically acceptable salt thereof. In some embodiments, the prior treatment comprises administering regorafenib or a pharmaceutically acceptable salt thereof. In some embodiments, the prior treatment comprises administering ripretinib or a pharmaceutically acceptable salt thereof. In some embodiments, the prior treatment comprises administering avapritinib or a pharmaceutically acceptable salt thereof. In some embodiments, the prior treatment comprises administering bezuclastinib or a pharmaceutically acceptable salt thereof. In some embodiments, the prior treatment comprises administering AZD3229 or a pharmaceutically acceptable salt thereof. In some embodiments, the prior treatment comprises administering BLU-263 or a pharmaceutically acceptable salt thereof.

In one aspect, the malignant disease (or cancer) treatable by the disclosed methods (e.g., GIST) is characterized by a primary activating KIT mutation. A "primary activating mutation" is an initial mutation that converts or contributes to the conversion of a normal cell to a cancer cell i.e., a primary activating mutation is responsible for initiating tumorigenesis and/or driving the cancer. In some embodiments, the primary activating KIT mutation is an exon 9 KIT mutation or an exon 11 KIT mutation, or a combination thereof. In some embodiments, the primary activating KIT mutation is an exon 9 KIT mutation, an exon 11 KIT mutation, an exon 13 KIT mutation or an exon 17 KIT mutation, or a combination thereof. In some embodiments, the primary activating KIT mutation is selected from an exon 9 KIT mutation, an exon 11 KIT mutation, an exon 13 KIT mutation and an exon 17 KIT mutation. For some embodiments, the primary activating KIT mutation is an exon 17 KIT mutation, e.g., an exon 17 KIT mutation selected from N822K, D816V, D816E, D816F, D816H, D816I, D816Y, D820E, D820Y and D823D. In one embodiment, the exon 17 KIT mutation is D816V. In some embodiments, the primary activating mutation is A829P. A829P is a mutation at the very start of exon 18 KIT but is commonly referred to as an "exon 17" mutation. In some embodiments, the primary activating KIT mutation is an exon 17 KIT mutation selected from N822K, D816V, D816E, D816F, D816H, D816I, D816Y, D820E, D820Y, D823D and A829P. In some embodiments, the primary activating KIT mutation is an exon 17 KIT mutation selected from N822K, D816V, D816E, D816F, D816H, D816I, D816Y, D820E, D820Y and D823D. In some embodiments the primary activating KIT mutation is an exon 13 KIT mutation, e.g., an exon 13 KIT mutation such as V654A, N655K or K642E. In another aspect, the cancer is GIST and the primary activating mutation is an exon 9 KIT mutation, an exon 11 KIT mutation, an exon 13 KIT mutation or an exon 17 KIT mutation, or a combination thereof. In another aspect, the cancer is GIST and the primary activating mutation is selected from an exon 9 and/or an exon 11 mutation. In another aspect, the cancer is GIST and the primary activating mutation is an exon 9 KIT mutation or an exon 11 KIT mutation, or a combination thereof. In another aspect, the cancer is GIST and the primary activating mutation is an exon 13 KIT mutation. In another aspect, the cancer is GIST and the primary activating mutation is an exon 17 KIT mutation.

For patients with Primary GIST, complete surgical resection is the initial treatment of choice for patients, with surgery being effective in approximately 50% of GIST patients. As used herein, the term "Primary GIST" is GIST with only primary activating mutations (e.g., without secondary resistance conferring mutations). Of the remaining patients, tumor recurrence is frequent. For patients with reoccurrence of GIST after surgical resection, patients are administered one or more anti-cancer agents for the treatment of GIST. In one aspect, the one or more anticancer agents are administered prior to treatment with the KIT inhibitors of the invention. For example, the KIT inhibitors of the invention are administered after the GIST progressed after the prior treatment. For example, the patient's tumor, was or upon treatment became resistant (or refactory) to the prior agent, the patient was intolerant to the treatment with the prior agent or the GIST re-occurred after the prior treatment. Examples of agents studied and used in the prior treatment of GIST are selected from: imatinib (*e.g*., the methanesulfonic acid salt), sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus and larotrectinib, and pharmaceutically acceptable salts thereof. In some embodiments, agents studied and used in the prior treatment of GIST are selected from: imatinib (*e.g*., the methanesulfonic acid salt), sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, and bezuclastinib, and pharmaceutically acceptable salts thereof (Vallilas, C., et al. International Journal of Molecular Sciences. 2021; 22, 493). Further examples of agents studied and used in the prior treatment of GIST are selected from: AZD3229, BLU-263, everolimus and larotrectinib, and pharmaceutically acceptable salts thereof. (S)-2-(4-(4-(4-(5-(1-Amino-1-(4-fluorophenyl)ethyl)pyrimidin-2-yl)piperazin-1-yl)pyrrolo[2,1-f][1,2,4]triazin-6-yl)-1H-pyrazol-1-yl)ethanol (also known as BLU-263 and elenestinib) is another KIT inhibitor for use in the treatment of GIST. N-(4-{[5-Fluoro-7-(2-methoxyethoxy)quinazolin-4-yl]amino}phenyl)-2-[4-(propan-2-yl)-1H-1,2,3-triazol-1-yl]acetamide (also known as AZD3229 and NB003) is another KIT inhibitor for use in the treatment of GIST. In some embodiments, agents studied and used in the prior treatment of GIST are selected from: AZD3229, BLU-263, everolimus and larotrectinib, and pharmaceutically acceptable salts thereof.

In some aspects, patients with GIST are administered "Primary KIT Inhibitors." Specifically, Primary KIT inhibitors are administered to inhibit primary activating KIT mutations in patients with Primary GIST. For example, imatinib is used as a Primary KIT inhibitor to inhibit primary activating KIT mutations in exon 9 and/or exon 11. Treatment with Primary KIT Inhibitors, such as imatinib, has also been shown to be sufficient for initial treatment, i.e., for treating of patients with Primary GIST, e.g., patients with exon 9 and/or exon 11 activating KIT mutations. However, despite the beneficial effect of a targeted therapy such as imatinib, eventually 50% of patients develop primary or secondary resistance to imatinib after 2 years. These secondary imatinib-resistant mutations are most frequently located on KIT exon 11, 13, 14, 17, 18 or combinations thereof.

Sunitinib is the standard of care second line treatment for most imatinib-resistant tumors and is used to treat GIST containing KIT mutations in exons 11, 13 and 14. However, secondary KIT mutations in exons 17 and 18 are resistant to sunitinib treatment and, furthermore, tumors containing tertiary resistance mutations in exon 17 and 18 emerge several months after sunitinib treatment.

Regorafenib has shown promising results in a phase 3 clinical trial of imatinib-resistant and sunitinib-resistant GISTs with activity against several, but not all, exon 17 and 18 mutations, of which D816 is one. Regorafenib has been approved for third line GIST treatment.

Ripretinib is a KIT and PDGFRA kinase inhibitor that is approved for fourth line GIST treatment.

Additional options for treating GIST include: cabozantinib, dasatinib, everolimus, nilotinib, pazopanib, sorafenib, and larotrectinib.

Avapritinib is a selective exon 17 and exon 18 KIT inhibitor, which is currently approved for patients with GIST harboring an exon 18 PDGFRA mutation. Avapritinib has been reported to be active against one or more KIT mutations in exon 17 (e.g., D816V, D816Y, D816F, D816K, D816H, D816A, D816G, D820A, D820E, D820G, N822K, N822H, Y823D, and A829P), see U.S Patent No. 9,200,002.

Bezuclastinib, also known as CGT9486 and PLX9486, has the following structure:

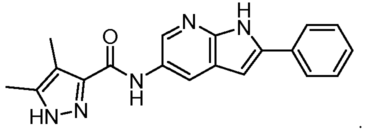

AZD3229, also known as NB003, has the following structure:

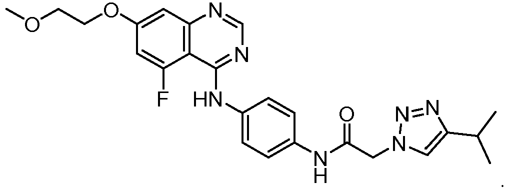

In some embodiments, prior treatment in patients with GIST involves treating the patient with one or more agents *(e.g.,* 1, 2, 3 or 4 agents) prior to treatment with a KIT inhibitor of the invention or a pharmaceutically acceptable salt thereof *(e.g.,* a compound of the invention, including a compound of Formula (I), (II), (III), (IV), (V), (VI), (VII), (VIII), (IX) or (X), or a compound of Table 1, or a pharmaceutically acceptable salt of any of the foregoing), or prior to treatment with a pharmaceutical composition containing a KIT inhibitor of the invention or a pharmaceutically acceptable salt thereof *(e.g.,* a compound of the invention, including a compound of Formula (I), (II), (III), (IV), (V), (VI), (VII), (VIII), (IX) or (X), or a compound of Table 1, or a pharmaceutically acceptable salt of any of the foregoing). In one embodiment, the prior treatment comprises administering one or more agents selected from: imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, everolimus, larotrectinib, bezuclastinib, AZD3229 and BLU-263, and pharmaceutically acceptable salts thereof. In one embodiment, prior treatment comprises administering a Primary KIT Inhibitor (e.g., imatinib or a pharmaceutically acceptable salt thereof; imatinib mesylate is a pharmaceutically acceptable salt of imatinib). The prior treatment is continued until the GIST is identified as progressed or becomes resistant, refractory or intolerant to the Primary KIT Inhibitor (e.g., imatinib or pharmaceutically acceptable salt thereof). In another embodiment, the prior treatment comprises administering an exon 17 KIT inhibitor. Examples of exon 17 inhibitors are avapritinib, BLU-263, ripretinib, and AZD3229. Examples of selective exon 17 inhibitors are avapritinib, BLU-263, and bezuclastinib. In one embodiment, the prior treatment comprises administering an exon 13 KIT inhibitor. An example of an exon 13 KIT inhibitor is sunitinib. In some embodiments, the exon 17 KIT inhibitor is selected from avapritinib, BLU-263, ripretinib, bezuclastinib, and AZD3229. In some embodiments, the exon 17 KIT inhibitor is avapritinib. In some embodiments, the exon 17 KIT inhibitor is BLU-263. In some embodiments, the exon 17 KIT inhibitor is ripretinib. In some embodiments, the exon 17 KIT inhibitor is bezuclastinib. In some embodiments, the exon 17 KIT inhibitor is AZD3229. Examples of selective exon 17 inhibitors are avapritinib, BLU-263, and bezuclastinib. In some embodiments, the exon 17 KIT inhibitor is a selective exon 17 KIT inhibitor. In some embodiments, the selective exon 17 KIT inhibitor is selected from avapritinib, BLU-263, and bezuclastinib. In some embodiments, the selective exon 17 KIT inhibitor is avapritinib. In some embodiments, the selective exon 17 KIT inhibitor is BLU-263. In some embodiments, the selective exon 17 KIT inhibitor is bezuclastinib. In another embodiment, the prior treatment comprises administering one or more agents selected from: imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, everolimus, larotrectinib, bezuclastinib, AZD3229 and BLU-263, and pharmaceutically acceptable salts thereof. In another embodiment, the prior treatment comprises administering one or more agents selected from: regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, everolimus and larotrectinib, and pharmaceutically acceptable salts thereof.

In another embodiment, prior treatment comprises administering a Primary KIT Inhibitor (e.g., imatinib or a pharmaceutically acceptable salt thereof). The prior treatment is continued until the GIST is identified as progressed or becomes resistant, refractory or intolerant to the Primary KIT Inhibitor (e.g., imatinib or pharmaceutically acceptable salt thereof); after which prior treatment further comprises administering sunitinib or a pharmaceutically acceptable salt thereof (e.g., until the GIST is identified as progressed or becomes resistant, refractory or intolerant to the sunitinib or pharmaceutically acceptable salt thereof).

In another embodiment, prior treatment comprises administering a Primary KIT Inhibitor (e.g., imatinib or a pharmaceutically acceptable salt thereof). The prior treatment is continued until the GIST is identified as progressed or becomes resistant, refractory or intolerant to the Primary KIT Inhibitor (e.g., imatinib or pharmaceutically acceptable salt thereof); after which prior treatment further comprises administering an exon 17 KIT inhibitor or a pharmaceutically acceptable salt thereof (e.g., until the GIST is identified as progressed or becomes resistant, refractory or intolerant to the exon 17 inhibitor or pharmaceutically acceptable salt thereof).

In another embodiment, prior treatment comprises administering a Primary KIT Inhibitor (e.g., imatinib or a pharmaceutically acceptable salt thereof). The prior treatment is continued until the GIST is identified as progressed or becomes resistant, refractory or intolerant to the Primary KIT Inhibitor (e.g., imatinib or pharmaceutically acceptable salt thereof), after which prior treatment further comprises administering sunitinib or a pharmaceutically acceptable salt thereof (e.g., until the GIST is identified progressed or becomes resistant, refractory or intolerant to the sunitinib or pharmaceutically acceptable salt thereof); after which prior treatment further comprises administering regorafenib or a pharmaceutically acceptable amount thereof (e.g., until the GIST is identified as progressed or becomes resistant, refractory or intolerant to the regorafenib or pharmaceutically acceptable salt thereof).

In another embodiment, prior treatment comprises administering a Primary KIT Inhibitor (e.g., imatinib or a pharmaceutically acceptable salt thereof). The prior treatment is continued until the GIST is identified as progressed or becomes resistant, refractory or intolerant to the Primary KIT Inhibitor (e.g., imatinib or pharmaceutically acceptable salt thereof), after which prior treatment further comprises administering sunitinib or a pharmaceutically acceptable salt thereof (e.g., until the GIST is identified progressed or becomes resistant, refractory or intolerant to the sunitinib or pharmaceutically acceptable salt thereof); after which prior treatment further comprises administering regorafenib or a pharmaceutically acceptable amount thereof (e.g., until the GIST is identified as progressed or becomes resistant, refractory or intolerant to the regorafenib or pharmaceutically acceptable salt thereof); after which prior treatment further comprises administering repretinib or a pharmaceutically acceptable amount thereof (e.g., until the GIST is identified as progressed or becomes resistant, refractory or intolerant to the ripretinib or pharmaceutically acceptable salt thereof).

In another embodiment, prior treatment comprises administering an effective amount of a Primary KIT Inhibitor (e.g., imatinib or a pharmaceutically acceptable salt thereof). The prior treatment is continued until the GIST is identified as progressed or becomes resistant, refractory, or intolerant to the Primary KIT Inhibitor (e.g., imatinib or pharmaceutically acceptable salt thereof), after which prior treatment further comprises administering at least one agent selected from nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, and crenolanib or a pharmaceutically acceptable salt thereof (e.g., until the GIST is identified as progressed or becomes reistant, refractory, or intolerant to the agent(s) or pharmaceutically acceptable salt(s) thereof).

The tumor is or has become mutation resistant to the agent used in the prior treatment because a mutation is present in the tumor that renders the tumor resistant or refractory to the prior agent, i.e., a "resistant mutation". For example, the tumor may have become mutation resistance to a Primary KIT Inhibitor. In some embodiments, the tumor has a mutation that is resistant to an exon 9 KIT inhibitor or an exon 11 KIT inhibitor, or a combination thereof. In some embodiments, the tumor has a mutation that is resistant to an exon 9 KIT inhibitor, an exon 11 KIT inhibitor, an exon 13 KIT mutation, an exon 17 KIT mutation, or an exon 14 KIT mutation, or a combination thereof. In some embodiments, the prior agent is imatinib and the mutation is an imatinib-resistant mutation. In one embodiment, the tumor has a mutation that is resistant to a prior agent. In one embodiment, the mutation that is resistant to the prior agent is selected from an exon 13 KIT mutation, an exon 17 KIT mutation, an exon 18 KIT mutation, and an exon 14 KIT mutation, and combinations thereof. In some embodiments, the mutation that is resistant to the prior agent is an exon 13 KIT mutation, e.g., an exon 13 KIT mutation selected from V654A, N655K and K642E, and combinations thereof. In some embodiments, the mutation that is resistant to the prior agent is an exon 13 KIT mutation, e.g., an exon 13 KIT mutation selected from V654A, N655K, and a combination thereof. In some embodiments, the mutation that is resistant to the prior agent is an exon 17 KIT mutation, e.g., an exon 17 KIT mutation selected from N822K, D816V, D816E, D816F, D816H, D816I, D816Y, D820E, D820Y, D823D and a combination thereof. In some embodiments, the mutation that is resistant to the prior agent is an exon 17 KIT mutation, e.g., an exon 17 KIT mutation, e.g., an exon 17 KIT mutation selected from N822K, D816V, D816E, D816F, D816H, D816I, D816Y, D820E, D820Y and D823D, and combinations thereof. In some embodiments, the exon 17 KIT mutation is A829P. In some embodiments, the exon 18 KIT mutation is A829P. In some embodiments, the mutation that is resistant to the prior agent is an exon 17 KIT mutation selected from N822K, D816V, D816E, D816F, D816H, D816I, D816Y, D820E, D820Y, D823D, A829P, and a combination thereof. In some embodiments, the mutation that is resistant to the prior agent is an exon 17 KIT mutation, e.g., an exon 17 KIT mutation selected from N822K, D816V, D816E, D816F, D816H, D816I, D816Y, D820E, D820Y, D823D and A829P, and combinations thereof. In some embodiments, the mutation that is resistant to the prior agent is an exon 14 KIT mutation, e.g., an exon 14 KIT mutation such as N680K. In some embodiments, the tumor is or has become resistant to the prior agent has one or more mutations.

In some embodiments, the tumor is mutation resistant to the prior treatment as a consequence of one or more exon 17 mutation(s). Examples of exon 17 inhibitors that can inhibit such exon 17 mutation(s), which is causing the tumor to be resistant to the prior treatment, and thereby treat the tumor are selected from avapritinib, BLU-263, ripretinib, AZD3229 and bezuclastinib. Other mutations that may be inhibited by the exon 17 inhibitors described above include N655K, N680K, or a combination thereof.

In some embodiments, the tumor after prior treatment (e.g., with Primary KIT Inhibitor such as imatinib) has one or more exon 13 mutation(s), such as exon 13 KIT mutations selected from V654A, N655K and K642E, and combinations thereof. In some embodiments, the tumor after prior treatment (e.g., with Primary KIT Inhibitor such as imatinib) has one or more exon 13 mutation(s), such as exon 13 KIT mutations selected from V654A, N655K, and a combination thereof.

In one embodiment, the invention provides a method of treating a patient suffering from gastrointestinal stromal tumor (GIST), comprising administering to the patient an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing). In some embodiments, the patient received one or more prior treatments for the GIST. In some embodiments, the GIST progressed after the prior treatment. In some embodiments, the prior treatment comprises administering one or more agents. In some embodiments, the one or more agents comprises imatinib or a pharmaceutically acceptable salt thereof. In some embodiments, the one or more agents comprises avapritinib or a pharmaceutically acceptable salt thereof. In some embodiments, the prior treatment comprises administering one or more agents, each independently selected from: imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus and larotrectinib, and pharmaceutically acceptable salts thereof. In some embodiments, the prior treatment comprises administering imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus or larotrectinib, or a pharmaceutically acceptable salt thereof, or a combination thereof. In some embodiments, the prior treatment comprises administering imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus or larotrectinib, or a pharmaceutically acceptable salt thereof. In some embodiments, the prior treatment comprises administering imatinib or a pharmaceutically acceptable salt thereof. In some embodiments, the prior treatment comprises administering sunitinib or a pharmaceutically acceptable salt thereof. In some embodiments, the prior treatment comprises administering regorafenib or a pharmaceutically acceptable salt thereof. In some embodiments, the prior treatment comprises administering ripretinib or a pharmaceutically acceptable salt thereof. In some embodiments, the prior treatment comprises administering avapritinib or a pharmaceutically acceptable salt thereof. In some embodiments, the prior treatment comprises administering bezuclastinib or a pharmaceutically acceptable salt thereof. In some embodiments, the prior treatment comprises administering AZD3229 or a pharmaceutically acceptable salt thereof. In some embodiments, the prior treatment comprises administering BLU-263 or a pharmaceutically acceptable salt thereof. In some embodiments, the GIST is characterized by a tumor with one or more KIT mutations. In some embodiments, the tumor has a primary activating KIT mutation. In some embodiments, the GIST is characterized by a tumor with one or more KIT mutations, each independently selected from an exon 9 KIT mutation, an exon 11 KIT mutation, an exon 13 KIT mutation and an exon 17 KIT mutation, and combinations thereof. In some embodiments, each of the one or more KIT mutations is independently selected from N822K, D816V, D816E, D816F, D816H, D816I, D816Y, D820E, D820Y, D823D, A829P, K642E, V654A and N655K, and combinations thereof. In some embodiments, each of the one or more KIT mutations is independently selected from N822K, D816V, D816E, D816F, D816H, D816I, D816Y, D820E, D820Y, D823D, A829P and K642E, and combinations thereof. In some embodiments, the tumor has an exon 9 KIT mutation. In some embodiments, the tumor has an exon 11 KIT mutation. In some embodiments, the tumor has an exon 17 KIT mutation. In some embodiments, the exon 17 KIT mutation is selected from N822K, D816V, D816E, D816F, D816H, D816I, D816Y, D820E, D820Y, D823D, and A829P. In some embodiments, the exon 17 KIT mutation is selected from N822K, D816V, D816E, D816F, D816H, D816I, D816Y, D820E, D820Y and D823D. In some embodiments, the exon 17 KIT mutation is D816V. In some embodiments, the tumor has an exon 13 KIT mutation. In some embodiments, the exon 13 KIT mutation is selected from K642E, V654A and N655K, and combinations thereof. In some embodiments, the exon 13 KIT mutation is K642E. In some embodiments, the exon 17 mutation is A829P. In some embodiments, the exon 18 mutation is A829P. In some embodiments, the tumor is mutation resistant to the one or more prior treatments. In some embodiments, the tumor is mutation resistant to imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus or larotrectinib, or a pharmaceutically acceptable salt thereof, or a combination thereof. In some embodiments, the tumor is mutation resistant to imatinib. In some embodiments, the tumor has an imatinib-resistant mutation. In some embodiments, the tumor has an exon 13 inhibitor-resistant mutation. In some embodiments, the tumor has an exon 14 inhibitor-resistant mutation. In some embodiments, the tumor has an exon 17 inhibitor-resistant mutation. In some embodiments, the exon 17 inhibitor is selected from avapritinib, BLU-263, ripretinib, AZD3229 and bezuclastinib, and pharmaceutically acceptable salts thereof. In some embodiments, the exon 17 inhibitor is avapritinib or a pharmaceutically acceptable salt thereof. In some embodiments, the exon 17 inhibitor-resistant mutation is N655K. In some embodiments, the exon 17 inhibitor-resistant mutation is N680K. In some embodiments, the imatinib-resistant mutation is selected from an exon 13 KIT mutation, an exon 17 KIT mutation and an exon 14 KIT mutation, and combinations thereof. In some embodiments, the imatinib-resistant mutation is an exon 13 KIT mutation. In some embodiments, the exon 13 KIT mutation is V654A or N655K. In some embodiments, the imatinib-resistant mutation is an exon 17 KIT mutation. In some embodiments, the exon 17 KIT mutation is selected from N822K, D816V, D816E, D816F, D816H, D816I, D816Y, D820E, D820Y, D823D, and A829P. In some embodiments, the exon 17 KIT mutation is selected from N822K, D816V, D816E, D816F, D816H, D816I, D816Y, D820E, D820Y and D823D. In some embodiments, the exon 17 KIT mutation is D816V. In some embodiments, the exon 17 KIT mutation is D816E. In some embodiments, the exon 17 KIT mutation is A829P. In some embodiments, the exon 18 KIT mutation is A829P. In some embodiments, the imatinib-resistant mutation is an exon 14 KIT mutation. In some embodiments, the exon 14 KIT mutation is N680K.

Another embodiment of the invention is a method of treating a patient suffering from gastrointestinal stromal tumor (GIST), comprising administering to the patient a combination comprising (i) a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), and (ii) one or more agents *(e.g.,* 1, 2, 3, or 4 additional agents).

In one embodiment the patient suffering from GIST is administered a combination comprising (i) an effective amount of a compound of the invention, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or a pharmaceutical composition disclosed herein *(e.g.,* a pharmaceutical composition comprising a pharmaceutically acceptable carrier or excipient and a compound disclosed herein or a pharmaceutically acceptable salt thereof, *e.g.,* a compound of Formula (I), (II), (III), (IV), (V), (VI), (VII), (VIII), (IX) or (X), or a compound of Table 1, or a pharmaceutically acceptable salt of any of the foregoing) and (ii) a Primary Kit Inhibitor (e.g., imatinib) as the additional agent. In one embodiment, the patient suffering from GIST is administered a combination comprising (i) a compound of the invention, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or a pharmaceutical composition disclosed herein *(e.g.,* a pharmaceutical composition comprising a pharmaceutically acceptable carrier or excipient and a compound disclosed herein or a pharmaceutically acceptable salt thereof, *e.g.,* a compound of Formula (I), (II), (III), (IV), (V), (VI), (VII), (VIII), (IX) or (X), or a compound of Table 1, or a pharmaceutically acceptable salt of any of the foregoing) and (ii) an exon 9 or exon 11 inhibitor as the additional agent. In one embodiment, the patient suffering from GIST is administered a combination comprising (i) an effective amount of a compound of the invention, or a pharmaceutically acceptable salt thereof (e.g., a compound of Formula (I), (II), (III), (IV), (V), (VI), (VII), (VIII), (IX) or (X), or a compound of Table 1, or a pharmaceutically acceptable salt of any of the foregoing), or a pharmaceutical composition disclosed herein *(e.g.,* a pharmaceutical composition comprising a pharmaceutically acceptable carrier or excipient and a compound disclosed herein or a pharmaceutically acceptable salt thereof, *e.g.,* a compound of Formula (I), (II), (III), (IV), (V), (VI), (VII), (VIII), (IX) or (X), or a compound of Table 1, or a pharmaceutically acceptable salt of any of the foregoing) and (ii) an exon 17 inhibitor as the additional agent.

In another embodiment the patient suffering from GIST is administered a combination comprising (i) an effective amount of a compound of the invention, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or a pharmaceutical composition disclosed herein *(e.g.,* a pharmaceutical composition comprising a pharmaceutically acceptable carrier or excipient and a compound disclosed herein or a pharmaceutically acceptable salt thereof, *e.g.,* a compound of Formula (I), (II), (III), (IV), (V), (VI), (VII), (VIII), (IX) or (X), or a compound of Table 1, or a pharmaceutically acceptable salt of any of the foregoing), and as additional agents: (ii) a Primary Kit Inhibitor (e.g., imatinib), or a pharmaceutically acceptable salt thereof, and (iii) at least one agent selected from regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, sunitinib, avapritinib, BLU-263, ripretinib, AZD3229, bezuclastinib, everolimus and larotrectinib, and pharmaceutically acceptable salts thereof. In one embodiment, the additional agent (iii) is selected from regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, sunitinib, avapritinib, BLU-263, ripretinib, AZD3229 and bezuclastinib, and pharmaceutically acceptable salts thereof. In one embodiment, the additional agent (iii) is an exon 17 inhibitor. In one embodiment, the additional agent (iii) is selected from avapritinib, BLU-263, ripertinib, AZD3229, and bezuclastinib, and pharmaceutically acceptable salts thereof. In one embodiment, the additional agent (iii) is avapritinib or a pharmaceutically acceptable salt thereof. In one embodiment, the additional agent (iii) is BLU-263 or a pharmaceutically acceptable salt thereof. In one embodiment, the additional agent (iii) is ripertinib or a pharmaceutically acceptable salt thereof. In one embodiment, the additional agent (iii) is AZD3229 or a pharmaceutically acceptable salt thereof. In one embodiment, the additional agent (iii) is bezuclastinib or a pharmaceutically acceptable salt thereof.

In another embodiment the patient suffering from GIST is administered a combination *(e.g.,* an effective combination) comprising (i) a compound disclosed herein or the pharmaceutically acceptable salt thereof *(e.g.,* a compound of Formula (I), (II), (III), (IV), (V), (VI), (VII), (VIII), (IX), (X), or a compound of Table 1, or a pharmaceutically acceptable salt of any of the foregoing), or a pharmaceutical composition disclosed herein *(e.g.,* a pharmaceutical composition comprising a pharmaceutically acceptable carrier or excipient and a compound disclosed herein or a pharmaceutically acceptable salt thereof, *e.g.,* a compound of Formula (I), (II), (III), (IV), (V), (VI), (VII), (VIII), (IX) or (X), or a compound of Table 1, or a pharmaceutically acceptable salt of any of the foregoing), and as additional agents: (ii) a Primary Kit Inhibitor (e.g., imatinib), or a pharmaceutically acceptable salt thereof, (iii) an exon 17 inhibitor and (iv) at least one agent selected from regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib and sunitinib, and pharmaceutically acceptable salts thereof. In any of the foregoing embodiments, the exon 17 inhibitor is selected from avapritinib, BLU-263, ripertinib, AZD3229, and bezuclastinib. In one embodiment, the exon 17 inhibitor is selected from avapritinib, BLU-263, ripertinib, AZD3229, and bezuclastinib. In one embodiment, the exon 17 inhibitor is avapritinib or a pharmaceutically acceptable salt thereof. In one embodiment, the exon 17 inhibitor is BLU-263 or a pharmaceutically acceptable salt thereof. In one embodiment, the exon 17 inhibitor is ripertinib or a pharmaceutically acceptable salt thereof. In one embodiment, the exon 17 inhibitor is AZD3229 or a pharmaceutically acceptable salt thereof. In one embodiment, the exon 17 inhibitor is bezuclastinib or a pharmaceutically acceptable salt thereof.

In one aspect the combination therapy described in this paragraph can be administered as a first line therapy, i.e., before resistant mutations have been identified, or as second, third, or fourth line therapy, i.e., after one or more resistance conferring mutations have emerged. When a combination of agents is administered, the agents in the combination can be administered simultaneously (in the same or different formulations) or concurrently in any order.

Another embodiment of the invention is a method of treating a patient suffering from gastrointestinal stromal tumor (GIST), comprising administering to the patient an effective combination comprising a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), and at least one additional agent. In some embodiments, the additional agent is an exon 9 KIT inhibitor or exon 11 KIT inhibitor. In some embodiments, the additional agent is selected from imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus and larotrectinib, and pharmaceutically acceptable salts thereof. In some embodiments, the additional agent is imatinib or a pharmaceutically acceptable salt thereof. In some embodiments, the additional agent is an exon 17 KIT inhibitor. In some embodiments, the additional agent is selected from avapritinib, BLU-263, ripretinib, AZD3229, and bezuclastinib. In some embodiments, the additional agent is selected from avapritinib, BLU-263, ripretinib, AZD3229, and bezuclastinib.

Another embodiment of the invention is a method of treating a patient suffering from gastrointestinal stromal tumor (GIST), comprising administering to the patient a combination comprising: an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), and one or more agents. In some embodiments, the agent is an exon 9 KIT inhibitor or exon 11 KIT inhibitor. In some embodiments, the agent is selected from imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus and larotrectinib, and pharmaceutically acceptable salts thereof. In some embodiments, the agent is imatinib or a pharmaceutically acceptable salt thereof. In some embodiments, the additional agent is an exon 17 KIT inhibitor. In some embodiments, the agent is selected from avapritinib, BLU-263, ripretinib, AZD3229, and bezuclastinib.

Another embodiment of the invention is a method of treating a patient suffering from a malignant disease (e.g., GIST) characterized by a tumor with an exon 9 KIT mutation, an exon 11 KIT mutation, or an exon 17 KIT mutation, or a combination thereof. The method comprises: (a) obtaining a biological sample from the patient; (b) detecting the presence or absence of an exon 13 KIT mutation or exon 14 KIT mutation; and (c) administering an effective amount of a compound of the invention, or a pharmaceutically acceptable salt thereof, or a pharmaceutical composition disclosed herein, to the patient, if the mutation is detected. In one aspect, the presence of absence of an exon 13 KIT mutation is detected in step b), for example, wherein the exon 13 KIT mutation is V654A or N655K. In another aspect, the presence or absence of an exon 14 KIT mutation is detected in step (b), for example, wherein the exon 14 KIT mutation is N680K. The presence of absence of exon 13 mutations V654A or N655K or exon 14 mutation N680K is detected according to methods disclosed in WO 2020/102095.

Another embodiment of the invention is a method of treating a patient suffering from a malignant disease characterized by a tumor with an exon 9 KIT mutation, an exon 11 KIT mutation, or an exon 17 KIT mutation, or a combination thereof, comprising (a) obtaining a biological sample from the patient; (b) detecting the presence or absence of an exon 13 KIT mutation or exon 14 KIT mutation; and (c) administering an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), to the patient, if the mutation is present. In one aspect, the presence of absence of an exon 13 KIT mutation is detected in step (b), for example, wherein the exon 13 KIT mutation is V654A or N655K. In another aspect, the presence or absence of an exon 14 KIT mutation is detected in step (b), for example, wherein the exon 14 KIT mutation is N680K. The presence of absence of exon 13 mutations V654A or N655K or exon 14 mutation N680K is detected according to methods disclosed in WO 2020/102095.

Another embodiment of the invention is a method of treating a patient suffering from a malignant disease characterized by a tumor with an exon 9 KIT mutation, an exon 11 KIT mutation or an exon 17 KIT mutation, or a combination thereof, comprising administering an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), to the patient, if an exon 13 KIT mutation or exon 14 KIT mutation is detected. In some embodiments, the exon 13 KIT mutation is V654A or N655K. In some embodiments, the exon 14 KIT mutation is N680K.

A method of treating a patient suffering from a malignant disease characterized by a tumor with an exon 9 KIT mutation, an exon 11 KIT mutation or an exon 17 KIT mutation, or a combination thereof, comprising administering an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or administering an effective amount of a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), to the patient, if an exon 13 KIT mutation or exon 14 KIT mutation is present. In some embodiments, the exon 13 KIT mutation is V654A or N655K. In some embodiments, the exon 14 KIT mutation is N680K.

Another embodiment of the invention is a method of treating a patient suffering from gastrointestinal stromal tumor (GIST), comprising administering to the patient an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), wherein the GIST has a mutation resistant to a KIT inhibitor administered to treat GIST with a primary activating mutation in exon 9 or exon 11. In some embodiments, the KIT inhibitor is selected from imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus and larotrectinib, and pharmaceutically acceptable salts thereof. In some embodiments, the KIT inhibitor is imatinib or a pharmaceutically acceptable salt thereof. In some embodiments, the KIT inhibitor is bezuclastinib or a pharmaceutically acceptable salt thereof. In some embodiments, the KIT inhibitor is AZD3229 or a pharmaceutically acceptable salt thereof. In some embodiments, the KIT inhibitor is BLU-263 or a pharmaceutically acceptable salt thereof. In some embodiments, the mutation is an exon 13 KIT mutation. In some embodiments, the exon 13 KIT mutation is V654A or N655K.

Another embodiment of the invention is a method of treating a patient suffering from a primary gastrointestinal stromal tumor (GIST) characterized by a primary activating mutation in exon 9 KIT or exon 11 KIT, comprising administering to the patient: (i) an effective amount of a compound disclosed herein, or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing), or a pharmaceutical composition comprising a pharmaceutically acceptable carrier or excipient and a compound disclosed herein or a pharmaceutically acceptable salt thereof *(e.g.,* a compound of Formula (I), (II), (III), (IV), (V), (VI), (VII), (VIII), (IX) or (X), or a compound of Table 1, or a pharmaceutically acceptable salt of any of the foregoing); and (ii) an effective amount of a KIT inhibitor effective against GIST characterized by a primary activating mutation in exon 9 KIT or exon 11 KIT. In some embodiments, the KIT inhibitor is selected from imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus and larotrectinib, and pharmaceutically acceptable salts thereof. In some embodiments, the KIT inhibitor is imatinib or a pharmaceutically acceptable salt thereof. In some embodiments, the KIT inhibitor is bezuclastinib or a pharmaceutically acceptable salt thereof. In some embodiments, the KIT inhibitor is AZD3229 or a pharmaceutically acceptable salt thereof. In some embodiments, the KIT inhibitor is BLU-263 or a pharmaceutically acceptable salt thereof.

### Pharmaceutical Compositions

While it is possible for a compound of the invention to be administered alone, it is preferable to administer the compound as a pharmaceutical formulation, where the compound is combined with one or more pharmaceutically acceptable excipients or carriers. The compounds of the invention or pharmaceutically acceptable salts thereof may be formulated for administration in any convenient way for use in human or veterinary medicine. In certain embodiments, the compound included in the pharmaceutical preparation may be active itself, or may be a prodrug, e.g., capable of being converted to an active compound in a physiological setting.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Examples of pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; (21) cyclodextrins; and (22) other non-toxic compatible substances employed in pharmaceutical formulations.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Solid dosage forms (e.g., capsules, tablets, pills, dragees, powders, granules and the like) can include one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents.

Liquid dosage forms can include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Suspensions, in addition to compounds of the invention or pharmaceutically acceptable salts thereof, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Ointments, pastes, creams and gels may contain, in addition to compounds of the invention or pharmaceutically acceptable salts thereof, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to compounds of the invention or pharmaceutically acceptable salts thereof, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of compound of the invention or pharmaceutically acceptable salt thereof that can be combined with a carrier material to produce a single dosage form will generally be that amount of the compound which produces a therapeutic effect.

Dosage forms for the topical or transdermal administration of a compound of this invention or pharmaceutically acceptable salts thereof, include powders, sprays, ointments, pastes, creams, lotions, gels, solutions, patches and inhalants. The active compound may be mixed under sterile conditions with a pharmaceutically acceptable carrier, and with any preservatives, buffers, or propellants that may be required.

When the compounds of the invention or pharmaceutically acceptable salths thereof are administered as pharmaceuticals, to humans and animals, they can be given per se or as a pharmaceutical composition containing, for example, 0.1 to 99.5% (more preferably, 0.5 to 90%) of active ingredient in combination with a pharmaceutically acceptable carrier.

The formulations can be administered topically, orally, transdermally, rectally, vaginally, parentally, intranasally, intrapulmonary, intraocularly, intravenously, intramuscularly, intraarterially, intrathecally, intracapsularly, intradermally, intraperitoneally, subcutaneously, subcuticularly, or by inhalation.

### Dosages

Actual dosage levels of the active ingredients in the pharmaceutical compositions of this invention may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular active ingredient employed, the route of administration, the time of administration, the rate of excretion of the particular active ingredient being employed, the duration of the treatment, other drugs, compounds and/or materials used in combination with the particular active ingredient employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound that is the lowest dose effective to produce a therapeutic effect. Such an effective dose will generally depend upon the factors described above.

The terms "administer", "administering", "administration", and the like, as used herein, refer to methods that may be used to enable delivery of compositions to the desired site of biological action. These methods include, but are not limited to, intraarticular (in the joints), intravenous, intramuscular, intratumoral, intradermal, intraperitoneal, subcutaneous, orally, topically, intrathecally, inhalationally, transdermally, rectally, and the like. Administration techniques that can be employed with the agents and methods described herein are found in *e.g.,* Goodman and Gilman, The Pharmacological Basis of Therapeutics, current ed.; Pergamon; and Remington's, Pharmaceutical Sciences (current edition), Mack Publishing Co., Easton, Pa.

The particular mode of administration and the dosage regimen will be selected by the attending clinician, taking into account the particulars of the case *(e.g.* the subject, the disease, the disease state involved, the particular treatment, and whether the treatment is prophylactic). Treatment can involve daily or multi-daily or less than daily (such as weekly or monthly etc.) doses over a period of a few days to months, or even years.

The pharmaceutical composition of the invention is formulated to be compatible with its intended route of administration. In an embodiment, the composition is formulated in accordance with routine procedures as a pharmaceutical composition adapted for intravenous, subcutaneous, intramuscular, oral, intranasal, or topical administration to human beings. In preferred embodiments, the pharmaceutical composition is formulated for intravenous administration.

"Pharmaceutically acceptable excipient" and "pharmaceutically acceptable carrier" refer to a substance that aids the formulation and/or administration of an active agent to and/or absorption by a subject and can be included in the compositions of the present invention without causing a significant adverse toxicological effect on the subject. Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxymethycellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with or interfere with the activity of the compounds provided herein. One of ordinary skill in the art will recognize that other pharmaceutical excipients are suitable for use with disclosed compounds.

### EXEMPLIFICATION

The following examples are intended to be illustrative and are not meant in any way to be limiting.

### Abbreviations

Abbreviations and acronyms used herein include the following:
AcOH means acetic acid;
AIBN means 2,2'-azobis(2-methylpropionitrile);
t-AmOH means tert-amyl alcohol;
Aq. means aqueous;
Boc means tert-butoxy carbonyl;
(BPin)₂ means 4,4,4',4',5,5,5',5'-Octamethyl-2,2'-bi-1,3,2-dioxaborolane;
br means broad;
Brettphos means 2-(Dicyclohexylphosphino)3,6-dimethoxy-2',4',6'-triisopropyl-1,1'-biphenyl;
BrettPhos Pd G3 means [(2-Di-cyclohexylphosphino-3,6-dimethoxy-2',4',6'- triisopropyl-1,1'-biphenyl)-2-(2'-amino-1,1'-biphenyl)]palladium(II) methane sulfonate;
BrettPhos Pd G4 means dicyclohexyl-[3,6-dimethoxy-2-[2,4,6-tri(propan-2-yl)phenyl] phenyl]phosphane;methanesulfonic acid;*N*-methyl-2-phenylaniline;palladium
n-BuOH means butan-1-ol;
t-BuOK means potassium tert-butoxide;
d means doublet;
dd means doublet of doublets;
DAST means Diethylaminosulfur trifluoride;
DCM means dichloromethane;
DIAD means diisopropyl azodicarboxylate;
DIPEA means N-ethyldiisopropylamine or N,N-diisopropylethylamine;
DMA means N,N-Dimethylacetamide;
DMF means N,N-dimethylformamide;
DMSO means Dimethylsulfoxide;
EtOAc means ethyl acetate;
EtOH means ethanol;
EtONa means sodium ethoxide;
Eq. means equivalent;
FA means formic acid;
HATU means 1-[bis(dimethylamino)methylene]-1H-1,2,3-triazolo[4,5-b]pyridinium 3-oxid hexafluorophosphate;
IPA means 2-propanol;
[Ir(OMe)(1,5-cod)]₂ means Bis(1,5-cyclooctadiene)di-µ-methoxydiiridium(I);
KOAc means potassium acetate;
LCMS means liquid chromatography mass spectrometry;
m means multiplet;
MeCN means acetonitrile;
MeI means iodomethane;
MeNH₂ means methylamine;
MeOH means methanol;
MeOH-d₄ means deutero-methanol;
MPLC means medium pressure liquid chromatography;
MS m/z means mass spectrum peak;
NBS means N-bromosuccinimide;
NMP means N-methyl pyrrolidine;
PE means petroleum ether;
Pd(amphos)Cl₂ means Bis(di-tert-butyl(4-dimethylaminophenyl)phosphine) dichloro palladium(II);
Pd(t-Bu₃P)₂ means Bis(tri-tert-butylphosphine)palladium(0);
Pd₂(dba)₃ means tris(dibenzylideneacetone)dipalladium (0);
Pd(dppf)Cl₂ means [1,1'-bis(diphenylphosphino)ferrocene]dichloropalladium(II);
PG means protecting group;
i-PrMgCl means isopropyl magnesium chloride;
q means quartet;
rt means room temperature;
RT means retention time;
s means singlet;
sat. means saturated;
SFC means supercritical fluid chromatography;
t means triplet;
T3P means 2,4,6-Tripropyl-1,3,5,2,4,6-trioxatriphosphorinane-2,4,6-trioxide;
TBAF means Tetrabutylammonium fluoride;
TBDMS means tert-Butyldimethylsilane;
TBDMSCl means tert-Butyl(chloro)dimethylsilane;
TEA means triethylamine;
TFA means trifluoroacetic acid;
TfOH means trifluoroethanesulfonic acid;
THF means tetrahydrofuran;
TLC means thin layer chromatography;
TMSN₃ means trimethylsilyl azide;
Xantphos means 4,5-Bis(diphenylphosphino)-9,9-dimethylxanthene; and
XPhos Pd G2 means Chloro(2-dicyclohexylphosphino-2',4',6'-triisopropyl-1,1'-biphenyl)[2-(2'-amino-1,1'-biphenyl)]palladium(II).

### General Synthetic Methods and Intermediates

According to a first process, compounds of Formula (I) may be prepared from the compounds of Formulae (ii) and (iii), as illustrated by Scheme 1.

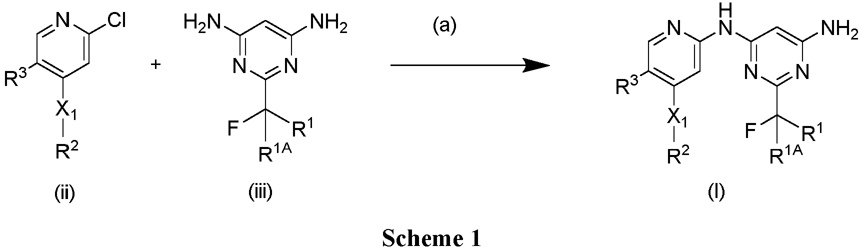

The compound of Formula (I) may be prepared from the compounds of Formulae (ii) and (iii) according to process step (a) a Buchwald-Hartwig cross-coupling. Typical conditions comprise, reaction of the amine of Formula (iii) with the chloride of Formula (ii) in the presence of a suitable inorganic base, a suitable catalyst in a suitable solvent at elevated temperature. Preferred conditions comprise, reaction of the compounds of Formulae (ii) and (iii) in the presence of, BrettPhos Pd G3, BrettPhos Pd G4, Pd(t-Bu₃P)₂ or Xantphos, optionally in conjunction with Pd₂(dba)₃ or BrettPhos in the presence of a suitable base such as Cs₂CO₃, K₂CO₃, or KOAc in a suitable solvent such as dioxane, toluene, t-AmOH, NMP or DMF, at between 90°C and 130°C.

According to a second process, the compound of Formula (I) may be prepared from the compounds of Formulae (ii), (iv) and (v), as illustrated in Scheme 2.

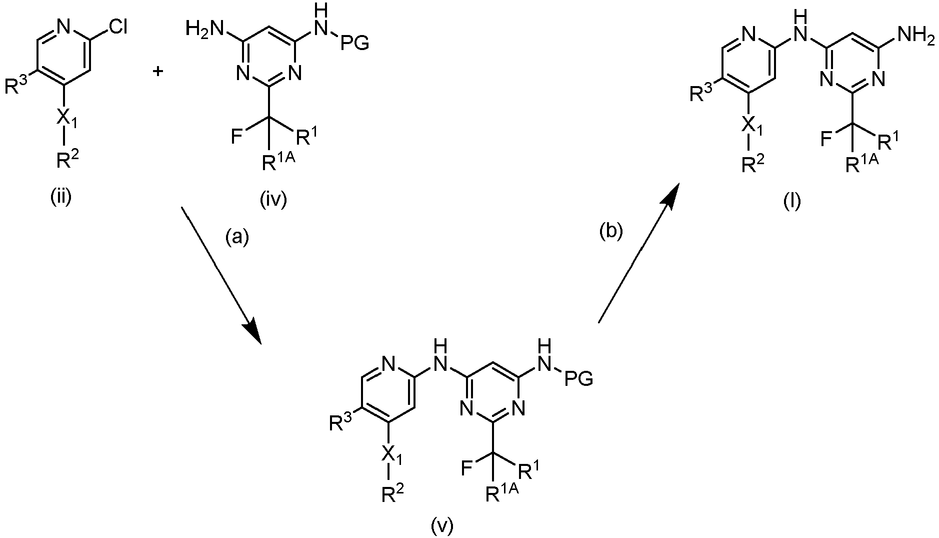

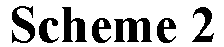

PG is a suitable amine protecting group, preferably 2,4-dimethoxybenzyl or tButyl.

The compound of Formula (v) may be prepared from the compounds of Formulae (ii) and (iv) according to process step (a) a Buchwald-Hartwig cross-coupling reaction as previously described in Scheme 1.

The compound of Formula (I) may be prepared from the protected compound of Formula (v) by process step (b), a de-protection reaction. Typical conditions comprise reaction of the compound of Formula (v) with a suitable acid, such as HCl, or TFA in a suitable solvent such as dioxane, THF or DCM at about rt.

According to a third process, the compound of Formula (v) may be prepared from the compounds of Formulae (vi), and (vii) as illustrated in Scheme 3.

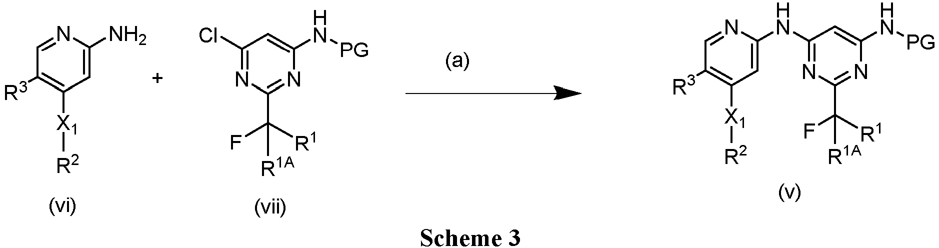

The compound of Formula (v) may be prepared from the compounds of Formulae (vi) and (vii) by process step (a), a Buchwald-Hartwig cross-coupling reaction, as previously described in Scheme 1.

According to a fourth process, the compound of Formula (vi) may be prepared from the compounds of Formulae (ii) and (viii), as illustrated in Scheme 4.

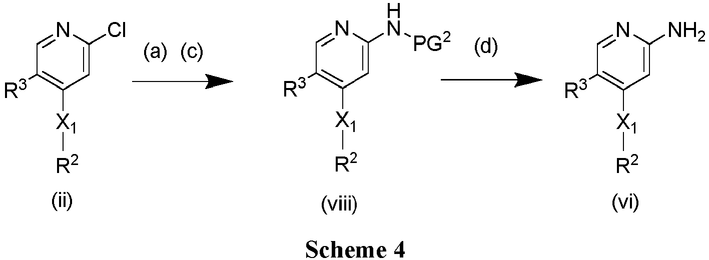

PG² is a suitable nitrogen protecting group, preferably benzyl or diphenylmethylene

The compound of Formula (viii) may be prepared from the compound of Formula (ii) and PG²NH₂ by process Step (c), an amination reaction, wherein PG² is a benzyl group. Typical conditions comprise reaction of the compound of Formula (ii), with PG²NH₂ at elevated temperature, such as 200°C and under microwave irradition.

Alternatively, wherein PG² is a diphenylmethylene group, the compound of Formula (viii) may be prepared from the compound of Formula (ii) and PG²NH₂ by process Step (a), a cross coupling reaction, as previously described in Scheme 1.

The compound of Formula (vi) may be prepared from the compound of Formula (viii) by process Step (d) a deprotection reaction. Typical conditions comprise reaction of the compound of Formula (viii) with a suitable acid such as triflic acid or HCl in a suitable solvent, such as DCM at about rt.

According to a fifth process, the compound of Formula (ii) may be prepared from the compounds of Formula (ix), (x), (xi), (xii), (xiii), (xiv), (xv) and (xvi) as illustrated in Scheme 5. 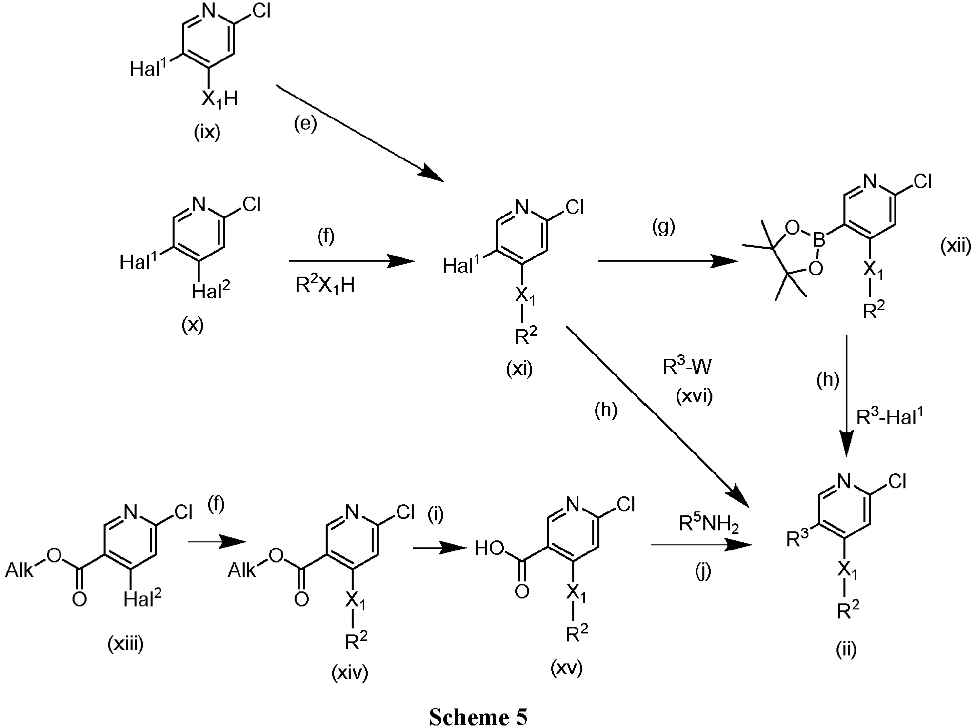
Hal¹ is a halogen, preferably Br or I
Hal² is a halogen, preferably Cl or F
W is Sn(n-Bu)₃ or B(Pin)
Alk is a C₁-C₄ alkyl group

Wherein X₁ is O, the compound of Formula (xi) may be prepared from the compound of Formula (ix) by process step (e). Typical conditions comprise reaction of the compound of Formula (ix) with a suitable alkyl fluorinating agent such as ethyl 2-bromo-2,2-difluoroacetate, in the presence of a suitable base such as K₂CO₃, in a suitable solvent such as DMF, at elevated temperature, typically at about 60°C.

The compound of Formula (xi) may be prepared from the compound of Formula (x) by process step Step (f). Typical conditions comprise reaction of the compound of Formula (x) with R²X₁H, in the presence of a suitable organic or inorganic base such as DIPEA or TEA, Cs₂CO₃, t-BuOK, NaH or KF, optionally in a suitable solvent such as THF, DMF, DMA, DMSO, dioxane, n-BuOH or MeCN at between 0°C and 140°C, optionally under microwave irradiation.

The compound of Formula (xii) may be prepared from the compound of Formula (xi), according to process step (g), a boronate ester formation achieved by treatment with a suitable boronate such as (BPin)₂, in the presence of a suitable inorganic base, such as K₂CO₃ or KOAc and a suitable catalyst, such as, Pd(dppf)Cl₂ in a suitable non-polar solvent at between rt and elevated temperature. Preferred conditions comprise, treatment of the compound of Formula (xi) with (BPin)₂ in the presence of Pd(dppf)Cl₂ and KOAc in dioxane, at 90°C.

Wherein, R³ is a 5 membered aromatic heterocycle, as defined previously, the compound of Formula (ii) may be prepared from the compound of Formula (xi) by process step (h), a palladium catalysed cross-coupling reaction. Such cross-coupling reactions may include a Suzuki type reaction, wherein W is a boronic acid or ester, or a Stille type cross coupling reaction, when W is an alkyl stannane.

Typical cross-coupling conditions comprise a palladium catalyst containing suitable phosphine ligands, such as Pd(amphos)Cl₂, Pd(dppf)Cl₂, Pd(PPh₃)Cl₂ or Xphos Pd G2, in the presence of the compound of Formula (xvi), in the presence of an inorganic or organic base, such as Cs₂CO₃, K₂CO₃, Na₂CO₃, K₃PO₄, TEA or KOAc in a suitable solvent, such as, EtOH, dioxane, aqueous dioxane or DMF at between rt and elevated temperature.

Wherein, R³ is a 5 membered aromatic heterocycle, as defined previously, the compound of Formula (ii) may be prepared from the compound of Formula (xii) and R³-Hal¹, according to process step (h), as previously described.

Wherein R³ is C(O)NHR⁵, the compound of Formula (ii) may be prepared from the compounds of Formulae (xiii), (xiv) and (xv).

The compound of Formula (xiv) may be prepared from the compound of Formula (xiii) and R²X₁H according to process step (f), as previously described.

The compound of Formula (xv) may be prepared from the compound of Formula (xiv) by process step (i), a hydrolysis reaction. Typical conditions comprise reaction of the compound of Formula (xiv) with an alkali metal hydroxide, such as LiOH or NaOH, in aqueous solvent such as MeOH, EtOH or THF at between rt and about 80°C.

The compound of Formula (ii) may be prepared by from the compound of Formula (xv) and R⁵NH₂ according to process step (j), an amide bond formation. Typical conditions comprise reaction of the acid of Formula (xv) with R⁵NH₂, in the presence of a suitable coupling agent and organic base in a suitable polar aprotic solvent. Preferred conditions, comprise the reaction of the acid of Formula (xv) with R⁵NH₂, in the presence of HATU or T₃P, in the presence of a suitable organic base, typically DIPEA or TEA, in a suitable solvent, such as DMF or DCM, at room temperature.

Alternatively, the compound of Formula (ii) may be prepared by the in-situ formation of the acid chloride of the acid of Formula (xv), typically using oxalyl chloride or thionyl chloride in a suitable solvent such as THF or DCM at rt and the subsequent amide bond formation of the acid chloride and the amine R⁵NH₂ in the presence of a suitable organic base, typically DIPEA at 0°C.

According to a sixth process, the compound of Formula (ii) may be prepared from the compounds of Formula (x), (xvi), (xvii), (xviii), (xix) and (xx) as illustrated in Scheme 6.

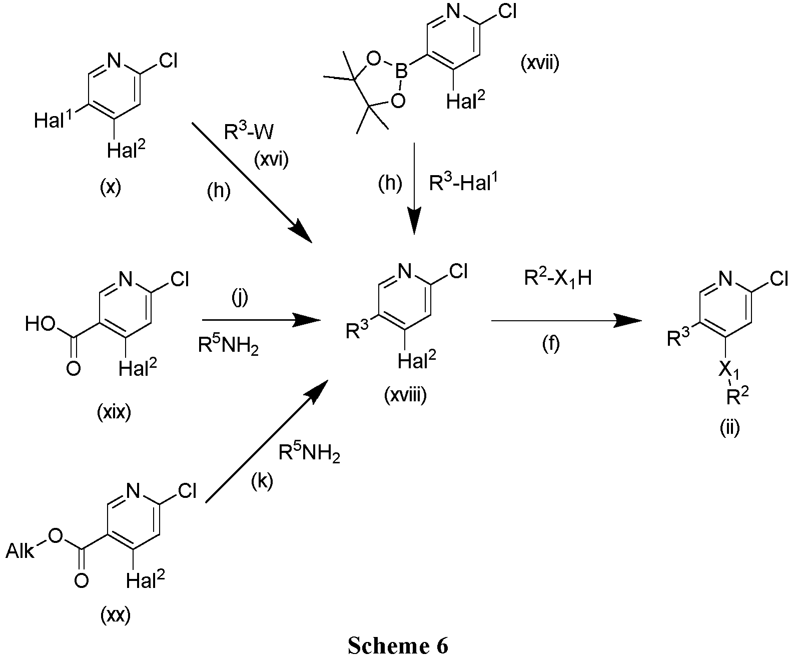

Wherein, R³ is a 5 membered aromatic heterocycle, as defined previously, the compound of Formula (xviii) may be prepared from the compounds of Formulae (x) and (xvi), according to process step (h), as previously described in Scheme 5.

Wherein, R³ is a 5 membered aromatic heterocycle, as defined previously, the compound of Formula (xviii) may be prepared from the compound of Formulae (xvii) and R³-Hal¹, according to process step (h), as previously described in Scheme 5.

Wherein, R³ is C(O)NHR⁵, the compound of Formula (xviii) may be prepared from the compound of Formulae (xix) and R⁵NH₂, according to process step (j), as previously described in Scheme 5.

Wherein, R³ is C(O)NHR⁵, the compound of Formula (xviii) may be prepared from the compound of Formulae (xx) and R⁵NH₂, according to process step (k), an aminolysis reaction. Typical conditions comprise reaction of the compound of Formula (xx) with R⁵NH₂ at elevated temperature such as 70°C in a sealed vessel.

The compound of Formula (ii) may be prepared from the compound of Formulae (xviii) and R²X₁H, according to process step (f), as previously described in Scheme 5.

According to a seventh process, compounds of Formula (vi), may be prepared from compounds of the Formulae (xxi) and (xxii), as illustrated by Scheme 7.

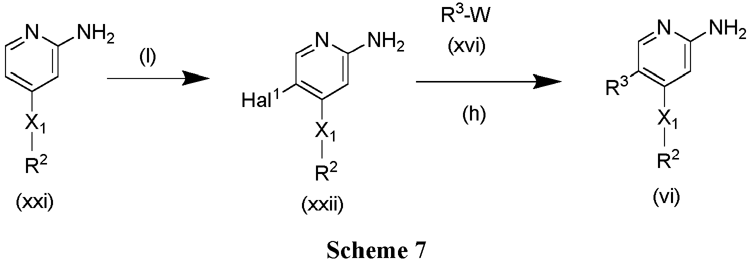

The compound of Formula (xxii) may be prepared from the compound of Formula (xxi) according to process step (l) a halogenation reaction, preferably a bromination reaction. Typical conditions comprise, reaction of the compound of Formula (xxi) with NBS in DMF at rt.

The compound of Formula (vi) may be prepared from the compounds of Formulae (xxii) and (xvi) according to process step (h), as previously described in Scheme 5.

According to an eighth process, compounds of Formula (I) may be prepared from the compounds of Formulae (iii), (xviii) and (xxiii) as illustrated in Scheme 8.

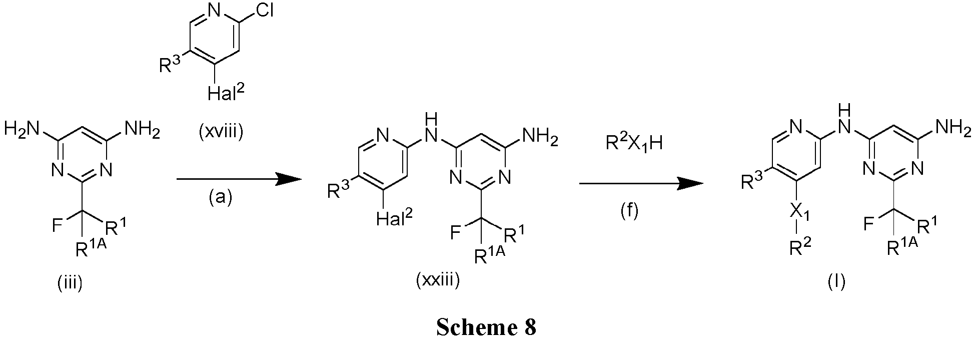

The compound of Formula (xxiii) may be prepared from the compounds of Formulae (iii) and (xviiii) by process step (a), a Buchwald-Hartwig coupling as previously described in Scheme 1.

The compound of Formula (I) may be prepared from the compound of Formula (xxiii) and R²X₁H by process step (f) as previously described in Scheme 5.

According to a ninth process, compounds of Formula (v), wherein R³ is C(O)NHR⁵, may be prepared from the compounds of Formulae (iv), (xiv) and (xxiv) as illustrated in Scheme 9.

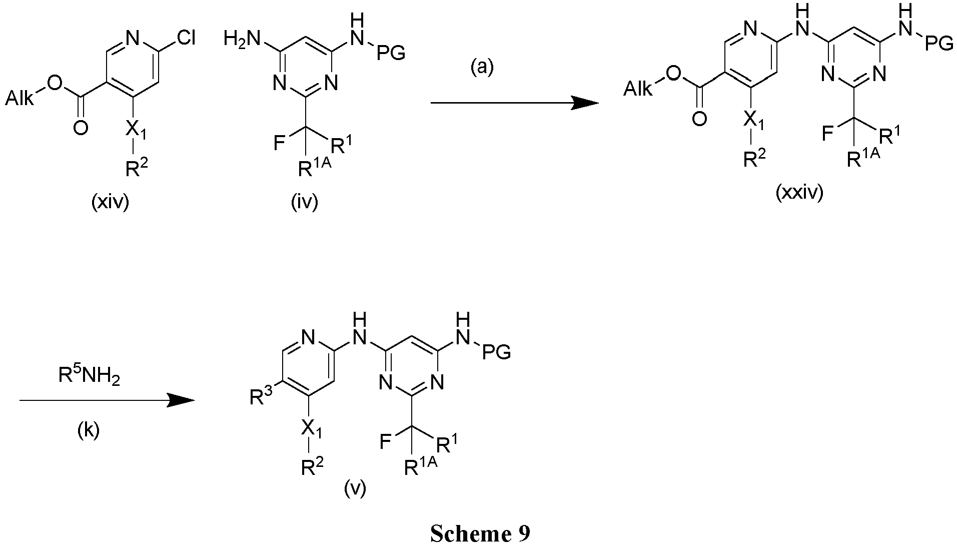

The compound of Formula (xxiv) may be prepared from the compounds of Formulae (xiv) and (iv) according to process step (a), as previously described in Scheme 1.

The compound of Formula (v) may be prepared from the compound of Formula (xxiv) according to process step (k), an aminolysis reaction, as previously described in Scheme 6.

The compounds of Formulae (iii), (iv), (vii), (ix), (x), (xiii), (xvi), (xvii), (xix), (xx) and (xxi) are either commercially available or may be prepared by analogy to methods known in the literature, or the methods described in the experimental sections below.

Compounds of Formulae (I), (ii), (v), (vi) (viii), (xviii) and (xxiii) may be converted to alternative compounds of Formulae (I), (ii), (v), (vi) (viii), (xviii) and (xxiii) by standard chemical transformations, known to those skilled in the art. Examples of these transformations include, but are not limited to reductive amination reaction, reduction of ester to a secondary alcohol using NaBH₄, fluorination, using DAST, bromination using AIBN and NBS, and methods for the synthesis of 5 membered heterocycles from functional groups such as a nitrile, as described in Comprehensive Heterocyclic Chemistry or in the literature.

It will be appreciated by those skilled in the art, that it may be necessary to utilise a suitable protecting group strategy for the preparation of compounds of Formula (I). Typical protecting groups may comprise, carbamate and preferably Boc for the protection of aliphatic amines, or a TBDMS group for the protection of a primary alcohol.

It will be further appreciated that it may be necessary or desirable to carry out the transformations in a different order from that described in the schemes, or to modify one or more of the transformations, to provide the desired compound disclosed herein.

Methods for preparing compounds of the invention can be carried out in suitable solvents which can be readily selected by one of skill in the art of organic synthesis. Suitable solvents can be substantially non-reactive with the starting materials (reactants), intermediates, or products at the temperatures at which the reactions are carried out, *e*.*g*., temperatures which can range from the solvent's freezing temperature to the solvent's boiling temperature. A given reaction can be carried out in one solvent or a mixture of more than one solvent. Depending on the particular reaction step, suitable solvents for a particular reaction step can be selected by the skilled artisan.

Preparation of compounds of the invention can involve the protection and deprotection of various chemical groups. The need for protection and deprotection, and the selection of appropriate protecting groups, can be readily determined by one skilled in the art. The chemistry of protecting groups can be found, for example, in Wuts and Greene, Protective Groups in Organic Synthesis, 5th ed., John Wiley & Sons: New Jersey, (2014).

Reactions can be monitored according to any suitable method known in the art. For example, product formation can be monitored by spectroscopic means, such as nuclear magnetic resonance (NMR) spectroscopy (*e*.*g*., ¹H or ¹³C), infrared (IR) spectroscopy, spectrophotometry (*e*.*g*., UV-visible), mass spectrometry (MS), or by chromatographic methods such as high performance liquid chromatography (HPLC) or thin layer chromatography (TLC). Analytical instruments and methods for compound characterization:

One of ordinary skill in the art will recognize that modifications of the gradient, column length, and flow rate are possible and that some conditions may be more suitable for compound characterization than others, depending on the chemical species being analyzed.

Prep LC-MS: Preparative HPLC was performed using the conditions described below.

| Prep-HPLC Code | Conditions |
|---|---|
| HPLC-1 | Kromasil C18, 250 x 50mm x10 µm; [water (10 mM NH₄HCO₃)-MeCN] |
| HPLC-2 | Welch Xtimate C18 250 x 70mm x 10µm; water (0.05%NH₃H₂O)-MeCN |
| HPLC-3 | Welch Xtimate 150A 250 x 21.2mm x 10µm; water (0.1% NH₄HCO₃), 15-95% |
| HPLC-4 | Welch Xtimate C18 150 x 25mm x 5µm; water (0.05%NH₃H₂O)-MeCN |
| HPLC-5 | Welch Xtimate C18 150 x 40 x 10µm, MeCN/H₂O (0.05% NH₄OH + 10 mM NH₄HCO₃) |
| HPLC-6 | Waters Xbridge Prep OBD C18 150 x 40mm x 10µm; [water (0.04%NH₃H₂O+10 mM NH₄HCO₃)-MeCN] |
| HPLC-7 | prep-HPLC column: Waters Xbridge BEH C18 100 x 30mm x 10µm;mobile phase: water(10mM NH₄HCO₃)-MeCN |
| HPLC-8 | Phenomenex Luna C18 75 x 30mm x 3µm; water (0.225%FA)-MeCN |
| HPLC-9 | Phenomenex Luna C18 100 x 40mm x 5µm; water (0.04% NH₃H₂O+10 mM NH₄HCO₃)-MeCN |
| HPLC-10 | Phenomenex Luna C18 150 x 25mm x 10µm; water (0.1% TFA)-MeCN |
| HPLC-11 | Phenomenex Gemini-NX C18 75 x 30 mm x 3 µm; water (0.05% NH₃H₂O + 10 mM NH₄HCO₃)-MeCN |

### Preparation of Intermediates

### Preparation 1: 2-(difluoromethyl)pyrimidine-4,6-diol

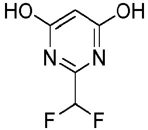

To a solution of malonamide (600 g, 5.88 mol) in EtOH (12 L), was added EtONa (799. g, 11.75 mol) and the solution stirred at 25 °C for 1 h. Ethyl 2,2-difluoroacetate (875.11 g, 7.05 mol) was added dropwise and the reaction was heated at 90 °C for 15 h. The cooled reaction mixture was filtered, the solid washed with EtOH/EtOAc (1:1) and dried *in vacuo* to give the title compound, (800 g, 71.4%) as a yellow solid.

### Preparation 2: 4,6-dichloro-2-(difluoromethyl)pyrimidine

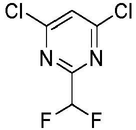

To a solution of 2-(difluoromethyl)pyrimidine-4,6-diol (Preparation 1, 150 g, 740.31 mmol) in toluene (3 L), was added POCl₃ (300.15 mL, 3.23 mol) dropwise followed by DIPEA (526.95 mL, 3.03 mol) and the reaction heated at 120 °C for 16 h. The cooled reaction mixture was concentrated under reduced pressure, the residue diluted with EtOAc (900 mL) and saturated aq. NaHCO₃ added to adjust the pH to 7-8. The layers were separated, the organic layer washed with brine (300 mL x 3), dried over Na₂SO₄, filtered and concentrated under reduced pressure. The residue was purified by column chromatography on silica gel (ISCO 0~5% EtOAc/PE) to give the title compound (85 g, 57.1%) as a yellow oil. ¹H NMR (400 MHz, DMSO-d₆) δ ppm 7.18-6.83 (m, 1 H) 8.31 (s, 1 H)

### Preparation 3: 6-chloro-2-(difluoromethyl)-N-(2,4-dimethoxybenzyl)pyrimidin-4-amine

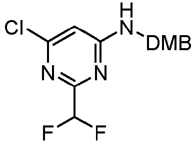

To a solution of 4,6-dichloro-2-(difluoromethyl)pyrimidine (Preparation 2, 50.0 g, 251 mmol) and 2,4-dimethoxybenzylamine (46.2 g, 276 mmol) in NMP (250 mL) was added DIPEA (64.9 g, 502 mmol) and the reaction stirred at 140 °C for 2 h. The cooled reaction mixture was poured into water (500 mL) and extracted with EtOAc (400 mL x 3), the combined organic layers were washed with brine (500 mL), dried over Na₂SO₄, filtered and the filtrate was concentrated under reduced pressure. The crude product was purified by column chromatography (PE/EtOAc =50/1 to 10/1) to give the title compound (60.0 g, 70.3% yield) as a yellow solid. LCMS m/z = 330 [M+H]⁺; ¹H NMR (400 MHz, DMSO-d₆) δ: 8.61-8.23 (m, 1H), 7.24-7.06 (m, 1H), 6.80-6.44 (m, 4H), 4.50-4.23 (m, 2H), 3.79 (s, 3H), 3.74 (s, 3H).

### Preparation 4: 6-chloro-2-(difluoromethyl)pyrimidin-4-amine

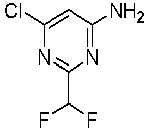

A solution of 6-chloro-2-(difluoromethyl)-N-(2,4-dimethoxybenzyl)pyrimidin-4-amine (Preparation 3, 60.0 g, 176 mmol) in HCl/EtOAc (4 M, 257 mL) was stirred at 25 °C for 12 h. The pH of the mixture was adjusted to 8 with saturated NaHCO₃ solution, the mixture extracted with EtOAc (500 mL x 3) and the combined organic layer was evaporated under reduced pressure, to give the title compound, 30 g, which was used without further purification. LCMS m/z = 180 [M+H]⁺

### Preparation 5: 2-(difluoromethyl)-N4-(2,4-dimethoxybenzyl)pyrimidine-4,6-diamine

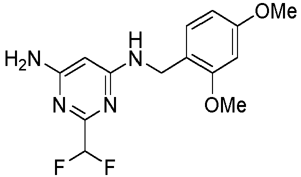

The title compound was obtained as an off-white solid, 25.31 g, 47.8% yield, from 6-chloro-2-(difluoromethyl)pyrimidin-4-amine (Preparation 4) and 2,4-dimethoxybenzylamine following a similar procedure to that described in Preparation 3. LCMS m/z = 311.1 [M+H]⁺; ¹H NMR(400 MHz, DMSO-d₆) δ 7.16 (t, 1H), 7.07 (d, 1H), 6.58-6.19 (m, 5H), 5.35 (s, 1H), 4.24 (s, 2H), 3.80 (s, 3H), 3.73 (s, 3H).

### Preparation 6: N,N'-(2-(difluoromethyl)pyrimidine-4,6-diyl)bis(1,1-diphenyl methanimine)

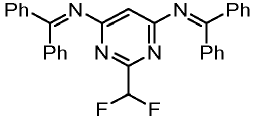

To a solution of 4,6-dichloro-2-(difluoromethyl)pyrimidine (Preparation 2, 145 g, 721.41 mmol) in dioxane (2 L), was added diphenylmethanimine (326.86 g, 1.80 mol), Cs₂CO₃ (705.15 g, 2.16 mol), Xantphos (41.74 g, 72.14 mmol) and Pd₂(dba)₃ (33.03 g, 36.07 mmol) under N₂ and the reaction was heated at 100 °C for 8 h. The cooled reaction mixture was filtered and concentrated under reduced pressure. The residue was purified by silica gel chromatography (ISCO, 0~10 % EtOAc/PE) to give the title compound (250 g, 67%) as a yellow oil. ¹H NMR (400 MHz, DMSO-d₆) δ ppm 6.39-6.57 (m, 2H) 7.27-7.60 (m, 20H)

### Preparation 7: 2-(difluoromethyl)pyrimidine-4,6-diamine hydrochloride

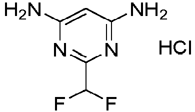

To a solution of N,N'-(2-(difluoromethyl)pyrimidine-4,6-diyl)bis(1,1-diphenyl methanimine) (Preparation 6, 250 g, 511.74 mmol) in dioxane (1.5 L), was added HCl/dioxane (4 M, 800 mL) and the reaction stirred at 25 °C for 16 h. The reaction was concentrated under reduced pressure and the residue was triturated with THF (1 L) at 25 °C for 30 min. The solid was filtered off to give the title compound (113 g, 68.6% yield) as a brown solid. LCMS m/z= 160 [M+H]⁺

### Preparation 8: 2-(difluoromethyl)pyrimidine-4,6-diamine

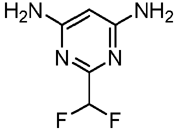

7M NH₃ in MeOH was added to a solution of diethyl malonimidate dihydrochloride (10 g, 43.9 mmol) in MeOH (20 mL) and the reaction was stirred for 16 h at 50°C. The mixture was evaporated under reduced pressure to give crude malonimidamide, 7 g. This was dissolved in EtOH (40 mL), ethyl 2,2-difluoroacetate (4.5 g, 36.26 mmol) and EtONa (7.5 g, 110.3 mmol) added and the reaction heated at 80°C for 2 h. The cooled mixture was extracted with DCM and the combined organic extracts concentrated *in vacuo.* The residue was purified by column chromatography on silica gel eluting with MeOH/DCM (1/10) to give title compound (700 mg, yield 12.6%).

### Preparation 9: 2-(1-fluoroethyl)pyrimidine-4,6-diol

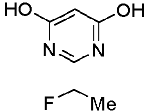

To a solution of malonamide (30 g, 294 mmol) in EtOH (300 mL) was added EtONa (40.0 g, 588 mmol) and the solution stirred at 20 °C for 1 h. Ethyl 2-fluoropropanoate (38.7 g, 323 mmol) was added drop-wise and the reaction stirred at 100 °C for 2 h. The pH of the mixture was adjusted to pH = 6 with 1 N HCl, and then concentrated *in vacuo.* The residue was adjusted to pH = 2 with 1 N HCl and filtered to provide the title compound (31 g, 66% yield) as a yellow solid, which was used in the next step without further purification. LCMS m/z = 159 [M+H]⁺.

### Preparation 10: 4,6-dichloro-2-(1-fluoroethyl)pyrimidine

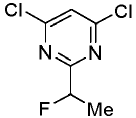

To a stirred solution of 2-(1-fluoroethyl)pyrimidine-4,6-diol (Preparation 9, 31 g, 196 mmol) in toluene (300 mL) was added POCl₃ (72.8 mL, 784 mmol) at 25 °C. TEA (54.4 mL, 392 mmol) was added drop-wise and the reaction stirred at 100 °C for 2 h. The reaction mixture was poured into warm water and extracted with EtOAc (x 3). The combined organic layers were washed with brine, dried over Na₂SO₄, filtered and the filtrate was evaporated under reduced pressure to give the title compound, 40 g.

### Preparation 11: 6-chloro-N-(2,4-dimethoxybenzyl)-2-(1-fluoroethyl)pyrimidin-4-amine

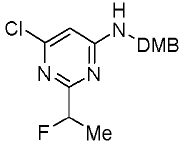

A solution of 4,6-dichloro-2-(1-fluoroethyl)pyrimidine (Preparation 10, 40 g, 196 mmol), 2,4-dimethoxybenzylamine (32.7 g, 196 mmol) and DIPEA (50.5 g, 392 mmol) in NMP (150 mL) was stirred at 100 °C for 1 h. The mixture was poured into water (500 mL) and extracted with EtOAc (300 mL x 3). The combined organics were washed with brine (300 mL x 3), dried over Na₂SO₄ and evaporated under reduced pressure to give the title compound (22 g, crude) as a yellow solid, which was used in the next step directly. LCMS m/z = 326 [M+H]⁺.

### Preparation u: 6-chloro-2-(1-fluoroethyl)pyrimidin-4-amine

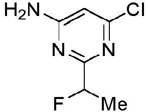

A solution of 6-chloro-N-(2,4-dimethoxybenzyl)-2-(1-fluoroethyl)pyrimidin-4-amine (Preparation 11, 22 g, 67.6 mmol) in HCl/EtOAc (4.0 M, 85 mL) was stirred at 20 °C for 12 h. The mixture was concentrated *in vacuo,* the pH of the residue adjusted to pH = 8 with saturated NaHCO₃ solution, and the mixture extracted with DCM (300 mL x 3). The combined organics were washed with brine (300 mL), dried over Na₂SO₄ and concentrated *in vacuo.* The residue was triturated with PE/EtOAc = 1/1 and filtered to give the title compound (10 g, 84% yield) as a gray solid. LCMS m/z = 176 [M+H]⁺.

### Preparation 13 and Preparation 14: (S)-N4-(2,4-dimethoxybenzyl)-2-(1-fluoroethyl) pyrimidine-4,6-diamine and (R)-N4-(2,4-dimethoxybenzyl)-2-(1-fluoroethyl)pyrimidine-4,6-diamine

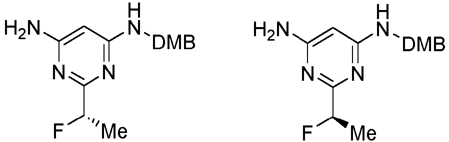

A solution of 6-chloro-2-(1-fluoroethyl)pyrimidin-4-amine (Preparation 12, 10.0 g, 57 mmol), 2,4-dimethoxybenzylamine (9.5 g, 57 mmol) and DIPEA (29.3 g, 228 mmol) in NMP (60 mL) was stirred at 140 °C for 2 h. The cooled mixture was poured into water (500 mL), extracted with EtOAc (300 mL x 3) and the combined organics were washed with brine (300 mL x 2), dried over Na₂SO₄ and concentrated. The residue was purified by silica gel chromatography to provide N4-(2,4-dimethoxybenzyl)-2-(1-fluoroethyl)pyrimidine-4,6-diamine (6.6 g, 37%) as a yellow solid.

This product was further purified by SFC, using an OZ 20 x 250mm, 10µm (Daicel) column, eluting with 35% MeOH (0.2% MeOH/NH₃) at 100 g/min, to obtain the first eluting enantiomer: (S)-N4-(2,4-dimethoxybenzyl)-2-(1-fluoroethyl)pyrimidine-4,6-diamine (2.1 g) as a yellow solid. LCMS m/z = 307 [M+H]⁺. (Preparation 13)
Further elution provided the second eluting enantiomer, (R)-N4-(2,4-dimethoxybenzyl)-2-(1-fluoroethyl)pyrimidine-4,6-diamine. (Preparation 14)
The stereochemistry of Preparation 13 was confirmed by X-ray.

### Preparation 15: 2-(fluoromethyl)pyrimidine-4,6-diol

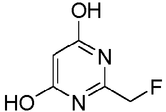

To a solution of malonamide (60.0 g, 588 mmol) in EtOH (600 mL) was added EtONa (80.0 g, 1.18 mol) and the solution stirred for 1 h. Ethyl 2-fluoroacetate (68.6 g, 646 mmol) was added dropwise the reaction stirred at 100°C for 2 h. The pH of the reaction mixture was adjusted to 6 using 1N HCl and concentrated under reduced pressure. The pH of the residue was adjusted to 2 using 1M HCl and the mixture filtered to provide the title compound as a yellow solid, 32.0 g 37.8% yield. ¹H NMR: (400 MHz, DMSO-d₆) δ 11.91 (brs, 2H), 5.26 (d, 2H), 5.14 (s, 1H).

### Preparation 16: 4,6-dichloro-2-(fluoromethyl)pyrimidine

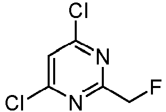

To a solution of 2-(fluoromethyl)pyrimidine-4,6-diol (Preparation 15, 32.0 g, 222 mmol) in toluene (300 mL) was added POCl₃ (82.8 mL, 891 mmol) dropwise at 25°C. TEA (61.8 mL, 444 mmol) was added dropwise and the reaction was stirred at 100°C for 2 h. The reaction mixture was poured into warm water (500 mL) and extracted with EtOAc (300 mL x 3). The combined organic layer was washed with brine (300 mL), dried over Na₂SO₄, filtered and the filtrate was concentrated *in vacuo.* The residue was purified by MPLC (silica gel, PE/EtOAc = 100/1-10/1) to give the title compound (30.0 g, 74.6% yield) as a white solid. ¹H NMR: (400 MHz, CDCl₃) δ: 5.43 (s, 1H), 5.54 (s, 1H), 7.40 (s, 1H).

### Preparation 17: N4,N6-bis(2,4-dimethoxybenzyl)-2-(fluoromethyl)pyrimidine-4,6-diamine

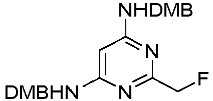

To a solution of 4,6-dichloro-2-(fluoromethyl)pyrimidine (Preparation 16, 1.10 g, 6.08 mmol) in DMSO (15 mL) was added 2,4-dimethoxybenzylamine (5.08 g, 30.39 mmol) and the reaction stirred at 100°C for 24 h. The cooled reaction was quenched with H₂O (30 mL) at 0°C and extracted with EtOAc (20 mL x 5). The combined organic layers were washed with brine (10 mL), dried over Na₂SO₄, filtered and concentrated under reduced pressure. The residue was purified by silica gel column chromatography (PE: EtOAc = 5: 1 to 1: 1) to give the title compound (940 mg, 35.0%) as yellow solid.

### Preparation 18: 2-(fluoromethyl)pyrimidine-4,6-diamine

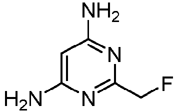

To a solution of N4,N6-bis(2,4-dimethoxybenzyl)-2-(fluoromethyl)pyrimidine-4,6-diamine (Preparation 17, 940 mg, 2.12 mmol) in DCM (9 mL) was added TFA (3 mL) and the reaction stirred at 25°C for 1 h. The reaction mixture was concentrated under reduced pressure and the residue was purified by prep-HPLC-1 to give the title compound, (130 mg, 43.1%) as a white solid. ¹H-NMR (400 MHz, DMSO-d₆) δ: 6.19 (s, 4H), 5.31 (s, 1H), 5.05-4.93 (m, 2H).

### Preparation 19: N4-(2,4-dimethoxybenzyl)-2-(fluoromethyl)pyrimidine-4,6-diamine

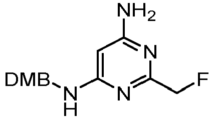

A solution of 4,6-dichloro-2-(fluoromethyl)pyrimidine (Preparation 16, 30.0 g, 166 mmol), 2,4-dimethoxybenzylamine (27.7 g, 166 mmol) and DIPEA (42.8 g, 332 mmol) in NMP (150 mL) was stirred at 100 °C for 1 h. The mixture was poured into water (500 mL) and extracted with EtOAc (300 mL x 3). The combined organics were washed with brine (300 mL x 3), dried over Na₂SO₄ and evaporated under reduced pressure to give the title compound (50.0 g, crude) as a yellow solid. LCMS m/z = 312 [M+H]⁺

### Preparation 20: 5-bromo-2-chloro-4-methoxypyridine

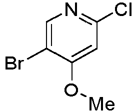

To a solution of MeOH (704 mg, 22.0 mmol) in THF (30 mL) at 0 °C was added NaH (60% dispersion, 1.76 g, 44.0 mmol), the mixture stirred at 0 °C for 30 mins, then 5-bromo-2,4-dichloropyridine (5 g, 22.0 mmol) in THF (10 mL) was added. The reaction was stirred at rt for 12 h. The solution was quenched with H₂O, extracted with EtOAc and the combined organic layers were concentrated *in vacuo.* The residue was purified by silica gel chromatography to give the title compound as a white solid (3.5 g, 72%). LCMS m/z = 222 [M+H]⁺.

### Preparation 21: 5-bromo-2-chloro-4-isopropoxypyridine

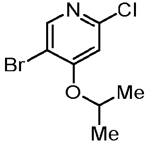

To a solution of IPA (1.32 g, 22.0 mmol) in THF (30 mL) was added NaH (1.76 g, 44.0 mmol) at 0 °C, the solution stirred for 30 min, then 5-bromo-2,4-dichloropyridine (5 g, 22.0 mmol) in THF (10 mL) was added. The mixture was stirred at rt for 12 h, diluted with EtOAc (300 mL) then washed with water (80 mL) and brine (80 mL). The combined organic layers were dried over Na₂SO₄, filtered and concentrated *in vacuo.* The residue was purified by silica gel column chromatography (PE/EtOAc = 10/1 to 2/1) to afford the title compound (4.0 g, 73%) as a white solid. LCMS m/z = 250 [M+H]⁺.

### Preparation 22: 2-chloro-5-iodo-4-methoxypyridine

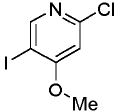

To a solution of 2-chloro-4-fluoro-5-iodopyridine (800 mg, 3.11 mmol) in MeOH (10 mL) was added a solution of MeONa in MeOH (4 M, 1.16 mL) and the reaction stirred at rt for 4 h. Water (30 mL) was added, the mixture extracted with EtOAc (50 mL× 3) and the combined organic layers were concentrated *in vacuo* to give the crude product. This was purified by silica gel chromatography (PE: EtOAc =5:1) to give the title compound (750 mg, 90%) as a yellow solid. LCMS m/z = 270 [M+H]⁺.

### Preparation 23: 2-chloro-5-iodo-4-(methoxy-d3)pyridine

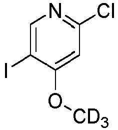

A mixture of methanol-d₃ (511 mg, 14.6 mmol) and NaH (875 mg, 21.9 mmol) in THF (50 mL) was stirred at 0 °C for 30 min. 2,4-Dichloro-5-iodopyridine (2 g, 7.30 mmol) was added and the reaction stirred for 4 h at rt. The mixture was concentrated *in vacuo* and the residue purified by column chromatography on silica gel eluting with EtOAc/PE (1/4) to afford the title compound (1.8 g, 90%) as a white solid. LCMS m/z = 273 [M+H]⁺.

### Preparation 24: 2-chloro-4-cyclopropoxy-5-iodopyridine

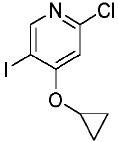

At 0 °C, to a suspension of NaH (420 mg, 10.5 mmol) in DMF (10 mL), was added a solution of cyclopropanol (406 mg, 7 mmol) in DMF (2 mL) and the mixture stirred at rt for 30 mins. 2-Chloro-4-fluoro-5-iodopyridine (1.8 g, 7 mmol) in DMF (10 mL) was added and the reaction stirred at rt for 2 h. Water (30 mL) was added and the mixture extracted with EtOAc (50 mL× 3). The combined organic layer was concentrated *in vacuo* and the crude product was purified by silica gel chromatography (PE: EtOAc =3: 1) to give the title compound (1.2 g, 58.3 %) as a yellow solid. LCMS m/z = 296 [M+H]⁺.

### Preparation 25: 2-chloro-4-((1-fluoropropan-2-yl)oxy)-5-iodopyridine

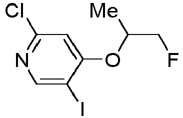

To a solution of 2-chloro-4-fluoro-5-iodopyridine (350 mg, 1.36 mmol) in THF was added NaH (65.3 mg, 2.72 mmol) and the solution stirred for 30 mins at 0°C. 1-Fluoropropan-2-ol (106.2 mg, 1.36 mmol) was added and the reaction stirred at rt overnight. The reaction was quenched with water and concentrated *in vacuo.* The residue was purified by column chromatography on silica gel eluting with EtOAc/PE (1/1) to afford the title compound (320 mg, 75% yield). LCMS m/z = 316 [M+H]⁺.

### Preparation 26: 2-chloro-5-iodo-4-isopropoxypyridine

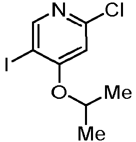

The title compound was obtained as a white solid, 140 mg, 61% yield, from 2-chloro-4-fluoro-5-iodopyridine and isopropanol, following the procedure described in Preparation 25. LCMS m/z = 298 [M+H]⁺

### Preparation 27: 5-bromo-2-chloro-4-cyclopropoxypyridine

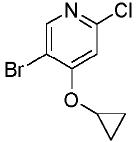

To a solution of cyclopropanol (8.70 g, 150 mmol) in THF (200 mL) was added NaH (5.99 g, 150 mmol, 60% in mineral oil) at 0 °C and the mixture was stirred at 0 °C for 0.5 h. 5-Bromo-2,4-dichloropyridine (34.0 g, 150 mmol) was added and the reaction stirred at 60 °C for 3 h. The reaction mixture was cooled to 0 °C and quenched by addition of saturated aq. NH₄Cl solution (100 mL) and then extracted with EtOAc (100 mL x 3). The combined organic layers were dried over Na₂SO₄, filtered and concentrated under reduced pressure. The residue was purified by column chromatography on silica gel eluting with PE/EtOAc = 1:0 to 80 :1 to give the title compound (10.5 g, 27.4% yield) as a colorless oil. LCMS m/z = 250 [M+H]⁺

### Preparation 28: 5-bromo-2-chloro-4-(oxetan-3-yloxy)pyridine

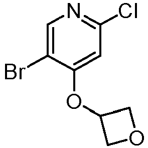

The title compound was obtained as a white solid, 1.97 g, 67.6%, from oxetan-3-ol and 5-bromo-2,4-dichloropyridine following the procedure described in Preparation 27.

### Preparation 29: 2-chloro-4-(difluoromethoxy)-5-iodopyridine

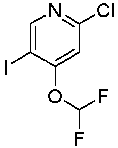

A mixture of 2-chloro-5-iodopyridin-4-ol (1.0 g, 3.92 mmol), ethyl 2-bromo-2,2-difluoroacetate (1.59 g, 7.84 mmol) and K₂CO₃ (1.62 g, 11.76 mmol) in DMF (20 mL) was stirred at 60 °C under N₂ for 16 h. The cooled mixture was diluted with EtOAc, washed with water, and concentrated *in vacuo.* The crude product was purified by silica gel chromatography to give the title product (500 mg, 42%). LCMS m/z = 306 [M+H]⁺.

### Preparation 30: 1,1-diphenyl-N-(4-(trifluoromethoxy)pyridin-2-yl)methanimine

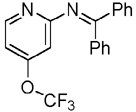

To a mixture of 2-chloro-4-(trifluoromethoxy)pyridine (2.8 g, 14.2 mmol), Pd₂(dba)₃ (280 mg, 0.306 mmol), XantPhos (560 mg, 0.968 mmol) and Cs₂CO₃ (6.93 g, 21.3 mmol) in dry dioxane (400 mL) was added diphenylmethanimine (2.83 g, 15.6 mmol) and the reaction stirred at 100 °C under N₂ for 16 h. The cooled mixture was concentrated *in vacuo* and purified by silica gel chromatography to give the title product (2.6 g, 53%). LCMS m/z = 343 [M+H]⁺.

### Preparation 31: 4-(trifluoromethoxy)pyridin-2-amine

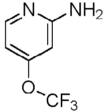

To a mixture of 1,1-diphenyl-N-(4-(trifluoromethoxy)pyridin-2-yl)methanimine (Preparation 30, 2.6 g, 7.6 mmol) in DCM (20 mL) was added HCl/dioxane (20 mL, 4 M) and the reaction stirred at rt for 1 h. The mixture was concentrated *in vacuo* and purified by silica gel chromatography to give the title product (850 mg, 62%). LCMS m/z =179 [M+H]⁺.

### Preparation 32: 5-bromo-4-(trifluoromethoxy)pyridin-2-amine

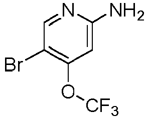

To a mixture of 4-(trifluoromethoxy)pyridin-2-amine (Preparation 31, 850 mg, 4.78 mmol) in DMF (20 mL) was added NBS (1.02 g, 5.74 mmol) and the reaction stirred at rt for 1 h. The mixture was diluted with EtOAc, washed with water and concentrated *in vacuo.* The crude product was purified by silica gel chromatography to give the title compound (1.1 g, 89%). LCMS m/z =257 [M+H]⁺.

### Preparation 33: 1-(2-((tert-butyldimethylsilyl)oxy)ethyl)-4-(4,4,5,5-tetramethyl-1,3,2-dioxaborolan-2-yl)-1H-pyrazole

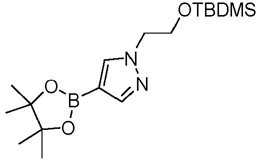

A mixture of 2-(4-(4,4,5,5-tetramethyl-1,3,2-dioxaborolan-2-yl)-1H-pyrazol-1-yl)ethan-1-ol (1 g, 4.2 mmol), 1H-imidazole (571 mg, 8.4 mmol) and TBDMSCl (945 mg, 6.3 mmol) in DCM (20 mL) was stirred at rt for 16 h. The mixture was concentrated *in vacuo* and the residue was purified by column chromatography on silica gel eluting with EtOAc/PE (1/5) to afford the title compound (1 g, yield: 63%) as a white solid. LCMS m/z = 353 [M+H]⁺.

### Preparation 34: 1-(oxetan-2-ylmethyl)-4-(4,4,5,5-tetramethyl-1,3,2-dioxaborolan-2-yl)-1H-pyrazole

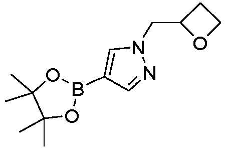

A mixture of 4-(4,4,5,5-tetramethyl-1,3,2-dioxaborolan-2-yl)-1H-pyrazole (800 mg, 4.12 mmol), 2-(bromomethyl)oxetane (622 mg, 4.12 mmol) and Cs₂CO₃ (2.68 g, 8.24 mmol) in MeCN (20 mL) was stirred at 80 °C for 16 h. The cooled mixture was concentrated in *vacuo.* The residue was purified by column chromatography on silica gel eluting with EtOAc/PE (1/1) to afford the title compound (700 mg, 65% yield) as colorless oil. LCMS m/z = 265 [M+H]⁺.

### Preparation 35: 2-methyl-4-(4-(4,4,5,5-tetramethyl-1,3,2-dioxaborolan-2-yl)-1H-pyrazol-1-yl)butan-2-ol

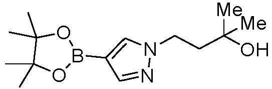

The title compound was obtained as a brown oil, (800 mg, 61% yield), from 2-methyl-4-((4-methylbenzenesulfonyl)oxy)butan-2-ol and 4-(4,4,5,5-tetramethyl-1,3,2-dioxaborolan-2-yl)-1H-pyrazole following a similar procedure to that described in Preparation 34. LCMS m/z = 281 [M+H]⁺

### Preparation 36: 1-(4-(4,4,5,5-tetramethyl-1,3,2-dioxaborolan-2-yl)-1H-pyrazol-1-yl)propan-2-one

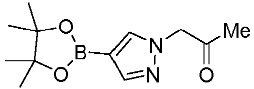

The title compound was obtained ((1 g, yield 77%) from 4-(4,4,5,5-tetramethyl-1,3,2-dioxaborolan-2-yl)-1H-pyrazole and 1-chloropropan-2-one, following the procedure described in Preparation 34. LCMS m/z = 251 [M+H]⁺.

### Preparation 37: tert-butyl methyl(2-(4-(4,4,5,5-tetramethyl-1,3,2-dioxaborolan-2-yl)-1H-pyrazol-1-yl)ethyl)carbamate

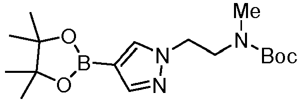

To a solution of 4-(4,4,5,5-tetramethyl-1,3,2-dioxaborolan-2-yl)-1H-pyrazole (4.50 g, 23.19 mmol) and tert-butyl N-(2-hydroxyethyl)-N-methyl-carbamate (4.06 g, 23.19 mmol) in THF (30 mL) was added DIAD (5.16 g, 25.51 mmol) and PPh₃ (6.69 g, 25.51 mmol) and the reaction stirred at 25°C for 12 h under N₂. The reaction mixture was concentrated under reduced pressure and the residue was purified by prep-HPLC-2 to give the title compound (2.11 g, 25.9%) as a yellow oil.

### Preparation 38: tert-butyl 2-((4-(4,4,5,5-tetramethyl-1,3,2-dioxaborolan-2-yl)-1H-pyrazol-1-yl)methyl)azetidine-1-carboxylate

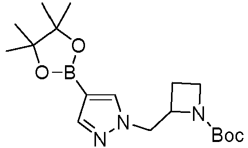

A mixture of 2 tert-butyl 2-(hydroxymethyl)azetidine-1-carboxylate (300 mg, 1.6 mmol) and 4-(4,4,5,5-tetramethyl-1,3,2-dioxaborolan-2-yl)-1H-pyrazole (315 mg, 1.6 mmol) in THF (20 mL) was added PPh₃ (630 mg, 2.4 mmol), the mixture stirred at 0°C and DIAD added (0.3 mL). The reaction was stirred at rt overnight and was concentrated *in vacuo.* The residue was purified by column chromatography on silica gel eluting with EtOAc/PE (1/3) to give the title compound (300 mg, yield 52%). LCMS m/z = 364 [M+H]⁺.

### Preparation 39: tert-butyl (1-(4-bromo-1H-pyrazol-1-yl)-2-methylpropan-2-yl)carbamate

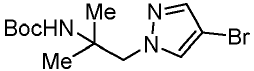

A mixture of 4-bromo-1H-pyrazole (2.5 g, 17.0 mmol), tert-butyl 4,4-dimethyl-1,2,3-oxathiazolidine-3-carboxylate 2,2-dioxide (4.69 g, 18.7 mmol) and Cs₂CO₃ (8.35 g, 25.5 mmol) in anhydrous DMF (50 mL) was stirred at 80°C overnight. The cooled mixture was partitioned between water and EtOAc, the layers separated and the organic phase concentrated *in vacuo.* The residue was purified by silica gel chromatography EtOAc/PE (1/5) to give the title compound (4.4 g, 81 % yield) as a colorless oil. LCMS m/z = 318, 320 [M+H]⁺

### Preparation 40: tert-butyl (1-(4-bromo-1H-pyrazol-1-yl)-2-methylpropan-2-yl)(methyl)carbamate

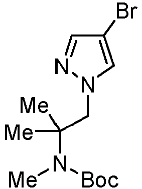

To an ice-cooled mixture of tert-butyl (1-(4-bromo-1H-pyrazol-1-yl)-2-methylpropan-2-yl)carbamate (Preparation 39, 2.3 g, 7.23 mmol) in dry THF (50 mL) was added NaH (578 mg, 14.4 mmol) and the mixture was stirred at 0°C for 1 h. MeI (1.54 g, 10.84 mmol) was added and the reaction stirred at rt for 2 h. The mixture was diluted with EtOAc, washed with water and the organic layer was concentrated *in vacuo.* The residue was purified by column chromatography on silica gel eluting with EtOAc/PE (1/10) to give the title compound (1.7 g, yield 71%). LCMS m/z = 332 [M+H]⁺.

### Preparation 41: tert-butyl methyl(2-methyl-1-(4-(4,4,5,5-tetramethyl-1,3,2-dioxaborolan-2-yl)-1H-pyrazol-1-yl)propan-2-yl)carbamate

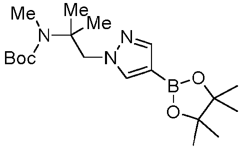

A mixture of tert-butyl (1-(4-bromo-1H-pyrazol-1-yl)-2-methylpropan-2-yl)(methyl) carbamate (Preparation 40, 1.7 g, 5.14 mmol), bis(pinacolato)diboron (1.96 g, 7.71 mmol), KOAc (1.51 g, 15.4 mmol) and Pd(dppf)Cl₂ (200 mg, 2.6 mmol) in dioxane (20 mL) was stirred at 100°C under N₂ for 16 h. The cooled mixture was concentrated *in vacuo* and the residue was purified by column chromatography on silica gel eluting with EtOAc/PE (1/10) to give the title compound (600 mg, yield 30%). LCMS m/z = 380 [M+H]⁺.

### Preparation 42: 1-(4-(6-chloro-4-methoxypyridin-3-yl)-1H-pyrazol-1-yl)-2-methylpropan-2-ol

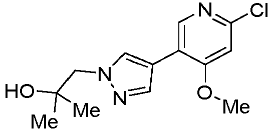

A mixture of 2-chloro-5-iodo-4-methoxypyridine (Preparation 22, 750 mg, 2.79 mmol), 2-methyl-1-(4-(4,4,5,5-tetramethyl-1,3,2-dioxaborolan-2-yl)-1H-pyrazol-1-yl)propan-2-ol (891 mg, 3.35 mmol), Pd(dppf)Cl₂ (204 mg, 0.279 mmol) and K₂CO₃ (770 mg, 5.58 mmol) in dioxane (12 mL) and H₂O (3 mL) was stirred at 90°C for 4 h under N₂. The cooled mixture was concentrated *in vacuo* to give the crude product which was purified by silica gel chromatography (PE: EtOAc =1:2) to give the title compound (560 mg, 71.5%) as a yellow oil. LCMS m/z = 282 [M+H]^{+.}

### Preparation 43: 1-(4-(6-chloro-4-(methoxy-d3)pyridin-3-yl)-1H-pyrazol-1-yl)-2-methylpropan-2-ol

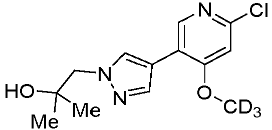

A mixture of 2-chloro-5-iodo-4-(methoxy-d3)pyridine (Preparation 23, 2 g, 7.33 mmol), 2-methyl-1-(4-(4,4,5,5-tetramethyl-1,3,2-dioxaborolan-2-yl)-1H-pyrazol-1-yl)propan-2-ol (2.14 g, 8.06 mmol), K₂CO₃ (2.01 g, 14.6 mmol) and Pd(dppf)Cl₂ (536 mg, 0.733 mmol) in dioxane/water (40 mL/8 mL) was stirred at 80 °C for 6 h under N₂. The cooled mixture was concentrated *in vacuo* and the residue was purified by column chromatography on silica gel eluting with EtOAc/PE= 2/1 to afford the title compound (1.2 g, yield: 57%) as a white solid. LCMS m/z = 285 [M+H]⁺.

### Preparation 44: 2-chloro-4-isopropoxy-5-(1-methyl-1H-pyrazol-4-yl)pyridine

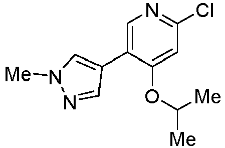

A mixture of 5-bromo-2-chloro-4-isopropoxypyridine (Preparation 21, 120 mg, 0.479 mmol), 1-methyl-4-(4,4,5,5-tetramethyl-1,3,2-dioxaborolan-2-yl)-1H-pyrazole (79.73 mg, 0.383 mmol), Pd(dppf)Cl₂ (35.05 mg, 0.048 mmol) and K₂CO₃ (132.40 mg, 0.958 mmol) in dioxane (2 mL) and H₂O (0.10 mL) was degassed and purged with N₂ and the reaction stirred at 90°C for 2 h. The cooled reaction mixture was concentrated under reduced pressure and the residue was purified by prep-TLC (PE: EtOAc = 1:1) to give the title compound (50 mg, 41.5%) as a yellow solid.

### Alternative synthesis:

A mixture of NaH (60%, 53 mg, 1.32 mmol) in IPA (5 mL) was stirred at 0 °C for 2 h, then 2-chloro-4-fluoro-5-(1-methyl-1H-pyrazol-4-yl)pyridine (Preparation 97, 70 mg, 0.33 mmol) was added. The mixture was stirred at rt for 2 h. The mixture was concentrated *in vacuo* and the residue was purified by column chromatography on silica gel eluting with EtOAc/PE =1/2 to afford the title compound (60 mg, 72% yield) as a white solid. LCMS m/z = 252 [M+H]⁺.

### Preparation 45: 1-(4-(6-chloro-4-isopropoxypyridin-3-yl)-1H-pyrazol-1-yl)-2-methylpropan-2-ol

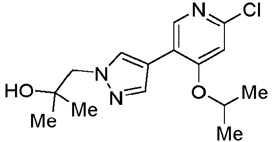

A mixture of 5-bromo-2-chloro-4-isopropoxypyridine (Preparation 21, 2.0g, 8.06 mmol), 2-methyl-1-(4-(4,4,5,5-tetramethyl-1,3,2-dioxaborolan-2-yl)-1H-pyrazol-1-yl)propan-2-ol (2.15 g, 8.06 mmol), K₂CO₃ (3.34 g, 24.2 mmol) and Pd(dppf)Cl₂ (592 mg, 0.81 mmol) in dioxane/water (20 mL/ 4 mL) was stirred at 80 °C for 3 h. The cooled mixture was concentrated *in vacuo* and the residue was purified by column chromatography on silica gel (PE/EtOAc = 10/1 to 2/1) to afford the title compound (2.2 g, 88% yield) as a yellow solid. LCMS m/z = 310 [M+H]⁺.

### Preparation 46: 1-(4-(6-chloro-4-cyclopropoxypyridin-3-yl)-1H-pyrazol-1-yl)-2-methylpropan-2-ol

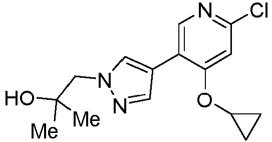

A mixture of 2-chloro-4-cyclopropoxy-5-iodopyridine (Preparation 24, 1.18 g, 4 mmol), 2-methyl-1-(4-(4,4,5,5-tetramethyl-1,3,2-dioxaborolan-2-yl)-1H-pyrazol-1-yl)propan-2-ol (1.28 g, 4.8 mmol), Pd(dppf)Cl₂ (150 mg, 0.2 mmol) and K₂CO₃ (1.1 g, 8 mmol) in dioxane (20 mL) and H₂O (3 mL) was stirred at 90°C for 4 h under N₂. The cooled mixture was concentrated to give the crude product which was purified by silica gel chromatography (PE: EtOAc =1:2) to give the title compound (900 mg, 73.3 %) as a beige solid. LCMS m/z =308 [M+H]⁺.

### Preparation 47: 1-(4-(6-chloro-4-(difluoromethoxy)pyridin-3-yl)-1H-pyrazol-1-yl)-2-methylpropan-2-ol

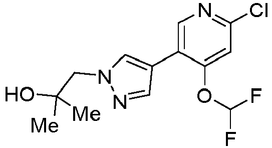

A mixture of 2-chloro-4-(difluoromethoxy)-5-iodopyridine (Preparation 29, 100 mg, 0.33 mmol), 2-methyl-1-(4-(4,4,5,5-tetramethyl-1,3,2-dioxaborolan-2-yl)-1H-pyrazol-1-yl)propan-2-ol (105 mg, 0.39 mmol), Pd(dppf)Cl₂ (20 mg, 0.027 mmol) and K₂CO₃ (45 mg, 0.66 mmol) in dioxane/water (10 mL/1 mL) was stirred at 70 °C under N₂ for 16 h. The cooled mixture was concentrated *in vacuo* and the residue purified by silica gel chromatography to give the title compound (50 mg, 47%). LCMS m/z = 318 [M+H]⁺.

### Preparation 48: 2-chloro-4-isopropoxy-5-(1-(oxetan-3-yl)-1H-pyrazol-4-yl)pyridine

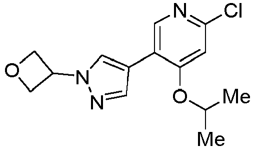

A mixture of 5-bromo-2-chloro-4-isopropoxypyridine (Preparation 21, 300 mg, 1.19 mmol), 1-(oxetan-3-yl)-4-(4,4,5,5-tetramethyl-1,3,2-dioxaborolan-2-yl)-1H-pyrazole (355 mg, 1.42 mmol), Pd(dppf)Cl₂ (174 mg, 0.238 mmol) and K₂CO₃ (492 mg, 3.56 mmol) in dioxane/H₂O (15 mL/5 mL) was stirred at 100 °C for 4 h. The reaction mixture was diluted with water, extracted with DCM (200 mL x 3), the organic phase was washed with brine, dried over anhydrous Na₂SO₄, and concentrated *in vacuo.* The crude product was purified by column chromatography on silica gel (PE/EtOAc= 2/1) to give the title compound (140 mg, 40.1 %) as white solid. LCMS m/z = 294 [M+H]⁺.

### Preparation 49: 2-chloro-4-isopropoxy-5-(1-(tetrahydrofuran-3-yl)-1H-pyrazol-4-yl)pyridine

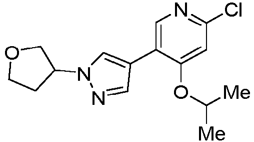

The title compound was obtained as a white solid, 100 mg, 32% yield, from 2-chloro-5-iodo-4-isopropoxypyridine (Preparation 26), following a similar procedure to that described in Preparation 48. LCMS m/z = 308 [M+H]⁺

### Preparation 50: tert-butyl 3-(4-(6-chloro-4-(difluoromethoxy)pyridin-3-yl)-1H-pyrazol-1-yl)pyrrolidine-1-carboxylate

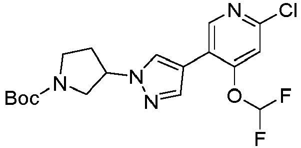

The title compound was obtained, 210 mg, 77% yield, from 2-chloro-4-(difluoro methoxy)-5-iodopyridine (Preparation 29) and tert-butyl 3-(4-(4,4,5,5-tetramethyl-1,3,2-dioxaborolan-2-yl)-1H-pyrazol-1-yl)pyrrolidine-1-carboxylate, following the procedure described in Preparation 48. LCMS m/z = 415 [M+H]⁺.

### Preparations 51 to 74

A mixture of the appropriate chloropyridine (1.0 equiv.) and alkylated (4-(4,4,5,5-tetramethyl-1,3,2-dioxaborolan-2-yl)-1H-pyrazole (1 equiv. to 1.2 equiv.), K₂CO₃ (2.0 to 3.0 equiv.) and Pd(dppf)Cl₂ (0.03 to 0.2 equiv.) in dioxane/water (10/1 to 3/1 V/V) was stirred at between 70°C and 100 °C until the starting materials had been consumed. The cooled mixture was concentrated *in vacuo* and the residue was purified by column chromatography on silica gel (eluting with PE/EtOAc or MeOH/DCM) to provide the desired compound.

| **Preparation No** | **Name/Structure/Starting Materials (SM)/Data** |
|---|---|
| **Using 5-bromo-2-chloro-4-methoxypyridine (Preparation 20)** | |
| **51** | 2-chloro-4-methoxy-5-(1-(2-methoxyethyl)-1H-pyrazol-4-yl)pyridine |
| | 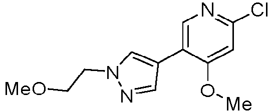 |
| | SM: 1-(2-methoxyethyl)-4-(4,4,5,5-tetramethyl-1,3,2-dioxaborolan-2-yl)-1H-pyrazole |
| | LCMS m/z = 268 [M+H]⁺ |
| **52** | 1-(4-(6-chloro-4-methoxypyridin-3-yl)-1H-pyrazol-1-yl)propan-2-one |
| | 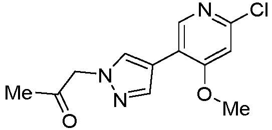 |
| | SM: 1-(4-(4,4,5,5-tetramethyl-1,3,2-dioxaborolan-2-yl)-1H-pyrazol-1-yl)propan-2-one (Preparation 36) |
| | LCMS m/z = 266 [M+H]⁺ |
| **53** | 2-chloro-4-methoxy-5-(1-(oxetan-2-ylmethyl)-1H-pyrazol-4-yl)pyridine |
| | 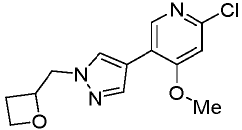 |
| | SM: 1-(oxetan-2-ylmethyl)-4-(4,4,5,5-tetramethyl-1,3,2-dioxaborolan-2-yl)-1H-pyrazole (Preparation 34). |
| | 100 mg, 67% yield as a white solid. LCMS m/z = 280 [M+H]⁺ |
| **54** | 2-chloro-4-methoxy-5-(1-(tetrahydrofuran-3-yl)-1H-pyrazol-4-yl)pyridine |
| | 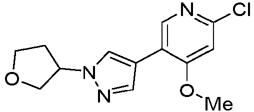 |
| | SM: 1-(tetrahydrofuran-3-yl)-4-(4,4,5,5-tetramethyl-1,3,2-dioxaborolan-2-yl)-1H-pyrazole |
| | 180 mg, 53% as yellow solid. LCMS m/z = 280 [M+H]⁺ |
| **55** | tert-butyl 3-(4-(6-chloro-4-methoxypyridin-3-yl)-1H-pyrazol-1-yl)pyrrolidine-1-carboxylate |
| | 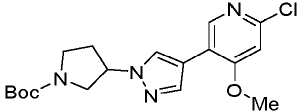 |
| | SM: tert-butyl 3-(4-(4,4,5,5-tetramethyl-1,3,2-dioxaborolan-2-yl)-1H-pyrazol-1-yl)pyrrolidine-1-carboxylate |
| | LCMS m/z = 379 [M+H]⁺ |
| **56** | 2-(4-(6-chloro-4-methoxypyridin-3-yl)-1H-pyrazol-1-yl)-2-methylpropan-1-ol |
| | 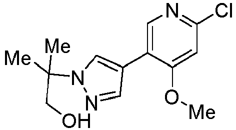 |
| | SM: 2-methyl-2-(4-(4,4,5,5-tetramethyl-1,3,2-dioxaborolan-2-yl)-1H-pyrazol-1-yl)propan-1-ol |
| | yellow solid, 100 mg, 53% yield, LCMS m/z = 282 [M+H]⁺ |

| **Using 5-bromo-2-chloro-4-cyclopropoxypyridine (Preparation 27)** | |
|---|---|
| **57^{A}** | 5-(1-(2-((tert-butyldimethylsilyl)oxy)ethyl)-1H-pyrazol-4-yl)-2-chloro-4-cyclopropoxypyridine |
| | 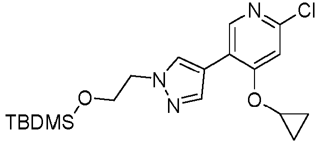 |
| | SM: 1-(2-((tert-butyldimethylsilyl)oxy)ethyl)-4-(4,4,5,5-tetramethyl-1,3,2-dioxaborolan-2-yl)-1H-pyrazole (Preparation 33) |
| | 160 mg, 48% yield as a white solid. LCMS m/z = 394 [M+H]⁺. |
| **58^{A}** | 2-chloro-4-cyclopropoxy-5-(1-(oxetan-3-yl)-1H-pyrazol-4-yl)pyridine |
| | 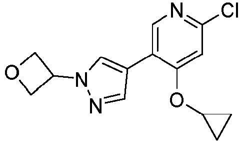 |
| | SM: 1-(oxetan-3-yl)-4-(4,4,5,5-tetramethyl-1,3,2-dioxaborolan-2-yl)-1H-pyrazole |
| | white solid, 180 mg, 61% yield. LCMS m/z = 292 [M+H]⁺ |
| **59^{A}** | 2-chloro-4-cyclopropoxy-5-(1-(2-methoxyethyl)-1H-pyrazol-4-yl)pyridine |
| | 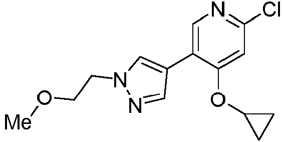 |
| | SM: 1-(2-methoxyethyl)-4-(4,4,5,5-tetramethyl-1,3,2-dioxaborolan-2-yl)-1H-pyrazole |
| | white solid, 110 mg, 55% yield. LCMS m/z = 294 [M+H]⁺ |
| **60^{A}** | 2-chloro-4-cyclopropoxy-5-(1-(oxetan-2-ylmethyl)-1H-pyrazol-4-yl)pyridine |
| | 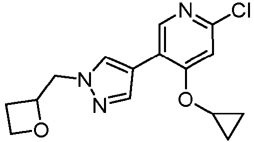 |
| | SM: 1-(oxetan-2-ylmethyl)-4-(4,4,5,5-tetramethyl-1,3,2-dioxaborolan-2-yl)-1H-pyrazole (Preparation 34) |
| | white solid, 220 mg, 88% yield, LCMS m/z = 306 [M+H]⁺ |
| **61^{A}** | 2-chloro-4-cyclopropoxy-5-(1-(tetrahydrofuran-3-yl)-1H-pyrazol-4-yl)pyridine |
| | 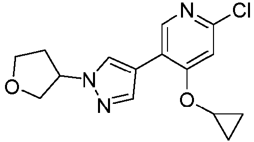 |
| | SM: 1-(tetrahydrofuran-3-yl)-4-(4,4,5,5-tetramethyl-1,3,2-dioxaborolan-2-yl)-1H-pyrazole |
| | white solid, 160 mg, 65% yield, LCMS m/z = 306 [M+H]⁺. |
| **62^{A}** | 2-(4-(6-chloro-4-cyclopropoxypyridin-3-yl)-1H-pyrazol-1-yl)-N,N-dimethylethan-1-amine |
| | 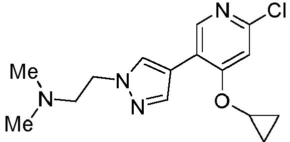 |
| | SM: N,N-dimethyl-2-(4-(4,4,5,5-tetramethyl-1,3,2-dioxaborolan-2-yl)-1H-pyrazol-1-yl)ethan-1-amine |
| | Brown solid, 100 mg, 43% yield. LCMS m/z = 307 [M+H]⁺ |
| **63** | 4-(4-(6-chloro-4-cyclopropoxypyridin-3-yl)-1H-pyrazol-1-yl)-2-methylbutan-2-ol |
| | 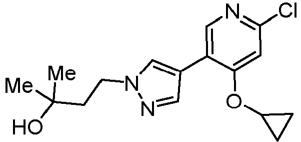 |
| | SM: 2-methyl-4-(4-(4,4,5,5-tetramethyl-1,3,2-dioxaborolan-2-yl)-1H-pyrazol-1-yl)butan-2-ol (Preparation 35). |
| | White solid, 100 mg, 58% yield. LCMS m/z = 322 [M+H]⁺ |
| **64** | 1-(4-(6-chloro-4-cyclopropoxypyridin-3-yl)-1H-pyrazol-1-yl)propan-2-one |
| | 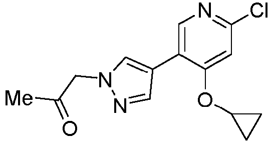 |
| | SM: 1-(4-(4,4,5,5-tetramethyl-1,3,2-dioxaborolan-2-yl)-1H-pyrazol-1-yl)propan-2-one (Preparation 36) |
| | 90 mg, 38% yield. LCMS m/z = 292 [M+H]⁺ |
| **65** | tert-butyl (1-(4-(6-chloro-4-cyclopropoxypyridin-3-yl)-1H-pyrazol-1-yl)-2-methylpropan-2-yl)(methyl)carbamate |
| | 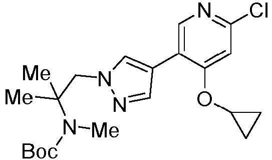 |
| | SM: tert-butyl methyl(2-methyl-1-(4-(4,4,5,5-tetramethyl-1,3,2-dioxaborolan-2-yl)-1H-pyrazol-1-yl)propan-2-yl)carbamate (Preparation 41). |
| | 60 mg, 35% yield. LCMS m/z = 421 [M+H]⁺ |
| **66^{A}** | tert-butyl 3-(4-(6-chloro-4-cyclopropoxypyridin-3-yl)-1H-pyrazol-1-yl)pyrrolidine-1-carboxylate |
| | 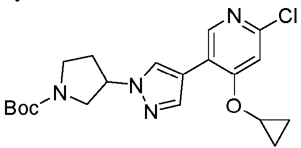 |
| | SM: tert-butyl 3-(4-(4,4,5,5-tetramethyl-1,3,2-dioxaborolan-2-yl)-1H-pyrazol-1-yl)pyrrolidine-1-carboxylate |
| | 180 mg, 55% yield, as a white solid. LCMS m/z = 405 [M+H]⁺ |

| **Using 2-chloro-4-(difluoromethoxy)-5-iodopyridine (Preparation 29)** | |
|---|---|
| **67** | 2-chloro-4-(difluoromethoxy)-5-(1-methyl-1H-pyrazol-4-yl)pyridine |
| | 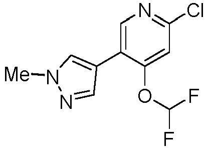 |
| | SM: 1-methyl-4-(4,4,5,5-tetramethyl-1,3,2-dioxaborolan-2-yl)-1H-pyrazole |
| | 50 mg, 58% yield. LCMS m/z = 260 [M+H]⁺ |
| **68** | 2-chloro-5-(1-cyclopropyl-1H-pyrazol-4-yl)-4-(difluoromethoxy)pyridine |
| | 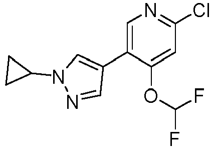 |
| | SM: 1-cyclopropyl-4-(4,4,5,5-tetramethyl-1,3,2-dioxaborolan-2-yl)-1H-pyrazole |
| | 70 mg, 74%. LCMS m/z = 286 [M+H]⁺ |
| **69** | 2-(4-(6-chloro-4-(difluoromethoxy)pyridin-3-yl)-1H-pyrazol-1-yl)-N,N-dimethylethan-1-amine |
| | 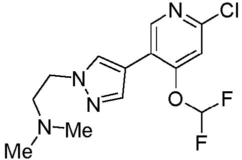 |
| | SM: N,N-dimethyl-2-(4-(4,4,5,5-tetramethyl-1,3,2-dioxaborolan-2-yl)-1H-pyrazol-1-yl)ethan-1-amine |
| | 90 mg, 86% yield. LCMS /z = 317 [M+H]⁺ |
| **70** | tert-butyl (1-(4-(6-chloro-4-(difluoromethoxy)pyridin-3-yl)-1H-pyrazol-1-yl)-2-methylpropan-2-yl)(methyl)carbamate |
| | 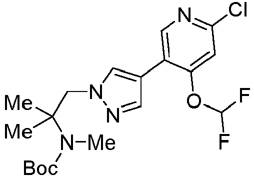 |
| | SM: tert-butyl methyl(2-methyl-1-(4-(4,4,5,5-tetramethyl-1,3,2-dioxaborolan-2-yl)-1H-pyrazol-1-yl)propan-2-yl)carbamate (Preparation 41) |
| | 80 mg, 56% yield. LCMS /z = 431 [M+H]⁺ |
| **71^{A}** | 5-(1-(2-(azetidin-1-yl)ethyl)-1H-pyrazol-4-yl)-2-chloro-4-(difluoromethoxy)pyridine |
| | 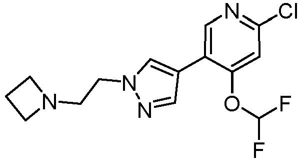 |
| | SM: 1-(2-(azetidin-1-yl)ethyl)-4-(4,4,5,5-tetramethyl-1,3,2-dioxaborolan-2-yl)-1H-pyrazole |
| | White solid, 100 mg, 62% yield. LCMS m/z = 329 [M+H]⁺ |
| **72** | 1-(4-(6-chloro-4-(difluoromethoxy)pyridin-3-yl)-1H-pyrazol-1-yl)propan-2-one |
| | 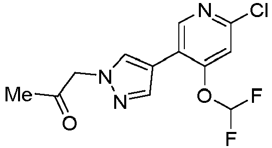 |
| | SM: 1-(4-(4,4,5,5-tetramethyl-1,3,2-dioxaborolan-2-yl)-1H-pyrazol-1-yl)propan-2-one (Preparation 36). |
| | 140 mg, 70% yield. LCMS m/z = 302 [M+H]⁺ |

| **Using 2-chloro-4-((1-fluoropropan-2-yl)oxy)-5-iodopyridine (Preparation 25)** | |
|---|---|
| **73** | 1-(4-(6-chloro-4-((1-fluoropropan-2-yl)oxy)pyridin-3-yl)-1H-pyrazol-1-yl)-2-methylpropan-2-ol |
| | 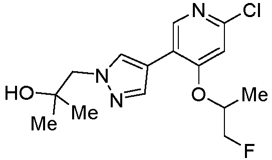 |
| | SM: 2-methyl-1-(4-(4,4,5,5-tetramethyl-1,3,2-dioxaborolan-2-yl)-1H-pyrazol-1-yl)propan-2-ol |
| | white solid, 300 mg, 72% yield. LCMS m/z = 316 [M+H]⁺ |
| **74** | 2-chloro-4-((1-fluoropropan-2-yl)oxy)-5-(1-methyl-1H-pyrazol-4-yl)pyridine |
| | 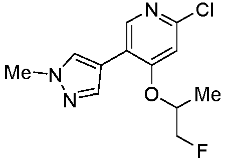 |
| | SM: 1-methyl-4-(4,4,5,5-tetramethyl-1,3,2-dioxaborolan-2-yl)-1H-pyrazole |
| | 90 mg, 33% yield. LCMS m/z = 270 [M+H]⁺ |

| | |
|---|---|
| ^{A}Cs₂CO₃ was used instead of K₂CO₃ | |

### Preparation 75: 2-chloro-4-methoxy-5-(1-(2-((tetrahydro-2H-pyran-2-yl)oxy)ethyl)-1H-pyrazol-4-yl)pyridine

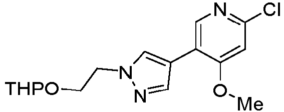

A mixture of 1-(2-((tetrahydro-2H-pyran-2-yl)oxy)ethyl)-4-(4,4,5,5-tetramethyl-1,3,2-dioxaborolan-2-yl)-1H-pyrazole (348 mg, 1.079 mmol), 5-bromo-2-chloro-4-methoxypyridine (Preparation 20, 200 mg, 0.899 mmol), PdCl₂(dppf):DCM (73.4 mg, 0.090 mmol) was purged with N₂. 2M aq. Na₂CO₃ (1.349 mL, 2.70 mmol) and dioxane (3.60 mL) were added and the reaction stirred at 90 °C overnight under N_{2.} The cooled mixture was concentrated *in vacuo* and the residue purified by silica gel chromatography (0-70% EtOAc in hexanes) to give the title compound (256 mg, 84.2%) as a light brown glass. LCMS m/z = 338 [M+H]⁺

### Preparation 76: tert-butyl 2-((4-(6-chloro-4-methoxypyridin-3-yl)-1H-pyrazol-1-yl)methyl)azetidine-1-carboxylate

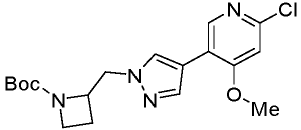

A mixture of tert-butyl 2-((4-(4,4,5,5-tetramethyl-1,3,2-dioxaborolan-2-yl)-1H-pyrazol-1-yl)methyl)azetidine-1-carboxylate (Preparation 38, 400 mg, 1.10 mmol), 5-bromo-2-chloro-4-methoxypyridine (Preparation 20, 244 mg, 1.10 mmol), Pd(dppf)Cl₂ (34 mg, 0.0465 mmol), and TEA (260 mg, 2.57 mmol) in dioxane (6 mL) and H₂O (1.5 mL) was stirred at 80°C for 2 h. The cooled mixture was concentrated *in vacuo* and the residue purified by silica gel chromatography to give the title compound as a yellow solid (150 mg, 36%). LCMS m/z = 379 [M+H]⁺.

### Preparation 77: tert-butyl (2-(4-(6-chloro-4-methoxypyridin-3-yl)-1H-pyrazol-1-yl)ethyl)carbamate

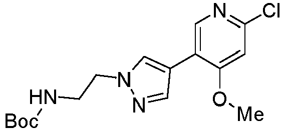

The title compound was obtained as a yellow solid, 180 mg, 50%, from 5-bromo-2-chloro-4-methoxypyridine (Preparation 20) and tert-butyl (2-(4-(4,4,5,5-tetramethyl-1,3,2-dioxaborolan-2-yl)-1H-pyrazol-1-yl)ethyl)carbamate, following the procedure described in Preparation 76. LCMS m/z = 353 [M+H]⁺

### Preparation 78: tert-butyl 2-((4-(6-chloro-4-cyclopropoxypyridin-3-yl)-1H-pyrazol-1-yl)methyl)azetidine-1-carboxylate

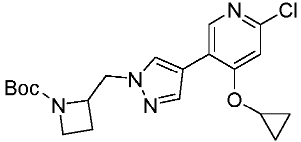

The title compound was obtained as a white solid, 120 mg, 43% yield, from 5-bromo-2-chloro-4-cyclopropoxypyridine (Preparation 27) and tert-butyl 2-((4-(4,4,5,5-tetramethyl-1,3,2-dioxaborolan-2-yl)-1H-pyrazol-1-yl)methyl)azetidine-1-carboxylate (Preparation 38), following the procedure described in Preparation 76. LCMS m/z = 405 [M+H]⁺

### Preparation 79: tert-butyl (2-(4-(6-chloro-4-methoxypyridin-3-yl)-1H-pyrazol-1-yl)ethyl)(methyl)carbamate

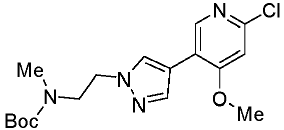

To a solution of 5-bromo-2-chloro-4-methoxypyridine (Preparation 20, 46 mg, 0.207 mmol), tert-butyl methyl(2-(4-(4,4,5,5-tetramethyl-1,3,2-dioxaborolan-2-yl)-1H-pyrazol-1-yl)ethyl)carbamate (Preparation 37, 79.89 mg, 0.227 mmol) in EtOH (3 mL) and H₂O (0.20 mL) was added Pd(amphos)Cl₂ (14.64 mg, 0.0207 mmol) and KOAc (40.59 mg, 0.413 mmol) and the reaction stirred at 80°C for 2 h under N₂. The cooled reaction mixture was concentrated under reduced pressure and the residue was purified by prep-TLC (PE: EtOAc = 0:1) to give the title compound (50 mg, 65.9%) as a yellow oil.

### Preparations 80 to 86

The compounds in the following table were prepared from the appropriate pyrazole pinacol ester and bromopyridine, following a similar procedure to that described in Preparation 79.

| **Prep. No** | **Name/Structure/Starting Materials (SM)/Data** |
|---|---|
| **80** | 2-chloro-5-(1-isopropyl-1H-pyrazol-4-yl)-4-methoxypyridine |
| | 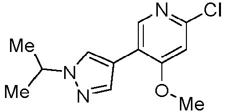 |
| | SM: 1-isopropyl-4-(4,4,5,5-tetramethyl-1,3,2-dioxaborolan-2-yl)-1H-pyrazole and 5-bromo-2-chloro-4-methoxypyridine (Preparation 20) |
| | 84 mg, 37.1% as a yellow oil. |
| **81** | 2-chloro-5-(1-cyclopropyl-1H-pyrazol-4-yl)-4-methoxypyridine |
| | 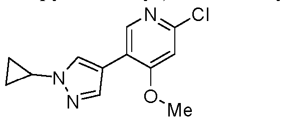 |
| | SM: 1-cyclopropyl-4-(4,4,5,5-tetramethyl-1,3,2-dioxaborolan-2-yl)-1H-pyrazole and 5-bromo-2-chloro-4-methoxypyridine (Preparation 20) |
| | 80 mg, 71.3% as a yellow solid |
| **82** | 2-chloro-4-methoxy-5-(1-(oxetan-3-yl)-1H-pyrazol-4-yl)pyridine |
| | 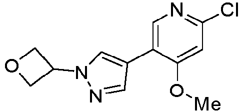 |
| | SM: 1-(oxetan-3-yl)-4-(4,4,5,5-tetramethyl-1,3,2-dioxaborolan-2-yl)-1H-pyrazole and 5-bromo-2-chloro-4-methoxypyridine (Preparation 20) |
| | 33 mg, 39.5% as a yellow solid |
| **83** | tert-butyl 3-(4-(6-chloro-4-methoxypyridin-3-yl)-1H-pyrazol-1-yl)azetidine-1-carboxylate |
| | 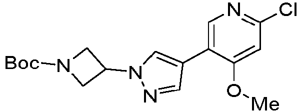 |
| | SM: tert-butyl 3-(4-(4,4,5,5-tetramethyl-1,3,2-dioxaborolan-2-yl)-1H-pyrazol-1-yl)azetidine-1-carboxylate and 5-bromo-2-chloro-4-methoxypyridine (Preparation 20) |
| | 230 mg, crude, as a yellow oil |
| **84** | tert-butyl (2-(4-(6-chloro-4-cyclopropoxypyridin-3-yl)-1H-pyrazol-1-yl)ethyl)(methyl)carbamate |
| | 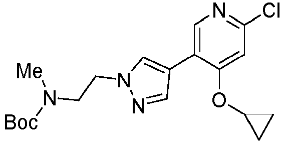 |
| | SM: tert-butyl methyl(2-(4-(4,4,5,5-tetramethyl-1,3,2-dioxaborolan-2-yl)-1H-pyrazol-1-yl)ethyl)carbamate (Preparation 37) and 5-bromo-2-chloro-4-cyclopropoxypyridine (Preparation 27) |
| | yellow oil, 93 mg, 58.8% |
| **85** | tert-butyl 3-(4-(6-chloro-4-cyclopropoxypyridin-3-yl)-1H-pyrazol-1-yl)azetidine-1-carboxylate |
| | 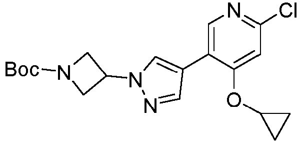 |
| | SM: tert-butyl 3-(4-(4,4,5,5-tetramethyl-1,3,2-dioxaborolan-2-yl)-1H-pyrazol-1-yl)azetidine-1-carboxylate and 5-bromo-2-chloro-4-cyclopropoxypyridine (Preparation 27) |
| | yellow oil, 75 mg, 67% |
| **86** | tert-butyl (2-(4-(6-chloro-4-(oxetan-3-yloxy)pyridin-3-yl)-1H-pyrazol-1-yl)ethyl)(methyl)carbamate |
| | 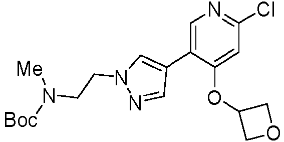 |
| | SM: tert-butyl methyl(2-(4-(4,4,5,5-tetramethyl-1,3,2-dioxaborolan-2-yl)-1H-pyrazol-1-yl)ethyl)carbamate (Preparation 37) and 5-bromo-2-chloro-4-(oxetan-3-yloxy)pyridine (Preparation 28) |
| | yellow oil, 105 mg, 67.9% |

### Preparation 87: tert-butyl 3-(4-(6-chloro-4-(oxetan-3-yloxy)pyridin-3-yl)-1H-pyrazol-1-yl)azetidine-1-carboxylate

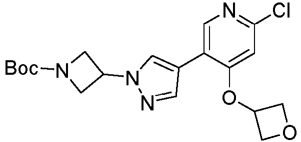

To a solution of 5-bromo-2-chloro-4-(oxetan-3-yloxy)pyridine (Preparation 28, 500 mg, 1.89 mmol), and tert-butyl 3-(4-(4,4,5,5-tetramethyl-1,3,2-dioxaborolan-2-yl)-1H-pyrazol-1-yl)azetidine-1-carboxylate (990.25 mg, 2.84 mmol) in dioxane (5 mL) and H₂O (0.50 mL) was added Xphos Pd G2 (148.73 mg, 0.189 mmol) and K₃PO₄ (802.51 mg, 3.78 mmol) and the reaction stirred at 80 °C for 3 h under N₂. The cooled reaction mixture was concentrated under reduced pressure. The residue was purified by prep HPLC-6 to give the title compound (120 mg, 15.6 %) as a white solid.

### Preparation 88: tert-butyl (1-(4-(6-chloro-4-methoxypyridin-3-yl)-1H-pyrazol-1-yl)-2-methylpropan-2-yl)carbamate

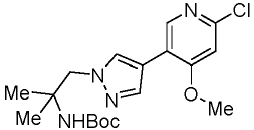

A mixture of tert-butyl (1-(4-bromo-1H-pyrazol-1-yl)-2-methylpropan-2-yl)carbamate (Preparation 39, 543 mg, 1.706 mmol), bis(pinacolato)diboron (520 mg, 2.048 mmol), Pd(dppf)Cl₂:DCM (209 mg, 0.256 mmol) and KOAc (502 mg, 5.12 mmol) in dioxane (6.83 mL) was stirred at 90°C for 9 h. The reaction was cooled to rt then more bis(pinacolato)diboron (200 mg, 0.787 mmol) was added. The reaction mixture was stirred for a further 2 h at 90°C. 5-Bromo-2-chloro-4-methoxypyridine (Preparation 20, 380 mg, 1.706 mmol) and 2M aq. Na₂CO₃ (2.56 mL, 5.12 mmol) were added. The reaction mixture was stirred overnight at 90°C then concentrated *in vacuo* onto silica and purified by silica gel chromatography (0-100% EtAOc in hexanes) to give the title compound (135 mg, 20.8 % yield). LCMS m/z = 381 [M+H]⁺

### Preparation 89: N-(2-(4-(6-chloro-4-methoxypyridin-3-yl)-1H-pyrazol-1-yl)ethyl)-2,2,2-trifluoroethan-1-amine

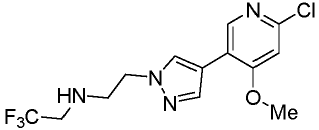

To a solution of tert-butyl (2-(4-(6-chloro-4-methoxypyridin-3-yl)-1H-pyrazol-1-yl)ethyl)carbamate (Preparation 77, 180 mg, 0.510 mmol) in dioxane (5 mL) was added HCl/dioxane (5 mL) and the reaction stirred at rt for 12 h. The mixture was evaporated under reduced pressure. The residue was dissolved in MeOH (5 mL) and DCM (5 mL), 2,2,2-trifluoroacetaldehyde (347 mg, 3.55 mmoL) and AcOH (0.2 mL) added and the solution stirred at rt for 5 mins. Na(CN)BH₃ (5.0 eq) was added and the reaction stirred at rt for 16 h. The solution was quenched with H₂O, extracted with DCM, the combined organic layers were concentrated *in vacuo* and purified by Prep-HPLC-3 to give the title compound as a colorless oil (25 mg, 11%). LCMS m/z = 335 [M+H]⁺.

### Preparation 90: 1-(4-(6-chloro-4-methoxypyridin-3-yl)-1H-pyrazol-1-yl)-N-methylpropan-2-amine

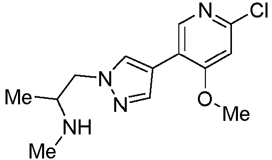

The title compound was obtained as a yellow solid, 200 mg, 64%, from 1-(4-(6-chloro-4-methoxypyridin-3-yl)-1H-pyrazol-1-yl)propan-2-one (Preparation 52) and methanamine, following a similar procedure to that described in Preparation 89. LCMS m/z = 281 [M+H]⁺

### Preparation 91: 2-chloro-4-(difluoromethoxy)-5-(1-(1-methylpyrrolidin-3-yl)-1H-pyrazol-4-yl)pyridine

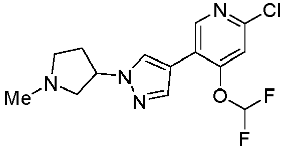

To a solution of tert-butyl 3-(4-(6-chloro-4-(difluoromethoxy)pyridin-3-yl)-1H-pyrazol-1-yl)pyrrolidine-1-carboxylate (Preparation 50, 200 mg, 0.482 mmol) in DCM was added TFA (274 mg, 2.4 mmol) and the reaction stirred at rt for 16 h. The reaction mixture was concentrated *in vacuo* and purified by column chromatography on silica gel (PE/ EtOAc= 1/1) to give 2-chloro-4-(difluoromethoxy)-5-(1-(pyrrolidin-3-yl)-1H-pyrazol-4-yl)pyridine trifluoroacetate.

To a solution of 2-chloro-4-(difluoromethoxy)-5-(1-(pyrrolidin-3-yl)-1H-pyrazol-4-yl)pyridine trifluoroacetate (200 mg) in MeOH (4 mL) was added formaldehyde (95 mg, 3.2 mmol) and AcOH (2 drops) and the solution stirred for 10 mins. Na(CN)BH₃ (201 mg, 3.2 mmol) was added and the reaction stirred at rt for 16 h. The reaction was quenched with 1 M HCl, concentrated *in vacuo* and the residue purified by column chromatography on silica gel (MeOH/ DCM= 5%) to give the title compound (130 mg, 62%). LCMS m/z = 329 [M+H]⁺.

### Preparation 92: 2-chloro-4-methoxy-5-(1-(1-methylpyrrolidin-3-yl)-1H-pyrazol-4-yl)pyridine

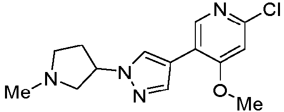

The title compound was obtained as a colorless oil, (120 mg, 64%) from tert-butyl 3-(4-(6-chloro-4-methoxypyridin-3-yl)-1H-pyrazol-1-yl)pyrrolidine-1-carboxylate (Preparation 55), following a similar procedure to that described in Preparation 91. LCMS m/z = 293 [M+H]⁺.

### Preparation 93: 5-(1-(azetidin-2-ylmethyl)-1H-pyrazol-4-yl)-2-chloro-4-methoxypyridine

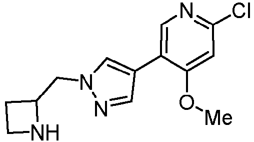

To a solution of tert-butyl 2-((4-(6-chloro-4-methoxypyridin-3-yl)-1H-pyrazol-1-yl)methyl)azetidine-1-carboxylate (Preparation 76, 280 mg, 0.739 mmol) in DCM (2 mL) was added TFA (1 mL) and the reaction stirred at rt for 12 h. The mixture was concentrated *in vacuo* and purified by prep-HPLC-3 to give the title compound as a white solid (160 mg, 78%). LCMS m/z = 279 [M+H]⁺

### Preparations 94 and 95: (R)-2-chloro-4-methoxy-5-(1-((1-methylazetidin-2-yl) methyl)-1H-pyrazol-4-yl)pyridine and (S)-2-chloro-4-methoxy-5-(1-((1-methylazetidin-2-yl)methyl)-1H-pyrazol-4-yl)pyridine

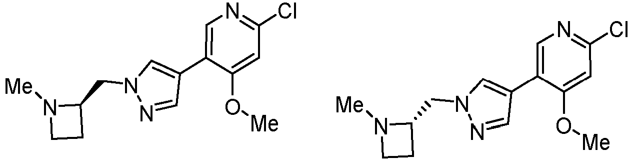

To a solution of 5-(1-(azetidin-2-ylmethyl)-1H-pyrazol-4-yl)-2-chloro-4-methoxy pyridine (Preparation 93, 160 mg, 0.574 mmol) in MeOH (5 mL) and DCM (5 mL) at rt was added HCHO (5.0 eq) and AcOH (0.2 mL) and the solution stirred for 5 min. Na(CN)BH₃ (5.0 eq) was added and the reaction stirred at rt for 16 h. The solution was quenched with H₂O, the mixture extracted with DCM and the combined organic layers were concentrated *in vacuo.* The residue was purified by Prep HPLC-3 to provide 2-chloro-4-methoxy-5-(1-((1-methylazetidin-2-yl)methyl)-1H-pyrazol-4-yl)pyridine as a colorless oil (80 mg, 48%). LCMS m/z = 293[M+H]⁺.

The compound was further purified by SFC using an IG 20 x 250mm, 10µm (Daicel) column, mobile phase: CO₂/MeOH (0.2% MeOH/NH₃) = 50/50, at a flow rate of 100 g/min, to give
First eluting Enantiomer 1 (Preparation 94): (S)-2-chloro-4-methoxy-5-(1-((1-methy lazetidin-2-yl)methyl)-1H-pyrazol-4-yl)pyridine or (R)-2-chloro-4-methoxy-5-(1-((1-methyl azetidin-2-yl)methyl)-1H-pyrazol-4-yl)pyridine, 30 mg
Second eluting Enantiomer 2 (Preparation 95): (R)-2-chloro-4-methoxy-5-(1-((1-methyl azetidin-2-yl)methyl)-1H-pyrazol-4-yl)pyridine or (S)-2-chloro-4-methoxy-5-(1-((1-methyl azetidin-2-yl)methyl)-1H-pyrazol-4-yl)pyridine, 30 mg.

### Preparation 96: 2-chloro-4-fluoro-5-(4,4,5,5-tetramethyl-1,3,2-dioxaborolan-2-yl)pyridine

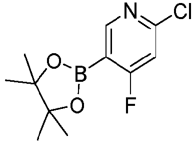

A mixture of 2-chloro-4-fluoropyridine (10 g, 76.0 mmol), bis(pinacolato)diboron (9.64 g, 38.0 mmol), [Ir(OMe)(1,5-cod)]₂ (251 mg, 0.380 mmol) and dtbpy (270 mg, 0.760 mmol) in THF (150 mL) was stirred at 80 °C for 16 h. The mixture was concentrated *in vacuo* and the residue was purified by column chromatography on silica gel eluting with EtOAc/PE (1/10) to afford the title compound (15 g, 77% yield) as colorless oil. LCMS m/z = 258 [M+H]⁺.

### Preparation 97: 2-chloro-4-fluoro-5-(1-methyl-1H-pyrazol-4-yl)pyridine

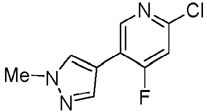

A mixture of 2-chloro-4-fluoro-5-(4,4,5,5-tetramethyl-1,3,2-dioxaborolan-2-yl)pyridine (Preparation 96, 15 g, 58.36 mmol), 4-bromo-1-methyl-1H-pyrazole (9.4 g, 58.36 mmol), K₂CO₃ (16.1 g, 116.73 mmol) and Pd(dppf)Cl₂ (4.27 g, 5.83 mmol) in dioxane/water (80 mL/ 20 mL) was stirred at 80 °C for 6 h. The mixture was concentrated *in vacuo* and the residue was purified by column chromatography on silica gel eluting with EtOAc/PE (1/2) to afford the title compound (5 g, 41% yield) as a brown solid. ¹H-NMR (400 MHz, DMSO-d₆) *δ* ppm 8.83-8.80 (m, 1H), 8.27-8.26 (m, 1H), 8.00 (s, 1H), 7.69-7.67 (m, 1H), 3.90 (s, 3H).

### Preparation 98: 2-chloro-4-ethoxy-5-(1-methyl-1H-pyrazol-4-yl)pyridine

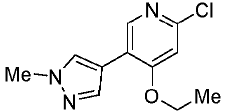

A mixture of 2-chloro-4-fluoro-5-(1-methyl-1H-pyrazol-4-yl)pyridine (Preparation 97, 1.0 g, 4.73 mmol) and EtONa (643 mg, 9.45 mmol) in EtOH (20 mL) was stirred at 80 °C for 16 h. The cooled mixture was concentrated *in vacuo* and purified by silica gel column chromatography to give the title compound (900 mg, 80%). LCMS m/z = 238 [M+H]⁺

### Preparation 99: 2-chloro-4-cyclopropoxy-5-(1-methyl-1H-pyrazol-4-yl)pyridine

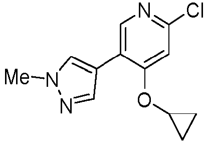

A mixture of 2-chloro-4-fluoro-5-(1-methyl-1H-pyrazol-4-yl)pyridine (Preparation 97, 100 mg, 0.47 mmol), cyclopropanol (54 mg, 0.94 mmol) and Cs₂CO₃ (308 mg, 0.95 mmol) in MeCN (15 mL) was stirred at 80 °C for 16 h. The mixture was concentrated *in vacuo* and the residue was purified by column chromatography on silica gel eluting with EtOAc/PE (1/2) to give the title compound (70 mg, yield 59%). LCMS m/z = 250 [M+H]⁺.

### Preparation 100: 1-(4-(6-amino-4-(trifluoromethoxy)pyridin-3-yl)-1H-pyrazol-1-yl)-2-methylpropan-2-ol

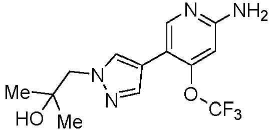

A mixture of 5-bromo-4-(trifluoromethoxy)pyridin-2-amine (Preparation 32, 100 mg, 0.39 mmol), 2-methyl-1-(4-(4,4,5,5-tetramethyl-1,3,2-dioxaborolan-2-yl)-1H-pyrazol-1-yl)propan-2-ol (155 mg, 0.58 mmol), Pd(dppf)Cl₂ (20 mg, 0.027 mmol) and K₂CO₃ (108 mg, 0.78 mmol) in dioxane/water (10 mL/1 mL) was stirred at 100 °C under N₂ for 16 h. The cooled mixture was concentrated *in vacuo* and the residue was purified by silica gel chromatography eluting with MeOH/DCM (1/20) to give the title compound (110 mg, 89%). LCMS m/z = 317 [M+H]⁺.

### Preparations 101 to 105

The compounds in the following table were prepared from 5-bromo-4-(trifluoromethoxy) pyridin-2-amine (Preparation 32) and the appropriate pyrazole pinacol ester, following a similar procedure to that described in Preparation 100.

| **Preparation No** | **Name/Structure/Starting Materials (SM)/Data** |
|---|---|
| **101** | 5-(1-methyl-1H-pyrazol-4-yl)-4-(trifluoromethoxy)pyridin-2-amine |
| | 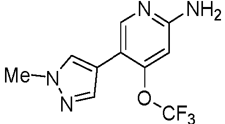 |
| | SM: 1-methyl-4-(4,4,5,5-tetramethyl-1,3,2-dioxaborolan-2-yl)-1H-pyrazole |
| | 50 mg, 84% LCMS m/z = 259 [M+H]⁺ |
| **102** | 5-(1-cyclopropyl-1H-pyrazol-4-yl)-4-(trifluoromethoxy)pyridin-2-amine |
| | 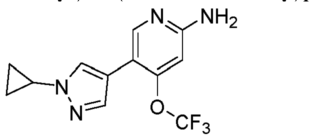 |
| | SM: 1-cyclopropyl-4-(4,4,5,5-tetramethyl-1,3,2-dioxaborolan-2-yl)-1H-pyrazole |
| | 70 mg, 100% yield, LCMS m/z = 285 [M+H]⁺ |
| **103** | 5-(1-(2-(dimethylamino)ethyl)-1H-pyrazol-4-yl)-4-(trifluoromethoxy)pyridin-2-amine |
| | 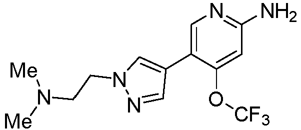 |
| | SM: N,N-dimethyl-2-(4-(4,4,5,5-tetramethyl-1,3,2-dioxaborolan-2-yl)-1H-pyrazol-1-yl)ethan-1-amine |
| | 120 mg, 50% yield, LCMS m/z = 316 [M+H]⁺ |
| **104** | tert-butyl (1-(4-(6-amino-4-(trifluoromethoxy)pyridin-3-yl)-1H-pyrazol-1-yl)-2-methylpropan-2-yl)(methyl)carbamate |
| | 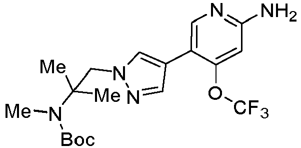 |
| | SM: tert-butyl methyl(2-methyl-1-(4-(4,4,5,5-tetramethyl-1,3,2-dioxaborolan-2-yl)-1H-pyrazol-1-yl)propan-2-yl)carbamate (Preparation 41) |
| | 80 mg, 47%. LCMS m/z = 430 [M+H]⁺ |
| **105** | 1-(4-(6-amino-4-(trifluoromethoxy)pyridin-3-yl)-1H-pyrazol-1-yl)propan-2-one |
| | 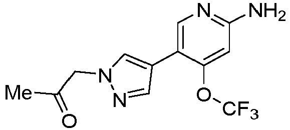 |
| | SM: 1-(4-(4,4,5,5-tetramethyl-1,3,2-dioxaborolan-2-yl)-1H-pyrazol-1-yl)propan-2-one (Preparation 36) |
| | 140 mg, 58% yield. LCMS m/z = 301 [M+H]⁺ |

### Preparation 106: 5-(1-(2-(methylamino)propyl)-1H-pyrazol-4-yl)-4-(trifluoromethoxy)pyridin-2-amine

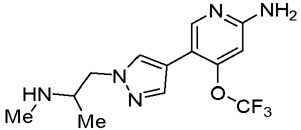

A mixture of 1-(4-(6-amino-4-(trifluoromethoxy)pyridin-3-yl)-1H-pyrazol-1-yl)propan-2-one (Preparation 105, 140 mg, 0.46 mmol) and MeNH₂ (1 mL, 2 M in THF) in DCM (10 mL) was stirred at rt for 0.5 h. Na(CN)BH₃ (58 mg, 0.92 mmol) was added and the reaction stirred at rt for 16 h. The mixture was concentrated *in vacuo* and the residue was purified by column chromatography on silica gel eluting with MeOH/DCM (1/10) to give the title compound (110 mg, yield 75%). LCMS m/z = 316 [M+H]⁺.

### Preparation 107: 1-(4-(6-chloro-4-(difluoromethoxy)pyridin-3-yl)-1H-pyrazol-1-yl)propan-2-ol

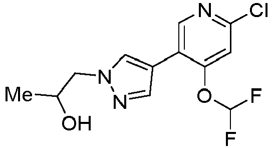

To a solution of 1-(4-(6-chloro-4-(difluoromethoxy)pyridin-3-yl)-1H-pyrazol-1-yl) propan-2-one (Preparation 72, 140 mg, 0.464 mmol) in THF was added NaBH₄ (69.9 mg, 1.85 mmol) at 0 °C and the reaction stirred at rt for 1 h. The reaction mixture was diluted with water and extracted with EtOAc (100 mL x 3). The organic phase was washed with brine, dried over anhydrous Na₂SO₄, concentrated *in vacuo* and purified by column chromatography on silica gel (DCM/ MeOH= 5%) to give the title compound (45 mg, 32%) as white solid. LCMS m/z = 304 [M+H]⁺.

### Preparation 108: 1-(4-(6-((2-(difluoromethyl)-6-((2,4-dimethoxybenzyl)amino) pyrimidin-4-yl)amino)-4-(trifluoromethoxy)pyridin-3-yl)-1H-pyrazol-1-yl)-2-methyl propan-2-ol

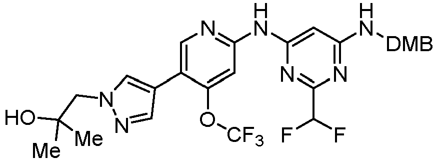

A mixture of 1-(4-(6-amino-4-(trifluoromethoxy)pyridin-3-yl)-1H-pyrazol-1-yl)-2-methylpropan-2-ol (Preparation 100, 110 mg, 0.34 mmol), 6-chloro-2-(difluoromethyl)-N-(2,4-dimethoxybenzyl)pyrimidin-4-amine (Preparation 3, 132 mg, 0.40 mmol), BrettPhos Pd G4 (20 mg, 0.0278 mmol) and Cs₂CO₃ (248 mg, 0.76 mmol) in dry dioxane (10 mL) was stirred at 100 °C under N₂ for 16 h. The cooled mixture was concentrated *in vacuo* and the residue purified by silica gel chromatography to give the title compound (180 mg, 77%). LCMS m/z = 610 [M+H]⁺.

### Preparations 109 to 113

The compounds in the following table were prepared from 6-chloro-2-(difluoromethyl)-N-(2,4-dimethoxybenzyl)pyrimidin-4-amine (Preparation 3) and the appropriate pyridin-2-amine, following a similar procedure to that described in Preparation 108.

| **Preparation No** | **Name/Structure/Starting Materials (SM)/Data** |
|---|---|
| **109** | 2-(difluoromethyl)-N4-(2,4-dimethoxybenzyl)-N6-(5-(1-methyl-1H-pyrazol-4-yl)-4-(trifluoromethoxy)pyridin-2-yl)pyrimidine-4,6-diamine |
| | 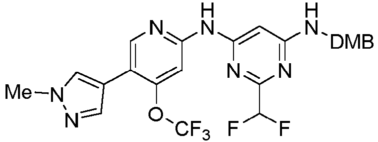 |
| | SM: 5-(1-methyl-1H-pyrazol-4-yl)-4-(trifluoromethoxy)pyridin-2-amine (Preparation 101). |
| | 50 mg, 47% yield. LCMS m/z = 552 [M+H]⁺ |
| **110** | N4-(5-(1-cyclopropyl-1H-pyrazol-4-yl)-4-(trifluoromethoxy)pyridin-2-yl)-2-(difluoromethyl)-N6-(2,4-dimethoxybenzyl)pyrimidine-4,6-diamine |
| | 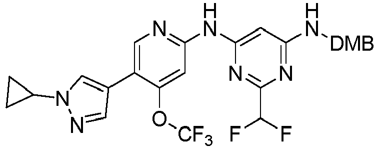 |
| | SM: 5-(1-cyclopropyl-1H-pyrazol-4-yl)-4-(trifluoromethoxy)pyridin-2-amine (Preparation 102) |
| | 60 mg, 44%. LCMS m/z = 564 [M+H]⁺ |
| **111** | 2-(difluoromethyl)-N4-(2,4-dimethoxybenzyl)-N6-(5-(1-(2-(dimethyl amino)ethyl)-1H-pyrazol-4-yl)-4-(trifluoromethoxy)pyridin-2-yl)pyrimidine-4,6-diamine |
| | 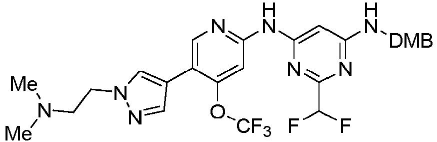 |
| | SM: 5-(1-(2-(dimethylamino)ethyl)-1H-pyrazol-4-yl)-4-(trifluoromethoxy) pyridin-2-amine (Preparation 103) |
| | 200 mg, 86%, LCMS m/z = 609 [M+H]⁺ |
| **112** | 2-(difluoromethyl)-N4-(2,4-dimethoxybenzyl)-N6-(5-(1-(2-(methylamino) propyl)-1H-pyrazol-4-yl)-4-(trifluoromethoxy)pyridin-2-yl)pyrimidine-4,6-diamine |
| | 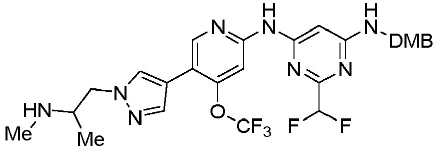 |
| | SM: 5-(1-(2-(methylamino)propyl)-1H-pyrazol-4-yl)-4-(trifluoromethoxy) pyridin-2-amine (Preparation 106) |
| | 100 mg, 48%. LCMS m/z = 608 [M+H]⁺ |
| **113** | tert-butyl (1-(4-(6-((2-(difluoromethyl)-6-((2,4-dimethoxybenzyl) amino)pyrimidin-4-yl)amino)-4-(trifluoromethoxy)pyridin-3-yl)-1H-pyrazol-1-yl)-2-methylpropan-2-yl)(methyl)carbamate |
| | 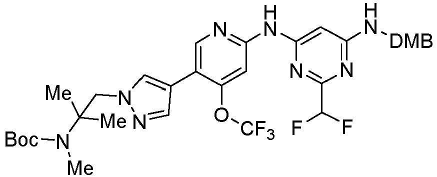 |
| | SM: tert-butyl (1-(4-(6-amino-4-(trifluoromethoxy)pyridin-3-yl)-1H-pyrazol-1-yl)-2-methylpropan-2-yl)(methyl)carbamate (Preparation 104) |
| | 50 mg, 38% yield. LCMS m/z = 723 [M+H]⁺ |

### Preparation 114: (S)-1-(4-(6-((6-((2,4-dimethoxybenzyl)amino)-2-(1-fluoroethyl) pyrimidin-4-yl)amino)-4-isopropoxypyridin-3-yl)-1H-pyrazol-1-yl)-2-methylpropan-2-ol

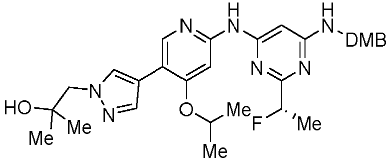

A mixture of 1-(4-(6-chloro-4-isopropoxypyridin-3-yl)-1H-pyrazol-1-yl)-2-methylpropan-2-ol (Preparation 45, 800 mg, 2.59 mmol), (S)-N4-(2,4-dimethoxybenzyl)-2-(1-fluoroethyl)pyrimidine-4,6-diamine (Preparation 13, 800 mg, 2.59 mmol), BrettPhos Pd G4 (397 mg, 0.26 mmol) and Cs₂CO₃ (2.54 g, 7.77 mmol) in dioxane (20 mL) was stirred at 100 °C for 16 h. The cooled mixture was concentrated *in vacuo* and the residue was purified by column chromatography on silica gel (EtOAc/MeOH = 10/1 to 3/1) to afford the title compound (840 mg, 56% yield) as a yellow solid. LCMS m/z = 580 [M+H]⁺.

### Preparation 115: 1-(4-(4-(difluoromethoxy)-6-((2-(difluoromethyl)-6-((2,4-dimethoxybenzyl)amino)pyrimidin-4-yl)amino)pyridin-3-yl)-1H-pyrazol-1-yl)-2-methyl propan-2-ol

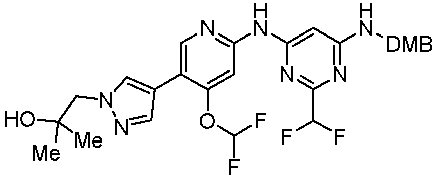

A mixture of 1-(4-(6-chloro-4-(difluoromethoxy)pyridin-3-yl)-1H-pyrazol-1-yl)-2-methylpropan-2-ol (Preparation 47, 50 mg, 0.16 mmol), 2-(difluoromethyl)-N4-(2,4-dimethoxy benzyl)pyrimidine-4,6-diamine (Preparation 5, 54 mg, 0.17 mmol), BrettPhos Pd G4 (10 mg, 0.0109 mmol) and Cs₂CO₃ (104 mg, 0.32 mmol) in dry dioxane (4 mL) was stirred at 100 °C under N₂ for 16 h. The cooled mixture was concentrated *in vacuo* and the crude product was purified by silica gel chromatography to give the title compound (50 mg, 52%). LCMS m/z = 592 [M+H]⁺.

### Preparation 116: tert-butyl (1-(4-(6-((2-(difluoromethyl)-6-((2,4-dimethoxybenzyl) amino)pyrimidin-4-yl)amino)-4-methoxypyridin-3-yl)-1H-pyrazol-1-yl)-2-methylpropan-2-yl)carbamate

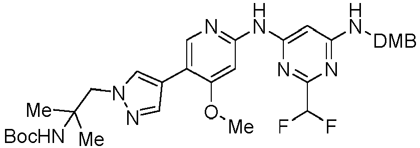

A mixture of tert-butyl (1-(4-(6-chloro-4-methoxypyridin-3-yl)-1H-pyrazol-1-yl)-2-methylpropan-2-yl)carbamate (Preparation 88, 135 mg, 0.354 mmol), 2-(difluoromethyl)-N4-(2,4-dimethoxybenzyl)pyrimidine-4,6-diamine (Preparation 5, 110 mg, 0.354 mmol), BrettPhos Pd G4 (32.7 mg, 0.035 mmol) and Cs₂CO₃ (346 mg, 1.063 mmol) in dioxane (2 mL) was purged with N₂ and the reaction stirred at 80 °C overnight. Additional BrettPhosPd G4 (12 mg, 0.013 mmol) and Cs₂CO₃ (115 mg, 0.353 mmol) were added and the reaction stirred at 100 °C for a further 10 h. The cooled mixture was concentrated onto silica gel and purified by silica gel chromatography (0-100% EtOAc in hexanes) to give the title compound (178 mg, 77 %). LCMS m/z = 655 [M+H]⁺

### Preparation 117: (S)-N4-(2,4-dimethoxybenzyl)-2-(1-fluoroethyl)-N6-(4-isopropoxy-5-(1-(oxetan-3-yl)-1H-pyrazol-4-yl)pyridin-2-yl)pyrimidine-4,6-diamine

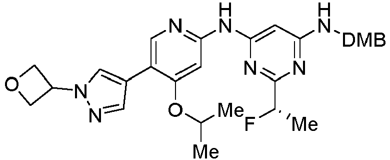

A mixture of 2-chloro-4-isopropoxy-5-(1-(oxetan-3-yl)-1H-pyrazol-4-yl)pyridine (Preparation 48, 100 mg, 0.340 mmol), (S)-N4-(2,4-dimethoxybenzyl)-2-(1-fluoroethyl) pyrimidine-4,6-diamine (Preparation 13, 124 mg, 0.407 mmol), BrettPhos Pd G4 (62.56 mg, 0.068 mmol) and Cs₂CO₃ ( 221 mg, 0.680 mmol) in dioxane (2 mL) was stirred at 70 °C for 12 h. The mixture was concentrated *in vacuo* and the residue was purified by column chromatography on silica gel (MeOH/DCM = 0 to 10 %) to afford the title compound (80 mg, 42% yield) as a white solid. LCMS m/z = 564 [M+H]⁺.

### Preparations 118 to 137

A mixture of the appropriate chloro pyridine (1 equiv.), the appropriate protected pyrimidine-4,6-diamine (1.2 equiv.), BrettPhos Pd G4 (0.03 equiv. to 0.2 equiv.) and Cs₂CO₃ (2-3 equiv.) in dioxane was stirred at between 70-100 °C until the starting materials had been consumed. The cooled mixture was concentrated *in vacuo.* The crude product was purified by silica gel column chromatography using an appropriate solvent system (eg MeOH/DCM or PE/EtOAc) to provide the desired compound.

| **Preparation No.** | **Name/Structure/Starting Materials (SM)/Data** |
|---|---|
| **118** | (S)-N4-(2,4-dimethoxybenzyl)-2-(1-fluoroethyl)-N6-(4-isopropoxy-5-(1-methyl-1H-pyrazol-4-yl)pyridin-2-yl)pyrimidine-4,6-diamine |
| | 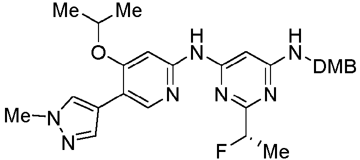 |
| | SM: 2-chloro-4-isopropoxy-5-(1-methyl-1H-pyrazol-4-yl)pyridine (Preparation 44) and (S)-N4-(2,4-dimethoxybenzyl)-2-(1-fluoroethyl) pyrimidine-4,6-diamine (Preparation 13) |
| | yellow solid, 40 mg, 38%, LCMS m/z = 522 [M+H]⁺ |
| **119** | N4-(2,4-dimethoxybenzyl)-2-(fluoromethyl)-N6-(4-isopropoxy-5-(1-methyl-1H-pyrazol-4-yl)pyridin-2-yl)pyrimidine-4,6-diamine |
| | 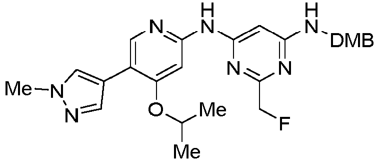 |
| | SM: N4-(2,4-dimethoxybenzyl)-2-(fluoromethyl)pyrimidine-4,6-diamine (Preparation 19) and 2-chloro-4-isopropoxy-5-(1-methyl-1H-pyrazol-4-yl)pyridine (Preparation 44) |
| | yellow solid, 80 mg, 66%. LCMS m/z = 508 [M+H]⁺ |
| **120** | N4-(4-cyclopropoxy-5-(1-methyl-1H-pyrazol-4-yl)pyridin-2-yl)-N6-(2,4-dimethoxybenzyl)-2-(fluoromethyl)pyrimidine-4,6-diamine |
| | 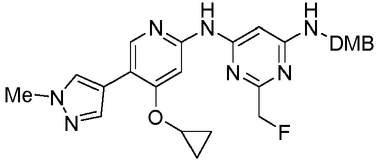 |
| | SM: N4-(2,4-dimethoxybenzyl)-2-(fluoromethyl)pyrimidine-4,6-diamine (Preparation 19) and 2-chloro-4-cyclopropoxy-5-(1-methyl-1H-pyrazol-4-yl)pyridine (Preparation 99) |
| | 40 mg, 24% yield. LCMS m/z = 506 [M+H]⁺ |
| **Using 2-(difluoromethyl)-N4-(2,4-dimethoxybenzyl)pyrimidine-4,6-diamine (Preparation 5)** | |
| **121** | 1-(4-(6-((2-(difluoromethyl)-6-((2,4-dimethoxybenzyl)amino)pyrimidin-4-yl)amino)-4-methoxypyridin-3-yl)-1H-pyrazol-1-yl)propan-2-one |
| | 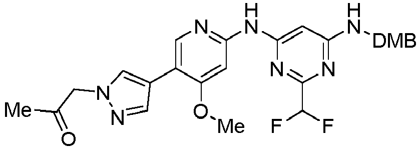 |
| | SM: 1-(4-(6-chloro-4-methoxypyridin-3-yl)-1H-pyrazol-1-yl)propan-2-one (Preparation 52) |
| | white solid, 200 mg, 39%, LCMS m/z = 540 [M+H]⁺ |
| **122** | 2-(difluoromethyl)-N4-(2,4-dimethoxybenzyl)-N6-(4-methoxy-5-(1-(2-((tetrahydro-2H-pyran-2-yl)oxy)ethyl)-1H-pyrazol-4-yl)pyridin-2-yl)pyrimidine-4,6-diamine |
| | 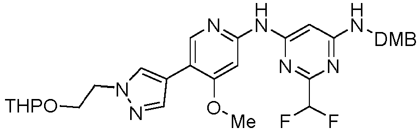 |
| | SM: 2-chloro-4-methoxy-5-(1-(2-((tetrahydro-2H-pyran-2-yl)oxy)ethyl)-1H-pyrazol-4-yl)pyridine (Preparation 75) |
| | LCMS m/z = 612 [M+H]⁺ |
| **123** | tert-butyl 3-(4-(6-((2-(difluoromethyl)-6-((2,4-dimethoxybenzyl)amino)pyrimidin-4-yl)amino)-4-methoxypyridin-3-yl)-1H-pyrazol-1-yl)pyrrolidine-1-carboxylate |
| | 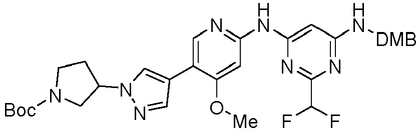 |
| | SM: tert-butyl 3-(4-(6-chloro-4-methoxypyridin-3-yl)-1H-pyrazol-1-yl)pyrrolidine-1-carboxylate (Preparation 55) |
| | LCMS m/z = 653 [M+H]⁺ |
| **124** | 2-(4-(6-((2-(difluoromethyl)-6-((2,4-dimethoxybenzyl)amino)pyrimidin-4-yl)amino)-4-methoxypyridin-3-yl)-1H-pyrazol-1-yl)-2-methylpropan-1-ol |
| | 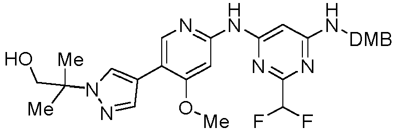 |
| | SM: 2-(4-(6-chloro-4-methoxypyridin-3-yl)-1H-pyrazol-1-yl)-2-methylpropan-1-ol (Preparation 56) |
| | LCMS m/z = 556 [M+H]⁺ |
| **125** | N4-(5-(1-cyclopropyl-1H-pyrazol-4-yl)-4-(difluoromethoxy)pyridin-2-yl)-2-(difluoromethyl)-N6-(2,4-dimethoxybenzyl)pyrimidine-4,6-diamine |
| | 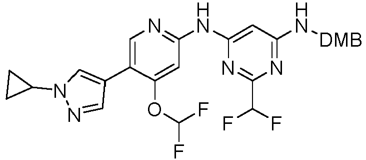 |
| | SM: 2-chloro-5-(1-cyclopropyl-1H-pyrazol-4-yl)-4-(difluoromethoxy)pyridine (Preparation 68) |
| | 80 mg, 59% yield. LCMS m/z = 560 [M+H]⁺ |
| **126** | 1-(4-(4-(difluoromethoxy)-6-((2-(difluoromethyl)-6-((2,4-dimethoxybenzyl)amino)pyrimidin-4-yl)amino)pyridin-3-yl)-1H-pyrazol-1-yl)propan-2-ol |
| | 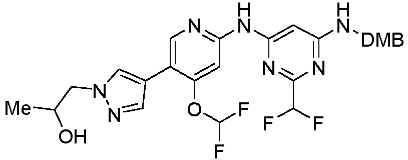 |
| | SM: 1-(4-(6-chloro-4-(difluoromethoxy)pyridin-3-yl)-1H-pyrazol-1-yl)propan-2-ol (Preparation 107). |
| | 15 mg, 17%. LCMS m/z = 578 [M+H]⁺ |
| **127** | N4-(4-(difluoromethoxy)-5-(1-(2-(dimethylamino)ethyl)-1H-pyrazol-4-yl)pyridin-2-yl)-2-(difluoromethyl)-N6-(2,4-dimethoxybenzyl)pyrimidine-4,6-diamine |
| | 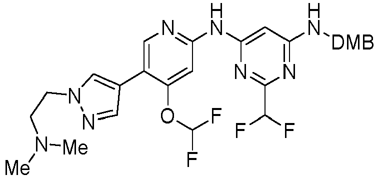 |
| | SM: 2-(4-(6-chloro-4-(difluoromethoxy)pyridin-3-yl)-1H-pyrazol-1-yl)-N,N-dimethylethan-1-amine (Preparation 69) |
| | 100 mg, 60% yield. LCMS m/z = 591 [M+H]⁺ |
| **128**^{A} | tert-butyl (1-(4-(4-(difluoromethoxy)-6-((2-(difluoromethyl)-6-((2,4-dimethoxybenzyl)amino)pyrimidin-4-yl)amino)pyridin-3-yl)-1H-pyrazol-1-yl)-2-methylpropan-2-yl)(methyl)carbamate |
| | 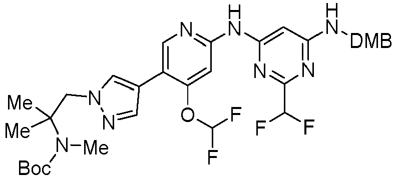 |
| | SM: tert-butyl (1-(4-(6-chloro-4-(difluoromethoxy)pyridin-3-yl)-1H-pyrazol-1-yl)-2-methylpropan-2-yl)(methyl)carbamate (Preparation 70) |
| | 40 mg, 31% yield. LCMS m/z = 705 [M+H]⁺ |
| **129** | N4-(4-(difluoromethoxy)-5-(1-(1-methylpyrrolidin-3-yl)-1H-pyrazol-4-yl)pyridin-2-yl)-2-(difluoromethyl)-N6-(2,4-dimethoxybenzyl)pyrimidine-4,6-diamine |
| | 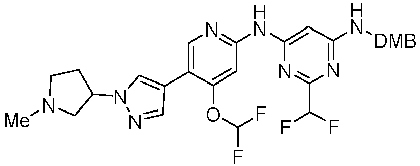 |
| | SM: 2-chloro-4-(difluoromethoxy)-5-(1-(1-methylpyrrolidin-3-yl)-1H-pyrazol-4-yl)pyridine (Preparation 91). |
| | 120 mg, 59%. LCMS m/z = 603 [M+H]⁺ |
| **130** | 1-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)propan-2-one |
| | 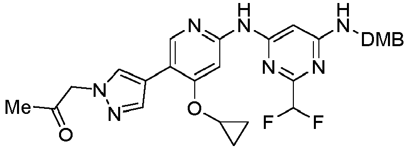 |
| | SM: 1-(4-(6-chloro-4-cyclopropoxypyridin-3-yl)-1H-pyrazol-1-yl)propan-2-one (Preparation 64) |
| | 80 mg, 47% yield. LCMS m/z = 566 [M+H]⁺ |
| **131** | N4-(4-cyclopropoxy-5-(1-(2-methoxyethyl)-1H-pyrazol-4-yl)pyridin-2-yl)-2-(difluoromethyl)-N6-(2,4-dimethoxybenzyl)pyrimidine-4,6-diamine |
| | 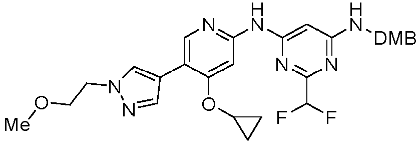 |
| | SM: 2-chloro-4-cyclopropoxy-5-(1-(2-methoxyethyl)-1H-pyrazol-4-yl)pyridine (Preparation 59). |
| | 80 mg, 37% yield as a yellow solid. LCMS m/z = 568 [M+H]⁺ |
| **132** | 4-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)-2-methylbutan-2-ol |
| | 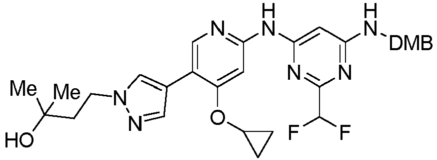 |
| | SM: 4-(4-(6-chloro-4-cyclopropoxypyridin-3-yl)-1H-pyrazol-1-yl)-2-methylbutan-2-ol (Preparation 63) |
| | White solid, 50 mg, 54% yield. LCMS m/z = 596 [M+H]⁺ |
| **133** | N4-(4-cyclopropoxy-5-(1-(2-(dimethylamino)ethyl)-1H-pyrazol-4-yl)pyridin-2-yl)-2-(difluoromethyl)-N6-(2,4-dimethoxybenzyl)pyrimidine-4,6-diamine |
| | 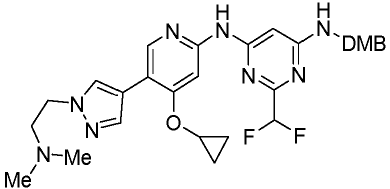 |
| | SM: 2-(4-(6-chloro-4-cyclopropoxypyridin-3-yl)-1H-pyrazol-1-yl)-N,N-dimethylethan-1-amine (Preparation 62) |
| | Brown solid, 60 mg, 32% yield. LCMS m/z = 581 [M+H]⁺ |
| **134** | tert-butyl (1-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)-2-methylpropan-2-yl)(methyl)carbamate |
| | 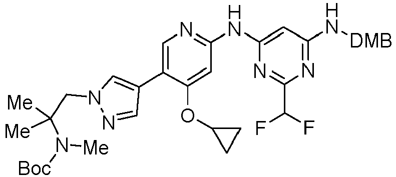 |
| | SM: tert-butyl (1-(4-(6-chloro-4-cyclopropoxypyridin-3-yl)-1H-pyrazol-1-yl)-2-methylpropan-2-yl)(methyl)carbamate (Preparation 65) |
| | 60 mg, 61% yield. LCMS m/z = 695 [M+H]⁺ |
| **135^{A}** | N4-(4-cyclopropoxy-5-(1-(oxetan-2-ylmethyl)-1H-pyrazol-4-yl)pyridin-2-yl)-2-(difluoromethyl)-N6-(2,4-dimethoxybenzyl)pyrimidine-4,6-diamine |
| | 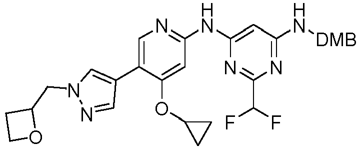 |
| | SM: 2-chloro-4-cyclopropoxy-5-(1-(oxetan-2-ylmethyl)-1H-pyrazol-4-yl)pyridine (Preparation 60). |
| | 80 mg, 21% yield as a white solid. LCMS m/z = 580 [M+H]⁺ |
| **136** | N4-(4-cyclopropoxy-5-(1-(oxetan-3-yl)-1H-pyrazol-4-yl)pyridin-2-yl)-2-(difluoromethyl)-N6-(2,4-dimethoxybenzyl)pyrimidine-4,6-diamine |
| | 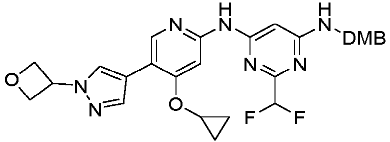 |
| | SM: 2-chloro-4-cyclopropoxy-5-(1-(oxetan-3-yl)-1H-pyrazol-4-yl)pyridine (Preparation 58). |
| | 200 mg, 57% yield as a yellow solid. LCMS m/z = 566 [M+H]⁺ |
| **137** | N4-(4-cyclopropoxy-5-(1-(tetrahydrofuran-3-yl)-1H-pyrazol-4-yl)pyridin-2-yl)-2-(difluoromethyl)-N6-(2,4-dimethoxybenzyl)pyrimidine-4,6-diamine |
| | 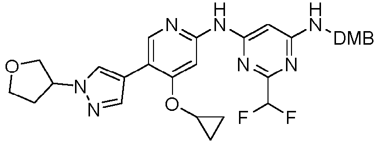 |
| | SM: 2-chloro-4-cyclopropoxy-5-(1-(tetrahydrofuran-3-yl)-1H-pyrazol-4-yl)pyridine (Preparation 61). |
| | White solid, 150 mg, 49% yield. LCMS m/z = 580 [M+H]⁺ |

| | |
|---|---|
| ^{A}-K₂CO₃ was used in place of Cs₂CO₃ | |

### Preparation 138: 1-(4-(4-(difluoromethoxy)-6-((2-(difluoromethyl)-6-((2,4-dimethoxybenzyl)amino)pyrimidin-4-yl)amino)pyridin-3-yl)-1H-pyrazol-1-yl)propan-2-one

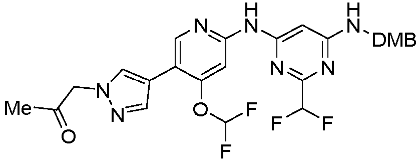

A mixture of 1-(4-(6-chloro-4-(difluoromethoxy)pyridin-3-yl)-1H-pyrazol-1-yl)propan-2-one (Preparation 72, 140 mg, 0.46 mmol), 2-(difluoromethyl)-N4-(2,4-dimethoxybenzyl) pyrimidine-4,6-diamine (Preparation 5, 143 mg, 0.28 mmol), BrettPhos Pd G4 (10 mg, 0.011 mmol) and Cs₂CO₃ (300 mg, 0.92 mmol) in dioxane (5 mL) was stirred at 100°C under N₂ for 16 h. The cooled mixture was concentrated *in vacuo* and the residue purified by column chromatography on silica gel eluting with MeOH/DCM (100%~10%) to give title compound (110 mg, yield 41%). LCMS m/z = 576 [M+H]⁺.

### Preparation 139: 1-(4-(6-((2-(difluoromethyl)-6-((2,4-dimethoxybenzyl)amino) pyrimidin-4-yl)amino)-4-(methoxy-d3)pyridin-3-yl)-1H-pyrazol-1-yl)-2-methylpropan-2-ol

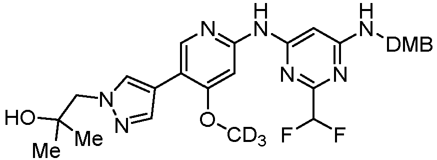

A mixture of 1-(4-(6-chloro-4-(methoxy-d3)pyridin-3-yl)-1H-pyrazol-1-yl)-2-methyl propan-2-ol (Preparation 43, 400 mg, 1.40 mmol), 2-(difluoromethyl)-N4-(2,4-dimethoxy benzyl)pyrimidine-4,6-diamine (Preparation 5, 434 mg, 1.40 mmol), Cs₂CO₃ (910 mg, 2.80 mmol) and Brettphos Pd G4 (100 mg) in dioxane (10 mL) was stirred at 100 °C for 16 h under N₂. The mixture was concentrated *in vacuo* and the residue was purified by column chromatography on silica gel eluting with MeOH/DCM (1/20) to afford the title compound (300 mg, 38%) as a white solid. LCMS m/z = 559 [M+H]⁺.

### Preparations 140 and 141: tert-butyl (S)-3-(4-(4-cyclopropoxy-6-((2-(difluoro methyl)-6-((2,4-dimethoxybenzyl)amino)pyrimidin-4-yl)amino)pyridin-3-yl)-1H-pyrazol-1-yl)pyrrolidine-1-carboxylate and tert-butyl (R)-3-(4-(4-cyclopropoxy-6-((2-(difluoro methyl)-6-((2,4-dimethoxybenzyl)amino)pyrimidin-4-yl)amino)pyridin-3-yl)-1H-pyrazol-1-yl)pyrrolidine-1-carboxylate

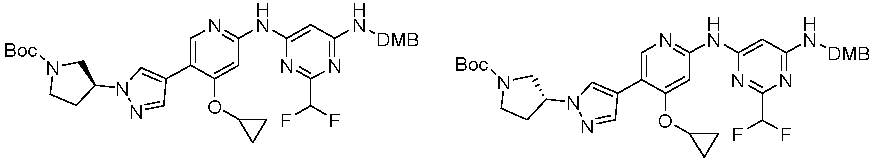

A mixture of tert-butyl 3-(4-(6-chloro-4-cyclopropoxypyridin-3-yl)-1H-pyrazol-1-yl)pyrrolidine-1-carboxylate (Preparation 66, 180 mg, 0.44 mmol), 2-(difluoromethyl)-N4-(2,4-dimethoxybenzyl)pyrimidine-4,6-diamine (Preparation 5, 137 mg, 0.44 mmol), Cs₂CO₃ (290 mg, 0.89 mmol) and Brettphos Pd G4 (40 mg, 0.043 mmol) in dioxane (4 mL) was stirred at 100 °C for 16 h under N₂. The cooled reaction mixture was concentrated *in vacuo* and the residue was purified by Prep HPLC-3. The product was further purified by SFC using an AD 20x250mm, 10µm (Daicel) column, mobile phase: CO₂/MEOH (1% MeOH/NH₃) = 50/50 at 100 g/min to afford:
First eluting Enantiomer 1: tert-butyl (S)-3-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)pyrrolidine-1-carboxylate or tert-butyl (R)-3-(4-(4-cyclopropoxy-6-((2-(difluoromethyl)-6-((2,4-dimethoxy benzyl)amino)pyrimidin-4-yl)amino)pyridin-3-yl)-1H-pyrazol-1-yl)pyrrolidine-1-carboxylate (Preparation 140), (20 mg, 7% yield) as a white solid. LCMS m/z = 679 [M+H]⁺ and
Second eluting Enantiomer 2: tert-butyl (R)-3-(4-(4-cyclopropoxy-6-((2-(difluoro methyl)-6-((2,4-dimethoxybenzyl)amino)pyrimidin-4-yl)amino)pyridin-3-yl)-1H-pyrazol-1-yl)pyrrolidine-1-carboxylate or tert-butyl (S)-3-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)pyrrolidine-1-carboxylate (Preparation 141), (20 mg, 7% yield) as a white solid. LCMS m/z = 679 [M+H]⁺.

### Preparations 142 and 143: tert-butyl (R)-2-((4-(4-cyclopropoxy-6-((2-(difluoro methyl)-6-((2,4-dimethoxybenzyl)amino)pyrimidin-4-yl)amino)pyridin-3-yl)-1H-pyrazol-1-yl)methyl)azetidine-1-carboxylate and tert-butyl (S)-2-((4-(4-cyclopropoxy-6-((2-(difluoro methyl)-6-((2,4-dimethoxybenzyl)amino)pyrimidin-4-yl)amino)pyridin-3-yl)-1H-pyrazol-1-yl)methyl)azetidine-1-carboxylate

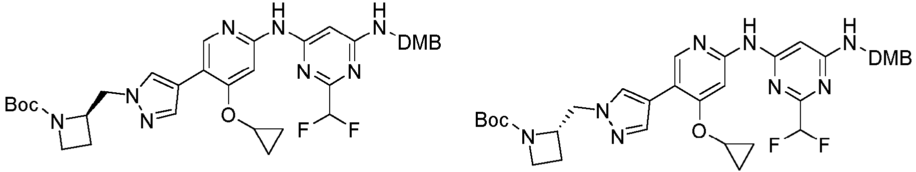

A mixture of tert-butyl 2-((4-(6-chloro-4-cyclopropoxypyridin-3-yl)-1H-pyrazol-1-yl)methyl)azetidine-1-carboxylate (Preparation 78, 100 mg, 0.246 mmol), 2-(difluoromethyl)-N4-(2,4-dimethoxybenzyl)pyrimidine-4,6-diamine (Preparation 5, 76.0 mg, 0.245 mmol), K₂CO₃ (67.7 mg, 0.491 mmol) and Brettphos Pd G4 (20 mg) in dioxane (4 mL) was stirred at 100 °C for 16 h under N₂. The cooled reaction mixture was concentrated *in vacuo.* The residue was purified by Prep-HPLC-3. The product was further purified by chiral-SFC using an AD 20 x 250mm, 10µm (Daicel) column, mobile phase: CO₂/EtOH (1% MeOH/NH₃) = 50/50 at 100 g/min, to afford:
First eluting Enantiomer 1: tert-butyl (R)-2-((4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)methyl)azetidine-1-carboxylate or tert-butyl (S)-2-((4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)methyl)azetidine-1-carboxylate (Preparation 142) (12 mg, 7% yield) as a white solid. LCMS m/z = 679 [M+H]⁺ and
Second eluting Enantiomer 2: tert-butyl (S)-2-((4-(4-cyclopropoxy-6-((2-(difluoro methyl)-6-((2,4-dimethoxybenzyl)amino)pyrimidin-4-yl)amino)pyridin-3-yl)-1H-pyrazol-1-yl)methyl)azetidine-1-carboxylate or tert-butyl (R)-2-((4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)methyl)azetidine-1-carboxylate (Preparation 143) (12 mg, 7% yield) as a white solid. LCMS m/z = 679 [M+H]⁺.

### Preparations 144 and 145: N4-(2,4-dimethoxybenzyl)-2-((S)-1-fluoroethyl)-N6-(4-isopropoxy-5-(1-((R)-tetrahydrofuran-3-yl)-1H-pyrazol-4-yl)pyridin-2-yl)pyrimidine-4,6-diamine and N4-(2,4-dimethoxybenzyl)-2-((S)-1-fluoroethyl)-N6-(4-isopropoxy-5-(1-((S)-tetrahydrofuran-3-yl)-1H-pyrazol-4-yl)pyridin-2-yl)pyrimidine-4,6-diamine

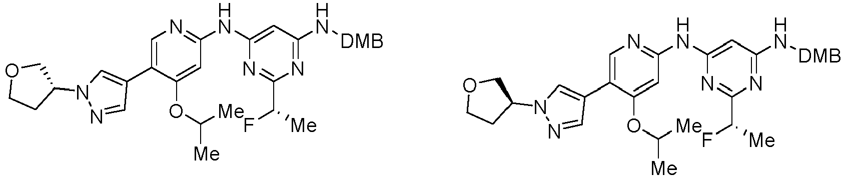

A mixture of 2-chloro-4-isopropoxy-5-(1-(tetrahydrofuran-3-yl)-1H-pyrazol-4-yl) pyridine (Preparation 49, 100 mg, 0.325 mmol), (S)-N4-(2,4-dimethoxybenzyl)-2-(1-fluoro ethyl)pyrimidine-4,6-diamine (Preparation 13, 120 mg, 0.390 mmol), BrettPhos Pd G4 (0.2 equiv.) and Cs₂CO₃ (212 mg, 0.650 mmol) in dioxane was stirred at 100 °C overnight. The cooled solution was concentrated *in vacuo* and the residue was purified by column chromatography on silica gel (DCM/ MeOH= 10%) to give N4-(2,4-dimethoxybenzyl)-2-((S)-1-fluoroethyl)-N6-(4-isopropoxy-5-(1-(tetrahydrofuran-3-yl)-1H-pyrazol-4-yl)pyridin-2-yl)pyrimidine-4,6-diamine. (50 mg, 27 %). LCMS m/z = 578 [M+H]⁺
This was further separated by prep-SFC using an AD 20 x 250mm, 10µm (Daicel) column, mobile phase: CO₂/EtOH (0.2% MeOH/NH₃) = 55/45, at 100 g/min to give:
First eluting Diastereoisomer 1: N4-(2,4-dimethoxybenzyl)-2-((S)-1-fluoroethyl)-N6-(4-isopropoxy-5-(1-((R)-tetrahydrofuran-3-yl)-1H-pyrazol-4-yl)pyridin-2-yl)pyrimidine-4,6-diamine or N4-(2,4-dimethoxybenzyl)-2-((S)-1-fluoroethyl)-N6-(4-isopropoxy-5-(1-((S)-tetrahydrofuran-3-yl)-1H-pyrazol-4-yl)pyridin-2-yl)pyrimidine-4,6-diamine (Preparation 144), 16 mg and
Second eluting Diastereoisomer 2: N4-(2,4-dimethoxybenzyl)-2-((S)-1-fluoroethyl)-N6-(4-isopropoxy-5-(1-((S)-tetrahydrofuran-3-yl)-1H-pyrazol-4-yl)pyridin-2-yl)pyrimidine-4,6-diamine or N4-(2,4-dimethoxybenzyl)-2-((S)-1-fluoroethyl)-N6-(4-isopropoxy-5-(1-((R)-tetrahydrofuran-3-yl)-1H-pyrazol-4-yl)pyridin-2-yl)pyrimidine-4,6-diamine (Preparation 145), 12 mg.

### Preparation 146: 1-(4-(6-((2-(difluoromethyl)-6-((2,4-dimethoxybenzyl)amino) pyrimidin-4-yl)amino)-4-methoxypyridin-3-yl)-1H-pyrazol-1-yl)-2-methylpropan-2-ol

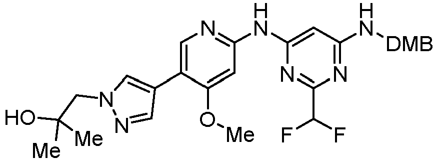

A mixture of 1-(4-(6-chloro-4-methoxypyridin-3-yl)-1H-pyrazol-1-yl)-2-methylpropan-2-ol (Preparation 42, 560 mg, 1.99 mmol), 2-(difluoromethyl)-N4-(2,4-dimethoxybenzyl) pyrimidine-4,6-diamine (Preparation 5, 679 mg, 2.19 mmol), Pd(t-Bu₃P)₂ (200 mg, 0.392 mmol) and Cs₂CO₃ (1.29 g, 3.98 mmol) in dioxane (12 mL) was stirred at 100°C for 16 h under N₂. The mixture was poured into water (80 mL) and extracted with EtOAc (100 mL x 3). The combined organic layer was concentrated *in vacuo* and the crude product was purified by silica gel chromatography (PE: EtOAc =1:10) to give the title compound (480 mg, 43.6%) as a yellow oil. LCMS m/z = 556 [M+H]⁺.

### Preparation 147: 1-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)-2-methylpropan-2-ol

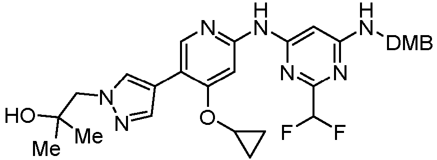

A mixture of 1-(4-(6-chloro-4-cyclopropoxypyridin-3-yl)-1H-pyrazol-1-yl)-2-methyl propan-2-ol (Preparation 46, 900 mg, 2.93 mmol), 2-(difluoromethyl)-N4-(2,4-dimethoxy benzyl)pyrimidine-4,6-diamine (Preparation 5, 1 g, 3.22 mmol), Pd(t-Bu₃P)₂ (300 mg, 0.586 mmol) and Cs₂CO₃ (1.9 g, 5.86 mmol) in dioxane (20 mL) was stirred at 100°C for 16 h under N₂. The cooled mixture was poured into water (80 mL) and extracted with EtOAc (100 mL x 3). The combined organic layer was concentrated *in vacuo* and the crude product was purified by silica gel chromatography (PE: EtOAc =1:10) to give the title compound (800 mg, 47.1%) as a beige solid. LCMS m/z = 582 [M+H]⁺.

### Preparation 148: 2-(difluoromethyl)-N4-(2,4-dimethoxybenzyl)-N6-(4-((1-fluoro propan-2-yl)oxy)-5-(1-methyl-1H-pyrazol-4-yl)pyridin-2-yl)pyrimidine-4,6-diamine

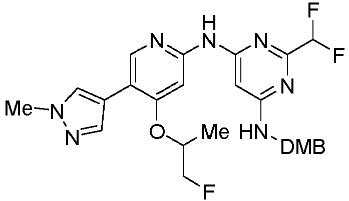

The title compound was obtained, 80 mg, 44% yield, from 2-chloro-4-((1-fluoropropan-2-yl)oxy)-5-(1-methyl-1H-pyrazol-4-yl)pyridine (Preparation 74) and 2-(difluoromethyl)-N4-(2,4-dimethoxybenzyl)pyrimidine-4,6-diamine (Preparation 5), following a similar procedure to that described in Preparation 147. LCMS m/z = 544 [M+H]⁺

### Preparation 149: N4-(5-(1-(2-((tert-butyldimethylsilyl)oxy)ethyl)-1H-pyrazol-4-yl)-4-cyclopropoxypyridin-2-yl)-2-(difluoromethyl)-N6-(2,4-dimethoxybenzyl)pyrimidine-4,6-diamine

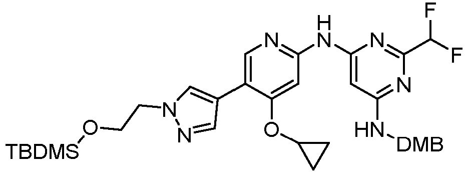

The title compound was obtained, 80 mg, 29% yield, from 5-(1-(2-((tert-butyldimethyl silyl)oxy)ethyl)-1H-pyrazol-4-yl)-2-chloro-4-cyclopropoxypyridine (Preparation 57) and 2-(difluoromethyl)-N4-(2,4-dimethoxybenzyl)pyrimidine-4,6-diamine (Preparation 5), following a similar procedure to that described in Preparation 147. LCMS m/z = 668 [M+H]⁺

### Preparation 150: N4-(4-(difluoromethoxy)-5-(1-(2-(methylamino)propyl)-1H-pyrazol-4-yl)pyridin-2-yl)-2-(difluoromethyl)-N6-(2,4-dimethoxybenzyl)pyrimidine-4,6-diamine

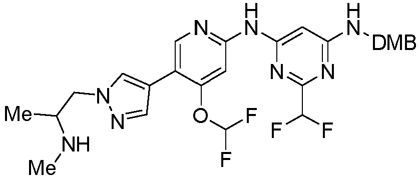

The title compound was obtained, 80 mg, 71% yield, from 1-(4-(4-(difluoromethoxy)-6-((2-(difluoromethyl)-6-((2,4-dimethoxybenzyl)amino)pyrimidin-4-yl)amino)pyridin-3-yl)-1H-pyrazol-1-yl)propan-2-one (Preparation 138) and MeNH₂, following a similar procedure to that described in Preparation 106. LCMS m/z = 591 [M+H]⁺.

### Preparation 151: 1-(4-(6-((2-(difluoromethyl)-6-((2,4-dimethoxybenzyl) amino)pyrimidin-4-yl)amino)-4-methoxypyridin-3-yl)-1H-pyrazol-1-yl)propan-2-ol

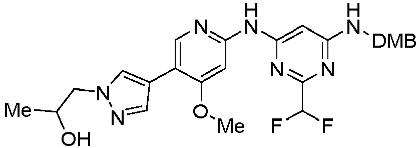

To a solution of 1-(4-(6-((2-(difluoromethyl)-6-((2,4-dimethoxybenzyl)amino)pyrimidin-4-yl)amino)-4-methoxypyridin-3-yl)-1H-pyrazol-1-yl)propan-2-one (Preparation 121, 100 mg, 0.185 mmol) in MeOH (5 mL) was added NaBH₄ (21.0 mg, 0.56 mmol) and the reaction stirred at rt for 2 h. The mixture was concentrated *in vacuo* and the residue purified by silica gel chromatography to give the title compound as a white solid (50 mg, 50%). LCMS m/z = 542 [M+H]⁺.

### Preparation 152: 2-(difluoromethyl)-N4-(2,4-dimethoxybenzyl)-N6-(5-(1-(2-(dimethylamino)propyl)-1H-pyrazol-4-yl)-4-methoxypyridin-2-yl)pyrimidine-4,6-diamine

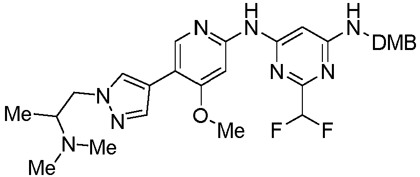

To a solution of 1-(4-(6-((2-(difluoromethyl)-6-((2,4-dimethoxybenzyl)amino)pyrimidin-4-yl)amino)-4-methoxypyridin-3-yl)-1H-pyrazol-1-yl)propan-2-one (Preparation 121, 100 mg, 0.185 mmol) in DCM/MeOH (1/1, 5 mL) was added dimethylamine in THF (2 mL, 2 N) and AcOH (5 drops) and the solution stirred for 5 mins. Na(CN)BH₃ (34.85 mg, 0.56 mmol) was added and the reaction stirred at rt for 16 h. The solution was concentrated *in vacuo* and the residue purified by silica gel chromatography to give the title compound as a yellow solid (65 mg, 62%) LCMS m/z = 569 [M+H]⁺.

### Preparation 153: N4-(4-cyclopropoxy-5-(1-(2-(methylamino)propyl)-1H-pyrazol-4-yl)pyridin-2-yl)-2-(difluoromethyl)-N6-(2,4-dimethoxybenzyl)pyrimidine-4,6-diamine

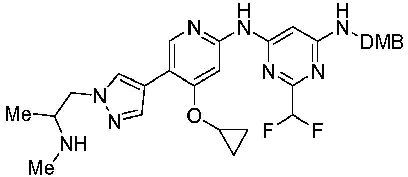

The title compound was obtained, 50 mg, 61% yield, from 1-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)propan-2-one (Preparation 130) and MeNH₂ following a similar procedure to that described in Preparation 106. LCMS m/z = 581 [M+H]⁺.

### Preparation 154: tert-butyl 3-(4-(6-((6-amino-2-(fluoromethyl)pyrimidin-4-yl)amino)-4-methoxypyridin-3-yl)-1H-pyrazol-1-yl)azetidine-1-carboxylate

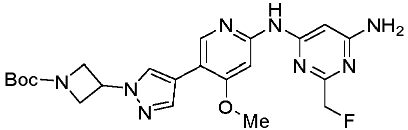

To a solution of 2-(fluoromethyl)pyrimidine-4,6-diamine (Preparation 18, 15.58 mg, 0.11 mmol), tert-butyl 3-(4-(6-chloro-4-methoxypyridin-3-yl)-1H-pyrazol-1-yl)azetidine-1-carboxylate (Preparation 83, 50 mg, 0.137 mmol) in dioxane (2 mL) was added BrettPhos Pd, G4 (12.62 mg, 0.0137 mmol) and Cs₂CO₃ (89.31 mg, 0.274 mmol) and the reaction stirred at 90°C for 2 h under N₂. The cooled reaction mixture was concentrated under reduced pressure and the residue purified by prep-TLC (EtOAc: MeOH = 10: 1) to give the title compound (15 mg, 23.26%) as a yellow oil.

### Preparations 155 to 160

The compounds in the following table were prepared from 2-(difluoromethyl)pyrimidine-4,6-diamine hydrochloride (Preparation 7) and the appropriate chloro-pyridine, following a similar procedure to that described in Preparation 154.

| **Prep No** | **Name/Structure/Starting chloropyridine/Data** |
|---|---|
| **155** | tert-butyl (2-(4-(6-((6-amino-2-(difluoromethyl)pyrimidin-4-yl)amino)-4-methoxypyridin-3-yl)-1H-pyrazol-1-yl)ethyl)(methyl)carbamate |
| | 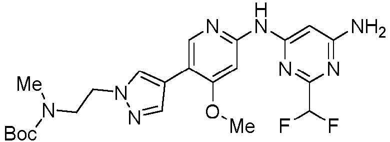 |
| | Chloropyridine: tert-butyl (2-(4-(6-chloro-4-methoxypyridin-3-yl)-1H-pyrazol-1-yl)ethyl)(methyl)carbamate (Preparation 79) |
| | yellow oil, 50 mg crude |
| **156** | tert-butyl 3-(4-(6-((6-amino-2-(difluoromethyl)pyrimidin-4-yl)amino)-4-methoxypyridin-3-yl)-1H-pyrazol-1-yl)azetidine-1-carboxylate |
| | 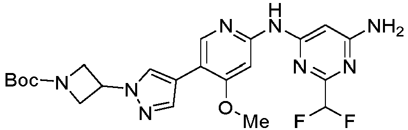 |
| | Chloropyridine: tert-butyl 3-(4-(6-chloro-4-methoxypyridin-3-yl)-1H-pyrazol-1-yl)azetidine-1-carboxylate (Preparation 83) |
| | brown solid, 50 mg, crude |
| **157** | tert-butyl (2-(4-(6-((6-amino-2-(difluoromethyl)pyrimidin-4-yl)amino)-4-cyclopropoxypyridin-3-yl)-1H-pyrazol-1-yl)ethyl)(methyl)carbamate |
| | 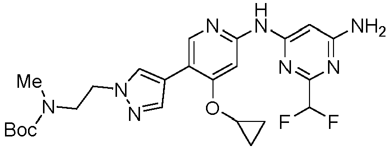 |
| | Chloropyridine: tert-butyl (2-(4-(6-chloro-4-cyclopropoxypyridin-3-yl)-1H-pyrazol-1-yl)ethyl)(methyl)carbamate (Preparation 84) |
| | yellow oil, 24 mg, 60.9% |
| **158** | tert-butyl 3-(4-(6-((6-amino-2-(difluoromethyl)pyrimidin-4-yl)amino)-4-cyclopropoxypyridin-3-yl)-1H-pyrazol-1-yl)azetidine-1-carboxylate |
| | 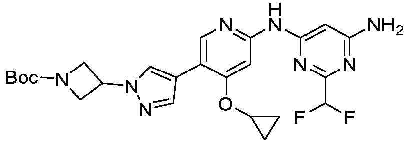 |
| | Chloropyridine: tert-butyl 3-(4-(6-chloro-4-cyclopropoxypyridin-3-yl)-1H-pyrazol-1-yl)azetidine-1-carboxylate (Preparation 85) |
| | white solid, 50 mg, 54.2% |
| **159** | tert-butyl 3-(4-(6-((6-amino-2-(difluoromethyl)pyrimidin-4-yl)amino)-4-(oxetan-3-yloxy)pyridin-3-yl)-1H-pyrazol-1-yl)azetidine-1-carboxylate |
| | 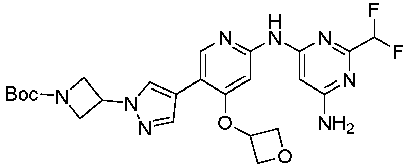 |
| | Chloropyridine: tert-butyl 3-(4-(6-chloro-4-(oxetan-3-yloxy)pyridin-3-yl)-1H-pyrazol-1-yl)azetidine-1-carboxylate (Preparation 87) |
| | yellow oil, 62 mg, 43.2% |
| **160** | tert-butyl (2-(4-(6-((6-amino-2-(difluoromethyl)pyrimidin-4-yl)amino)-4-(oxetan-3-yloxy)pyridin-3-yl)-1H-pyrazol-1-yl)ethyl)(methyl)carbamate |
| | 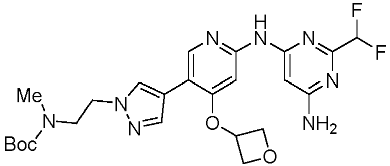 |
| | Chloropyridine: tert-butyl (2-(4-(6-chloro-4-(oxetan-3-yloxy)pyridin-3-yl)-1H-pyrazol-1-yl)ethyl)(methyl)carbamate (Preparation 86) |
| | yellow oil, 26 mg, 66.5% yield |

### Preparations 161 and 162: tert-butyl (S)-3-(4-(6-((6-amino-2-(difluoromethyl) pyrimidin-4-yl)amino)-4-methoxypyridin-3-yl)-1H-pyrazol-1-yl)pyrrolidine-1-carboxylate and tert-butyl (R)-3-(4-(6-((6-amino-2-(difluoromethyl)pyrimidin-4-yl)amino)-4-methoxy pyridin-3-yl)-1H-pyrazol-1-yl)pyrrolidine-1-carboxylate

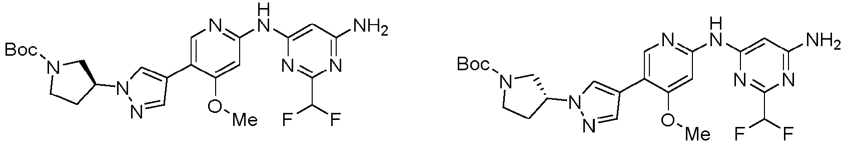

A mixture of tert-butyl 3-(4-(6-chloro-4-methoxypyridin-3-yl)-1H-pyrazol-1-yl)pyrrolidine-1-carboxylate (Preparation 55, 160 mg, 0.422 mmol), 2-(difluoromethyl) pyrimidine-4,6-diamine (Preparation 8, 67.5 mg, 0.422 mmol), BrettPhos Pd G4 (77.65 mg, 0.084 mmol) and Cs₂CO₃ (412 mg, 1.266 mmol) in dioxane (2 mL) was stirred at 100 °C for 16 h. The cooled mixture was purified by silica gel chromatography and Prep HPLC-3 to provide tert-butyl 3-(4-(6-((6-amino-2-(difluoromethyl)pyrimidin-4-yl)amino)-4-methoxypyridin-3-yl)-1H-pyrazol-1-yl)pyrrolidine-1-carboxylate as a white solid (100 mg, 47%). LCMS m/z = 503 [M+H]⁺

This was further purified by chiral SFC using an AD 20 x 250mm, 10µm (Daicel) column, mobile phase: CO₂ MeOH (0.2% MeOH/NH₃) = 60/40, at 80 g/min to give:
First eluting Enantiomer 1: tert-butyl (S)-3-(4-(6-((6-amino-2-(difluoromethyl)pyrimidin-4-yl)amino)-4-methoxypyridin-3-yl)-1H-pyrazol-1-yl)pyrrolidine-1-carboxylate or tert-butyl (R)-3-(4-(6-((6-amino-2-(difluoromethyl)pyrimidin-4-yl)amino)-4-methoxypyridin-3-yl)-1H-pyrazol-1-yl)pyrrolidine-1-carboxylate (Preparation 161) and
Second eluting Enantiomer 2: tert-butyl (R)-3-(4-(6-((6-amino-2-(difluoromethyl)pyrimidin-4-yl)amino)-4-methoxypyridin-3-yl)-1H-pyrazol-1-yl)pyrrolidine-1-carboxylate or tert-butyl (S)-3-(4-(6-((6-amino-2-(difluoromethyl)pyrimidin-4-yl)amino)-4-methoxypyridin-3-yl)-1H-pyrazol-1-yl)pyrrolidine-1-carboxylate (Preparation 162).

### Preparations 163 and 164: tert-butyl (R)-2-((4-(6-((6-amino-2-(difluoromethyl) pyrimidin-4-yl)amino)-4-methoxypyridin-3-yl)-1H-pyrazol-1-yl)methyl)azetidine-1-carboxylate and tert-butyl (S)-2-((4-(6-((6-amino-2-(difluoromethyl)pyrimidin-4-yl)amino)-4-methoxypyridin-3-yl)-1H-pyrazol-1-yl)methyl)azetidine-1-carboxylate

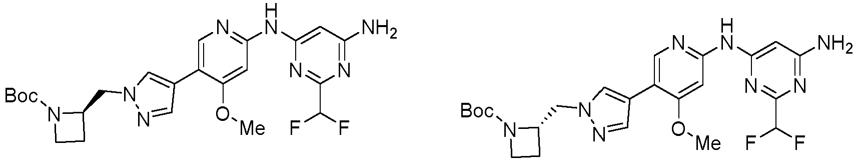

tert-Butyl 2-((4-(6-((6-amino-2-(difluoromethyl)pyrimidin-4-yl)amino)-4-methoxy pyridin-3-yl)-1H-pyrazol-1-yl)methyl)azetidine-1-carboxylate was obtained as a white solid, 80 mg, 40% from 2-(difluoromethyl)pyrimidine-4,6-diamine (Preparation 8) and tert-butyl 2-((4-(6-chloro-4-methoxypyridin-3-yl)-1H-pyrazol-1-yl)methyl)azetidine-1-carboxylate (Preparation 76), following the procedure described in Preparation 161 and 162. This was further purified by SFC using an AY 20 x 250mm, 10µm (Daicel) column, mobile phase: CO₂/EtOH (0.5% MeOH/NH₃) =65/35 Flow rate: 80 g/min to give:
First eluting Enantiomer 1: tert-butyl (R)-2-((4-(6-((6-amino-2-(difluoromethyl) pyrimidin-4-yl)amino)-4-methoxypyridin-3-yl)-1H-pyrazol-1-yl)methyl)azetidine-1-carboxylate or tert-butyl (S)-2-((4-(6-((6-amino-2-(difluoromethyl)pyrimidin-4-yl)amino)-4-methoxypyridin-3-yl)-1H-pyrazol-1-yl)methyl)azetidine-1-carboxylate (Preparation 163).
Second eluting Enantiomer 2: tert-butyl (S)-2-((4-(6-((6-amino-2-(difluoromethyl) pyrimidin-4-yl)amino)-4-methoxypyridin-3-yl)-1H-pyrazol-1-yl)methyl)azetidine-1-carboxylate or tert-butyl (R)-2-((4-(6-((6-amino-2-(difluoromethyl)pyrimidin-4-yl)amino)-4-methoxypyridin-3-yl)-1H-pyrazol-1-yl)methyl)azetidine-1-carboxylate (Preparation 164).

### Preparation 165: tert-butyl 3-(4-(6-chloro-4-fluoropyridin-3-yl)-1H-pyrazol-1-yl)azetidine-1-carboxylate

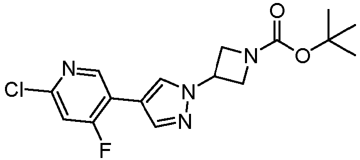

To tert-butyl 3-(4-(4,4,5,5-tetramethyl-1,3,2-dioxaborolan-2-yl)-1H-pyrazol-1-yl) azetidine-1-carboxylate (1.5 g, 7.13 mmol) and 5-bromo-2-chloro-4-fluoropyridine (4.32 g, 12.8 mmol) in dioxane (30 mL) was added Na₂CO₃ (3.78 g, 35.64 mmol), H₂O (5 mL) and Pd(PPh₃)₄ (4.12 g, 3.56 mmol) under N₂ and the mixture stirred at 100 °C for 16 h. The reaction was diluted with EtOAc (50 mL) and extracted with H₂O (3x 20 mL). The combined organics were washed with H₂O (3x 10 mL), dried (Na₂SO₄) and concentrated under reduced pressure. The residue was purified by column chromatography (ISCO^{®}; 0-25% EtOAc/PE) to afford the title compound as a yellow oil (1.6 g, 57%).

### Preparation 166: tert-butyl (2-(4-(6-chloro-4-fluoropyridin-3-yl)-1H-pyrazol-1-yl)ethyl)carbamate

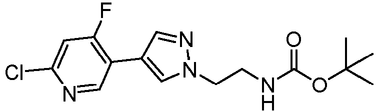

The title compound was prepared from tert-butyl (2-(4-(4,4,5,5-tetramethyl-1,3,2-dioxaborolan-2-yl)-1H-pyrazol-1-yl)ethyl)carbamate and 5-bromo-2-chloro-4-fluoropyridine using an analogous method to that described for Preparation 165. ¹H NMR (400 MHz, DMSO-d₆) δ: 8.83 (d, 1H), 8.23 (d, 1H), 8.05 (s, 1H), 7.69 (d, 1H), 7.02-6.89 (m, 1H), 4.30-4.11 (m, 2H), 3.39-3.34 (m, 2H), 1.34 (s, 9H)

### Preparation 167: tert-butyl (2-(4-(6-chloro-4-fluoropyridin-3-yl)-1H-pyrazol-1-yl)ethyl)(methyl)carbamate

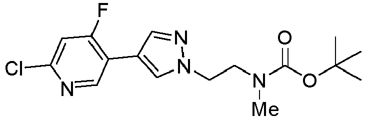

tert-Butyl (2-(4-(6-chloro-4-fluoropyridin-3-yl)-1H-pyrazol-1-yl)ethyl)carbamate (Preparation 166, 1.0 g, 2.93 mmol) was dissolved in THF (10.0 mL), then NaH (176.0 mg, 4.40 mmol, 60.0% purity) and MeI (625.0 mg, 4.40 mmol) were added at 0 °C under N₂ and the reaction stirred at 25 °C for 2 h. The reaction mixture was washed with H₂O (5 mL), dried over MgSO₄, filtered and concentrated under reduced pressure. The residue was purified by column chromatography on silica gel (ISCO^{®}; 0~20% EtOAc/PE) to give the title compound (0.86 g, 74.3% yield) as a white solid.

### Preparation 168: tert-butyl 3-(4-(6-((6-amino-2-(difluoromethyl)pyrimidin-4-yl)amino)-4-fluoropyridin-3-yl)-1H-pyrazol-1-yl)azetidine-1-carboxylate

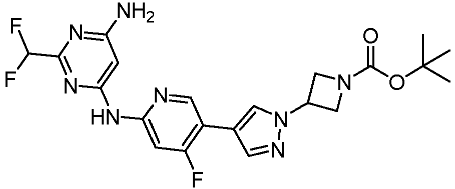

To a solution of tert-butyl 3-(4-(6-chloro-4-fluoropyridin-3-yl)-1H-pyrazol-1-yl)azetidine-1-carboxylate (Preparation 165, 1.6 g, 4.08 mmol) and 2-(difluoromethyl) pyrimidine-4,6-diamine (Preparation 8, 726 mg, 4.08 mmol) in t-AmOH (30 mL) was added BrettPhos Pd G3 (370 mg, 0.408 mmol) and Cs₂CO₃ (2.66 g, 8.16 mmol) under N₂ and the mixture stirred at 120 °C for 2 h. The reaction mixture was diluted with EtOAc (50 mL) and washed with H₂O 30 mL (10 mL x 3). The combined organics were dried (Na₂SO₄) and evaporated to dryness *in vacuo.* The residue was purified by silica gel chromatography ((ISCO^{®}; 0-75% EtOAc/PE) to afford the title compound as a yellow solid (800 mg, 41%).

### Preparation 169: tert-butyl (2-(4-(6-((6-amino-2-(difluoromethyl)pyrimidin-4-yl)amino)-4-fluoropyridin-3-yl)-1H-pyrazol-1-yl)ethyl)carbamate

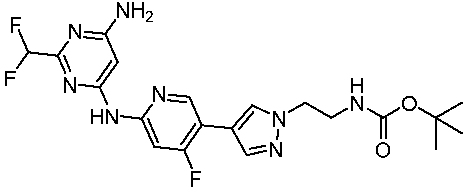

The title compound was prepared as a white solid (380 mg, 40%) from tert-butyl (2-(4-(6-chloro-4-fluoropyridin-3-yl)-1H-pyrazol-1-yl)ethyl)carbamate (Preparation 166) and 2-(difluoromethyl)pyrimidine-4,6-diamine (Preparation 8) using an analogous method to that described for Preparation 168. ¹H NMR (400 MHz, DMSO-d₆) δ: 10.09 (s, 1 H) 8.59 (d, 1H), 8.14-8.04 (m, 1H), 7.95 (s, 1H), 7.57-7.48 (m, 1H), 7.02-6.96 (m, 2H), 6.68-6.64 (m, 1H), 6.54 (s, 1H), 6.45 (s, 1H), 4.19 (br t, 2H), 3.41 (br d, 2H), 1.36 (s, 10H).

### Preparation 170: tert-butyl (2-(4-(6-((6-amino-2-(difluoromethyl)pyrimidin-4-yl)amino)-4-fluoropyridin-3-yl)-1H-pyrazol-1-yl)ethyl)(methyl)carbamate

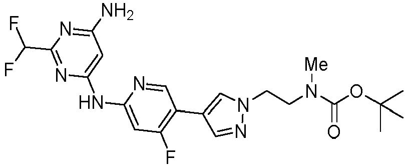

The title compound was prepared as a yellow solid (860 mg, 42.7%) from tert-butyl (2-(4-(6-chloro-4-fluoropyridin-3-yl)-1H-pyrazol-1-yl)ethyl)(methyl)carbamate (Preparation 167) and 2-(difluoromethyl)pyrimidine-4,6-diamine (Preparation 8) using an analogous method to that described for Preparation 168.
1H NMR (400 MHz, DMSO-d₆) δ ppm 10.09 (s, 1H) 8.58 (d, 1H) 8.10 (d, 1H) 7.95 (s, 1H) 7.52 (d, 1H) 6.99 (br s, 1H) 6.64 (s, 1H) 6.54 (s, 1H) 4.35-4.23 (m, 3H) 3.63-3.54 (m, 3H) 2.75-2.64 (m, 4H) 1.36 (s, 9H)

### Preparation 171: 2-(difluoromethyl)-N4-(4-fluoro-5-(1-methyl-1H-pyrazol-4-yl)pyridin-2-yl)pyrimidine-4,6-diamine

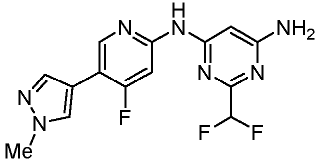

To a solution of 2-chloro-4-fluoro-5-(1-methyl-1H-pyrazol-4-yl)pyridine (Preparation 97, 1.9 g, 8.98 mmol) and 2-(difluoromethyl)pyrimidine-4,6-diamine (Preparation 8, 1.73 g, 10.77 mmol) in t-AmOH (40 mL) was added Cs₂CO₃ (8.78 g, 26.93 mmol) and BrettPhos Pd G3 (407 mg, 0.449 mmol,) under N₂ and the mixture stirred at 120 °C for 2 h. The reaction mixture was concentrated under reduced pressure to remove solvent. The residue was diluted with H₂O (30 mL) and extracted with EtOAc (3x 20 mL). The combined organics were washed with H₂O (3x 20 mL), dried (Na₂SO₄) and evaporated to dryness *in vacuo* to give the title compound as a brown solid (1.7 g, 54%). LCMS m/z = 336 [M+H]⁺.

### Preparation 172: (4,6-dichloropyrimidin-2-yl)methyl acetate

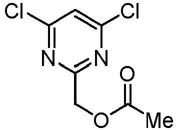

A mixture of 4,6-dichloro-2-(chloromethyl)pyrimidine (1.5 g, 7.59 mmol) and NaI (1.69 g, 11.3 mmol) in acetone (50 mL) was stirred at rt for 1 h. The mixture was concentrated *in vacuo,* the residue was diluted with dioxane/H₂O (1:1, 50 mL), KOAc (1.48 g, 15.1 mmol) added and the reaction was stirred at 60 °C for 16 h. The mixture was concentrated *in vacuo* and the residue purified by column chromatography (1:10 EtOAc/PE) to afford the title compound as a colourless oil (1.5 g, 89%). LCMS m/z = 221 [M+H]⁺.

### Preparation 173: (4,6-bis((diphenylmethylene)amino)pyrimidin-2-yl)methanol

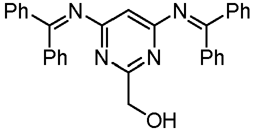

A mixture of (4,6-dichloropyrimidin-2-yl)methyl acetate (Preparation 172, 700 mg, 3.16 mmol), diphenylmethanimine (1.14 g, 6.32 mmol), Cs₂CO₃ (2.05 g, 6.32 mmol), Pd₂(dba)₃ (289 mg, 0.316 mmol) and Xantphos (182 mg, 0.316 mmol) in dioxane (20 mL) was stirred at 100 °C for 16 h. The mixture was concentrated *in vacuo* and the residue purified by column chromatography (1: 10 MeOH/DCM) to afford the title compound as a yellow solid (500 mg, 33%). LCMS m/z = 469 [M+H]⁺.

### Preparation 174: (4,6-diaminopyrimidin-2-yl)methanol

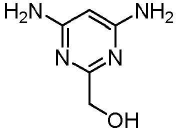

A mixture of (4,6-bis((diphenylmethylene)amino)pyrimidin-2-yl)methanol (Preparation 173, 500 mg, 1.06 mmol) in dioxane/HCl (10 mL) was stirred at rt for 4 h. The mixture was concentrated *in vacuo* and the residue treated with NH₃ in MeOH to adjust the pH to ~10. The resulting mixture was evaporated to dryness *in vacuo* and the residue purified by column chromatography (1:10 MeOH/DCM) to afford the title compound as a white solid (120 mg, 81%). LCMS m/z = 141 [M+H]⁺.

### Preparation 175: 6-chloro-4-fluoronicotinic acid

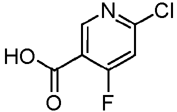

To a solution of 2-chloro-4-fluoro-5-iodopyridine (70.0 g, 272 mmol) in THF (700 mL) was added i-PrMgCl (2.00 M, 136 mL) at 0 °C under N₂. After 1 h at 0 °C the reaction mixture was stirred at 0 °C under CO₂ for 30 mins. The reaction mixture was poured into H₂O (1 L) and washed with EtOAc (2x 500 mL). Then combined aqueous phase was adjusted pH = 1 ~ 2 with 1N HCl (600 mL) and extracted with EtOAc (3x 400 mL). The combined organics were dried (Na₂SO₄) and concentrated under reduced pressure to afford the title compound as a yellow solid (25.5 g, 52 %). LCMS m/z = 176 [M+H]⁺.

### Preparation 176: 6-chloro-4-fluoro-N-methylnicotinamide

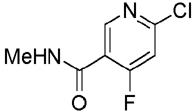

To a solution of 6-chloro-4-fluoronicotinic acid (Preparation 175, 11.48 g, 65.40 mmol) and methylamine hydrochloride (4.42 g, 65.40 mmol) in MeCN (120 mL) was slowly added T₃P (83.23 g, 130.79 mmol, 50% purity) and DIPEA (16.9 g, 130.79 mmol) and the mixture stirred at 25 °C for 16 h. The reaction mixture was diluted with H₂O (50 mL) and extracted with EtOAc (3x 100 mL). The combined organics were washed with brine (100 mL), dried (MgSO₄) and evaporated to dryness *in vacuo* to afford the title compound as a yellow solid (8.7 g, 71%) which was used without further purifcation. ¹H NMR (400 MHz, DMSO-*d*₆) δ: 8.63 (d, 1H) 8.54 (br s, 1H) 7.77 (d, 1H) 2.79 (d, 3H)

### Preparation 177: 4,6-dichloro-N-methylnicotinamide

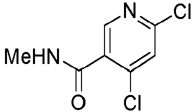

A drop of DMF was added to a mixture of 4,6-dichloronicotinic acid (10 g, 52 mmol) in SOCl₂ (50 mL) and the mixture stirred at 70 °C for 2 h. The reaction mixture was evaporated to dryness *in vacuo* and the residue diluted with THF (50 mL) and added to a solution of MeNH₂.HCl (4.21 g, 62.4 mmol) and DIPEA (20.1 g, 156 mmol) in THF (50 mL). The resulting mixture was stirred at rt for 16 h. The reaction was quenched with H₂O and extracted with EtOAc. The combined organics were washed with H₂O and brine, dried (Na₂SO₄) and evaporated to dryness *in vacuo.* The residue was purified by column chromatography (10:1 to 5:1 PE/EtOAc) to afford the title compound as a yellow solid (8 g, 75%). LCMS m/z = 205 [M+H]⁺.

### Preparation 178: 4,6-dichloro-N-cyclopropylnicotinamide

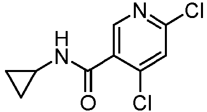

A mixture of 4,6-dichloronicotinic acid (1 g, 5.2 mmol), cyclopropanamine (297 mg, 5.2 mmol), HATU (3 g, 7.8 mmol) and TEA (1.6 g, 15.6 mmol) in DMF (20 mL) was stirred at rt for 12 h under N₂. The mixture concentrated *in vacuo* and the residue purified by column chromatography (50% EtOAc/PE) to afford the title compound (800 mg, 66%). LCMS m/z = 231 [M+H]⁺.

### Preparation 179: tert-butyl 6-(4,6-dichloronicotinamido)-2-azaspiro[3.3]heptane-2-carboxylate

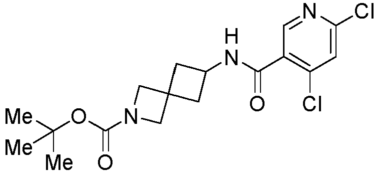

A solution of 4,6-dichloronicotinic acid (600 mg, 3.1 mmol), tert-butyl 6-amino-2-azaspiro[3.3]heptane-2-carboxylate (658 mg, 3.1 mmol), HATU (1.8 g, 4.7 mmol) and TEA (951 mg, 9.4 mmol) in DMF (10 mL) was stirred at rt for 12 h under N₂. The reaction mixture was evaporated to dryness *in vacuo* and the residue was purified by column chromatography (50% EtOAc/PE) to afford the title compound (900 mg, 74%). LCMS m/z = 386 [M+H]⁺.

### Preparation 180: 6-chloro-N-methyl-4-(methylamino)nicotinamide

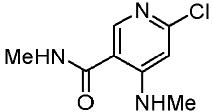

MeNH₂ in THF (20 mL, 2N) was added to a mixture of 4,6-dichloro-N-methyl nicotinamide (Preparation 177, 300 mg, 1.46 mmol) in DCM (20 mL) and the mixture stirred at rt for 2 h. The mixture was evaporated to dryness *in vacuo* and purified by column chromatography (1:5 EtOAc /PE) to give the title compound as a colourless oil (300 mg, 100%). LCMS m/z = 200 [M+H]⁺.

### Preparation 181: 6-chloro-4-(isopropylamino)-N-methylnicotinamide

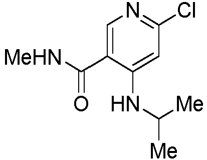

A mixture of 4,6-dichloro-N-methylnicotinamide (Preparation 177, 1.0 g, 4.88 mmol), isopropylamine (0.419 mL, 4.88 mmol), N,N-dimethylacetamide (1 mL) and DIPEA (0.937 mL, 10.76 mmol) was stirred at 70 °C overnight. The reaction was evaporated to dryness *in vacuo* and the residue was purified by silica gel chromatography to afford the title compound (450 mg, 41%). LCMS m/z = 228 [M+H]⁺.

### Preparation 182: 6-chloro-N-cyclopropyl-4-(methylamino)nicotinamide

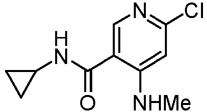

A solution of 4,6-dichloro-N-cyclopropylnicotinamide (Preparation 178, 800 mg, 3.5 mmol), methanamine (323 mg, 10.4 mmol) and DIPEA (1.3 g, 10.4 mmol) in MeCN (20 mL) was stirred at rt for 16 h under N₂. The reaction mixture was evaporated to dryness *in vacuo* and the residue purified by column chromatography (50% EtOAc/PE) to afford the title compound (500 mg, 64%). LCMS m/z = 226 [M+H]⁺.

### Preparation 183: tert-butyl 6-(6-chloro-4-(ethylamino)nicotinamido)-2-azaspiro [3.3]heptane-2-carboxylate

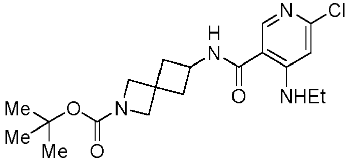

The title compound was prepared from tert-butyl 6-(4,6-dichloronicotinamido)-2-azaspiro[3.3]heptane-2-carboxylate (Preparation 179) and ethylamine using an analogous method to that described for Preparation 182. Yield: 600 mg, 65%; LCMS m/z = 395 [M+H]⁺.

### Preparation 184: 6-chloro-4-(ethylamino)-N-methylnicotinamide

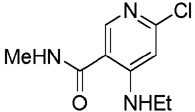

To a solution of 4,6-dichloro-N-methylnicotinamide (Preparation 177, 190 mg, 0.927 mol) and ethylamine hydrochloride (90.7 mg, 1.11 mmol) in n-BuOH (5 mL) was added DIPEA (359 mg, 2.78 mmol) and the mixture heated at 100 °C for 1 h. The reaction mixture was evaporated to dryness, diluted with H₂O and extracted with EtOAc (3x 10 mL). The combined organics were dried (Na₂SO₄) and evaporated to dryness *in vacuo* to afford the title compound as a yellow solid (150 mg). LCMS m/z = 214 [M+H]⁺.

### Preparation 185: 6-chloro-N-cyclopropyl-4-(ethylamino)nicotinamide

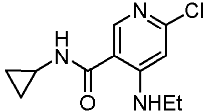

To a mixture of ethanamine hydrochloride (186 mg, 2.28 mmol) and 4,6-dichloro-N-cyclopropylnicotinamide (Preparation 178, 440 mg, 1.90 mmol) in n-BuOH (4 mL) was added DIPEA (738 mg, 5.71 mmol) and the resulting mixture stirred at 100 °C for 1 h. The reaction mixture was filtered and the filtrate was concentrated under reduced pressure to give a residue which was purified by prep-HPLC-6, to give the title compound as a gray gum (100 mg, 22%). LCMS m/z = 240 [M+H]⁺.

### Preparation 186: (S)-6-chloro-4-((1-fluoropropan-2-yl)amino)-N-methyl nicotinamide

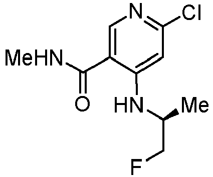

A mixture of 4,6-dichloro-N-methylnicotinamide (Preparation 177, 200 mg, 0.975 mmol), (2S)-1-fluoropropan-2-amine hydrochloride (110 mg, 0.975 mmol) and DIPEA (376 mg, 2.92 mmol) in dioxane (5 mL) was stirred in a sealed tube at 140 °C for 16 h. The reaction was quenched with H₂O and extracted into EtOAc. The combined organics were washed (H₂O and brine), dried (Na₂SO₄) and evaporated to dryness *in vacuo.* The residue was purified by column chromatography on silica gel (10:1 to 1:1 PE/EtOAc) to afford the title compound as a white solid (210 mg, 82%). LCMS m/z = 246 [M+H]⁺.

### Preparation 187: 6-chloro-4-((1,3-difluoropropan-2-yl)amino)-N-methyl nicotinamide

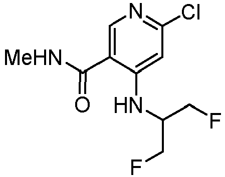

The title compound was prepared from 4,6-dichloro-N-methylnicotinamide (Preparation 177) and 1,3-difluoropropan-2-amine using an analogous method to that described for Preparation 186. The compound was additionally purified by Prep HPLC-3 to afford the title compound as a white solid (60 mg, 18%). LCMS m/z = 264 [M+H]⁺.

### Preparation 188: (R)-6-chloro-4-((1,1-difluoropropan-2-yl)amino)-N-methyl nicotinamide

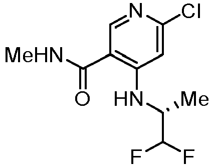

A mixture of (R)-1,1-difluoropropan-2-amine (120 mg, 1.26 mmol), 4,6-dichloro-N-methylnicotinamide (Preparation 177, 258 mg, 1.26 mmol) and DIPEA (489 mg, 3.78 mmol) in dioxane (5 mL) was stirred at 140 °C for 16 h. The reaction mixture was evaporated to dryness *in vacuo* and the residue purified by Prep HPLC-3 to afford the title compound as a white solid (80 mg, 24%). LCMS m/z = 264 [M+H]⁺.

### Preparation 189 to 198

The title compounds were prepared from the appropriate chloropyridine (RCl) and amine (RNH₂) using an analogous method to that described for Preparation 188.

| **Prep No** | **Name/Structure/RNH₂/Purification/Data** |
|---|---|
| **RCl: 4,6-dichloro-N-methylnicotinamide (Preparation 177)** | |
| **189** | (S)-6-chloro-4-((1,1-difluoropropan-2-yl)amino)-N-methylnicotinamide |
| | 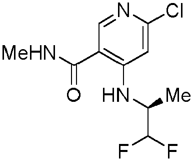 |
| | RNH₂: (S)-1,1-difluoropropan-2-amine |
| | Prep HPLC-3. Yield: 100 mg, 41%; LCMS m/z = 264 [M+H]⁺. |
| **190** | - |
| | 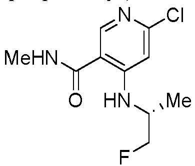 |
| | RNH₂: (2R)-1-fluoropropan-2-amine hydrochloride |
| | SiO₂, 33% EtOAc/PE; Yield: 200 mg, 56%; LCMS m/z = 246 [M+H]⁺. |
| **191** | (R)-6-chloro-N-methyl-4-((tetrahydrofuran-3-yl)amino)nicotinamide |
| | 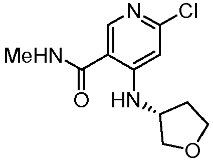 |
| | RNH₂: (R)-tetrahydrofuran-3-amine |
| | SiO₂, 33% EtOAc/PE; Yield: 43 mg, 70%; LCMS m/z = 256 [M+H]⁺. |
| **192** | (S)-6-chloro-N-methyl-4-((tetrahydrofuran-3-yl)amino)nicotinamide |
| | 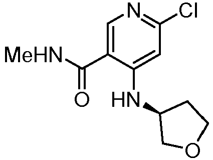 |
| | RNH₂: (S)-tetrahydrofuran-3-amine |
| | SiO2, 33% EtOAc/PE; Yield: 46 mg, 74%; LCMS m/z = 256 [M+H]⁺. |
| **193** | 6-chloro-4-(cyclobutylamino)-N-methylnicotinamide |
| | 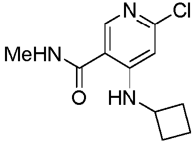 |
| | RNH₂: cyclobutylamine |
| | SiO2, 33% EtOAc/PE; Yield: 55 mg, 95%; LCMS m/z = 240 [M+H]⁺. |
| **194** | 6-chloro-N-methyl-4-(oxetan-3 -ylamino)nicotinamide |
| | 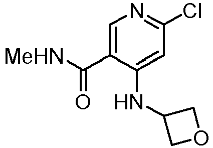 |
| | RNH₂: oxetane-3-amine |
| | SiO₂, 33% EtOAc/PE; Yield: 46 mg, 74%; LCMS m/z = 242 [M+H]⁺. |

| **RCl: 4,6-dichloro-N-cyclopropylnicotinamide (Preparation 178)** | |
|---|---|
| **195** | (S)-6-chloro-N-cyclopropyl-4-((1-fluoropropan-2-yl)amino)nicotinamide |
| | 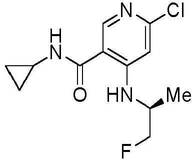 |
| | RNH₂: (2S)-1-fluoropropan-2-amine hydrochloride |
| | SiO2, 33% EtOAc/PE; Yield: 150 mg, 64%; LCMS m/z = 272 [M+H]⁺. |
| **196** | (R)-6-chloro-N-cyclopropyl-4-((1-fluoropropan-2-yl)amino)nicotinamide |
| | 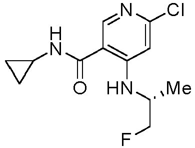 |
| | RNH₂: (2R)-1-fluoropropan-2-amine hydrochloride |
| | SiO2, 33% EtOAc/PE; Yield: 200 mg, 57%; LCMS m/z = 272 [M+H]⁺. |
| **197** | 6-chloro-N-methyl-4-((methyl-d3)amino)nicotinamide |
| | 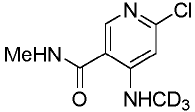 |
| | RNH₂: methan-d₃-amine |
| | SiO₂, 50% EtOAc/PE; Yield: 120 mg, 59%; LCMS m/z = 203 [M+H]⁺. |
| **198** | 6-chloro-4-(cyclopropylamino)-N-methylnicotinamide |
| | 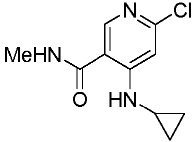 |
| | RNH₂: cyclopropylamine |
| | SiO2, 50% EtOAc/PE; Yield: 200 mg, 90%; LCMS m/z = 226 [M+H]⁺. |

### Preparation 199: (S)-6-chloro-4-((1-fluoropropan-2-yl)amino)-N-(pyridin-2-yl methyl)nicotinamide

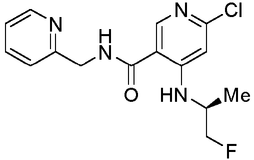

**Part 1:** A mixture of 4,6-dichloropyridine-3-carboxylic acid (500 mg, 2.60 mmol) and oxalyl chloride (659 mg, 5.20 mmol) in THF (10 mL) was stirred at rt for 2 h. The mixture was concentrated *in vacuo* and the residue and dissolved in THF (10 mL) and DIPEA (1006 mg, 7.80 mmol) and 1-(pyridin-2-yl)methanamine (421 mg, 3.90 mmol) added and the resulting mixture stirred at rt for 2 h. The reaction mixture was evaporated to dryness *in vacuo* and the residue purified by column chromatography (SiO₂, 25% EtOAc/PE) to afford 4,6-dichloro-N-(pyridin-2-ylmethyl)nicotinamide as a white solid (470 mg, 63%).

**Part 2:** A mixture of 4,6-dichloro-N-(pyridin-2-ylmethyl)nicotinamide (200 mg, 0.708 mmol), (2S)-1-fluoropropan-2-amine hydrochloride (96.4 mg, 0.849 mmol) and DIPEA (273 mg, 2.12 mmol) in dioxane (4 mL) and stirred at 140 °C for 16 h. The cooled mixture was concentrated *in vacuo.* The residue was purified by column chromatography on silica gel eluting with EtOAc/PE (1/3) to give the title compound (180 mg, 79% yield) as a white solid. LCMS mz = 323 [M+H]⁺.

### Preparation 200: 6-chloro-4-(ethylamino)-N-(oxazol-4-ylmethyl)nicotinamide

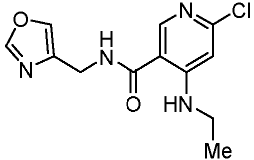

4,6-Dichloro-N-(oxazol-4-ylmethyl)nicotinamide was prepared as a yellow solid, 300 mg, 67%, from 1-(1,3-oxazol-4-yl)methanamine hydrochloride and 4,6-dichloropyridine-3-carboxylic acid using s similar method to that described for Preparation 199, Part 1.

A mixture of 4,6-dichloro-N-(oxazol-4-ylmethyl)nicotinamide (300 mg, 1.10 mmol), ethanamine (74.3 mg, 1.65 mmol), and DIPEA (650 mg, 3.3 mmol) in MeCN (10 mL) was stirred at rt for 16 h. The mixture was purified by column chromatography on silica gel eluting with EtOAc/PE (1/3) to give the title compound as a white solid (200 mg, 65%). LCMS m/z = 281 [M+H]⁺.

### Preparation 201: 6-chloro-4-(isopropylamino)nicotinic acid

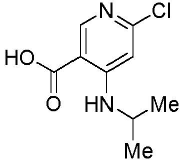

A mixture of 6-chloro-4-fluoronicotinic acid (Preparation 175, 1 g, 5.70 mmol), propan-2-amine (840 mg, 14.2 mmol) and DIPEA (4 mL) in EtOH (20 mL) was stirred at 80 °C for 16 h. The mixture was concentrated and purified by column chromatography on silica gel (10:1 DCM/MeOH) to give the title compound (980 mg, 80%). LCMS m/z = 215 [M+H]⁺.

### Preparation 202: 6-chloro-4-(ethylamino)nicotinic acid

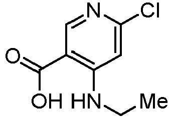

A mixture of methyl 6-chloro-4-(ethylamino)nicotinate (600 mg, 2.79 mmol) and NaOH (6 mL, 2M in water) in EtOH (6 mL) was stirred at 80 °C for 1 h. The mixture was cooled to 0 °C, the pH adjusted to 4-5 with HCl (2M) and the solid filtered off to give the title compound (500 mg, yield: 89%). LCMS m/z = 201 [M+H]⁺.

### Preparation 203: 6-chloro-4-(cyclopropylamino)nicotinic acid

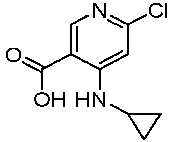

A solution of methyl 4,6-dichloronicotinate (3.0 g, 14.5 mmol), cyclopropanamine (1.65 g, 29.0 mmol) and DIPEA (5.61 g, 43.5 mmol) in dioxane (30 mL) was stirred at rt for 16 h under N₂. The mixture was evaporated to dryness *in vacuo* and the residue purified by column chromatography on silica gel (50% EtOAc/PE ) to afford methyl 6-chloro-4-(cyclopropylamino) nicotinate as a white solid (1.2 g, 36.5%).

A solution of methyl 6-chloro-4-(cyclopropylamino)nicotinate (1.2 g,5.29 mmol) and LiOH (378 mg, 15.8 mmol) in MeOH (6 mL), H₂O (6 mL) and THF (6 mL) was stirred at rt for 16 h under N₂. The reaction mixture was evaporated to dryness *in vacuo* and the residue purified by column chromatography on silica gel (50% EtOAc/PE) to afford the title compound as a white solid (1.1g, 98%). LCMS m/z = 213 [M+H]⁺.

### Preparation 204: 6-chloro-N-cyclopropyl-4-(cyclopropylamino)nicotinamide

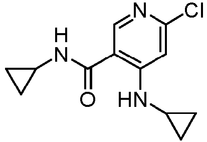

HATU (535 mg, 1.41 mmol) and TEA (213 mg, 2.11 mmol) were added to a mixture of 6-chloro-4-(cyclopropylamino)nicotinic acid (Preparation 203, 150 mg, 0.705 mmol) and cyclopropanamine (59.9 mg, 1.05 mmol) in DMF (8 mL) and the mixture stirred at rt for 16 h under N₂. The reaction mixture was diluted with EtOAc and washed with water. The organic layer was concentrated *in vacuo* and the residue purified by column chromatography on silica gel (1:1 EtOAc/PE) to afford the title compound as a white solid (110mg, 62%). LCMS m/z = 252 [M+H]⁺.

### Preparation 205 to 220

The title compounds were prepared from the appropriate carboxylic acid and amine using an analogous method to that described for Preparation 204.

| **Prep No** | **Name/Structure/Amine/Yield/Data** |
|---|---|
| **Using 6-chloro-4-(cyclopropylamino)nicotinic acid (Preparation 203)** | |
| **205** | 6-chloro-4-(cyclopropylamino)-N-ethylnicotinamide |
| | 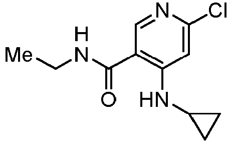 |
| | Amine: ethanamine |
| | white solid (130 mg, 77%). LCMS m/z = 240 [M+H]⁺. |
| **206** | 6-chloro-4-(cyclopropylamino)-N-propylnicotinamide |
| | 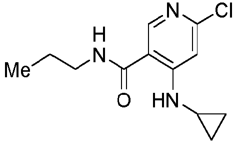 |
| | Amine: propylamine. White solid (120 mg, 67%); LCMS m/z = 254 [M+H]⁺. |
| **207** | 6-chloro-4-(cyclopropylamino)-N-(cyclopropylmethyl)nicotinamide |
| | 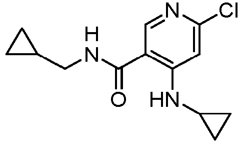 |
| | Amine: cyclopropylmethylamine. |
| | White solid (120 mg, 67%); LCMS m/z = 266 [M+H]⁺. |
| **208** | 6-chloro-4-(cyclopropylamino)-N-(methyl-d3)nicotinamide |
| | 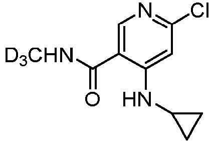 |
| | Amine: methan-d3-amine hydrochloride. |
| | White solid (323 mg, 100%); ¹H NMR (500 MHz, DMSO-d₆) δ: 8.53 (d, 2H), 8.33 (s, 1H), 6.92 (s, 1H), 2.54 (dd, 1H), 0.85 - 0.80 (m, 2H), 0.50 (dd, 2H). |
| **209** | 6-chloro-4-(cyclopropylamino)-N-isopropylnicotinamide |
| | 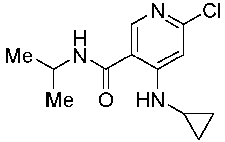 |
| | Amine: isopropylamine. |
| | White solid (320 mg, 83%); ¹H NMR (500 MHz, DMSO-d₆) δ: 8.50 (s, 1H), 8.37 (d, 1H), 8.34 (d, 1H), 6.91 (d, 1H), 4.04 (h, 1H), 1.14 (dd, 6H), 0.91-0.75 (m, 2H), 0.51 (dt, 2H). |

| **Acid: 6-chloro-4-(methylamino)nicotinic acid (**US 2020017510 A1**)** | |
|---|---|
| **210** | 6-chloro-N-(methyl-d3)-4-(methylamino)nicotinamide |
| | 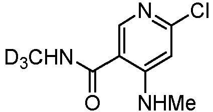 |
| | Amine: methan-d3-amine hydrochloride. |
| | White solid (302 mg, 93%); ¹H NMR (500 MHz, DMSO-d₆) δ: 8.53 (s, 1H), 8.36 (d, 1H), 8.33 (d, 1H), 6.67 (s, 1H), 2.87 (d, 3H). |
| **211** | 6-chloro-N-isopropyl-4-(methylamino)nicotinamide |
| | 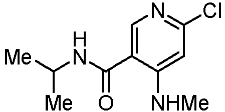 |
| | Amine: isopropylamine. |
| | White solid (366 mg, 71%); ¹H NMR (500 MHz, DMSO-d₆) δ: 8.29 (d, 2H), 8.23 (d, 1H), 6.60 (d, 1H), 4.06 (h, 1H), 2.81 (dd, 3H), 1.15 (dd, 6H). |
| **212** | 6-chloro-N-(cyanomethyl)-4-(methylamino)nicotinamide |
| | 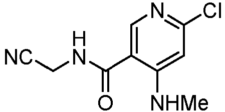 |
| | Amine: cyanomethylamine hydrochloride. |
| | White solid (321 mg, 96%); ¹H NMR (500 MHz, DMSO-d₆) δ: 9.22 (d, 1H), 8.34 (d, 1H), 8.27 (s, 1H), 6.68 (d, 1H), 4.28 (d, 2H), 2.85 (d, 3H). |
| **213**^{A, B} | 6-chloro-N-(3-(hydroxymethyl)bicyclo[1.1.1]pentan-1-yl)-4-(methylamino)nicotinamide |
| | 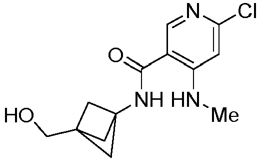 |
| | Amine: (3-aminobicyclo[1.1.1]pentan-1-yl)methanol |
| | 200 mg, 82% yield, LCMS m/z = 282 [M+H]⁺ |
| **214**^{**A,** B} | 6-chloro-4-(ethylamino)-N-(3-(hydroxymethyl)bicyclo[1.1.1]pentan-1-yl)nicotinamide |
| | 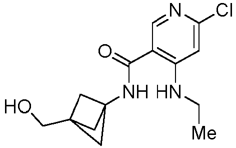 |
| | SM: 6-chloro-4-(ethylamino)nicotinic acid (Preparation 202) and (3-aminobicyclo[1.1.1]pentan-1-yl)methanol |
| | 400 mg, 90%. LCMS m/z = 296 [M+H]⁺ |

| **Acid: 6-chloro-4-(isopropylamino)nicotinic acid (Preparation 201)** | |
|---|---|
| **215** | 6-chloro-N-(cyclopropylmethyl)-4-(isopropylamino)nicotinamide |
| | 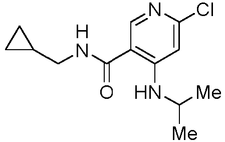 |
| | Amine: cyclopropylmethylamine. Yellow oil, 160 mg, 64% |
| **216** | 6-chloro-4-(isopropylamino)-N-propylnicotinamide |
| | 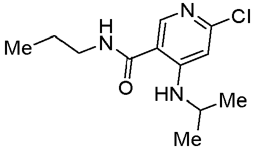 |
| | Amine: propan-1-amine. |
| | Yellow oil, 150 mg, 63%. LCMS m/z = 256 [M+H]⁺ |
| **217** | 6-chloro-N-cyclopropyl-4-(isopropylamino)nicotinamide |
| | 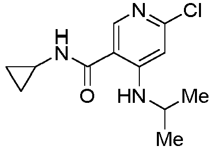 |
| | Amine: cyclopropylamine |
| | White solid, 170 mg, 72% yield. ¹H NMR (400 MHz, CDCl₃): δ ppm 8.41-8.30 (m, 1H), 8.09 (s, 1H), 6.52 (s, 1H), 6.24 (s, 1H), 3.69-3.58 (m, 1H), 2.85-2.76 (m, 1H), 1.28 (d, 6H), 0.91-0.84 (m, 2H), 0.67-0.62 (m, 2H) |
| **218** | 6-chloro-4-(isopropylamino)-N-(pyridin-2-ylmethyl)nicotinamide |
| | 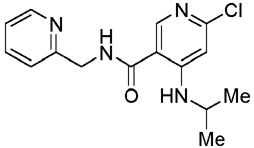 |
| | Amine: 1-(pyridin-2-yl)methanamine. |
| | Yellow solid, 160 mg, 57%. LCMS m/z 305 [M+H]⁺ |
| **219** | 6-chloro-4-(isopropylamino)-N-(oxazol-2-ylmethyl)nicotinamide |
| | 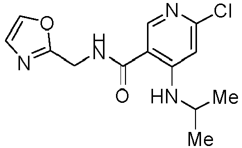 |
| | Amine: oxazol-2-ylmethanamine. |
| | Yellow solid, 150 mg, 54% yield. LCMS m/z = 295 [M+H]⁺ |
| **220** | 6-chloro-4-(isopropylamino)-N-(oxazol-4-ylmethyl)nicotinamide |
| | 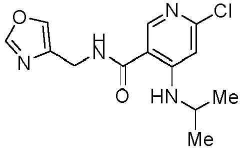 |
| | Amine: oxazol-4-ylmethanamine |
| | 500 mg, 72%. LCMS m/z = 295 [M+H]⁺. |

| | |
|---|---|
| ^{A} DCM was the reaction solvent ^{B} DIPEA was used instead of TEA | |

### Preparation 222: methyl (S)-6-chloro-4-((1-fluoropropan-2-yl)amino)nicotinate

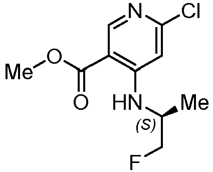

A mixture of methyl 4,6-dichloropyridine-3-carboxylate (300 mg, 1.45 mmol), (2S)-1-fluoropropan-2-amine hydrochloride (246 mg, 2.17 mmol) and DIPEA (561 mg, 4.35 mmol) in dioxane (5 mL) was stirred at 140 °C for 16 h. The cooled reaction was quenched with water and extracted with EtOAc. The combined organic layer was washed with water and brine, dried over Na₂SO₄, filtered and concentrated. The residue was purified by column chromatography on silica gel eluting with EtOAc/PE (1/2) to get the title compound (200 mg, 53% yield) as a white solid. LCMS m/z = 247 [M+H]⁺.

### Preparation 223: (S)-6-chloro-4-((1-fluoropropan-2-yl)amino)nicotinic acid

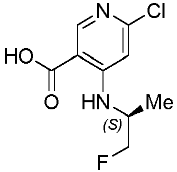

A mixture of methyl (S)-6-chloro-4-((1-fluoropropan-2-yl)amino)nicotinate (Preparation 222, 200 mg, 0.81 mmol), LiOH (57.9 mg, 2.42 mmol) and H₂O (1 mL) in THF (4 mL) was stirred at rt for 16 h. HCl (1N) was added to adjust the pH = 3, the mixture was diluted with water and extracted with EtOAc. The combined organic layer was dried over Na₂SO₄, filtered and concentrated to give the title compound (200 mg, 100% yield) as a white solid. LCMS m/z = 233 [M+H]⁺.

### Preparation 224: (S)-6-chloro-4-((1-fluoropropan-2-yl)amino)-N-(3-(hydroxy methyl)bicyclo[1.1.1]pentan-1-yl)nicotinamide

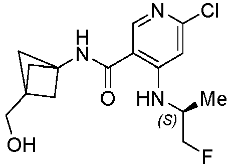

A mixture of (S)-6-chloro-4-((1-fluoropropan-2-yl)amino)nicotinic acid (Preparation 223, 150 mg, 0.644 mmol), DIPEA (248 mg, 1.93 mmol) and HATU (293 mg, 0.772 mmol) in DCM (10 mL) was stirred at rt for 5 min. (3-Aminobicyclo[1.1.1]pentan-1-yl)methanol (87.3 mg, 0.772 mmol) was added and the reaction stirred at rt for 4 h. The mixture was concentrated *in vacuo* and the residue was purified by column chromatography on silica gel eluting with EtOAc/PE (1/1) to give the title compound (150 mg, 71% yield) as a white solid. LCMS m/z = 328 [M+H]⁺.

### Preparation 225: 5-bromo-2-chloro-N-(oxetan-3-yl)pyridin-4-amine

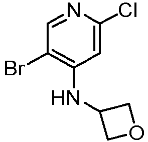

To a mixture of 5-bromo-2,4-dichloropyridine and oxetan-3-amine (580 mg, 7.93 mmol) in DMF (5 mL) was added TEA (2.01 g, 19.83 mmol) and the reaction was heated at 70 °C for 12 h. The mixture was diluted with H₂O (30 mL) and extracted with EtOAc (3x 50 mL). The combined organics were dried (Na₂SO₄) and evaporated to dryness *in vacuo.* The residue was purified by column chromatography on silica gel (1:20 to 1:1 EtOAc/PE) to afford the title compound as a yellow solid (800 mg, 46%). LCMS m/z = 263 [M+H]⁺.

### Preparation 226: 5-bromo-2-chloro-N-methylpyridin-4-amine

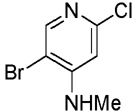

The title compound was prepared from 5-bromo-2,4-dichloropyridine and methylamine using an analogous method to that described for Preparation 225. LCMS m/z = 221 [M+H]⁺

### Preparation 227: 2-chloro-5-(5-methyl-1,3,4-thiadiazol-2-yl)-N-(oxetan-3-yl)pyridin-4-amine

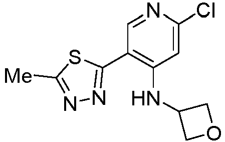

**Part 1.** To a solution of bis(pinacolato)diboron (925 mg, 3.64 mmol) and 5-bromo-2-chloro-N-(oxetan-3-yl)pyridin-4-amine (Preparation 225, 800 mg, 3.04 mmol) in dioxane (5 mL) was added Pd(dppf)Cl₂.DCM (248 mg, 0.304 mmol) and KOAc (596 mg, 6.07 mmol) and the reaction was heated at 90 °C for 2 h under N₂. The reaction mixture was evaporated to dryness *in vacuo* to afford 2-chloro-N-(oxetan-3-yl)-5-(4,4,5,5-tetramethyl-1,3,2-dioxaborolan-2-yl)pyridin-4-amine as a yellow oil (crude).

**Part 2.** To a solution of 2-chloro-N-(oxetan-3-yl)-5-(4,4,5,5-tetramethyl-1,3,2-dioxaborolan-2-yl)pyridin-4-amine (Part 1) and 2-bromo-5-methyl-1,3,4-thiadiazole (470 mg, 2.63 mmol) in dioxane (5 mL) and H₂O (0.20 mL) was added XPhos Pd G2 (207 mg, 0.263 mmol) and K₃PO₄ (1.12 g, 5.25 mmol) and the resulting mixture heated at 80 °C for 2 h under N₂. The reaction mixture was evaporated to dryness and the residue purified by prep-HPLC-9, to give the title compound as a yellow oil (60 mg, 8%). LCMS m/z = 283 [M+H]⁺

### Preparation 228: 2-chloro-N-methyl-5-(5-methyl-1,3,4-thiadiazol-2-yl)pyridin-4-amine

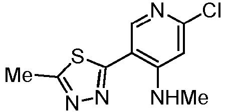

The title compound was prepared from 5-bromo-2-chloro-N-methylpyridin-4-amine and 2-bromo-5-methyl-1,3,4-thiadiazole using an analogous method to that described for Preparation 227, except Prep HPLC-3 was used. LCMS m/z = 241 [M+H]⁺

### Preparation 229: 2,4-dichloro-5-((trimethylsilyl)ethynyl)pyridine

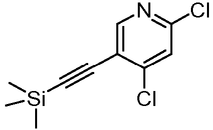

A mixture of 2,4-dichloro-5-iodopyridine (1 g, 3.65 mmol), ethynyltrimethylsilane (358 mg, 3.65 mmol), Pd(PPh₃)Cl₂ (512 mg, 0.73 mmol), CuI (139 mg, 0.73 mmol) and TEA (1.1 g, 10.95 mmol) in THF (5 mL) was stirred at 40 °C for 16 h. The mixture was concentrated *in vacuo* and the residue purified by column chromatography on silica gel (50% EtOAc/PE) to afford the title compound as a yellow solid (800 mg, 90%). LCMS m/z = 244 [M+H]⁺

### Preparation 230: 2,4-dichloro-5-ethynylpyridine

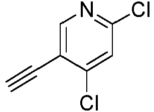

TBAF (2 mL) was added to a solution of 2,4-dichloro-5-((trimethylsilyl)ethynyl)pyridine (Preparation 229, 800 mg, 3.27 mmol) in THF and the mixture stirred at rt for 1 h. The solution was diluted with EtOAc (100 mL) and washed with water and brine. The combined organic were evaporated to dryness and the residue purified by column chromatography on silica gel (50% EtOAc/PE) to afford the title compound as a yellow solid (380 mg, 68%). LCMS m/z = 172 [M+H]⁺

### Preparation 231: 2,4-dichloro-5-(1H-1,2,3-triazol-4-yl)pyridine

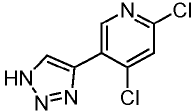

To a solution of 2,4-dichloro-5-ethynylpyridine (Preparation 230, 380 mg, 2.2 mmol) in DMF (4 mL) and MeOH (1.5 mL) was added CuI (83.8 mg, 0.44 mmol) and TMSN₃ (380 mg, 3.3 mmol) and the mixture stirred at 40 °C for 16 h. The reaction was quenched with NH₄OH and extracted with EtOAc. The combined organics were concentrated and purified by column chromatography on silica gel (50% EtOAc/PE) to give the title compound as a yellow solid (200 mg, 42%). LCMS m/z = 215 [M+H]⁺

### Preparation 232 and 233: 2,4-dichloro-5-(1-methyl-1H-1,2,3-triazol-4-yl)pyridine and 2,4-dichloro-5-(2-methyl-2H-1,2,3-triazol-4-yl)pyridine

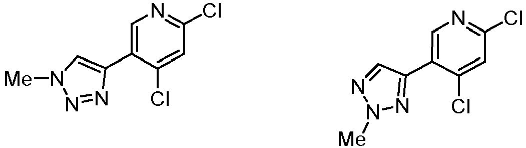

To a solution of 2,4-dichloro-5-(1H-1,2,3-triazol-4-yl)pyridine (Preparation 231, 100 mg, 0.47 mmol) in THF (5 mL) was added TBAF (243 mg, 0.93 mmol) and MeI (792 mg, 0.56 mmol) at 0 °C and the mixture stirred at rt for 1 h. The reaction mixture was diluted with EtOAc (100 mL) and washed with brine and water. The combined organics were concentrated and purified by column chromatography on silica gel (50% EtOAc/PE) to afford 2,4-dichloro-5-(1-methyl-1H-1,2,3-triazol-4-yl)pyridine as a yellow solid (70 mg, 45%). LCMS m/z = 229 [M+H]⁺ and 2,4-dichloro-5-(2-methyl-2H-1,2,3-triazol-4-yl)pyridine as a yellow solid (40 mg, 28%). LCMS m/z = 229 [M+H]⁺

### Preparation 234: 2-chloro-N-methyl-5-(1-methyl-1H-1,2,3-triazol-4-yl)pyridin-4-amine

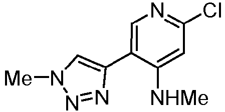

A mixture of 2,4-dichloro-5-(1-methyl-1H-1,2,3-triazol-4-yl)pyridine (Preparation 232, 70 mg, 0.31 mmol), methanamine (28 mg, 0.92 mmol) and DIPEA (118 mg, 0.915 mmol) in MeCN (2 mL) was stirred at 120 °C for 16 h. The reaction mixture was concentrated and purified by column chromatography on silica gel (5% MeOH/DCM) to afford the title compound as a white solid (50 mg, 73%). LCMS m/z = 224 [M+H]⁺

### Preparation 235: 2-chloro-N-methyl-5-(2-methyl-2H-1,2,3-triazol-4-yl)pyridin-4-amine

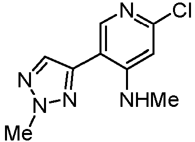

The title compound was obtained as a white solid, 30 mg, 77% yield, from 2,4-dichloro-5-(2-methyl-2H-1,2,3-triazol-4-yl)pyridine (Preparation 233), following the procedure described in Preparation 234. LCMS m/z = 224 [M+H]⁺

### Preparation 236: 2-chloro-5-iodo-N-methylpyridin-4-amine

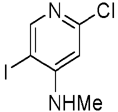

Methanamine (34 mg, 1.1 mmol) was added to a solution of 2,4-dichloro-5-iodopyridine (300 mg, 1.1 mmol) and DIPEA (426 mg, 3.3 mmol) in MeCN (5 mL) and the resulting mixture stirred at rt for 16 h under N₂. The mixture was concentrated *in vacuo* and the residue purified by column chromatography on silica gel (50% EtOAc/PE) to afford the title compound (240 mg, 81%). LCMS m/z = 269 [M+H]⁺

### Preparation 237: 2-chloro-N-methyl-5-(5-methylthiazol-2-yl)pyridin-4-amine

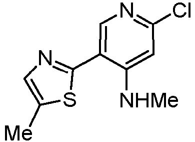

A solution of 2-chloro-5-iodo-N-methylpyridin-4-amine (Preparation 236, 120 mg, 0.45 mmol), 5-methyl-2-(tributylstannyl)thiazole (173.5 mg, 0.447 mmol), Pd(PPh₃)Cl₂ (470.3 mg, 0.67 mmol) and K₂CO₃ (180 mg, 1.3 mmol) in DMF (4 mL) was stirred at 100 °C for 16 h under N₂. The reaction mixture was evaporated to dryness *in vacuo* and the residue purified by column chromatography on silica gel (5% MeOH/DCM) to afford the title compound (100 mg, 93%). LCMS m/z = 240 [M+H]⁺

### Preparation 238: 2-(6-chloro-4-fluoropyridin-3-yl)-5-methyl-1,3,4-thiadiazole

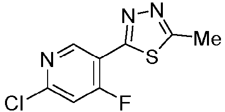

Under an inert atmosphere of N₂ were combined 2-bromo-5-methyl-1,3,4-thiadiazole (160 g, 894 mmol), dioxane (2.4 L), DMF (0.48 L), 2-chloro-4-fluoro-5-(tetramethyl-1,3,2-dioxaborolan-2-yl)pyridine (340 g, 1.32 mol), Na₂CO₃ (285.8 g, 2.70 mol), Pd(dppf)Cl₂.DCM (36.6 g, 44.91 mmol) and CuCl (89 g, 899 mmol) and the resulting mixture stirred for 4 h at 80 °C. The reaction mixture was cooled to rt and diluted with EtOAc (2 L). The solids were removed by filtration and the filtrate was concentrated *in vacuo.* The residue was purified by silica gel chromatography on silica gel (5-50% EtOAc/PE) and the resulting solid recrystallised from toluene to afford the title compound as a yellow solid (45g, 22%).

### Preparation 239: 2-(bromomethyl)-5-(6-chloro-4-fluoropyridin-3-yl)-1,3,4-thiadiazole

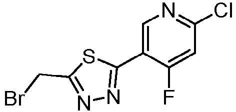

A mixture of 2-(6-chloro-4-fluoropyridin-3-yl)-5-methyl-1,3,4-thiadiazole (Preparation 238, 200 mg, 0.87 mmol), NBS (195 mg, 0.87 mmol) and AIBN (14 mg, 0.08 mmol) in CCl₄ (10 mL) was stirred at 90°C for 16 h. The mixture was evaporated *in vacuo* and the residue purified by column chromatography on silica gel (10:1 PE/EtOAc) to give the title compound (50 mg, 18%). LCMS m/z = 308 [M+H]⁺

### Preparation 240: (5-(6-chloro-4-fluoropyridin-3-yl)-1,3,4-thiadiazol-2-yl)methyl acetate

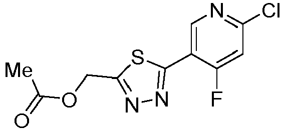

A mixture of 2-(bromomethyl)-5-(6-chloro-4-fluoropyridin-3-yl)-1,3,4-thiadiazole (Preparation 239, 50 mg, 0.16 mmol) and KOAc (32 mg, 0.32 mmol) in dioxane (5 mL) was stirred at 110 °C for 16 h. The mixture was concentrated *in vacuo* and the residue purified by column chromatography on silica gel (10:1 PE/EtOAc) to give title compound (20 mg, 43%). LCMS m/z = 288 [M+H]⁺

### Preparation 241: (5-(6-chloro-4-(methylamino)pyridin-3-yl)-1,3,4-thiadiazol-2-yl)methanol

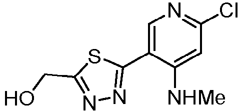

A mixture of (5-(6-chloro-4-fluoropyridin-3-yl)-1,3,4-thiadiazol-2-yl)methyl acetate (Preparation 240, 20 mg, 0.07 mmol), MeNH₂ (0.5 mL, 2 M in THF) and DIPEA (18 mg, 0.14 mmol) in dioxane (2 mL) was stirred at 80 °C for 16 h. The reaction mixture was concentrated *in vacuo* and the residue purified by column chromatography on silica gel (50% EtOAc/PE) to afford the title compound (11 mg, 61%). LCMS m/z = 257 [M+H]⁺

### Preparation 242: (5-(6-((2-(difluoromethyl)-6-((2,4-dimethoxybenzyl)amino) pyrimidin-4-yl)amino)-4-(methylamino)pyridin-3-yl)-1,3,4-thiadiazol-2-yl)methanol

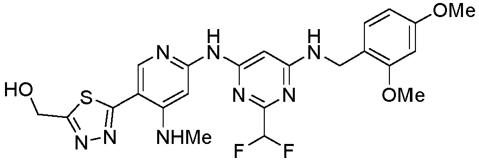

A mixture of (5-(6-chloro-4-(methylamino)pyridin-3-yl)-1,3,4-thiadiazol-2-yl)methanol (Preparation 241, 11 mg, 0.04 mmol), 2-(difluoromethyl)-N4-(2,4-dimethoxybenzyl)pyrimidine-4,6-diamine (Preparation 5, 14 mg, 0.04 mmol), BrettPhos Pd G4 (2 mg) and Cs₂CO₃ (39 mg, 0.12 mmol) in dioxane (2 mL) was stirred at 100 °C under N₂ for 16 h. The mixture was concentrated *in vacuo* and the residue purified by column chromatography on silica gel (1:20 MeOH/DCM) to afford the title compound (12 mg, 56%). LCMS m/z = 531 [M+H]⁺.

### Preparation 243 to 246

The title compounds were prepared from 2-(difluoromethyl)-N4-(2,4-dimethoxybenzyl) pyrimidine-4,6-diamine (Preparation 5) and the appropriate chloropyridine (RCl) using an analogous method to that described for Preparation 242.

| **Preparation No.** | **Name/Structure/ RCl/Catalyst/Data** |
|---|---|
| **243** | 6-((2-(difluoromethyl)-6-((2,4-dimethoxybenzyl)amino)pyrimidin-4-yl)amino)-N-(methyl-d3)-4-(methylamino)nicotinamide |
| | 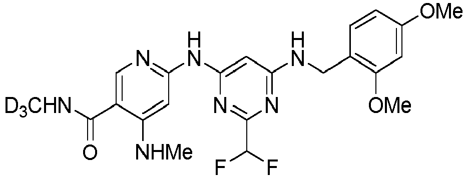 |
| | RCl: 6-chloro-N-(methyl-d3)-4-(methylamino)nicotinamide (Preparation 210) |
| | Yield: 489 mg, 69%; LCMS m/z = 477 [M+H]⁺. |
| **244** | 4-(cyclopropylamino)-6-((2-(difluoromethyl)-6-((2,4-dimethoxybenzyl) amino)pyrimidin-4-yl)amino)-N-(methyl-d3)nicotinamide |
| | 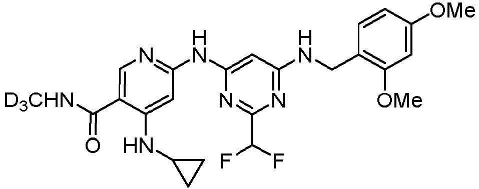 |
| | RCl: 6-chloro-4-(cyclopropylamino)-N-(methyl-d3)nicotinamide (Preparation 208) |
| | White solid: 266 mg, 82%; LCMS m/z = 503 [M+H]⁺. |
| **245** | 6-((2-(difluoromethyl)-6-((2,4-dimethoxybenzyl)amino)pyrimidin-4-yl)amino)-N-isopropyl-4-(methylamino)nicotinamide |
| | 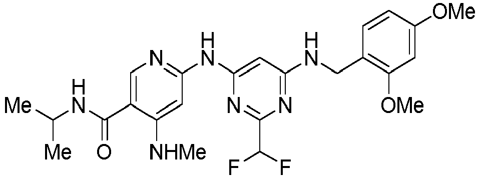 |
| | RCl: 6-chloro-N-isopropyl-4-(methylamino)nicotinamide (Preparation 211) |
| | White solid: 653 mg, 79%; LCMS m/z = 502 [M+H]⁺. |
| **246** | 4-(cyclopropylamino)-6-((2-(difluoromethyl)-6-((2,4-dimethoxy benzyl)amino)pyrimidin-4-yl)amino)-N-isopropylnicotinamide 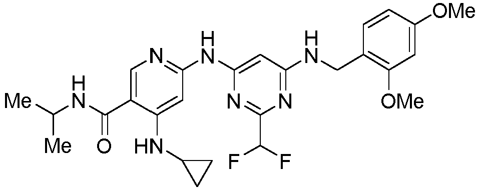 |
| | RCl: 6-chloro-4-(cyclopropylamino)-N-isopropylnicotinamide (Preparation 209) |
| | White solid: 119 mg, 26%; LCMS m/z = 528 [M+H]⁺. |

### Preparation 247 and 248: N-(cyanomethyl)-6-((2-(difluoromethyl)-6-((2,4-dimethoxybenzyl)amino)pyrimidin-4-yl)amino)-4-(methylamino)nicotinamide and 6-((2-(difluoromethyl)-6-((2,4-dimethoxybenzyl)amino)pyrimidin-4-yl)amino)-4-(methylamino)nicotinamide

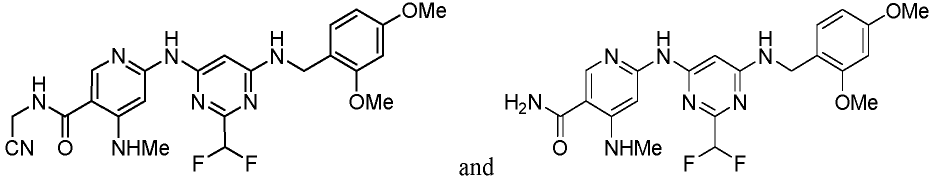

A solution of 6-chloro-N-(cyanomethyl)-4-(methylamino)nicotinamide (Preparation 212, 195 mg, 0.868 mmol), 2-(difluoromethyl)-N4-(2,4-dimethoxybenzyl)pyrimidine-4,6-diamine (Preparation 5, 296 mg, 0.955 mmol), BrettPhos Pd G4 (80 mg, 0.087 mmol) and Cs₂CO₃ (848 mg, 2.60 mmol) in dioxane (4.34 mL) was purged with N₂ for 5 min before stirring at 100 °C overnight. The reaction mixture was filtered and the filtrate washed with 10% MeOH in DCM. The combined organics were concentrated and the residue purified by reverse-phase chromatography on silica gel (20-100% MeCN/H₂O (+0.1% TFA) to afford the title compounds.

N-(cyanomethyl)-6-((2-(difluoromethyl)-6-((2,4-dimethoxybenzyl)amino)pyrimidin-4-yl)amino)-4-(methylamino)nicotinamide (Preparation 247, 29 mg, 6.7%); LCMS m/z = 499 [M+H]⁺.

6-((2-(difluoromethyl)-6-((2,4-dimethoxybenzyl)amino)pyrimidin-4-yl)amino)-4-(methylamino)nicotinamide (Preparation 248, 47 mg, 11.8%); LCMS m/z = 460 [M+H]⁺.

### Preparation 249: (R)-6-((6-((2,4-dimethoxybenzyl)amino)-2-(1-fluoroethyl) pyrimidin-4-yl)amino)-4-(isopopylamino)-N-methylnicotinamide

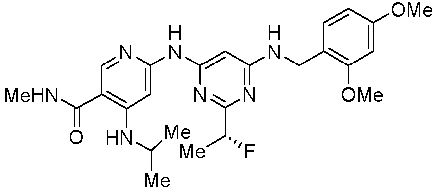

A mixture of 6-chloro-4-(isopropylamino)-N-methylnicotinamide (Preparation 181, 50 mg, 0.22 mmol), (R)-N4-(2,4-dimethoxybenzyl)-2-(1-fluoroethyl)pyrimidine-4,6-diamine (Preparation 14, 67.3 mg, 0.22 mmol), BrettPhos Pd G4 (40.5 mg, 0.044 mmol) and Cs₂CO₃ (215 mg, 0.659 mmol) in dioxane (2 mL) was stirred at 100 °C for 16 h under N₂. The mixture was concentrated *in vacuo* and the residue was purified column chromatography on silica gel (5% MeOH/DCM) to afford the title compound (50 mg, 45%). LCMS m/z = 498 [M+H]⁺.

### Preparation 250: (R)-6-((6-((2,4-dimethoxybenzyl)amino)-2-(1-fluoroethyl) pyrimidin-4-yl)amino)-4-(ethylamino)-N-methylnicotinamide

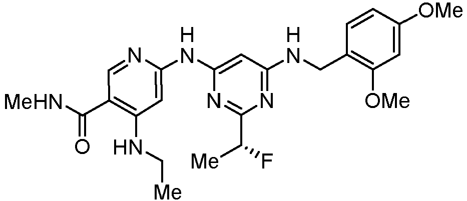

A mixture of 6-chloro-4-(ethylamino)-N-methylnicotinamide (Preparation 184, 60 mg, 0.280 mmol), (R)-N4-(2,4-dimethoxybenzyl)-2-(1-fluoroethyl)pyrimidine-4,6-diamine (Preparation 14, 85.7 mg, 0.280 mmol), Cs₂CO₃ (182 mg, 0.560 mmol) and Pd(t-Bu₃P)₂ (71.5 mg, 0.140 mmol) in dioxane (2 mL) was stirred at 100 °C for 16 h under N₂. The reaction mixture was concentrated *in vacuo* and the residue purified by column chromatography on silica gel eluting with MeOH/DCM (1/20) to afford the title compound as a white solid (60 mg, 44%). LCMS m/z = 484 [M+H]⁺.

### Preparations 251 to 254

The title compounds were prepared from the appropriate chloropyridine (RCl) and the appropriate amine (RNH₂) using an analogous method to that described for Preparation 250. Alternative catalyst systems used are noted in the table.

| **Preparation Number** | **Name/Structure/ RCl/RNH₂/Data** |
|---|---|
| **251** | (S)-6-((6-((2,4-dimethoxybenzyl)amino)-2-(1-fluoroethyl)pyrimidin-4-yl)amino)-4-(ethylamino)-N-methylnicotinamide |
| | 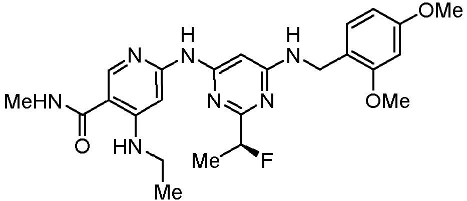 |
| | RCl: 6-chloro-4-(ethylamino)-N-methylnicotinamide (Preparation 184); RNH₂: (S)-N4-(2,4-dimethoxybenzyl)-2-(1-fluoroethyl)pyrimidine-4,6-diamine (Preparation 13). Yellow solid (80 mg, 63%); LCMS m/z = 484 [M+H]⁺. |
| **252** | (S)-6-((6-((2,4-dimethoxybenzyl)amino)-2-(1-fluoroethyl)pyrimidin-4-yl)amino)-4-(methylamino)-N-methylnicotinamide |
| | 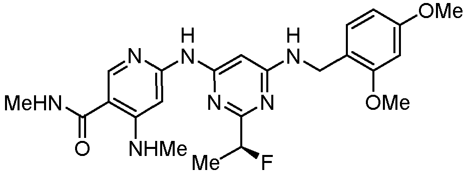 |
| | RCl: 6-chloro-N-methyl-4-(methylamino)nicotinamide (Preparation 180); RNH₂: (S)-N4-(2,4-dimethoxybenzyl)-2-(1-fluoroethyl)pyrimidine-4,6-diamine (Preparation 13). Yellow solid (80 mg, 63%); LCMS m/z = 470 [M+H]⁺. |
| **253** | (S)-6-((6-((2,4-dimethoxybenzyl)amino)-2-(1-fluoroethyl)pyrimidin-4-yl)amino)-4-(isopropylamino)-N-methylnicotinamide |
| | 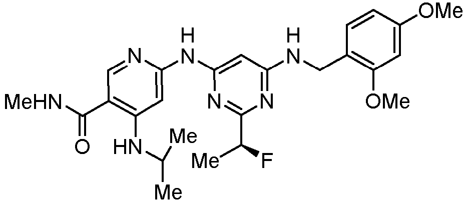 |
| | RCl: 6-chloro-4-(isopropylamino)-N-methylnicotinamide (Preparation 181); RNH₂: (S)-N4-(2,4-dimethoxybenzyl)-2-(1-fluoroethyl)pyrimidine-4,6-diamine (Preparation 13). Yellow solid (90 mg, 70%); LCMS m/z = 498 [M+H]⁺. |
| **254** | (S)-4-(cyclopropylamino)-6-((6-((2,4-dimethoxybenzyl)amino)-2-(1-fluoroethyl)pyrimidin-4-yl)amino)-N-methylnicotinamide |
| | 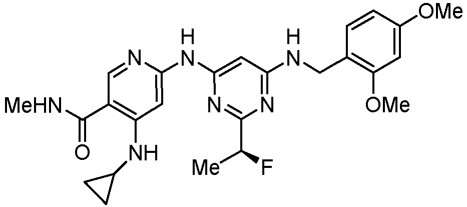 |
| | RCl: 6-chloro-4-(cyclopropylamino)-N-methylnicotinamide (Preparation 198); RNH₂: (S)-N4-(2,4-dimethoxybenzyl)-2-(1-fluoroethyl)pyrimidine-4,6-diamine (Preparation 13). |
| | Catalyst: BrettPhos Pd G4. |
| | Yellow solid (30 mg, 28%); LCMS m/z = 496 [M+H]⁺. |

### Preparation 255: N-(tert-butyl)-6-chloro-2-(difluoromethyl)pyrimidin-4-amine

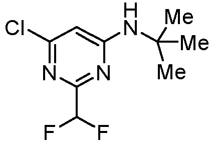

A solution of 6-dichloro-2-(difluoromethyl)pyrimidine (Preparation 2, 330 mg, 1.65 mmol), 2-methylpropan-2-amine (361 mg, 4.94 mmol) and DIPEA (252 mg, 4.95 mmol) in NMP was stirred at 100 °C for 1 h. The reaction mixture was diluted with EtOAc (100 mL) and washed with brine and H₂O. The combined organics were evaporated to dryness *in vacuo* and the residue purified by column chromatography on silica gel (50% EtOAc/PE) to afford the title compound as a yellow solid (300 mg, 77%). LCMS m/z = 236 [M+H]⁺.

### Preparation 256: 6-((diphenylmethylene)amino)-4-(isopropylamino)-N-(oxazol-4-ylmethyl)nicotinamide

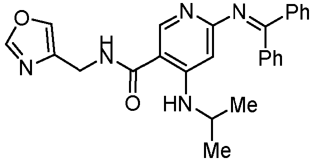

A mixture of 6-chloro-4-(isopropylamino)-N-(oxazol-4-ylmethyl)nicotinamide (Preparation 220, 500 mg, 1.81 mmol), diphenylmethanimine (360 mg, 1.99 mmol), Pd₂(dba)₃ (50 mg), XantPhos (50 mg) and Cs₂CO₃ (1.18 g, 3.62 mmol) in dioxane (100 mL) was stirred at 100 °C for 16 h. The mixture was concentrated *in vacuo* and the residue purified by column chromatography on silica gel (20:1 DCM/MeOH) to afford the title product (398 mg, 50%). LCMS m/z = 440 [M+H]⁺.

### Preparation 257: 6-amino-4-(isopropylamino)-N-(oxazol-4-ylmethyl)nicotinamide

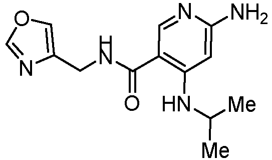

HCl/dioxane (2 mL, 4M) was added to a solution of 6-((diphenylmethylene)amino)-4-(isopropylamino)-N-(oxazol-4-ylmethyl)nicotinamide (Preparation 256, 398 mg, 0.9 mmol) in DCM (2 mL) and the mixture stirred at rt for 1 h. The mixture was concentrated *in vacuo* and the residue was purified by column chromatography on silica gel (10:1 DCM/MeOH) to give the title compound (198 mg, 80%). LCMS m/z = 440 [M+H]⁺.

### Preparation 258: 6-((6-(tert-butylamino)-2-(difluoromethyl)pyrimidin-4-yl)amino)-4-(isopropylamino)-N-(oxazol-4-ylmethyl)nicotinamide

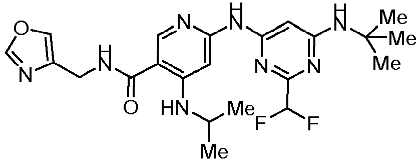

A mixture of compound N-(tert-butyl)-6-chloro-2-(difluoromethyl)pyrimidin-4-amine (Preparation 255, 120 mg, 0.509 mmol), 6-amino-4-(isopropylamino)-N-(oxazol-4-ylmethyl) nicotinamide (Preparation 257, 150 mg, 0.509 mmol), Pd₂(dba)₃ (93.2 mg, 0.102 mmol), XantPhos (58.9 mg, 0.102 mmol) and Cs₂CO₃ (498 mg, 1.5 mmol) in dioxane ( 2 mL) was stirred at 100 °C for 12 h. The reaction mixture was evaporated to dryness *in vacuo* and the residue and purified by column chromatography on silica gel (5% MeOH/DCM) to afford the title compound as a yellow solid (100 mg, 41%). LCMS m/z = 475 [M+H]⁺.

### Preparation 259: 6-((6-amino-2-(hydroxymethyl)pyrimidin-4-yl)amino)-4-(isopropylamino)-N-methylnicotinamide

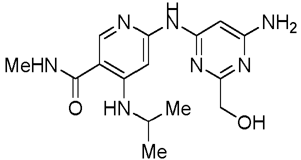

A mixture of (4,6-diaminopyrimidin-2-yl)methanol (Preparation 174, 60 mg, 0.428 mmol), 6-chloro-4-(isopropylamino)-N-methylnicotinamide (Preparation 181, 97.4 mg, 0.428 mmol), Cs₂CO₃ (278 mg, 0.856 mmol) and Brettphos Pd G4 (40 mg, 0.043 mmol) in NMP (3 mL) was stirred at 100 °C for 16 h. The reaction mixture was evaporated to dryness *in vacuo* and the residue purified by Prep-HPLC-3 to get the title compound as a white solid (40 mg, 28%). LCMS m/z = 332 [M+H]⁺.

### Preparation 260: 6-((6-amino-2-(difluoromethyl)pyrimidin-4-yl)amino)-4-fluoro-N-methylnicotinamide

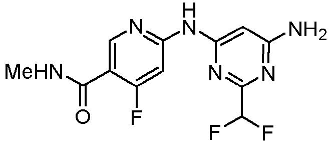

To a mixture of 6-chloro-4-fluoro-N-methylnicotinamide (Preparation 176, 1.4 g, 7.45 mmol), 2-(difluoromethyl)pyrimidine-4,6-diamine (Preparation 8, 4.77 g, 29.8 mmol) and Cs₂CO₃ (7.26 g, 22.35 mmol), BrettPhos Pd G3 (337.49 mg, 0.373 mmol) in t-AmOH (60 mL) was degassed and purged (x3) with N₂ and the mixture stirred at 120 °C for 2 h under N₂. The reaction mixture was concentrated under reduced pressure and H₂O (30 mL) added. The mixture was extracted with EtOAc (3x 30 mL). The resulting solids were removed by filtration and the filtrate evaporated to dryness *in vacuo* to afford the title compound (1.5 g, 45%) which was used without further purification. LCMS m/z = 313 [M+H]⁺.

### Preparation 261: 5-(4,6-dichloropyridin-3-yl)-3-methyl-1,2,4-oxadiazole

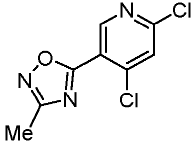

**Part 1.** A mixture of compound 4,6-dichloronicotinamide (2 g, 10 mmol) and 1,1-dimethoxy-N,N-dimethylethan-1-amine (1.67 g, 12 mmol) in DMF (20 mL) was stirred at 70 °C for 2 h. The reaction mixture was diluted with EtOAc and washed with water and brine. The combined organics were evaporated to dryness *in vacuo* to afford (E)-4,6-dichloro-N-(1-(dimethylamino)ethylidene)nicotinamide (2 g) which was without further purification. LCMS m/z = 260 [M+H]⁺.

**Part 2.** A mixture of the compound of Part 1 (2 g, 7.7 mmol), NH₂OH (492 mg, 15.4 mmol) and AcOH (1 mL) in dioxane/water (20 mL/1 mL) was stirred at 70 °C for 1 h. The reaction mixture was concentrated and purified by column chromatography on silica gel (5:1 PE/ EtOAc) to give the title compound (700 mg, 39%). LCMS m/z = 230 [M+H]⁺.

### Preparation 262: 2-chloro-N-isopropyl-5-(3-methyl-1,2,4-oxadiazol-5-yl)pyridin-4-amine

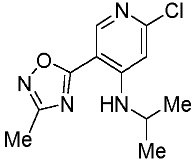

A mixture of 5-(4,6-dichloropyridin-3-yl)-3-methyl-1,2,4-oxadiazole (700 mg, 3.0 mmol) and propan-2-amine (360 mg, 6.1 mmol) in EtOH (20 mL) was stirred at 80 °C for 16 h. The reaction mixture was evaporated to dryness *in vacuo* and the residue purified by column chromatography on silica gel (20:1 DCM/MeOH) to afford the title compound (600 mg, 79%). LCMS m/z = 253 [M+H]⁺.

### Preparation 263: N2-benzyl-N4-isopropyl-5-(3-methyl-1,2,4-oxadiazol-5-yl) pyridine-2,4-diamine

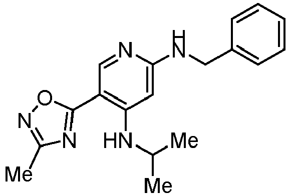

A mixture of 2-chloro-N-isopropyl-5-(3-methyl-1,2,4-oxadiazol-5-yl)pyridin-4-amine (Preparation 262, 220 mg, 0.870 mmol) and benzylamine (932 mg, 8.70 mmol) was stirred at 200 °C under microwave irradiation for 1 h. The reaction was diluted with EtOAc and washed with brine and water. The organics were evaporated to dryness *in vacuo* and the residue was purified by column chromatography on silica gel (5% MeOH/DCM) to afford the title compound as a yellow solid (230 mg, 82%). LCMS m/z = 324 [M+H]⁺.

### Preparation 264: N4-isopropyl-5-(3-methyl-1,2,4-oxadiazol-5-yl)pyridine-2,4-diamine

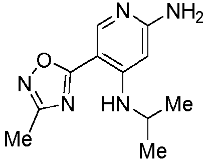

To a solution of N2-benzyl-N4-isopropyl-5-(3-methyl-1,2,4-oxadiazol-5-yl)pyridine-2,4-diamine (Preparation 263, 230 mg, 0.711 mmol) in DCM (10 mL) was added TfOH (534 mg, 3.6 mmol) and the resulting mixture stirred at rt for 3 h. The reaction mixture was treated with aqueous NaHCO₃ to adjust the pH to approx. pH 10. The solution was evaporated *in vacuo* and the residue purified by column chromatography on silica gel (5% MeOH/DCM) to afford the title compound as a white solid (110 mg, 67%). LCMS m/z = 234 [M+H]⁺.

### Preparation 265: N4-(tert-butyl)-2-(difluoromethyl)-N6-(4-(isopropylamino)-5-(3-methyl-1,2,4-oxadiazol-5-yl)pyridin-2-yl)pyrimidine-4,6-diamine

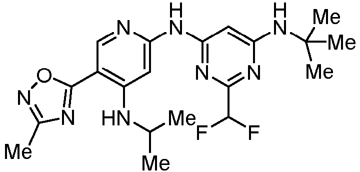

A mixture of N-(tert-butyl)-6-chloro-2-(difluoromethyl)pyrimidin-4-amine (Preparation 255, 100 mg, 0.424 mmol), N4-isopropyl-5-(3-methyl-1,2,4-oxadiazol-5-yl)pyridine-2,4-diamine (Preparation 264, 98.9 mg, 0.424 mmol), Pd(t-Bu₃P)₂ (43.3 mg, 0.085 mmol) and Cs₂CO₃ (414 mg, 1.3 mmol) in dioxane (2 mL) was stirred at 100 °C for 16 h. The reaction mixture was evaporated to dryness *in vacuo* and the residue purified by column chromatography on silica gel (5% MeOH/DCM) to afford the title compound as a white solid (80 mg, 44%). LCMS m/z = 433 [M+H]⁺.

### Preparation 266: tert-butyl 6-(6-((6-amino-2-(difluoromethyl)pyrimidin-4-yl)amino)-4-(ethylamino)nicotinamido)-2-azaspiro[3.3]heptane-2-carboxylate

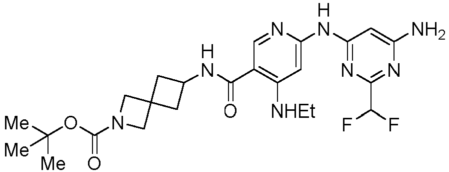

The title compound was prepared from tert-butyl 6-(6-chloro-4-(ethylamino) nicotinamido)-2-azaspiro[3.3]heptane-2-carboxylate (Preparation 183) and 2-(difluoromethyl) pyrimidine-4,6-diamine (Preparation 8) using an analogous method to that described for Preparation 265. Yield: 120 mg, 45%; LCMS m/z = 519 [M+H]⁺.

### Preparation 267: methyl 6-chloro-4-(((1R,2S)-2-fluorocyclopropyl)amino)nicotinate

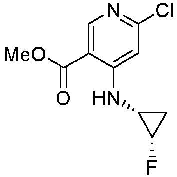

A mixture of methyl 6-chloro-4-fluoropyridine-3-carboxylate (300 mg, 1.58 mmol), (1R,2S)-2-fluorocyclopropan-1-amine hydrochloride (176 mg, 1.58 mmol) and DIPEA (611 mg, 4.74 mmol) in dioxane (10 mL) was stirred at rt for 16 h. The reaction mixture was evaporated to dryness *in vacuo* and the residue was purified by column chromatography on silica gel (1:5 EtOAc/PE) to afford the title compound as a white solid (120 mg, 31%). LCMS m/z = 245 [M+H]⁺.

### Preparation 268: methyl 6-chloro-4-(((1S,2R)-2-fluorocyclopropyl)amino)nicotinate

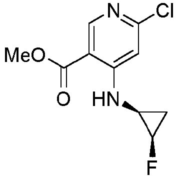

The title compound we prepared as a white solid (100 mg, 26%) from methyl 6-chloro-4-fluoropyridine-3-carboxylate and (1S,2R)-2-fluorocyclopropan-1-amine 4-methylbenzene sulfonate using an analogous method to that described for Preparation 267. LCMS m/z = 245 [M+H]⁺.

### Preparation 269: methyl 6-((4-(difluoromethyl)-6-((3,5-dimethoxybenzyl) amino)pyridin-2-yl)amino)-4-(((1R,2S)-2-fluorocyclopropyl)amino)nicotinate

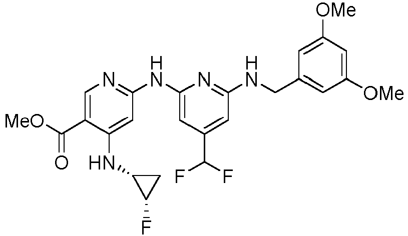

The title compound was prepared as a white solid (60 mg, 28%) from methyl 6-chloro-4-(((1R,2S)-2-fluorocyclopropyl)amino)nicotinate (Preparation 267) and 2-(difluoromethyl)-N4-(2,4-dimethoxybenzyl)pyrimidine-4,6-diamine (Preparation 5) using a similar method to that described for Preparation 242. LCMS m/z = 519 [M+H]⁺.

### Preparation 270: methyl 6-((4-(difluoromethyl)-6-((3,5-dimethoxybenzyl) amino)pyridin-2-yl)amino)-4-(((1S,2R)-2-fluorocyclopropyl)amino)nicotinate

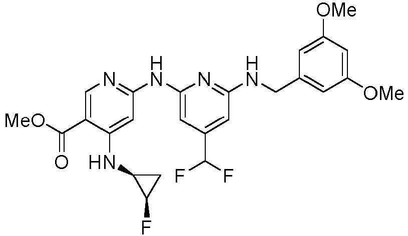

The title compound was prepared as a white solid (60 mg, 28%) from methyl 6-chloro-4-(((1S,2R)-2-fluorocyclopropyl)amino)nicotinate (Preparation 268) and 2-(difluoromethyl)-N4-(2,4-dimethoxybenzyl)pyrimidine-4,6-diamine (Preparation 5) using a similar method to that described for Preparation 242. LCMS m/z = 519 [M+H]⁺.

### Preparation 271: 6-((4-(difluoromethyl)-6-((3,4-dimethylbenzyl)amino)pyridin-2-yl)amino)-4-(((1R,2S)-2-fluorocyclopropyl)amino)-N-methylnicotinamide

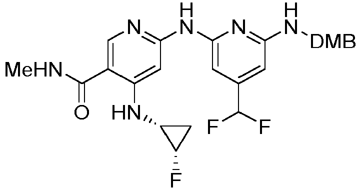

Methyl 6-((4-(difluoromethyl)-6-((3,4-dimethylbenzyl)amino)pyridin-2-yl)amino)-4-(((1R,2S)-2-fluorocyclopropyl)amino)nicotinate (60 mg, 0.115 mmol) in MeNH₂ (30% in MeOH, 5 mL) was stirred at 70°C for 16 h in a sealed vessel. The mixture was concentrated *in vacuo* to afford the title compound as a white solid (60 mg, 100%). LCMS m/z = 518 [M+H]⁺.

### Preparation 272: 6-((4-(difluoromethyl)-6-((3,4-dimethylbenzyl)amino)pyridin-2-yl)amino)-4-(((1S,2R)-2-fluorocyclopropyl)amino)-N-methylnicotinamide

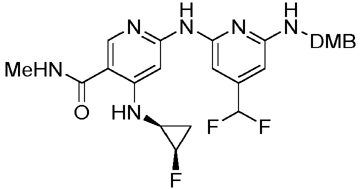

The title compound was prepared as a white solid (60 mg, 100%) from methyl 6-((4-(difluoromethyl)-6-((3,5-dimethoxybenzyl)amino)pyridin-2-yl)amino)-4-(((1S,2R)-2-fluoro cyclopropyl)amino)nicotinate (Preparation 270) using an analogous method to that described for Preparation 271. LCMS m/z = 518 [M+H]⁺.

### Preparation 273: N-(2-(4-bromo-1H-pyrazol-1-yl)ethyl)-2,2,2-trifluoro-N-methylethan-1-amine

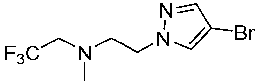

A mixture of 2-(4-bromo-1*H*-pyrazol-1-yl)-N-methylethan-1-amine hydrochloride (1 g, 4.16 mmol), triethylamine (2.318 ml, 16.63 mmol) and 2,2,2-trifluoroethyltrifluoromethane sulfonate (3.59 ml, 24.95 mmol) in DMF (8 ml) was stirred overnight under microwave irradiation at 80 °C. The cooled mixture was diluted with EtOAc and washed with water and brine, dried (Na₂SO₄) and concentrated *in vacuo.* The resulting residue was purified by silica gel chromatography (0~100% EtOAc/hexanes) to give the title compound as a colorless oil (888 mg, 74.7%). LCMS m/z = 286.0 [M+H]⁺.

### Preparation 274: 2,2,2-trifluoro-N-methyl-N-(2-(4-(4,4,5,5-tetramethyl-1,3,2-dioxaborolan-2-yl)-1H-pyrazol-1-yl)ethyl)ethan-1-amine

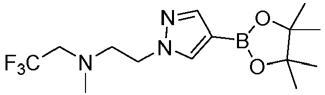

A mixture of N-(2-(4-bromo-1H-pyrazol-1-yl)ethyl)-2,2,2-trifluoro-N-methylethan-1-amine (Preparation 273, 445 mg, 1.555 mmol), bis(pinacolato)diboron (474 mg, 1.867 mmol), PdCl₂(dppf). DCM (191 mg, 0.233 mmol) and potassium acetate (458 mg, 4.67 mmol) in 1,4-dioxane (6 ml) was stirred at 90 °C overnight. Crude material in solution was used directly without further purification. LCMS m/z = 334.1 [M+H]⁺.

### Preparation 275: 1-(3-(benzyloxy)cyclobutyl)-4-(4,4,5,5-tetramethyl-1,3,2-dioxaborolan-2-yl)-1H-pyrazole

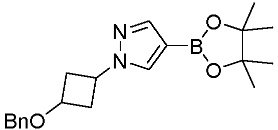

A solution of iPrMgCl.LiCl 1.3M in THF (4.89 ml, 6.35 mmol) was added dropwise to a solution of 1-(3-(benzyloxy)cyclobutyl)-4-iodo-1*H*-pyrazole (prepared according to US 2016/0256448,1.8 g, 5.08 mmol) in THF (20.33 ml) at 0 °C. After 30 min, 2-isopropoxy-4,4,5,5-tetramethyl-1,3,2-dioxaborolane (1.555 ml, 7.62 mmol) was added dropwise. The reaction mixture was then allowed to warm to rt. After 48 h, the reaction mixture was diluted with 50% saturated ammonium chloride solution (25 mL) and then extracted with EtOAc (50 mL x 2). The combined organics were washed with brine, dried over Na₂SO₄, filtered and concentrated under reduced pressure. The residue was purified by silica gel column chromatography (0~100% EtOAc/hexanes) to give the title compound as a colorless solid (635 mg, 35.3 %). LCMS m/z = 355.0 [M+H]⁺; 1H NMR (500 MHz, CDCl₃) δ ppm 7.85 (d, 1H), 7.74 (d, 1H), 7.38 (t, 5H), 4.97 (t, 1H), 4.49 (d, 2H), 4.47 - 4.41 (m, 1H), 2.90 - 2.73 (m, 2H), 2.65 (m, 2H), 1.34 (d, 12H).

### Preparation 276: tert-butyl (2-(4-(4,4,5,5-tetramethyl-1,3,2-dioxaborolan-2-yl)-1H-pyrazol-1-yl)propan-2-yl)carbamate

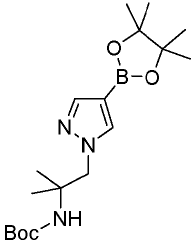

A mixture of 4-(4,4,5,5-tetramethyl-1,3,2-dioxaborolan-2-yl)-1*H*-pyrazole (358 mg, 1.845 mmol), tert-butyl 4,4-dimethyl-1,2,3-oxathiazolidine-3-carboxylate 2,2-dioxide (464 mg, 1.845 mmol) and cesium carbonate (1202 mg, 3.69 mmol) in acetonitrile (6ml) was stirred overnight at 70°C under microwave irradiation. The cooled reaction mixture was filtered, and the residue was washed with DCM. The combined filtrate was concentrated under reduced pressure and purified by silica gel column chromatography (0~100% EtOAc/hexanes) to afford the title compound (112 mg, 16.6%). LCMS m/z = 366.2 [M+H]⁺.

### Preparation of Exemplified Compounds

### Example 1: (S)-1-(4-(6-((6-amino-2-(1-fluoroethyl)pyrimidin-4-yl)amino)-4-isopropoxypyridin-3-yl)-1H-pyrazol-1-yl)-2-methylpropan-2-ol (167)

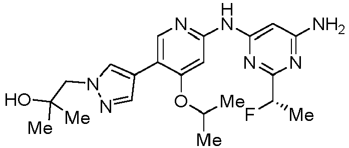

To a solution of (S)-1-(4-(6-((6-((2,4-dimethoxybenzyl)amino)-2-(1-fluoroethyl) pyrimidin-4-yl)amino)-4-isopropoxypyridin-3-yl)-1H-pyrazol-1-yl)-2-methylpropan-2-ol (Preparation 114, 840 mg, 1.45 mmol) in DCM (5 mL) was added TFA (10 mL) and the reaction stirred at rt for 4 h. The mixture was concentrated *in vacuo* and the residue was purified by prep HPLC-3 to afford the title compound (421.1 mg, 67% yield) as a white solid. LCMS m/z = 430 [M+H]⁺. ¹H-NMR (400 MHz, DMSO-d₆) *δ*: 9.54 (s, 1H), 8.34 (s, 1H), 8.05 (s, 1H), 7.88 (s, 1H), 7.55 (s, 1H), 6.67 (s, 1H), 6.62 (s, 2H), 5.37-5.23 (m, 1H), 4.71 (s, 1H), 4.68 (q, 1H), 4.04 (s, 2H), 1.57 (dd, 3H), 1.40 (d, 6H), 1.08 (s, 6H).

### Example 2: (S)-2-(1-fluoroethyl)-N4-(4-isopropoxy-5-(1-(oxetan-3-yl)-1H-pyrazol-4-yl)pyridin-2-yl)pyrimidine-4,6-diamine (129)

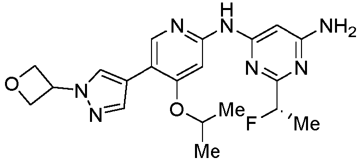

To a solution of (S)-N4-(2,4-dimethoxybenzyl)-2-(1-fluoroethyl)-N6-(4-isopropoxy-5-(1-(oxetan-3-yl)-1H-pyrazol-4-yl)pyridin-2-yl)pyrimidine-4,6-diamine (Preparation 117, 80 mg, 0.141 mmol) in DCM (1 mL) was added TFA (5 equiv.) and the reaction stirred at rt for 4 h. The mixture was concentrated *in vacuo* and the residue was purified by prep HPLC-3 to afford the title compound (13.1 mg, 23% yield) as a white solid. LCMS m/z = 414 [M+H]⁺. 1H-NMR (400 MHz, DMSO-d₆) δ: 9.57 (s, 1H), 8.36 (s, 1H), 8.19 (s, 1H), 8.04 (s, 1H), 7.55 (s, 1H), 6.67 (s, 1H), 6.64 (s, 2H), 5.67-5.60 (m, 1H), 5.39-5.22 (m, 1H), 4.95-4.90 (m, 4H), 4.73-4.67 (m, 1H), 1.57 (dd, 3H), 1.41 (d, 6H).

### Example 3: 1-(4-(6-((6-amino-2-(difluoromethyl)pyrimidin-4-yl)amino)-4-methoxypyridin-3-yl)-1H-pyrazol-1-yl)-2-methylpropan-2-ol (120)

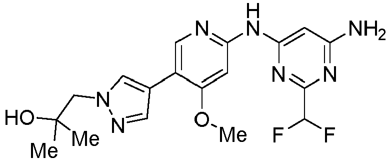

A solution of 1-(4-(6-((2-(difluoromethyl)-6-((2,4-dimethoxybenzyl)amino)pyrimidin-4-yl)amino)-4-methoxypyridin-3-yl)-1H-pyrazol-1-yl)-2-methylpropan-2-ol (Preparation 146, 480 mg, 0.86 mmol) in TFA (2 mL) and DCM (2 mL) was stirred at rt for 2 h. The mixture was concentrated *in vacuo* and sat. aq. NaHCO₃ solution (20 mL) was added. The precipitate was filtered and the solid was dissolved in MeOH (30 mL). The precipitate was filtered, and the filtrate was concentrated to give the crude product which was washed with DCM. The precipitate was filtered to provide the title compound (294 mg, 84%) as a light yellow solid. LCMS m/z = 406 [M+H]⁺. ¹HNMR (500 MHz, DMSO-d₆) δ 9.79 (s, 1H), 8.37 (s, 1H), 8.05 (s, 1H), 7.98 (s, 1H), 7.38 (s, 1H), 6.95-6.90 (m, 3H), 6.64-6.37 (m, 1H), 4.70 (s, 1H), 4.03 (s, 2H), 3.89 (s, 3H), 1.08 (s, 6H).

### Example 4: 1-(4-(6-((6-amino-2-(difluoromethyl)pyrimidin-4-yl)amino)-4-cyclopropoxypyridin-3-yl)-1H-pyrazol-1-yl)-2-methylpropan-2-ol (173)

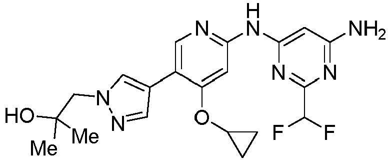

A mixture of 1-(4-(4-cyclopropoxy-6-((2-(difluoromethyl)-6-((2,4-dimethoxybenzyl) amino)pyrimidin-4-yl)amino)pyridin-3-yl)-1H-pyrazol-1-yl)-2-methylpropan-2-ol (Preparation 147, 800 mg, 1.37 mmol) in TFA (2 mL) and DCM (2 mL) was stirred at rt for 2 h. The mixture was concentrated *in vacuo* and saturated aq. NaHCO₃ solution (20 mL) was added. The precipitate was filtered and the solid dissolved in MeOH (30 mL). The mixture was filtered, and the filtrate concentrated *in vacuo.* The crude product was washed with DCM and the precipitate was filtered to give the title compound (440 mg, 74.6 %) as a beige solid. LCMS m/z = 432 [M+H]⁺. 1H NMR (500 MHz, DMSO-d₆) δ 9.84 (s, 1H), 8.37 (s, 1H), 7.98 (s, 1H), 7.86 (s, 1H), 7.79 (s, 1H), 6.89 (s, 3H), 6.50 (t, 1H), 4.70 (s, 1H), 4.03 (s, 2H), 3.96 - 3.86 (m, 1H), 1.08 (s, 6H), 0.93 - 0.75 (m, 4H).

### Example 5: 1-(4-(6-((6-amino-2-(difluoromethyl)pyrimidin-4-yl)amino)-4-(methoxy-d3)pyridin-3-yl)-1H-pyrazol-1-yl)-2-methylpropan-2-ol (126)

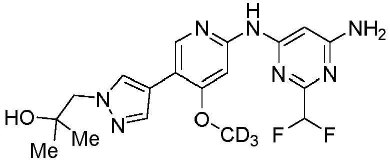

A mixture of 1-(4-(6-((2-(difluoromethyl)-6-((2,4-dimethoxybenzyl)amino)pyrimidin-4-yl)amino)-4-(methoxy-d3)pyridin-3-yl)-1H-pyrazol-1-yl)-2-methylpropan-2-ol (Preparation 139, 300 mg, 0.537 mmol) in DCM (10 mL) and TFA (2.5 mL) was stirred at rt for 16 h. The reaction mixture was concentrated *in vacuo,* and Na₂CO₃ (aq.) was added to the residue to adjust the pH to 10. The mixture was filtered, the solid was washed with water and DCM and dried to afford the title compound (147.4 mg, yield: 67%) as a white solid. LCMS m/z = 409 [M+H]⁺. ¹H-NMR (400 MHz, DMSO-d₆) δ: 9.82 (s, 1H), 8.38 (s, 1H), 8.06 (s, 1H), 7.91 (s, 1H), 7.37 (s, 1H), 6.96-6.92 (m, 3H), 6.51 (t, 1H), 4.74 (br, s, 1H), 4.04 (s, 2H), 1.08 (s, 6H).

### Example 6: 1-(4-(6-((6-amino-2-(difluoromethyl)pyrimidin-4-yl)amino)-4-(trifluoromethoxy)pyridin-3-yl)-1H-pyrazol-1-yl)-2-methylpropan-2-ol (198)

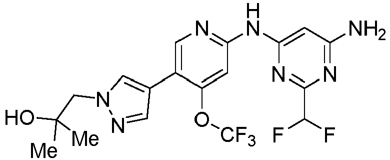

To a mixture of 1-(4-(6-((2-(difluoromethyl)-6-((2,4-dimethoxybenzyl)amino)pyrimidin-4-yl)amino)-4-(trifluoromethoxy)pyridin-3-yl)-1H-pyrazol-1-yl)-2-methylpropan-2-ol (Preparation 108, 180 mg, 0.3 mmol) in DCM (2 mL) was added TFA (1 mL) and the reaction stirred at rt for 1 h. The mixture was concentrated *in vacuo* and the crude purified by prep HPLC-3 to give the title product (85 mg, 62%). LCMS m/z = 460 [M+H]⁺. ¹H-NMR (400 MHz, DMSO-d₆) δ: 10.20 (s, 1H), 8.63 (s, 1H), 8.05-8.00 (m, 2H), 7.88 (s, 1H), 7.21-6.96 (m, 2H), 6.74 (s, 1H), 6.50 (t, 1H), 4.74 (s, 1H), 4.07 (s, 2H), 1.08 (s, 6H).

### Example 7: 1-(4-(6-((6-amino-2-(difluoromethyl)pyrimidin-4-yl)amino)-4-(difluoromethoxy)pyridin-3-yl)-1H-pyrazol-1-yl)-2-methylpropan-2-ol (181)

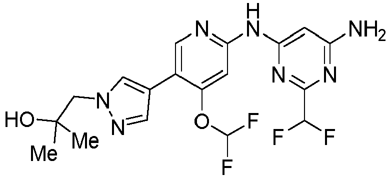

A mixture of 1-(4-(4-(difluoromethoxy)-6-((2-(difluoromethyl)-6-((2,4-dimethoxybenzyl) amino)pyrimidin-4-yl)amino)pyridin-3-yl)-1H-pyrazol-1-yl)-2-methylpropan-2-ol (Preparation 115, 50 mg, 0.08 mmol) and TFA (1 mL) in DCM (2 mL) was stirred at rt for 1 h. The mixture was concentrated *in vacuo* and purified by prep HPLC-3 to give the title product (26.2 mg, 69%). LCMS m/z = 442 [M+H]⁺. 1H-NMR (400 MHz, DMSO-d₆) δ: 10.06 (s, 1H), 8.55 (s, 1H), 8.05 (s, 1H), 7.90 (s, 1H), 7.63 (s, 1H), 7.38 (t, 1H), 6.97 (br s, 2H), 6.84 (s, 1H), 6.50 (t, 1H), 4.73 (s, 1H), 4.06 (s, 2H), 1.08 (s, 6H).

### Examples 8 to 38

The following compounds were prepared from the appropriate protected starting material (SM) and purified by HPLC-3, following a similar procedure to that described in Example 7.

| **Example No** | **Name, Structure, Starting Material (SM), Data** |
|---|---|
| **8** | 1-(4-(6-((6-amino-2-(difluoromethyl)pyrimidin-4-yl)amino)-4-methoxypyridin-3-yl)-1H-pyrazol-1-yl)propan-2-ol (85) |
| | 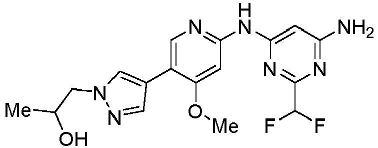 |
| | SM: 1-(4-(6-((2-(difluoromethyl)-6-((2,4-dimethoxybenzyl)amino)pyrimidin-4-yl)amino)-4-methoxypyridin-3-yl)-1H-pyrazol-1-yl)propan-2-ol (Preparation 151) |
| | LCMS m/z = 392 [M+H]⁺ |
| | 1H-NMR (500 MHz, DMSO-d₆) δ: 9.79 (s, 1H), 8.36 (s, 1H), 8.06 (s, 1H), 7.90 (s, 1H), 7.37 (s, 1H), 6.95-6.90 (m, 3H), 6.61 (m, 1H), 4.92 (d, 1H), 4.03-3.98 (m, 3H), 3.89 (s, 3H), 1.05 (d, 3H). |
| **9** | 2-(difluoromethyl)-N4-(5-(1-(2-(dimethylamino)propyl)-1H-pyrazol-4-yl)-4-methoxypyridin-2-yl)pyrimidine-4,6-diamine (150) |
| | 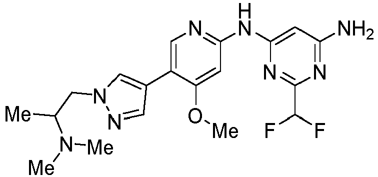 |
| | SM: 2-(difluoromethyl)-N4-(2,4-dimethoxybenzyl)-N6-(5-(1-(2-(dimethylamino)propyl)-1H-pyrazol-4-yl)-4-methoxypyridin-2-yl)pyrimidine-4,6-diamine (Preparation 152) |
| | white solid, 38.2 mg, 80%. LCMS m/z = 419 [M+H]⁺ |
| | ¹H-NMR (500 MHz, DMSO-d₆) δ: 9.79 (s, 1H), 8.35 (s, 1H), 8.08 (s, 1H), 7.88 (s, 1H), 7.37 (s, 1H), 6.95-6.90 (m, 3H), 6.61 (m, 1H), 4.21-4.17 (m, 2H), 4.06-3.96 (m, 1H), 3.89 (s, 3H), 2.19 (s, 6H), 0.85 (d, 3H). |
| **10** | (S)-2-(difluoromethyl)-N4-(4-methoxy-5-(1-(pyrrolidin-3-yl)-1H-pyrazol-4-yl)pyridin-2-yl)pyrimidine-4,6-diamine or (R)-2-(difluoromethyl)-N4-(4-methoxy-5-(1-(pyrrolidin-3-yl)-1H-pyrazol-4-yl)pyridin-2-yl)pyrimidine-4,6-diamine (99) |
| | 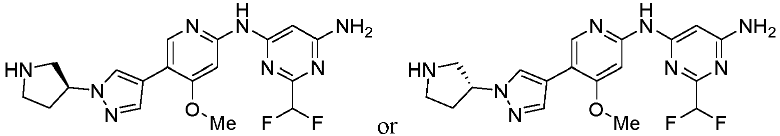 |
| | SM: tert-butyl (S)-3-(4-(6-((6-amino-2-(difluoromethyl)pyrimidin-4-yl)amino)-4-methoxypyridin-3-yl)-1H-pyrazol-1-yl)pyrrolidine-1-carboxylate or tert-butyl (R)-3-(4-(6-((6-amino-2-(difluoromethyl)pyrimidin-4-yl)amino)-4-methoxypyridin-3-yl)-1H-pyrazol-1-yl)pyrrolidine-1-carboxylate (Preparation 161) |
| | 13.5 mg, 42% as a white solid. LCMS m/z = 403 [M+H]⁺ |
| | 1H-NMR (500 MHz, DMSO-d₆) δ: 9.81 (s, 1H), 8.36 (s, 1H), 8.17-8.16 (m, 1H), 7.94-7.93 (m, 1H), 7.40 (s, 1H), 6.93-6.91 (m, 3H), 6.51 (t, 1H), 4.97-4.95 (m, 1H), 3.89 (s, 3H), 3.18-3.02 (m, 4H), 2.26-2.11 (m, 2H). |
| **11** | (R)-2-(difluoromethyl)-N4-(4-methoxy-5-(1-(pyrrolidin-3-yl)-1H-pyrazol-4-yl)pyridin-2-yl)pyrimidine-4,6-diamine or (S)-2-(difluoromethyl)-N4-(4-methoxy-5-(1-(pyrrolidin-3-yl)-1H-pyrazol-4-yl)pyridin-2-yl)pyrimidine-4,6-diamine (100) |
| | 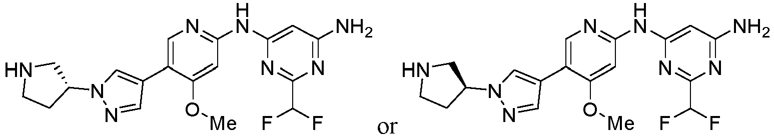 |
| | SM: tert-butyl (R)-3-(4-(6-((6-amino-2-(difluoromethyl)pyrimidin-4-yl)amino)-4-methoxypyridin-3-yl)-1H-pyrazol-1-yl)pyrrolidine-1-carboxylate or tert-butyl (S)-3-(4-(6-((6-amino-2-(difluoromethyl)pyrimidin-4-yl)amino)-4-methoxypyridin-3-yl)-1H-pyrazol-1-yl)pyrrolidine-1-carboxylate (Preparation 162) |
| | 15.2 mg, 48% as a white solid. LCMS m/z = 403 [M+H]⁺ |
| | 1H-NMR (500 MHz, DMSO-d₆) δ: 9.80 (s, 1H), 8.36-8.35 (m, 1H), 8.16-8.13 (m, 1H), 7.94-7.88 (m, 1H), 7.38 (s, 1H), 6.94-6.90 (m, 3H), 6.51 (t, 1H), 4.99-4.85 (m, 1H), 3.89 (s, 3H), 3.15-2.88 (m, 4H), 2.33-2.03 (m, 2H). |
| **12** | (R)-N4-(5-(1-(azetidin-2-ylmethyl)-1H-pyrazol-4-yl)-4-methoxypyridin-2-yl)-2-(difluoromethyl)pyrimidine-4,6-diamine or (S)-N4-(5-(1-(azetidin-2-ylmethyl)-1H-pyrazol-4-yl)-4-methoxypyridin-2-yl)-2-(difluoromethyl)pyrimidine-4,6-diamine (101) |
| | 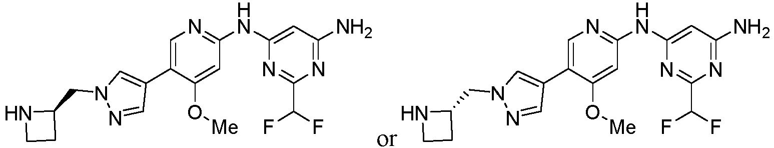 |
| | SM: tert-butyl (R)-2-((4-(6-((6-amino-2-(difluoromethyl)pyrimidin-4-yl)amino)-4-methoxypyridin-3-yl)-1H-pyrazol-1-yl)methyl)azetidine-1-carboxylate or tert-butyl (S)-2-((4-(6-((6-amino-2-(difluoromethyl)pyrimidin-4-yl)amino)-4-methoxypyridin-3-yl)-1H-pyrazol-1-yl)methyl)azetidine-1-carboxylate (Preparation 163) |
| | White solid, 14.5 mg, 45% yield. LCMS m/z = 403 [M+H]⁺ |
| | 1H-NMR (500 MHz, DMSO-d₆) δ: 9.79 (s, 1H), 8.36-8.35 (m, 1H), 8.10-8.07 (m, 1H), 7.94-7.90 (m, 1H), 7.37 (s, 1H), 6.95-6.90 (m, 3H), 6.51 (t, 1H), 4.41-4.14 (m, 3H), 3.89 (s, 3H), 3.50-3.44 (m, 2H), 3.22-3.18 (m, 1H), 2.24-2.18 (m, 1H), 2.10-1.99 (m, 1H). |
| **13** | (S)-N4-(5-(1-(azetidin-2-ylmethyl)-1H-pyrazol-4-yl)-4-methoxypyridin-2-yl)-2-(difluoromethyl)pyrimidine-4,6-diamine or (R)-N4-(5-(1-(azetidin-2-ylmethyl)-1H-pyrazol-4-yl)-4-methoxypyridin-2-yl)-2-(difluoromethyl)pyrimidine-4,6-diamine (102) |
| | 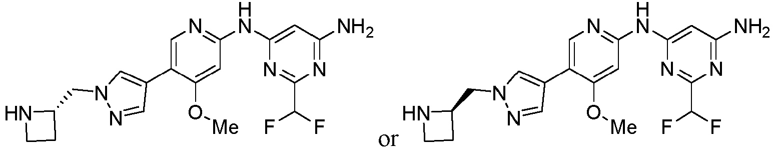 |
| | SM: tert-butyl (S)-2-((4-(6-((6-amino-2-(difluoromethyl)pyrimidin-4-yl)amino)-4-methoxypyridin-3-yl)-1H-pyrazol-1-yl)methyl)azetidine-1-carboxylate or tert-butyl (R)-2-((4-(6-((6-amino-2-(difluoromethyl)pyrimidin-4-yl)amino)-4-methoxypyridin-3-yl)-1H-pyrazol-1-yl)methyl)azetidine-1-carboxylate (Preparation 164) |
| | White solid, 10.2 mg, 32% yield. LCMS m/z = 403 [M+H]⁺ |
| | 1H-NMR (500 MHz, DMSO-d₆) δ: 9.79 (s, 1H), 8.36-8.35 (m, 1H), 8.10-8.07 (m, 1H), 7.94-7.89 (m, 1H), 7.37 (s, 1H), 6.95-6.90 (m, 3H), 6.51 (t, 1H), 4.41-4.11 (m, 3H), 3.89 (s, 3H), 3.48-3.43 (m, 2H), 3.20-3.16 (m, 1H), 2.23-2.17 (m, 1H), 2.08-1.99 (m, 1H). |
| **14** | N4-(5-(1-cyclopropyl-1H-pyrazol-4-yl)-4-(difluoromethoxy)pyridin-2-yl)-2-(difluoromethyl)pyrimidine-4,6-diamine (127) |
| | 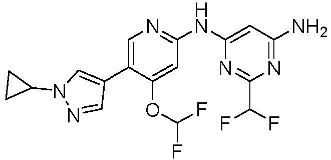 |
| | SM: N4-(5-(1-cyclopropyl-1H-pyrazol-4-yl)-4-(difluoromethoxy)pyridin-2-yl)-2-(difluoromethyl)-N6-(2,4-dimethoxybenzyl)pyrimidine-4,6-diamine (Preparation 125) |
| | 30 mg, 52% yield. LCMS m/z = 410 [M+H]⁺ |
| | 1H-NMR (400 MHz, DMSO-d₆) δ: 10.06 (s, 1H), 8.52 (s, 1H), 8.13 (s, 1H), 7.84 (s, 1H), 7.63 (s, 1H), 7.38 (t, 1H), 6.97 (s, 2H), 6.84 (s, 1H), 6.50 (t, 1H), 3.81-3.75 (m, 1H), 1.09-0.97 (m, 4H). |
| **15** | 1-(4-(6-((6-amino-2-(difluoromethyl)pyrimidin-4-yl)amino)-4-(difluoromethoxy)pyridin-3-yl)-1H-pyrazol-1-yl)propan-2-ol (155) |
| | 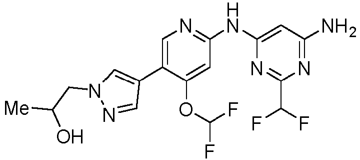 |
| | SM: 1-(4-(4-(difluoromethoxy)-6-((2-(difluoromethyl)-6-((2,4-dimethoxybenzyl)amino)pyrimidin-4-yl)amino)pyridin-3-yl)-1H-pyrazol-1-yl)propan-2-ol (Preparation 126) |
| | 1.4 mg, 19% as a white solid. LCMS m/z = 428 [M+H]⁺ |
| | 1H-NMR (400 MHz, DMSO-d₆) δ: 10.05 (s, 1H), 8.53 (s, 1H), 8.05 (s, 1H), 7.89 (s, 1H), 7.63 (s, 1H), 7.37 (t, 1H), 6.96 (s, 2H), 6.84 (s, 1H), 6.50 (t, 1H), 4.93 (d, 1H), 4.06-3.98 (m, 3H), 1.06 (d, 3H). |
| **16** | N4-(4-(difluoromethoxy)-5-(1-(2-(dimethylamino)ethyl)-1H-pyrazol-4-yl)pyridin-2-yl)-2-(difluoromethyl)pyrimidine-4,6-diamine (179) |
| | 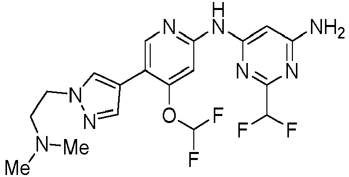 |
| | SM: N4-(4-(difluoromethoxy)-5-(1-(2-(dimethylamino)ethyl)-1H-pyrazol-4-yl)pyridin-2-yl)-2-(difluoromethyl)-N6-(2,4-dimethoxybenzyl)pyrimidine-4,6-diamine (Preparation 127) |
| | 46.6 mg, 66% yield. LCMS m/z = 441 [M+H]⁺ |
| | 1H-NMR (400 MHz, DMSO-d₆) δ: 10.06 (s, 1H), 8.52 (s, 1H), 8.11 (s, 1H), 7.88 (s, 1H), 7.63 (s, 1H), 7.38 (t, 1H), 6.97 (s, 2H), 6.84 (s, 1H), 6.50 (t, 1H), 4.26-4.22 (m, 2H), 2.70-2.67 (m, 2H), 2.18 (m, 6H). |
| **17** | N4-(4-(difluoromethoxy)-5-(1-(2-(methylamino)propyl)-1H-pyrazol-4-yl)pyridin-2-yl)-2-(difluoromethyl)pyrimidine-4,6-diamine (180) |
| | 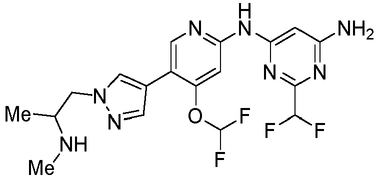 |
| | SM: N4-(4-(difluoromethoxy)-5-(1-(2-(methylamino)propyl)-1H-pyrazol-4-yl)pyridin-2-yl)-2-(difluoromethyl)-N6-(2,4-dimethoxybenzyl)pyrimidine-4,6-diamine (Preparation 128) |
| | 5.2 mg 9% yield. LCMS m/z = 441 [M+H]⁺ |
| | 1H-NMR (400 MHz, DMSO-d₆) δ: 10.05 (s, 1H), 8.53 (s, 1H), 8.07 (s, 1H), 7.88 (s, 1H), 7.63 (s, 1H), 7.36 (t, 1H), 6.96 (s, 2H), 6.84 (s, 1H), 6.50 (t, 1H), 4.13-3.98 (m, 2H), 2.92-2.87 (m, 2H), 2.27 (d, 3H), 0.95-0.92 (m, 3H). |
| **18** | N4-(4-(difluoromethoxy)-5-(1-(2-methyl-2-(methylamino)propyl)-1H-pyrazol-4-yl)pyridin-2-yl)-2-(difluoromethyl)pyrimidine-4,6-diamine (193) |
| | 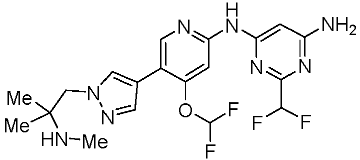 |
| | SM: tert-butyl (1-(4-(4-(difluoromethoxy)-6-((2-(difluoromethyl)-6-((2,4-dimethoxybenzyl)amino)pyrimidin-4-yl)amino)pyridin-3-yl)-1H-pyrazol-1-yl)-2-methylpropan-2-yl)(methyl)carbamate (Preparation 128) |
| | 10.1 mg, 39%. LCMS m/z = 455 [M+H]⁺ |
| | ¹H-NMR (400 MHz, DMSO-d₆) δ: 10.06 (s, 1H), 8.55 (s, 1H), 8.06 (s, 1H), 7.94 (s, 1H), 7.64 (s, 1H), 7.36 (t, 1H), 6.96 (s, 2H), 6.84 (s, 1H), 6.50 (t, 1H), 4.13 (s, 2H), 2.33 (s, 3H), 1.01 (s, 6H). |
| **19** | N4-(4-(difluoromethoxy)-5-(1-(1-methylpyrrolidin-3-yl)-1H-pyrazol-4-yl)pyridin-2-yl)-2-(difluoromethyl)pyrimidine-4,6-diamine (192) |
| | 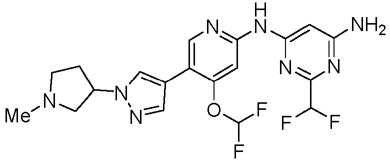 |
| | SM: N4-(4-(difluoromethoxy)-5-(1-(1-methylpyrrolidin-3-yl)-1H-pyrazol-4-yl)pyridin-2-yl)-2-(difluoromethyl)-N6-(2,4-dimethoxybenzyl)pyrimidine-4,6-diamine (Preparation 129) |
| | 48.6 mg, 65% as a white solid. LCMS m/z = 453 [M+H]⁺ |
| | 1H-NMR (400 MHz, DMSO-d₆) δ: 10.06 (s, 1H), 8.54 (s, 1H), 8.16 (s, 1H), 7.89 (s, 1H), 7.65 (s, 1H), 7.37 (t, 1H), 6.96 (s, 2H), 6.82 (s, 1H), 6.50 (t, 1H), 4.97 (s, 1H), 2.92-2.52 (m, 4H), 2.41-2.33 (m, 4H), 2.12-2.10 (m, 1H). |
| **20** | 2-(difluoromethyl)-N4-(5-(1-(2-methyl-2-(methylamino)propyl)-1H-pyrazol-4-yl)-4-(trifluoromethoxy)pyridin-2-yl)pyrimidine-4,6-diamine (200) |
| | 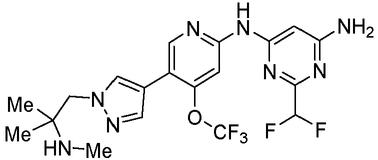 |
| | SM: tert-butyl (1-(4-(6-((2-(difluoromethyl)-6-((2,4-dimethoxybenzyl)amino)pyrimidin-4-yl)amino)-4-(trifluoromethoxy)pyridin-3-yl)-1H-pyrazol-1-yl)-2-methylpropan-2-yl)(methyl)carbamate (Preparation 113). |
| | 29.7 mg, 89% LCMS m/z = 473 [M+H]⁺. |
| | ¹H-NMR (400 MHz, DMSO-d₆) *δ*: 10.21 (s, 1H), 8.62 (s, 1H), 8.05 (s, 1H), 7.99 (s, 1H), 7.89 (s, 1H), 7.01 (s, 2H), 6.74 (s, 1H), 6.49 (t, 1H), 4.08 (s, 2H), 2.27 (s, 3H), 0.96 (s, 6H). |
| **21** | 2-(difluoromethyl)-N4-(5-(1-(2-(dimethylamino)ethyl)-1H-pyrazol-4-yl)-4-(trifluoromethoxy)pyridin-2-yl)pyrimidine-4,6-diamine (194) |
| | 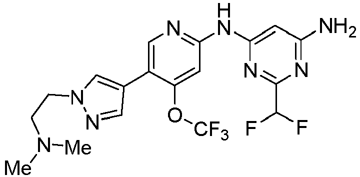 |
| | SM: 2-(difluoromethyl)-N4-(2,4-dimethoxybenzyl)-N6-(5-(1-(2-(dimethylamino)ethyl)-1H-pyrazol-4-yl)-4-(trifluoromethoxy)pyridin-2-yl)pyrimidine-4,6-diamine (Preparation 111) |
| | 81 mg, 54%, LCMS m/z = 459 [M+H]⁺ |
| | 1H-NMR (400 MHz, DMSO-d₆) δ: 10.20 (s, 1H), 8.61 (s, 1H), 8.15 (s, 1H), 8.00 (s, 1H), 7.88 (s, 1H), 7.01 (br s, 2H), 6.74 (s, 1H), 6.49 (t, 1H), 4.33-4.30 (m, 2H), 2.86-2.83 (m, 2H), 2.30 (s, 6H). |
| **22** | 2-(4-(6-((6-amino-2-(difluoromethyl)pyrimidin-4-yl)amino)-4-cyclopropoxypyridin-3-yl)-1H-pyrazol-1-yl)ethan-1-ol (108) |
| | 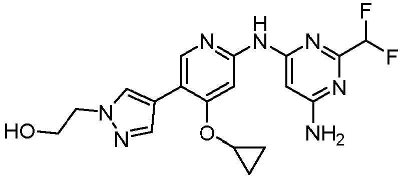 |
| | SM: N4-(5-(1-(2-((tert-butyldimethylsilyl)oxy)ethyl)-1H-pyrazol-4-yl)-4-cyclopropoxypyridin-2-yl)-2-(difluoromethyl)-N6-(2,4-dimethoxybenzyl)pyrimidine-4,6-diamine (Preparation 149) |
| | 27.8 mg, 57% as a white solid. LCMS m/z = 404 [M+H]⁺. |
| | 1H-NMR (400 MHz, DMSO-d₆) δ: 9.82 (s, 1H), 8.35 (s, 1H), 8.00 (s, 1H), 7.85 (s, 1H), 7.79 (s, 1H), 6.89 (s, 3H), 6.49 (t, 1H), 4.91-4.87 (m, 1H), 4.16 (d, 2H), 3.95-3.88 (m, 1H), 3.75-3.74 (m, 2H), 0.89-0.82 (m, 4H). |
| **23** | N4-(4-cyclopropoxy-5-(1-(2-methoxyethyl)-1H-pyrazol-4-yl)pyridin-2-yl)-2-(difluoromethyl)pyrimidine-4,6-diamine (146) |
| | 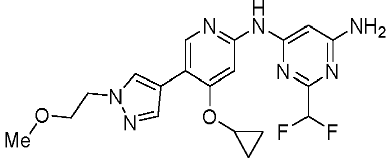 |
| | SM: N4-(4-cyclopropoxy-5-(1-(2-methoxyethyl)-1H-pyrazol-4-yl)pyridin-2-yl)-2-(difluoromethyl)-N6-(2,4-dimethoxybenzyl)pyrimidine-4,6-diamine (Preparation 131). |
| | 16.3 mg, 27% yield as a white solid. LCMS m/z = 418 [M+H]⁺ |
| | 1H-NMR (400 MHz, DMSO-d₆) δ: 9.83 (s, 1H), 8.35 (s, 1H), 8.00 (s, 1H), 7.86 (s, 1H), 7.78 (s, 1H), 6.89 (s, 3H), 6.49 (t, 1H), 4.28 (t, 2H), 3.92-3.91 (m, 1H), 3.70 (t, 2H), 3.24 (s, 3H), 0.89-0.82 (m, 4H). |
| **24** | 4-(4-(6-((6-amino-2-(difluoromethyl)pyrimidin-4-yl)amino)-4-cyclopropoxypyridin-3-yl)-1H-pyrazol-1-yl)-2-methylbutan-2-ol (185) |
| | 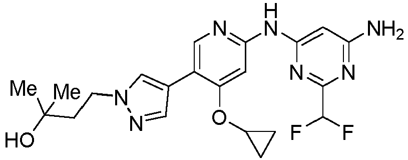 |
| | SM: 4-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)-2-methylbutan-2-ol (Preparation 132) |
| | White solid, 7.7 mg, 20% yield. LCMS m/z = 446 [M+H]⁺ |
| | ¹H-NMR (400 MHz, DMSO-*d₆*) *δ*: 9.83 (s, 1H), 8.34 (s, 1H), 8.01 (s, 1H), 7.83 (s, 1H), 7.78 (s, 1H), 6.88 (s, 3H), 6.49 (t, 1H), 4.44 (s, 1H), 4.21-4.17 (m, 2H), 3.92-3.89 (m, 1H), 1.91 (t, 2H), 1.14 (s, 6H), 0.89-0.83 (m, 4H). |
| | (S)-N4-(4-cyclopropoxy-5-(1-(pyrrolidin-3-yl)-1H-pyrazol-4-yl)pyridin-2-yl)-2-(difluoromethyl)pyrimidine-4,6-diamine or (R)-N4-(4-cyclopropoxy-5-(1-(pyrrolidin-3-yl)-1H-pyrazol-4-yl)pyridin-2-yl)-2-(difluoromethyl) pyrimidine-4,6-diamine (159) |
| **25** | 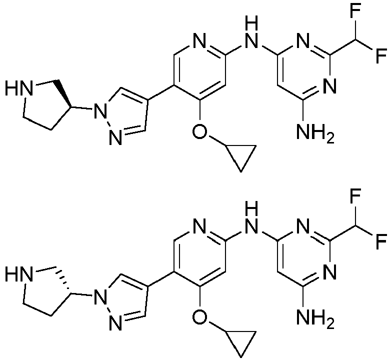 or |
| | SM: tert-butyl (S)-3-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)pyrrolidine-1-carboxylate or tert-butyl (R)-3-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)pyrrolidine-1-carboxylate (Preparation 140) |
| | 10.3 mg, 81% yield. LCMS m/z = 429 [M+H]⁺ |
| | 1H-NMR (400 MHz, DMSO-d₆) δ: 9.84 (s, 1H), 8.37-8.36 (m, 1H), 8.08 (s, 1H), 7.90-7.80 (m, 2H), 6.89-6.87 (s, 2H), 6.50 (t, 1H), 5.02-4.89 (m, 1H), 3.92-3.91 (m, 1H), 3.24-2.93 (m, 4H), 2.32-2.04 (m, 3H), 0.88-0.83 (m, 4H). |
| | (R)-N4-(4-cyclopropoxy-5-(1-(pyrrolidin-3-yl)-1H-pyrazol-4-yl)pyridin-2-yl)-2-(difluoromethyl)pyrimidine-4,6-diamine or (S)-N4-(4-cyclopropoxy-5-(1-(pyrrolidin-3-yl)-1H-pyrazol-4-yl)pyridin-2-yl)-2-(difluoromethyl)pyrimidine-4,6-diamine (160) |
| **26** | 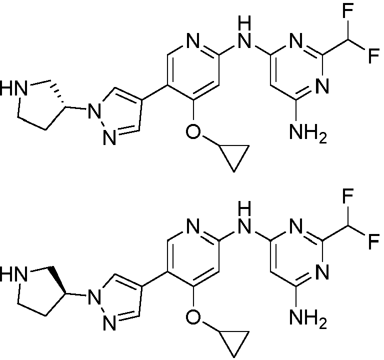 or |
| | SM: tert-butyl (R)-3-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)pyrrolidine-1-carboxylate or tert-butyl (S)-3-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)pyrrolidine-1-carboxylate (Preparation 141). |
| | 11.3 mg, 89% yield. LCMS m/z = 429 [M+H]⁺ |
| | 1H-NMR (400 MHz, DMSO-d₆) δ: 9.84 (s, 1H), 8.37-8.36 (m, 1H), 8.08 (s, 1H), 7.90-7.80 (m, 2H), 6.89-6.87 (s, 2H), 6.50 (t, 1H), 5.02-4.89 (m, 1H), 3.92-3.91 (m, 1H), 3.24-2.93 (m, 4H), 2.32-2.04 (m, 3H), 0.88-0.83 (m, 4H). |
| **27** | (R)-N4-(5-(1-(azetidin-2-ylmethyl)-1H-pyrazol-4-yl)-4-cyclopropoxypyridin-2-yl)-2-(difluoromethyl)pyrimidine-4,6-diamine or (S)-N4-(5-(1-(azetidin-2-ylmethyl)-1H-pyrazol-4-yl)-4-cyclopropoxypyridin-2-yl)-2-(difluoromethyl)pyrimidine-4,6-diamine (161) |
| | 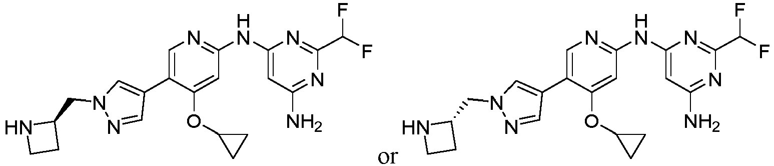 |
| | SM: tert-butyl (R)-2-((4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)methyl)azetidine-1-carboxylate or tert-butyl (S)-2-((4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)methyl)azetidine-1-carboxylate (Preparation 142) |
| | 4.2 mg, 55% yield as a white solid. LCMS m/z = 429 [M+H]⁺ |
| | 1H-NMR (400 MHz, DMSO-d₆) δ: 9.85 (s, 1H), 8.37 (s, 1H), 8.06 (s, 1H), 7.91 (s, 1H), 7.81 (s, 1H), 6.89-6.87 (s, 3H), 6.50 (t, 1H), 4.41-4.33 (m, 3H), 3.94-3.90 (m, 1H), 3.68-3.66 (m, 1H), 3.46-3.32 (m, 1H), 2.32-2.18 (m, 3H), 0.89-0.82 (m, 4H) |
| **28** | (S)-N4-(5-(1-(azetidin-2-ylmethyl)-1H-pyrazol-4-yl)-4-cyclopropoxypyridin-2-yl)-2-(difluoromethyl)pyrimidine-4,6-diamine or (R)-N4-(5-(1-(azetidin-2-ylmethyl)-1H-pyrazol-4-yl)-4-cyclopropoxypyridin-2-yl)-2-(difluoromethyl)pyrimidine-4,6-diamine (162) |
| | 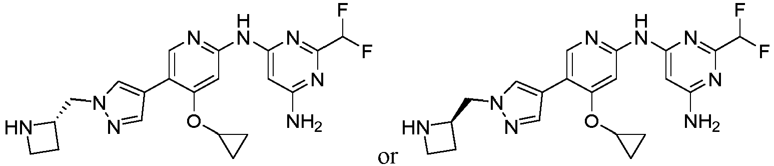 |
| | SM: tert-butyl (S)-2-((4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)methyl)azetidine-1-carboxylate or tert-butyl (R)-2-((4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)methyl)azetidine-1-carboxylate (Preparation 143) |
| | 1.7 mg, 22% yield. LCMS m/z = 429 [M+H]⁺ |
| | 1H-NMR (400 MHz, DMSO-d₆) δ: 9.85 (s, 1H), 8.37 (s, 1H), 8.06 (s, 1H), 7.91 (s, 1H), 7.81 (s, 1H), 6.89-6.87 (s, 3H), 6.50 (t, 1H), 4.41-4.33 (m, 3H), 3.94-3.90 (m, 1H), 3.68-3.66 (m, 1H), 3.46-3.32 (m, 1H), 2.32-2.18 (m, 3H), 0.89-0.82 (m, 4H) |
| **29** | N4-(4-cyclopropoxy-5-(1-(2-(methylamino)propyl)-1H-pyrazol-4-yl)pyridin-2-yl)-2-(difluoromethyl)pyrimidine-4,6-diamine (170) |
| | 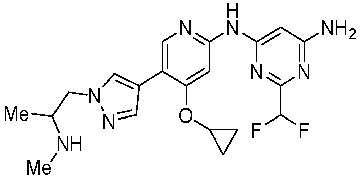 |
| | SM: N4-(4-cyclopropoxy-5-(1-(2-(methylamino)propyl)-1H-pyrazol-4-yl)pyridin-2-yl)-2-(difluoromethyl)-N6-(2,4-dimethoxybenzyl)pyrimidine-4,6-diamine (Preparation 153) |
| | 16.5 mg, 47% yield. LCMS m/z = 431 [M+H]⁺ |
| | 1H-NMR (400 MHz, DMSO-d₆) δ: 9.85 (s, 1H), 8.36 (s, 1H), 8.01-7.79 (m, 3H), 6.90-6.88 (m, 2H), 6.49 (t, 1H), 4.13-3.99 (m, 2H), 3.93-3.90 (m, 1H), 2.97-2.95 (m, 1H), 2.30 (s, 3H), 0.95-0.82 (m, 7H). |
| **30** | N4-(4-cyclopropoxy-5-(1-(2-(dimethylamino)ethyl)-1H-pyrazol-4-yl)pyridin-2-yl)-2-(difluoromethyl)pyrimidine-4,6-diamine (171) |
| | 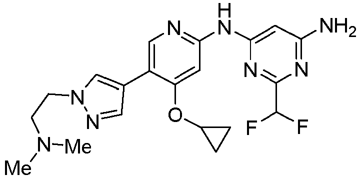 |
| | SM: N4-(4-cyclopropoxy-5-(1-(2-(dimethylamino)ethyl)-1H-pyrazol-4-yl)pyridin-2-yl)-2-(difluoromethyl)-N6-(2,4-dimethoxybenzyl)pyrimidine-4,6-diamine (Preparation 133) |
| | White solid, 13.2 mg, 35% yield. LCMS m/z = 431 [M+H]⁺ |
| | 1H-NMR (400 MHz, DMSO-d₆) δ: 9.83 (s, 1H), 8.35 (s, 1H), 8.04 (s, 1H), 7.85 (s, 1H), 7.79 (s, 1H), 6.88 (s, 3H), 6.49 (t, 1H), 4.24 (t, 2H), 3.94-3.90 (m, 1H), 2.77-2.73 (m, 2H), 2.24 (s, 6H), 0.89-0.80 (m, 4H). |
| **31^{A}** | N4-(4-cyclopropoxy-5-(1-(2-methyl-2-(methylamino)propyl)-1H-pyrazol-4-yl)pyridin-2-yl)-2-(difluoromethyl)pyrimidine-4,6-diamine (184) |
| | 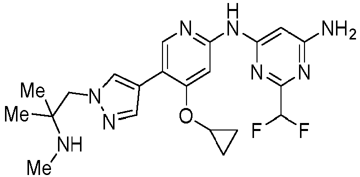 |
| | SM: tert-butyl (1-(4-(4-cyclopropoxy-6-((2-(difluoromethyl)-6-((2,4-dimethoxybenzyl)amino)pyrimidin-4-yl)amino)pyridin-3-yl)-1H-pyrazol-1-yl)-2-methylpropan-2-yl)(methyl)carbamate (Preparation 134). |
| | 20.3 mg, 52% yield. LCMS m/z = 444 [M+H]⁺ |
| | 1H-NMR (400 MHz, DMSO-d₆) δ: 9.85 (s, 1H), 8.36 (s, 1H), 7.97 (s, 1H), 7.87 (s, 1H), 7.79 (s, 1H), 6.92-6.98 (m, 3H), 6.49 (t, 1H), 4.04 (s, 2H), 3.92 (s, 1H), 2.27 (s, 3H), 0.96-0.81 (m, 10H). |
| **32** | N4-(4-cyclopropoxy-5-(1-(oxetan-3-yl)-1H-pyrazol-4-yl)pyridin-2-yl)-2-(difluoromethyl)pyrimidine-4,6-diamine (133) |
| | 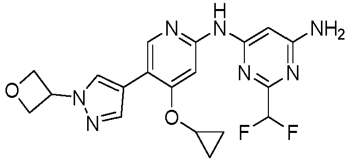 |
| | N4-(4-cyclopropoxy-5-(1-(oxetan-3-yl)-1H-pyrazol-4-yl)pyridin-2-yl)-2-(difluoromethyl)-N6-(2,4-dimethoxybenzyl)pyrimidine-4,6-diamine (Preparation 136) |
| | 37.7 mg, 25% yield as a white solid |
| | 1H-NMR (400 MHz, DMSO-d₆) δ: 9.85 (s, 1H), 8.38 (s, 1H), 8.14 (s, 1H), 8.00 (s, 1H), 7.80 (s, 1H), 6.89-6.88 (m, 3H), 6.49 (t, 1H), 5.65-5.62 (m, 1H), 4.92 (d, 4H), 3.92-3.91 (m, 1H), 0.89-0.83 (m, 4H). |
| **33** | N4-(4-cyclopropoxy-5-(1-(oxetan-2-ylmethyl)-1H-pyrazol-4-yl)pyridin-2-yl)-2-(difluoromethyl)pyrimidine-4,6-diamine (166) |
| | 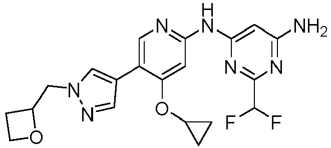 |
| | SM: N4-(4-cyclopropoxy-5-(1-(oxetan-2-ylmethyl)-1H-pyrazol-4-yl)pyridin-2-yl)-2-(difluoromethyl)-N6-(2,4-dimethoxybenzyl)pyrimidine-4,6-diamine (Preparation 135) |
| | 22.1 mg, 37% yield as a white solid. LCMS m/z = 430 [M+H]⁺ |
| | ¹H-NMR (400 MHz, DMSO-*d₆*) *δ*: 9.85 (s, 1H), 8.37 (s, 1H), 8.04 (s, 1H), 7.89 (s, 1H), 7.78 (s, 1H), 6.90-6.88 (m, 3H), 6.50 (t, 1H), 5.04-4.98 (m, 1H), 4.52-4.42 (m, 1H), 4.40-4.30 (m, 3H), 3.94-3.91 (m, 1H), 2.67-2.62 (m, 1H), 2.44-2.37 (m, 1H), 0.89-0.81 (m, 4H). |
| **34** | N4-(4-cyclopropoxy-5-(1-methyl-1H-pyrazol-4-yl)pyridin-2-yl)-2-(fluoromethyl)pyrimidine-4,6-diamine (32) |
| | 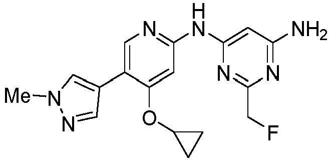 |
| | SM: N4-(4-cyclopropoxy-5-(1-methyl-1H-pyrazol-4-yl)pyridin-2-yl)-N6-(2,4-dimethoxybenzyl)-2-(fluoromethyl)pyrimidine-4,6-diamine (Preparation 120) |
| | 16.3 mg, 27% yield. LCMS m/z = 356 [M+H]⁺ |
| | 1H-NMR (500 MHz, DMSO-d₆) δ: 9.66 (s, 1H), 8.32 (s, 1H), 7.96 (s, 1H), 7.83-7.82 (m, 2H), 6.73-6.65 (m, 3H), 5.15 (d, 2H), 3.91-3.90 (m, 1H), 3.85 (s, 3H), 0.88-0.82 (m, 4H). |
| **35** | 2-(fluoromethyl)-N4-(4-isopropoxy-5-(1-methyl-1H-pyrazol-4-yl)pyridin-2-yl)pyrimidine-4,6-diamine (33) |
| | 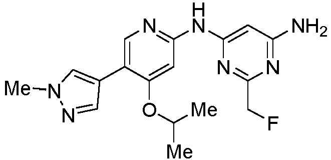 |
| | SM: N4-(2,4-dimethoxybenzyl)-2-(fluoromethyl)-N6-(4-isopropoxy-5-(1-methyl-1H-pyrazol-4-yl)pyridin-2-yl)pyrimidine-4,6-diamine (Preparation 119) |
| | 5.4 mg, 63% yield as a white solid. LCMS m/z = 358 [M+H]⁺. |
| | ¹H-NMR (400 MHz, DMSO-d₆) δ: 9.54 (s, 1H), 8.31 (s, 1H), 8.00 (s, 1H), 7.86 (s, 1H), 7.46 (s, 1H), 6.74 (s, 1H), 6.63 (s, 2H), 5.14 (d, 2H), 4.69-4.66 (m, 1H), 3.87 (s, 3H), 1.40 (d, 6H). |
| **36** | (S)-2-(1-fluoroethyl)-N4-(4-isopropoxy-5-(1-methyl-1H-pyrazol-4-yl)pyridin-2-yl)pyrimidine-4,6-diamine (51) |
| | 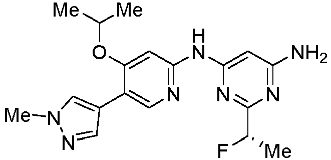 |
| | SM: (S)-N4-(2,4-dimethoxybenzyl)-2-(1-fluoroethyl)-N6-(4-isopropoxy-5-(1-methyl-1H-pyrazol-4-yl)pyridin-2-yl)pyrimidine-4,6-diamine (Preparation 118) |
| | white solid (14.8 mg, 52%). LCMS m/z = 372 [M+H]^{+.} |
| | 1H-NMR (500 MHz, DMSO-d₆) δ: 9.53 (s, 1H), 8.31 (s, 1H), 8.01 (s, 1H), 7.86 (s, 1H), 7.52 (s, 1H), 6.69-6.63 (m, 3H), 5.35-5.24 (m, 1H), 4.69-4.67 (m, 1H), 3.87 (s, 3H), 1.59-1.53 (m, 3H), 1.41-1.39 (m, 6H). |
| **37** | 2-((S)-1-fluoroethyl)-N4-(4-isopropoxy-5-(1-((R)-tetrahydrofuran-3-yl)-1H-pyrazol-4-yl)pyridin-2-yl)pyrimidine-4,6-diamine or 2-((S)-1-fluoroethyl)-N4-(4-isopropoxy-5-(1-((S)-tetrahydrofuran-3-yl)-1H-pyrazol-4-yl)pyridin-2-yl)pyrimidine-4,6-diamine (156) |
| | 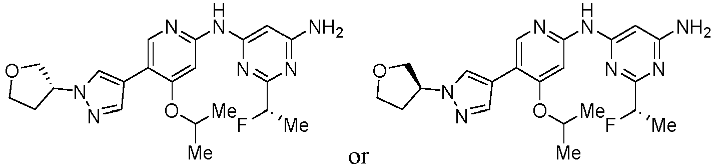 |
| | SM: N4-(2,4-dimethoxybenzyl)-2-((S)-1-fluoroethyl)-N6-(4-isopropoxy-5-(1-((R)-tetrahydrofuran-3-yl)-1H-pyrazol-4-yl)pyridin-2-yl)pyrimidine-4,6-diamine or N4-(2,4-dimethoxybenzyl)-2-((S)-1-fluoroethyl)-N6-(4-isopropoxy-5-(1-((S)-tetrahydrofuran-3-yl)-1H-pyrazol-4-yl)pyridin-2-yl)pyrimidine-4,6-diamine (Preparation 144) |
| | 3.2 mg, 27% yield as a white solid. LCMS m/z = 428 [M+H]⁺ |
| | 1H-NMR (400 MHz, DMSO-d₆) δ: 9.56 (s, 1H), 8.35 (s, 1H), 8.11 (s, 1H), 7.92 (s, 1H), 7.56 (s, 1H), 8.66-6.63 (m, 3H), 5.39-5.22 (m, 1H), 5.08-5.03 (m, 1H), 4.72-4.66 (m, 1H), 4.01-3.96 (m, 2H), 3.92-3.84 (m, 2H), 2.42-2.27 (m, 2H), 1.57 (dd, 3H), 1.40 (d, 6H). |
| **38** | 2-((S)-1-fluoroethyl)-N4-(4-isopropoxy-5-(1-((S)-tetrahydrofuran-3-yl)-1H-pyrazol-4-yl)pyridin-2-yl)pyrimidine-4,6-diamine or 2-((S)-1-fluoroethyl)-N4-(4-isopropoxy-5-(1-((R)-tetrahydrofuran-3-yl)-1H-pyrazol-4-yl)pyridin-2-yl)pyrimidine-4,6-diamine (157) |
| | 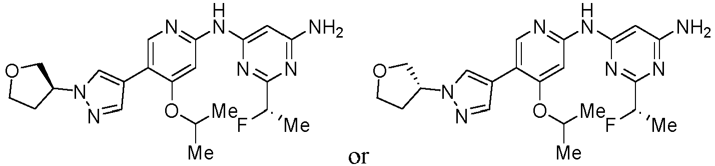 |
| | SM: N4-(2,4-dimethoxybenzyl)-2-((S)-1-fluoroethyl)-N6-(4-isopropoxy-5-(1-((S)-tetrahydrofuran-3-yl)-1H-pyrazol-4-yl)pyridin-2-yl)pyrimidine-4,6-diamine or N4-(2,4-dimethoxybenzyl)-2-((S)-1-fluoroethyl)-N6-(4-isopropoxy-5-(1-((R)-tetrahydrofuran-3-yl)-1H-pyrazol-4-yl)pyridin-2-yl)pyrimidine-4,6-diamine (Preparation 145) |
| | 6.2 mg, 70% yield as a white solid. LCMS m/z = 428 [M+H]⁺ |
| | 1H-NMR (400 MHz, DMSO-d₆) δ: 9.56 (s, 1H), 8.35 (s, 1H), 8.11 (s, 1H), 7.92 (s, 1H), 7.56 (s, 1H), 8.66-6.63 (m, 3H), 5.39-5.22 (m, 1H), 5.08-5.02 (m, 1H), 4.72-4.66 (m, 1H), 4.01-3.96 (m, 2H), 3.92-3.82 (m, 2H), 2.42-2.27 (m, 2H), 1.57 (dd, 3H), 1.40 (d, 6H). |

### Examples 39 and 40: (S)-2-(difluoromethyl)-N4-(5-(1-(2-(methylamino)propyl)-1H-pyrazol-4-yl)-4-(trifluoromethoxy)pyridin-2-yl)pyrimidine-4,6-diamine and (R)-2-(difluoromethyl)-N4-(5-(1-(2-(methylamino)propyl)-1H-pyrazol-4-yl)-4-(trifluoro methoxy)pyridin-2-yl)pyrimidine-4,6-diamine

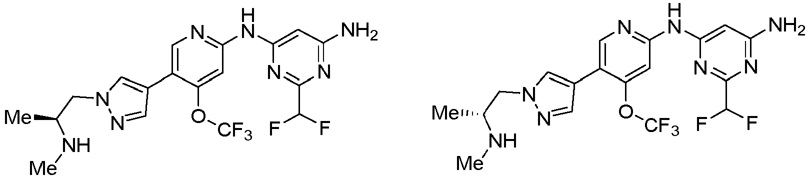

To a mixture of 2-(difluoromethyl)-N4-(2,4-dimethoxybenzyl)-N6-(5-(1-(2-(methyl amino)propyl)-1H-pyrazol-4-yl)-4-(trifluoromethoxy)pyridin-2-yl)pyrimidine-4,6-diamine (Preparation 112, 100 mg, 0.16 mmol) in DCM (2 mL) was added TFA (1 mL) and the reaction stirred at rt for 1 h. The mixture was concentrated *in vacuo* and the residue was purified by HPLC-3, to afford 2-(difluoromethyl)-N4-(5-(1-(2-(methylamino)propyl)-1H-pyrazol-4-yl)-4-(trifluoromethoxy)pyridin-2-yl)pyrimidine-4,6-diamine (50.0 mg, yield 61%).

The product was further purified by Prep-SFC using an AD-H (250 x 4.6mm 5µm) column, mobile Phase:n-Hexane (0.1% diethylamine):EtOH(0.1% diethylamine)=70:30 at 1.0 mL/min to give first eluting (S)-2-(difluoromethyl)-N4-(5-(1-(2-(methylamino)propyl)-1H-pyrazol-4-yl)-4-(trifluoromethoxy)pyridin-2-yl)pyrimidine-4,6-diamine or (R)-2-(difluoromethyl)-N4-(5-(1-(2-(methylamino)propyl)-1H-pyrazol-4-yl)-4-(trifluoromethoxy)pyridin-2-yl)pyrimidine-4,6-diamine, (195)(12.8 mg, yield 25%).

Further elution provided second eluting (R)-2-(difluoromethyl)-N4-(5-(1-(2-(methylamino)propyl)-1H-pyrazol-4-yl)-4-(trifluoromethoxy)pyridin-2-yl)pyrimidine-4,6-diamine or (S)-2-(difluoromethyl)-N4-(5-(1-(2-(methylamino)propyl)-1H-pyrazol-4-yl)-4-(trifluoromethoxy)pyridin-2-yl)pyrimidine-4,6-diamine, (196) (12.1 mg, yield 24%).

LCMS m/z = 459 [M+H]+.

¹H-NMR (400 MHz, DMSO-d₆) *δ*: 10.20 (s, 1H), 8.61 (s, 1H), 8.10 (s, 1H), 7.99 (s, 1H), 7.87 (s, 1H), 7.01 (s, 2H), 6.74 (s, 1H), 6.49 (t, 1H), 4.13-4.03 (m, 1H), 2.90-2.89 (m, 1H), 2.27 (s, 1H), 0.92 (d, 3H).

### Examples 41 and 42: (S)-2-(difluoromethyl)-N4-(4-((1-fluoropropan-2-yl)oxy)-5-(1-methyl-1H-pyrazol-4-yl)pyridin-2-yl)pyrimidine-4,6-diamine and (R)-2-(difluoromethyl)-N4-(4-((1-fluoropropan-2-yl)oxy)-5-(1-methyl-1H-pyrazol-4-yl)pyridin-2-yl)pyrimidine-4,6-diamine

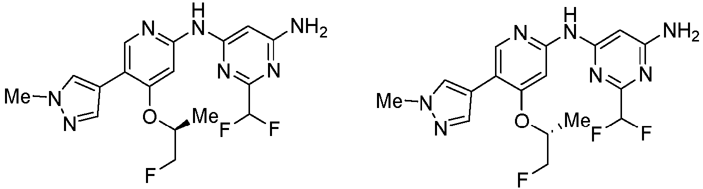

To a solution of 2-(difluoromethyl)-N4-(2,4-dimethoxybenzyl)-N6-(4-((1-fluoropropan-2-yl)oxy)-5-(1-methyl-1H-pyrazol-4-yl)pyridin-2-yl)pyrimidine-4,6-diamine (Preparation 148, 80 mg, 0.147 mmol) in DCM was added TFA (84 mg, 0.736 mmol) and the solution was stirred at rt overnight. The reaction was concentrated *in vacuo* and the crude product was purified by prep HPLC-3 to afford the title product (40 mg) as white solid which was further separated by SFC using an IG 20 x 250 mm, 10µm (Daicel) column, mobile phase CO₂/MeOH (0.2% MeOH/NH₃) = 50/50 at 100 g/min to obtain:
First Eluting Enantiomer 1, (S)-2-(difluoromethyl)-N4-(4-((1-fluoropropan-2-yl)oxy)-5-(1-methyl-1H-pyrazol-4-yl)pyridin-2-yl)pyrimidine-4,6-diamine or (R)-2-(difluoromethyl)-N4-(4-((1-fluoropropan-2-yl)oxy)-5-(1-methyl-1H-pyrazol-4-yl)pyridin-2-yl)pyrimidine-4,6-diamine (87) (3.0 mg, 5% yield) LCMS m/z = 394 [M+H]+.

1H-NMR (400 MHz, DMSO-d₆) δ: 9.74 (s, 1H), 8.37 (s, 1H), 8.03 (s, 1H), 7.89 (s, 1H), 7.44 (s, 1H), 6.89 (d, 3H), 6.50 (t, 1H), 4.77-4.75 (m, 3H), 3.87 (s, 3H), 1.37 (d, 3H).

Second Eluting Enantiomer 2: (R)-2-(difluoromethyl)-N4-(4-((1-fluoropropan-2-yl)oxy)-5-(1-methyl-1H-pyrazol-4-yl)pyridin-2-yl)pyrimidine-4,6-diamine or (S)-2-(difluoromethyl)-N4-(4-((1-fluoropropan-2-yl)oxy)-5-(1-methyl-1H-pyrazol-4-yl)pyridin-2-yl)pyrimidine-4,6-diamine (88) (5.5 mg, 9% yield).

¹H-NMR (400 MHz, DMSO-d₆) δ: 9.75 (s, 1H), 8.37 (s, 1H), 8.03 (s, 1H), 7.89 (s, 1H), 7.44 (s, 1H), 6.89 (d, 3H), 6.50 (t, 1H), 4.77-4.63 (m, 3H), 3.87 (s, 3H), 1.36 (d, 3H).

### Examples 43 and 44: (S)-N4-(4-cyclopropoxy-5-(1-(tetrahydrofuran-3-yl)-1H-pyrazol-4-yl)pyridin-2-yl)-2-(difluoromethyl)pyrimidine-4,6-diamine and (R)-N4-(4-cyclopropoxy-5-(1-(tetrahydrofuran-3-yl)-1H-pyrazol-4-yl)pyridin-2-yl)-2-(difluoro methyl)pyrimidine-4,6-diamine

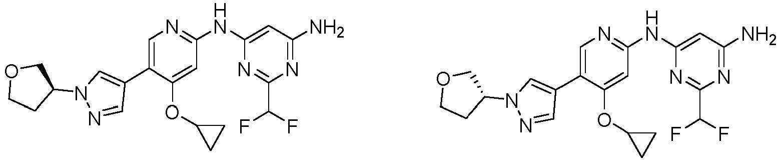

A mixture of N4-(4-cyclopropoxy-5-(1-(tetrahydrofuran-3-yl)-1H-pyrazol-4-yl)pyridin-2-yl)-2-(difluoromethyl)-N6-(2,4-dimethoxybenzyl)pyrimidine-4,6-diamine (Preparation 137, 150 mg, 0.26 mmol) in DCM/TFA (8 mL/2 mL) was stirred at rt for 4 h. The reaction mixture was concentrated *in vacuo.* The residue was purified by prep HPLC-3 to afford N4-(4-cyclo propoxy-5-(1-(tetrahydrofuran-3-yl)-1H-pyrazol-4-yl)pyridin-2-yl)-2-(difluoromethyl) pyrimidine-4,6-diamine (50 mg, 44% yield) as a white solid. LCMS m/z = 430 [M+H]⁺

This was further purified by SFC using an OJ 20 x 250mm, 10µm (Daicel) column, mobile phase: CO₂/MEOH(1% MeOH/NH₃) = 50/50 at 100 g/min; to afford:
First eluting Enantiomer 1: (S)-N4-(4-cyclopropoxy-5-(1-(tetrahydrofuran-3-yl)-1H-pyrazol-4-yl)pyridin-2-yl)-2-(difluoromethyl)pyrimidine-4,6-diamine or (R)-N4-(4-cyclopropoxy-5-(1-(tetrahydrofuran-3-yl)-1H-pyrazol-4-yl)pyridin-2-yl)-2-(difluoromethyl)pyrimidine-4,6-diamine (164) as a white solid (16.5 mg).

1H-NMR (400 MHz, DMSO-d₆) δ: 9.85 (s, 1H), 8.37 (m, 1H), 8.06 (s, 1H), 7.88 (s, 1H), 7.80 (s, 1H), 6.89-6.87 (m, 3H), 6.50 (t, 1H), 5.06-5.04 (m, 1H), 4.01-3.97 (m, 2H), 3.91-3.89 (m, 3H), 2.35-2.26 (m, 2H), 0.89-0.82 (m, 4H) and
Second eluting Enantiomer 2: (R)-N4-(4-cyclopropoxy-5-(1-(tetrahydrofuran-3-yl)-1H-pyrazol-4-yl)pyridin-2-yl)-2-(difluoromethyl)pyrimidine-4,6-diamine or (S)-N4-(4-cyclopropoxy-5-(1-(tetrahydrofuran-3-yl)-1H-pyrazol-4-yl)pyridin-2-yl)-2-(difluoromethyl)pyrimidine-4,6-diamine (165) as a white solid (22.5 mg).

¹H-NMR (400 MHz, DMSO-d₆) δ: 9.85 (s, 1H), 8.37 (m, 1H), 8.06 (s, 1H), 7.88 (s, 1H), 7.80 (s, 1H), 6.89-6.87 (m, 3H), 6.50 (t, 1H), 5.06-5.04 (m, 1H), 4.01-3.97 (m, 2H), 3.91-3.89 (m, 3H), 2.35-2.26 (m, 2H), 0.89-0.82 (m, 4H).

### Example 45: 2-(4-(6-((6-amino-2-(difluoromethyl)pyrimidin-4-yl)amino)-4-methoxypyridin-3-yl)-1H-pyrazol-1-yl)ethan-1-ol trifluoroacetate (59)

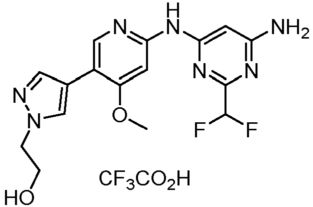

To a solution of 2-(difluoromethyl)-N4-(2,4-dimethoxybenzyl)-N6-(4-methoxy-5-(1-(2-((tetrahydro-2H-pyran-2-yl)oxy)ethyl)-1H-pyrazol-4-yl)pyridin-2-yl)pyrimidine-4,6-diamine (Preparation 122, 68 mg, 0.111 mmol) in DCM (2 mL) was added TFA (1 mL, 12.98 mmol) and the reaction stirred at rt for 10 mins. The reaction mixture was concentrated under reduced pressure, diluted with DMSO and purified by reverse phase chromatography (10-90% MeCN in water with 0.01% TFA) to afford the title compound as a white solid. LCMS m/z = 378 [M+H]⁺; 1H NMR (500 MHz, DMSO-d₆) δ 8.44 (s, 1H), 8.14 (s, 1H), 7.93 (s, 1H), 7.23 (s, 1H), 7.16 (s, 3H), 6.64 (t, 2H), 4.18 (t, 2H), 3.99 (s, 3H), 3.77 (t, 2H).

### Example 46: 2-(difluoromethyl)-N4-(5-(1-methyl-1H-pyrazol-4-yl)-4-(trifluoro methoxy)pyridin-2-yl)pyrimidine-4,6-diamine (95)

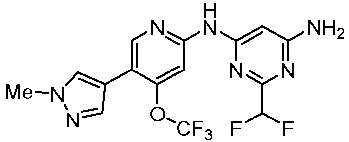

To a mixture of 2-(difluoromethyl)-N4-(2,4-dimethoxybenzyl)-N6-(5-(1-methyl-1H-pyrazol-4-yl)-4-(trifluoromethoxy)pyridin-2-yl)pyrimidine-4,6-diamine (Preparation 109, 50 mg, 0.09 mmol) in DCM (2 mL) was added HCl/dioxane (2 mL, 4M) and the reaction stirred at rt for 1 h. The mixture was concentrated *in vacuo* and the residue was purified by column chromatography on silica gel. The product was further purified by prep HPLC-3 to afford the title compound (24.9 mg, yield 68%). LCMS m/z = 402 [M+H]⁺. ¹H-NMR (400 MHz, DMSO-d₆) *δ* ppm 10.19 (s, 1H), 8.59 (s, 1H), 8.07 (s, 1H), 7.97 (s, 1H), 7.84 (s, 1H), 7.01 (br s, 2H), 6.76 (s, 1H), 6.49 (t, 1H), 3.90 (s, 3H).

### Example 47: 2-(difluoromethyl)-N4-(4-methoxy-5-(1-(pyrrolidin-3-yl)-1H-pyrazol-4-yl)pyridin-2-yl)pyrimidine-4,6-diamine (97)

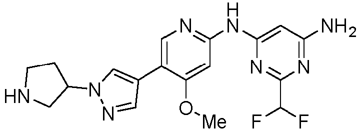

The title compound was obtained as a white solid, 5.1 mg, 7% yield, from tert-butyl 3-(4-(6-((2-(difluoromethyl)-6-((2,4-dimethoxybenzyl)amino)pyrimidin-4-yl)amino)-4-methoxypyridin-3-yl)-1H-pyrazol-1-yl)pyrrolidine-1-carboxylate (Preparation 123), following a similar procedure to that described in Example 46. LCMS m/z = 403 [M+H]⁺.

1H-NMR (400 MHz, DMSO-d₆) δ: 9.67 (s, 1H), 9.51 (s, 1H), 8.55 (s, 1H), 8.40 (s, 1H), 8.04 (s, 1H), 7.45-7.12 (m, 3H), 6.92-6.69 (m, 1H), 6.45 (br s, 1H), 5.27-5.25 (m, 1H), 4.05 (s, 3H), 3.67-3.65 (m, 2H), 3.53-3.38 (m, 2H), 2.42-2.37 (m, 1H), 2.27-2.25 (m, 1H).

### Example 48: 2-(4-(6-((6-amino-2-(difluoromethyl)pyrimidin-4-yl)amino)-4-methoxy pyridin-3-yl)-1H-pyrazol-1-yl)-2-methylpropan-1-ol (122)

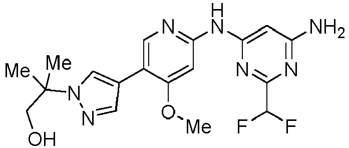

The title compound was obtained as a white solid, 41.2 mg, 48% yield, from 2-(4-(6-((2-(difluoromethyl)-6-((2,4-dimethoxybenzyl)amino)pyrimidin-4-yl)amino)-4-methoxypyridin-3-yl)-1H-pyrazol-1-yl)-2-methylpropan-1-ol (Preparation 124), following a similar procedure to that described in Example 46. LCMS m/z = 406 [M+H]^{+.}

¹H-NMR (500 MHz, DMSO-d₆) δ: 9.80 (s, 1H), 8.37 (s, 1H), 8. 10 (s, 1H), 7.89 (s, 1H), 7.40 (s, 1H), 6.94 (s, 1H), 6.90 (s, 2H), 6.51 (t, 1H), 4.98 (t, 1H), 3.89 (s, 3H), 3.61 (d, 2H), 1.50 (s, 6H).

### Example 49: N4-(5-(1-cyclopropyl-1H-pyrazol-4-yl)-4-(trifluoromethoxy)pyridin-2-yl)-2-(difluoromethyl)pyrimidine-4,6-diamine (154)

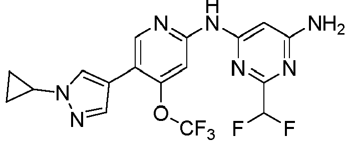

The title compound was obtained, 25.4 mg, 54%, from N4-(5-(1-cyclopropyl-1H-pyrazol-4-yl)-4-(trifluoromethoxy)pyridin-2-yl)-2-(difluoromethyl)-N6-(2,4-dimethoxybenzyl)pyrimidine-4,6-diamine (Preparation 110), following the procedure described in Example 46. LCMS m/z = 428 [M+H]⁺

¹H-NMR (400 MHz, DMSO-d₆) δ: 10.20 (s, 1H), 8.59 (s, 1H), 8.17 (s, 1H), 7.99 (s, 1H), 7.81 (s, 1H), 7.01 (br s, 2H), 6.74 (s, 1H), 6.49 (t, 1H), 3.82-3.78 (m, 1H), 1.10-0.97 (m, 4H).

### Example 50: N4-(5-(1-(azetidin-3-yl)-1H-pyrazol-4-yl)-4-methoxypyridin-2-yl)-2-(difluoromethyl)pyrimidine-4,6-diamine formate (76)

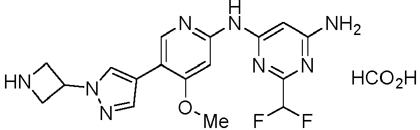

To a solution of tert-butyl 3-(4-(6-((6-amino-2-(difluoromethyl)pyrimidin-4-yl)amino)-4-methoxypyridin-3-yl)-1H-pyrazol-1-yl)azetidine-1-carboxylate (Preparation 156, 50 mg, 0.102 mmol) in DCM (2 mL) was added TFA (1 mL, 13.51 mmol) and the reaction stirred at 25°C for 1 hr. The reaction mixture was concentrated under reduced pressure to give a residue. The residue was purified by prep-HPLC-8, to give the title compound (13.40 mg, 27%) as yellow oil. LCMS m/z = 389 [M+H]⁺

¹H-NMR (400MHz, DMSO-d₆) δ: 9.83 (s, 1H), 8.39 (s, 1H), 8.27 (s, 1H), 8.07 (s, 1H), 7.41 (s, 1H), 6.94 (d, 3H), 6.71-6.33 (m, 1H), 5.39 (s, 2H), 4.22 (s, 4H), 3.91 (s, 3H).

### Examples 51 to 53

The compounds in the following table were prepared from the appropriate Boc protected compound, following a similar procedure to that described in Example 50.

| **Example No** | **Name/Structure/Starting Material (SM)/HPLC/Data** |
|---|---|
| **51** | N4-(5-(1-(azetidin-3-yl)-1H-pyrazol-4-yl)-4-methoxypyridin-2-yl)-2-(fluoromethyl)pyrimidine-4,6-diamine (50) |
| | 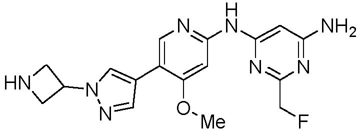 |
| | SM: tert-butyl 3-(4-(6-((6-amino-2-(fluoromethyl)pyrimidin-4-yl)amino)-4-methoxypyridin-3-yl)-1H-pyrazol-1-yl)azetidine-1-carboxylate (Preparation 154) |
| | HPLC-11, 6.00 mg, 50.8% as white solid. |
| | ¹H-NMR (400MHz, DMSO-d₆) δ: 9.60 (s, 1H), 8.31 (s, 1H), 8.16 (s, 1H), 7.92 (s, 1H), 7.43 (s, 1H), 6.75 (s, 1H), 6.62 (br s, 2H), 5.22 - 5.15 (m, 2H), 5.06 (s, 1H), 3.94-3.88 (m, 2H), 3.85 (s, 3H), 3.74-3.65 (m, 2H). |
| **52** | N4-(5-(1-(azetidin-3-yl)-1H-pyrazol-4-yl)-4-cyclopropoxypyridin-2-yl)-2-(difluoromethyl)pyrimidine-4,6-diamine (130) |
| | 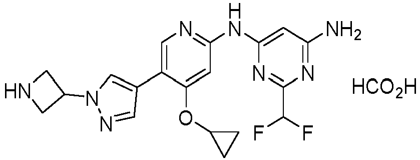 |
| | SM: tert-butyl 3-(4-(6-((6-amino-2-(difluoromethyl)pyrimidin-4-yl)amino)-4-cyclopropoxypyridin-3-yl)-1H-pyrazol-1-yl)azetidine-1-carboxylate (Preparation 158) |
| | HPLC-8, 11.40 mg, yield: 45.7% as a colorless oil. LCMS m/z = 414 [M+H]⁺ |
| | 1H-NMR (400MHz, DMSO-d₆) δ: 9.87 (s, 1H), 8.38 (s, 1H), 8.16 (s, 1H), 8.01 (s, 1H), 7.82 (s, 1H), 6.89 (d, 2H), 6.50 (t, 1H), 5.38 (br s, 1H), 4.17-4.10 (m, 5H), 3.93 (s, 1H), 0.87 (d, 4H). |
| | N4-(4-cyclopropoxy-5-(1-(2-(methylamino)ethyl)-1H-pyrazol-4-yl)pyridin-2-yl)-2-(difluoromethyl)pyrimidine-4,6-diamine (136) |
| **53** | 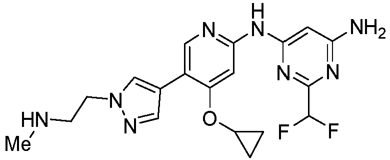 |
| | SM: tert-butyl (2-(4-(6-((6-amino-2-(difluoromethyl)pyrimidin-4-yl)amino)-4-cyclopropoxypyridin-3-yl)-1H-pyrazol-1-yl)ethyl)(methyl)carbamate (Preparation 157) |
| | HPLC-11, gray solid, 4.3 mg, 22.2% yield. LCMS m/z = 417 [M+H]⁺ |
| | 1H-NMR (400MHz, DMSO-d₆) δ: 9.84 (s, 1H), 8.36 (s, 1H), 8.02 (s, 1H), 7.87-7.83 (m, 1H), 7.85 (s, 1H), 6.90 (s, 3H), 6.65-6.35 (m, 1H), 4.18 (t, 2H), 3.96-3.89 (m, 1H), 2.87 (t, 2H), 2.29 (s, 3H), 0.93-0.79 (m, 4H). |

### Example 54: 2-(difluoromethyl)-N4-(4-methoxy-5-(1-(2-(methylamino)ethyl)-1H-pyrazol-4-yl)pyridin-2-yl)pyrimidine-4,6-diamine (80)

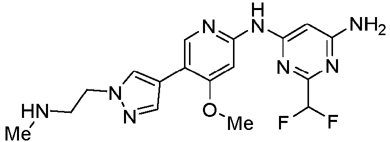

To a solution of tert-butyl (2-(4-(6-((6-amino-2-(difluoromethyl)pyrimidin-4-yl)amino)-4-methoxypyridin-3-yl)-1H-pyrazol-1-yl)ethyl)(methyl)carbamate (Preparation 155, 50 mg, 0.102 mmol) in DCM (2 mL) was added TFA (1 mL, 13.51 mmol) and the reaction was stirred at 25 °C for 1 h. The reaction mixture was concentrated under reduced pressure to give a residue and then adjusted with NH₃.H₂O to pH 5-6 to give the title compound (9.80 mg, 24.6%) as a pale-yellow solid.

¹H-NMR (400MHz, DMSO-d₆) δ: 9.80 (s, 1H), 8.36 (s, 1H), 8.10 (s, 1H), 7.90 (s, 1H), 7.38 (s, 1H), 7.02-6.86 (m, 3H), 6.50 (t, 1H), 4.19 (t, 2H), 3.90 (s, 3H), 2.89 (t, 2H), 2.30 (s, 3H).

### Example 55: N4-(5-(1-(azetidin-3-yl)-1H-pyrazol-4-yl)-4-(oxetan-3-yloxy)pyridin-2-yl)-2-(difluoromethyl)pyrimidine-4,6-diamine formate (168)

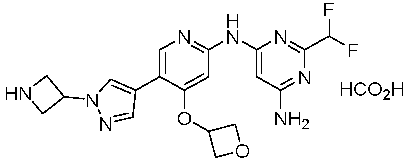

To a solution of tert-butyl 3-(4-(6-((6-amino-2-(difluoromethyl)pyrimidin-4-yl)amino)-4-(oxetan-3-yloxy)pyridin-3-yl)-1H-pyrazol-1-yl)azetidine-1-carboxylate (Preparation 159, 62 mg, 0.117 mmol) in DCM (1.0 mL) was added TFA (0.50 mL, 6.75 mmol) and the reaction stirred at 25 °C for 1 h. The reaction mixture was concentrated under reduced pressure to give a residue and the pH adjusted with NH₃.H₂O to pH 5-6. The residue was purified by prep HPLC-8, to give the title compound (25.0 mg, 42.9%) as colorless oil. LCMS m/z = 431 [M+H]⁺

¹H-NMR (400MHz, DMSO-d₆) δ: 9.81 (s, 1H), 8.45 (s, 1H), 8.34 (s, 2H), 8.13 (s, 1H), 7.05-6.85 (m, 4H), 6.73-6.36 (m, 1H), 5.51-5.31 (m, 2H), 5.00 (t, 2H), 4.83-4.69 (m, 2H), 4.25-4.07 (m, 4H).

### Example 56: 2-(difluoromethyl)-N4-(5-(1-(2-(methylamino)ethyl)-1H-pyrazol-4-yl)-4-(oxetan-3-yloxy)pyridin-2-yl)pyrimidine-4,6-diamine (175)

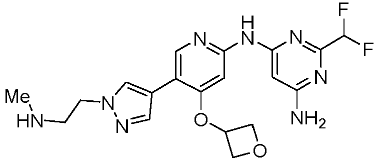

To a solution of tert-butyl (2-(4-(6-((6-amino-2-(difluoromethyl)pyrimidin-4-yl)amino)-4-(oxetan-3-yloxy)pyridin-3-yl)-1H-pyrazol-1-yl)ethyl)(methyl)carbamate (Preparation 160, 26 mg, 0.0488 mmol) in DCM (2 mL) was added TFA (1.54 g, 13.51 mmol). The mixture was stirred at 25°C for 1 hr. The reaction mixture was concentrated under reduced pressure to give a residue and then adjusted with NH₄OH to pH 7-8. The residue was purified by prep HPLC-11 to give the title compound (5.0 mg, 23.7%) as a gray oil. LCMS m/z = 433 [M+H]⁺

¹H-NMR (400MHz, DMSO-d₆) δ: 9.77 (s, 1H), 8.42 (s, 1H), 8.19 (s, 1H), 7.95 (s, 1H), 7.04-6.85 (m, 4H), 6.69-6.35 (m, 1H), 5.36 (quin, 1H), 4.99 (t, 2H), 4.73 (dd, 2H), 4.21 (t, 2H), 2.89 (t, 2H), 2.30 (s, 3H).

### Example 57: N4-(5-(1-(2-amino-2-methylpropyl)-1H-pyrazol-4-yl)-4-methoxy pyridin-2-yl)-2-(difluoromethyl)pyrimidine-4,6-diamine trifluoroacetate (116)

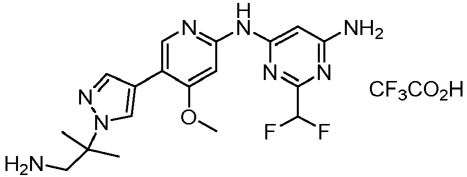

The title compound was obtained, 20 mg, 43.5%, from tert-butyl (1-(4-(6-((2-(difluoromethyl)-6-((2,4-dimethoxybenzyl)amino)pyrimidin-4-yl)amino)-4-methoxypyridin-3-yl)-1H-pyrazol-1-yl)-2-methylpropan-2-yl)carbamate (Preparation 116), following the procedure described in Example 45.

LCMS m/z = 405.2 [M+H]⁺; 1H NMR (500 MHz, DMSO-d₆) δ: 10.16 (s, 1H), 8.47 (s, 1H), 8.27 (s, 1H), 8.05 (s, 1H), 7.90 (s, 3H), 7.44 (s, 1H), 7.02 (s, 2H), 6.75 (s, 1H), 6.58 (t, 1H), 3.95 (s, 3H), 3.36 (d, 2H), 1.62 (s, 6H).

### Example 58: 2-(difluoromethyl)-N4-(5-(1-(1-methylazetidin-3-yl)-1H-pyrazol-4-yl)-4-(oxetan-3-yloxy)pyridin-2-yl)pyrimidine-4,6-diamine (183)

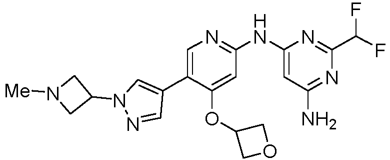

To a solution of N4-(5-(1-(azetidin-3-yl)-1H-pyrazol-4-yl)-4-(oxetan-3-yloxy)pyridin-2-yl)-2-(difluoromethyl)pyrimidine-4,6-diamine formate (Example 55, 15.0 mg, 0.0315 mmol) in MeOH (2.0 mL) and adjusted with AcOH (9.00 uL, 0.157 mmol) to pH 5-6. HCHO (3.07 mg, 0.0378 mmol) was added and the mixture stirred at 25°C for 1 h. Na(CN)BH₃ (2.97 mg, 0.047 mmol) was added and the reaction stirred at 25°C for 1 h. The reaction mixture was filtered and the filtrate was concentrated under reduced pressure to give a residue. The residue was purified by prep-HPLC-11, to give the title compound (9.30 mg, yield: 64.3%) as a gray oil. LCMS m/z = 445 [M+H]⁺

1H-NMR (400MHz, DMSO-d₆) δ: 9.80 (s, 1H), 8.44 (s, 1H), 8.30 (s, 1H), 8.05 (s, 1H), 7.05-6.85 (m, 4H), 6.71-6.39 (m, 1H), 5.37 (q, 1H), 5.12-4.95 (m, 3H), 4.79-4.68 (m, 2H), 3.80-3.69 (m, 2H), 3.43 (t, 2H), 2.36 (s, 3H).

### Example 59: 2-(difluoromethyl)-N4-(4-isopropoxy-5-(1-isopropyl-1H-pyrazol-4-yl)pyridin-2-yl)pyrimidine-4,6-diamine (56)

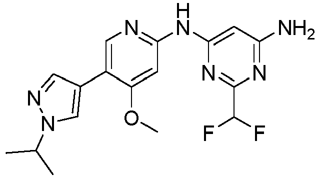

To a solution of 2-(difluoromethyl)pyrimidine-4,6-diamine hydrochloride (Preparation 7, 17.81 mg, 0.111 mmol) and 2-chloro-5-(1-isopropyl-1H-pyrazol-4-yl)-4-methoxypyridine (Preparation 80, 40.0 mg, 0.159 mmol) in dioxane (3 mL) was added BrettPhos Pd, G4 (14.63 mg, 0.0159 mmol) and Cs₂CO₃ (103.55 mg, 0.318 mmol) and the reaction stirred at 90 °C for 2 h under N₂. The cooled reaction mixture was filtered and the filtrate was concentrated under reduced pressure to give a residue. The residue was purified by prep-HPLC-11 to give the title compound (12.50 mg, 21.0%) as brown oil. LCMS m/z = 375 [M+H]⁺

¹H-NMR (400MHz, DMSO-d₆) δ: 9.76 (s, 1H), 8.32 (s, 1H), 8.07 (s, 1H), 7.84 (d, 1H), 7.35 (s, 1H), 6.89 (d, 3H), 6.62-6.33 (m, 1H), 4.49 (td, 1H), 3.86 (s, 3H), 1.41 (d, 6H).

### Example 60: N4-(5-(1-cyclopropyl-1H-pyrazol-4-yl)-4-methoxypyridin-2-yl)-2-(difluoromethyl)pyrimidine-4,6-diamine (52)

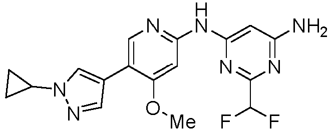

The title compound was obtained as a brown gum (10.50 mg, 19.2%) from 2-chloro-5-(1-cyclopropyl-1H-pyrazol-4-yl)-4-methoxypyridine (Preparation 81) and 2-(difluoromethyl)pyrimidine-4,6-diamine hydrochloride (Preparation 7) following a similar procedure to that described in Example 59. LCMS m/z = 373 [M+H]⁺

¹H-NMR (400MHz, DMSO-d₆) δ: 9.76 (s, 1H), 8.31 (s, 1H), 8.08 (s, 1H), 7.82 (s, 1H), 7.33 (s, 1H), 6.89 (d, 2H), 6.47 (t, 2H), 3.86 (s, 3H), 3.72-3.69 (m, 1H), 1.01 (d, 2H), 0.96-0.91 (m, 2H).

### Example 61: 2-(difluoromethyl)-N4-(4-methoxy-5-(1-(oxetan-3-yl)-1H-pyrazol-4-yl)pyridin-2-yl)pyrimidine-4,6-diamine (78)

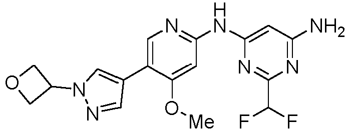

The title compound was obtained, 4.3 mg, 8.17%, from 2-(difluoromethyl)pyrimidine-4,6-diamine hydrochloride (Preparation 7) and 2-chloro-4-methoxy-5-(1-(oxetan-3-yl)-1H-pyrazol-4-yl)pyridine (Preparation 82), following a similar procedure to that described in Example 59. LCMS m/z = 390 [M+H]⁺

¹H-NMR (400MHz, DMSO-d₆) δ: 9.78 (s, 1H), 8.35 (s, 1H), 8.20 (s, 1H), 8.01 (s, 1H), 7.35 (s, 1H), 6.90 (br d, 3H), 6.64 - 6.31 (m, 1H), 5.59 (quin, 1H), 4.90 (d, 4H), 3.86 (s, 3H).

### Examples 62 to 72

To a mixture of the appropriate 2-chloropyridine starting material (SM) and 2-(difluoro methyl)pyrimidine-4,6-diamine hydrochloride (Preparation 7, 1-1.5 equiv.), in dioxane was added BrettPhos Pd, G4 (0.02-0.2 equiv.) and Cs₂CO₃ (2.0-3.0 equiv.) and the reaction was heated at 90°C-100°C until the starting materials had been consumed under N₂. The cooled reaction mixture was concentrated under reduced pressure to give a residue. The residue was purified by prep-HPLC to afford the desired compound.

| **Example No** | **Name/Structure/Starting chloro pyridine (SM)/HPLC/Data** |
|---|---|
| | 2-(difluoromethyl)-N4-(4-methoxy-5-(1-(2-methoxyethyl)-1H-pyrazol-4-yl)pyridin-2-yl)pyrimidine-4,6-diamine (84) |
| **62** | 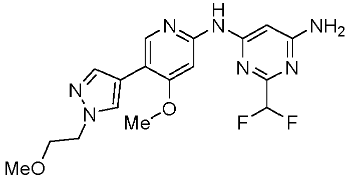 |
| | SM: 2-chloro-4-methoxy-5-(1-(2-methoxyethyl)-1H-pyrazol-4-yl)pyridine (Preparation 51) |
| | Prep HPLC-3, white solid (23.2 mg, 20%). LCMS m/z = 392 [M+H]⁺. |
| | ¹H-NMR (500 MHz, DMSO-d₆) δ: 9.80 (s, 1H), 8.36 (s, 1H), 8. 07 (s, 1H), 7.90 (s, 1H), 7.36 (s, 1H), 6.96 (s, 1H), 6.90 (s, 2H), 6.51 (t, 1H), 4.28 (t, 2H), 3.89 (s, 3H), 3.71 (t, 2H), 3.24 (s, 3H). |
| **63** | 2-(difluoromethyl)-N4-(4-methoxy-5-(1-(oxetan-2-ylmethyl)-1H-pyrazol-4-yl)pyridin-2-yl)pyrimidine-4,6-diamine (111) |
| | 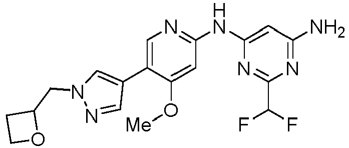 |
| | SM: 2-chloro-4-methoxy-5-(1-(oxetan-2-ylmethyl)-1H-pyrazol-4-yl)pyridine (Preparation 53) |
| | Prep-HPLC-3, 37.3 mg, 26% yield as a white solid. LCMS m/z = 404 [M+H]⁺ |
| | 1H-NMR (400 MHz, DMSO-d₆) δ: 9.80 (s, 1H), 8.37 (s, 1H), 8.11 (s, 1H), 7.93 (s, 1H), 7.38 (s, 1H), 6.95-6.90 (m, 3H), 6.51 (t, 1H), 5.04-4.99 (m, 1H), 4.52-4.31 (m, 4H), 3.89 (s, 3H), 2.67-2.63 (m, 1H), 2.44-2.40 (m, 1H). |
| **64^{A}** | 2-(difluoromethyl)-N4-(4-methoxy-5-(1-(2-((2,2,2-trifluoroethyl)amino) ethyl)-1H-pyrazol-4-yl)pyridin-2-yl)pyrimidine-4,6-diamine (197) |
| | 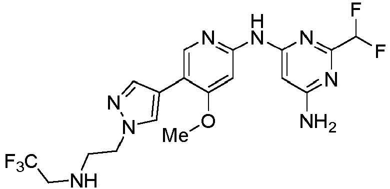 |
| | SM: N-(2-(4-(6-chloro-4-methoxypyridin-3-yl)-1H-pyrazol-1-yl)ethyl)-2,2,2-trifluoroethan-1-amine (Preparation 89) |
| | prep HPLC-3, 4.4 mg, 13% as a white solid. LCMS m/z = 459 [M+H]⁺ |
| | 1H-NMR (400 MHz, DMSO-d₆) δ: 9.79 (s, 1H), 8.35 (s, 1H), 8.11 (s, 1H), 7.91 (s, 1H), 7.36 (s, 1H), 6.96-6.89 (m, 3H), 6.50 (t, 1H), 4.19-4.17 (m, 2H), 3.89 (s, 3H), 3.26-3.23 (m, 2H), 3.04-3.02 (m, 2H). |
| **65^{A}** | 2-(difluoromethyl)-N4-(4-methoxy-5-(1-(1-methylpyrrolidin-3-yl)-1H-pyrazol-4-yl)pyridin-2-yl)pyrimidine-4,6-diamine (140) |
| | 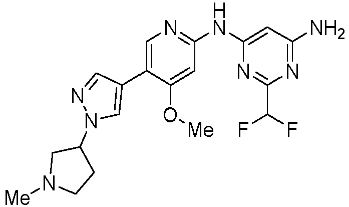 |
| | SM: 2-chloro-4-methoxy-5-(1-(1-methylpyrrolidin-3-yl)-1H-pyrazol-4-yl)pyridine (Preparation 92) |
| | prep HPLC-3, White solid, 41 mg, 24%. LCMS m/z = 417 [M+H]⁺ |
| | 1H-NMR (400 MHz, DMSO-d₆) δ: 9.79 (s, 1H), 8.35 (s, 1H), 8.13 (s, 1H), 7.89 (s, 1H), 7.39 (s, 1H), 6.94-6.89 (m, 3H), 6.50 (t, 1H), 4.94-4.93 (m, 1H), 3.89 (s, 3H), 2.90-2.87 (m, 1H), 2.75-2.72 (m, 2H), 2.55-2.51 (m, 1H), 2.36-2.32 (m, 1H), 2.31 (s, 3H), 2.15-2.12 (m, 1H). |
| **66** | (R)-2-(difluoromethyl)-N4-(4-methoxy-5-(1-((1-methylazetidin-2-yl)methyl)-1H-pyrazol-4-yl)pyridin-2-yl)pyrimidine-4,6-diamine or (S)-2-(difluoromethyl)-N4-(4-methoxy-5-(1-((1-methylazetidin-2-yl)methyl)-1H-pyrazol-4-yl)pyridin-2-yl)pyrimidine-4,6-diamine (138) |
| | 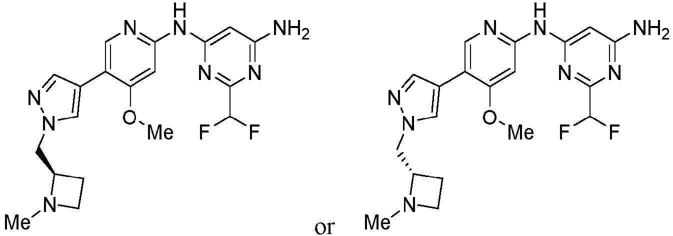 |
| | SM: (R)-2-chloro-4-methoxy-5-(1-((1-methylazetidin-2-yl)methyl)-1H-pyrazol-4-yl)pyridine or (S)-2-chloro-4-methoxy-5-(1-((1-methylazetidin-2-yl)methyl)-1H-pyrazol-4-yl)pyridine (Preparation 94) |
| | prep HPLC-3, White solid, 11 mg, 40%. LCMS m/z = 417 [M+H]⁺ |
| | 1H-NMR (400 MHz, DMSO-d₆) δ: 9.79 (s, 1H), 8.36 (s, 1H), 8.08 (s, 1H), 7.91 (s, 1H), 7.37 (s, 1H), 6.95-6.90 (m, 3H), 6.51 (t, 1H), 4.16 (d, 2H), 3.90 (s, 3H), 3.29-3.21 (m, 2H), 2.70-2.65 (m, 1H), 2.00 (s, 3H), 1.97-1.82 (m, 2H). |
| **67** | (S)-2-(difluoromethyl)-N4-(4-methoxy-5-(1-((1-methylazetidin-2-yl)methyl)-1H-pyrazol-4-yl)pyridin-2-yl)pyrimidine-4,6-diamine or (R)-2-(difluoromethyl)-N4-(4-methoxy-5-(1-((1-methylazetidin-2-yl)methyl)-1H-pyrazol-4-yl)pyridin-2-yl)pyrimidine-4,6-diamine (139) |
| | 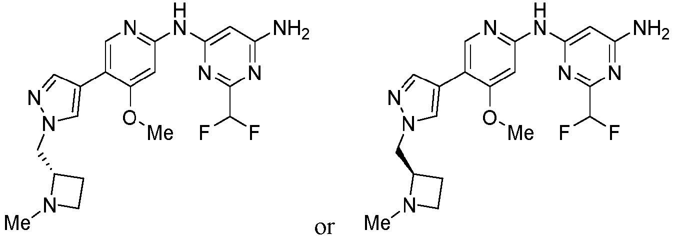 |
| | SM: (S)-2-chloro-4-methoxy-5-(1-((1-methylazetidin-2-yl)methyl)-1H-pyrazol-4-yl)pyridine or (R)-2-chloro-4-methoxy-5-(1-((1-methylazetidin-2-yl)methyl)-1H-pyrazol-4-yl)pyridine (Preparation 95) |
| | prep HPLC-3, White solid, 11.5 mg, 27% yield. LCMS m/z = 417 [M+H]⁺ |
| | 1H-NMR (400 MHz, DMSO-d₆) δ: 9.79 (s, 1H), 8.36 (s, 1H), 8.08 (s, 1H), 7.91 (s, 1H), 7.37 (s, 1H), 6.95-6.90 (m, 3H), 6.51 (t, 1H), 4.16 (d, 2H), 3.90 (s, 3H), 3.29-3.21 (m, 2H), 2.70-2.65 (m, 1H), 2.00 (s, 3H), 1.97-1.82 (m, 2H). |
| **68** | 2-(difluoromethyl)-N4-(4-isopropoxy-5-(1-methyl-1H-pyrazol-4-yl)pyridin-2-yl)pyrimidine-4,6-diamine (55) |
| | 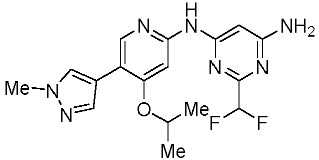 |
| | SM: 2-chloro-4-isopropoxy-5-(1-methyl-1H-pyrazol-4-yl)pyridine (Preparation 44) |
| | HPLC-11, 22.40 mg, 37.1% as a white solid. LCMS m/z = 376 [M+H]⁺ |
| | 1H-NMR (400MHz, MeOH-d₄) δ: 8.28 (s, 1H), 7.95 (s, 1H), 7.85 (s, 1H), 7.50 (s, 1H), 6.70 (s, 1H), 6.46-6.18 (m, 1H), 4.81-4.78 (m, 1H), 3.91 (s, 3H), 1.44 (d, 6H). |
| **69^{B}** | N4-(5-(1-(2-(azetidin-1-yl)ethyl)-1H-pyrazol-4-yl)-4-(difluoromethoxy) pyridin-2-yl)-2-(difluoromethyl)pyrimidine-4,6-diamine (191) |
| | 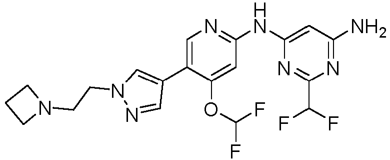 |
| | SM: 5-(1-(2-(azetidin-1-yl)ethyl)-1H-pyrazol-4-yl)-2-chloro-4-(difluoromethoxy)pyridine (Preparation 71) |
| | prep HPLC-3, White solid, 17.9 mg, 13% yield. LCMS m/z = 453 [M+H]⁺ |
| | 1H-NMR (400 MHz, DMSO-d₆) δ: 10.05 (s, 1H), 8.52 (s, 1H), 8.08 (s, 1H), 7.87 (s, 1H), 7.63 (s, 1H), 7.37 (t, 1H), 6.96 (s, 2H), 6.85 (s, 1H), 6.49 (t, 1H), 4.07 (t, 2H), 3.09 (t, 4H), 2.77-2.74 (m, 2H), 1.95-1.89 (m, 2H). |
| **70** | 2-(difluoromethyl)-N4-(4-ethoxy-5-(1-methyl-1H-pyrazol-4-yl)pyridin-2-yl)pyrimidine-4,6-diamine (34) |
| | 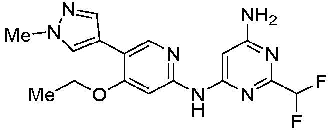 |
| | SM: 2-chloro-4-ethoxy-5-(1-methyl-1H-pyrazol-4-yl)pyridine (Preparation 98) |
| | prep HPLC-3, 232.7 mg, 28%. LCMS m/z = 362 [M+H]⁺. |
| | ¹H-NMR (400 MHz, DMSO-d₆) δ: 9.77 (s, 1H), 8.34 (s, 1H), 8.04 (s, 1H), 7.88 (s, 1H), 7.31 (s, 1H), 6.97-6.91 (m, 3H), 6.50 (t, 1H), 4.17-4.12 (m, 2H), 3.87 (s, 3H), 1.45 (t, 3H). |
| **71** | N4-(4-cyclopropoxy-5-(1-methyl-1H-pyrazol-4-yl)pyridin-2-yl)-2-(difluoromethyl)pyrimidine-4,6-diamine (53) |
| | 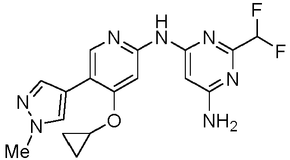 |
| | SM: 2-chloro-4-cyclopropoxy-5-(1-methyl-1H-pyrazol-4-yl)pyridine (Preparation 99) |
| | prep HPLC-3, 20.9 mg, yield 20%. LCMS m/z = 374 [M + H]⁺. |
| | 1H-NMR (500 MHz, DMSO-d₆) δ: 9.82 (s, 1H), 8.34 (s, 1H), 7.97 (s, 1H), 7.83-7.67 (m, 2H), 6.89 (s, 3H), 6.49 (t, 1H), 3.92-3.86 (m, 4H), 0.88-0.83 (m, 4H). |
| **72** | 1-(4-(6-((6-amino-2-(difluoromethyl)pyrimidin-4-yl)amino)-4-((1-fluoropropan-2-yl)oxy)pyridin-3-yl)-1H-pyrazol-1-yl)-2-methylpropan-2-ol (190) |
| | 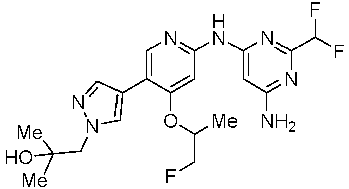 |
| | SM: 1-(4-(6-chloro-4-((1-fluoropropan-2-yl)oxy)pyridin-3-yl)-1H-pyrazol-1-yl)-2-methylpropan-2-ol (Preparation 73) |
| | prep HPLC-3, 63.9 mg, 25% yield as a white solid. LCMS m/z = 452 [M+H]+. |
| | 1H-NMR (400 MHz, DMSO-d₆) δ: 9.76 (s, 1H), 8.39 (s, 1H), 8.06 (s, 1H), 7.90 (s, 1H), 7.46 (s, 1H), 6.90-6.86 (m, 3H), 6.50 (t, 1H), 4.78-4.65 (m, 4H), 4.04 (s, 2H), 1.38-1.36 (m, 3H), 1.09 (s, 6H) |

| | |
|---|---|
| ^{A}-the crude residue was additionally purified by silica gel chromatography before HPLC. ^{B}-K₂CO₃ was used instead of Cs₂CO₃ | |

### Examples 73 and 74: (S)-2-(difluoromethyl)-N4-(4-methoxy-5-(1-(tetrahydrofuran-3-yl)-1H-pyrazol-4-yl)pyridin-2-yl)pyrimidine-4,6-diamine and (R)-2-(difluoromethyl)-N4-(4-methoxy-5-(1-(tetrahydrofuran-3-yl)-1H-pyrazol-4-yl)pyridin-2-yl)pyrimidine-4,6-diamine

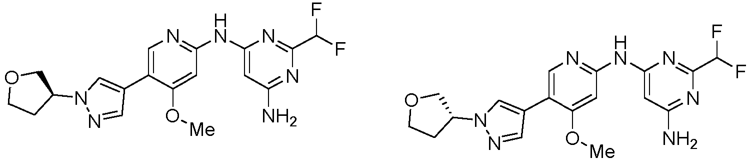

A mixture of 2-chloro-4-methoxy-5-(1-(tetrahydrofuran-3-yl)-1H-pyrazol-4-yl)pyridine (Preparation 54, 160 mg, 0.572 mmol), 2-(difluoromethyl)pyrimidine-4,6-diamine (Preparation 8, 91.5 mg, 0.572 mmol), BrettPhos-Pd G4 (20 mg, 0.0217 mmol) and Cs₂CO₃ (230 mg, 0.708 mmol) in dioxane (3 mL) was stirred at 100 °C for 16 h. The cooled mixture was concentrated *in vacuo* and the residue purified by prep HPLC-3 to provide N4-(4-cyclopropoxy-5-(1-(tetrahydrofuran-3-yl)-1H-pyrazol-4-yl)pyridin-2-yl)-2-(difluoromethyl)pyrimidine-4,6-diamine as a white solid (80 mg, 35%). LCMS m/z = 404 [M+H]⁺.

This was further purified by SFC using an OZ 20*250mm, 10µm (Daicel) column, mobile phase: CO₂/MeOH(0.2% MeOH/NH₃) = 65/35, at 100 g/min to provide:
First eluting Enantiomer 1: (S)-2-(difluoromethyl)-N4-(4-methoxy-5-(1-(tetrahydrofuran-3-yl)-1H-pyrazol-4-yl)pyridin-2-yl)pyrimidine-4,6-diamine or (R)-2-(difluoromethyl)-N4-(4-methoxy-5-(1-(tetrahydrofuran-3-yl)-1H-pyrazol-4-yl)pyridin-2-yl)pyrimidine-4,6-diamine (109), 22 mg.

1H-NMR (500 MHz, DMSO-d₆) δ: 9.79 (s, 1H), 8.36 (s, 1H), 8.13 (s, 1H), 7.93 (s, 1H), 7.39 (s, 1H), 6.95-6.90 (m, 3H), 6.51 (t, 1H), 5.06-5.04 (m, 1H), 4.02-3.82 (m, 7H), 2.36-2.29 (m, 2H).

Second eluting Enantiomer 2: (R)-2-(difluoromethyl)-N4-(4-methoxy-5-(1-(tetrahydrofuran-3-yl)-1H-pyrazol-4-yl)pyridin-2-yl)pyrimidine-4,6-diamine or (S)-2-(difluoromethyl)-N4-(4-methoxy-5-(1-(tetrahydrofuran-3-yl)-1H-pyrazol-4-yl)pyridin-2-yl)pyrimidine-4,6-diamine (110), 29 mg.

1H-NMR (500 MHz, DMSO-d₆) δ: 9.79 (s, 1H), 8.37 (s, 1H), 8.13 (s, 1H), 7.93 (s, 1H), 7.39 (s, 1H), 6.95-6.90 (m, 3H), 6.51 (t, 1H), 5.07-5.04 (m, 1H), 4.02-3.82 (m, 7H), 2.36-2.29 (m, 2H).

### Examples 75 and 76: (S)-2-(difluoromethyl)-N4-(4-methoxy-5-(1-(2-(methylamino) propyl)-1H-pyrazol-4-yl)pyridin-2-yl)pyrimidine-4,6-diamine and (R)-2-(difluoromethyl)-N4-(4-methoxy-5-(1-(2-(methylamino)propyl)-1H-pyrazol-4-yl)pyridin-2-yl)pyrimidine-4,6-diamine

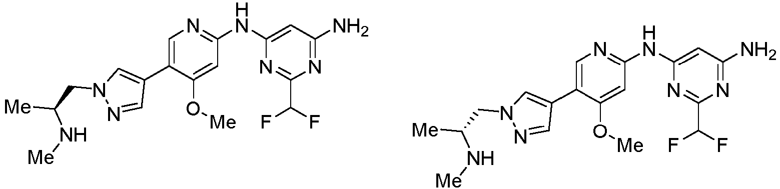

The title compounds were obtained from 1-(4-(6-chloro-4-methoxypyridin-3-yl)-1H-pyrazol-1-yl)-N-methylpropan-2-amine (Preparation 90) and 2-(difluoromethyl)pyrimidine-4,6-diamine (Preparation 8) following the procedure described in Examples 73 and 74.

LCMS m/z = 405 [M+H]⁺.

The compound was further purified by SFC using OZ 20 x 250mm, 10µm (Daicel) column, mobile phase: CO₂/MeOH (0.2%MeOH/NH₃) = 65/35, at 100 g/min to provide:
First eluting Enantiomer 1: (S)-2-(difluoromethyl)-N4-(4-methoxy-5-(1-(2-(methylamino)propyl)-1H-pyrazol-4-yl)pyridin-2-yl)pyrimidine-4,6-diamine or (R)-2-(difluoromethyl)-N4-(4-methoxy-5-(1-(2-(methylamino)propyl)-1H-pyrazol-4-yl)pyridin-2-yl)pyrimidine-4,6-diamine (117), 16 mg.

¹H-NMR (500 MHz, DMSO-d₆) δ: 9.79 (s, 1H), 8.36 (s, 1H), 8.083 (s, 1H), 7.90 (s, 1H), 7.38 (s, 1H), 6.95-6.90 (m, 3H), 6.51 (t, 1H), 4.12-4.07 (m, H),4.00-3.96 (m, 1H), 3.89 (s, 1H), 2.92-2.90 (m, 1H), 2.50 (s, 3 H), 0.93 (d, 3H).

Second eluting Enantiomer 2: (R)-2-(difluoromethyl)-N4-(4-methoxy-5-(1-(2-(methylamino)propyl)-1H-pyrazol-4-yl)pyridin-2-yl)pyrimidine-4,6-diamine or (S)-2-(difluoromethyl)-N4-(4-methoxy-5-(1-(2-(methylamino)propyl)-1H-pyrazol-4-yl)pyridin-2-yl)pyrimidine-4,6-diamine (118), 12 mg
¹H-NMR (500 MHz, DMSO-d₆) δ: 9.79 (s, 1H), 8.36 (s, 1H), 8.08 (s, 1H), 7.90 (s, 1H), 7.38 (s, 1H), 6.95-6.90 (m, 3H), 6.51 (t, 1H), 4.12-4.07 (m, H), 4.00-3.96 (m, 1H), 3.89 (s, 1H), 2.92-2.90 (m, 1H), 2.50 (s, 3 H), 0.93 (d, 3H).

### Example 77: N4-(4-(difluoromethoxy)-5-(1-methyl-1H-pyrazol-4-yl)pyridin-2-yl)-2-(difluoromethyl)pyrimidine-4,6-diamine (69)

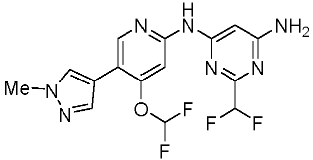

A mixture of 2-chloro-4-(difluoromethoxy)-5-(1-methyl-1H-pyrazol-4-yl)pyridine (Preparation 67, 50 mg, 0.218 mmol), 2-(difluoromethyl)pyrimidine-4,6-diamine (Preparation 8), Pd(t-Bu₃P)₂ (20 mg, 0.050 mmol) and Cs₂CO₃ (100 mg, 0.218 mmol), in dioxane (20 mL) was stirred at 100 °C for 16 h. The cooled mixture was concentrated and purified by prep HPLC-3 to give 18.1 mg, 20.8%, of the title compound as a white oil. LCMS m/z = 399 [M+H]+.

¹H NMR (400 MHz, DMSO-d₆) δ 10.06 (s, 1H), 8.51 (s, 1H), 8.05 (s, 1H), 7.86 (s, 1H), 7.61(s, 1H), 7.37 (t, 1H), 7.19 (s, 2H), 6.86 (s, 1H), 6.50 (t, 1H), 3.89 (s, 3H)

### Examples 78 to 86

The compounds in the following table were prepared following a similar procedure to that described in Example 45 using similar procedures described in preparations 122 and 75

| **Example No** | **Name/Structure/Starting boronate ester (SM)/Data** |
|---|---|
| 78 | 2-(difluoromethyl)-N4-(5-(1-(difluoromethyl)-1H-pyrazol-4-yl)-4-methoxypyridin-2-yl)pyrimidine-4,6-diamine (70) |
| | 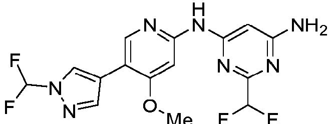 |
| | SM: 1-(difluoromethyl)-4-(4,4,5,5-tetramethyl-1,3,2-dioxaborolan-2-yl)-1H-pyrazole |
| | LCMS m/z = 384 [M+H]⁺ |
| | 1H NMR (500 MHz, DMSO-d₆) δ 10.40 (s, 1H), 8.61 (s, 1H), 8.53 (s, 1H), 8.31 (s, 1H), 7.86 (t, 1H), 7.34 (s, 1H), 7.14 (s, 2H), 6.82 - 6.46 (m, 2H), 3.99 (s, 3H). |
| **79** | 2-(difluoromethyl)-N4-(4-methoxy-5-(1-(trifluoromethyl)-1H-pyrazol-4-yl)pyridin-2-yl)pyrimidine-4,6-diamine (94) |
| | 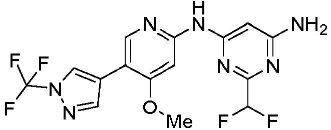 |
| | SM: 4-(4,4,5,5-tetramethyl-1,3,2-dioxaborolan-2-yl)-1-(trifluoromethyl)-1H-pyrazole |
| | LCMS m/z = 402 [M+H]⁺ |
| | 1H NMR (500 MHz, DMSO-d₆) δ 10.13 (s, 1H), 8.76 (s, 1H), 8.52 (d, 1H), 8.46 (s, 1H), 7.42 (s, 1H), 7.03 (s, 2H), 6.84 (s, 1H), 6.57 (t, 1H), 3.96 (d, 3H). |
| **80** | 2-(difluoromethyl)-N4-(5-(1-(2-(dimethylamino)ethyl)-1H-pyrazol-4-yl)-4-methoxypyridin-2-yl)pyrimidine-4,6-diamine (115) |
| | 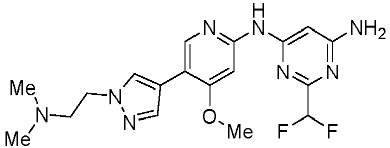 |
| | SM: N,N-dimethyl-2-(4-(4,4,5,5-tetramethyl-1,3,2-dioxaborolan-2-yl)-1H-pyrazol-1-yl)ethan-1-amine |
| | LCMS m/z = 405 [M+H]⁺ |
| | ¹H NMR (500 MHz, DMSO-d₆) δ 10.18 (s, 1H), 9.39 (s, 1H), 8.55 - 8.39 (m, 1H), 8.33 - 8.22 (m, 1H), 8.06 (s, 1H), 7.39 (s, 1H), 7.04 (s, 2H), 6.76 (s, 1H), 6.73 - 6.42 (m, 1H), 4.57 (t, 2H), 3.96 (s, 3H), 3.61 (q, 2H), 2.84 (d, 6H). |
| **81** | N4-(5-(1-(2-(azetidin-1-yl)ethyl)-1H-pyrazol-4-yl)-4-methoxypyridin-2-yl)-2-(difluoromethyl)pyrimidine-4,6-diamine (137) |
| | 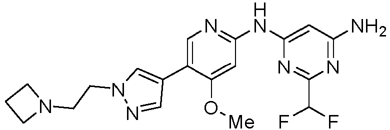 |
| | SM: 1-(2-(azetidin-1-yl)ethyl)-4-(4,4,5,5-tetramethyl-1,3,2-dioxaborolan-2-yl)-1H-pyrazole |
| | LCMS m/z = 417 [M+H]⁺ |
| | ¹H NMR (500 MHz, MeOH-d₄) δ 8.45 (s, 1H), 8.18 (s, 1H), 7.99 (s, 1H), 6.91 (s, 1H), 6.55 (t, 1H), 6.14 (s, 1H), 4.46 (t, 2H), 4.25 - 4.16 (m, 2H), 4.12 (s, 3H), 4.08 - 4.00 (m, 2H), 3.70 (t, 2H), 2.59 - 2.46 (m, 1H), 2.42 - 2.26 (m, 1H). |
| **82** | 2-(difluoromethyl)-N4-(4-methoxy-5-(1-(2-(methyl(2,2,2-trifluoroethyl)amino)ethyl)-1H-pyrazol-4-yl)pyridin-2-yl)pyrimidine-4,6-diamine (199) |
| | 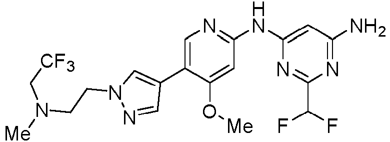 |
| | SM: 2,2,2-trifluoro-N-methyl-N-(2-(4-(4,4,5,5-tetramethyl-1,3,2-dioxaborolan-2-yl)-1H-pyrazol-1-yl)ethyl)ethan-1-amine (Preparation 274) |
| | LCMS m/z = 473 [M+H]⁺ |
| | ¹H NMR (500 MHz, DMSO-d₆) δ 8.42 (s, 1H), 8.18 (s, 1H), 7.94 (s, 1H), 7.23 (s, 1H), 7.16 (s, 3H), 6.81 - 6.49 (m, 2H), 4.25 (t, 2H), 3.99 (s, 3H), 3.24 (q, 2H), 3.01 (t, 2H), 2.41 (s, 3H). |
| **83** | 3-(4-(6-((6-amino-2-(difluoromethyl)pyrimidin-4-yl)amino)-4-methoxypyridin-3-yl)-1H-pyrazol-1-yl)cyclobutan-1-ol (112) |
| | 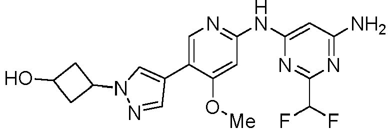 |
| | SM: 1-(3-(benzyloxy)cyclobutyl)-4-(4,4,5,5-tetramethyl-1,3,2-dioxaborolan-2-yl)-1H-pyrazole (Preparation 275) |
| | LCMS m/z = 404 [M+H]⁺ |
| | ¹H NMR (500 MHz, DMSO-*d*₆) δ 8.39 (s, 1H), 8.17 (s, 1H), 7.94 (s, 1H), 7.31 (s, 1H), 7.02 (s, 2H), 6.57 (t, 1H), 5.09 - 4.91 (m, 1H), 4.48 (t, 1H), 3.94 (s, 3H), 2.65 (dt, 3H), 2.45 - 2.27 (m, 2H). |
| **84** | N4-(4-cyclopropoxy-5-(1-(2-(methyl(2,2,2-trifluoroethyl)amino)ethyl)-1H-pyrazol-4-yl)pyridin-2-yl)-2-(difluoromethyl)pyrimidine-4,6-diamine (203) |
| | 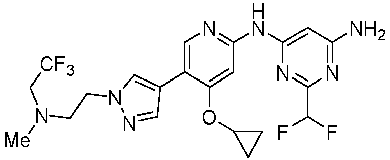 |
| | SM: 2,2,2-trifluoro-N-methyl-N-(2-(4-(4,4,5,5-tetramethyl-1,3,2-dioxaborolan-2-yl)-1H-pyrazol-1-yl)ethyl)ethan-1-amine (Preparation 274) |
| | LCMS m/z = 499 [M+H]⁺ |
| | 1H NMR (500 MHz, DMSO-d₆) δ 10.54 (s, 1H), 8.43 (s, 1H), 8.08 (s, 1H), 7.89 (s, 1H), 7.63 (s, 1H), 7.13 (d, 2H), 6.79 - 6.46 (m, 3H), 4.25 (t, 2H), 4.06 (s, 1H), 3.23 (q, 2H), 3.00 (t, 2H), 2.41 (s, 3H), 1.08 - 0.73 (m, 4H). |
| **85** | N4-(5-(1-(2-(azetidin-1-yl)ethyl)-1H-pyrazol-4-yl)-4-cyclopropoxy pyridin-2-yl)-2-(difluoromethyl)pyrimidine-4,6-diamine (182) |
| | 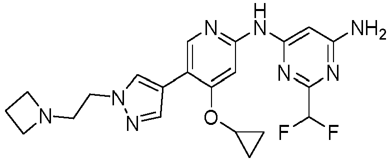 |
| | SM: 1-(2-(azetidin-1-yl)ethyl)-4-(4,4,5,5-tetramethyl-1,3,2-dioxaborolan-2-yl)-1H-pyrazole |
| | LCMS m/z = 443 [M+H]⁺ |
| | 1H NMR (500 MHz, DMSO-d₆) δ 10.14 (s, 1H), 9.68 (s, 1H), 8.43 (s, 1H), 8.10 (s, 1H), 8.01 (s, 1H), 7.80 (s, 1H), 7.05 - 6.93 (m, 2H), 6.74 (s, 1H), 6.56 (t, 1H), 4.41 (t, 2H), 4.01 (dq, 4H), 3.65 (q, 2H), 2.46 - 2.33 (m, 1H), 2.31 - 2.11 (m, 1H), 1.02 - 0.72 (m, 4H). |
| **86** | N4-(5-(1-(2-amino-2-methylpropyl)-1H-pyrazol-4-yl)-4-cyclopropoxy pyridin-2-yl)-2-(difluoromethyl)pyrimidine-4,6-diamine (169) |
| | 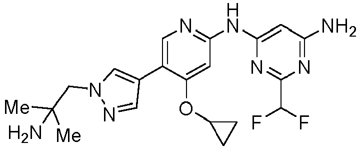 |
| | SM: tert-butyl (2-methyl-1-(4-(4,4,5,5-tetramethyl-1,3,2-dioxaborolan-2-yl)-1H-pyrazol-1-yl)propan-2-yl)carbamate (Preparation 276) |
| | LCMS m/z = 431 [M+H]⁺ |
| | 1H NMR (500 MHz, MeOH-d₄) δ 8.50 (s, 1H), 8.13 (d, 1H), 8.02 (d, 1H), 7.36 (d, 1H), 6.58 (td, 1H), 6.20 (s, 1H), 4.38 (d, 2H), 4.19 (d, 1H), 1.34 (d, 6H), 1.05 (d, 2H), 0.99 (s, 2H). |

### Example 87-89

The title compounds were prepared using a 2-part chemistry protocol using the appropriate fluoropyridine (R-F) and EtOH as outlined in the scheme and table below.

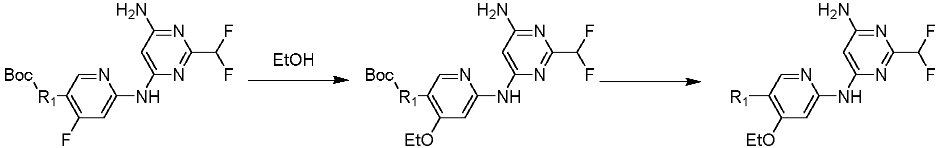

**Part 1.** To a mixture of the appropriate fluoride (R-F, 0.22 mmol, 1 eq.) in THF (1 mL) was added EtOH (0.33 mmol, 1.5 eq.) and t-BuOK (73.9 mg, 0.66 mmol, 3 eq.) and the mixture stirred at 100 °C for 4 h under microwave irradiation. The reaction was quenched with H₂O (3.0 mL) and extracted with EtOAc (3x 10 mL). The combined organics were evaporated to dryness by Speedvac and the residue used in Part 2 without further purification.

**Part 2.** A solution of the compound of Part 1 (crude, 0.22 mmol, 1 equiv.) in DCM (3 mL) and TFA (1 mL) was stirred at 30 °C for 2 h. The volatiles were removed by Speedvac and the residue purified by prep HPLC-4 to give the title compounds.

| **Example No** | **Name/Structure/R-F/Data** |
|---|---|
| **87** | N4-(5-(1-(azetidin-3-yl)-1H-pyrazol-4-yl)-4-ethoxypyridin-2-yl)-2-(difluoromethyl)pyrimidine-4,6-diamine (98) |
| | 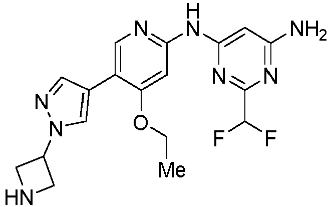 |
| | R-F: tert-butyl 3-(4-(6-((6-amino-2-(difluoromethyl)pyrimidin-4-yl)amino)-4-fluoropyridin-3-yl)-1H-pyrazol-1-yl)azetidine-1-carboxylate (Preparation 168). |
| | Yield: 4%; LCMS m/z = 403 [M+H]⁺. |
| **88** | N4-(5-(1-(2-aminoethyl)-1H-pyrazol-4-yl)-4-ethoxypyridin-2-yl)-2-(difluoromethyl)pyrimidine-4,6-diamine (81) |
| | 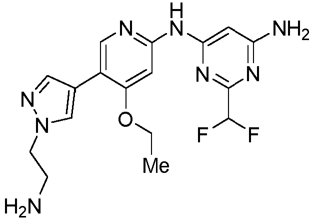 |
| | R-F: tert-butyl (2-(4-(6-((6-amino-2-(difluoromethyl)pyrimidin-4-yl)amino)-4-fluoropyridin-3-yl)-1H-pyrazol-1-yl)ethyl)carbamate (Preparation 169). |
| | Yield: 15%; LCMS m/z = 391 [M+H]⁺. |
| **89** | 2-(difluoromethyl)-N4-(4-ethoxy-5-(1-(2-(methylamino)ethyl)-1H-pyrazol-4-yl)pyridin-2-yl)pyrimidine-4,6-diamine (119) |
| | 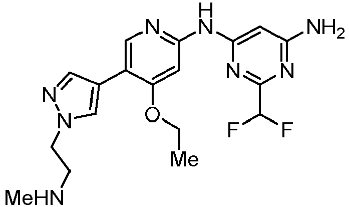 |
| | R-F: tert-butyl (2-(4-(6-((6-amino-2-(difluoromethyl)pyrimidin-4-yl)amino)-4-fluoropyridin-3-yl)-1H-pyrazol-1-yl)ethyl)(methyl)carbamate (Preparation 170). |
| | Yield: 22%; LCMS m/z = 405 [M+H]⁺. |

### Example 90-93

The title compounds were prepared using a 3-part chemistry protocol as outlined below.

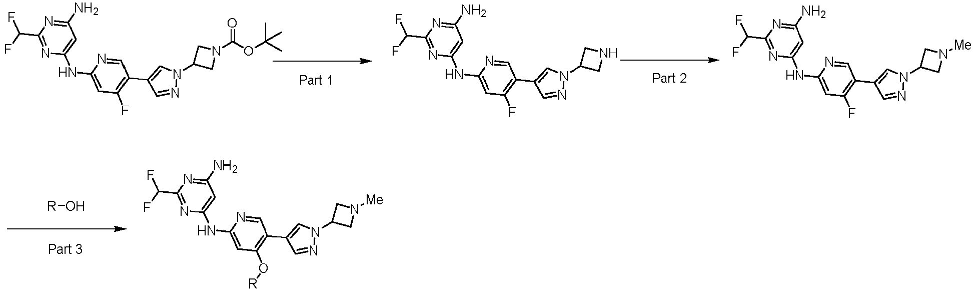

**Part 1.** A solution of tert-butyl 3-(4-(6-((6-amino-2-(difluoromethyl)pyrimidin-4-yl)amino)-4-fluoropyridin-3-yl)-1H-pyrazol-1-yl)azetidine-1-carboxylate (Preparation 168, 283.3 mg, 0.5 mmol, 1 eq.) in DCM (3 mL) and TFA (1 mL) was stirred at 30 °C for 2 h. The solvents were evaporated by Speedvac to afford N4-(5-(1-(azetidin-3-yl)-1H-pyrazol-4-yl)-4-fluoropyridin-2-yl)-2-(difluoromethyl)pyrimidine-4,6-diamine which was used in Part 2 without further purification.

**Part 2.** A solution of N4-(5-(1-(azetidin-3-yl)-1H-pyrazol-4-yl)-4-fluoropyridin-2-yl)-2-(difluoromethyl)pyrimidine-4,6-diamine (Part 1, 188mg, 0.5 mmol, 1 eq) and CH₃CO₂H (150 mg, 2.5 mmol, 5 eq.) and HCHO (48.6 mg, 0.6 mmol, 1.2 eq., 37% purity) in MeOH (5 mL) was stirred at 30 °C for 1 h. To this was added NaBH₃CN (47.1 mg, 0.75 mmol, 1.5 eq.) and the mixture stirred at 30 °C for 1 h. The volatiles were removed *in vacuo* by Speedvac to give 2-(difluoromethyl)-N4-(4-fluoro-5-(1-(1-methylazetidin-3-yl)-1H-pyrazol-4-yl)pyridin-2-yl)pyrimidine-4,6-diamine which was used in Part 3 without further purification.

**Part 3.** To a solution of 2-(difluoromethyl)-N4-(4-fluoro-5-(1-(1-methylazetidin-3-yl)-1H-pyrazol-4-yl)pyridin-2-yl)pyrimidine-4,6-diamine (Part 2, 85.9 mg, 0.22 mmol, 1 eq.) in THF (1 mL) was added the appropriate alcohol (ROH, 0.33 mmol, 1.5 eq.) and t-BuOK (73.9 mg, 0.66 mmol, 3 eq.) and the mixture heated under microwave irradiation at 100 °C for 4 h. The reaction mixtures were evaporated to dryness by Speedvac and the residues purified by prep HPLC-4 to give the title compounds.

| **Example No** | **Name/Structure/ROH/Data** |
|---|---|
| **90** | 2-(difluoromethyl)-N4-(4-methoxy-5-(1-(1-methylazetidin-3-yl)-1H-pyrazol-4-yl)pyridin-2-yl)pyrimidine-4,6-diamine (103) |
| | 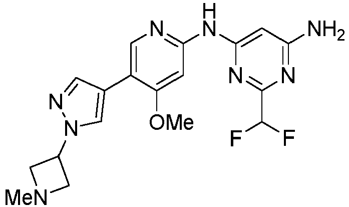 |
| | ROH: Methanol; Yield: 18%; LCMS m/z = 403 [M+H]⁺. |
| **91** | (S)-2-(difluoromethyl)-N4-(5-(1-(1-methylazetidin-3-yl)-1H-pyrazol-4-yl)-4-((tetrahydrofuran-2-yl)methoxy)pyridin-2-yl)pyrimidine-4,6-diamine (201) |
| | 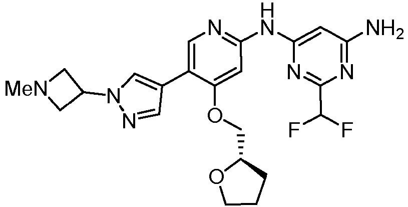 |
| | ROH: (S)-(tetrahydrofuran-2-yl)methanol; Yield: 9%; LCMS m/z = 473 [M+H]⁺. |
| **92** | (R)-2-(difluoromethyl)-N4-(5-(1-(1-methylazetidin-3-yl)-1H-pyrazol-4-yl)-4-((tetrahydrofuran-2-yl)methoxy)pyridin-2-yl)pyrimidine-4,6-diamine (202) |
| | 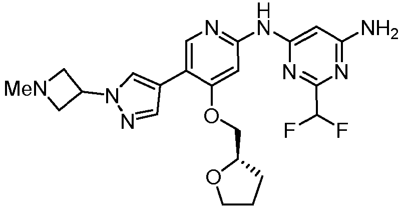 |
| | ROH: (R)-(tetrahydrofuran-2-yl)methanol; Yield: 8%; LCMS m/z = 473 [M+H]⁺. |
| **93** | 2-(difluoromethyl)-N4-(4-ethoxy-5-(1-(1-methylazetidin-3-yl)-1H-pyrazol-4-yl)pyridin-2-yl)pyrimidine-4,6-diamine (141) |
| | 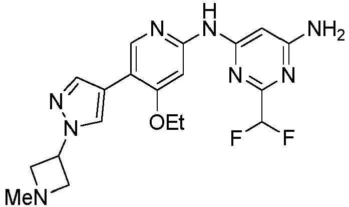 |
| | ROH: Ethanol; Yield: 7%; LCMS m/z = 417 [M+H]⁺. |

### Example 94: 1-(4-(6-((6-amino-2-(difluoromethyl)pyrimidin-4-yl)amino)-4-ethoxy pyridin-3-yl)-1H-pyrazol-1-yl)-2-methylpropan-2-ol (152)

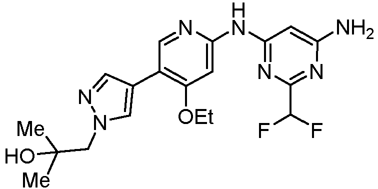

**Part 1.** To a solution of 5-bromo-2-chloro-4-fluoropyridine (315 mg, 1.5 mmol) and 2-methyl-1-(4-(4,4,5,5-tetramethyl-1,3,2-dioxaborolan-2-yl)-1H-pyrazol-1-yl)propan-2-ol (478 mg, 1.8 mmol) in dioxane (10 mL) was added Na₂CO₃ (2.6 mL, 15 mmol, 2M) and Pd(PPh₃)₄ (86.6 mg, 0.075 mmol) under N₂ atmosphere and the mixture stirred at 100 °C for 5.5 h. The reaction was diluted with H₂O (5.0 mL) and extracted with EtOAc (3x 10 mL). The combined organics were evaporated to dryness by Speedvac to afford 1-(4-(6-chloro-4-fluoropyridin-3-yl)-1H-pyrazol-1-yl)-2-methylpropan-2-ol which was used in Part 2 without further purification.

**Part 2.** To a solution of 1-(4-(6-chloro-4-fluoropyridin-3-yl)-1H-pyrazol-1-yl)-2-methylpropan-2-ol (Part 1, 351 mg, 1.3 mmol) and 2-(difluoromethyl)pyrimidine-4,6-diamine (Preparation 8, 416 mg, 2.6 mmol) in t-AmOH (10 mL) was added Cs₂CO₃ (1.27 g, 3.9 mmol) and Brettphos Pd G3 (59 mg, 0.065 mmol) under N₂ and the mixture stirred at 120 °C for 2 h. The reaction mixture was diluted with H₂O (5.0 mL) and extracted with EtOAc (3x 10 mL). The combined organics were evaporated to dryness by Speedvac to afford 1-(4-(6-((6-amino-2-(difluoromethyl)pyrimidin-4-yl)amino)-4-fluoropyridin-3-yl)-1H-pyrazol-1-yl)-2-methylpropan-2-ol which was used in Part 3 without further purification.

**Part 3.** To a mixture of 1-(4-(6-((6-amino-2-(difluoromethyl)pyrimidin-4-yl)amino)-4-fluoropyridin-3-yl)-1H-pyrazol-1-yl)-2-methylpropan-2-ol (Part 2, 86.5 mg, 0.22 mmol) in THF (1 mL) was added EtOH (0.33 mmol), t-BuOK (73.9 mg, 0.66 mmol) and the mixture heated at 100°C for 4 h under microwave irradiation. The reaction was evaporated to dryness by Speedvac and the residue purified by prep HPLC-4 to afford the title compound (23.7 mg, 26%). LCMS m/z = 420 [M+H]⁺.

### Example 95-96

The title compounds were prepared using a 1-step protocol outlined below.

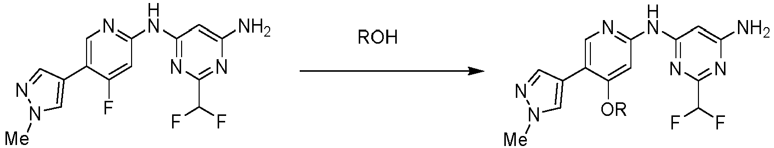

To a mixture of the appropriate alcohol (ROH, 0.33 mmol, 1.5 eq.) in DMF (4 mL) was added NaH (61.6 mg, 1.54 mmol, 7 eq., 60%) and the mixture was stirred at 30 °C for 2 h. 2-(Difluoromethyl)-N4-(4-fluoro-5-(1-methyl-1H-pyrazol-4-yl)pyridin-2-yl)pyrimidine-4,6-diamine (Preparation 171, 73.7 mg, 0.22 mmol, 1 eq.) was added to the mixture and the reaction stirred at 100 °C for 3 h. The reaction was quenched with H₂O (1.0 mL) and evaporated to dryness using a Speedvac. The residues were purified by prep HPLC-4 to give the title compounds.

| **Example No** | **Name/Structure/ROH/Data** |
|---|---|
| **95** | 2-(difluoromethyl)-N4-(5-(1-methyl-1H-pyrazol-4-yl)-4-(oxetan-2-ylmethoxy)pyridin-2-yl)pyrimidine-4,6-diamine (105) |
| | 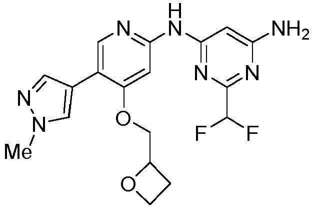 |
| | ROH: oxetan-2-ylmethanol |
| | Yield: 12%; LCMS m/z = 404 [M+H]⁺. |
| **96** | 2-(difluoromethyl)-N4-(5-(1-methyl-1H-pyrazol-4-yl)-4-((tetrahydrofuran-2-yl)methoxy)pyridin-2-yl)pyrimidine-4,6-diamine (143) |
| | 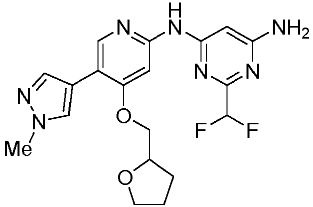 |
| | ROH: (tetrahydrofuran-2-yl)methanol |
| | Yield: 29%; LCMS m/z = 418 [M+H]⁺. |

### Example 97: 2-(difluoromethyl)-N4-(5-(1-methyl-1H-pyrazol-4-yl)-4-((tetrahydro-2H-pyran-2-yl)methoxy)pyridin-2-yl)pyrimidine-4,6-diamine (172)

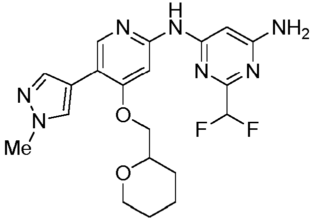

To a solution of 2-(difluoromethyl)-N4-(4-fluoro-5-(1-methyl-1H-pyrazol-4-yl)pyridin-2-yl)pyrimidine-4,6-diamine (Preparation 171, 73.7 mg, 0.22 mmol) in DMA (4 mL) was added (tetrahydro-2H-pyran-2-yl)methanol (38.3 mg, 0.33 mmol) and t-BuOK (73.9 mg, 0.66 mmol) and the mixture stirred at 100 °C for 6 h. The volatiles were removed by Speedvac and the residue purified by prep-HPLC-4 to afford the title compound. Yield: 11%; LCMS m/z = 432 [M+H]⁺.

### Example 98-108

The title compounds were prepared from 2-(difluoromethyl)-N4-(4-fluoro-5-(1-methyl-1H-pyrazol-4-yl)pyridin-2-yl)pyrimidine-4,6-diamine (Preparation 171) and the appropriate alcohol (ROH) using a similar method to that described for Example 97 using the solvent and heating method noted in the table. (Method A = DMA, 100 °C, 6 h; Method B = THF, 100 °C, microwave, 4 h; C = DMA, 100 °C, microwave, 4 h.). The crude products were purified by prep HPLC-4.

| **Example No** | **Name/Structure/Method/ROH/Data** |
|---|---|
| **98** | 3-((2-((6-amino-2-(difluoromethyl)pyrimidin-4-yl)amino)-5-(1-methyl-1H-pyrazol-4-yl)pyridin-4-yl)oxy)propan-1-ol (83) |
| | 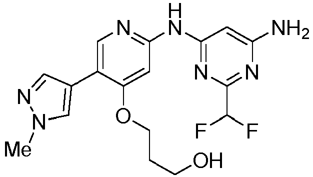 |
| | Method B; ROH: propane-1,3-diol; Yield: 8%; LCMS m/z = 392 [M+H]⁺. |
| **99** | (1r,3r)-3-(((2-((6-amino-2-(difluoromethyl)pyrimidin-4-yl)amino)-5-(1-methyl-1H-pyrazol-4-yl)pyridin-4-yl)oxy)methyl)cyclobutan-1-ol (144) |
| | 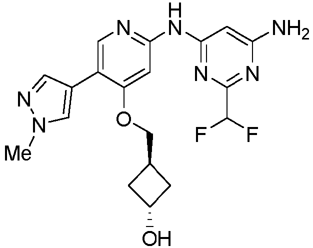 |
| | Method B; ROH: (1r,3r)-3-(hydroxymethyl)cyclobutan-1-ol; Yield: 4%; LCMS m/z = 418 [M+H]⁺. |
| **100** | 3-(((2-((6-amino-2-(difluoromethyl)pyrimidin-4-yl)amino)-5-(1-methyl-1H-pyrazol-4-yl)pyridin-4-yl)oxy)methyl)cyclobutan-1-ol (145) |
| | 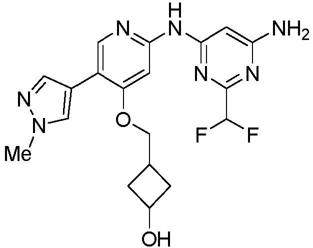 |
| | Method B; ROH: 3-(hydroxymethyl)cyclobutan-1-ol; Yield: 8%; LCMS m/z = 418 [M+H]⁺. |
| **101** | N4-(4-((3-aminobenzyl)oxy)-5-(1-methyl-1H-pyrazol-4-yl)pyridin-2-yl)-2-(difluoromethyl)pyrimidine-4,6-diamine (178) |
| | 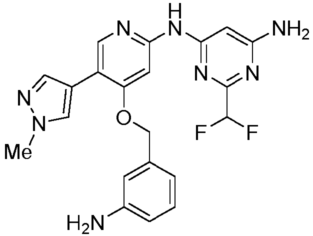 |
| | Method B; ROH: (3-aminophenyl)methanol; Yield: 21%; LCMS m/z = 439 [M+H]⁺. |
| **102** | 2-(difluoromethyl)-N4-(5-(1-methyl-1H-pyrazol-4-yl)-4-(oxetan-3-ylmethoxy)pyridin-2-yl)pyrimidine-4,6-diamine (104) |
| | 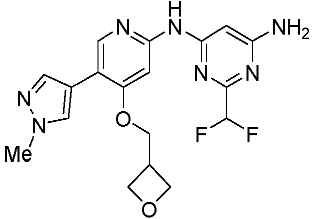 |
| | Method B; ROH: oxetan-3-ylmethanol; Yield: 18%; LCMS m/z = 404 [M+H]⁺. |
| **103** | 2-(difluoromethyl)-N4-(5-(1-methyl-1H-pyrazol-4-yl)-4-((tetrahydro-2H-pyran-3-yl)oxy)pyridin-2-yl)pyrimidine-4,6-diamine (142) |
| | 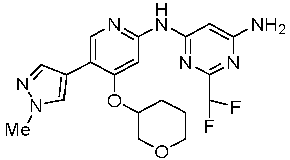 |
| | Method B; ROH: tetrahydro-2H-pyran-3-ol; Yield: 30%; LCMS m/z = 418 [M+H]⁺. |
| **104** | 2-(difluoromethyl)-N4-(5-(1-methyl-1H-pyrazol-4-yl)-4-(2,2,2-trifluoroethoxy)pyridin-2-yl)pyrimidine-4,6-diamine (132) |
| | 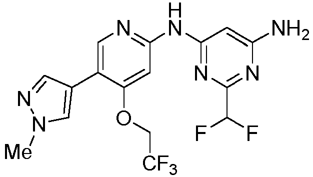 |
| | Method B; ROH: 2,2,2-trifluoroethan-1-ol; Yield: 61%; LCMS m/z = 416 [M+H]⁺. |
| **105** | (R)-2-(difluoromethyl)-N4-(5-(1-methyl-1H-pyrazol-4-yl)-4-((1,1,1-trifluoropropan-2-yl)oxy)pyridin-2-yl)pyrimidine-4,6-diamine (163) |
| | 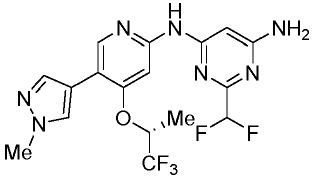 |
| | Method B; ROH: (R)-1,1,1-trifluoropropan-2-ol; Yield: 33%; LCMS m/z = 430 [M+H]⁺. |
| **106** | 2-(difluoromethyl)-N4-(4-((1,1-difluoropropan-2-yl)oxy)-5-(1-methyl-1H-pyrazol-4-yl)pyridin-2-yl)pyrimidine-4,6-diamine (128) |
| | ) 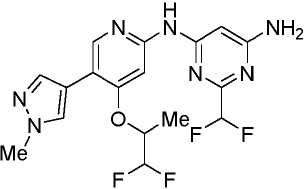 |
| | Method B; ROH: 1,1-difluoropropan-2-ol; Yield: 38%; LCMS m/z = 412 [M+H]⁺. |
| **107** | 2-(difluoromethyl)-N4-(5-(1-methyl-1H-pyrazol-4-yl)-4-((tetrahydrofuran-3-yl)oxy)pyridin-2-yl)pyrimidine-4,6-diamine (106) |
| | 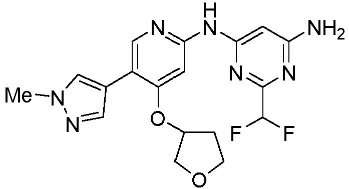 |
| | Method C; ROH: tetrahydrofuran-3-ol; Yield: 11%; LCMS m/z = 404 [M+H]⁺. |
| **108** | 2-(difluoromethyl)-N4-(5-(1-methyl-1H-pyrazol-4-yl)-4-(oxetan-3-yloxy)pyridin-2-yl)pyrimidine-4,6-diamine (77) |
| | 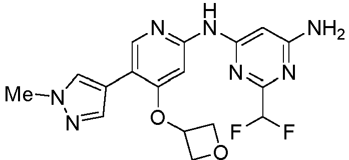 |
| | Method C; ROH: 1 oxetan-3-ol; Yield: 33%; LCMS m/z = 390 [M+H]⁺. |

### Example 109: (1s,3s)-3-((2-((6-amino-2-(difluoromethyl)pyrimidin-4-yl)amino)-5-(1-methyl-1H-pyrazol-4-yl)pyridin-4-yl)oxy)cyclobutan-1-ol (107)

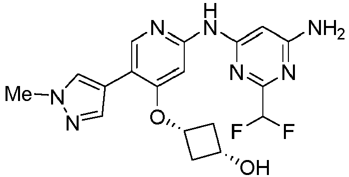

**Part 1.** To a solution of (1s,3s)-cyclobutane-1,3-diol (39.6 mg, 0.45 mmol) in THF (4 mL) was added NaH (60%, 84.0 mg, 2.1 mmol) and the mixture was stirred at 30 °C for 1 h. To this was added 2-chloro-4-fluoro-5-(1-methyl-1H-pyrazol-4-yl)pyridine (Preparation 97, 73.7 mg, 0.3 mmol) and the mixture stirred at 30 °C for 2 h. The reaction was quenched with H₂O (3.0 mL) and extracted with EtOAc (3x 10 mL). The combined organics were concentrated by Speedvac to afford (1s,3s)-3-((2-chloro-5-(1-methyl-1H-pyrazol-4-yl)pyridin-4-yl)oxy)cyclobutan-1-ol which was used in Part 2 without further purification.

**Part 2.** To a solution (1s,3s)-3-((2-chloro-5-(1-methyl-1H-pyrazol-4-yl)pyridin-4-yl)oxy)cyclobutan-1-ol (Part 1, 0.27 mmol) and 2-(difluoromethyl)pyrimidine-4,6-diamine (Preparation 8, 51.8 g, 0.32 mmol) in t-AmOH (3 mL) was added Cs₂CO₃ (263.9 g, 0.81 mmol) and Brettphos Pd G3 (12.24 mg, 0.014 mmol) and the mixture stirred at 120 °C for 2 h under N₂. The reaction was diluted with H₂O (3.0 mL) and extracted with EtOAc (3x 10 mL). The combined organics were evaporated to dryness by Speedvac and the residue purified by prep-HPLC-4 to give the title compound. Yield: 22%; LCMS m/z = 404 [M+H]⁺.

### Example 110: 2-(difluoromethyl)-N4-(4-methoxy-5-(1-methyl-1H-pyrazol-4-yl)pyridin-2-yl)pyrimidine-4,6-diamine (16)

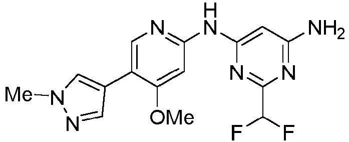

**Part 1.** A mixture of 2-chloro-4-fluoro-5-(1-methyl-1H-pyrazol-4-yl)pyridine (Preparation 97, 73.7 mg, 0.3 mmol), MeOH (14.4 mg, 0.45 mmol) in THF (4 mL) was added t-BuOK (101 mg, 0.9 mmol) and the mixture was stirred at 100 °C for 3 h. The reaction mixture were concentrated by Speedvac to afford 2-chloro-4-methoxy-5-(1-methyl-1H-pyrazol-4-yl)pyridine which was used in Part 2 without further purification.

**Part 2.** To a solution 2-chloro-4-methoxy-5-(1-methyl-1H-pyrazol-4-yl)pyridine (Part 1, 0.27 mmol) and 2-(difluoromethyl)pyrimidine-4,6-diamine (Preparation 8, 51.8 g, 0.32 mmol) in t-AmOH (3 mL) was added Cs₂CO₃ (263.9 g, 0.81 mmol) and Brettphos Pd G3 (12.24 mg, 0.014 mmol) and the mixture was stirred at 120 °C for 2 h under N₂. The reaction was diluted with H₂O (3.0 mL) and extracted with EtOAc (3x 10 mL). The combined organics were evaporated to dryness by Speedvac and the residue purified by prep-HPLC-4 to give the title compound (14 mg, 9%; LCMS m/z = 348 [M+H]⁺.

### Example 112-123

The title compounds were prepared from the appropriate protected starting material (SM) using a similar method to that described for Example 111. Alternative conditions (Method B) using HCl (2N in EtOAc) as noted in the table.

| **Example No** | **Name/Structure/Starting material (SM)/Data** |
|---|---|
| **112** | 6-((6-amino-2-(difluoromethyl)pyrimidin-4-yl)amino)-N-(methyl-d3)-4-(methylamino)nicotinamide (6) |
| | 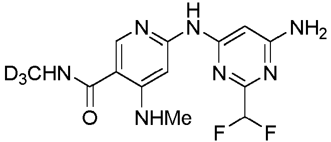 |
| | SM: 6-((2-(difluoromethyl)-6-((2,4-dimethoxybenzyl)amino)pyrimidin-4-yl)amino)-N-(methyl-d3)-4-(methylamino)nicotinamide (Preparation 243) |
| | White solid (101 mg, 30%); LCMS m/z = 327 [M+H]⁺; |
| | ¹H NMR (500 MHz, DMSO-d₆) δ: 13.69 (s, 1H), 11.00 (s, 1H), 8.80 (s, 1H), 8.60 (s, 1H), 8.22 (s, 1H), 7.35 (s, 2H), 6.68 (t, 1H), 6.31 (s, 1H), 6.00 (s, 1H), 2.83 (d, 3H). |
| **113** | 6-((6-amino-2-(difluoromethyl)pyrimidin-4-yl)amino)-4-(cyclopropylamino)-N-(methyl-d3)nicotinamide (31) |
| | 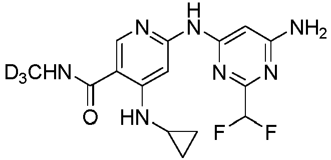 |
| | SM: 4-(cyclopropylamino)-6-((2-(difluoromethyl)-6-((2,4-dimethoxybenzyl)amino)pyrimidin-4-yl)amino)-N-(methyl-d3)nicotinamide (Preparation 244) |
| | White solid (119 mg, 64%); LCMS m/z = 353 [M+H]⁺; |
| | ¹H NMR (500 MHz, DMSO-d₆) δ: 13.92 (s, 1H), 11.08 (s, 1H), 9.00 (s, 1H), 8.67 (s, 1H), 8.36 (s, 1H), 7.38 (s, 2H), 6.95 (s, 1H), 6.73 (t, 1H), 6.19 (s, 1H), 2.60 (s, 1H), 0.90-0.88 (m, 2H), 0.63-0.61 (m, 2H). |
| **114** | 6-((6-amino-2-(difluoromethyl)pyrimidin-4-yl)amino)-N-isopropyl-4-(methylamino)nicotinamide (29) |
| | 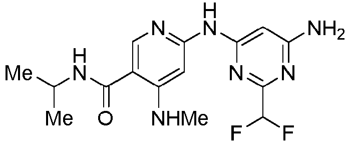 |
| | SM: 6-((2-(difluoromethyl)-6-((2,4-dimethoxybenzyl)amino)pyrimidin-4-yl)amino)-N-isopropyl-4-(methylamino)nicotinamide (Preparation 245) |
| | White solid (223 mg, 65%); LCMS m/z = 352 [M+H]⁺; |
| | ¹H NMR (500 MHz, DMSO-d₆) δ: 11.15 (s, 1H), 8.83 (s, 1H), 8.55 (s, 1H), 8.42-8.26 (m, 1H), 7.43 (s, 2H), 6.76 (t, 1H), 6.41 (s, 1H), 6.10 (s, 1H), 4.07-4.05 (m, 1H), 2.91 (d, 3H), 1.17 (dd, 6H). |
| **115** | 6-((6-amino-2-(difluoromethyl)pyrimidin-4-yl)amino)-4-(cyclopropylamino)-N-isopropylnicotinamide (63) |
| | 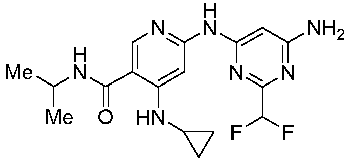 |
| | SM: 4-(cyclopropylamino)-6-((2-(difluoromethyl)-6-((2,4-dimethoxybenzyl)amino)pyrimidin-4-yl)amino)-N-isopropylnicotinamide (Preparation 246) |
| | White solid (41 mg, 48%); LCMS m/z = 378 [M+H]⁺; |
| | ¹H NMR (500 MHz, DMSO-d₆) δ: 13.93 (s, 1H), 11.14 (s, 1H), 9.05 (d, 1H), 8.56 (s, 1H), 8.39 (d, 1H), 7.42 (s, 2H), 6.75 (t, 1H), 6.25 (d, 1H), 5.91 (ddt, 1H), 5.35-5.15 (m, 1H), 4.05 (tq, 1H), 3.96 (s, 1H), 1.17 (dd, 6H), 0.89 (d, 1H), 0.63 (s, 1H). |
| **116** | 6-((6-amino-2-(difluoromethyl)pyrimidin-4-yl)amino)-N-(cyanomethyl)-4-(methylamino)nicotinamide (22) |
| | 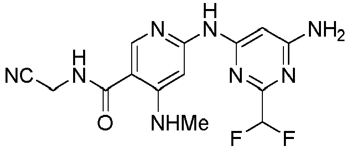 |
| | SM: N-(cyanomethyl)-6-((2-(difluoromethyl)-6-((2,4-dimethoxybenzyl) amino)pyrimidin-4-yl)amino)-4-(methylamino)nicotinamide (Preparation 247) |
| | Yield: 4 mg, 19%; LCMS m/z = 349 [M+H]⁺; |
| | ¹H NMR (500 MHz, MeOH-d₄) δ: 8.34 (d, 1H), 6.53 (t, 1H), 6.21 (s, 1H), 6.05 (s, 1H), 4.31 (s, 2H), 3.01 (s, 3H). |
| **117** | 6-((6-amino-2-(difluoromethyl)pyrimidin-4-yl)amino)-4-(methylamino)nicotinamide (2) |
| | 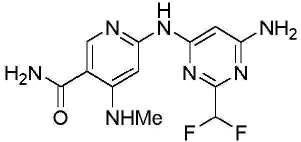 |
| | SM: 6-((2-(difluoromethyl)-6-((2,4-dimethoxybenzyl)amino)pyrimidin-4-yl)amino)-4-(methylamino)nicotinamide (Preparation 248) |
| | White solid (7 mg, 22%); LCMS m/z = 310 [M+H]⁺; |
| | ¹H NMR (500 MHz, MeOH-d₄) δ: 8.39 (s, 1H), 6.52 (t, 1H), 6.25 (s, 1H), 6.08 (s, 1H), 2.99 (s, 3H). |
| **118** | (S)-6-((6-amino-2-(1-fluoroethyl)pyrimidin-4-yl)amino)-4-(cyclopropyl amino)-N-methylnicotinamide (15) |
| | 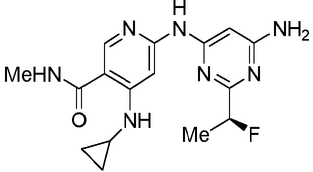 |
| | SM: (S)-4-(cyclopropylamino)-6-((6-((2,4-dimethoxybenzyl)amino)-2-(1-fluoroethyl)pyrimidin-4-yl)amino)-N-methylnicotinamide (Preparation 254) |
| | White solid (7.5 mg, 36%); LCMS m/z = 346 [M+H]⁺; |
| | ¹H NMR (500 MHz, DMSO-d₆) δ: 9.56-9.49 (m, 1H), 8.50-8.42 (m, 1H), 8.30-8.28 (m, 2H), 7.48 (s, 0.5H), 7.06 (s, 0.5H), 6.63-6.60 (m, 2H), 5.35-5.14 (m, 2H), 3.81 (m, 1H), 2.73-2.70 (m, 3H), 1.59-1.53 (m, 3H), 0.79-0.77 (m, 1H), 0.47 (s, 1H). |
| **119** | (R)-6-((6-amino-2-(1-fluoroethyl)pyrimidin-4-yl)amino)-4-(ethylamino)-N-methylnicotinamide (10) |
| | 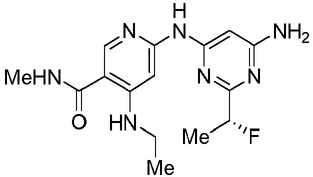 |
| | SM: (R)-6-((6-((2,4-dimethoxybenzyl)amino)-2-(1-fluoroethyl)pyrimidin-4-yl)amino)-4-(ethylamino)-N-methylnicotinamide (Preparation 250). |
| | Method B; Yellow solid (23 mg, 67%); LCMS m/z = 334 [M+H]⁺; |
| | ¹H NMR (400 MHz, DMSO-d₆) δ: 9.47 (s, 1H), 8.28-8.25 (m, 3H), 7.12 (s, 1H), 6.62-6.60 (m, 3H), 5.38-5.21 (m, 1H), 3.17-3.11 (m, 2H), 2.72 (d, 3H), 1.60-1.53 (m, 3H), 1.20 (t, 3H). |
| **120** | (S)-6-((6-amino-2-(1-fluoroethyl)pyrimidin-4-yl)amino)-4-(ethylamino)-N-methylnicotinamide (11) |
| | 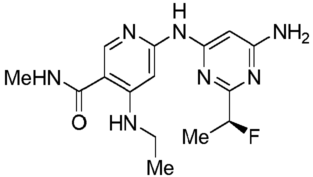 |
| | SM: (S)-6-((6-((2,4-dimethoxybenzyl)amino)-2-(1-fluoroethyl)pyrimidin-4-yl)amino)-4-(ethylamino)-N-methylnicotinamide (Preparation 251). |
| | Method B; Yellow solid (15 mg, 28%); LCMS m/z = 334 [M+H]⁺; |
| | ¹H NMR (500 MHz, DMSO-d₆) δ: 9.47 (s, 1H), 8.28-8.25 (m, 3H), 7.12 (s, 1H), 6.62-6.60 (m, 3H), 5.29 (dq, 1H), 3.13 (q, 2H), 2.71 (d, 2H), 1.60-1.53 (m, 3H), 1.19 (t, 1H). |
| **121** | (R)-6-((6-amino-2-(1-fluoroethyl)pyrimidin-4-yl)amino)-4-(isopropylamino)-N-methylnicotinamide (20) |
| | 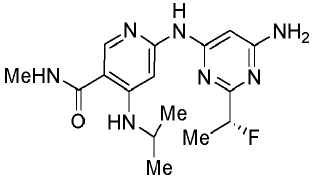 |
| | SM: (R)-6-((6-((2,4-dimethoxybenzyl)amino)-2-(1-fluoroethyl)pyrimidin-4-yl)amino)-4-(isopropylamino)-N-methylnicotinamide (Preparation 249). |
| | White solid, 13.2 mg, 37%. LCMS m/z = 348 [M+H]⁺; |
| | 1H-NMR (400 MHz, DMSO-d₆) δ ppm 9.46 (s, 1H), 8.34-8.24 (m, 3H), 7.19 (s, 1H), 6.62 (s, 2H), 6.56 (s, 1H), 5.36-5.20 (m, 1H), 3.59-3.55 (m, 1H), 2.71 (d, 3H), 1.56 (dd, 3H), 1.20 (d, 6H). |
| **122** | (S)-6-((6-amino-2-(1-fluoroethyl)pyrimidin-4-yl)amino)-N-methyl-4-(methylamino)nicotinamide (3) |
| | 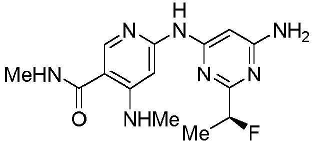 |
| | SM: (S)-6-((6-((2,4-dimethoxybenzyl)amino)-2-(1-fluoroethyl)pyrimidin-4-yl)amino)-4-(methylamino)-N-methylnicotinamide (Preparation 252). |
| | Method B; Yellow solid (10 mg, 19%); LCMS m/z = 320 [M+H]⁺; |
| | ¹H NMR (400 MHz, DMSO-d₆) δ: 9.49 (s, 1H), 8.27-8.16 (m, 3H), 7.07 (s, 1H), 6.63-6.61 (m, 3H), 5.30 (dq, 1H), 2.78 (d, 3H), 2.71 (d, 3H), 1.61-1.53 (m, 3H). |
| | (S)-6-((6-amino-2-(1-fluoroethyl)pyrimidin-4-yl)amino)-4-(isopropylamino)-N-methylnicotinamide (21) |
| **123** | 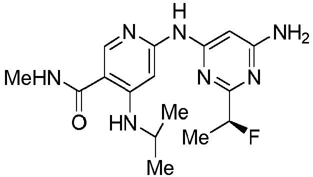 |
| | SM: (S)-6-((6-((2,4-dimethoxybenzyl)amino)-2-(1-fluoroethyl)pyrimidin-4-yl)amino)-4-(isopropylamino)-N-methylnicotinamide (Preparation 253). |
| | Method B; Yellow solid (16.7 mg, 27%); LCMS m/z = 348 [M+H]⁺; |
| | ¹H NMR (500 MHz, DMSO-d₆) δ: 9.47 (s, 1H), 8.34-8.25 (m, 3H), 7.19 (s, 1H), 6.62 (s, 2H), 6.56 (s, 1H), 5.29 (dq, 1H), 3.59-3.56 (m, 1H), 2.71 (d, 3H), 1.59-1.53 (m, 3H), 1.21-1.19 (m, 6H). |

### Example 124: 6-((6-amino-2-(difluoromethyl)pyrimidin-4-yl)amino)-N-cyclopropyl-4-(cyclopropylamino)nicotinamide (57)

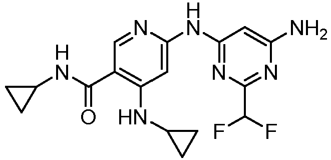

A mixture of 6-chloro-N-cyclopropyl-4-(cyclopropylamino)nicotinamide (Preparation 204, 65 mg, 0.258 mmol), 2-(difluoromethyl)pyrimidine-4,6-diamine (Preparation 8), Pd₂(dba)₃ (47.2 mg, 0.052 mmol), Xantphos (29.8 mg, 0.52 mmol) and Cs₂CO₃ (251, mg, 0.774 mmol) in dioxane (10 mL) was stirred at 100 °C for 16 h under N₂. The reaction mixture was evaporated to dryness *in vacuo* and the residue purified by column chromatography on silica gel (1: 1 EtOAc/PE) to afford the title compound as a white solid (15 mg, 15%). LCMS m/z = 376 [M+H]⁺. ¹H NMR (400 MHz, DMSO-d₆) δ: 9.78 (s, 1H), 8.41 (s, 1H), 8.33 (d, 1H), 8.29 (s, 1H), 7.44 (s, 1H), 6.89 (s, 2H), 6.78 (s, 1H), 6.49 (t, 1H), 2.77-2.75 (m, 1H), 2.45-2.40 (m, 1H), 0.82-0.78 (m, 2H), 0.67-0.65 (m, 2H), 0.55-0.47 (m, 4H).

### Example 125: (S)-6-((6-amino-2-(difluoromethyl)pyrimidin-4-yl)amino)-4-((1-fluoro propan-2-yl)amino)-N-methylnicotinamide (48)

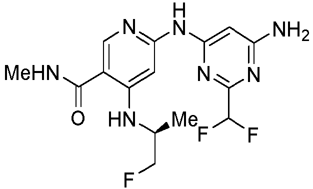

A mixture of (S)-6-chloro-4-((1-fluoropropan-2-yl)amino)-N-methylnicotinamide (Preparation 186, 150 mg, 0.610 mmol), 2-(difluoromethyl)pyrimidine-4,6-diamine (Preparation 8, 117 mg, 0.731 mmol), Cs₂CO₃ (397 mg, 1.22 mmol) and Pd(^{t}Bu₃P)₂ (155 mg, 0.305 mmol) in dioxane (4 mL) was stirred at 100 °C for 16 h under N₂. The reaction mixture was purified by column chromatography on silica gel (10:1 DCM/MeOH) followed by Prep HPLC-3 to afford the title compound as a white solid (41 mg, 18%). LCMS m/z = 370 [M+H]⁺. ¹H NMR (400 MHz, DMSO-d₆) δ: 9.68 (s, 1H), 8.56-8.54 (m, 1H), 8.34-8.33 (m, 2H), 7.09 (s, 1H), 6.91 (s, 2H), 6.79 (s, 1H), 6.62-6.35 (m, 1H), 4.54-4.40 (m, 2H), 3.78-3.72 (m, 1H), 2.73-2.72 (m, 3H), 1.22 (d, 3H).

### Example 126: (R)-6-((6-amino-2-(difluoromethyl)pyrimidin-4-yl)amino)-4-((1-fluoropropan-2-yl)amino)-N-methylnicotinamide (49)

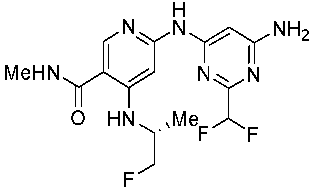

A mixture of (R)-6-chloro-4-((1-fluoropropan-2-yl)amino)-N-methylnicotinamide (Preparation 190, 75 mg, 0.305 mmol), 2-(difluoromethyl)pyrimidine-4,6-diamine (Preparation 8, 48.8 mg, 0.305 mmol), Cs₂CO₃ (298 mg, 0.92 mmol) and Pd(^{t}Bu₃P)₂ (31.2 mg, 0.061 mmol) in dioxane (2 mL) was stirred at 100 °C for 3 h under N₂. The reaction mixture was purified by column chromatography on silica gel (10:1 DCM/MeOH) followed by Prep HPLC-3, to afford the title compound as a white solid (22.6 mg, 20%). LCMS m/z = 370 [M+H]⁺. ¹H NMR (400 MHz, DMSO-d₆) δ: 9.69 (s, 1H), 8.56 (d, 1H), 8.35-8.33 (m, 2H), 7.10 (s, 1H), 6.92 (s, 2H), 6.80 (s, 1H), 6.49 (t, 1H), 4.55-4.41 (m, 2H), 3.77-3.71 (m, 1H), 2.73 (d, 3H), 1.30-1.21 (m, 3H).

### Example 127-146

The title compounds were prepared from the appropriate 2-chloropyridine (RCl) and pyrimidine-4,6-diamine (RNH₂) using a similar method to that described for Example 126.

| **Example No.** | **Name/Structure/RCl/RNH₂/HPLC/Data** |
|---|---|
| **127** | (R)-6-((6-amino-2-(difluoromethyl)pyrimidin-4-yl)amino)-4-((1,1-difluoro propan-2-yl)amino)-N-methylnicotinamide (73) |
| | 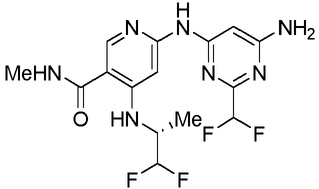 |
| | RCl: (R)-6-chloro-4-((1,1-difluoropropan-2-yl)amino)-N-methylnicotinamide (Preparation 188); RNH₂: 2-(difluoromethyl)pyrimidine-4,6-diamine (Preparation 8) |
| | Prep HPLC-3, 34.7 mg, 39%; LCMS m/z = 388 [M+H]⁺. |
| | ¹H NMR (400 MHz, DMSO-d₆) δ: 9.73 (s, 1H), 8.68 (d, 1H), 8.41 (d, 1H), 8.37 (s, 1H), 7.18 (s, 1H), 6.94 (s, 1H), 6.79 (s, 1H), 6.36 (t, 1H), 6.01 (t, 1H), 3.88-3.85 (m, 1H), 3.78-3.70 (m, 1H), 2.73 (d, 3H), 1.24 (d, 3H). |
| **128** | (S)-6-((6-amino-2-(difluoromethyl)pyrimidin-4-yl)amino)-4-((1,1-difluoropropan-2-yl)amino)-N-methylnicotinamide (74) |
| | 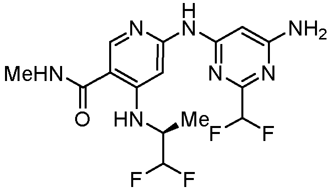 |
| | RCl: (S)-6-chloro-4-((1,1-difluoropropan-2-yl)amino)-N-methylnicotinamide (Preparation 189); RNH₂: 2-(difluoromethyl)pyrimidine-4,6-diamine (Preparation 8) |
| | Prep HPLC-3, 8.2 mg, 7%; LCMS m/z = 388 [M+H]⁺. |
| | ¹H NMR (400 MHz, DMSO-d₆) δ: 9.72 (s, 1H), 8.69-8.67 (m, 1H), 8.41-8.36 (m, 2H), 7.18 (s, H), 6.93 (s, 2H), 6.79 (s, 1H), 6.63-6.01 (m, 2H), 3.87-3.85 (m, 1H), 2.73 (d, 3H), 1.24 (d, 3H). |
| **129** | 6-((6-amino-2-(difluoromethyl)pyrimidin-4-yl)amino)-N-(cyclopropylmethyl)-4-(isopropylamino)nicotinamide (86) |
| | 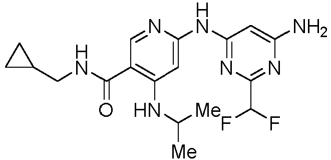 |
| | RCl: 6-chloro-N-(cyclopropylmethyl)-4-(isopropylamino)nicotinamide (Preparation 215); RNH₂: 2-(difluoromethyl)pyrimidine-4,6-diamine (Preparation 8) |
| | Prep HPLC-3, 15.5 mg, 13%; LCMS m/z = 392 [M+H]⁺; |
| | ¹H NMR (400 MHz, DMSO-d₆) δ: 9.69 (s, 1H), 8.45-8.43 (m, 1H), 8.42 (s, 1H), 8.31-8.29 (m, 1H), 7.09 (s, 1H), 6.89 (s, 2H),6.78 (s, 1H), 6.48 (t, 1H), 3.58-3.54 (m, 1H), 3.09-3.07 (m, 2 H), 1.20 (d, 6 H), 1.05-0.98 (m, 1H), 0.45-0.40 (m, 2H), 0.21-0.20 (m, 2H). |
| **130** | 6-((6-amino-2-(difluoromethyl)pyrimidin-4-yl)amino)-4-(ethylamino)-N-(oxazol-4-ylmethyl)nicotinamide (113) |
| | 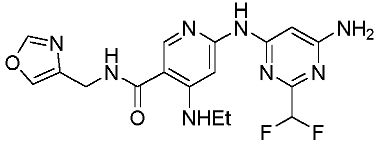 |
| | RCl: 6-chloro-4-(ethylamino)-N-(oxazol-4-ylmethyl)nicotinamide (Preparation 200); RNH₂: 2-(difluoromethyl)pyrimidine-4,6-diamine (Preparation 8) |
| | Prep HPLC-3, 31.2 mg, 22%; LCMS m/z = 404 [M+H]⁺. |
| | ¹H NMR (500 MHz, DMSO-d₆) δ: 9.69 (s, 1H), 8.80-8.76 (m, 1H), 8.38 (s, 1H), 8.31 (s, 1H), 8.23-8.22(m, 1H), 7.96 (s, 1H), 7.01(s, 1H), 6.89 (s, 2H), 6.81(s, 1H), 6.48 (t, 1H), 4.32-4.31(m, 2H), 3.15-3.12 (m, 2H), 3.09-3.07(m, 2 H), 1.21-1.18 (m, 3H). |
| **131** | 6-((6-amino-2-(difluoromethyl)pyrimidin-4-yl)amino)-N-cyclopropyl-4-(methylamino)nicotinamide (24) |
| | 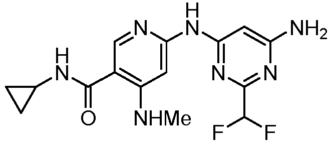 |
| | RCl: 6-chloro-N-cyclopropyl-4-(methylamino)nicotinamide (Preparation 182); RNH₂: 2-(difluoromethyl)pyrimidine-4,6-diamine (Preparation 8) |
| | Prep HPLC-3, 10.3 mg, 14%; LCMS m/z = 350 [M+H]⁺; |
| | ¹H NMR (400 MHz, DMSO-d₆) δ: 9.68 (s, 1H), 8.28 (s, 1H), 8.24(s,1H), 8.13(s, 1H), 6.94-6.84 (m, 4H), 6.49 (t, 1H), 2.78 (d, 4H), 0.67-0.65(m, 2H), 0.54-0.53(m, 2H). |
| **132** | 6-((6-amino-2-(fluoromethyl)pyrimidin-4-yl)amino)-N-cyclopropyl-4-(methylamino)nicotinamide (7) |
| | 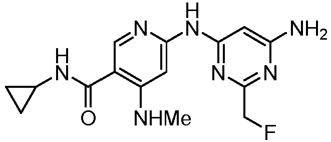 |
| | RCl: 6-chloro-N-cyclopropyl-4-(methylamino)nicotinamide (Preparation 182); RNH₂: 2-(fluoromethyl)pyrimidine-4,6-diamine (Preparation 18) |
| | Prep HPLC-3, 6.5 mg, 14%; LCMS m/z = 332 [M+H]⁺; |
| | ¹H NMR (400 MHz, DMSO-d₆) δ: 8.27-8.23 (m, 2H), 8.13 (s, 1H), 7.01 (s, 1H), 6.68-6.62 (m, 3H), 5.20 (s, 1H), 5.08 (s, 1H), 2.77 (d, 4H), 0.70-0.64 (m, 2H), 0.56-0.51 (m, 2H). |
| **133** | (S)-6-((6-amino-2-(fluoromethyl)pyrimidin-4-yl)amino)-4-((1,1-difluoropropan-2-yl)amino)-N-methylnicotinamide (46) |
| | 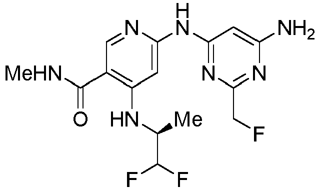 |
| | RCl: (S)-6-chloro-4-((1,1-difluoropropan-2-yl)amino)-N-methylnicotinamide (Preparation 189); RNH₂: 2-(fluoromethyl)pyrimidine-4,6-diamine (Preparation 18) |
| | Prep HPLC-3, 18 mg, 20%; LCMS m/z = 399 [M+H]⁺; |
| | ¹H NMR (400 MHz, DMSO-d₆) δ: 9.56 (s, 1H), 8.66 (d, 1H), 8.38- 8.35 (m, 2H), 7.28 (s, 1H), 6.68 (s, 2H), 6.61 (s, 1H), 6.14 (t, 1H), 5.19 (s, 1H), 5.08 (s, 1H), 3.87 (s, 1H), 2.71 (d, 3H), 1.23 (d, 3H). |
| **134** | (R)-6-((6-amino-2-(fluoromethyl)pyrimidin-4-yl)amino)-4-((1,1-difluoropropan-2-yl)amino)-N-methylnicotinamide (47) |
| | 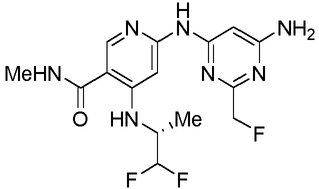 |
| | RCl: (R)-6-chloro-4-((1,1-difluoropropan-2-yl)amino)-N-methylnicotinamide (Preparation 188); RNH₂: 2-(fluoromethyl)pyrimidine-4,6-diamine (Preparation 18) |
| | Prep HPLC-3, 26 mg, 30%; LCMS m/z = 399 [M+H]⁺; |
| | ¹H NMR (400 MHz, DMSO-d₆) δ: 9.56 (s, 1H), 8.67 (d, 1H), 8.38- 8.35 (m, 2H), 7.28 (s, 1H), 6.68 (s, 2H), 6.61 (s, 1H), 6.14 (t, 1H), 5.19 (s, 1H), 5.08 (s, 1H), 3.87 (s, 1H), 2.71 (d, 3H), 1.23 (d, 3H). |
| **135** | 6-((6-amino-2-(fluoromethyl)pyrimidin-4-yl)amino)-4-(cyclopropylamino)-N-methylnicotinamide (8) |
| | 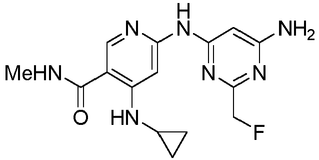 |
| | RCl: 6-chloro-4-(cyclopropylamino)-N-methylnicotinamide (Preparation 198); RNH₂: 2-(fluoromethyl)pyrimidine-4,6-diamine (Preparation 18) |
| | SiO₂, 20:1 DCM/MeOH; Yield: 26 mg, 30%; LCMS m/z = 399 [M+H]⁺; |
| | ¹H NMR (400 MHz, DMSO-d₆) δ: 9.59 (s, 1H), 8.40 (s, 1H), 8.29 (s, 2H), 7.45 (s, 1H), 6.65 (s, 3H), 5.14 (d, 2H), 2.71 (d, 3H), 0.79- 0.78 (m, 2H), 0.46 (s, 1H). |
| **136** | 6-((6-amino-2-(difluoromethyl)pyrimidin-4-yl)amino)-N-methyl-4-((methyl-d₃)amino)nicotinamide (5) |
| | 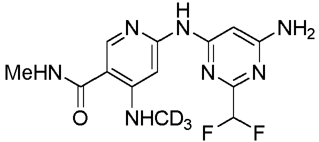 |
| | RCl: 6-chloro-N-methyl-4-((methyl-d3)amino)nicotinamide (Preparation 197); RNH₂: 2-(difluoromethyl)pyrimidine-4,6-diamine (Preparation 8) |
| | Prep HPLC-3, 25 mg, 30%; LCMS m/z = 327 [M+H]⁺; |
| | ¹H NMR (400 MHz, DMSO-d₆) δ: 9.68 (s, 1H), 8.29-8.27 (m, 2H), 8.13(s, 1H), 6.89 (d, 4H), 6.49 (t, 1H), 2.73 (d, 3H). |
| **137** | 6-((6-amino-2-(difluoromethyl)pyrimidin-4-yl)amino)-4-(isopropylamino)-N-(pyridin-2-ylmethyl)nicotinamide (158) |
| | 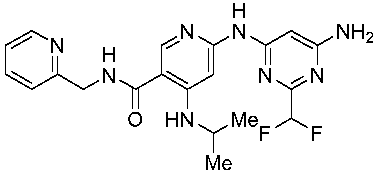 |
| | RCl: 6-chloro-4-(isopropylamino)-N-(pyridin-2-ylmethyl)nicotinamide (Preparation 218); RNH₂: 2-(difluoromethyl)pyrimidine-4,6-diamine (Preparation 8) |
| | Prep HPLC-3, 27.8 mg, 25%; LCMS m/z = 429 [M+H]⁺; |
| | ¹H NMR (400 MHz, DMSO-d₆) δ: 9.70 (s, 1H), 8.97-8.96(m, 1H), 8.51-8.50 (m, 2H), 8.31-8.29 (m, 1H), 7.78-7.74 (m, 1H), 7.33-7.24 (m, 2H), 7.11 (s, 1H), 6.89 (s, 2H), 6.79 (s, 1H), 6.48 (t, 1H,), 4.52-4.51(m, 2H), 3.60-3.55 (m, 1H), 1.20 (d, 6H). |
| **138** | 6-((6-amino-2-(difluoromethyl)pyrimidin-4-yl)amino)-4-(isopropylamino)-N-propylnicotinamide (67) |
| | 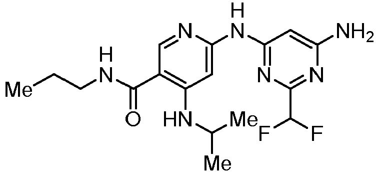 |
| | RCl: 6-chloro-4-(isopropylamino)-N-propylnicotinamide (Preparation 216); RNH₂: 2-(difluoromethyl)pyrimidine-4,6-diamine (Preparation 8) |
| | Prep HPLC-3, 20.4 mg, 17%; LCMS m/z = 380 [M+H]⁺; |
| | ¹H NMR (400 MHz, DMSO-d₆) δ: 9.66 (s, 1H), 8.33-8.27 (m, 3H), 7.06 (s, 1H), 6.87 (s, 2H), 6.79 (s, 1H), 6.47 (t, 1H), 3.58-3.55 (m, 1H), 3.17-3.13 (m, 2H), 1.53-1.48 (m, 2H), 1.20 (d, 6H), 0.90-0.86 (m, 3 H). |
| **139** | 6-((6-amino-2-(difluoromethyl)pyrimidin-4-yl)amino)-4-(isopropylamino)-N-(oxazol-2-ylmethyl)nicotinamide (148) |
| | 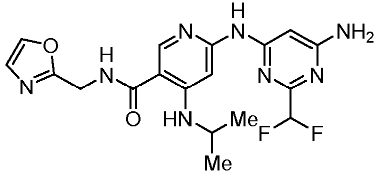 |
| | RCl: 6-chloro-4-(isopropylamino)-N-(oxazol-2-ylmethyl)nicotinamide (Preparation 219); RNH₂: 2-(difluoromethyl)pyrimidine-4,6-diamine (Preparation 8) |
| | Prep HPLC-3, 26.8 mg, 23%; LCMS m/z = 419 [M+H]⁺; |
| | ¹H NMR (500 MHz, DMSO-d₆) δ: 9.72 (s, 1H), 8.99 (t, 1H), 8.43 (s, 1H), 8.27 (d, 1H), 8.05 (s, 1H), 7.16 (s, 1H), 7.14 (s, 1H), 6.90 (s, 2H), 6.75 ( s, 1H), 6.48 (t, 1H), 4.52 (d, 2H), 3.59-3.57 (m, 1H), 1.20 (d, 6H). |
| **140** | (S)-6-((6-amino-2-(difluoromethyl)pyrimidin-4-yl)amino)-N-cyclopropyl-4-((1-fluoropropan-2-yl)amino)nicotinamide (90) |
| | 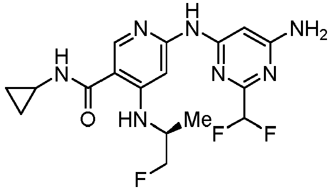 |
| | RCl: (S)-6-chloro-N-cyclopropyl-4-((1-fluoropropan-2-yl)amino) nicotinamide (Preparation 195); RNH₂: 2-(difluoromethyl)pyrimidine-4,6-diamine (Preparation 8) |
| | Prep HPLC-3, 19.1 mg, 13%; LCMS m/z = 396 [M+H]⁺; |
| | ¹H NMR (400 MHz, DMSO-d₆) δ: 9.69 (s, 1H), 8.56-8.54 (m, 1H), 8.34-8.31 (m, 1H), 7.15 (s, 1H), 6.88 (s, 2H), 6.76 (s, 1H), 6.48 (t, 2H), 4.57-4.40 (m, 2H), 3.79-3.71 (m, 1H), 2.79-2.75 (m, 1H), 1.23 (d, 6H), 0.69-0.65 (m, 2H), 0.56-0.53 (m, 2H). |
| **141** | (S)-6-((6-amino-2-(difluoromethyl)pyrimidin-4-yl)amino)-4-((1-fluoropropan-2-yl)amino)-N-(pyridin-2-ylmethyl)nicotinamide (186) |
| | 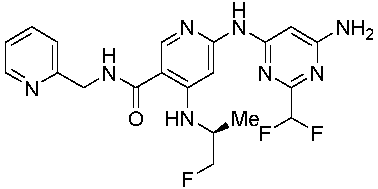 |
| | RCl: (S)-6-chloro-4-((1-fluoropropan-2-yl)amino)-N-(pyridin-2-ylmethyl)nicotinamide (Preparation 199); RNH₂: 2-(difluoromethyl)pyrimidine-4,6-diamine (Preparation 8) |
| | Prep HPLC-3, 32.3 mg, 23%; LCMS m/z = 447 [M+H]⁺; |
| | ¹H NMR (400 MHz, DMSO-d₆) δ: 9.73 (s, 1H), 9.01 (t, 1H), 8.54-8.50 (m, 3H), 7.78-7.74 (m, 1H), 7.33-7.31 (m, 1H), 7.27-7.25 (m, 1H), 7.16 (s, 1H), 6.91 (s, 2H), 6.78 (s, 1H), 6.49 (t, 1H), 4.56-4.39 (m, 4H), 3..80-3.72 (m, 1H), 1.22 (d, 3H). |
| **142** | (R)-6-((6-amino-2-(difluoromethyl)pyrimidin-4-yl)amino)-N-cyclopropyl-4-((1-fluoropropan-2-yl)amino)nicotinamide (91) |
| | 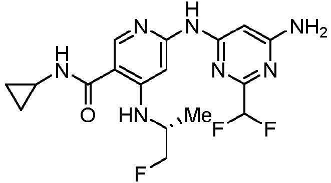 |
| | RCl: (R)-6-chloro-N-cyclopropyl-4-((1-fluoropropan-2-yl)amino) nicotinamide (Preparation 196); RNH₂: 2-(difluoromethyl)pyrimidine-4,6-diamine (Preparation 8) |
| | Prep HPLC-3, 20.1 mg, 9%; LCMS m/z = 396 [M+H]⁺; |
| | ¹H NMR (400 MHz, DMSO-d₆) δ: 9.69 (s, 1H), 8.56-8.54 (m, 1H), 8.35-8.31 (m, 2H), 7.14 (s, 1H), 6.89 (s, 2H), 6.75 (s, 1H), 6.48 (t, 1H), 4.56-4.41 (m, 2H), 3.79-3.73 (m, 1H), 2.79-2.75 (m, 1H), 1.23 (d, 3H), 0.69-0.52 (m, 4H). |
| **143** ^{A} | 6-((6-amino-2-(difluoromethyl)pyrimidin-4-yl)amino)-4-(cyclopropylamino)-N-ethylnicotinamide (38) |
| | 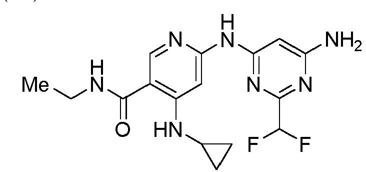 |
| | RCl: 6-chloro-4-(cyclopropylamino)-N-ethylnicotinamide (Preparation 205); RNH₂: 2-(difluoromethyl)pyrimidine-4,6-diamine (Preparation 8) |
| | Prep HPLC-3, 14 mg, 15%; LCMS m/z = 364 [M+H]⁺; |
| | ¹H NMR (400 MHz, DMSO-d₆) δ: 9.77 (s, 1H), 8.42-8.33 (m, 3H), 7.43 (s, 1H), 6.89 (s, 2H), 6.80(s, 1H), 6.48 (t, 1H), 3.24-3.21 (m, 2H), 2.41 (s, 1H), 1.10 (t, 3H), 0.80-0.78 (m, 2H), 0.49-0.47 (m, 2H). |
| **144** ^{A} | 6-((6-amino-2-(difluoromethyl)pyrimidin-4-yl)amino)-4-(cyclopropylamino)-N-propylnicotinamide (62) |
| | 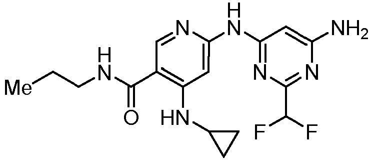 |
| | RCl: 6-chloro-4-(cyclopropylamino)-N-propylnicotinamide (Preparation 206); RNH₂: 2-(difluoromethyl)pyrimidine-4,6-diamine (Preparation 8) |
| | Prep HPLC-3, 15.8 mg, 16%; LCMS m/z = 378 [M+H]⁺; |
| | ¹H NMR (400 MHz, DMSO-d₆) δ: 9.77 (s, 1H), 8.39-8.35 (m, 3H), 7.41 (s, 1H), 6.89 (s, 2H), 6.82 (s, 1H), 6.49 (t, 1H), 3.33-3.13 (m, 2H), 2.41 (s, 1H), 1.54-1.48 (m, 2H), 0.88 (t, 3H), 0.80-0.78 (m, 2H), 0.48-0.47 (m, 2H). |
| **145** ^{A} | 6-((6-amino-2-(difluoromethyl)pyrimidin-4-yl)amino)-4-(cyclopropylamino)-N-(cyclopropylmethyl)nicotinamide (79) |
| | 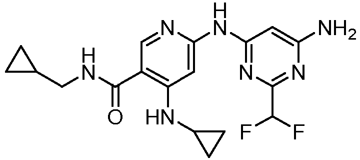 |
| | RCl: 6-chloro-4-(cyclopropylamino)-N-(cyclopropylmethyl)nicotinamide (Preparation 207); RNH₂: 2-(difluoromethyl)pyrimidine-4,6-diamine (Preparation 8) |
| | Prep HPLC-3, 18 mg, 18%; LCMS m/z = 390 [M+H]⁺; |
| | ¹H NMR (400 MHz, DMSO-d₆) δ: 9.78 (s, 1H), 8.47-8.37 (m, 3H), 7.44 (s, 1H), 6.89 (s, 2H), 6.80 (s, 1H), 6.49 (t, 1H), 3.10-3.07 (m, 2H), 2.42 (s, 1H), 1.01-1.00 (m, 1H), 0.80 (d, 2H), 0.48-0.41 (m, 4H), 0.23-0.21 (m, 2H). |

| | |
|---|---|
| ^{A} - 0.2 eq. Pd₂(dba)₃ + 0.2 eq. XantPhos was the catalyst | |

### Example 147: 6-((6-amino-2-(difluoromethyl)pyrimidin-4-yl)amino)-N-cyclopropyl-4-(isopropylamino)nicotinamide trifluoroacetate (60)

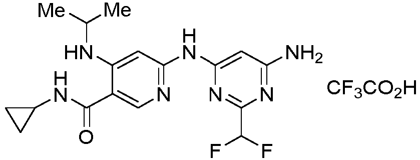

To a mixture of compound 6-chloro-N-cyclopropyl-4-(isopropylamino)nicotinamide (Preparation 217, 20 mg, 0.079 mmol) and 2-(difluoromethyl)pyrimidine-4,6-diamine (Preparation 8, 15 mg, 0.094 mmol) in dioxane (1 mL) was added Cs₂CO₃ (51.4 mg, 0.158 mmol), Brettphos (4.23 mg, 7.88 µmol) and BrettPhos Pd G4 (7.26 mg, 7.88 µmol) and the mixture was stirred at 80 °C for 1 h under N₂. The reaction was quenched with water (10 mL) and extracted with EtOAc (3x 15 mL). The combined organics were dried (Na₂SO₄) and evaporated to dryness *in vacuo.* The residue was purified by prep HPLC-10 to afford the title compound as a white solid (20 mg, 52%). LCMS m/z = 378 [M+H]⁺. ¹H NMR (400 MHz, MeOH-d₄) δ: 8.28 (s, 1H), 6.67-6.38 (m, 1H), 6.32 (s, 1H), 6.06 (s, 1H), 3.84-3.76 (m,, 1H), 2.86-2.79 (m, 1H), 1.34 (d, 6H), 0.85-0.79 (m, 2H), 0.65-0.60 (m, 2H).

### Example 148: 6-((6-amino-2-(difluoromethyl)pyrimidin-4-yl)amino)-N-cyclopropyl-4-(ethylamino)nicotinamide (39)

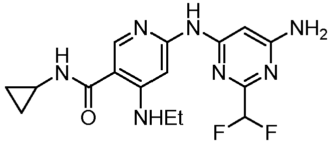

A mixture of 6-chloro-N-cyclopropyl-4-(ethylamino)nicotinamide (Preparation 185, 10 mg, 0.042 mmol), 2-(difluoromethyl)pyrimidine-4,6-diamine (Preparation 8, 6.68 mg, 0.042 mmol), Cs₂CO₃ (27.2 mg, 0.083 mmol) and BrettPhos Pd G4 (3.84 mg, 4.17 □mol) in dioxane (2 mL) was stirred at 90 °C for 1 h under N₂. The reaction mixture was filtered and the filtrate was concentrated under reduced pressure to give a residue which was purified by prep HPLC-3 to give the title compound as a pale yellow solid. LCMS m/z = 364 [M+H]+. ¹H NMR (400 MHz, DMSO-d₆) δ: 9.65 (s, 1H), 8.28 (d, 1H), 8.25 (s, 1H), 8.23 (t, 1H), 6.99 (s, 1H), 6.85 (s, 2H), 6.79 (s, 1H), 6.46 (t, 1H), 3.14-3.09 (m, 2H), 2.79-2.72 (m, 1H), 1.19 (t, 3H), 0.67-0.63 (m, 2H), 0.54-0.51 (m, 2H).

### Example 149-162

The title compounds were prepared from the appropriate 2-chloropyridine (RCl) and pyrimidine-4,6-diamine (RNH₂) using a similar method to that described for Example 148.

| **Example No** | **Name/Structure/R-Cl/HPLC/Yield/Data** |
|---|---|
| **149** | 6-((6-amino-2-(difluoromethyl)pyrimidin-4-yl)amino)-4-(isopropylamino)-N-methylnicotinamide (25) |
| | 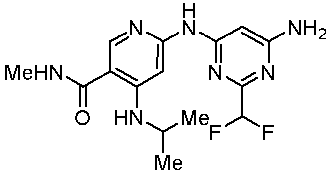 |
| | RCl: 6-chloro-4-(isopropylamino)-N-methylnicotinamide (Preparation 181); RNH₂: 2-(difluoromethyl)pyrimidine-4,6-diamine (Preparation 8) |
| | SiO₂: 0-20% MeOH/DCM; Yield: 13.8 mg, 6.8%; LCMS m/z = 352 [M+H]⁺. |
| | ¹H NMR (400 MHz, DMSO-d₆) δ: 10.35 (br, s, 1H), 8.82 (br, s, 1H), 8.42 (br s,1H), 8.33 (s, 1H), 7.05 (br s, 2H), 6.73-6.44 (m, 2H), 6.31 (br, s, 1H), 3.74-3.61 (m, 2H), 2.75 (d, 3H), 1.24 (d, 6H). |
| **150** | 6-((6-amino-2-(difluoromethyl)pyrimidin-4-yl)amino)-4-(ethylamino)-N-methylnicotinamide (12) |
| | 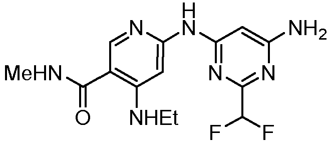 |
| | RCl: 6-chloro-4-(ethylamino)-N-methylnicotinamide (Preparation 184); RNH₂: 2-(difluoromethyl)pyrimidine-4,6-diamine (Preparation 8) |
| | Prep HPLC-6, 20.5 mg, 32%; LCMS m/z = 338 [M+H]⁺. |
| | ¹H NMR (400 MHz, MeOH-d₄) δ: 8.19 (s, 1H), 6.99 (s, 1H), 6.68 (s, 1H), 6.45-6.12 (m, 1H), 3.26-3.20 (m, 2H), 2.83 (s, 3H), 1.28 (t, 3H). |
| **151** | (R)-6-((6-amino-2-(fluoromethyl)pyrimidin-4-yl)amino)-N-methyl-4-((tetrahydrofuran-3-yl)amino)nicotinamide (35) |
| | 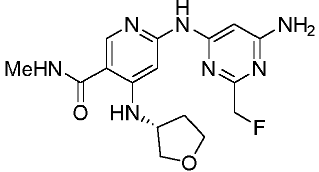 |
| | RCl: (R)-6-chloro-N-methyl-4-((tetrahydrofuran-3-yl)amino)nicotinamide (Preparation 191); RNH₂: 2-(fluoromethyl)pyrimidine-4,6-diamine (Preparation 18) |
| | Prep HPLC-3, 11 mg, 19%; LCMS m/z = 362 [M+H]⁺. |
| | ¹H NMR (500 MHz, DMSO-d₆) δ: 9.53 (s, 1H), 8.54-8.53 (m, 1H), 8.32-8.31 (m, 1H), 7.10 (s, 1H), 6.65-6.64 (m, 3H), 5.14 (d, 2H), 3.99-3.73 (m, 4H), 3.56-3.54 (m, 1H), 2.72-2.71 (m, 3H), 2.31-2.27 (m, 1H), 1.78-1.75 (m, 1H). |
| **152** | (S)-6-((6-amino-2-(fluoromethyl)pyrimidin-4-yl)amino)-N-methyl-4-((tetrahydrofuran-3-yl)amino)nicotinamide (36) |
| | 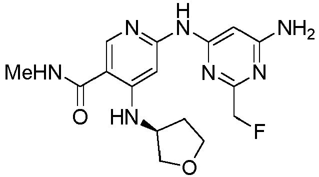 |
| | RCl: (S)-6-chloro-N-methyl-4-((tetrahydrofuran-3-yl)amino)nicotinamide (Preparation 192); RNH₂: 2-(fluoromethyl)pyrimidine-4,6-diamine (Preparation 18) |
| | Prep HPLC-3, 14.1 mg, 24%; LCMS m/z = 362 [M+H]⁺. |
| | ¹H NMR (500 MHz, DMSO-d₆) δ: 9.53 (s, 1H), 8.54-8.53 (m, 1H), 8.32-8.31 (m, 1H), 7.10 (s, 1H), 6.65-6.64 (m, 3H), 5.14 (d, 2H), 3.99-3.73 (m, 4H), 3.56-3.54 (m, 1H), 2.72-2.71 (m, 3H), 2.31-2.27 (m, 1H), 1.78-1.75 (m, 1H). |
| **153** | 6-((6-amino-2-(fluoromethyl)pyrimidin-4-yl)amino)-4-(cyclobutylamino)-N-methylnicotinamide (14) |
| | 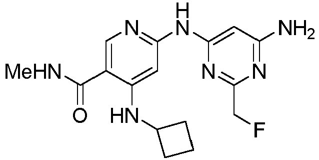 |
| | RCl: 6-chloro-4-(cyclobutylamino)-N-methylnicotinamide (Preparation 193); RNH₂: 2-(fluoromethyl)pyrimidine-4,6-diamine (Preparation 18) |
| | Prep HPLC-3, 17.3 mg, 21%; LCMS m/z = 346 [M+H]⁺. |
| | ¹H NMR (500 MHz, DMSO-d₆) δ: 9.50 (s, 1H), 8.44-8.43 (m, 1H), 8.29-8.27 (m, 1H), 6.95 (s, 1H), 6.66-6.63 (m, 3H), 5.14 (d, 2H), 3.86-3.82 (m, 1H), 2.72-2.71 (m, 3H), 2.44-2.42 (m, 2H), 1.85-1.74 (m, 4H). |
| **154** | 6-((6-amino-2-(fluoromethyl)pyrimidin-4-yl)amino)-N-methyl-4-(oxetan-3-ylamino)nicotinamide (19) |
| | 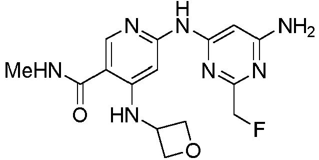 |
| | RCl: 6-chloro-N-methyl-4-(oxetan-3-ylamino)nicotinamide (Preparation 194); RNH₂: 2-(fluoromethyl)pyrimidine-4,6-diamine (Preparation 18) |
| | Prep HPLC-3, 15 mg, 21%; LCMS m/z = 348 [M+H]⁺. |
| | ¹H NMR (500 MHz, DMSO-d₆) δ: 9.53 (s, 1H), 8.74-8.73 (m, 1H), 8.36-8.33 (m, 1H), 6.78 (s, 1H), 6.65-6.62 (m, 3H), 5.15 (d, 2H), 4.90-4.88 (m, 2H), 4.53-4.51(m, 1H), 4.43-4.41 (m, 3H), 2.74-2.73 (m, 3H). |
| **155** | 6-((6-amino-2-(difluoromethyl)pyrimidin-4-yl)amino)-4-(ethylamino)-N-(3-(hydroxymethyl)bicyclo[1.1.1]pentan-1-yl)nicotinamide (151) |
| | 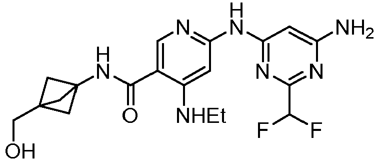 |
| | RCl: 6-chloro-4-(ethylamino)-N-(3-(hydroxymethyl)bicyclo[1.1.1]pentan-1-yl)nicotinamide (Preparation 214); RNH₂: 2-(difluoromethyl)pyrimidine-4,6-diamine (Preparation 8) |
| | Prep HPLC-3, 20.4 mg, 14%; LCMS m/z = 402 [M+H]⁺. |
| | ¹H NMR (400 MHz, DMSO-d₆) δ: 9.69 (s, 1H), 8.75 (s, 1H), 8.37-8.33 (m, 2H), 7.08 (s, 1H), 6.88 (s, 2H), 6.92 (s, 1H), 6.49 (t, 1H), 4.55-4.52 (m, 1H), 3.48-3.46 (m, 2H), 3.17-3.11 (m, 2H), 1.91 (s, 6H), 1.21-1.17 (m, 3H). |
| **156** | 6-((6-amino-2-(fluoromethyl)pyrimidin-4-yl)amino)-4-(ethylamino)-N-(3-(hydroxymethyl)bicyclo[1.1.1]pentan-1-yl)nicotinamide (96) |
| | 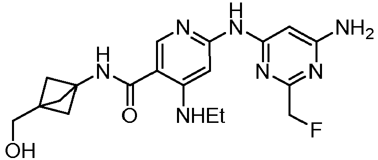 |
| | RCl: 6-chloro-4-(ethylamino)-N-(3-(hydroxymethyl)bicyclo[1.1.1]pentan-1-yl)nicotinamide (Preparation 214); RNH₂: 2-(fluoromethyl)pyrimidine-4,6-diamine (Preparation 18) |
| | Prep HPLC-3, 11.1 mg, 8%; LCMS m/z = 402 [M+H]⁺. ¹ |
| | H NMR (500 MHz, DMSO-d₆) δ: 9.52 (s, 1H), 8.73 (s, 1H), 8.34-8.32 (m, 2H), 7.15 (s, 1H), 6.63-6.56 (m, 3H), 5.13 (d, 2H), 4.55-4.52 (m, 1H), 3.48-3.47 (m, 2H), 3.17-3.10 (m, 2H), 1.91 (s, 6H), 1.21-1.17 (m, 3H). |
| **157** | 6-((6-amino-2-(difluoromethyl)pyrimidin-4-yl)amino)-N-(3-(hydroxy methyl)bicyclo[1.1.1]pentan-1-yl)-4-(methylamino)nicotinamide (121) |
| | 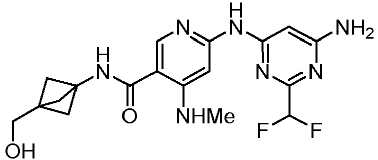 |
| | RCl: 6-chloro-N-(3-(hydroxymethyl)bicyclo[1.1.1]pentan-1-yl)-4-(methylamino)nicotinamide (Preparation 213); RNH₂: 2-(difluoromethyl)pyrimidine-4,6-diamine (Preparation 8) |
| | Prep HPLC-3, 27.9 mg, 20%; LCMS m/z = 406 [M+H]⁺. |
| | ¹H NMR (400 MHz, DMSO-d₆) δ: 9.70 (s, 1H), 8.75 (s, 1H), 8.31-8.24 (m, 2H), 7.00-6.76 (m, 4H), 6.49 (t, 1H,), 4.55-4.52 (m, 1H), 3.48-3.46 (m, 2H), 2.77-2.76 (m, 3H), 1.91 (s, 6H). |
| **158** | (S)-6-((6-amino-2-(fluoromethyl)pyrimidin-4-yl)amino)-4-((1-fluoropropan-2-yl)amino)-N-methylnicotinamide or (R)-6-((6-amino-2-(fluoromethyl) pyrimidin-4-yl)amino)-4-((1-fluoropropan-2-yl)amino)-N-methylnicotinamide (27) |
| | 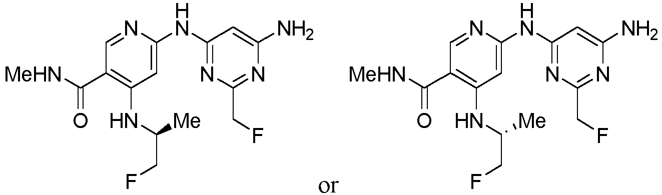 |
| | RCl: (S)-6-chloro-4-((1-fluoropropan-2-yl)amino)-N-methylnicotinamide (Preparation 186); RNH₂: 2-(fluoromethyl)pyrimidine-4,6-diamine (Preparation 18) |
| | Prep HPLC-3, 14 mg, 19%; LCMS m/z = 352 [M+H]⁺. |
| | ¹H NMR (400 MHz, DMSO-d₆) δ: 9.52 (s, 1H), 8.54-8.52 (m, 1H), 8.32-8.31 (m, 2H), 7.19 (s, 1H), 6.65-6.62 (m, 3H), 5.13 (d, 2H), 4.54-4.40 (m, 2H), 3.79-3.71 (m, 1H), 2.72 (d, 3H), 1.22 (d, 3H). |
| **159** | (S)-6-((6-amino-2-(difluoromethyl)pyrimidin-4-yl)amino)-4-((1-fluoropropan-2-yl)amino)-N-(3-(hydroxymethyl)bicyclo[1.1.1]pentan-1-yl)nicotinamide (188) |
| | 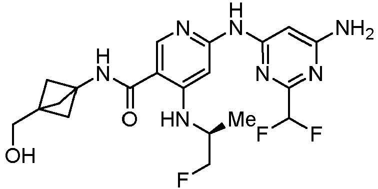 |
| | RCl: (S)-6-chloro-4-((1-fluoropropan-2-yl)amino)-N-(3-(hydroxymethyl)bicyclo[1.1.1]pentan-1-yl)nicotinamide (Preparation 224); RNH₂: 2-(difluoromethyl)pyrimidine-4,6-diamine (Preparation 8) |
| | Prep HPLC-3, 25.9 mg, 19%; LCMS m/z = 452 [M+H]⁺. |
| | ¹H NMR (400 MHz, DMSO-d₆) δ: 9.72 (s, 1H), 8.79 (s, 1H), 8.63-8.62 (m, 1H), 8.38 (s, 1H), 7.22 (s, 1H), 6.90 (s, 2H), 6.67 (s, 1H), 6.49 (t, 1H), 4.58-4.41 (m, 3H), 3.78-3.73 (m, 1H), 3.48 (s, 2H), 1.91 (s, 6H), 1.23 (d, 3H). |
| **160** | (R)-6-((6-amino-2-(fluoromethyl)pyrimidin-4-yl)amino)-4-((1-fluoropropan-2-yl)amino)-N-methylnicotinamide or (S)-6-((6-amino-2-(fluoromethyl) pyrimidin-4-yl)amino)-4-((1-fluoropropan-2-yl)amino)-N-methylnicotinamide (28) |
| | 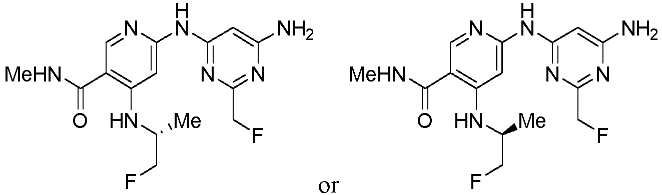 |
| | RCl: (R)-6-chloro-4-((1-fluoropropan-2-yl)amino)-N-methylnicotinamide (Preparation 190); RNH₂: 2-(fluoromethyl)pyrimidine-4,6-diamine (Preparation 18) |
| | Prep HPLC-3, 4 mg, 5%; LCMS m/z = 352 [M+H]⁺. |
| | ¹H NMR (400 MHz, DMSO-d₆) δ: 9.52 (s, 1H), 8.54-8.52 (m, 1H), 8.32-8.31 (m, 2H), 7.19 (s, 1H), 6.65 (s, 2H), 6.62 (s, 1H), 5.13 (d, 2H), 4.53-4.39 (m, 2H), 3.78-3.70 (m, 1H), 2.72 (d, 3H), 1.22 (d, 3H). |
| **161** ^{A} | 6-((6-amino-2-(difluoromethyl)pyrimidin-4-yl)amino)-4-((1,3-difluoropropan-2-yl)amino)-N-methylnicotinamide (75) |
| | 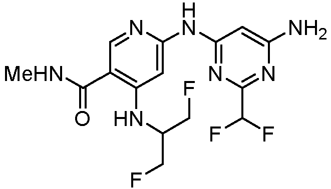 |
| | RCl: 6-chloro-4-((1,3-difluoropropan-2-yl)amino)-N-methylnicotinamide (Preparation 187); RNH₂: 2-(difluoromethyl)pyrimidine-4,6-diamine (Preparation 8) |
| | Prep HPLC-3, 16.4 mg, 19%; LCMS m/z = 388 [M+H]⁺. |
| | ¹H NMR (400 MHz, DMSO-d₆) δ: 9.71 (s, 1H), 8.83 (d, 1H), 8.40-8.38(m, 2H), 7.18(s, 1H), 6.93 (s, 2H), 6.81(s, 1H), 6.49 (t, 1H), 4.67-4.56 (m, 4H), 4.01-3.95(m, 1H), 2.74(d, 3H). |
| **162** ^{A} | 6-((6-amino-2-(fluoromethyl)pyrimidin-4-yl)amino)-N-methyl-4-(methylamino)nicotinamide (1) |
| | 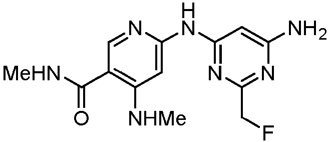 |
| | RCl: 6-chloro-N-methyl-4-(methylamino)nicotinamide (Preparation 180); RNH₂: 2-(fluoromethyl)pyrimidine-4,6-diamine (Preparation 18) |
| | SiO₂, 20:1 DCM/MeOH, 180 mg, 90%; LCMS m/z = 305 [M+H]⁺. |
| | ¹H NMR (400 MHz, DMSO-d₆) δ: 9.49 (s, 1H), 8.26-8.24 (m, 2H), 8.15 (d, 1H), 6.97 (s, 1H), 6.71 (s, 1H), 6.63 (s, 2H), 5.19 (s, 1H), 5.07 (s, 1H), 2.77 (d, 3H), 2.70 (d, 3H). |
| **163** | 2-(difluoromethyl)-N4-(5-(1-methyl-1H-1,2,3-triazol-4-yl)-4-(methylamino)pyridin-2-yl)pyrimidine-4,6-diamine (17) |
| | 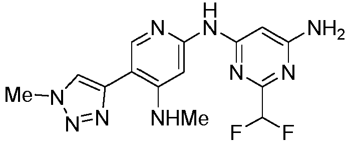 |
| | RCl: 2-chloro-N-methyl-5-(1-methyl-1H-1,2,3-triazol-4-yl)pyridin-4-amine (Preparation 234); RNH₂: 2-(difluoromethyl)pyrimidine-4,6-diamine (Preparation 8) |
| | Prep HPLC-3, White solid (18.9 mg, 23%), LCMS m/z = 348 [M+H]⁺. |
| | ¹H NMR (400 MHz, DMSO-d₆) δ: 9.63 (s, 1H), 8.34 (s, 1H), 8.24 (s, 1H), 7.17-7.15 (m, 1H), 6.98-6.93 (m, 2H), 6.86 (s, 2H), 6.49 (t, 1H), 4.21 (s, 3H), 2.87 (d, 3H). |
| 164 | 2-(difluoromethyl)-N4-(5-(2-methyl-2H-1,2,3-triazol-4-yl)-4-(methyl amino)pyridin-2-yl)pyrimidine-4,6-diamine (18) |
| | 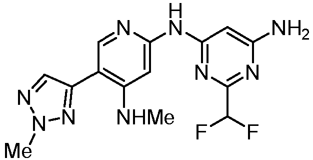 |
| | RCl: 2-chloro-N-methyl-5-(2-methyl-2H-1,2,3-triazol-4-yl)pyridin-4-amine (Preparation 235); RNH₂: 2-(difluoromethyl)pyrimidine-4,6-diamine (Preparation 8) |
| | Prep HPLC-3. White solid, 20.7 mg, 44%. LCMS m/z = 348 [M+H]⁺ |
| | 1H-NMR (500 MHz, DMSO-d₆) δ ppm 9.63 (s, 1H), 8.34 (s, 1H), 8.24 (s, 1H), 7.17-7.15 (m, 1H), 6.98-6.93 (m, 2H), 6.86 (s, 2H), 6.49 (t, 1H), 4.21 (s, 3H), 2.87 (d, 3H). |

| | |
|---|---|
| ^{A} - Pd(t-Bu₃P)₂ was used as the reaction catalyst | |

### Example 165: 6-((6-amino-2-(fluoromethyl)pyrimidin-4-yl)amino)-4-(isopropyl amino)-N-methylnicotinamide (9)

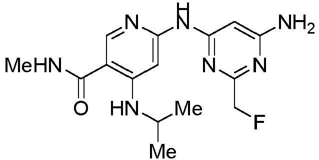

A mixture of 6-((6-amino-2-(hydroxymethyl)pyrimidin-4-yl)amino)-4-(isopropylamino)-N-methylnicotinamide (Preparation 259, 30 mg, 0.091 mmol) and DAST (72.7 mg, 0.452 mmol) in DCM (5 mL) was stirred at 0 °C for 4 h. The reaction was quenched with NaHCO₃ (aq.) and concentrated to dryness *in vacuo.* The residue was purified by Prep HPLC-3 to afford the title compound as a white solid (11.2 mg, 37%). LCMS m/z = 333 [M+H]⁺. ¹H NMR (400 MHz, DMSO-d₆) δ: 9.50 (s, 1H), 8.31-8.27 (m, 3H), 7.10 (s, 1H), 6.65-6.63 (m, 3H), 5.13 (d, 2H), 3.58-3.53 (m, 1H), 2.71 (d, 3H), 1.19 (d, 6H).

### Example 168-181

The title compounds were prepared using a single step protocol using 6-((6-amino-2-(difluoromethyl)pyrimidin-4-yl)amino)-4-fluoro-N-methylnicotinamide (Preparation 260) and the appropriate primary amine (RNH₂) as shown in the scheme below.

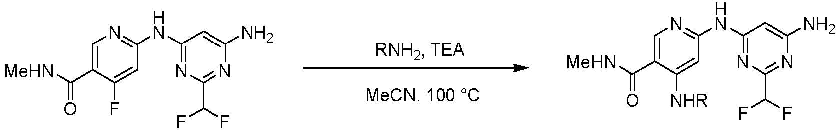

To a mixture of 6-((6-amino-2-(difluoromethyl)pyrimidin-4-yl)amino)-4-fluoro-N-methylnicotinamide (Preparation 260, 46.8 mg, 0.15 mmol, 1 eq.) and TEA (0.45 mmol, 3 eq.) in MeCN (3 mL) was added the appropriate amine (RNH₂, 0.30 mmol, 2 eq.) and the resulting mixture shaken at 100 °C for 3 h. The solids were removed by filtration and the filtrate evaporated to dryness using a Speedvac. The residue was purified prep-HPLC-4 to afford the title compound.

| **Example No** | **Name/Structure/ RNH₂/Data** |
|---|---|
| **168** | 6-((6-amino-2-(difluoromethyl)pyrimidin-4-yl)amino)-4-(benzylamino)-N-methylnicotinamide (92) |
| | 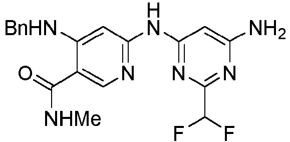 |
| | RNH₂: benzylamine; Yield: 46%; LCMS m/z = 400 [M+H]⁺. |
| **169** | 6-((6-amino-2-(difluoromethyl)pyrimidin-4-yl)amino)-4-(isopentylamino)-N-methylnicotinamide (66) |
| | 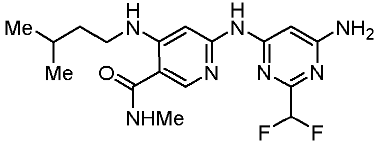 |
| | RNH₂: 3-methylbutan-1-amine; Yield: 52%; LCMS m/z = 380 [M+H]⁺. |
| **170** | 6-((6-amino-2-(difluoromethyl)pyrimidin-4-yl)amino)-4-(isobutylamino)-N-methylnicotinamide (43) |
| | 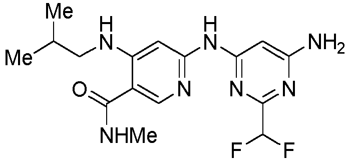 |
| | RNH₂: 2-methylpropan-1-amine; Yield: 41%; LCMS m/z = 366 [M+H]⁺. |
| **171** | 6-((6-amino-2-(difluoromethyl)pyrimidin-4-yl)amino)-N-methyl-4-((2,2,2-trifluoroethyl)amino)nicotinamide (82) |
| | 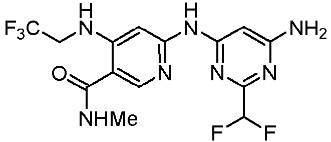 |
| | RNH₂: 2,2,2-trifluoroethan-1-amine; Yield: 6%; LCMS m/z = 392 [M+H]⁺. |
| **172** | 6-((6-amino-2-(difluoromethyl)pyrimidin-4-yl)amino)-N-methyl-4-(oxetan-3-ylamino)nicotinamide (42) |
| | 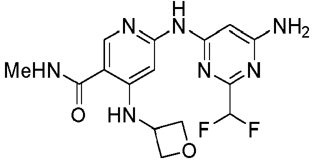 |
| | RNH₂: oxetan-3-amine; Yield: 31%; LCMS m/z = 366 [M+H]⁺. |
| **173** | 6-((6-amino-2-(difluoromethyl)pyrimidin-4-yl)amino)-N-methyl-4-((4-methyltetrahydro-2H-pyran-4-yl)amino)nicotinamide (125) |
| | 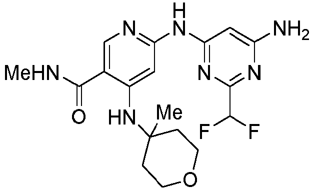 |
| | RNH₂: 4-methyltetrahydro-2H-pyran-4-amine; Yield: 9%; LCMS m/z = 408 [M+H]⁺. |
| **174** | 6-((6-amino-2-(difluoromethyl)pyrimidin-4-yl)amino)-N-methyl-4-((3-methyltetrahydrofuran-3-yl)amino)nicotinamide (89) |
| | 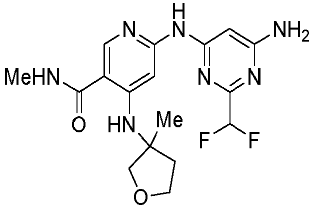 |
| | RNH₂: 3-methyltetrahydrofuran-3-amine; Yield: 27%; LCMS m/z = 394 [M+H]⁺. |
| **175** | 6-((6-amino-2-(difluoromethyl)pyrimidin-4-yl)amino)-4-((cyclobutylmethyl)amino)-N-methylnicotinamide (61) |
| | 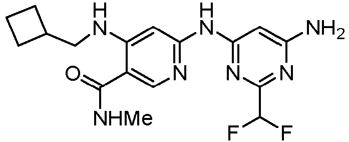 |
| | RNH₂: cyclobutylmethanamine; Yield: 36%; LCMS m/z = 378 [M+H]⁺. |
| **176** | 6-((6-amino-2-(difluoromethyl)pyrimidin-4-yl)amino)-4-(cyclobutylamino)-N-methylnicotinamide (37) |
| | 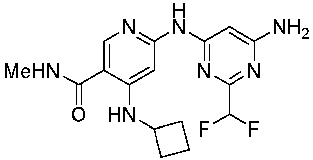 |
| | RNH₂: cyclobutylamine; Yield: 40%; LCMS m/z = 364 [M+H]⁺. |
| **177** | 6-((6-amino-2-(difluoromethyl)pyrimidin-4-yl)amino)-N-methyl-4-((tetrahydrofuran-3-yl)amino)nicotinamide (64) |
| | 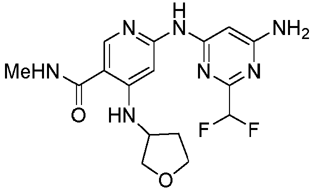 |
| | RNH₂: tetrahydrofuran-3-amine; Yield: 21%; LCMS m/z = 380 [M+H]⁺. |
| **178** | 6-((6-amino-2-(difluoromethyl)pyrimidin-4-yl)amino)-N-methyl-4-(propylamino)nicotinamide (26) |
| | 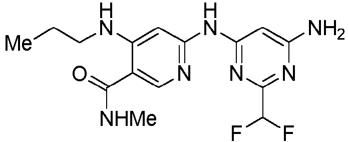 |
| | RNH₂: propan-1-amine; Yield: 38%; LCMS m/z = 352 [M+H]⁺. |
| **179** | 6-((6-amino-2-(difluoromethyl)pyrimidin-4-yl)amino)-N-methyl-4-((2-methylbutyl)amino)nicotinamide (65) |
| | 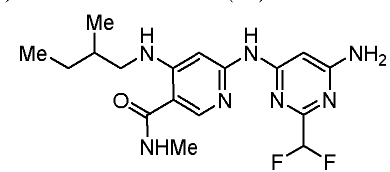 |
| | RNH₂: 2-methylbutan-1-amine; Yield: 39%; LCMS m/z = 380 [M+H]⁺. |
| **180** | 6-((6-amino-2-(difluoromethyl)pyrimidin-4-yl)amino)-4-(cyclopropylamino)-N-methylnicotinamide (23) |
| | 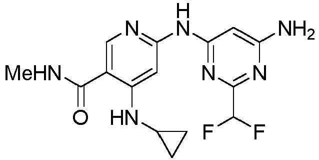 |
| | RNH₂: cyclopropylamine; Yield: 9%; LCMS m/z = 350 [M+H]⁺. |
| **181** | 6-((6-amino-2-(difluoromethyl)pyrimidin-4-yl)amino)-N-methyl-4-(methylamino)nicotinamide (4) |
| | 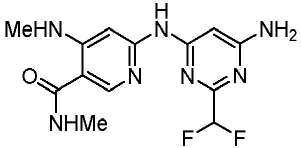 |
| | RNH₂: methylamine; Yield: 36%; LCMS m/z = 324 [M+H]⁺. |

### Example 182-194

The title compounds were prepared using a 3-step chemistry protocol using 6-chloro-4-fluoronicotinic acid, R¹NH₂, 2-(difluoromethyl)pyrimidine-4,6-diamine hydrochloride (Preparation 7) and R²NH₂ as outlined in the scheme and table below.

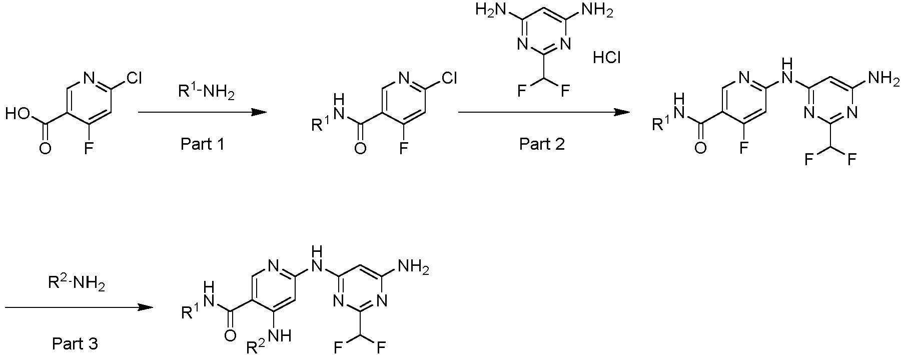

**Part 1.** To a solution of 6-chloro-4-fluoronicotinic acid (87.5 mg, 0.5 mmol, 1 eq) and the appropriate amine (R¹NH₂, 0.5 mmol, 1 eq) in MeCN (4 mL) was added DIPEA (0.702 mL, 4 mmol, 8 eq.) and T₃P (0.5 mL) and the mixture stirred at 30 °C for 16 hrs. The reaction was quenched with 5 M Na₂CO₃ (4 mL) and extracted with EtOAc (3x 10 mL). The combined organics were evaporated to dryness in a Speedvac to afford a residue that was used in Part 2 without further purification.

**Part 2.** Cs₂CO₃ (440 mg, 1.35 mmol, 3 eq) and Brettphos Pd G3 (20.41 mg, 22.5 □mol, 0.05 eq.) were added to a solution of the compound of Part 1 (0.45 mmol, 1 eq.) and 2-(difluoromethyl)pyrimidine-4,6-diamine hydrochloride (Preparation 7, 144 mg, 0.9 mmol, 2 eq.) under a N₂ atmosphere and the mixture stirred at 120 °C for 2 h. The reactions were diluted with H₂O (3 mL) and extracted with EtOAc (3x 10 mL). The organic layer was collected and evaporated to dryness by Speedvac to afford a residue that was used in Part 3 without further purification.

**Part 3.** The second amine displacement was carried out using 2 alternative conditions (Method A or Method B) as described below and noted in the following table.

Method A: TEA (0.26 mL, 1.8 mmol, 6 eq.) was added to a solution of the compound of Part 2 (0.3 mmol, 1 eq) and the appropriate amine (R²NH₂, 0.6 mmol, 2 eq.) in MeCN (4 mL) and the resulting mixture stirred at 100 °C for 36 h. The reaction mixture was evaporated to dryness by Speedvac and the residue purified by prep-HPLC-4 to afford the title compound.

Method B: KF (53.2 mg, 0.9 mmol, 3 eq.) was added to a solution of the compound of Part 2 (0.3 mmol, 1 eq.) and the appropriate amine (R²NH₂, 0.6 mmol, 2 eq.) in DMSO (2 mL) and the mixture stirred at 100 °C for 16 h. The reaction was evaporated to dryness by Speedvac and the residue purified by prep-HPLC-4 to give to afford the title compound.

| **Example No** | **Name/Structure/R¹NH₂/R²NH₂/Method/Data** |
|---|---|
| **182** | 6-((6-amino-2-(difluoromethyl)pyrimidin-4-yl)amino)-N-ethyl-4-(ethylamino)nicotinamide (30) |
| | 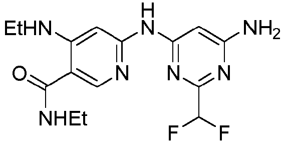 |
| | R¹NH₂: ethylamine; R²NH₂: ethylamine |
| | Method A |
| | Yield: 32%; LCMS m/z = 352 [M+H]⁺. |
| **183** | 6-((6-amino-2-(difluoromethyl)pyrimidin-4-yl)amino)-N-ethyl-4-(methylamino)nicotinamide (13) |
| | 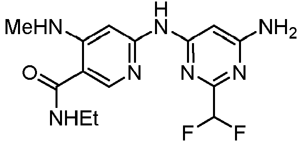 |
| | R¹NH₂: ethylamine; R²NH₂: methylamine |
| | Method A |
| | Yield: 27%; LCMS m/z = 338 [M+H]⁺. |
| **184** | (S)-6-((6-amino-2-(difluoromethyl)pyrimidin-4-yl)amino)-N-ethyl-4-((1-fluoropropan-2-yl)amino)nicotinamide (71) |
| | 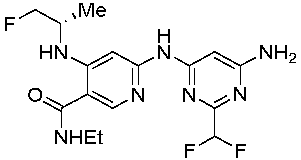 |
| | R¹NH₂: ethylamine; R²NH₂: (S)-1-fluoropropan-2-amine |
| | Method A |
| | Yield: 26%; LCMS m/z = 384 [M+H]⁺. |
| **185** | (R)-6-((6-amino-2-(difluoromethyl)pyrimidin-4-yl)amino)-N-ethyl-4-((1-fluoropropan-2-yl)amino)nicotinamide (72) |
| | 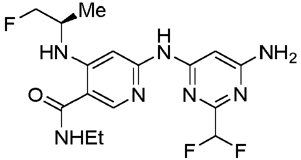 |
| | R¹NH₂: ethylamine; R²NH₂: (R)-1-fluoropropan-2-amine |
| | Method A |
| | Yield: 10%; LCMS m/z = 384 [M+H]⁺. |
| **186** | 6-((6-amino-2-(difluoromethyl)pyrimidin-4-yl)amino)-4-(cyclopropylamino)-N-(oxazol-4-ylmethyl)nicotinamide (134) |
| | 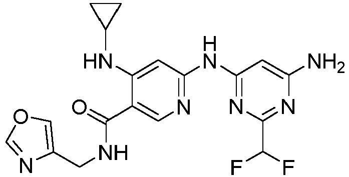 |
| | R¹NH₂: oxazol-4-ylmethanamine; R²NH₂: cyclopropylamine |
| | Method A |
| | Yield: 16%; LCMS m/z = 417 [M+H]⁺. |
| **187** | (S)-6-((6-amino-2-(difluoromethyl)pyrimidin-4-yl)amino)-4-((1-fluoropropan-2-yl)amino)-N-(oxazol-4-ylmethyl)nicotinamide (176) |
| | 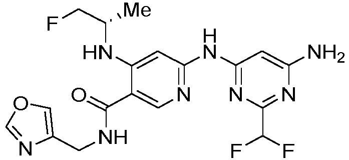 |
| | R¹NH₂: oxazol-4-ylmethanamine; R²NH₂: (S)-1-fluoropropan-2-amine |
| | Method A |
| | Yield: 6%; LCMS m/z = 437 [M+H]⁺. |
| **188** | (R)-6-((6-amino-2-(difluoromethyl)pyrimidin-4-yl)amino)-4-((1-fluoropropan-2-yl)amino)-N-(oxazol-4-ylmethyl)nicotinamide (177) |
| | 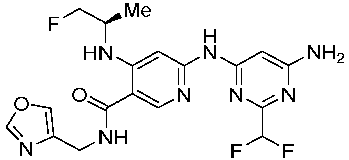 |
| | R¹NH₂: oxazol-4-ylmethanamine; R²NH₂: (R)-1-fluoropropan-2-amine |
| | Method A |
| | Yield: 5%; LCMS m/z = 437 [M+H]⁺. |
| **189** | 6-((6-amino-2-(difluoromethyl)pyrimidin-4-yl)amino)-4-(cyclopropylamino)-N-(3-(hydroxymethyl)bicyclo[1.1.1]pentan-1-yl)nicotinamide (174) |
| | 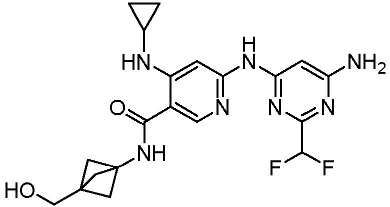 |
| | R¹NH₂: (3-aminobicyclo[1.1.1]pentan-1-yl)methanol; R²NH₂: cyclopropylamine |
| | Method B |
| | Yield: 3%; LCMS m/z = 432 [M+H]⁺. |
| **190** | (R)-6-((6-amino-2-(difluoromethyl)pyrimidin-4-yl)amino)-4-((1-fluoropropan-2-yl)amino)-N-(3-(hydroxymethyl)bicyclo[1.1.1]pentan-1-yl)nicotinamide (189) |
| | 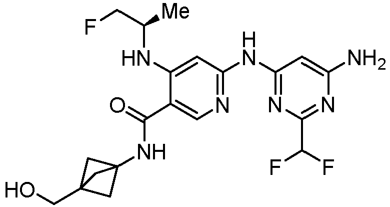 |
| | R¹NH₂: (3-aminobicyclo[1.1.1]pentan-1-yl)methanol; R²NH₂: (R)-1-fluoropropan-2-amine |
| | Method B |
| | Yield: 6%; LCMS m/z = 452 [M+H]⁺. |
| **191** | 6-((6-amino-2-(difluoromethyl)pyrimidin-4-yl)amino)-4-(ethylamino)-N-(pyridin-2-ylmethyl)nicotinamide (131) |
| | 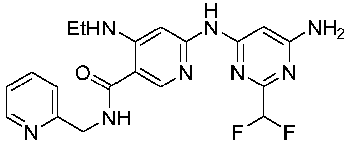 |
| | R¹NH₂: pyridin-2-ylmethanamine; R²NH₂: ethylamine |
| | Method B |
| | Yield: 5%; LCMS m/z = 415 [M+H]⁺. |
| **192** | 6-((6-amino-2-(difluoromethyl)pyrimidin-4-yl)amino)-4-(methylamino)-N-(pyridin-2-ylmethyl)nicotinamide (93) |
| | 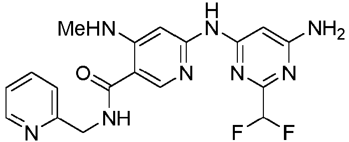 |
| | R¹NH₂: pyridin-2-ylmethanamine; R²NH₂: methylamine |
| | Method B |
| | Yield: 17%; LCMS m/z = 401 [M+H]⁺. |
| **193** | 6-((6-amino-2-(difluoromethyl)pyrimidin-4-yl)amino)-4-(cyclopropylamino)-N-(pyridin-2-ylmethyl)nicotinamide (153) |
| | 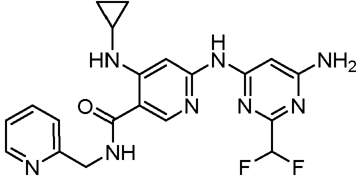 |
| | R¹NH₂: pyridin-2-ylmethanamine; R²NH₂: cyclopropylamine |
| | Method B |
| | Yield: 5%; LCMS m/z = 471 [M+H]⁺. |
| **194** | (R)-6-((6-amino-2-(difluoromethyl)pyrimidin-4-yl)amino)-4-((1-fluoropropan-2-yl)amino)-N-(pyridin-2-ylmethyl)nicotinamide (187) |
| | 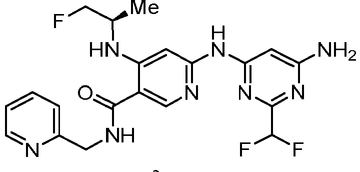 |
| | R¹NH₂: pyridin-2-ylmethanamine; R²NH₂: (R)-1-fluoropropan-2-amine |
| | Method B |
| | Yield: 15%; LCMS m/z = 447 [M+H]⁺. |

### Example 195-198

The title compounds were prepared from 6-chloro-4-fluoronicotinic acid using a 3-step chemistry protocol as shown in Scheme below.

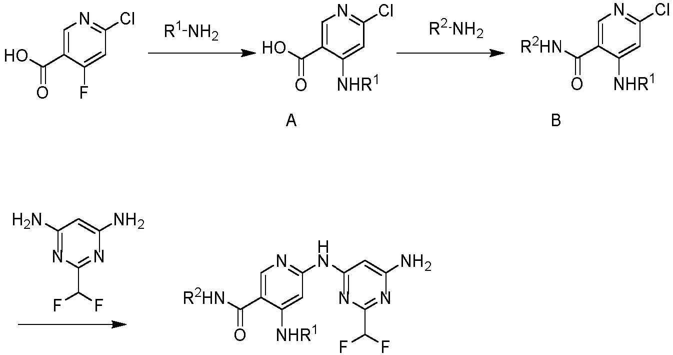

**Part 1.** To a solution of 6-chloro-4-fluoronicotinic acid (87.5 mg, 0.5 mmol, 1 eq.) and the appropriate amine (R¹NH₂, 0.45 mmol, 0.9 eq.) in MeCN (4 mL) was added TEA (0.580 mL, 4 mmol, 8 eq) and the mixture stirred at 100 °C for 16 h. The solvent was removed by Speedvac to give Compound A which was used in Part 2 without further purification.

**Part 2.** To a solution of the appropriate Compound A (Part 1, 0.45 mmol, 1 eq.) and the appropriate amine (R²NH₂, 0.675 mmol, 1.5 eq.) in DMF (4 mL) was added TEA (0.391 mL, 2.7 mmol, 6 eq.) and HATU (855 mg, 2.25 mmol, 5 eq.) and the mixture stirred at 30 °C for 2 h. The reaction was quenched with 5N Na₂CO₃ (4.0 mL) and extracted with EtOAc (3x 10 mL). The combined organics were concentrated by Speedvac to afford crude Compound B which was used in Part 3 without further purification.

**Part 3.** To a solution of the appropriate Compound B (Part 2, 0.3 mmol, 1 eq.) and 2-(difluoromethyl)pyrimidine-4,6-diamine (Preparation 8, 96 mg, 0.6 mmol, 2 eq.) in t-AmOH (4 mL) was added Cs₂CO₃ (293.4 mg, 0.9 mmol, 3 eq.) and Brettphos Pd G3 (13.6 mg, 15 □mol, 0.05 eq.) under N₂ and the mixture stirred at 120 °C for 2 h. The reaction mixture was diluted with H₂O (3.0 mL) and extracted with EtOAc (3x 10 mL). The combined organics were concentrated by Speedvac and the residue purified by prep-HPLC-4 to give the title compounds.

| **Example No** | **Name/Structure/R¹NH₂/R²NH₂/Data** |
|---|---|
| **195** | 6-((6-amino-2-(difluoromethyl)pyrimidin-4-yl)amino)-N-(cyanomethyl)-4-(cyclopropylamino)nicotinamide (54) |
| | 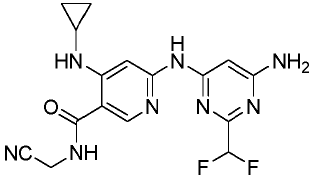 |
| | R¹NH₂: cyclopropylamine; R²NH₂: aminoacetonitrile |
| | Yield: 4%; LCMS m/z = 375 [M+H]⁺. |
| **196** | 6-((6-amino-2-(difluoromethyl)pyrimidin-4-yl)amino)-4-(ethylamino)-N-(oxazol-2-ylmethyl)nicotinamide (114) |
| | 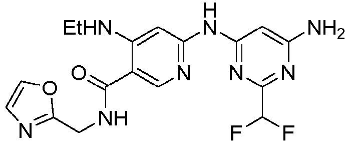 |
| | R¹NH₂: ethylamine; R²NH₂: oxazol-2-ylmethanamine |
| | Yield: 8%; LCMS m/z = 405 [M+H]⁺. |
| **197** | 6-((6-amino-2-(difluoromethyl)pyrimidin-4-yl)amino)-4-(cyclopropylamino)-N-(oxazol-2-ylmethyl)nicotinamide (135) |
| | 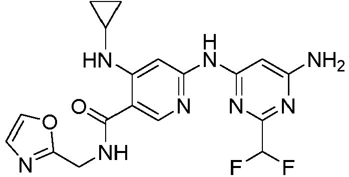 |
| | R¹NH₂: cyclopropylamine; R²NH₂: oxazol-2-ylmethanamine |
| | Yield: 6%; LCMS m/z = 417 [M+H]⁺. |
| **198** | 6-((6-amino-2-(difluoromethyl)pyrimidin-4-yl)amino)-4-(cyclopropyl amino)-N-((1s,3s)-3-hydroxycyclobutyl)nicotinamide (123) |
| | 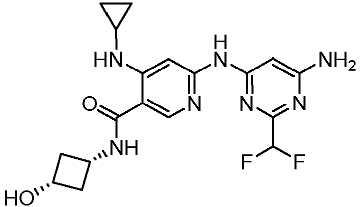 |
| | R¹NH₂: cyclopropylamine; R²NH₂: (1s,3s)-3-aminocyclobutan-1-ol |
| | Yield: 11%; LCMS m/z = 406 [M+H]⁺. |

### Example 199: 6-((6-amino-2-(difluoromethyl)pyrimidin-4-yl)amino)-4-(isopropyl amino)-N-(oxazol-4-ylmethyl)nicotinamide (147)

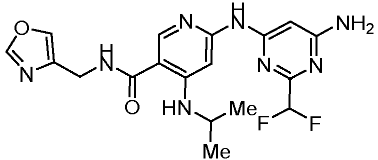

TFA (2 mL) was added to a solution of 6-((6-(tert-butylamino)-2-(difluoromethyl) pyrimidin-4-yl)amino)-4-(isopropylamino)-N-(oxazol-4-ylmethyl)nicotinamide (Preparation 258, 100 mg, 0.210 mmol) in THF and the mixture stirred at 70 °C for 12 h. The reaction was concentrated *in vacuo* and the residue purified by prep-HPLC-3, to afford the title compound as a white solid (45.6 mg, 52%). LCMS m/z = 419 [M+H]⁺. ¹H NMR (500 MHz, DMSO-d₆) δ: 9.70 (s, 1H), 8.79 (t, 1H), 8.40 (s, 1H), 8.32 (s, 1H), 8.29 (d, 1H), 7.70 (s, 1H), 7.11 (s, 1H), 6.89 (s, 2H), 6.76 (s, 1H), 6.48 (t, 1H), 4.31 (d, 2H), 3.59-3.55 (m, 1H), 1.20 (d, 6H).

### Example 200: 2-(difluoromethyl)-N4-(4-(isopropylamino)-5-(3-methyl-1,2,4-oxadiazol-5-yl)pyridin-2-yl)pyrimidine-4,6-diamine (58)

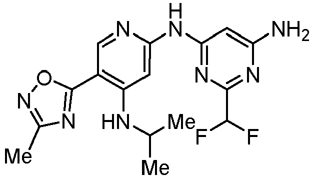

The title compound was prepared as a white solid (33.6 mg, 49%), from N4-(tert-butyl)-2-(difluoromethyl)-N6-(4-(isopropylamino)-5-(3-methyl-1,2,4-oxadiazol-5-yl)pyridin-2-yl)pyrimidine-4,6-diamine (Preparation 265) using a similar method to that described for Example 199. LCMS m/z = 377 [M+H]⁺; ¹H NMR (500 MHz, DMSO-d₆) δ: 9.96 (s, 1H), 8.61 (s, 1H), 7.73 (d, 1H), 7.36 (s, 1H), 6.97 (s, 2H), 6.74 (s, 1H), 6.51 (t, 1H), 3.76-3.70 (m, 1H), 2.42 (s, 1H), 1.29 (d, 6H).

### Example 201: 6-((6-amino-2-(difluoromethyl)pyrimidin-4-yl)amino)-4-(ethyl amino)-N-(2-azaspiro[3.3]heptan-6-yl)nicotinamide (149)

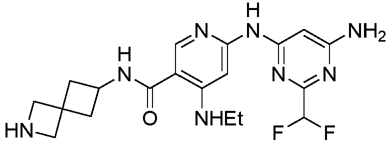

TFA (137 mg,1.2 mmol) was added to a solution of tert-butyl 6-(6-((6-amino-2-(difluoro methyl)pyrimidin-4-yl)amino)-4-(ethylamino)nicotinamido)-2-azaspiro[3.3]heptane-2-carboxylate (Preparation 266, 120 mg, 0.231 mmol) in DCM and the mixture stirred at rt for 12 h. The mixture was concentrated *in vacuo* and the residue was purified by Prep HPLC-3 to afford the title compound as a white solid (45.7 mg, 47%). LCMS m/z = 419 [M+H]⁺; ¹H NMR (400 MHz, DMSO-d₆) δ: 9.69 (s, 1H), 8.43 (s, 1H), 8.33 (s, 1H), 8.21-8.19 (m, 1H), 7.02 (s, 1H), 6.88 (s, 2H), 6.80 (s, 1H), 6.48 (t, 1H), 4.18 (s, 1H), 3.83-3.10 (m, 7H), 2.43-2.05 (m, 4H), 1.18 (t, 3H).

### Example 202: 6-((6-amino-4-(difluoromethyl)pyridin-2-yl)amino)-4-(((1R,2S)-2-fluorocyclopropyl)amino)-N-methylnicotinamide (44)

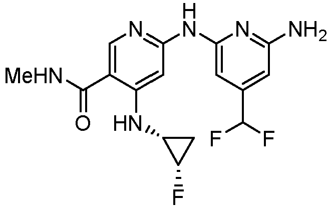

TFA (1 mL) was added to a solution of 6-((4-(difluoromethyl)-6-((2,4-dimethoxy benzyl)amino)pyridin-2-yl)amino)-4-(((1R,2S)-2-fluorocyclopropyl)amino)-N-methyl nicotinamide (Preparation 271, 60 mg, 0.115 mmol) in DCM (4 mL) was stirred at rt for 16 h. The reaction mixture was evaporated to dryness *in vacuo* and the residue purified by prep HPLC-3 to afford the title compound as a white solid (9.3 mg, 22%). LCMS m/z = 368 [M+H]⁺; ¹H NMR (400 MHz, DMSO-d₆) δ: 9.77 (s, 1H), 8.56 (s, 1H), 8.34-8.33 (m, 2H), 7.37 (s, 1H), 6.90-6.89 (m, 2H), 6.79 (s, 1H), 6.49 (t, 1H), 4.88 (d, 1H), 3.77 (s, 1H), 2.72 (d, 3H), 1.21-1.14 (m, 1H), 0.89-0.81 (m, 1H).

### Example 203: 6-((6-amino-4-(difluoromethyl)pyridin-2-yl)amino)-4-(((1S,2R)-2-fluorocyclopropyl)amino)-N-methylnicotinamide (45)

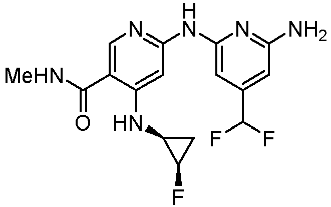

The title compound was prepared as a white solid (9.1 mg, 21%) from 6-((4-(difluoro methyl)-6-((2,4-dimethoxybenzyl)amino)pyridin-2-yl)amino)-4-(((1S,2R)-2-fluorocyclopropyl) amino)-N-methylnicotinamide (Preparation 272) using a similar method to that described for Example 202. LCMS m/z = 368 [M+H]⁺; ¹H NMR (400 MHz, DMSO-d₆) δ: 9.77 (s, 1H), 8.56 (s, 1H), 8.36-8.35 (m, 2H), 7.38 (s, 1H), 6.91 (s, 2H), 6.79 (s, 1H), 6.49 (t, 1H), 4.98-4.81 (m, 1H), 3.85-3.78 (m, 1H), 2.73-2.66 (m, 3H), 1.22-1.17 (m, 1H), 0.90-0.84 (m, 1H).

Examples 111, 146, 166 and 167 were intentionally omitted from the Preparation of Exemplified Compounds.

### Inhibition of autophosphorylation of KIT

The results obtained in these experiments for compounds prepared according to the examples are summarized in Table 1 below. Table 1 shows the activity of the exemplified compounds in the pKIT assay according to the Meso Scale Discovery (MSD) assay described herein.

In this MSD assay, specific capture antibodies for the analytes were coated in arrays in each well of a 96-well carbon electrode plate surface. The detection system used SULFO-TAG^{™} labels that emit light upon electrochemical stimulation initiated at the electrode surfaces of the MULTIARRAY and MULTI-SPOT^{®} plates. The electrical stimulation was decoupled from the output signal, which was light and generated assays with minimal background. The goal of this assay was to assess the percent inhibition of autophosphorylation of c-kit in cells when treated with compounds of the invention in a dose-dependent manner.

Materials included:
HMC 1.1 (exon 11)
HMC 1.1 (exon 11/13)
IMDM medium (Gibco: 12440053)
IMDM medium (phenol red free) (Gibco: 21056023)
Penicillin-Steptomycin (Gibco: 15140122)
HyClone^{™} Calf Serum, supplemented with Iron (GE Healthcare Life Sciences: SH3007203.03)
Screen Matrix 384-well cone-bottom plate (Thermo: 4309)
96-well clear U-bottom plates (individually wrapped, sterile) (Falcon: 353077)
96-well clear V-bottom plate (sterile) (Grenier: 651161)
AlphaLISA 5X Lysis buffer (PerkinElmer: AL003F)
Halt^{™} Protease Inhibitor Cocktail, EDTA-Free (100X) (ThermoFisher: 87785)
Phospho c-Kit (Tyr721) Assay Whole Cell Lysate Kit (MSD: 515DPD)*

HMC1.2 cells were maintained in IMDM media supplemented with 10% Calf Serum with Iron and 100 units/mL Penicillin-Streptomycin and grown in a 37°C humidified tissue culture incubator. HMC1.2 cells were passaged by 1:10 dilution into fresh IMDM media supplemented with 10% Calf Serum with Iron and 100 units/mL Penicillin-Streptomycin every 2-3 days and were discarded after 20 passages.

HMC1.1 cells were maintained in IMDM media supplemented with 20% Calf Serum with Iron and 100 units/mL Penicillin-Streptomycin and grown in a 37°C humidified tissue culture incubator. HMC1.1 cells were passaged by 1:5 dilution into fresh IMDM media supplemented with 20% Calf Serum with Iron and 100 units/mL Penicillin-Streptomycin every 5-7 days and were discarded after 20 passages.

Seeding was performed as follows: HMC cells were brought to 1x10⁶ cells/mL in fresh phenol red free, serum-free, iron-free IMDM media with Pen/Strep; cells were seeded/plated into respective U-bottom plate by dispensing 50µL/well (50,000 cells) using the Multidrop, and covered and incubated cells at 37°C in a humidified tissue culture incubator for 4 hours (starvation).

Dosing was performed as follows: The compound mother plate was prepared by adding 16µL of compound to 24µL of DMSO to row A, odd-numbered columns of screen matrix plate. The Bravo protocol 5 cpds 8-point 4-fold serial dilution vertical for 96-well was used to: add 30µL DMSO to all other columns and serially dilute compounds 1:4 from 4mM to 244nM. Intermediate plates were prepared in V-bottom plates by dispensing 98µL of phenol red free, serum free IMDM media with Pen/Strep into all wells. 2µL DMSO or 2µL 10mM staurosporine was added to appropriate control wells. At the end of four hours, the Bravo protocol 10 cpds vertical dose MSD 2 U-plates 96-well was used to: add 2µL from the compound plate to the intermediate plate and dose cells with 7µL from the intermediate plate. The cells were covered and incubated at 37°C in a humidified tissue culture incubator for 90 min. The complete lysis buffer was prepared with AlphaLISA 5X Lysis Buffer supplemented with 1X protease inhibitor cocktail and kept on ice. The cells were pelleted by centrifuging plate at 2500 xg for 5min, 4°C and media removed. Complete lysis buffer was added (14µL/well). The cells were covered and shaken at 4°C on a plate shaker at 600rpm for 30min and kept cold

The MSD protocol was preformed as follows: Tris Wash Buffer (2L) was prepared by diluting 10X stock of Tris Wash Buffer to 1X in water. Blocking Solution was prepared by dissolving 600mg Blocker A in 20mL 1X Trish Wash Buffer (per plate). The Antibody Dilution Buffer was prepared by mixing 1mL Blocking solution and 2mL 1X Trish Wash Buffer (per plate) and kept on ice. Blocking Solution was added (150µL/well) to each MSD plate using a multichannel repeater pipette. The plate was covered and incubated at room temperature on a plate shaker at 700rpm for 1hr. The Bio Tek protocol RL-96w-wash-MSD_stacker_fromC was used to wash plates 3X with 150µL/well. Cells were pelleted by centrifuging lysate plates at 2500 xg for 5 mins. The Bravo protocol was used (transfer 25 ul supernatants from V-96 to assay plate) to transfer 25µL sample lysate per well to each MSD plate. The plates were covered and incubated at room temperature on a plate shaker at 700rpm for 1hr. The Bio Tek protocol RL-96w-wash-MSD_stacker_fromC was used to wash plates 3X with 150µL/well. The Detection Antibody Solution was prepared by diluting the 60µL of the 50X antibody stock in 2.94mL of the Antibody Dilution Buffer (per plate) and kept on ice. 25µl/well of detection antibody solution was added to each MSD plate. The plates were covered and incubated at room temperature on a plate shaker at 700rpm for 1hr. The Read Buffer was prepared by diluting 5mL of 4X stock of Read Buffer to 1X in 15mL water (per plate). The Bio Tek protocol RL-96w-wash-MSD_stacker_fromC was used to wash plates 3X with 150µL/well. Read Buffer was added 150µL/well to each MSD plate. The plate was read on an MSD Sector Imager within 5 minutes of adding read buffer.

Table 1 shows the activity of the exemplified compounds in the pKIT assay according to the Meso Scale Discovery (MSD) assay described herein. "A" represents IC₅₀ of less than or equal to 10.0 nM (IC₅₀ ≤ 10.0 nm), "B" represents IC50 of greater than 10.0 nM and equal to or less than 40.0 nM (10.0 nM < IC₅₀ ≤ 40.0 nM), and "C" represents IC50 of greater than 40.0 nM (IC₅₀ > 40.0).

**Table 1**

| **Cpd. No.** | **Structure** | **Code** |
|---|---|---|
| **1** | 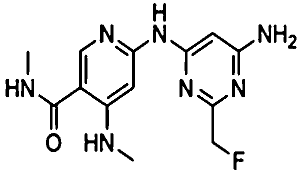 | B |
| **2** | 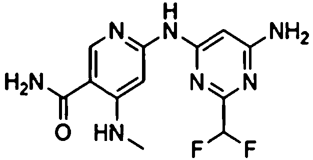 | C |
| **3** | 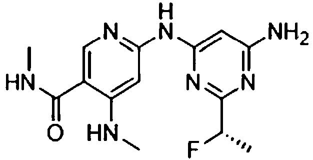 | B |
| **4** | 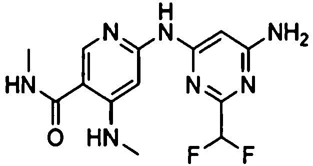 | A |
| **5** | 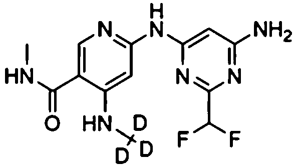 | A |
| **6** | 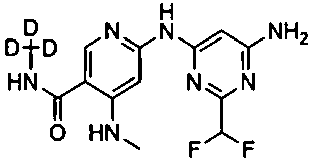 | B |
| **7** | 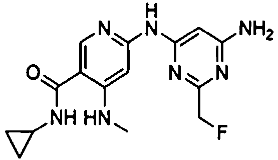 | B |
| **8** | 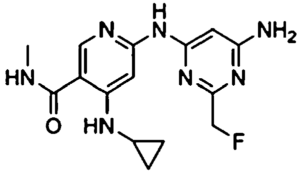 | A |
| **9** | 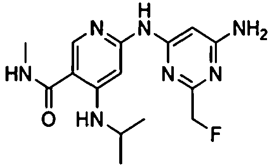 | A |
| **10** | 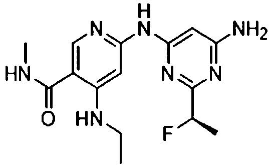 | C |
| **11** | 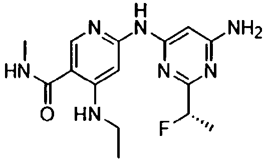 | A |
| **12** | 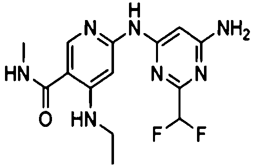 | A |
| **13** | 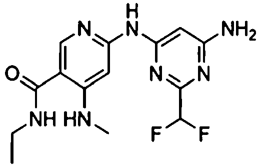 | B |
| **14** | 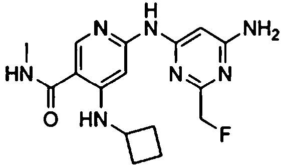 | A |
| **15** | 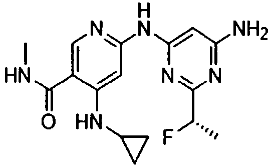 | B |
| **16** | 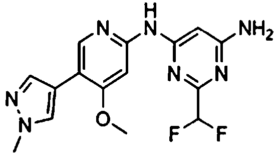 | A |
| **17** | 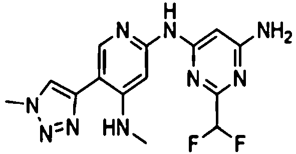 | B |
| **18** | 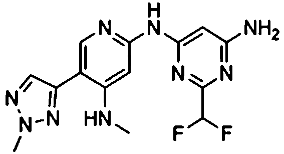 | B |
| **19** | 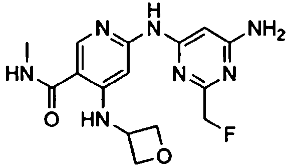 | C |
| **20** | 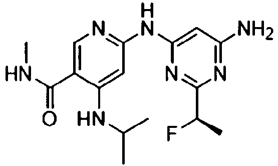 | C |
| **21** | 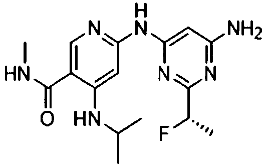 | A |
| **22** | 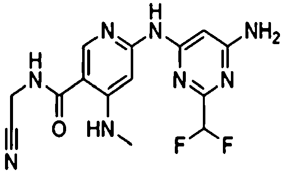 | C |
| **23** | 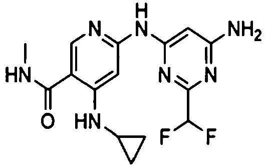 | A |
| **24** | 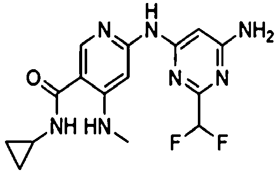 | A |
| **25** | 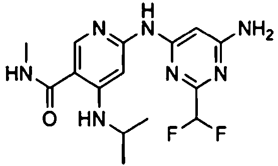 | A |
| **26** | 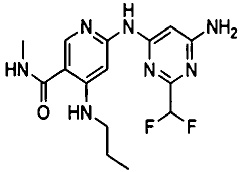 | A |
| **27** | 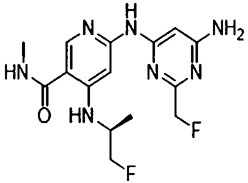 | B |
| **28** | 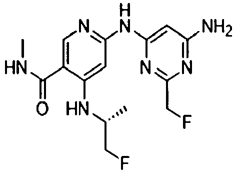 | C |
| **29** | 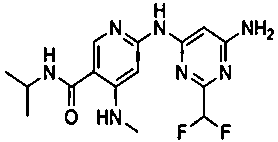 | A |
| **30** | 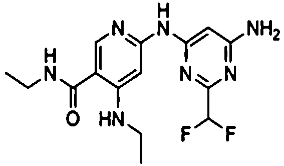 | B |
| **31** | 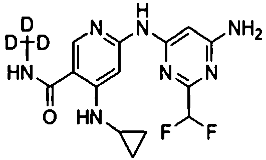 | A |
| **32** | 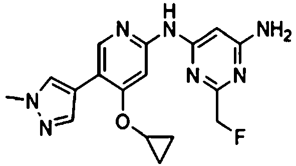 | A |
| **33** | 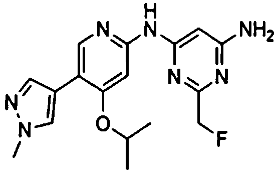 | A |
| **34** | 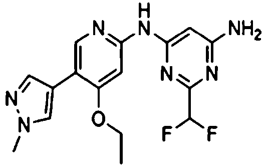 | A |
| **35** | 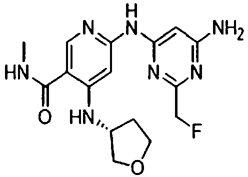 | C |
| **36** | 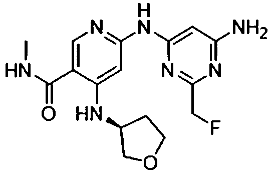 | C |
| **37** | 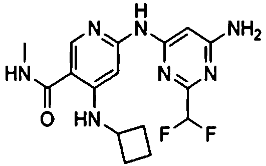 | A |
| **38** | 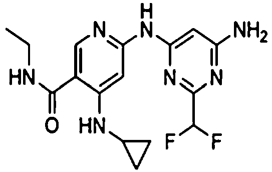 | A |
| **39** | 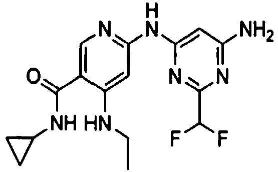 | A |
| **42** | 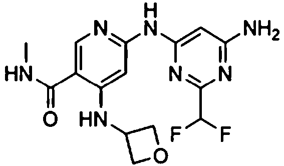 | B |
| **43** | 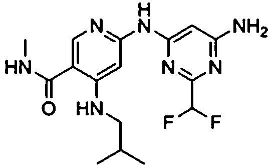 | A |
| **44** | 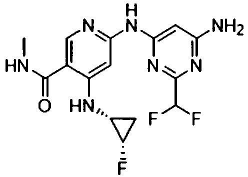 | B |
| **45** | 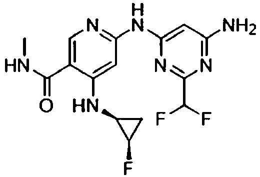 | B |
| **46** | 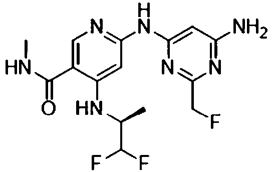 | A |
| **47** | 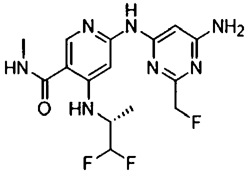 | A |
| **48** | 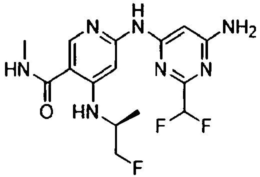 | B |
| **49** | 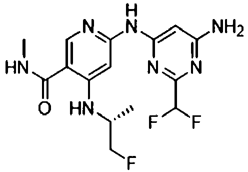 | A |
| **50** | 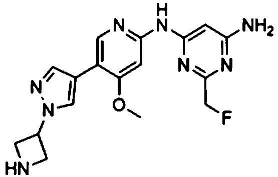 | C |
| **51** | 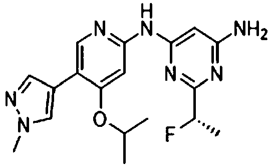 | A |
| **52** | 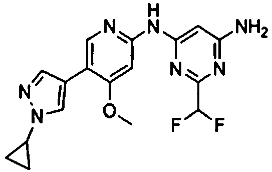 | A |
| **53** | 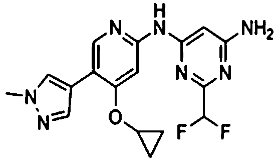 | A |
| **54** | 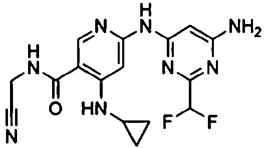 | B |
| **55** | 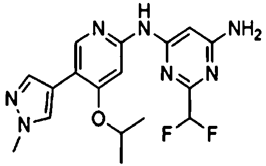 | A |
| **56** | 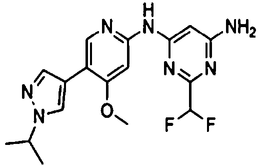 | A |
| **57** | 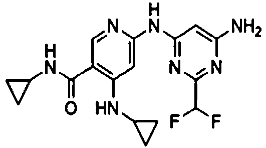 | A |
| **58** | 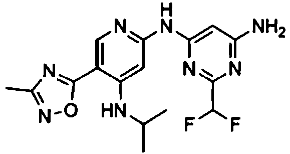 | C |
| **59** | 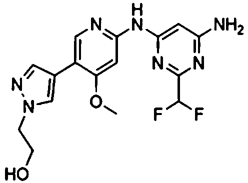 | B |
| **60** | 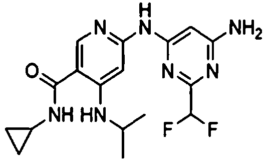 | A |
| **61** | 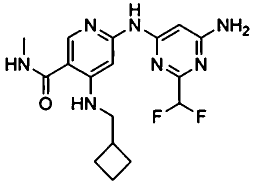 | B |
| **62** | 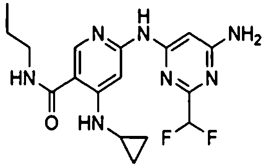 | B |
| **63** | 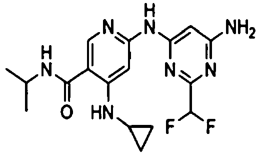 | A |
| **64** | 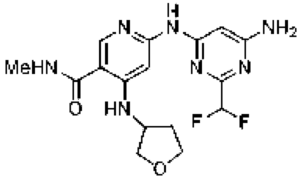 | B |
| 65 | 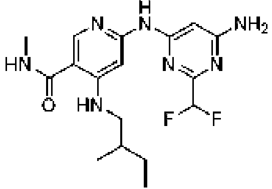 | B |
| 66 | 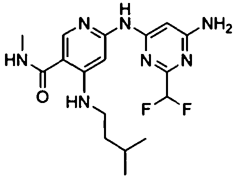 | B |
| 67 | 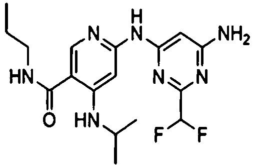 | A |
| 69 | 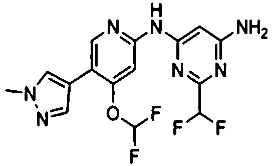 | B |
| 70 | 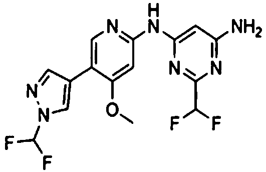 | A |
| 7**1** | 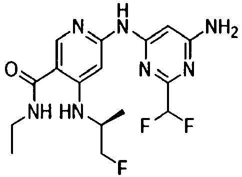 | A |
| 72 | 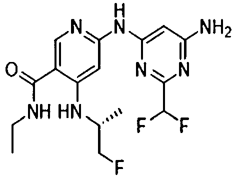 | A |
| 73 | 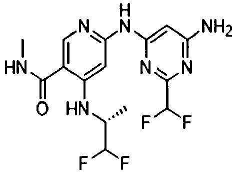 | A |
| 74 | 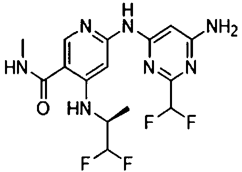 | A |
| 75 | 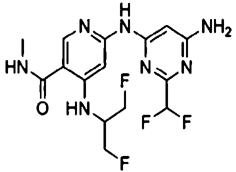 | A |
| 76 | 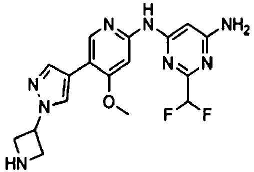 | C |
| 77 | 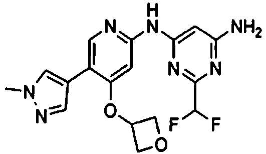 | A |
| 78 | 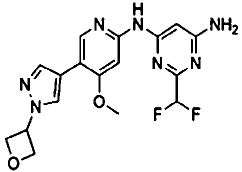 | A |
| 79 | 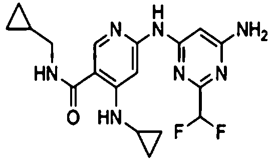 | B |
| 80 | 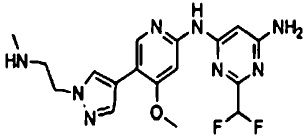 | A |
| 81 | 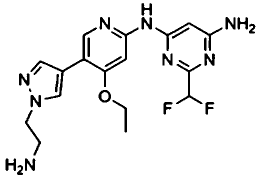 | B |
| 82 | 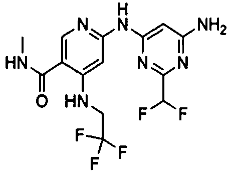 | A |
| **83** | 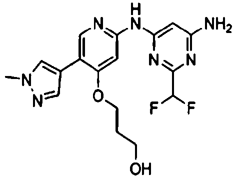 | B |
| **84** | 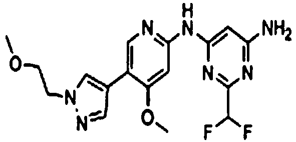 | A |
| **85** | 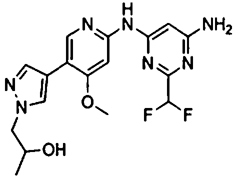 | B |
| **86** | 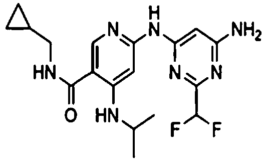 | B |
| **87** | 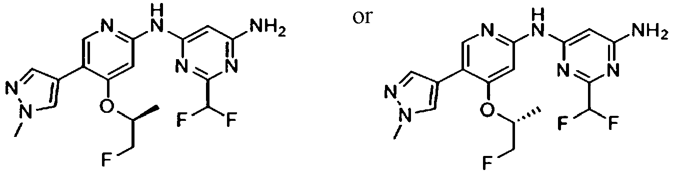 | A |
| **88** | 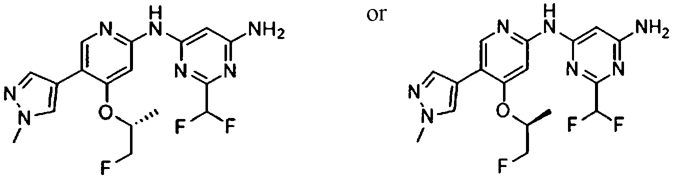 | A |
| **89** | 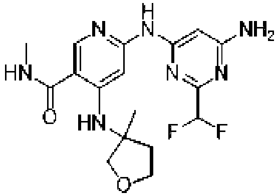 | A |
| **90** | 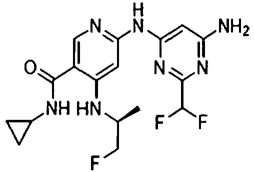 | A |
| **91** | 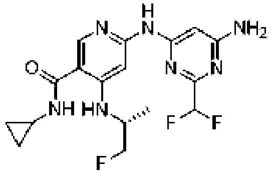 | A |
| **92** | 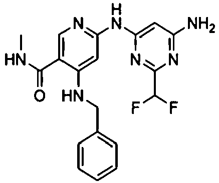 | B |
| **93** | 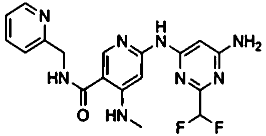 | B |
| **94** | 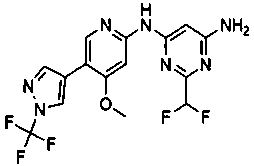 | B |
| **95** | 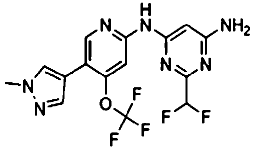 | A |
| **96** | 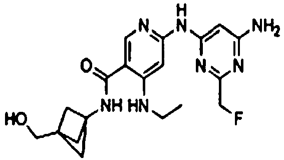 | B |
| **97** | 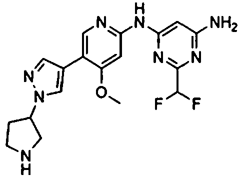 | B |
| **98** | 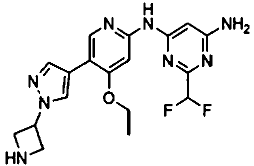 | B |
| **99** | 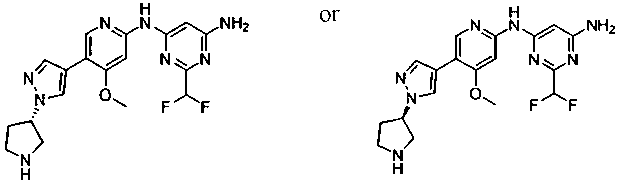 | B |
| **100** | 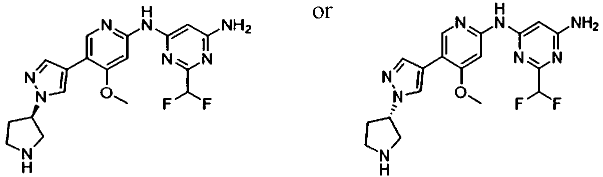 | B |
| **101** | 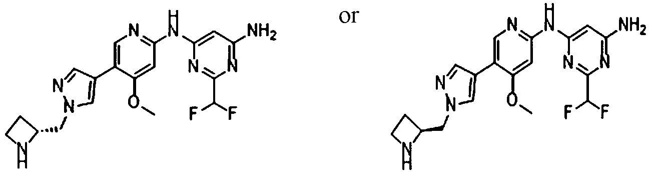 | C |
| **102** | 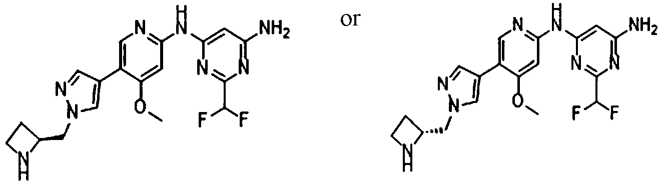 | A |
| **103** | 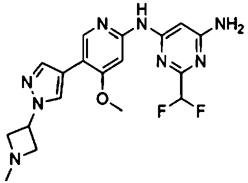 | A |
| **104** | 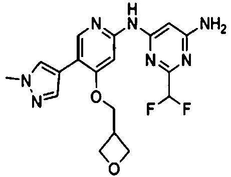 | A |
| **105** | 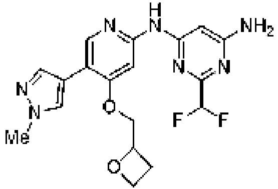 | B |
| **106** | 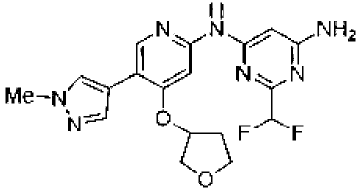 | A |
| **107** | 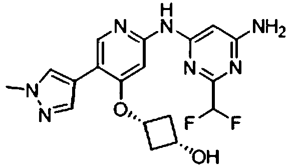 | B |
| **108** | 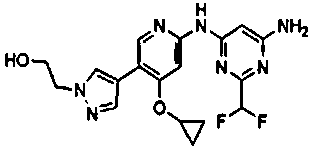 | A |
| **109** | 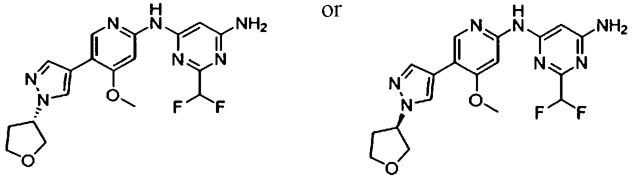 | A |
| **110** | 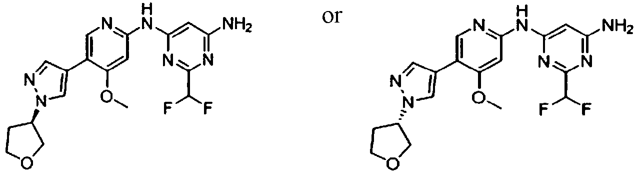 | A |
| **111** | 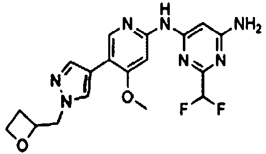 | A |
| **112** | 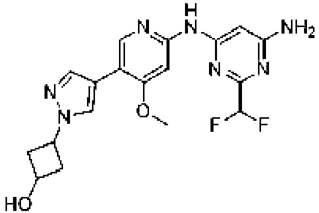 | B |
| **113** | 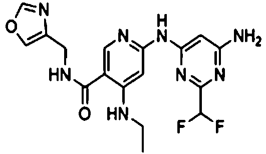 | A |
| **114** | 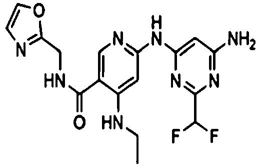 | B |
| **115** | 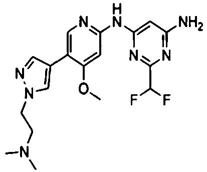 | B |
| **116** | 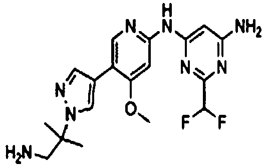 | B |
| **117** | 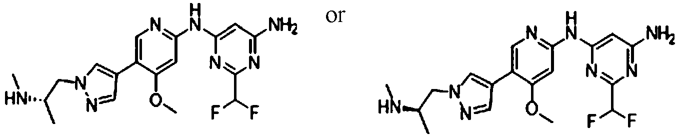 | A |
| **118** | 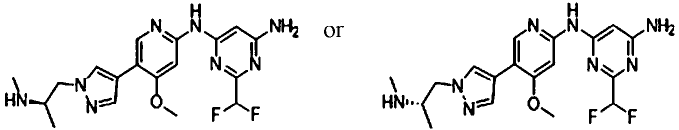 | B |
| **119** | 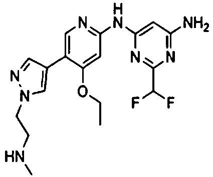 | A |
| **120** | 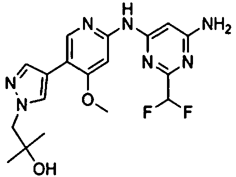 | A |
| **121** | 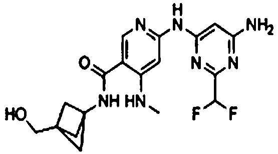 | B |
| **122** | 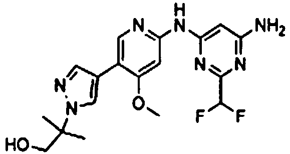 | A |
| **123** | 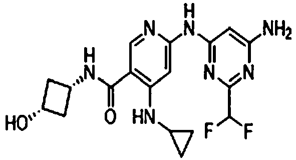 | B |
| **125** | 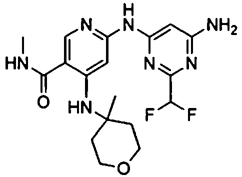 | A |
| **126** | 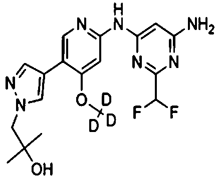 | A |
| **127** | 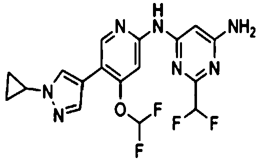 | A |
| **128** | 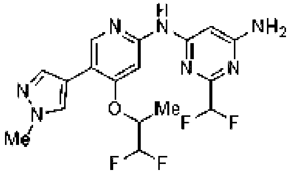 | A |
| **129** | 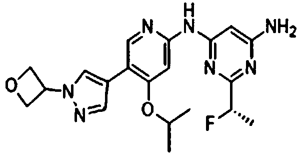 | A |
| **130** | 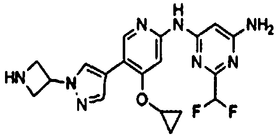 | B |
| **131** | 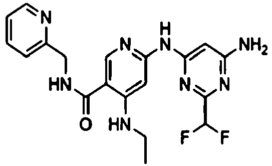 | A |
| **132** | 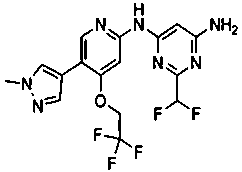 | A |
| **133** | 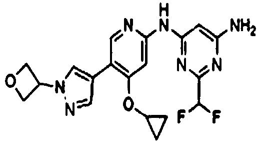 | A |
| **134** | 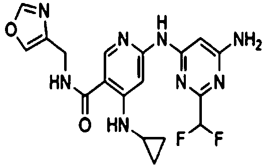 | A |
| **135** | 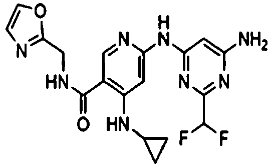 | A |
| **136** | 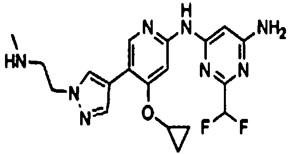 | A |
| **137** | 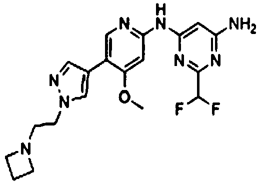 | B |
| **138** | 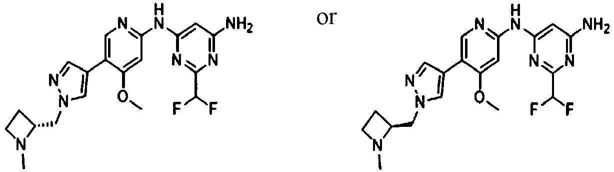 | A |
| **139** | 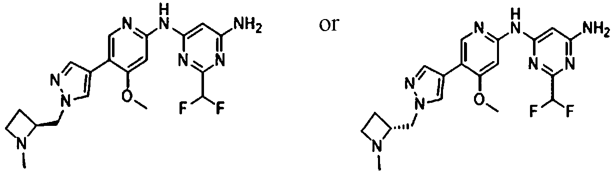 | A |
| **140** | 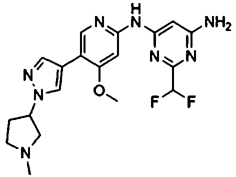 | A |
| **141** | 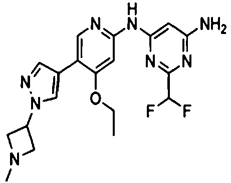 | A |
| **142** | 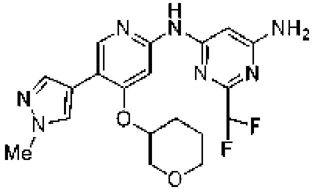 | A |
| **143** | 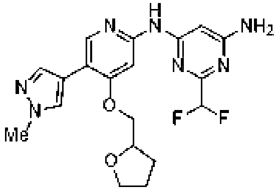 | A |
| **144** | 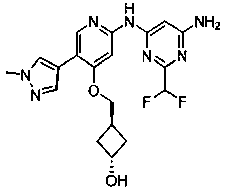 | B |
| **145** | 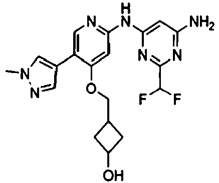 | B |
| **146** | 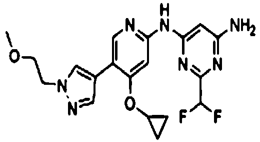 | A |
| **147** | 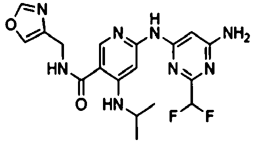 | A |
| **148** | 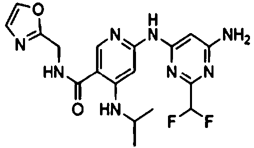 | A |
| **149** | 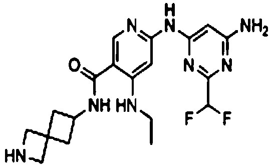 | C |
| **150** | 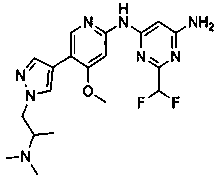 | A |
| **151** | 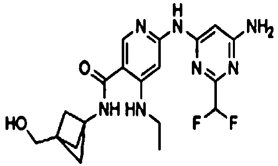 | A |
| **152** | 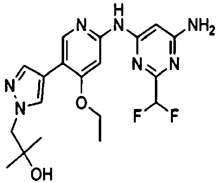 | A |
| **153** | 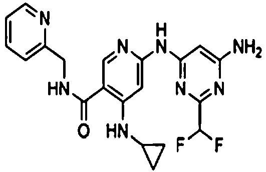 | A |
| **154** | 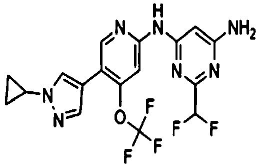 | A |
| **155** | 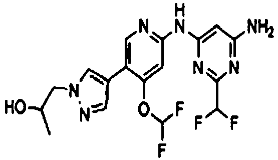 | A |
| **156** | 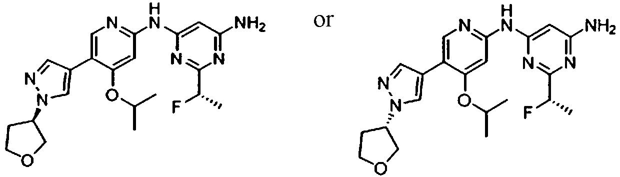 | A |
| **157** | 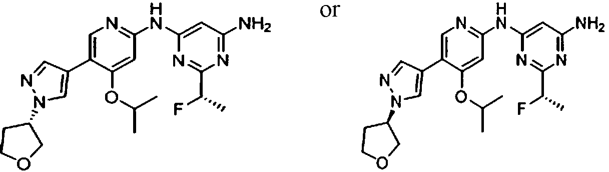 | A |
| **158** | 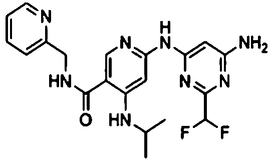 | A |
| **159** | 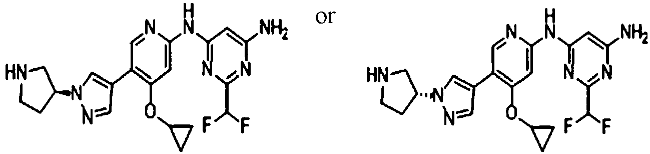 | A |
| **160** | 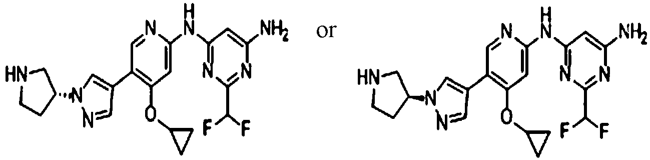 | A |
| **161** | 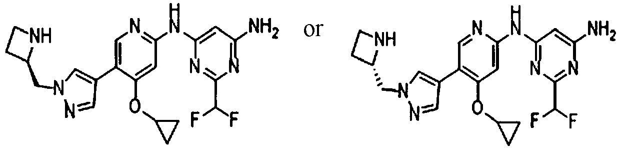 | A |
| **162** | 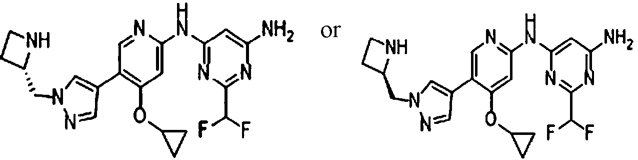 | A |
| **163** | 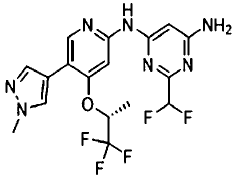 | A |
| **164** | 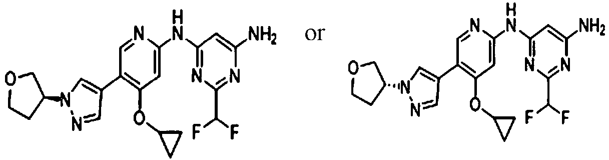 | A |
| **165** | 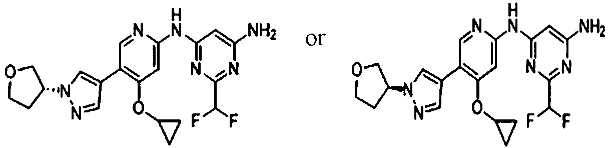 | A |
| **166** | 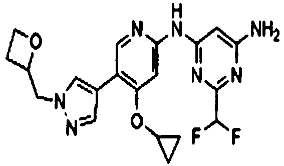 | A |
| **167** | 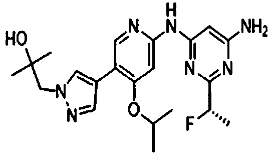 | A |
| **168** | 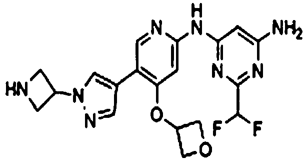 | C |
| **169** | 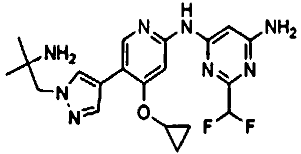 | A |
| **170** | 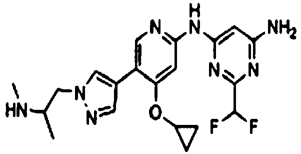 | A |
| **171** | 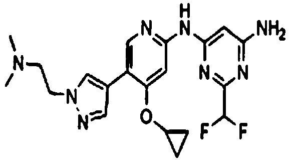 | A |
| **172** | 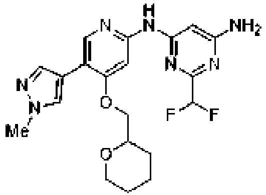 | B |
| **173** | 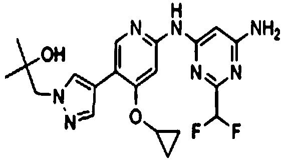 | A |
| **174** | 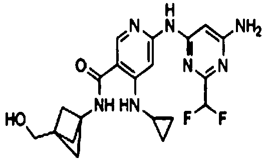 | A |
| **175** | 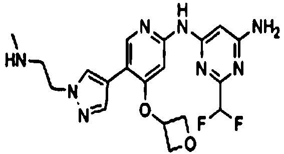 | C |
| **176** | 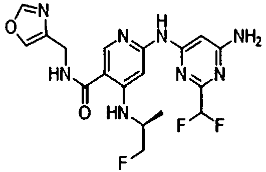 | A |
| **177** | 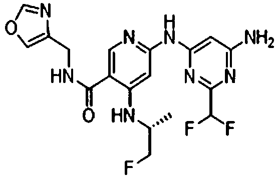 | B |
| **178** | 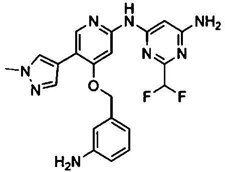 | A |
| **179** | 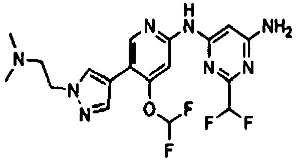 | A |
| **180** | 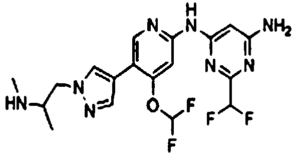 | A |
| **181** | 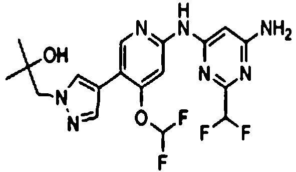 | A |
| **182** | 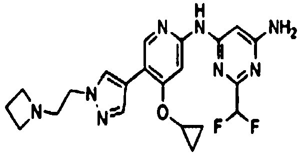 | A |
| **183** | 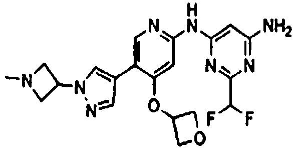 | C |
| **184** | 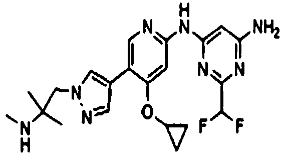 | A |
| **185** | 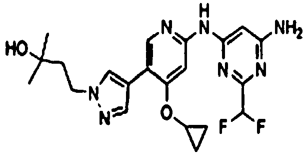 | A |
| **186** | 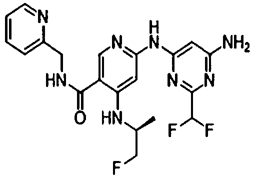 | A |
| **187** | 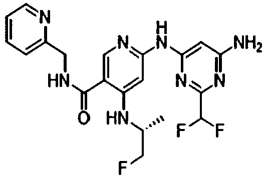 | B |
| **188** | 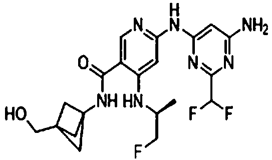 | B |
| **189** | 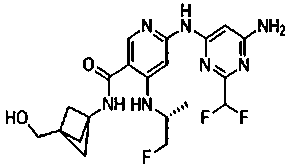 | B |
| **190** | 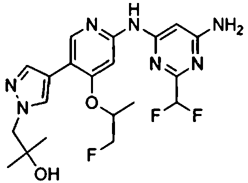 | A |
| **191** | 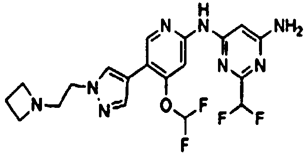 | A |
| **192** | 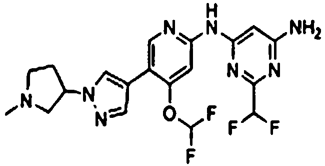 | A |
| **193** | 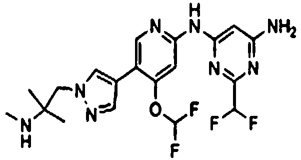 | A |
| **194** | 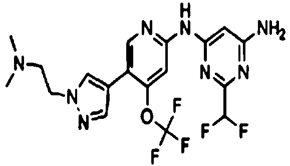 | A |
| **195** | 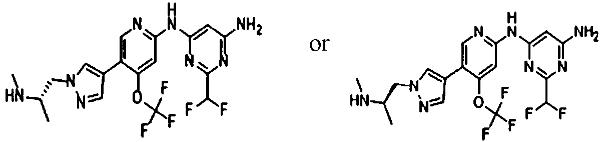 | A |
| **196** | 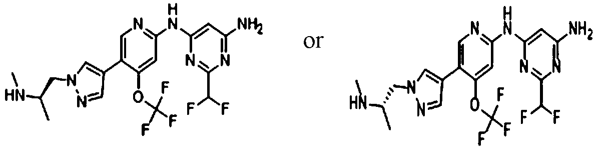 | A |
| **197** | 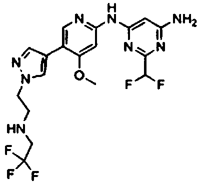 | A |
| **198** | 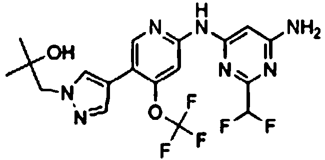 | A |
| **199** | 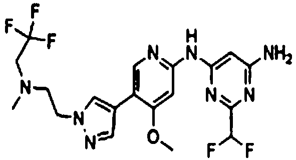 | A |
| **200** | 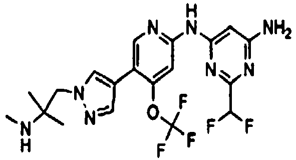 | A |
| **201** | 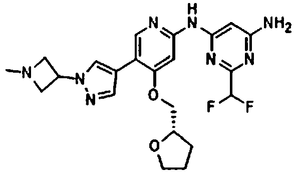 | B |
| **202** | 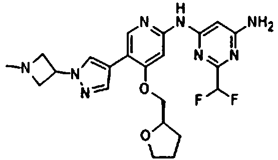 | B |
| **203** | 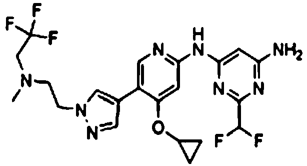 | A |

Compounds 40, 41, 68 and 124 were intentionally omitted from Table 1.

Additional Compounds 204-211 fall within the scope of Formula (I), see Table 2 below, were also tested in the pKIT assay (pKIT HMC1.1_V654A) and all of these compounds had inhibitory activities of greater than 625 nM.

**Table 2**

| **Cpd. No.** | **Structure** |
|---|---|
| **204** | 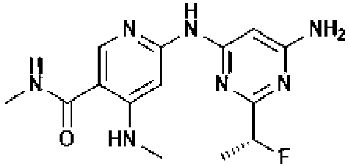 |
| **205** | 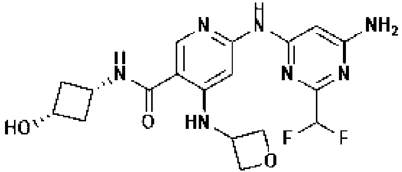 |
| **206** | 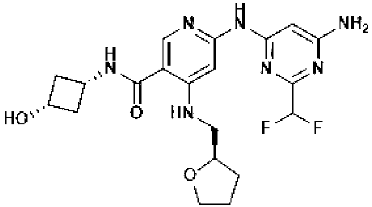 |
| **207** | 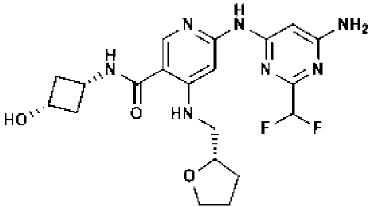 |
| **208** | 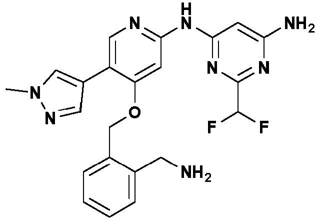 |
| **209** | 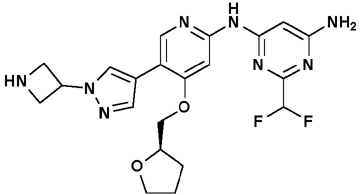 |
| **210** | 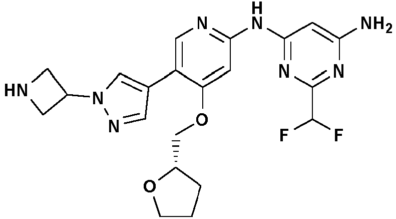 |
| **211** | 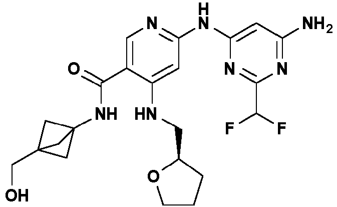 |

## Claims

1. A compound of Formula (I): 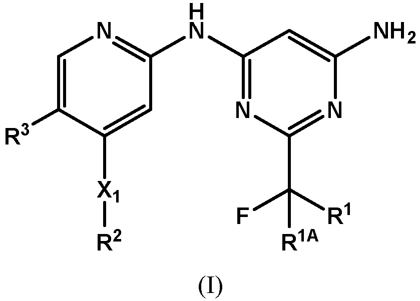
or a pharmaceutically acceptable salt thereof, wherein:
R¹ and R^{1A} are each independently selected from H, halogen, and CH₃,
or R¹ and R^{1A} taken together with the carbon to which they are attached form cyclopropyl;
R² is selected from C₁₋₅alkyl, CD₃, C₃₋₆cycloalkyl, bicyclo[1.1.1]pentane, and 4- to 6-membered heterocycle containing O, wherein said alkyl, cycloalkyl or heterocycle is optionally substituted with 1-3 R⁴;
each R⁴ is independently selected from halogen, CH₃, C₂₋₃alkenyl, OH, CH₂OH, C₄₋₆cycloalkyl, 4- to 6-membered heterocycle containing O, and phenyl, wherein said alkyl, cycloalkyl, heterocycle or phenyl is optionally substituted with OH or NH₂, C₁₋₂alkyl, CH₂NH₂, or halogen;
R³ is C(O)NHR⁵, 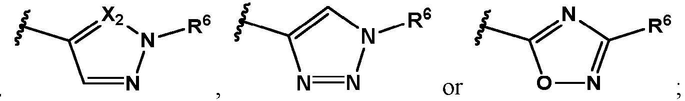
X₁ is NH or O;
X₂ is N or CH;
X₃ is N or CH;
R⁵ is selected from H, C₁₋₃alkyl, CD₃, C₃₋₄cycloalkyl and bicyclo[1.1.1] pentane, wherein said alkyl, cycloalkyl or bicyclo[1.1.1.]pentane is optionally substituted with 1-2 R⁷;
each R⁷ is independently selected from CN, NH₂, OH, CH₂OH, cyclopropyl, pyridinyl, and oxazolyl, or taken together two R⁷ attached to the same carbon atom form 4-membered heterocycle containing N;
R⁶ is selected from C₁₋₅alkyl, CHF₂, CF₃, 4- or 5-membered heterocycle containing N or O, and C₃₋₄cycloalkyl, wherein said alkyl or heterocycle is optionally substituted with one R⁸;
R⁸ is selected from OH, NR⁹R⁹, OCH₃, CH₃ and 4-membered heterocycle containing N or O, wherein said alkyl or heterocycle is optionally substituted with one R¹⁰;
each R⁹ is independently selected from H, CH₃ and CH₂CF₃; and
R¹⁰ is selected from CH₃ and CF₃.

2. The compound of claim 1, or a pharmaceutically acceptable salt thereof, wherein:
R¹ and R^{1A} are each independently selected from H, halogen and CH₃;
R² is selected from C₁₋₅alkyl, CD₃, C₃₋₆cycloalkyl and 4- to 6-membered heterocycle containing O, wherein said alkyl, cycloalkyl or heterocycle is optionally substituted with 1-3 R⁴;
each R⁴ is independently selected from halogen, CH₃, OH, C₄₋₆cycloalkyl, 4- to 6-membered heterocycle containing O, and phenyl, wherein said cycloalkyl or phenyl is optionally substituted with OH or NH₂;
each R⁷ is independently selected from CN, OH, CH₂OH, cyclopropyl, pyridinyl, and oxazolyl, or taken together two R⁷ attached to the same carbon atom form 4-membered heterocycle containing N; and
R¹⁰ is CH₃.

3. The compound of claim 1 or 2, wherein the compound is represented by Formula (II): 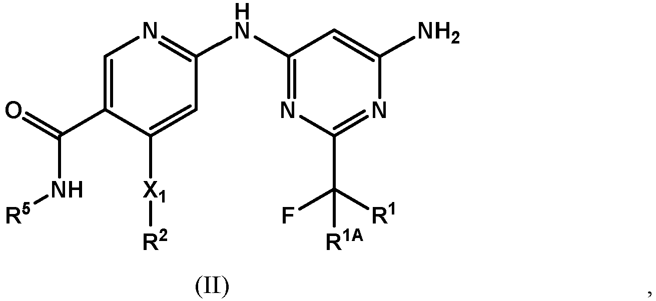
or a pharmaceutically acceptable salt thereof; or
wherein the compound is represented by Formula (III): 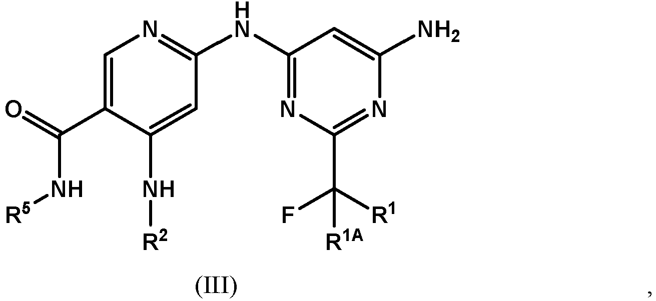
or a pharmaceutically acceptable salt thereof.

4. The compound of claim 3, or a pharmaceutically acceptable salt thereof, wherein:
R¹ and R^{1A} are each independently selected from H, halogen and CH₃;
R² is selected from C₁₋₅alkyl, CD₃, C₃₋₄cycloalkyl and 4- to 6-membered heterocycle containing O, wherein said alkyl, cycloalkyl or heterocycle is optionally substituted with 1-3 R⁴;
each R⁴ is independently selected from halogen, CH₃ and phenyl;
R⁵ is selected from H, C₁₋₃alkyl, CD₃, C₃₋₄cycloalkyl and bicyclo[1.1.1] pentane, wherein said alkyl, cycloalkyl or bicyclo[1.1.1.]pentane is optionally substituted with 1-2 R⁷; and
each R⁷ is independently selected from CN, OH, CH₂OH, cyclopropyl, pyridinyl and oxazolyl, or taken together two R⁷ attached to the same carbon atom form 4-membered heterocycle containing N; and/or
wherein R² is selected from CH₃, CD₃, CH₂CH₃, CH₂CH₂CH₃, CH(CH₃)₂, CH₂CH(CH₃)₂, CH(CH₃)CH₂CH₃, cyclobutyl, cyclopropyl, oxetanyl, tetrahydrofuranyl, tetrahydrofuranyl and tetrahydropyranyl, each of which is optionally substituted with 1-3 R⁴; and/or
wherein R² is selected from 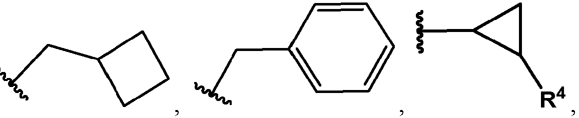 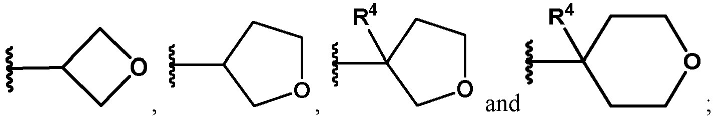 and/or
wherein each R⁴ is independently selected from F, CH₃, cyclobutyl and phenyl; and/or
wherein R⁵ is selected from H, CH₃, CD₃, CH₂CH₃, CH₂CH₂CH₃, CH(CH₃)₂, cyclobutyl, cyclopropyl and bicylo[1.1.1]pentanyl, wherein said CH₃, CH₂CH₃, CH₂CH₂CH₃, CH(CH₃)₂, cyclobutyl, cyclopropyl or bicylo[1.1.1]pentanyl are optionally substituted with 1-2 R⁷; and/or
wherein R⁵ is selected from 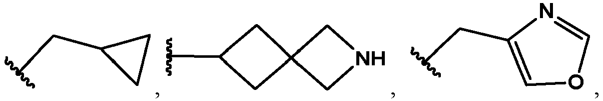 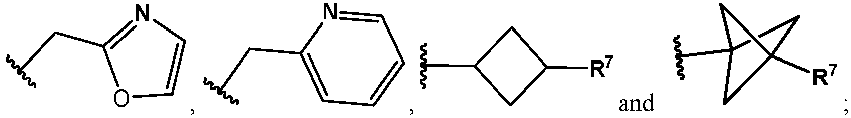 and/or
wherein each R⁷ is independently selected from CN, CH₂OH, cyclopropyl, 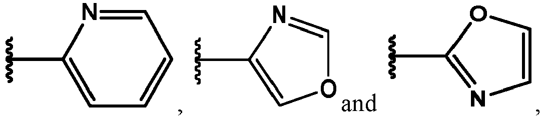 or taken together two R⁷ attached to the same carbon atom form 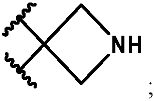 and/or
wherein R¹ and R^{1A} are each independently selected from H, F and CH₃.

5. The compound of claim 1 or 2, wherein the compound is represented by Formula (IV), (V) or (VI): 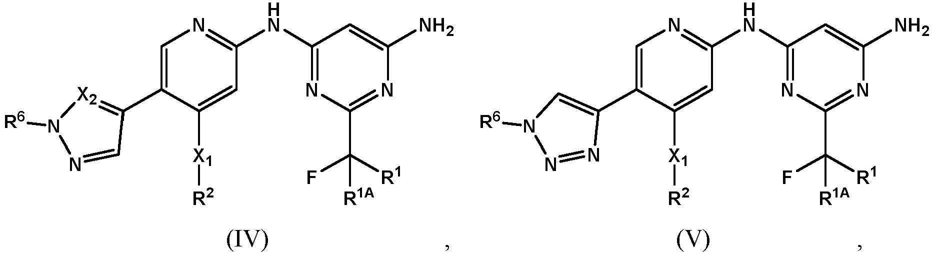 or 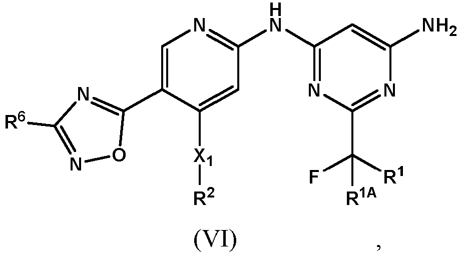 or a pharmaceutically acceptable salt thereof; or
wherein the compound is represented by Formula (VII), (VIII), (IX) or (X): 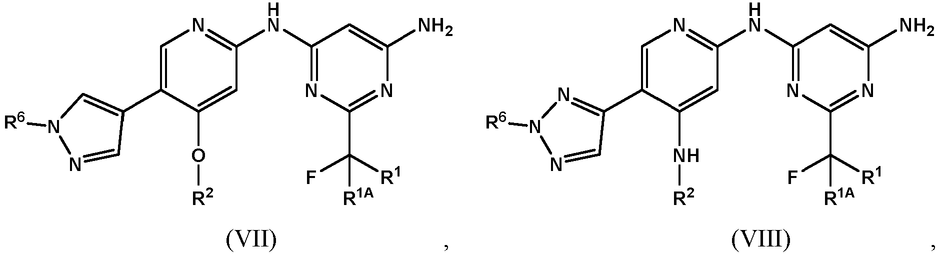 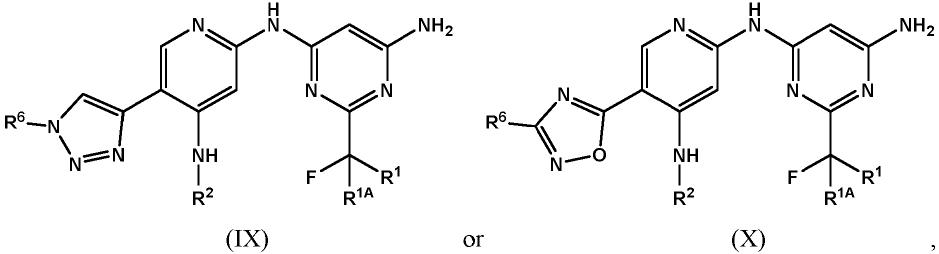 or a pharmaceutically acceptable salt thereof.

6. The compound of claim 5, or a pharmaceutically acceptable salt thereof, wherein:
R¹ and R^{1A} are each independently selected from H, halogen, and CH₃;
R² is selected from C₁₋₃alkyl, CD₃, C₃₋₄cycloalkyl and 4- to 6-membered heterocycle containing one O, wherein said alkyl, cycloalkyl, or heterocycle is optionally substituted with 1-3 R⁴;
each R⁴ is independently selected from halogen, OH, cyclopropyl, 4- to 6-membered heterocycle containing one O, and phenyl, wherein said cyclopropyl or phenyl is optionally substituted with OH or NH₂;
R⁶ is selected from C₁₋₄alkyl, CHF₂, CF₃, 4- or 5-membered heterocycle containing N or O, and C₃₋₄cycloalkyl, wherein said alkyl, heterocycle or cycloalkyl is optionally substituted with R⁸;
R⁸ is selected from OH, NR⁹R⁹, OCH₃, CH₃ and 4-membered heterocycle containing N or O, wherein said heterocycle is optionally substituted with CH₃; and
each R⁹ is independently selected from H, CH₃, and CH₂CF₃; and/or
wherein R² is selected from CH₃, CD₃, CH₂CH₃, CH₂CH₂CH₃, CH(CH₃)₂, cyclobutyl, cyclopropyl, oxetanyl, tetrahydrofuranyl, tetrahydrofuranyl and tetrahydropyranyl, each of which is optionally substituted with 1-3 R⁴; and/or
wherein R² is selected from 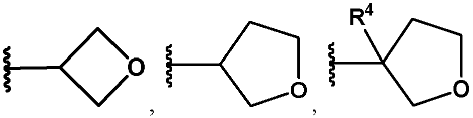 and 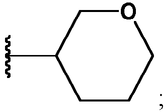 and/or
wherein each R⁴ is independently selected from F, OH, cyclobutyl, oxetanyl, oxetanyl, phenyl, tetrahydrofuranyl and tetrahydropyranyl, wherein said cyclobutyl, oxetanyl, oxetanyl, phenyl, tetrahydrofuranyl or tetrahydropyranyl is optionally substituted with OH or NH₂; and/or
wherein each R⁴ is independently selected from F, OH, 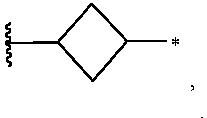 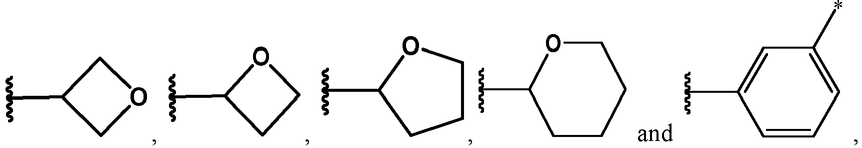 wherein *- represents OH or NH₂; and/or
wherein R⁶ is selected from CH₃, CH₂CH₃, CH₂CH₂CH₃, CH(CH₃)₂, CH₂CH(CH₃)₂, C(CH₃)₃, CH₂CH₂CH(CH₃)₂, CHF₂, CF₃, cyclopropyl, cyclobutyl, azetidinyl, oxetanyl, tetrahydrofuranyl and pyrrolidinyl, each of which is optionally substituted with R⁸; or
wherein R⁶ is selected from 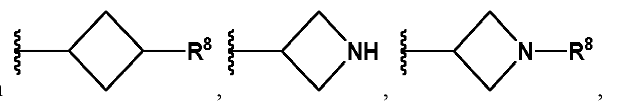 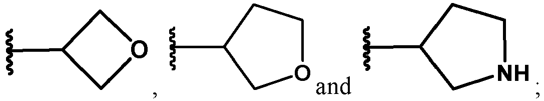 and/or
wherein R⁸ is selected from OH, OCH₃, CH₃, NH₂, NHCH₃, N(CH₃)₂, NHCH₂CF₃, N(CH₃)CH₂CF₃, azetidinyl, azetidinyl and oxetanyl; and/or
wherein R⁸ is selected from 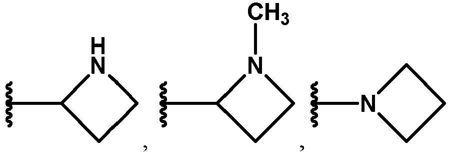 and 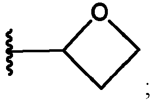 and/or
wherein R¹ and R^{1A} are each independently selected from H, F and CH₃.

7. The compound of claim 1 or 2, wherein the compound is represented by Formula (III) or (VII): 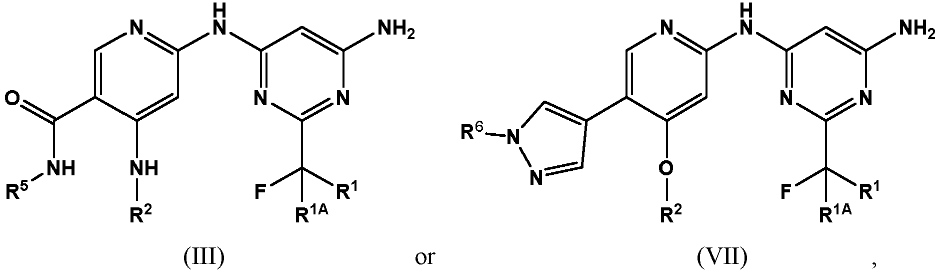 or a pharmaceutically acceptable salt thereof, wherein:
R¹ and R^{1A} are each independently selected from H, F and CH₃;
R² is selected from C₁₋₃alkyl, CD₃, C₃₋₄cycloalkyl and 4-membered heterocycle containing O, wherein said alkyl is optionally substituted with 1-3 halo;
R⁵ is selected from CH₃, CH₂CH₃ and cyclopropyl;
R⁶ is selected from C₁₋₅alkyl, CHF₂, CF₃ and 4- to 5-membered heterocycle containing O or N, wherein said alkyl or heterocycle is optionally substituted with R⁸;
R⁸ is selected from OH, NR⁹R⁹, OCH₃, CH₃ and 4-membered heterocycle containing O or N; and
each R⁹ is independently selected from H and CH₃;
optionally wherein R² is selected from CH₃, CHF₂, CF₃, CD₃, CH₂CH₃, CH₂CF₃, CH₂CH₂CH₃, CH(CH₃)₂, CH(CH₃)CH₂F, CH(CH₃)CHF₂, cyclobutyl, cyclopropyl and

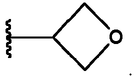

8. The compound of claim 7, or a pharmaceutically acceptable salt thereof, wherein R⁶ is selected from CH₃, CH₂CH₃, CH₂CH₂CH₃, CH₂CH(CH₃)₂, CH₂CH₂CH(CH₃)₂, CHF₂, azetidinyl, oxetanyl, tetrahydrofuranyl and pyrrolidinyl, each of which is optionally substituted with one R⁸; and/or
wherein R⁶ is selected from 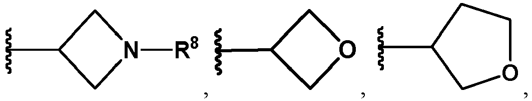 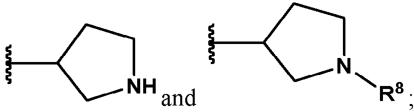 and/or
wherein R⁸ is selected from OH, OCH₃, NH₂, NHCH₃, N(CH₃)₂, CH₃, 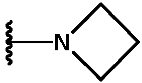 and

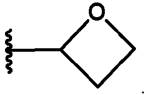

9. A compound according to claim 1 selected from the group consisting of the compounds in the table below, or a pharmaceutically acceptable salt thereof.

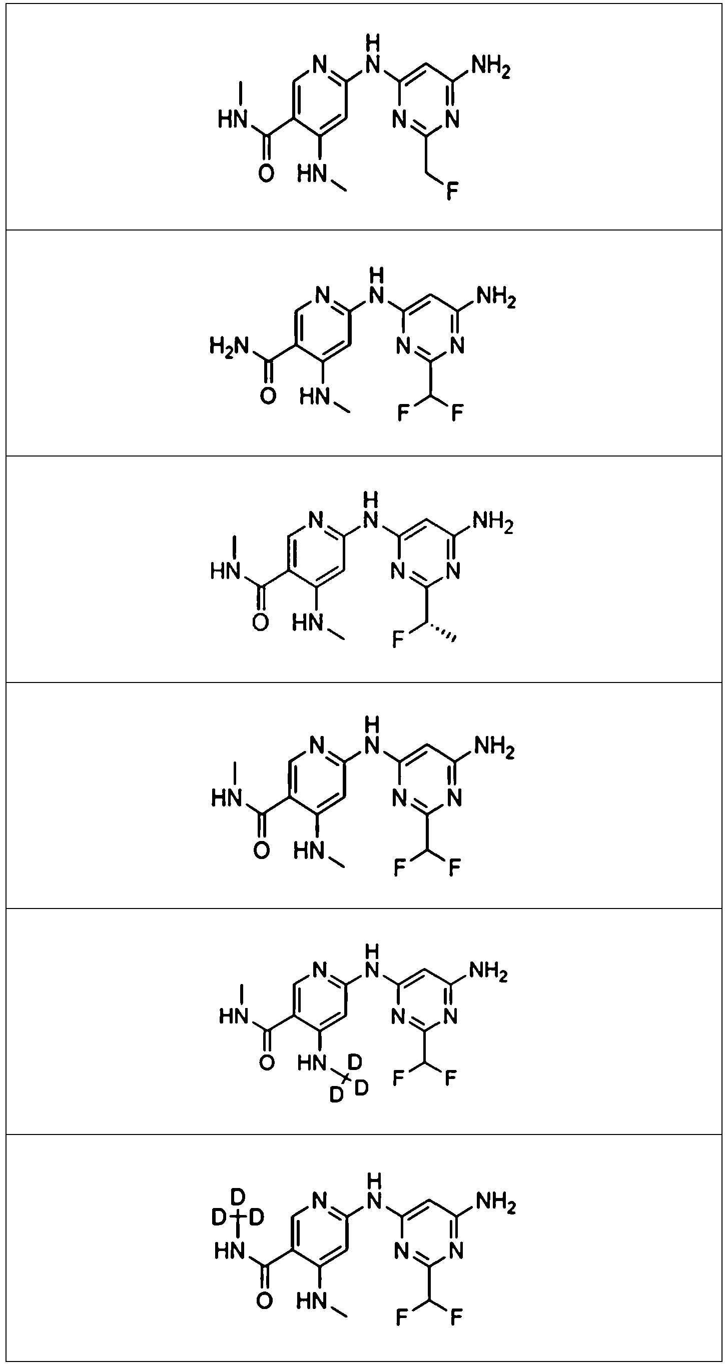

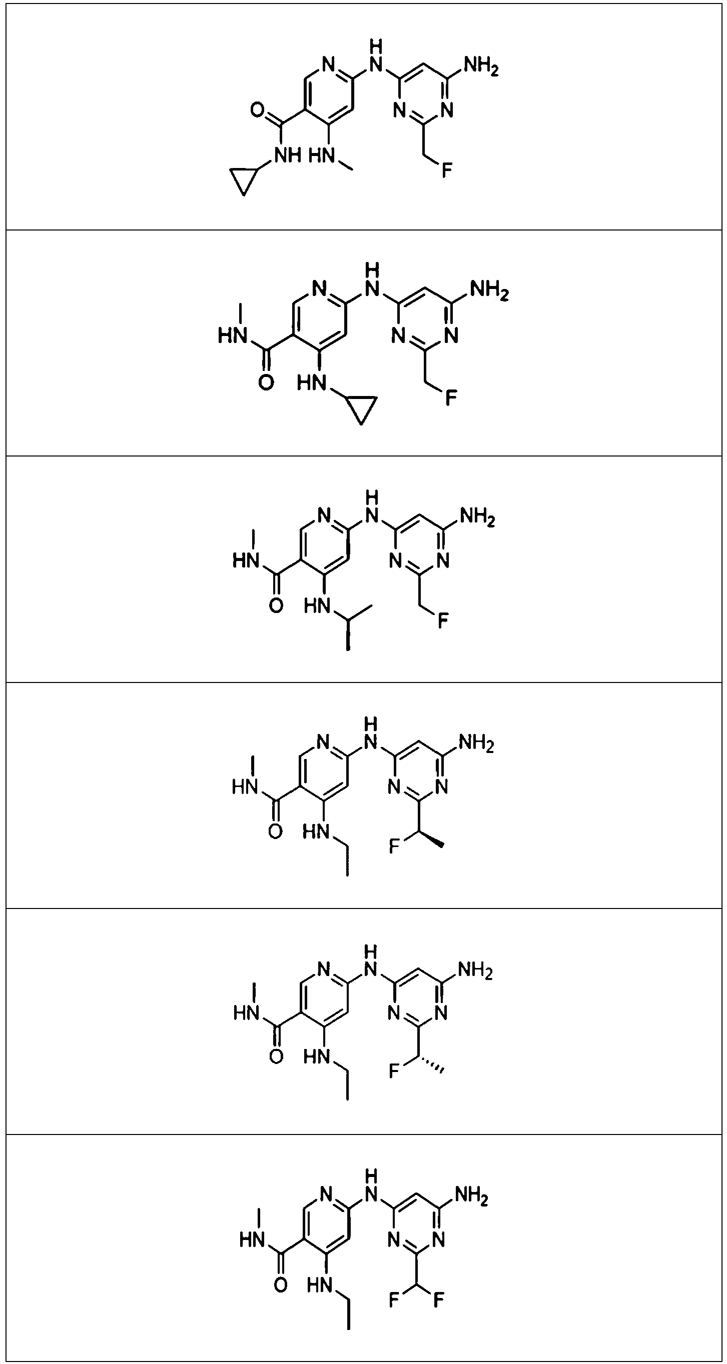

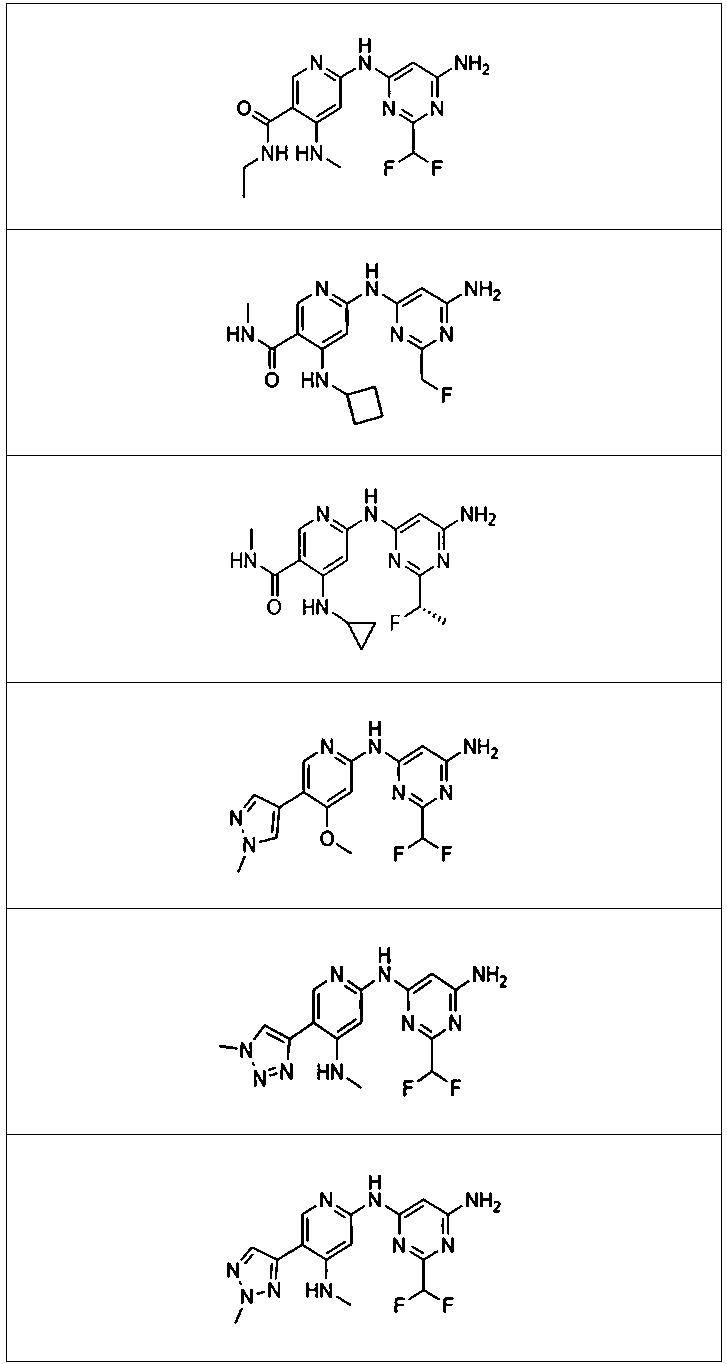

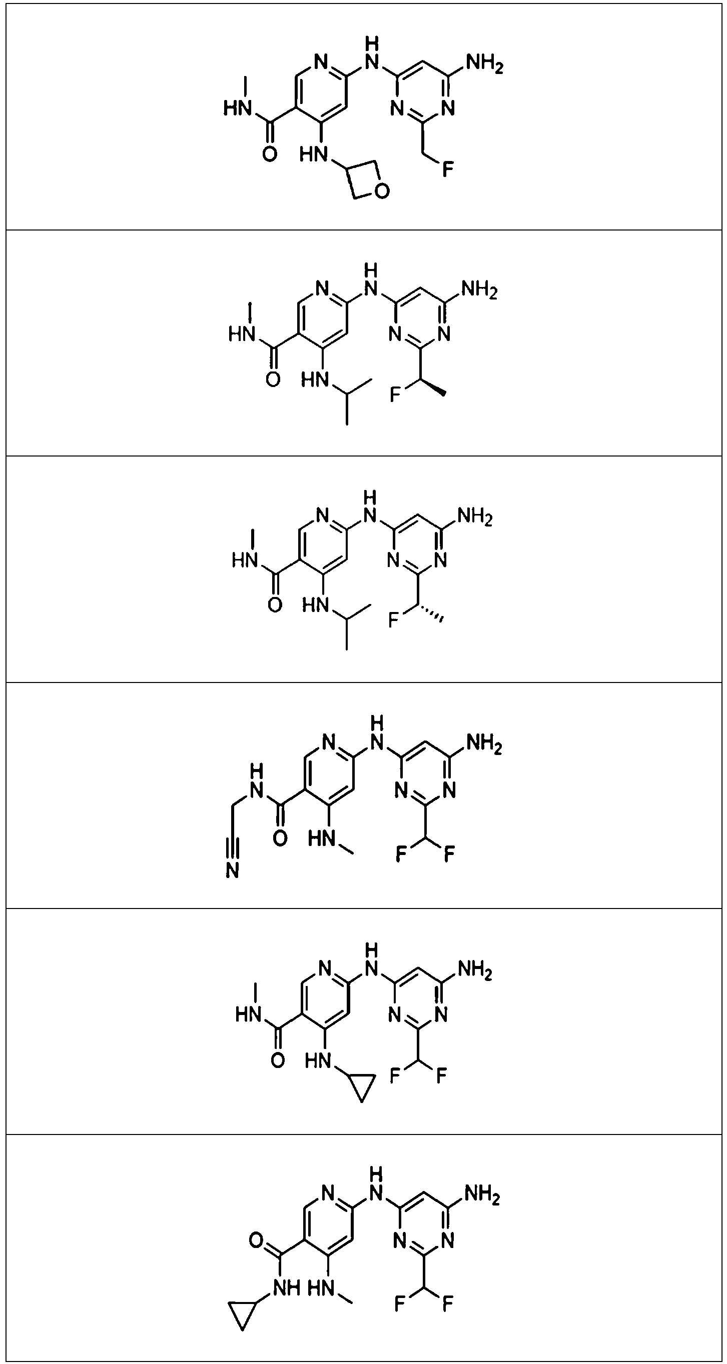

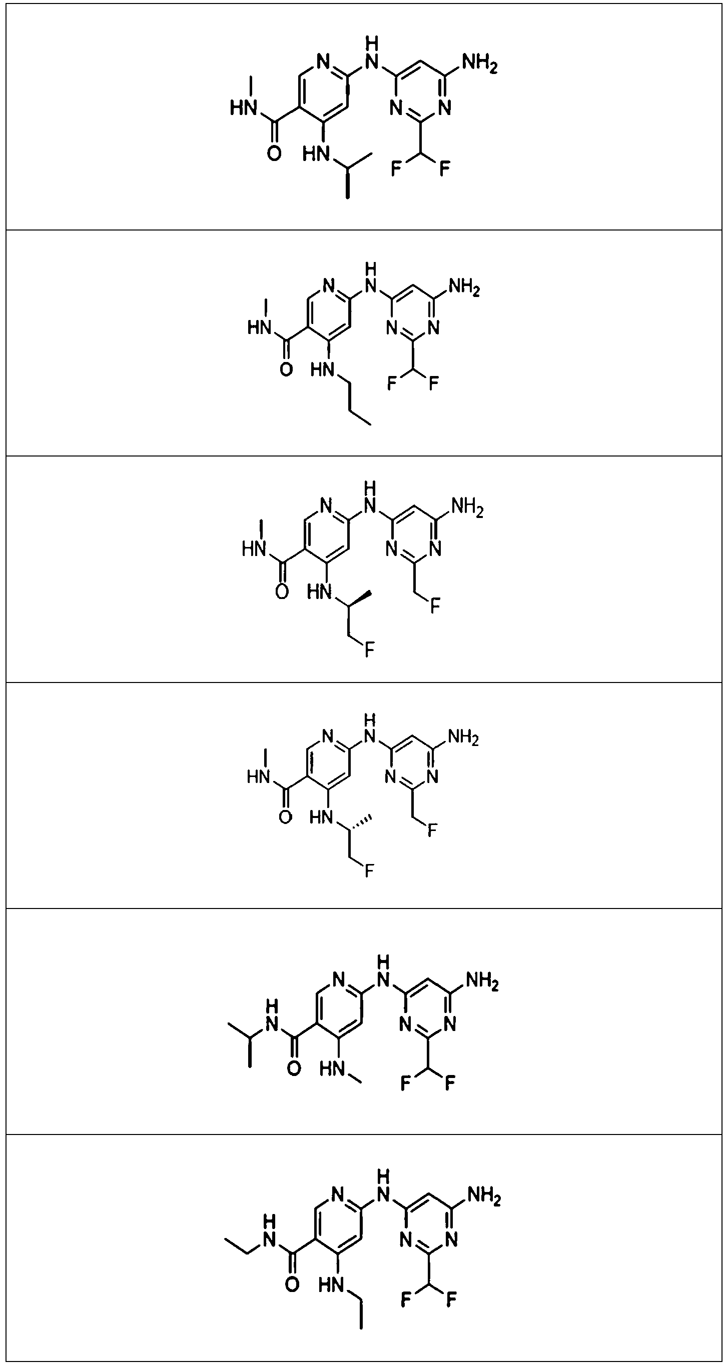

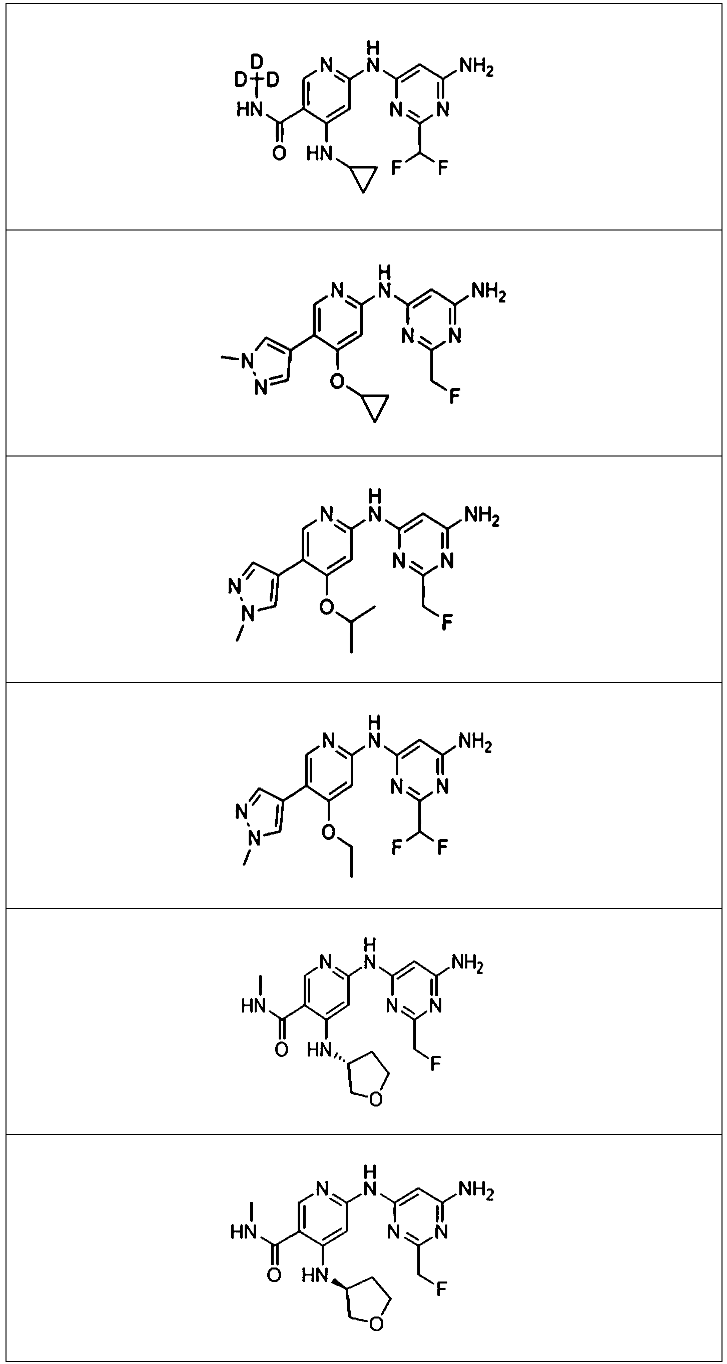

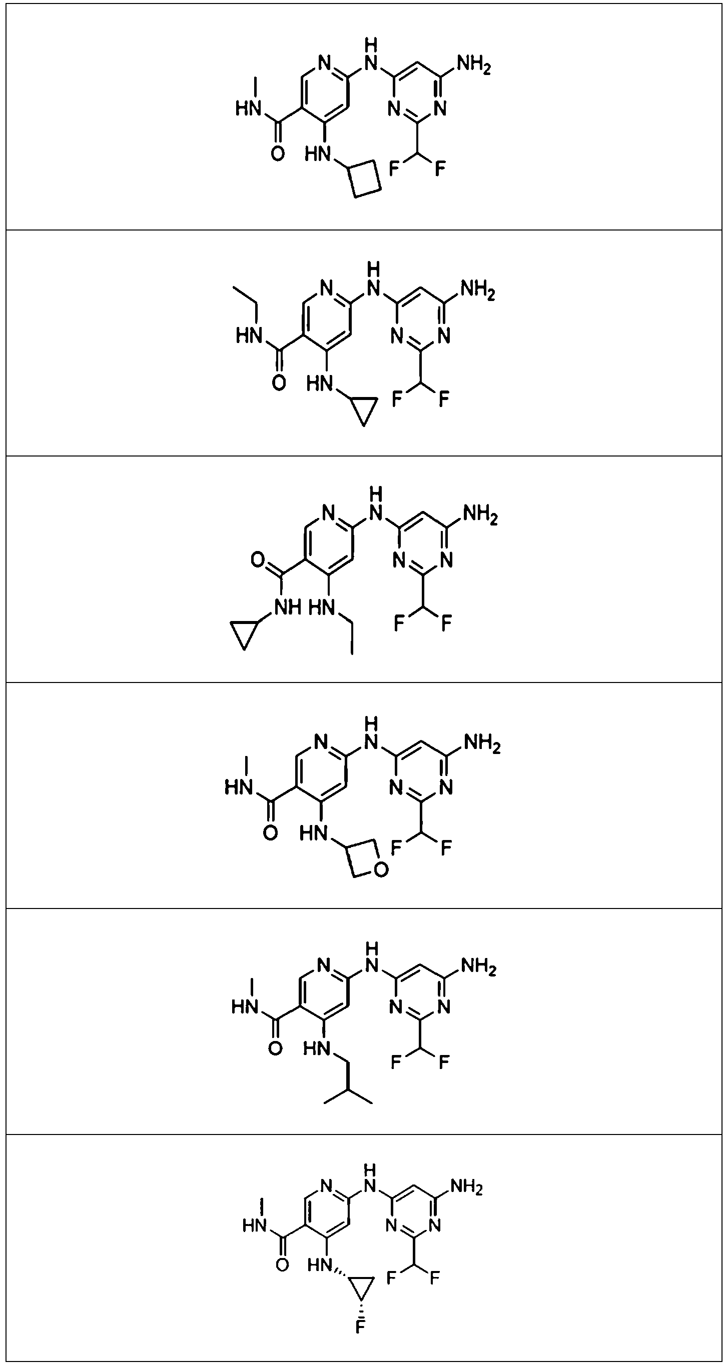

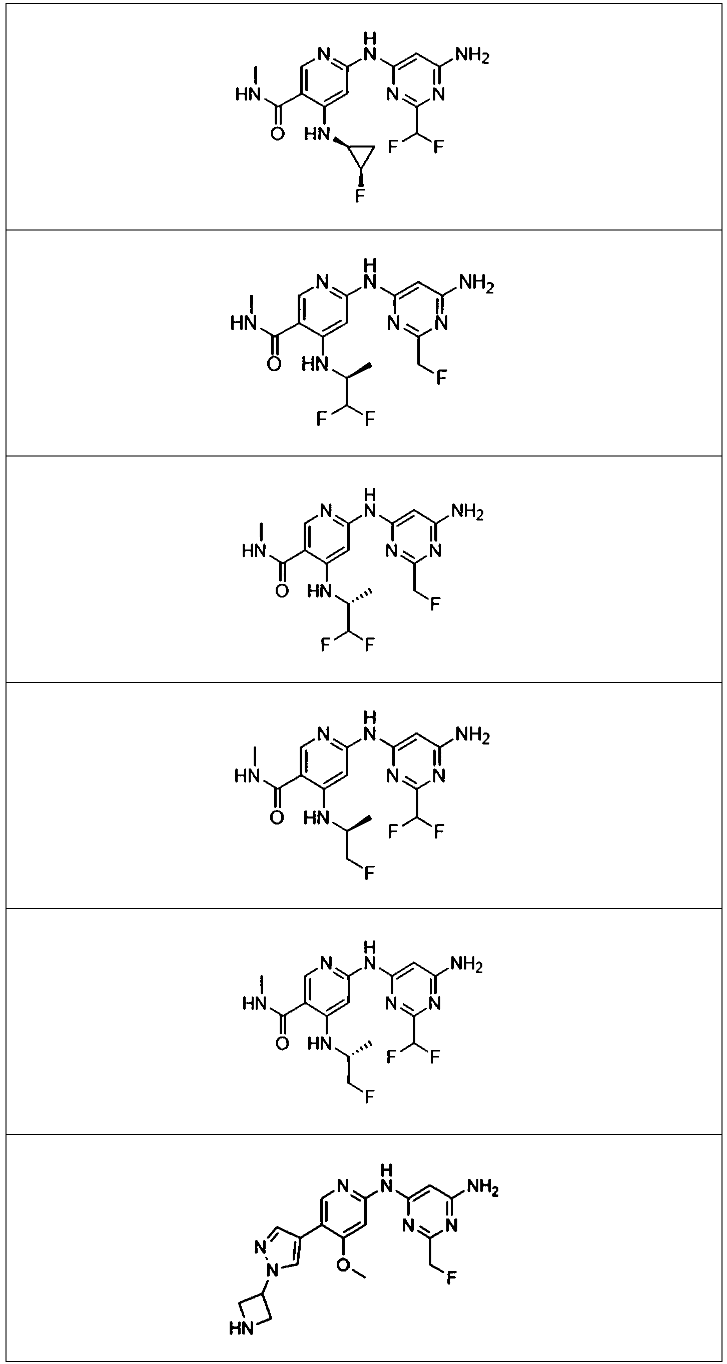

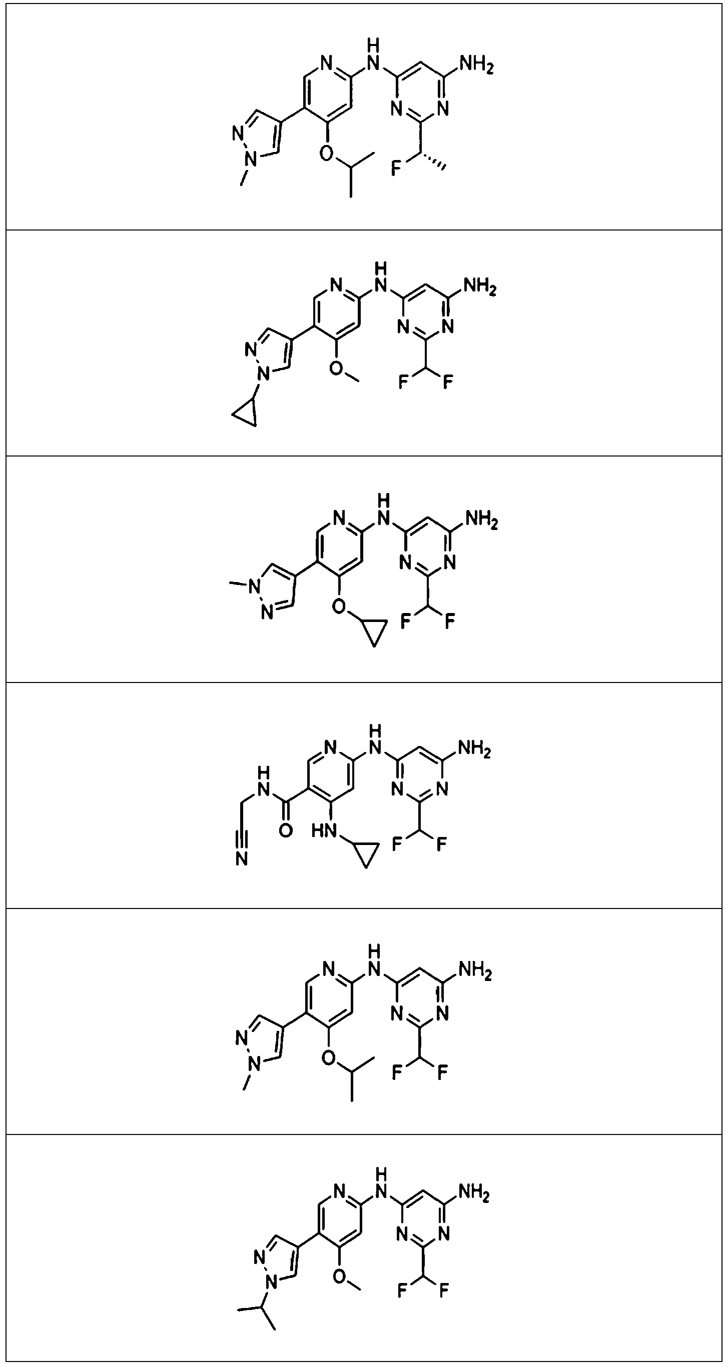

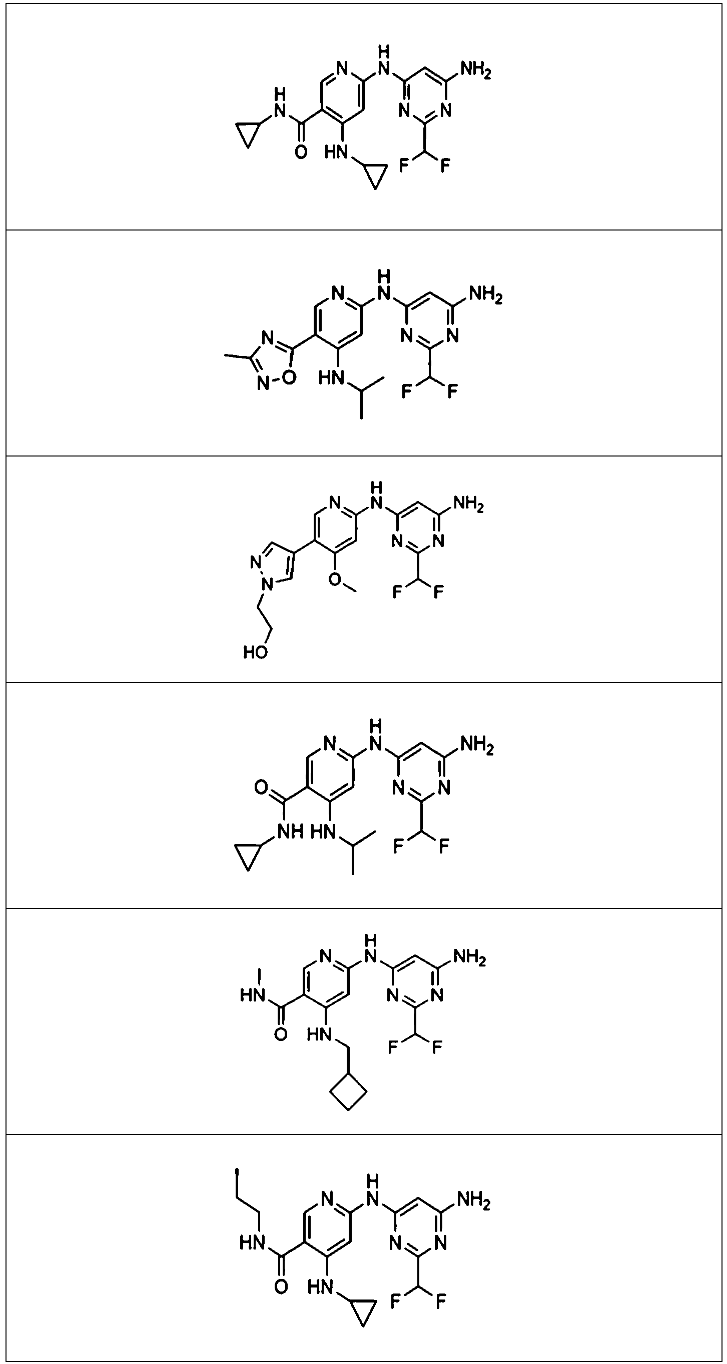

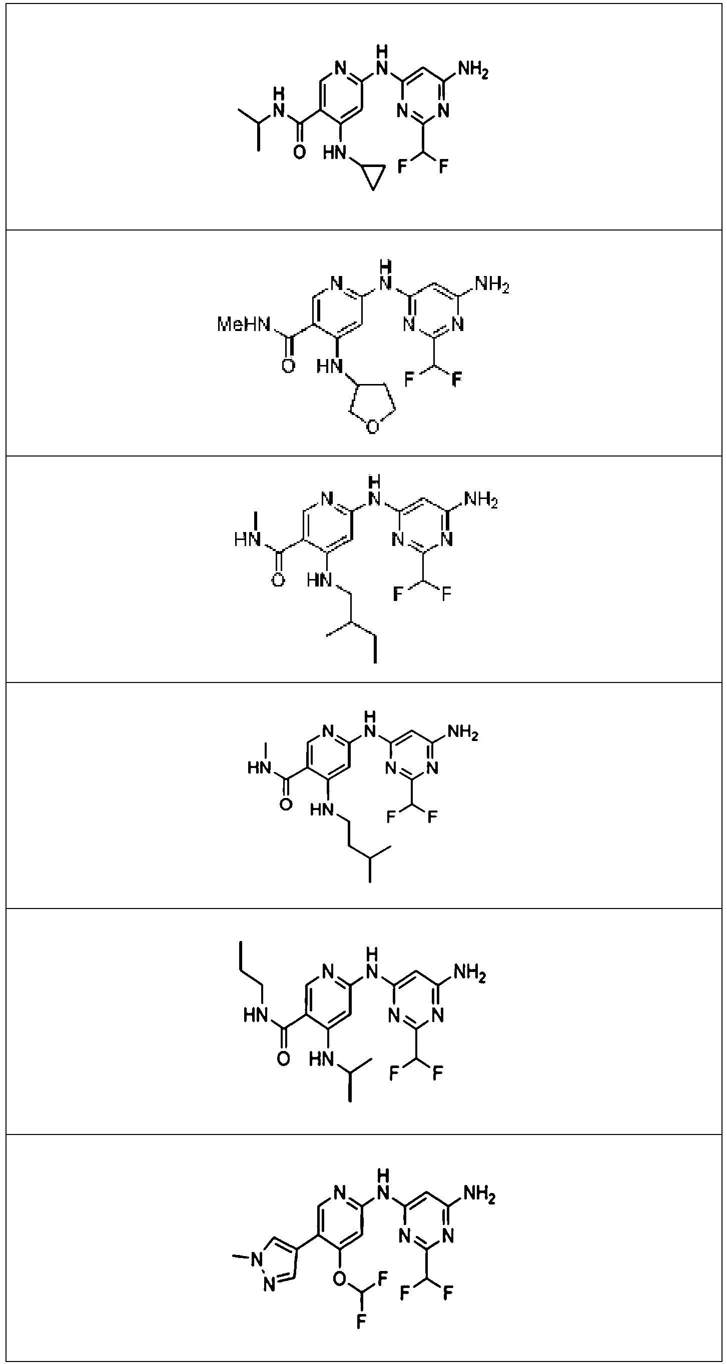

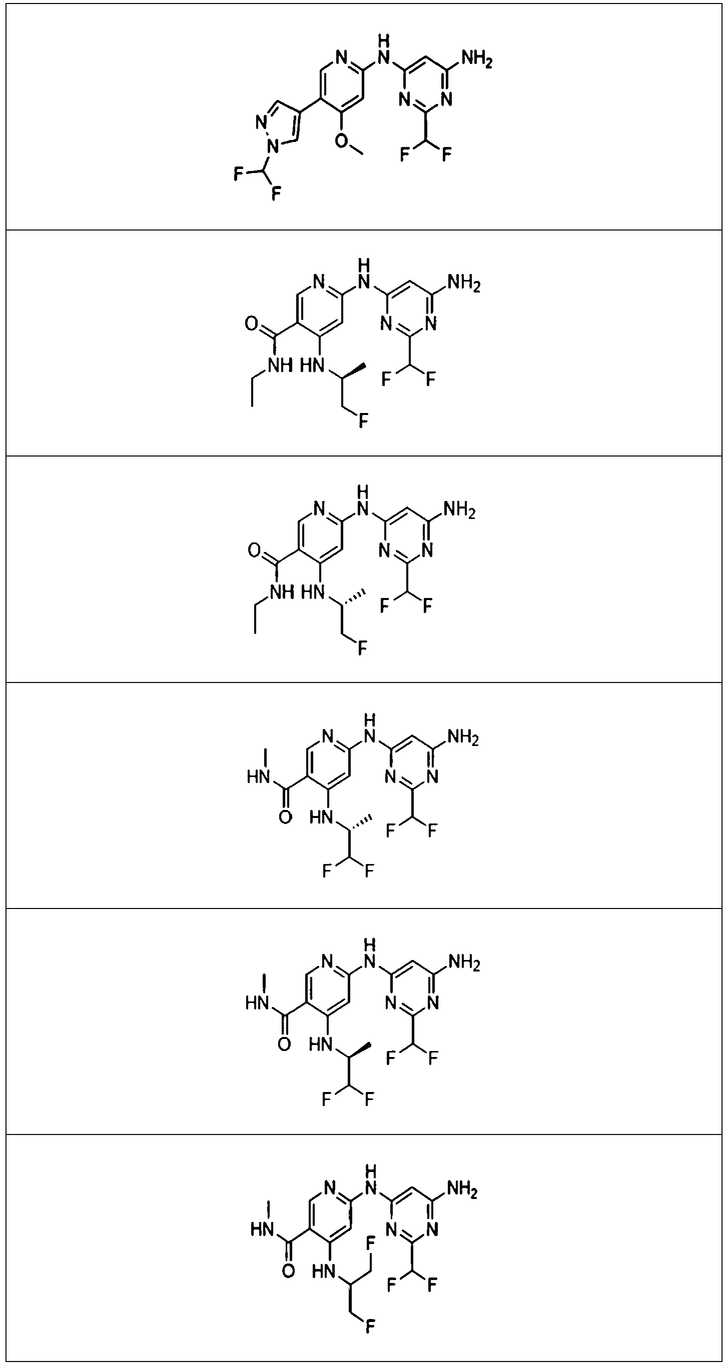

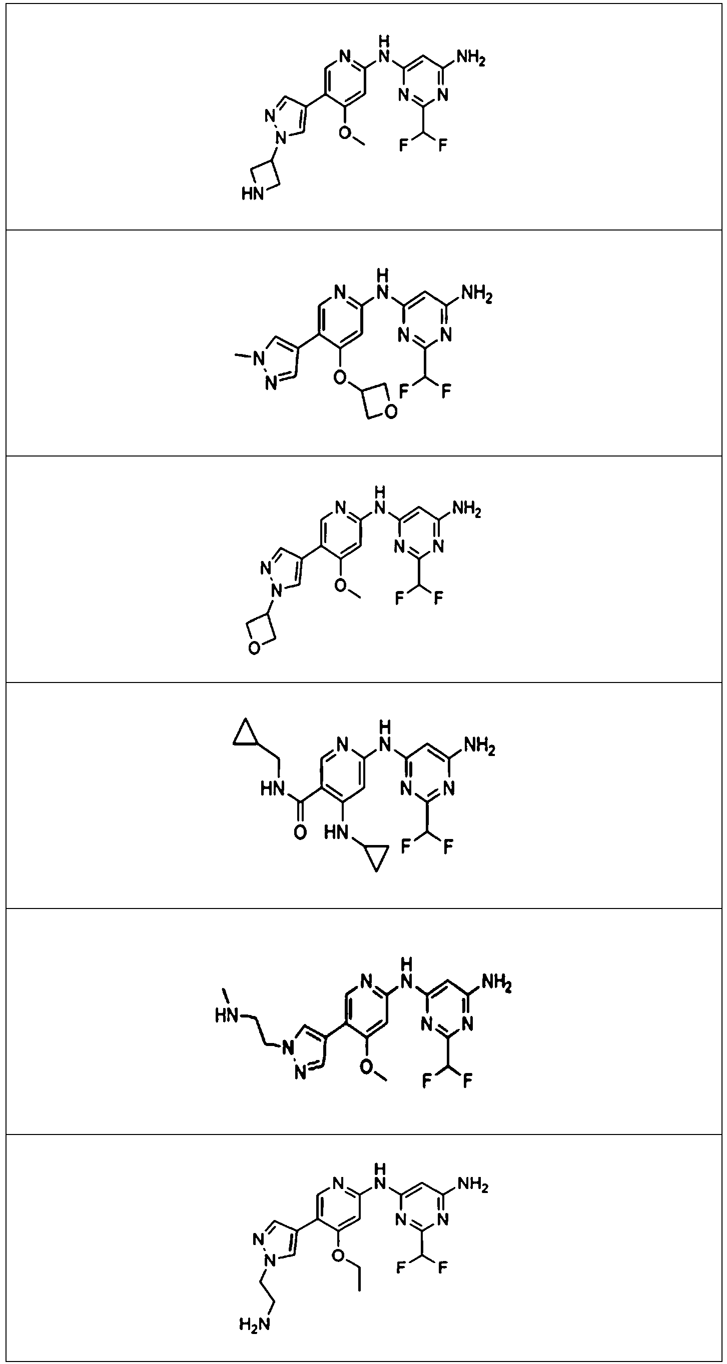

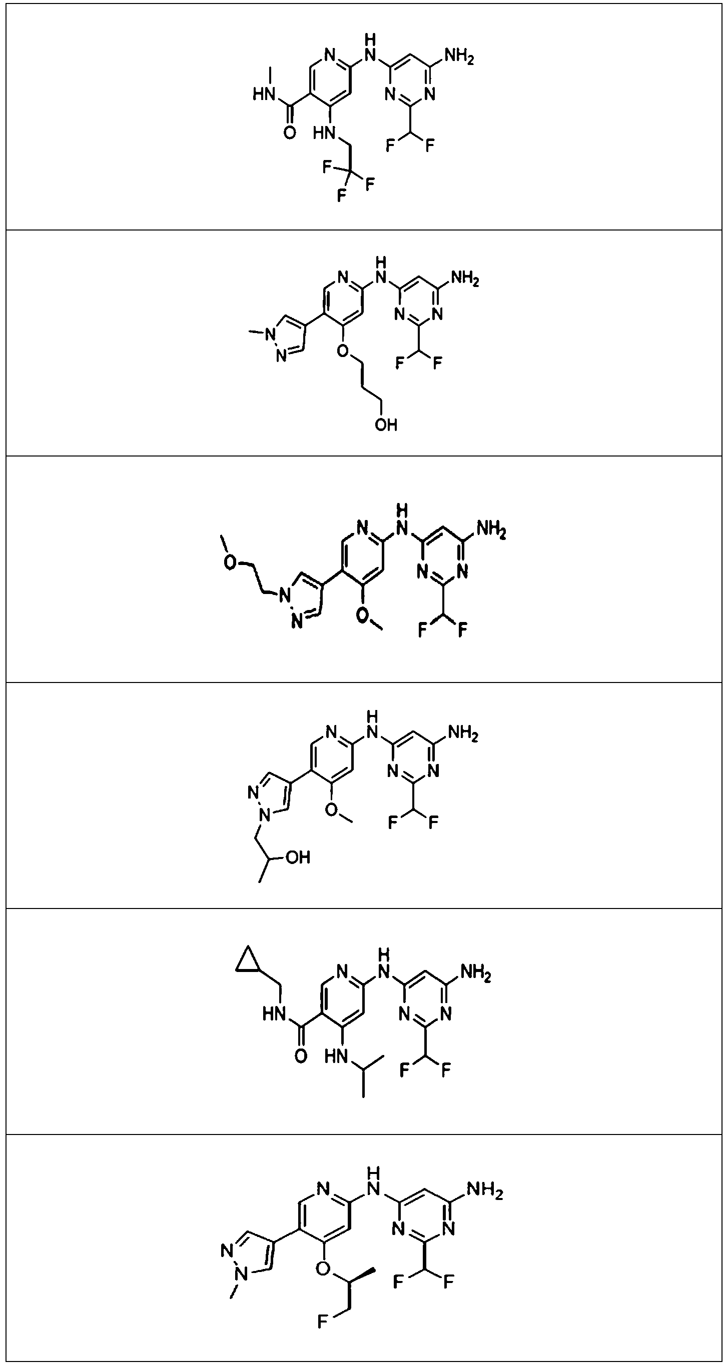

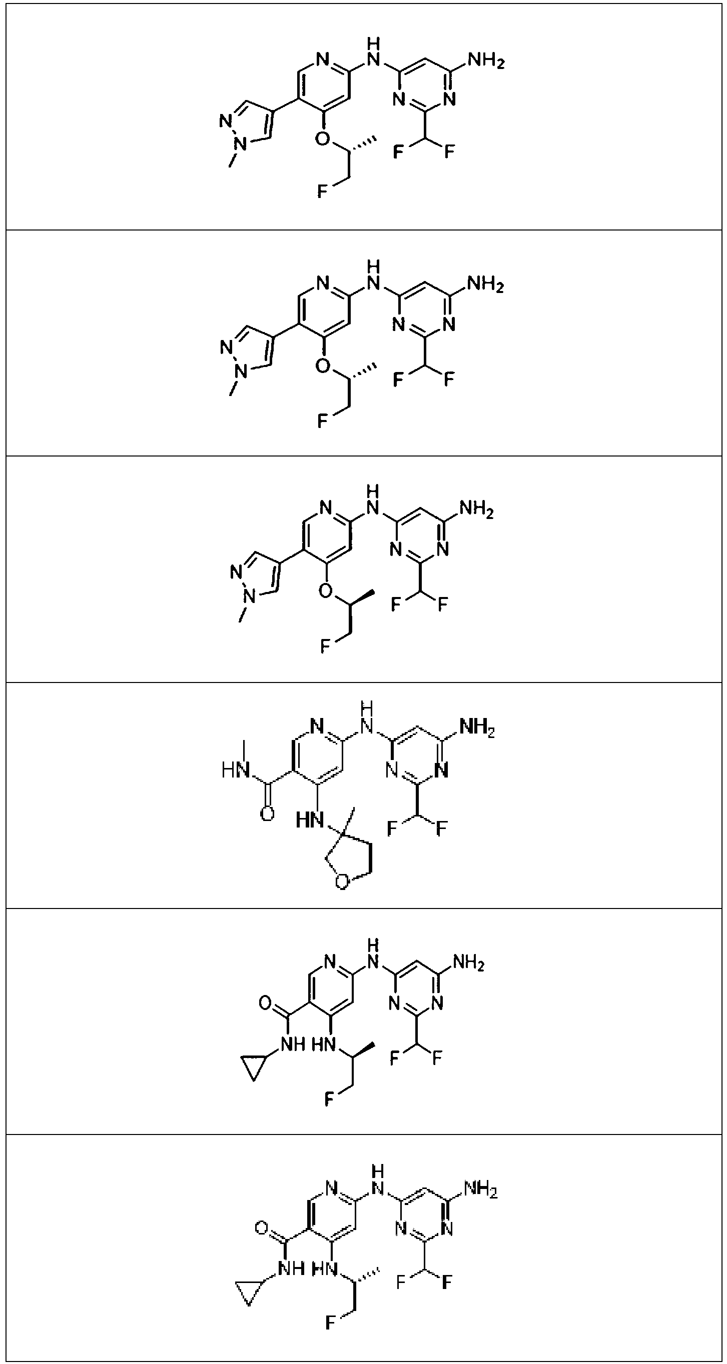

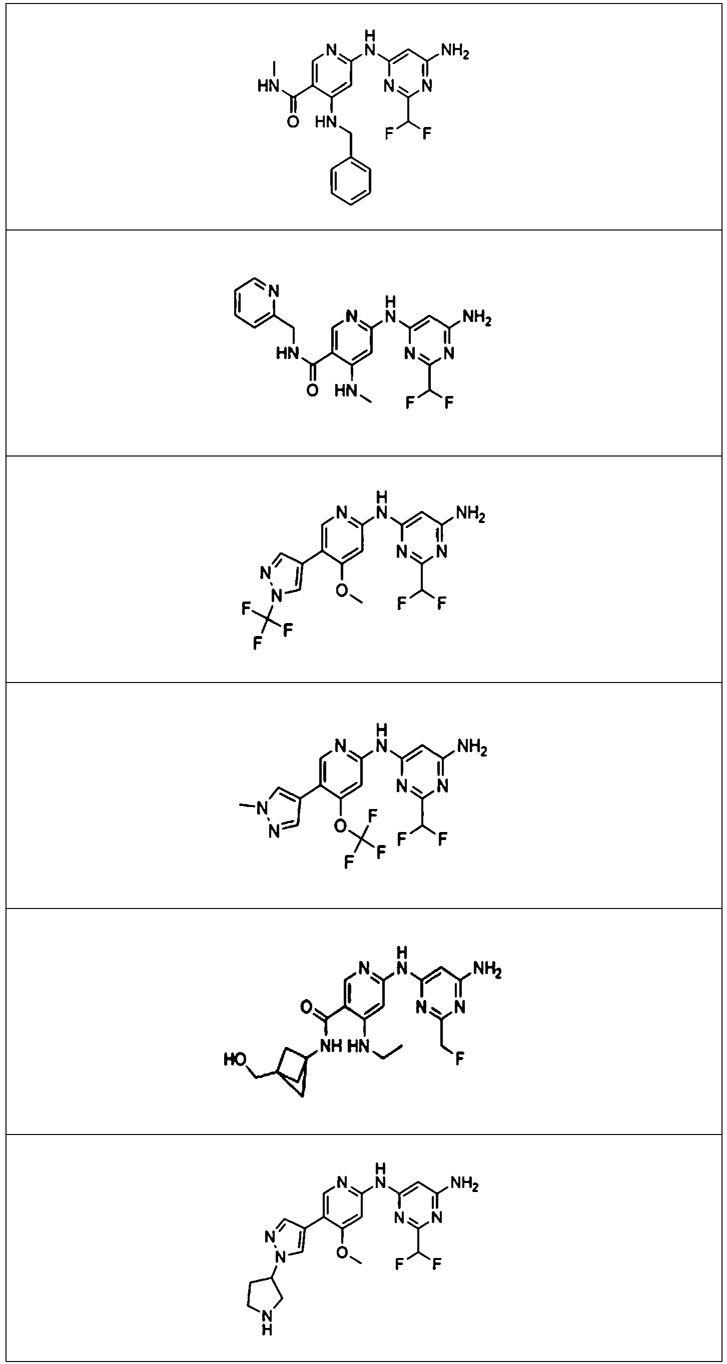

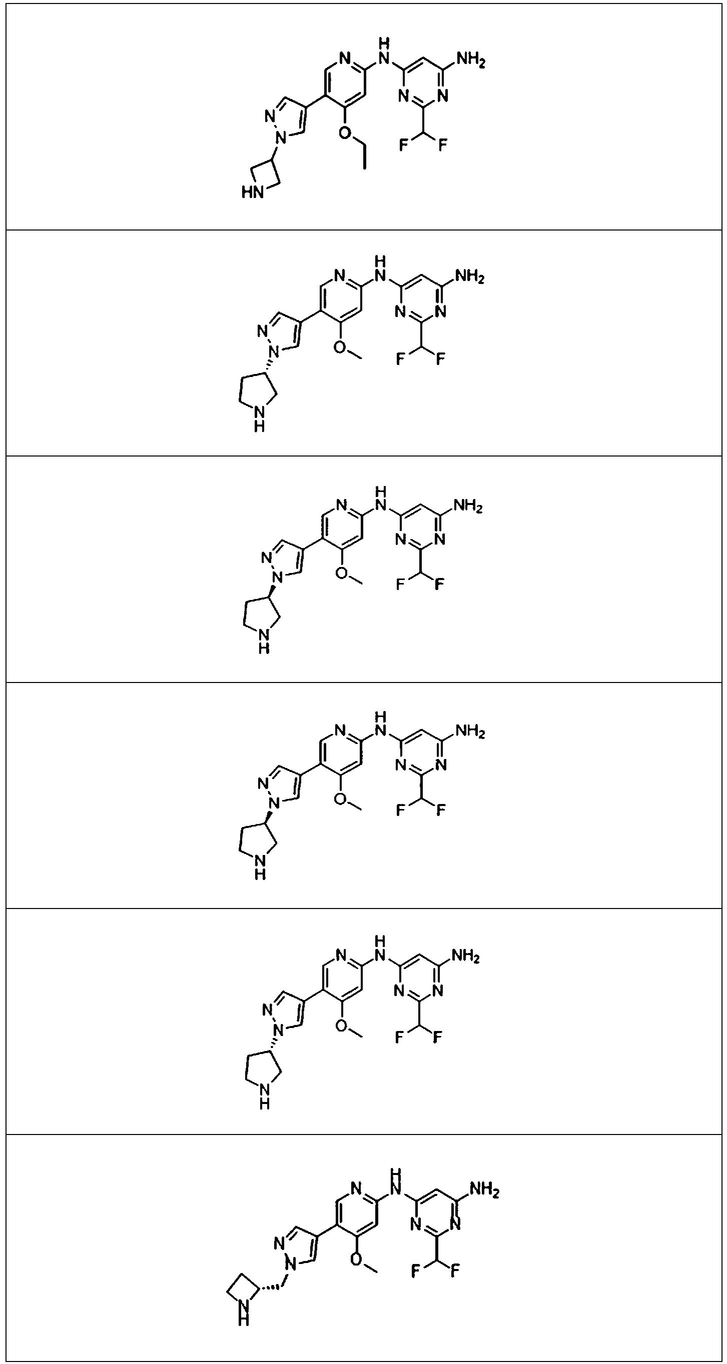

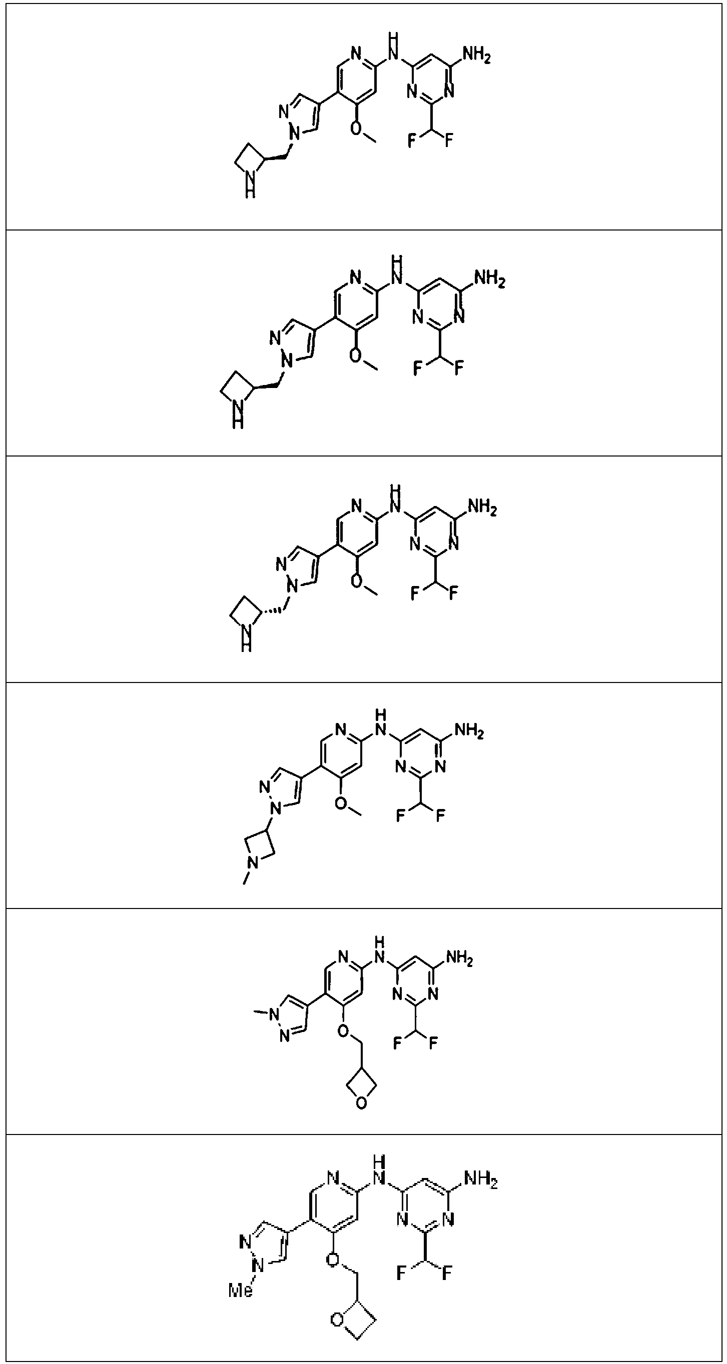

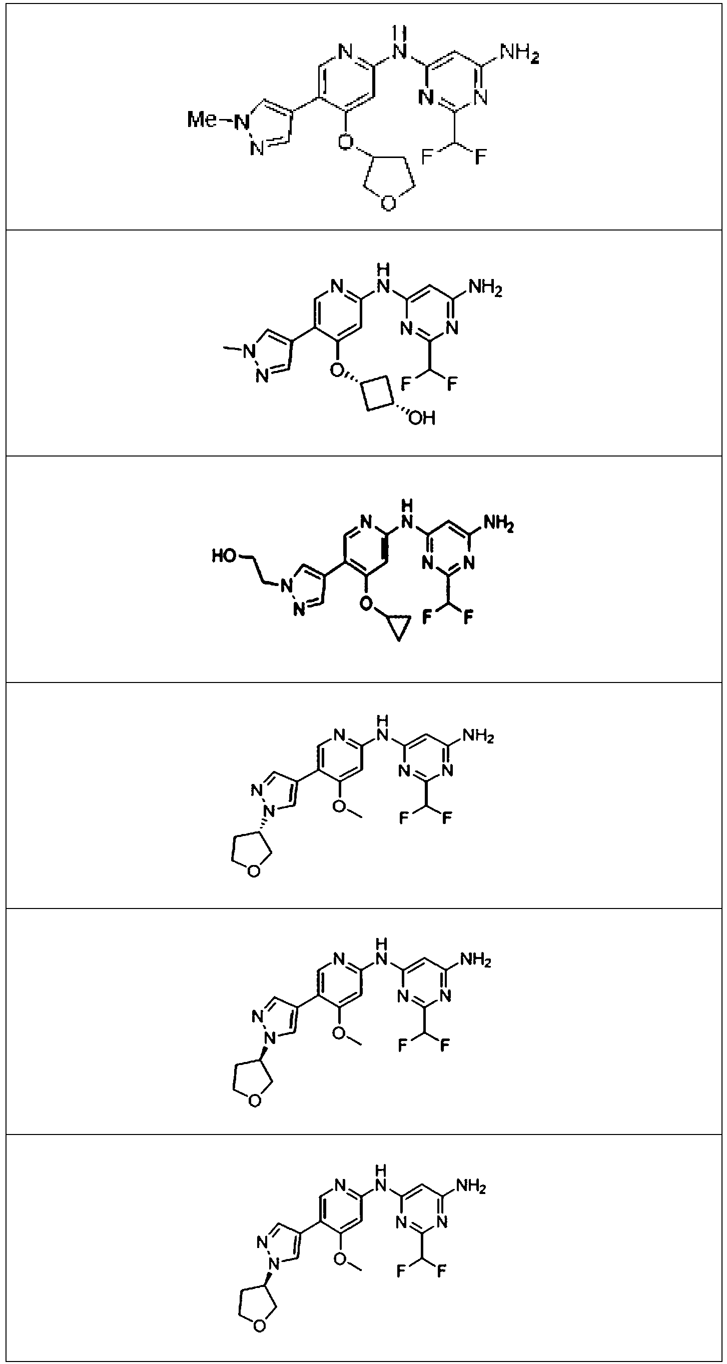

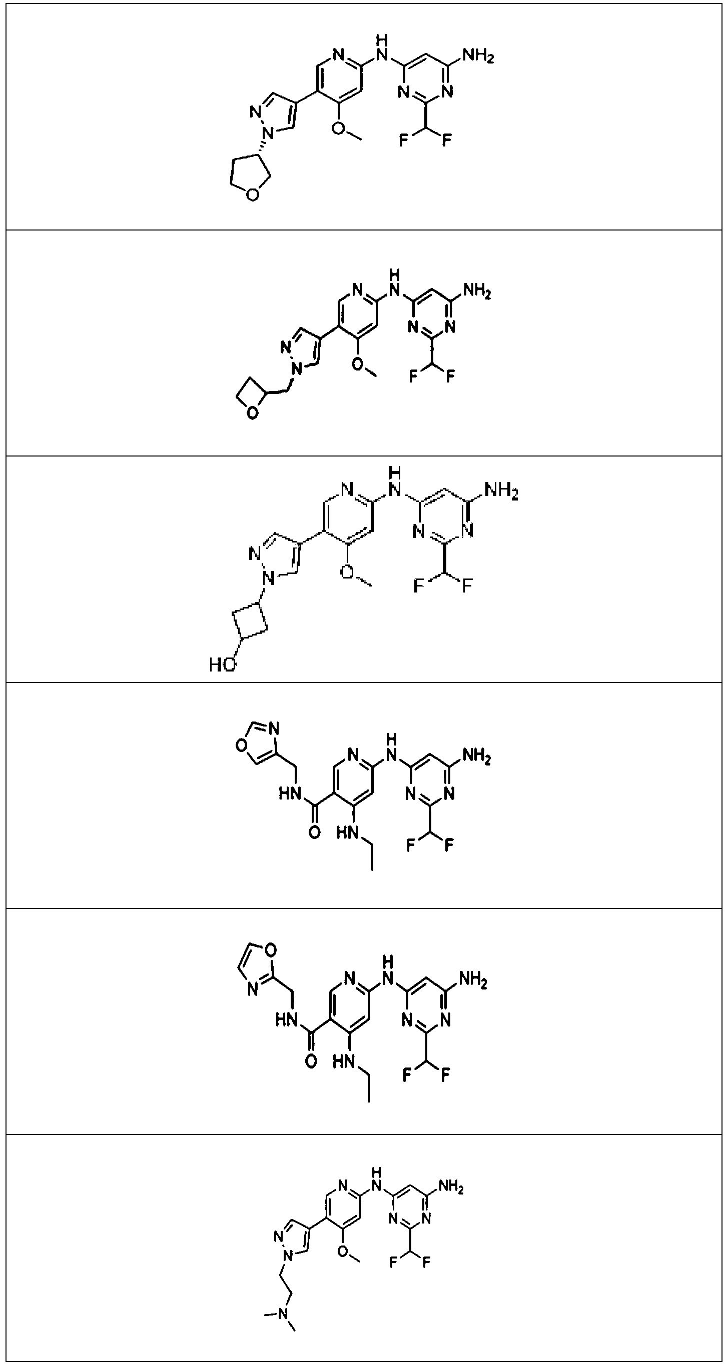

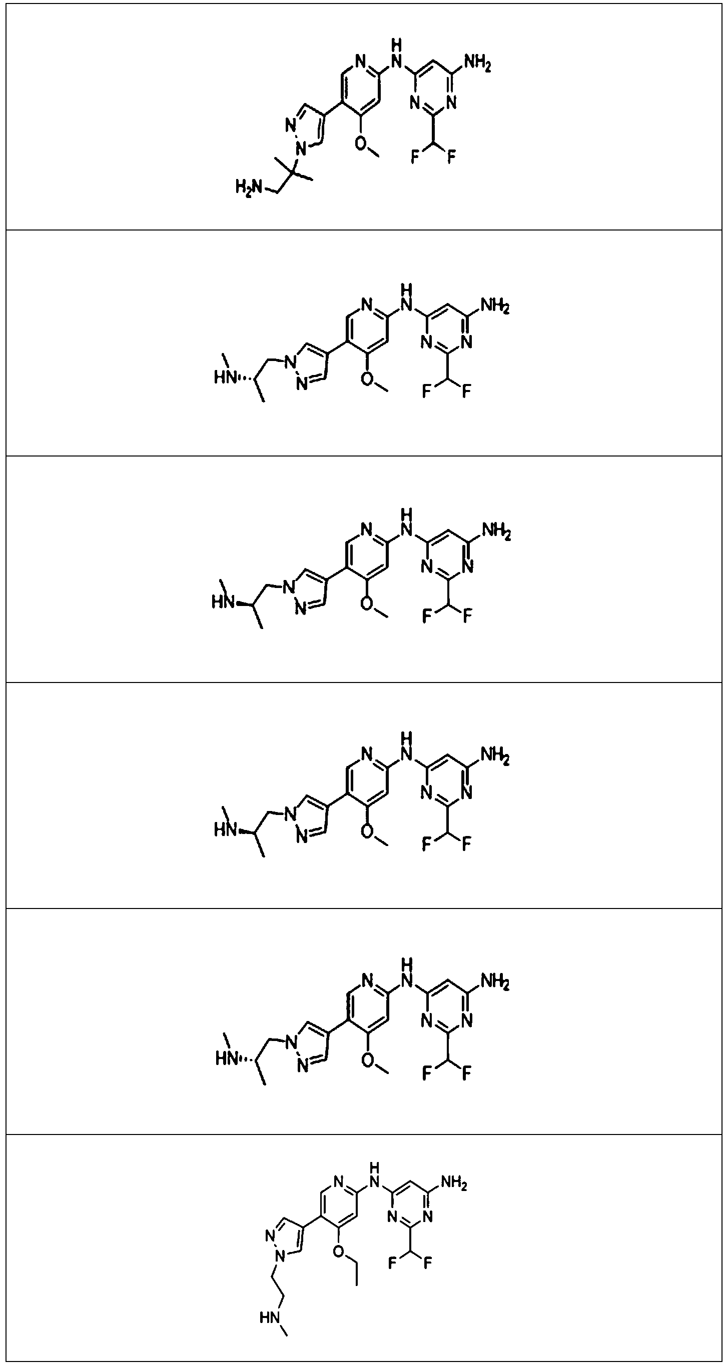

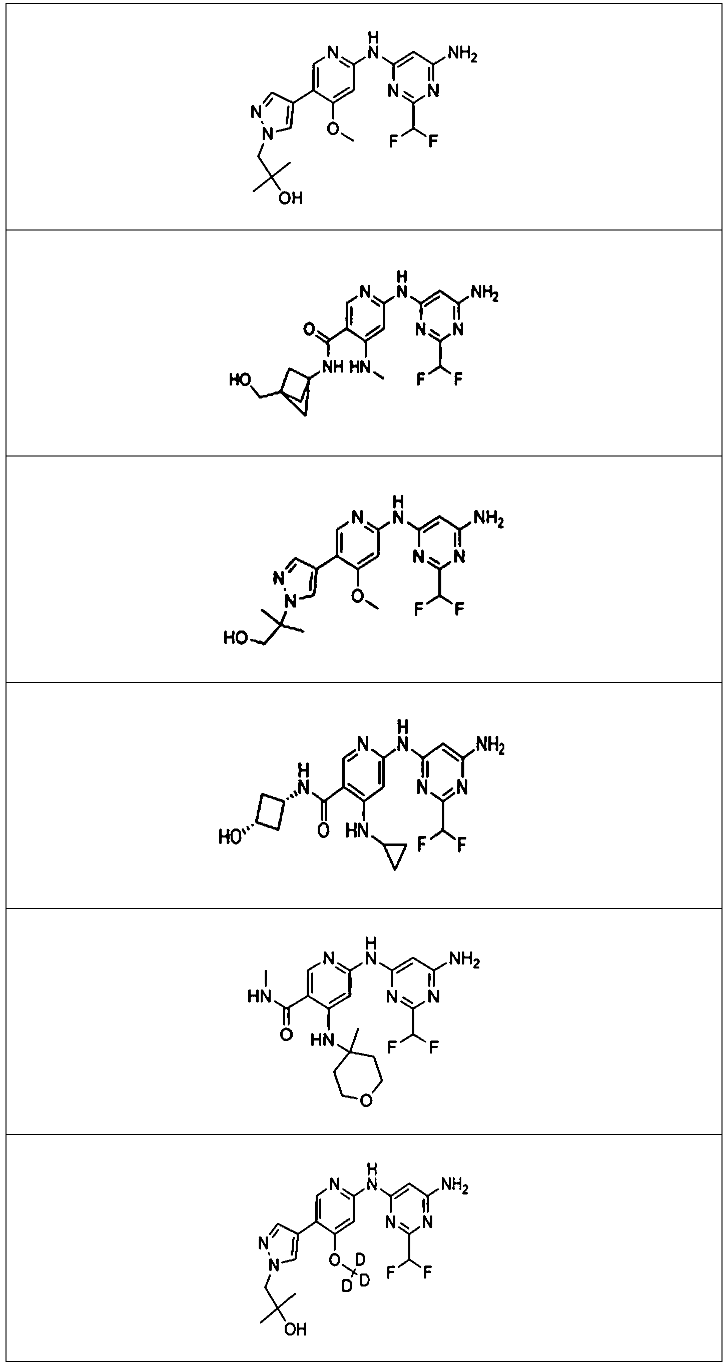

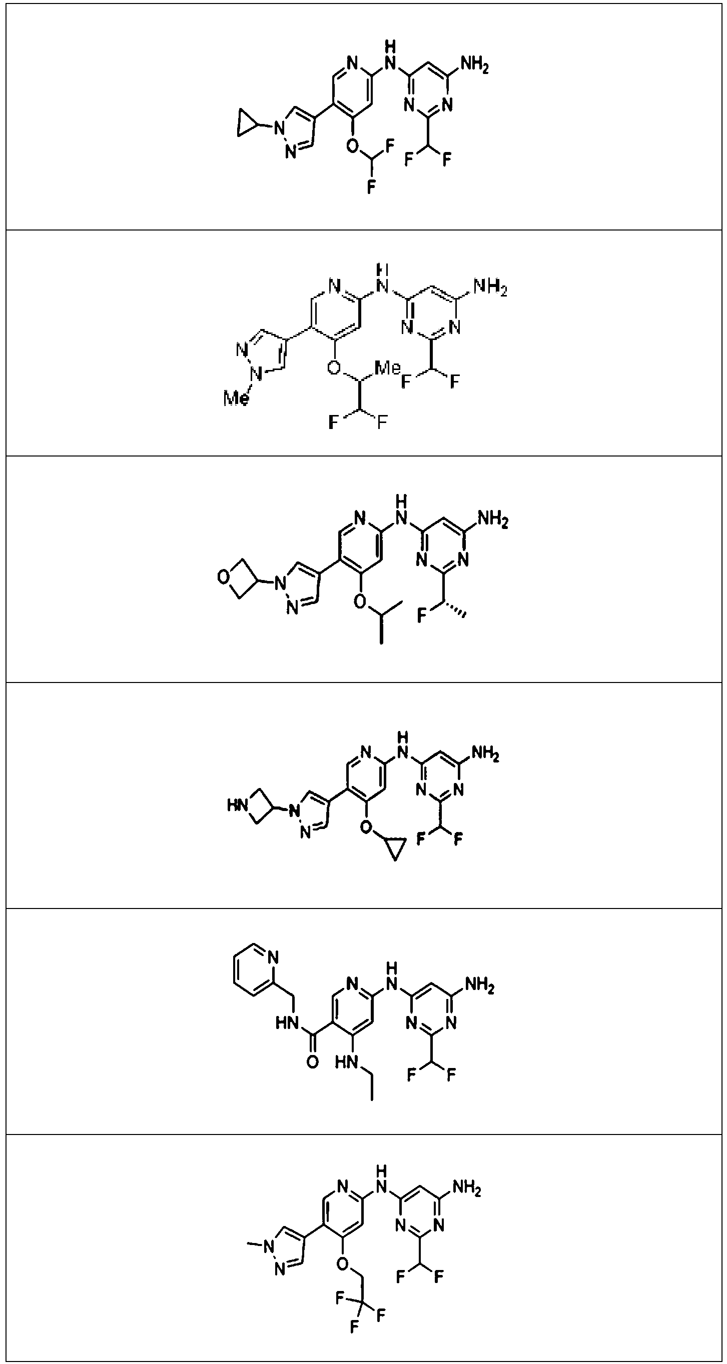

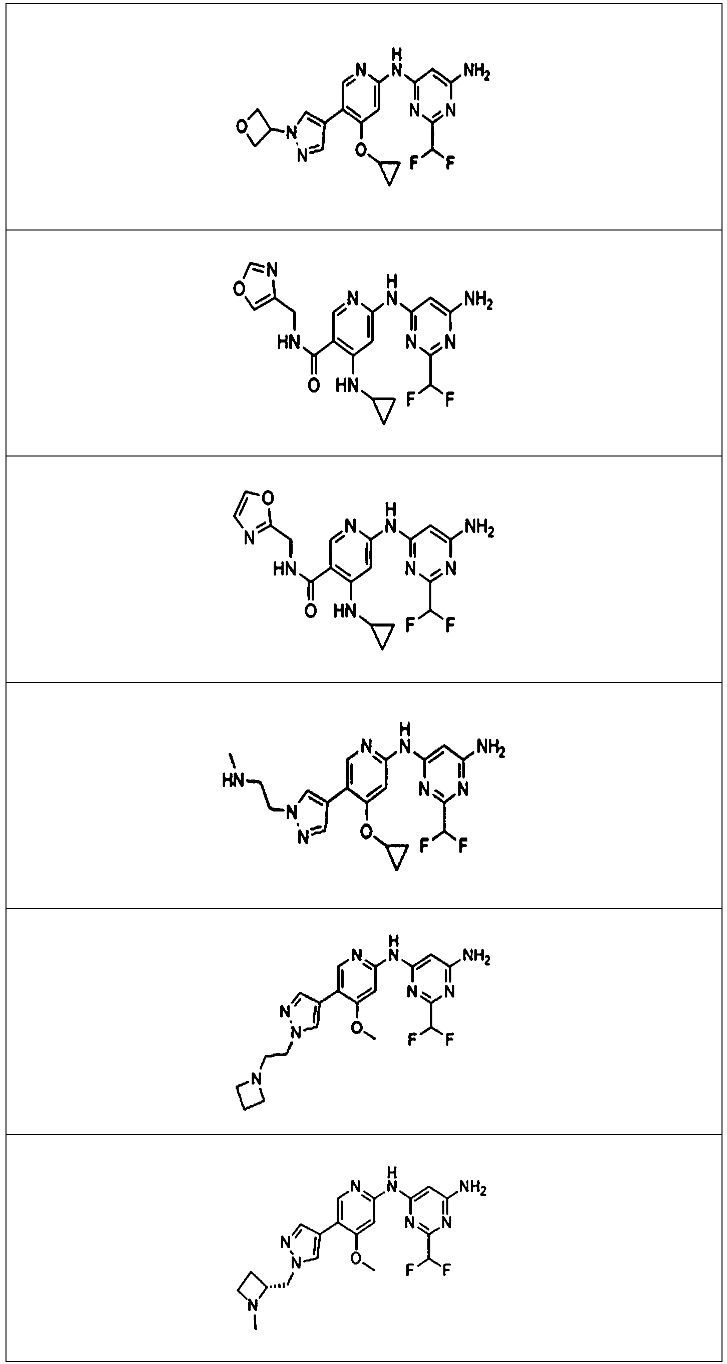

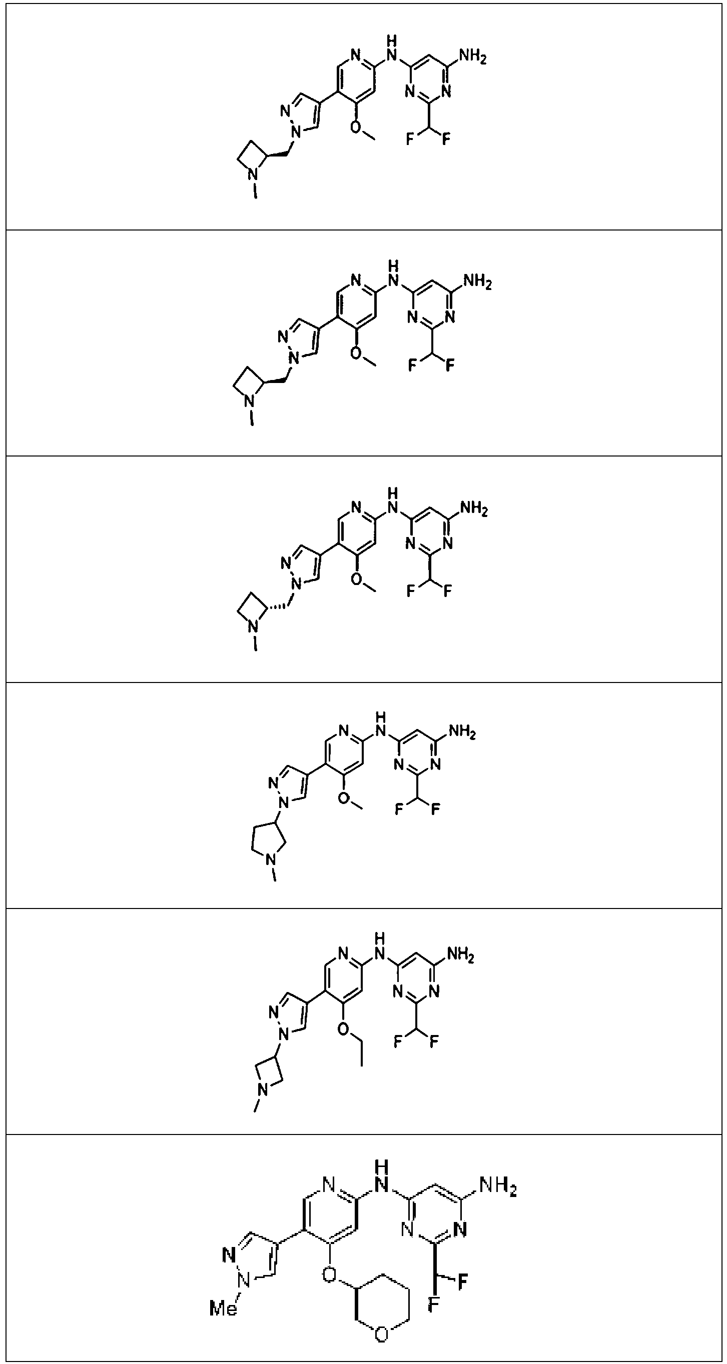

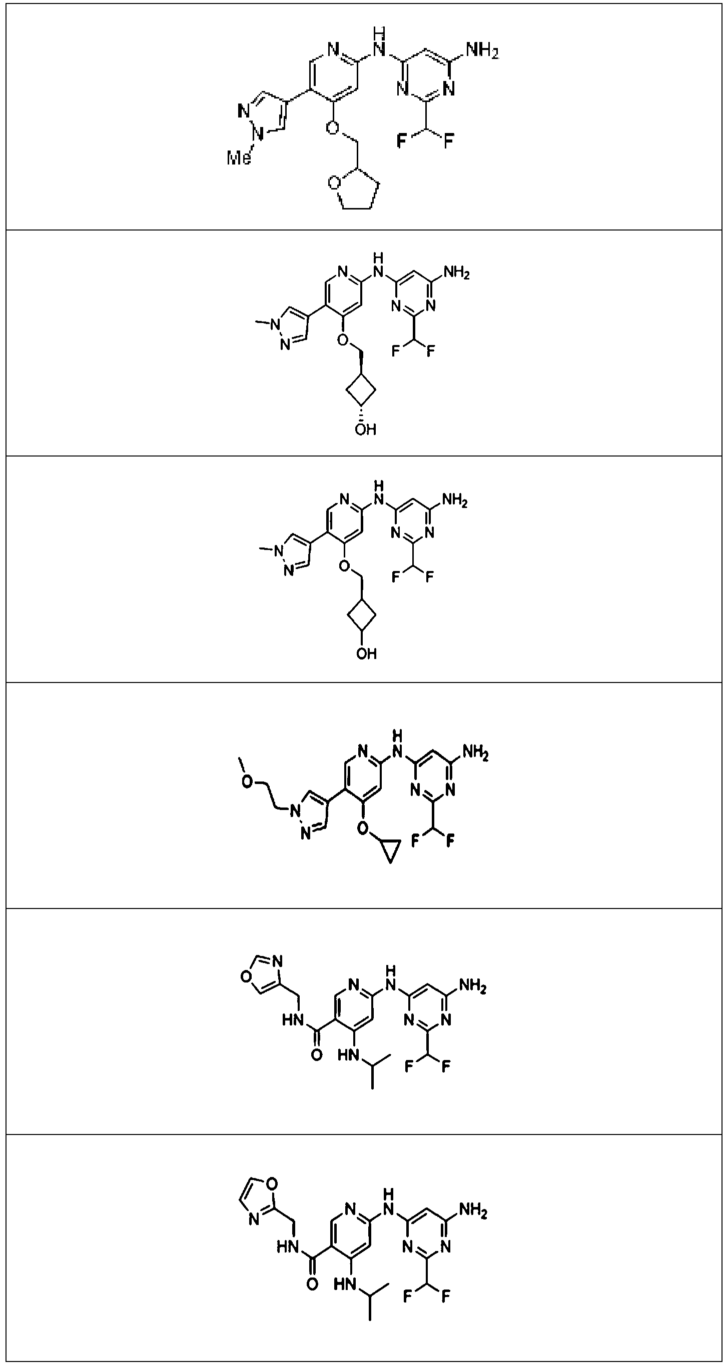

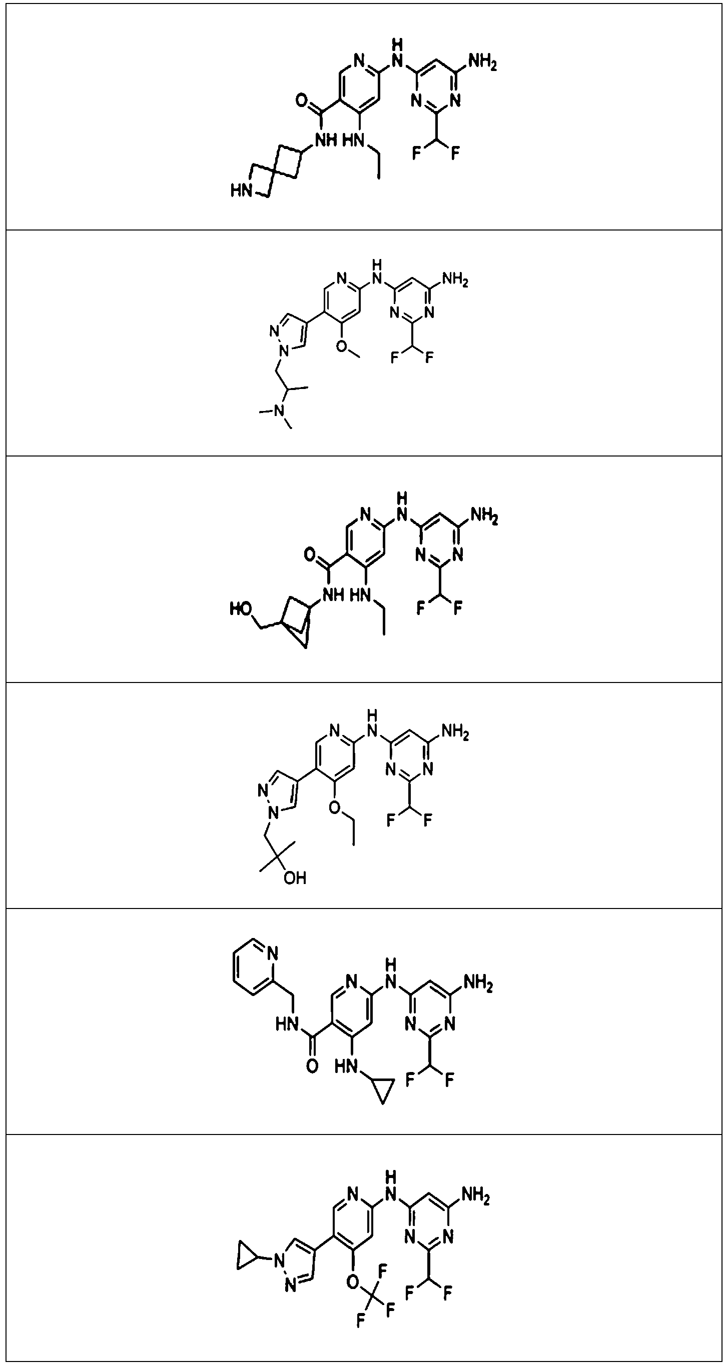

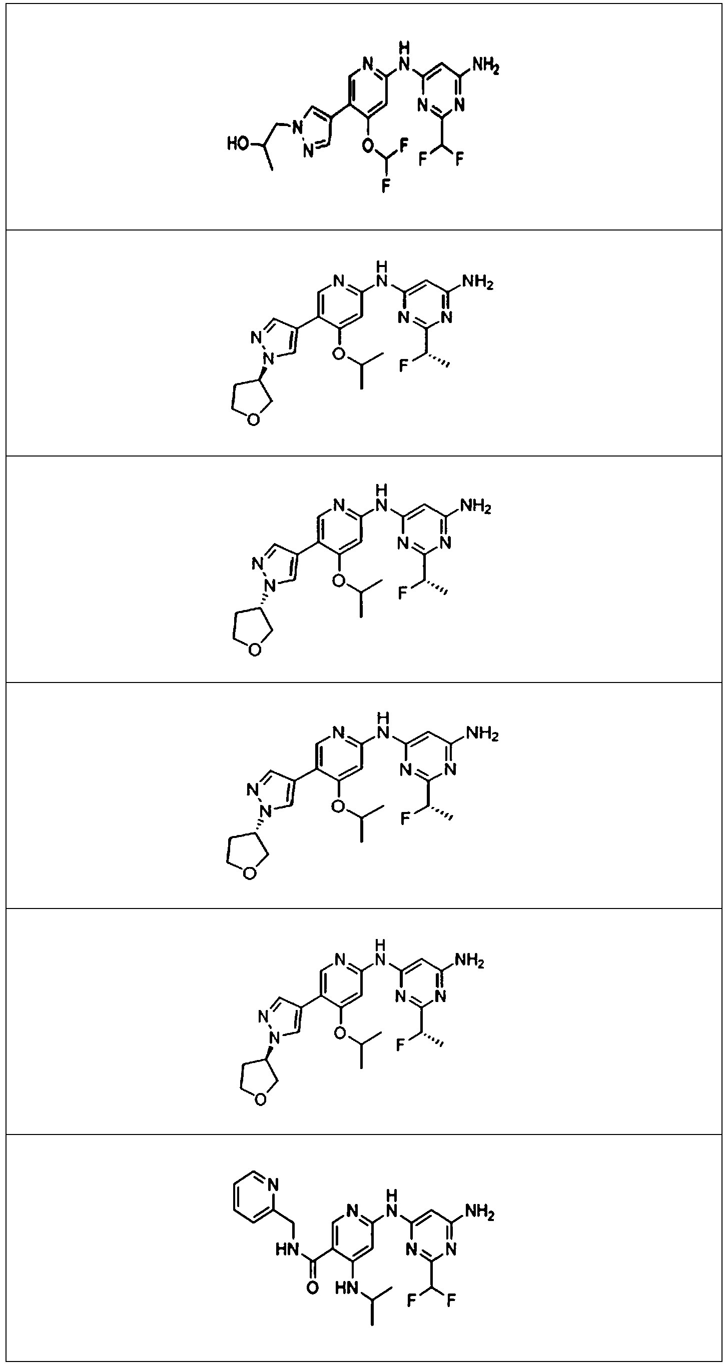

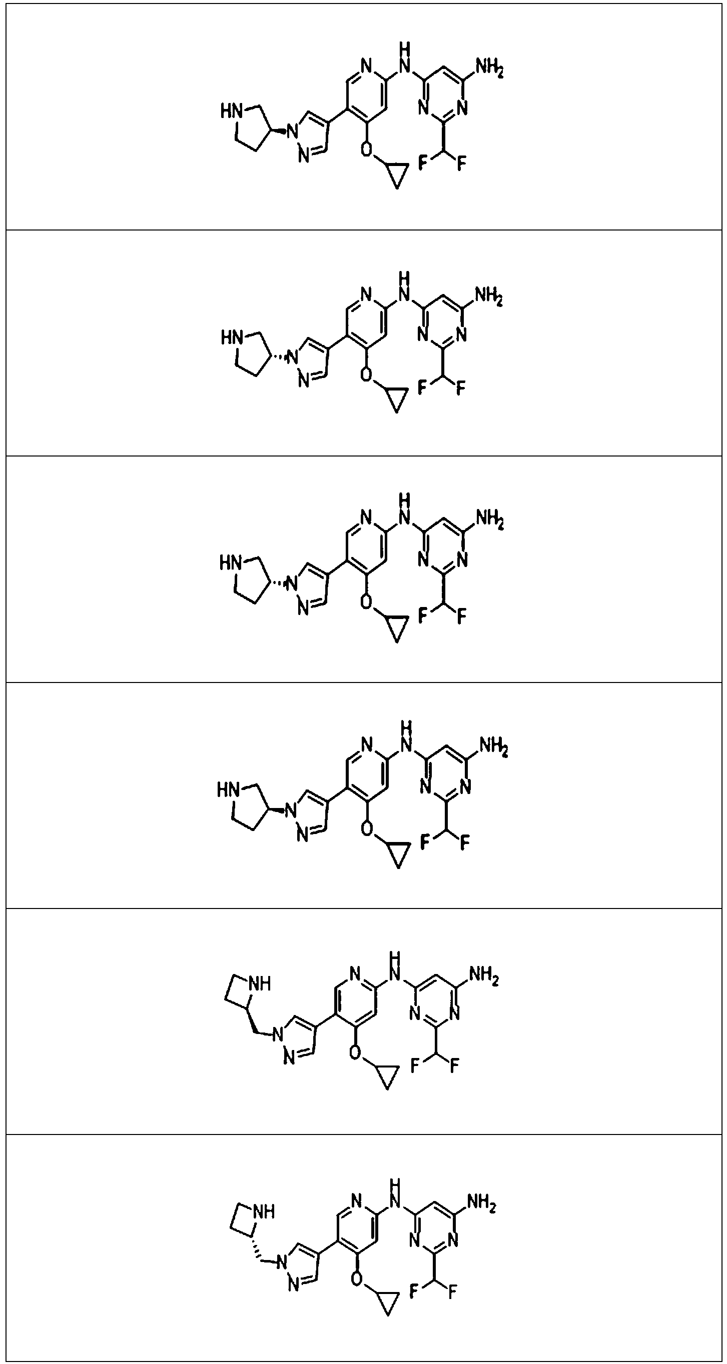

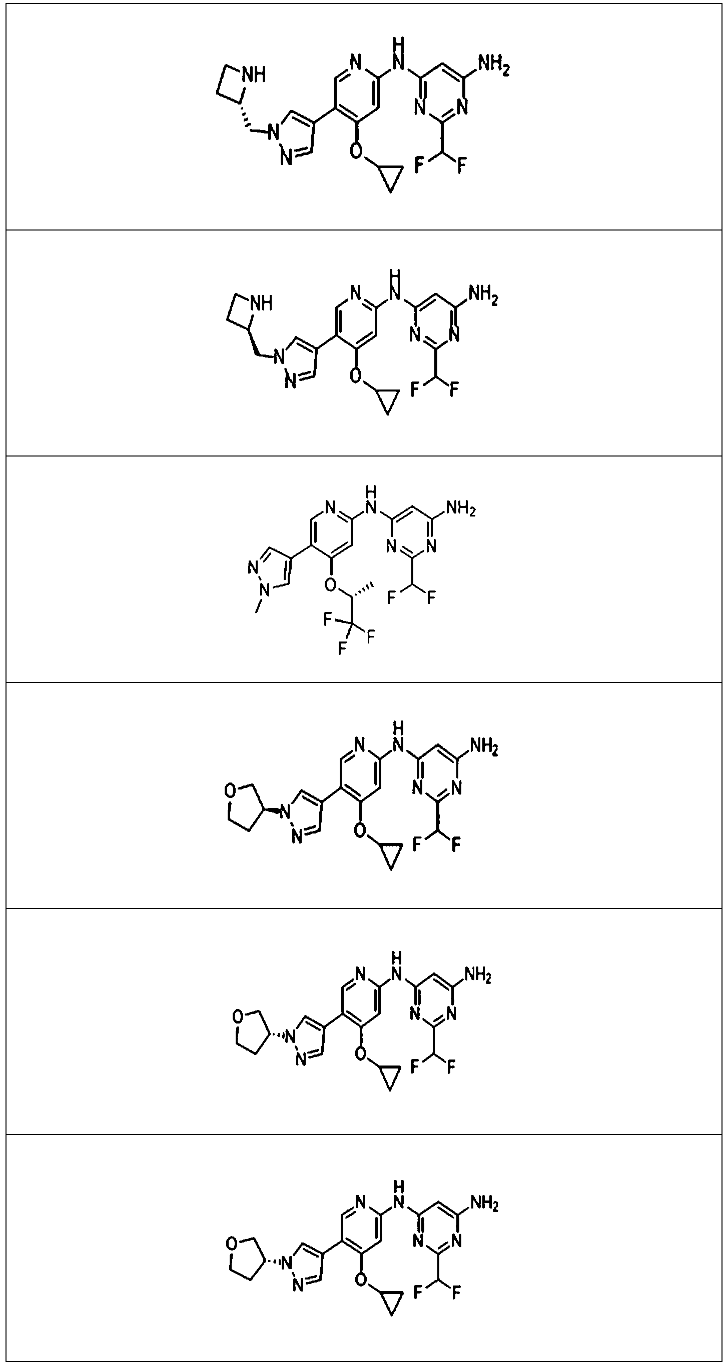

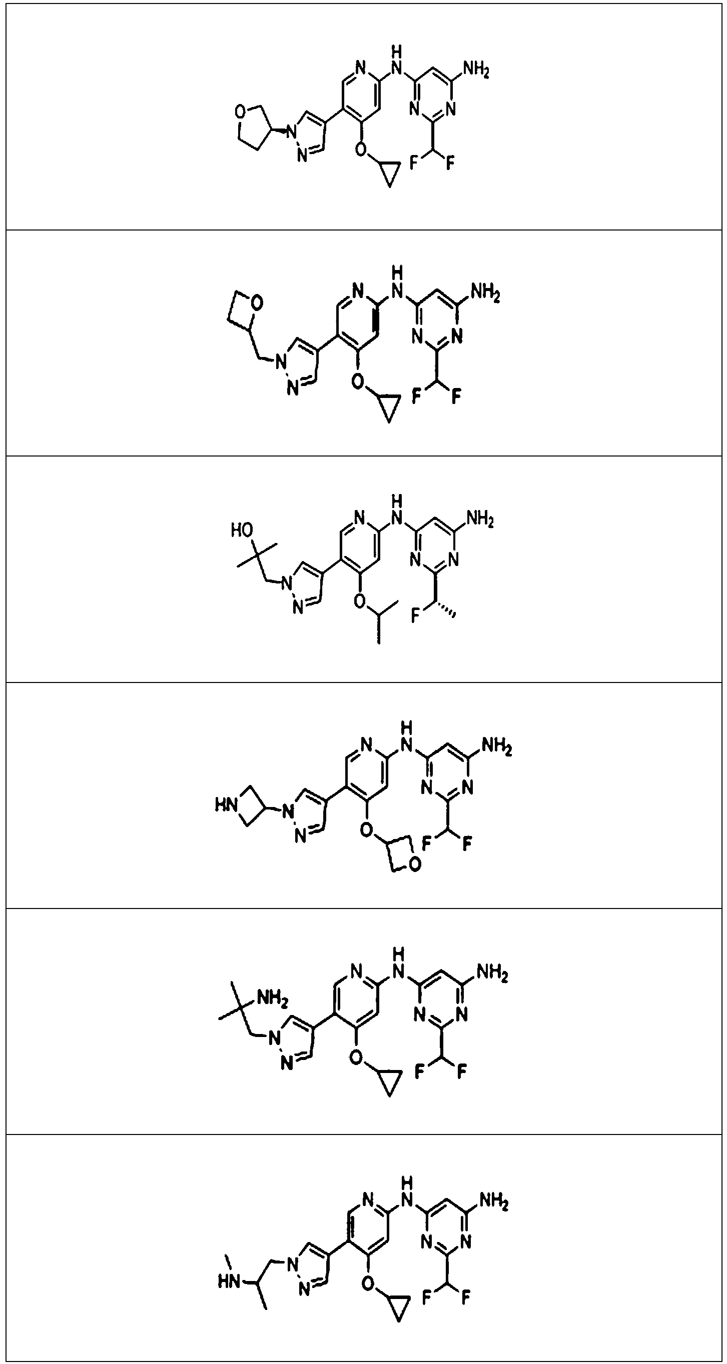

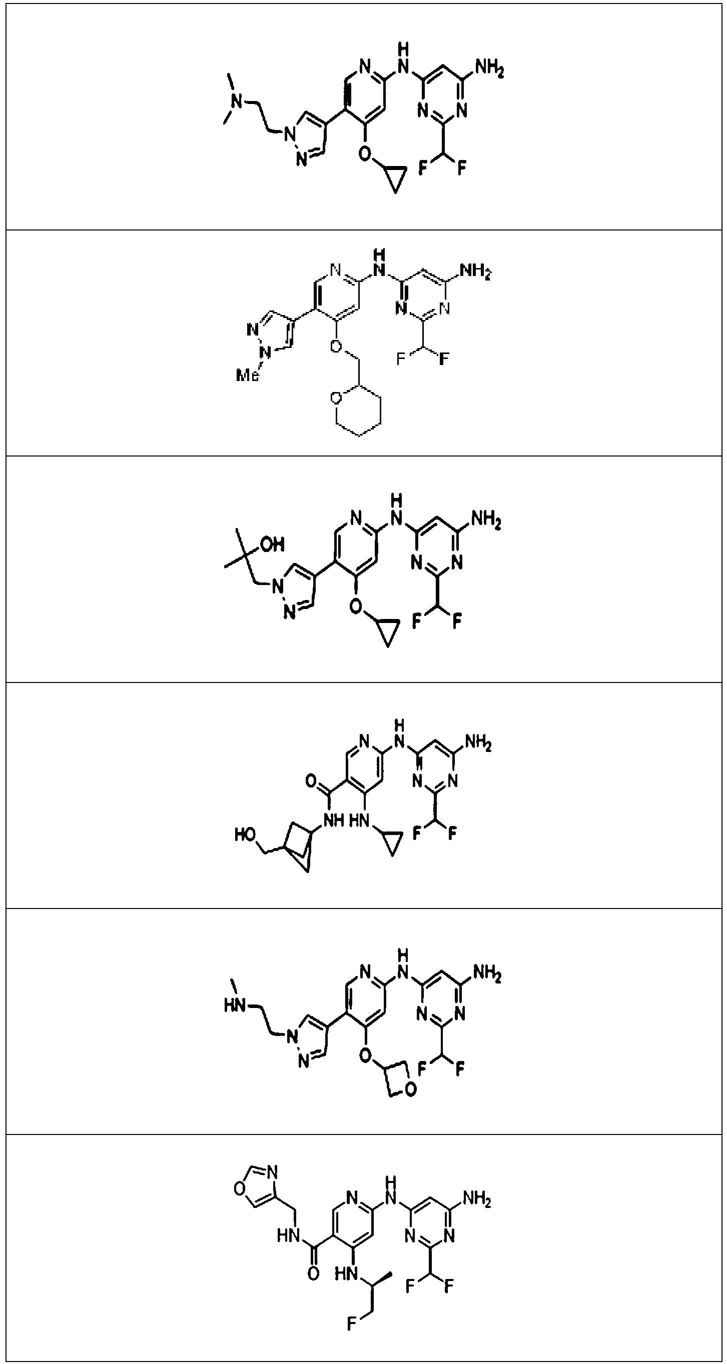

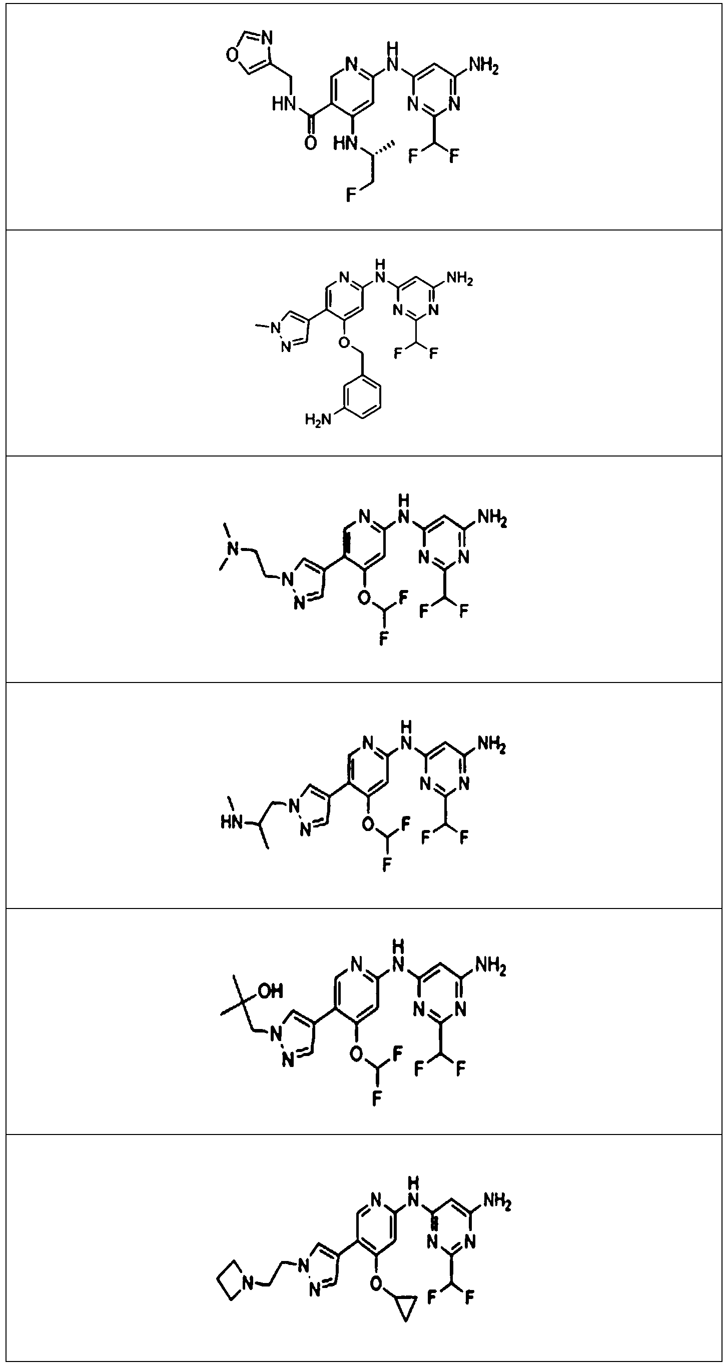

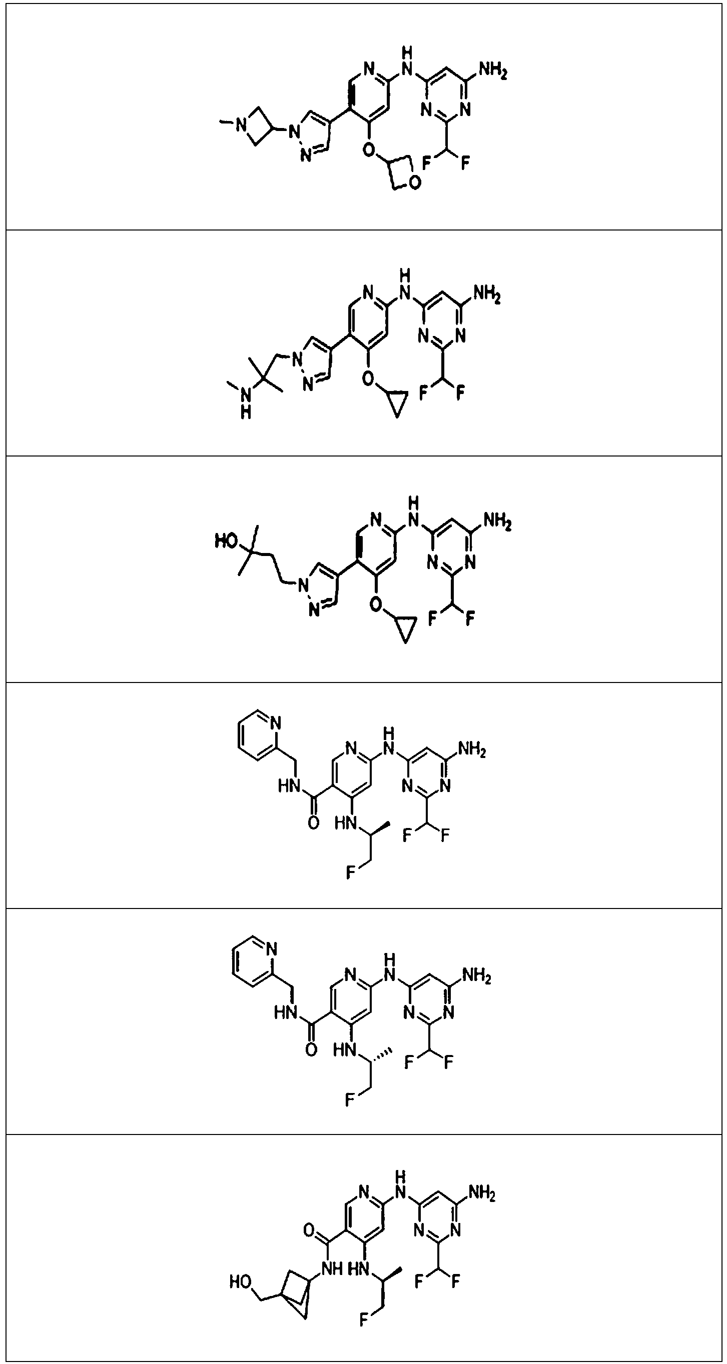

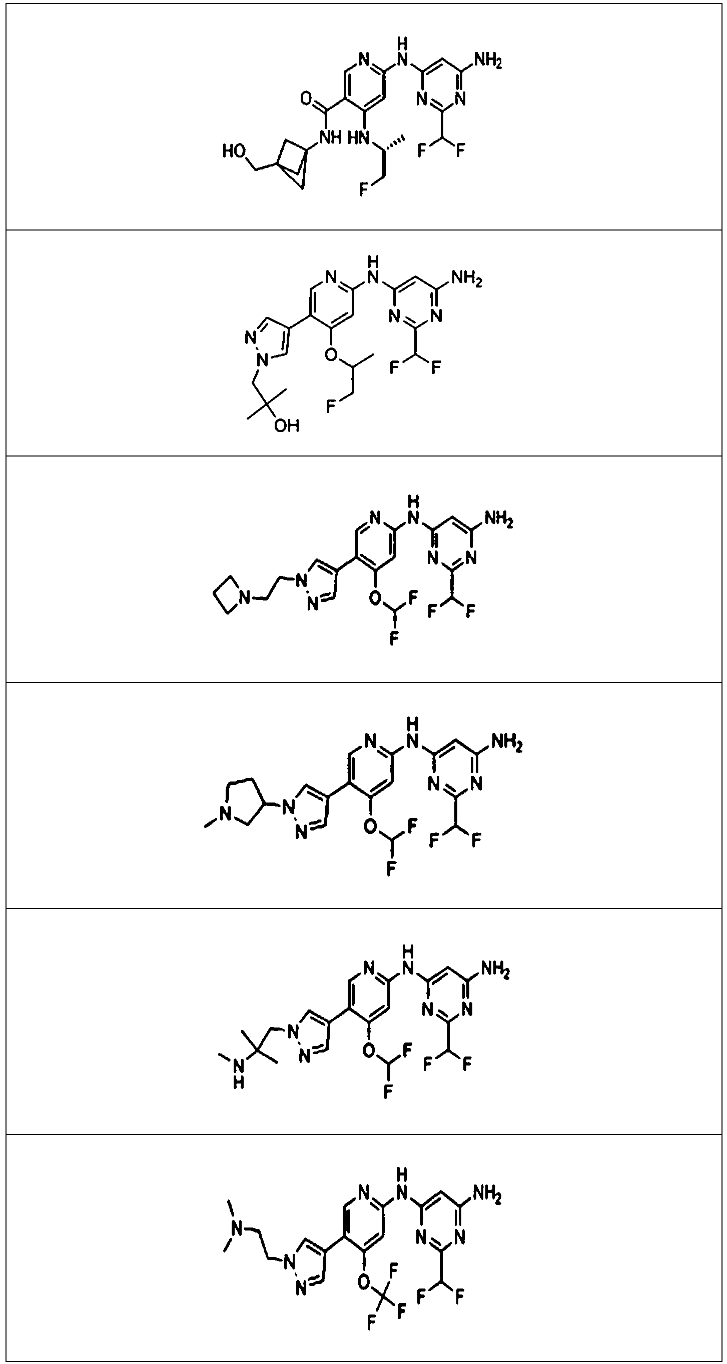

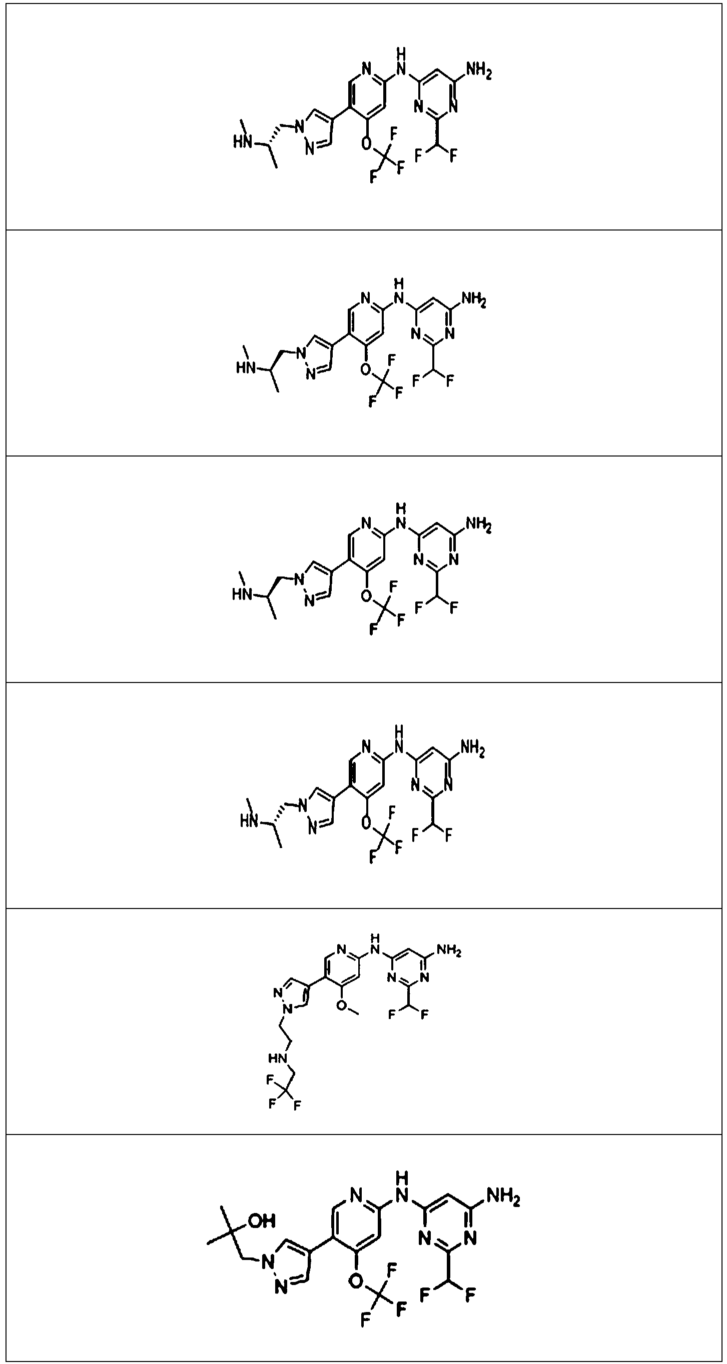

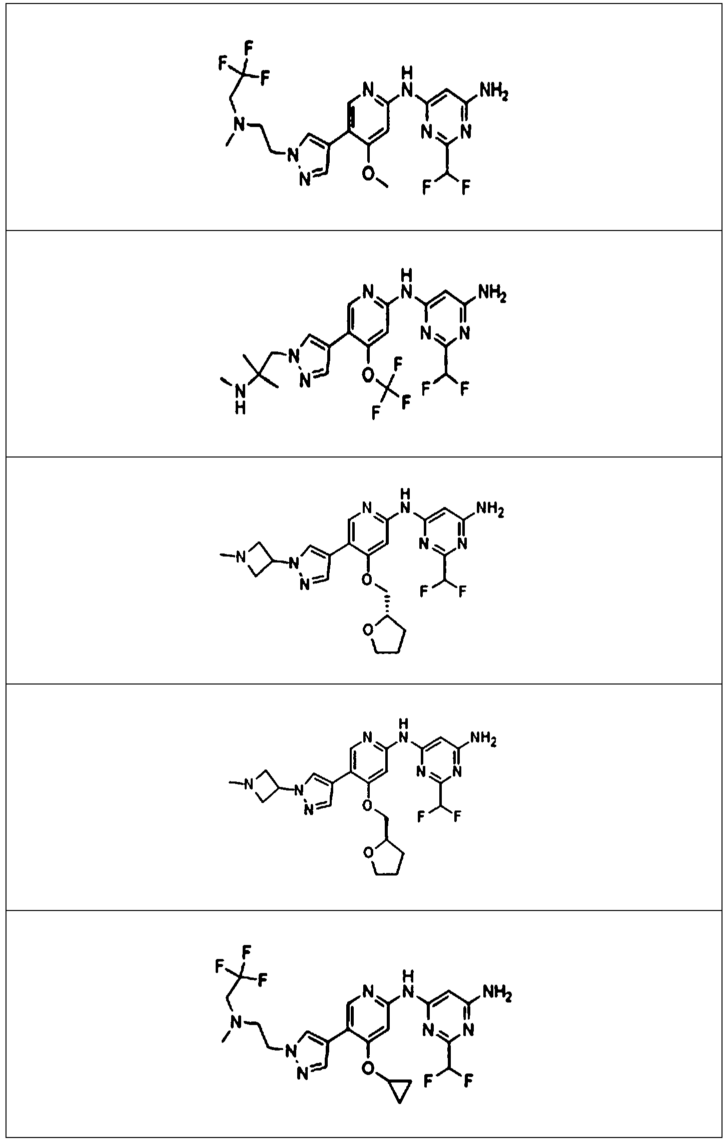

10. A compound according to claim 9, wherein the compound is selected from the group consisting of 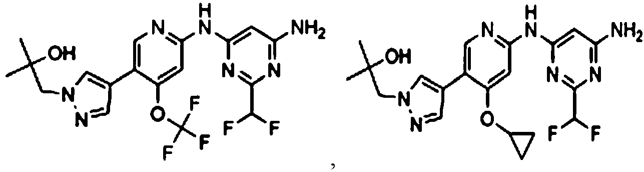 and 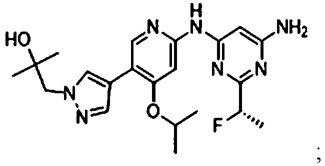 or a pharmaceutically acceptable salt thereof.

11. A pharmaceutical composition comprising the compound of any one of claims 1-10 or a pharmaceutically acceptable salt thereof, and a pharmaceutically acceptable carrier or excipient.

12. The compound of any one of claims 1 to 10 or pharmaceutical composition according to claim 11 for use in treating a patient suffering from gastrointestinal stromal tumor (GIST), comprising administering to the patient an effective amount of the compound of any one of claims 1-10, or a pharmaceutically acceptable salt thereof, or the pharmaceutical composition of claim 11.

13. The compound or pharmaceutical composition for use according to claim 12, wherein the patient received one or more prior treatments for the GIST; optionally wherein the GIST progressed after the prior treatment and optionally wherein the prior treatment comprises administering one or more agents, optionally wherein the prior treatment comprises administering one or more agents, each independently selected from: imatinib, sunitinib, regorafenib, nilotinib, cabozantinib, pazopanib, ponatinib, dasatinib, binimetinib, vandetanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus and larotrectinib, and pharmaceutically acceptable salts thereof; or
wherein the GIST is **characterized by** a tumor with one or more KIT mutations, optionally wherein the tumor has a primary activating KIT mutation and/or wherein the GIST is **characterized by** a tumor with one or more KIT mutations, each independently selected from an exon 9 KIT mutation, an exon 11 KIT mutation, an exon 13 KIT mutation and an exon 17 KIT mutation, and combinations thereof, optionally wherein each of the one or more KIT mutations is independently selected from N822K, D816V, D816E, D816F, D816H, D8161, D816Y, D820E, D820Y, D823D, A829P, K642E, V654A and N655K, and combinations thereof; or
wherein each of the one or more KIT mutations is independently selected from N822K, D816V, D816E, D816F, D816H, D8161, D816Y, D820E, D820Y, D823D, A829P and K642E, and combinations thereof; or
wherein the tumor has an exon 9 KIT mutation; or
wherein the tumor has an exon 11 KIT mutation; or
wherein the tumor has an exon 17 KIT mutation, optionally wherein the exon 17 KIT mutation is selected from N822K, D816V, D816E, D816F, D816H, D8161, D816Y, D820E, D820Y, D823D, and A829P; or
wherein the tumor has an exon 13 KIT mutation, optionally wherein the exon 13 KIT mutation is selected from K642E, V654A and N655K, and combinations thereof; and/or
wherein the tumor is mutation resistant to the one or more prior treatments, optionally where the tumor has an imatinib-resistant mutation, optionally wherein the imatinib-resistant mutation is selected from an exon 13 KIT mutation, an exon 17 KIT mutation and an exon 14 KIT mutation, and combinations thereof.

14. The compound of any one of claims 1 to 10 or pharmaceutical composition according to claim 11 for use in a method of treating a patient suffering from a malignant disease **characterized by** an exon 9 KIT mutation, an exon 11 KIT mutation, an exon 13 KIT mutation or an exon 17 KIT mutation, or a combination thereof, comprising administering to the patient an effective amount of the compound of any one of claims 1-10, or a pharmaceutically acceptable salt thereof, or the pharmaceutical composition of claim 12;
optionally wherein the malignant disease is selected from gastrointestinal stromal tumor (GIST), AML (acute myeloid leukemia), melanoma, lung cancer, uterine cancer, astrocytoma, liver cancer, seminoma, renal cell carcinoma, intercranial germ cell tumor, pancreatic cancer and mediastinal B-cell lymphoma.

15. A combination for use in a method of treating a patient suffering from gastrointestinal stromal tumor (GIST), comprising administering to the patient an effective combination comprising:
a compound of any one of claims 1-10, or a pharmaceutically acceptable salt thereof, or a pharmaceutical composition of claim 11, and
at least one additional agent;
optionally wherein the additional agent is an exon 9 KIT inhibitor or exon 11 KIT inhibitor; or
wherein the additional agent is imatinib or a pharmaceutically acceptable salt thereof or
wherein the additional agent is an exon 17 KIT inhibitor, optionally wherein the additional agent is selected from avapritinib, BLU-263, ripretinib, AZD3229, and bezuclastinib, and pharmaceutically acceptable salts thereof.

16. The compound of any one of claims 1 to 10 or pharmaceutical composition according to claim 11 for use in a method of treating a patient suffering from a malignant disease **characterized by** a tumor with an exon 9 KIT mutation, an exon 11 KIT mutation, or an exon 17 KIT mutation, or a combination thereof, comprising:
(a) obtaining a biological sample from the patient;
(b) detecting the presence or absence of an exon 13 KIT mutation or exon 14 KIT mutation, optionally wherein the exon 13 KIT mutation is V654A or N655K or wherein the exon 14 KIT mutation is N680K; and
(c) administering an effective amount of a compound of any one of claims 1-10, or a pharmaceutically acceptable salt thereof, or a pharmaceutical composition of claim 11, to the patient, if the mutation is detected.

17. The compound of any one of claims 1 to 10, or pharmaceutical composition according to claim 11, for use in therapy.

## Patentansprüche

1. Verbindung der Formel (I): 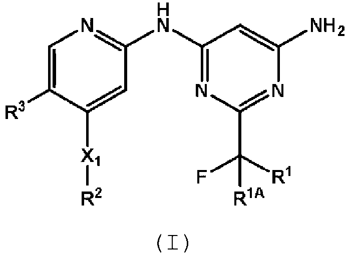 oder ein pharmazeutisch akzeptables Salz davon, wobei:
R¹ und R^{1A} jeweils unabhängig aus H, Halogen und CH₃ ausgewählt sind, oder R¹ und R^{1A} zusammengenommen mit dem Kohlenstoff, an den sie aus gebunden sind, Cyclopropyl bilden;
R² aus C ₁₋₅ Alkyl, CD₃, C₃₋₆Cycloalkyl, bicyclischem[1.1.1]Pentan und 4- bis 6-gliedrigem O-haltigem Heterocyclus ausgewählt ist, wobei das Alkyl, Cycloalkyl oder der Heterocyclus optional durch 1-3 R⁴ substituiert ist;
jedes R⁴ unabhängig aus Halogen, CH₃, C ₂₋₃Alkenyl, OH, CH₂OH, C₄₋₆Cycloalkyl, 4- bis 6-gliedrigem Heterocyclus, der O enthält, und Phenyl ausgewählt ist, wobei das Alkyl, Cycloalkyl, der Heterocyclus oder das Phenyl optional mit OH oder NH₂, C₁₋₂Alkyl, CH₂NH₂ oder Halogen substituiert ist;
oder;
R³ C(O)NHR⁵, 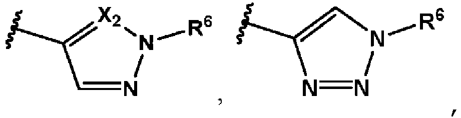 oder 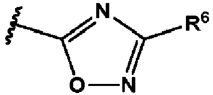 ist,
X₁ NH oder O ist;
X₂ N oder CH ist;
X₃ N oder CH ist;
R⁵ aus H, C₁₋₃Alkyl, CD₃, C₃₋₄Cycloalkyl und Bicyclo[1.1.1]Pentan ausgewählt ist, wobei das Alkyl, Cycloalkyl oder Bicyclo[1.1.1]Pentan optional durch 1-2 R⁷ substituiert ist;
jedes R⁷ unabhängig von CN, NH₂, OH, CH₂ OH, Cyclopropyl, Pyridinyl und Oxazolyl ausgewählt ist, oder zwei R⁷, die an dasselbe Kohlenstoffatom gebunden sind, zusammengenommen einen 4-gliedrigen Heterocyclus bilden, der N enthält;
R⁶ aus C₁₋₅Alkyl, CHF₂, CF₃, 4- oder 5-gliedrigem Heterocyclus, der N oder O enthält, und C₃₋₄Cycloalkyl ausgewählt ist, wobei das Alkyl oder der Heterocyclus optional mit einem R⁸ substituiert ist;
R⁸ aus OH, NR⁹R⁹, OCH₃, CH₃ und 4-gliedrigem Heterocyclus, der N oder O enthält, ausgewählt ist, wobei das Alkyl oder der Heterocyclus optional durch ein R¹⁰ substituiert ist;
jedes R⁹ unabhängig aus H, CH₃ und CH₂CF₃ ausgewählt ist; und
R¹⁰ aus CH₃ und CF₃ ausgewählt ist.

2. Verbindung nach Anspruch 1 oder ein pharmazeutisch akzeptables Salz davon, wobei:
R¹ und R^{1A} jeweils unabhängig aus H, Halogen und CH₃ ausgewählt sind;
R² C ₁₋₅Alkyl, CD₃, C₃₋₆Cycloalkyl und 4- bis 6-gliedrigem O-haltigem Heterocyclus ausgewählt ist, wobei das Alkyl, Cycloalkyl oder der Heterocyclus optional durch 1-3 R⁴ substituiert ist;
jedes R⁴ unabhängig aus Halogen, CH₃, OH, C₄₋₆Cycloalkyl, 4- bis 6-gliedrigem O-haltigem Heterozyklus und Phenyl ausgewählt ist, wobei das Cycloalkyl oder Phenyl optional durch OH oder NH₂ substituiert ist;
jedes R⁷ unabhängig aus CN, OH, CH₂OH, Cyclopropyl, Pyridinyl und Oxazolyl ausgewählt ist, oder zwei R⁷, die an dasselbe Kohlenstoffatom gebunden sind, zusammengenommen 4-gliedrigen Heterocyclus bilden, der N enthält; und
R¹⁰ CH₃ ist.

3. Verbindung nach Anspruch 1 oder 2, wobei die Verbindung durch Formel (II) dargestellt ist: 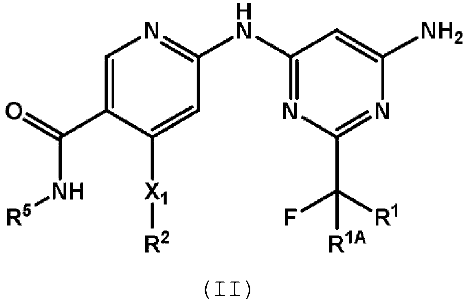
oder ein pharmazeutisch akzeptables Salz davon; oder
wobei die Verbindung durch Formel (III) dargestellt ist: 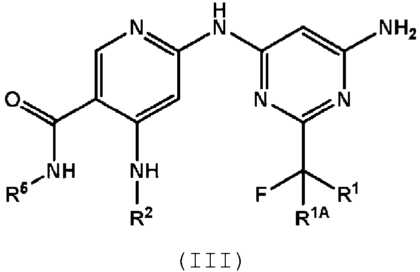
oder ein pharmazeutisch akzeptables Salz davon.

4. Verbindung nach Anspruch 3 oder ein pharmazeutisch akzeptables Salz davon, wobei:
R¹ und R^{1A} jeweils unabhängig aus H, Halogen und CH₃ ausgewählt sind;
R² aus C ₁₋₅Alkyl, CD₃, C₃₋₄Cycloalkyl und 4- bis 6-gliedrigem O-haltigem Heterocyclus ausgewählt ist, wobei das Alkyl, Cycloalkyl oder der Heterocyclus optional durch 1-3 R⁴ substituiert ist;
jedes R⁴ unabhängig aus Halogen, CH₃ und Phenyl ausgewählt ist;
R⁵ aus H, C₁₋₃Alkyl, CD₃, C₃₋₄Cycloalkyl und Bicyclo[1.1.1]Pentan ausgewählt ist, wobei das Alkyl, Cycloalkyl oder Bicyclo[1.1.1]Pentan optional durch 1-2 R⁷ substituiert ist; und
jedes R⁷ unabhängig aus CN, OH, CH₂OH, Cyclopropyl, Pyridinyl und Oxazolyl ausgewählt ist, oder zwei R⁷, die an dasselbe Kohlenstoffatom gebunden sind, zusammengenommen 4-gliedrigen Heterocyclus bilden, der N enthält; und/oder
wobei R² aus CH₃, CD₃, CH₂CH₃, CH₂CH₂CH₃, CH(CH₃)₂, CH₂CH(CH₃)₂, CH(CH₃)CH₂CH₃, Cyclobutyl, Cyclopropyl, Oxetanyl, Tetrahydrofuranyl, Tetrahydrofuranyl und Tetrahydropyranyl ausgewählt ist, die jeweils optional durch 1-3 R⁴ substituiert sind; und/oder
wobei R² aus 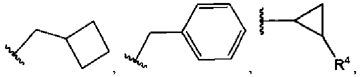 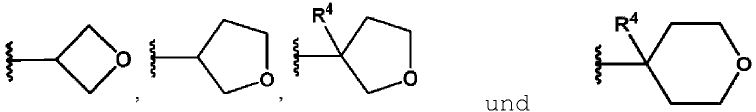 ausgewählt ist; und/oder
wobei jedes R⁴ unabhängig von F, CH₃, Cyclobutyl und Phenyl ausgewählt ist; und/oder
wobei R⁵ aus H, CH₃, CD₃, CH₂CH₃, CH₂CH₂CH₃, CH(CH₃)₂, Cyclobutyl, Cyclopropyl und Bicyclo[1.1.1]Pentanyl ausgewählt ist, wobei die CH₃, CH₂CH₃, CH₂CH₂CH₃, CH(CH₃)₂, Cyclobutyl, Cyclopropyl oder Bicyclo[1.1.1]Pentanyl optional mit 1-2 R⁷ substituiert sind; und/oder
wobei R⁵ aus 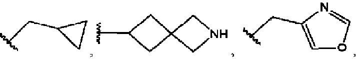 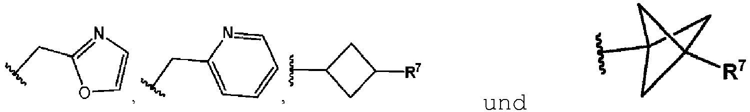 ausgewählt ist; und/oder
wobei jedes R⁷ unabhängig aus CN, CH₂OH, Cyclopropyl, 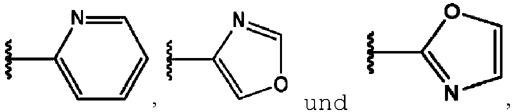 ausgewählt ist, oder zwei R⁷, die an dasselbe Kohlenstoffatom gebunden sind, zusammengenommen 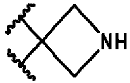 bilden; und/oder
wobei R¹ und R^{1A} jeweils unabhängig voneinander aus H, F und CH₃ ausgewählt sind.

5. Verbindung nach Anspruch 1 oder 2, wobei die Verbindung durch Formel (IV), (V) oder (VI) dargestellt ist: 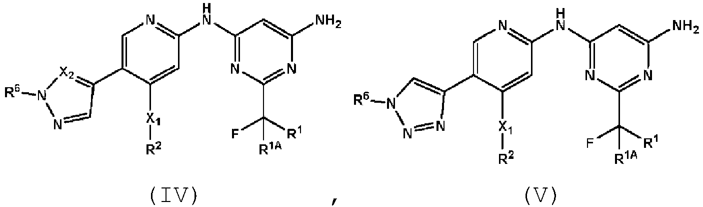 oder 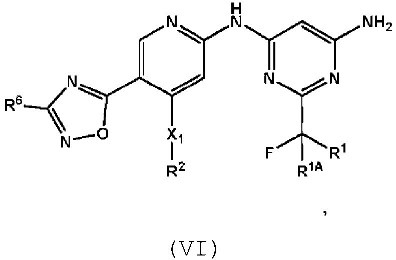
oder ein pharmazeutisch akzeptables Salz davon; oder
wobei die Verbindung durch Formel (VII), (VIII), (IX) oder (X) dargestellt ist: 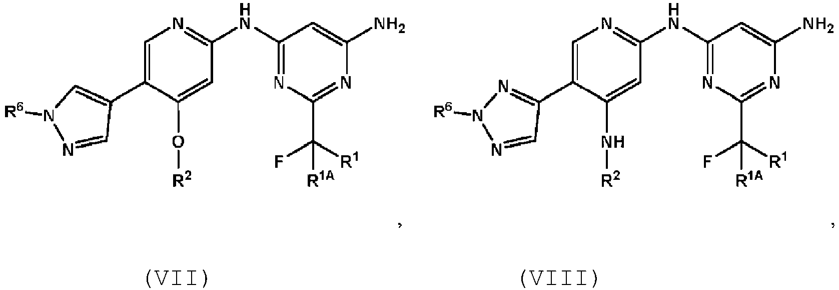 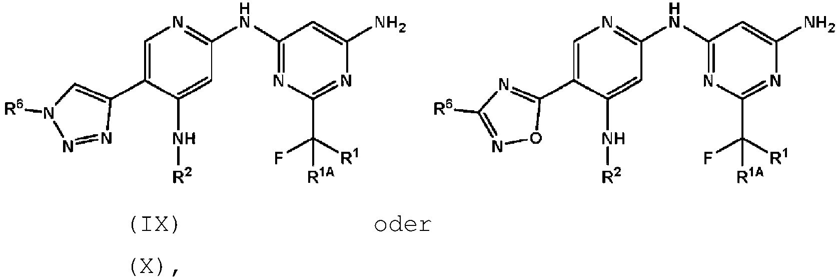
oder ein pharmazeutisch akzeptables Salz davon.

6. Verbindung nach Anspruch 5 oder ein pharmazeutisch akzeptables Salz davon, wobei:
R¹ und R^{1A} jeweils unabhängig aus H, Halogen und CH₃ ausgewählt sind;
R² aus C₁₋₃Alkyl, CD₃, C₃₋₄Cycloalkyl und 4- bis 6-gliedrigem Heterocyclus, der ein O enthält, ausgewählt ist, wobei das Alkyl, Cycloalkyl oder der Heterocyclus optional durch 1-3R⁴ substituiert ist;
jedes R⁴ unabhängig aus Halogen, OH, Cyclopropyl, 4- bis 6-gliedrigem Heterocyclus, der ein O enthält, und Phenyl ausgewählt ist, wobei das Cyclopropyl oder Phenyl optional durch OH oder NH₂ substituiert ist;
R⁶ aus C₁₋₄Alkyl, CHF₂, CF₃, 4- oder 5-gliedrigem Heterocyclus, der N oder O enthält, und C₃₋₄Cycloalkyl ausgewählt ist, wobei das Alkyl, der Heterocyclus oder das Cycloalkyl optional durch R⁸ substituiert ist;
R⁸ aus OH, NR⁹R⁹, OCH₃, CH₃ und 4-gliedrigem Heterocyclus, der N oder O enthält, ausgewählt ist, wobei der Heterozyklus optional durch CH₃ substituiert ist; und
jedes R⁹ unabhängig aus H, CH₃ und CH₂CF₃ ausgewählt ist; und/oder
wobei R² aus CH₃, CD₃, CH₂CH₃, CH₂CH₂CH₃, CH(CH₃)₂, Cyclobutyl, Cyclopropyl, Oxetanyl, Tetrahydrofuranyl, Tetrahydrofuranyl und Tetrahydropyranyl ausgewählt ist, die jeweils optional durch 1-3 R⁴ substituiert sind; und/oder
wobei R² ausgewählt ist aus 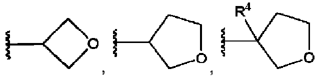 und 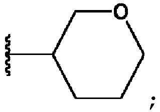 und/oder
wobei jedes R⁴ unabhängig aus F, OH, Cyclobutyl, Oxetanyl, Oxetanyl, Phenyl, Tetrahydrofuranyl und Tetrahydropyranyl ausgewählt ist, wobei das Cyclobutyl, Oxetanyl, Oxetanyl, Phenyl, Tetrahydrofuranyl oder Tetrahydropyranyl optional durch OH oder NH₂ substituiert ist; und/oder
wobei jedes R⁴ unabhängig aus F, OH, 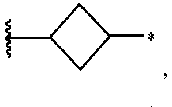 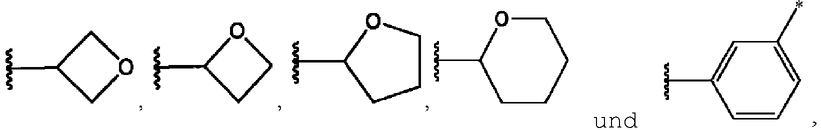 ausgewählt ist, wobei *- OH oder NH₂ darstellt; und/oder
wobei R⁶ aus CH₃, CH₂CH₃, CH₂CH₂CH₃, CH(CH₃)₂, CH₂CH(CH₃)₂, C(CH₃)₃, CH₂CH₂CH(CH₃)₂, CHF₂, CF₃, Cyclopropyl, Cyclobutyl, Azetidinyl, Oxetanyl, Tetrahydrofuranyl und Pyrrolidinyl ausgewählt ist, die jeweils optional durch R⁸ substituiert sind; oder
wobei R⁶ aus 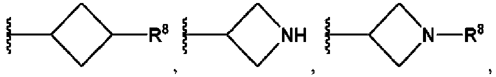 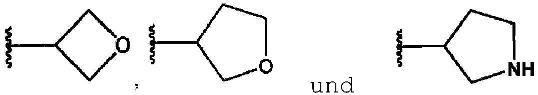 ausgewählt ist; und/oder
wobei R⁸ ausgewählt ist aus OH, OCH₃, CH₃, NH₂, NHCH₃, N(CH₃)₂, NHCH₂CF₃, N(CH₃)CH₂CF₃, Azetidinyl, Azetidinyl und Oxetanyl; und/oder
wobei R⁸ aus 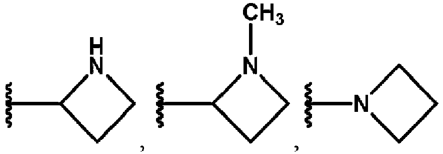 und 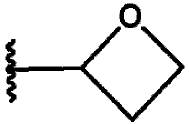 ausgewählt ist; und/oder
wobei R¹ und R^{1A} jeweils unabhängig voneinander aus H, F und CH₃ ausgewählt sind.

7. Verbindung nach Anspruch 1 oder 2, wobei die Verbindung durch Formel (III) oder (VII) dargestellt ist: 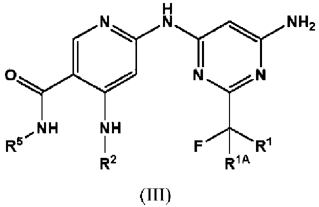 oder 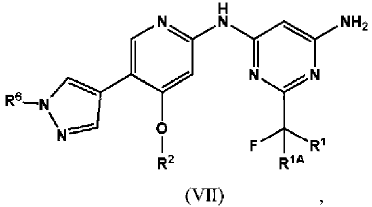 oder ein pharmazeutisch akzeptables Salz davon, wobei:
R¹ und R^{1A} jeweils unabhängig aus H, F und CH₃ ausgewählt sind;
R² aus C₁₋₃Alkyl, CD₃, C₃₋₄Cycloalkyl und 4-gliedrigem O-haltigem Heterocyclus ausgewählt ist, wobei das Alkyl optional durch 1-3 Halo substituiert ist;
R⁵ aus CH₃, CH₂CH₃ und Cyclopropyl ausgewählt ist;
R⁶ aus C₁₋₅Alkyl, CHF₂, CF₃ und 4- bis 5-gliedrigem O- oder N-haltigem Heterocyclus ausgewählt ist, wobei das Alkyl oder der Heterocyclus optional durch R⁸ substituiert ist;
R⁸ aus OH, NR⁹R⁹, OCH₃, CH₃ und 4-gliedrigem O- oder N-haltigem Heterocyclus ausgewählt ist; und
jedes R⁹ unabhängig aus H und CH₃ ausgewählt ist;
optional wobei R² aus CH₃, CHF₂, CF₃, CD₃, CH₂CH₃, CH₂CF₃, CH₂CH₂CH₃, CH(CH₃)₂, CH(CH₃)CH₂F, CH(CH₃)CHF₂, Cyclobutyl, Cyclopropyl und 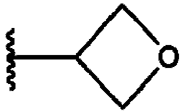 ausgewählt ist.

8. Verbindung nach Anspruch 7 oder ein pharmazeutisch akzeptables Salz davon, wobei R⁶ aus CH₃, CH₂CH₃, CH₂CH₂CH₃, CH₂CH(CH₃)₂, CH₂CH₂CH(CH₃)₂, CHF₂, Azetidinyl, Oxetanyl, Tetrahydrofuranyl und Pyrrolidinyl ausgewählt ist, die jeweils optional durch ein R⁸ substituiert sind; und/oder
wobei R⁶ aus 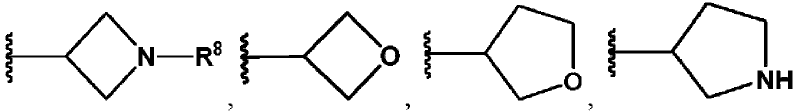 und 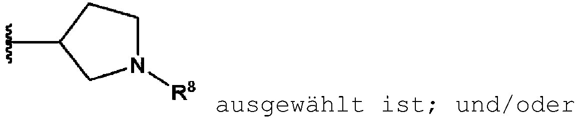
wobei R⁸ aus OH, OCH₃, NH₂, NHCH₃, N (CH₃)₂, CH₃, 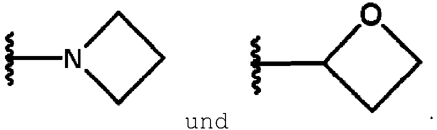 ausgewählt ist.

9. Verbindung nach Anspruch 1, ausgewählt aus der Gruppe bestehend aus den Verbindungen in der nachstehenden Tabelle oder einem pharmazeutisch akzeptablen Salz davon.

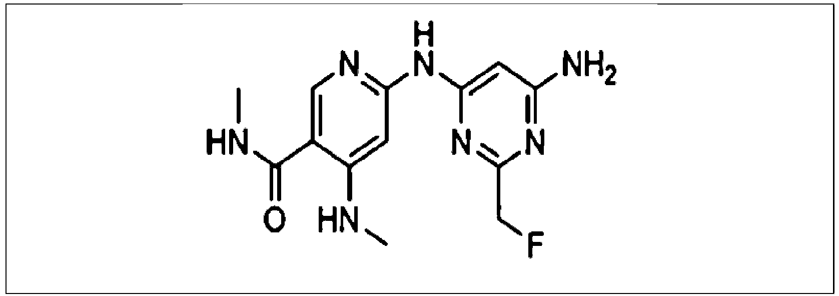

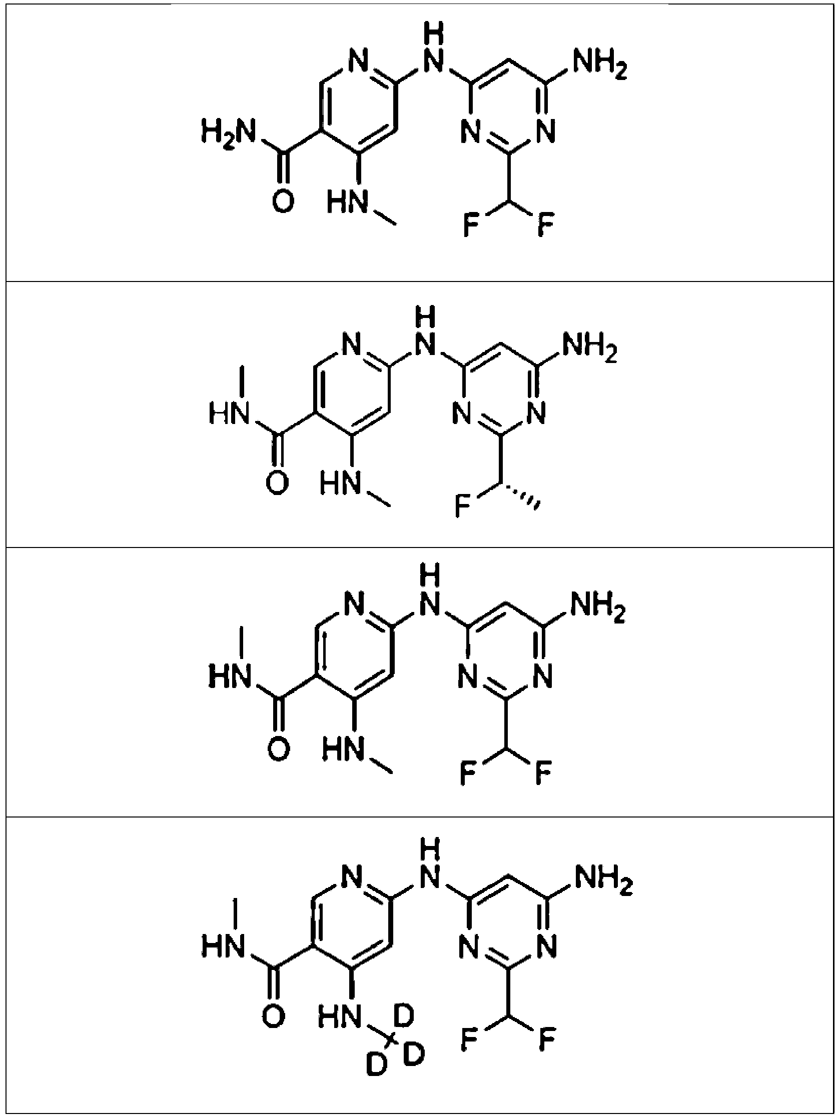

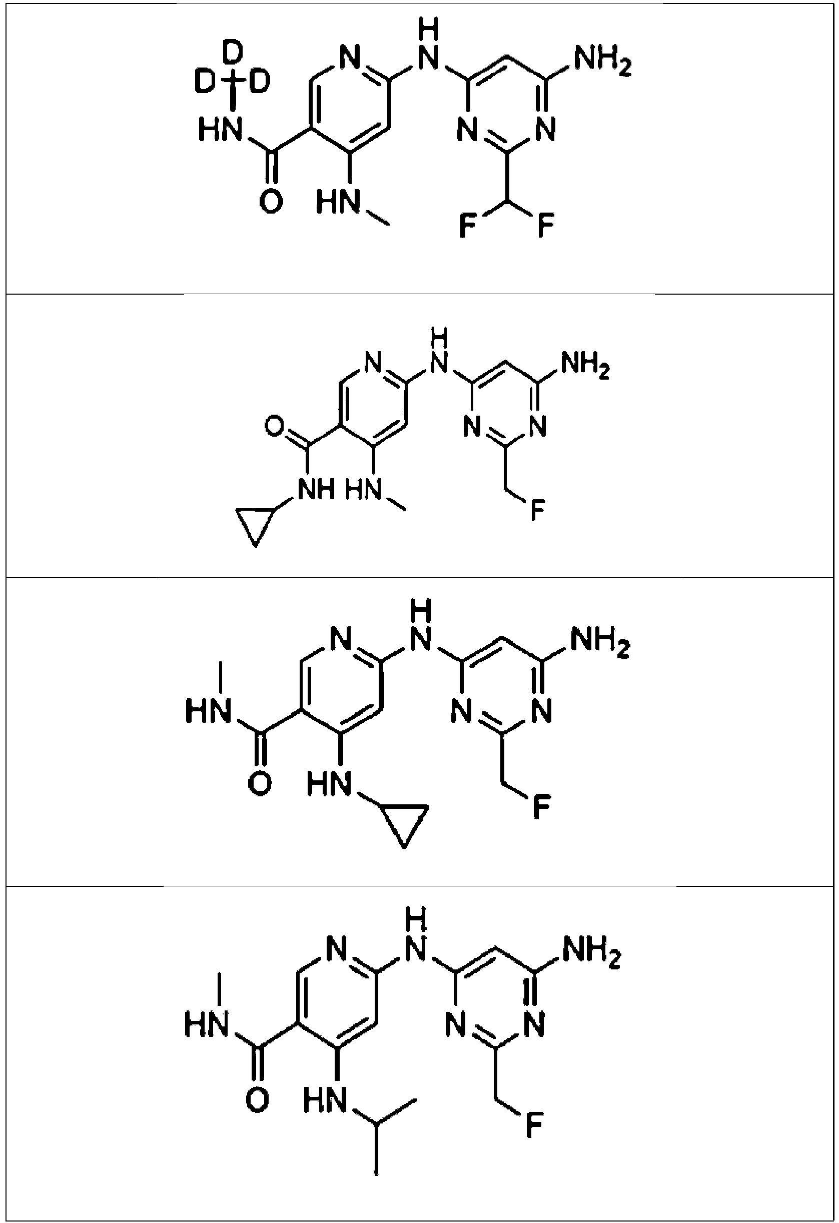

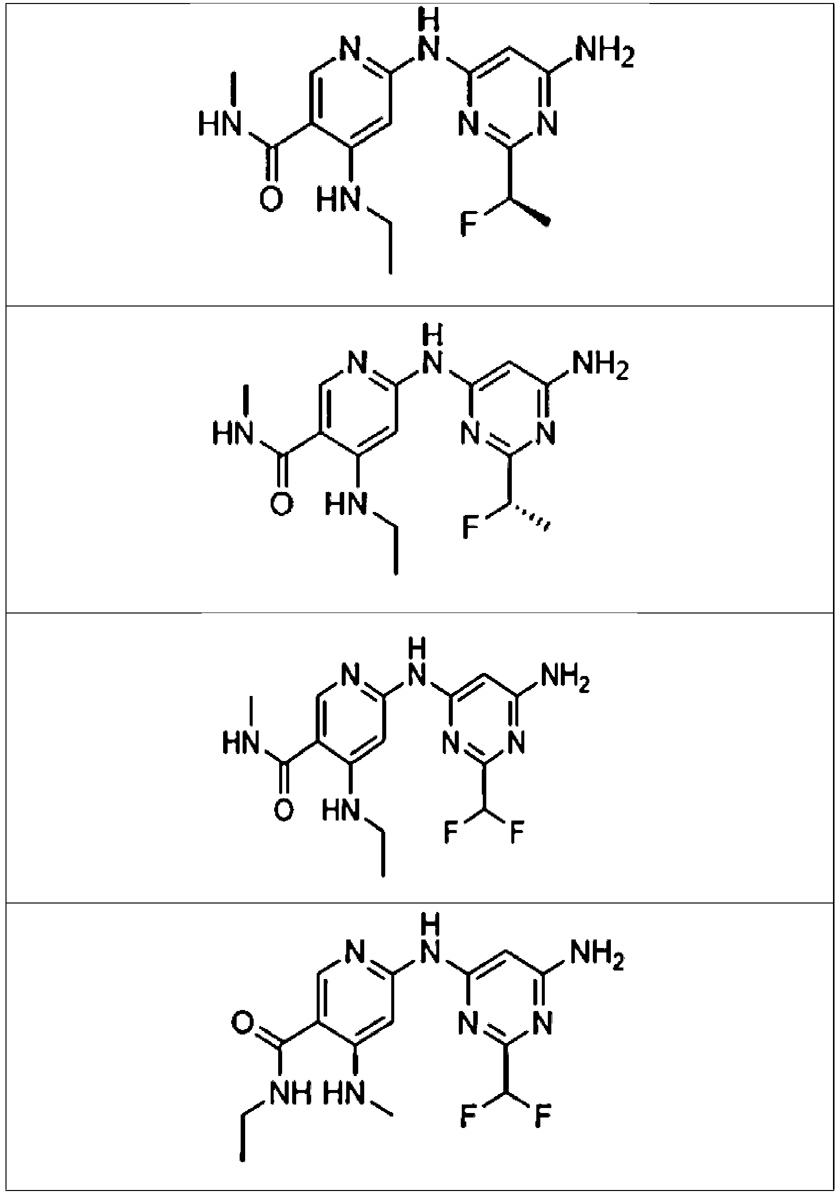

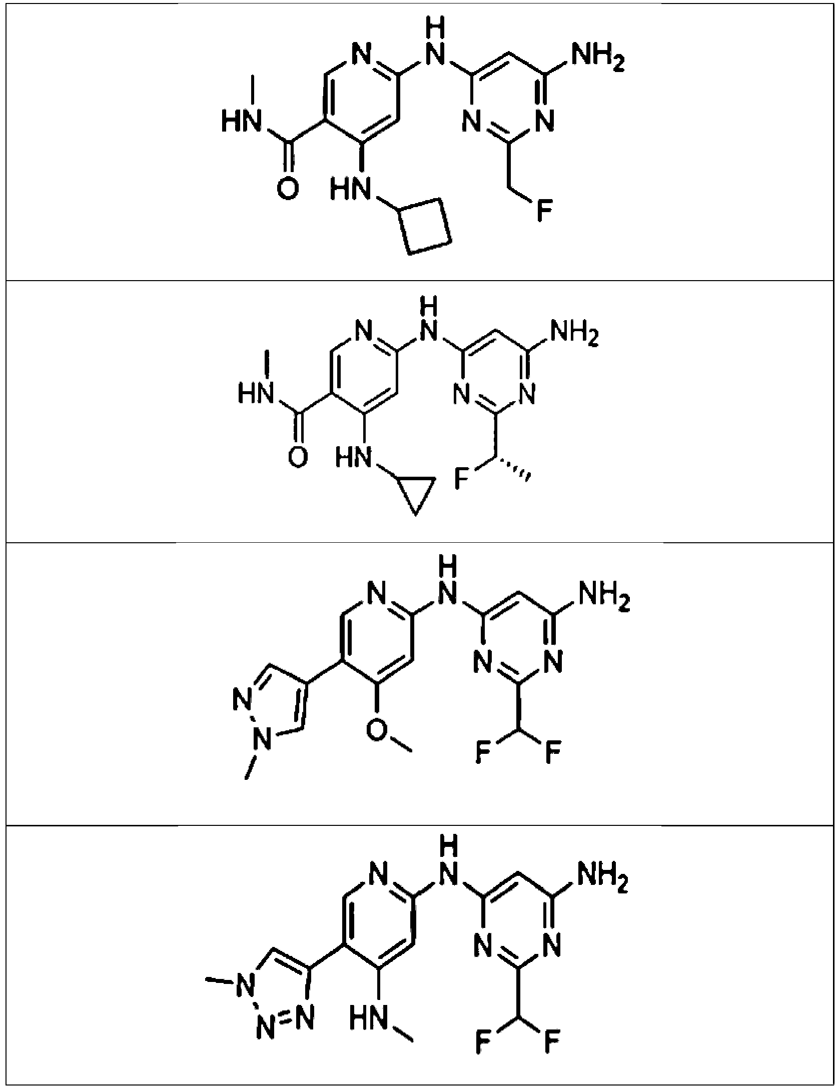

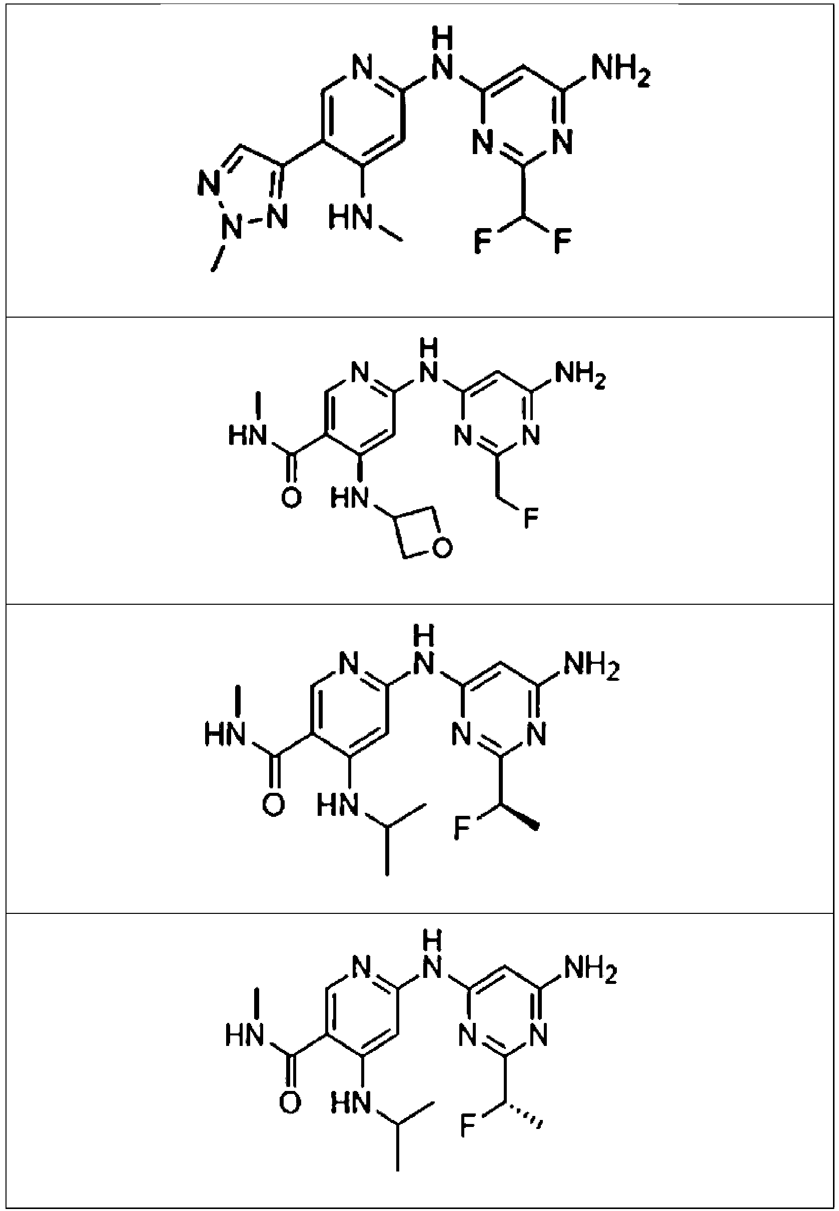

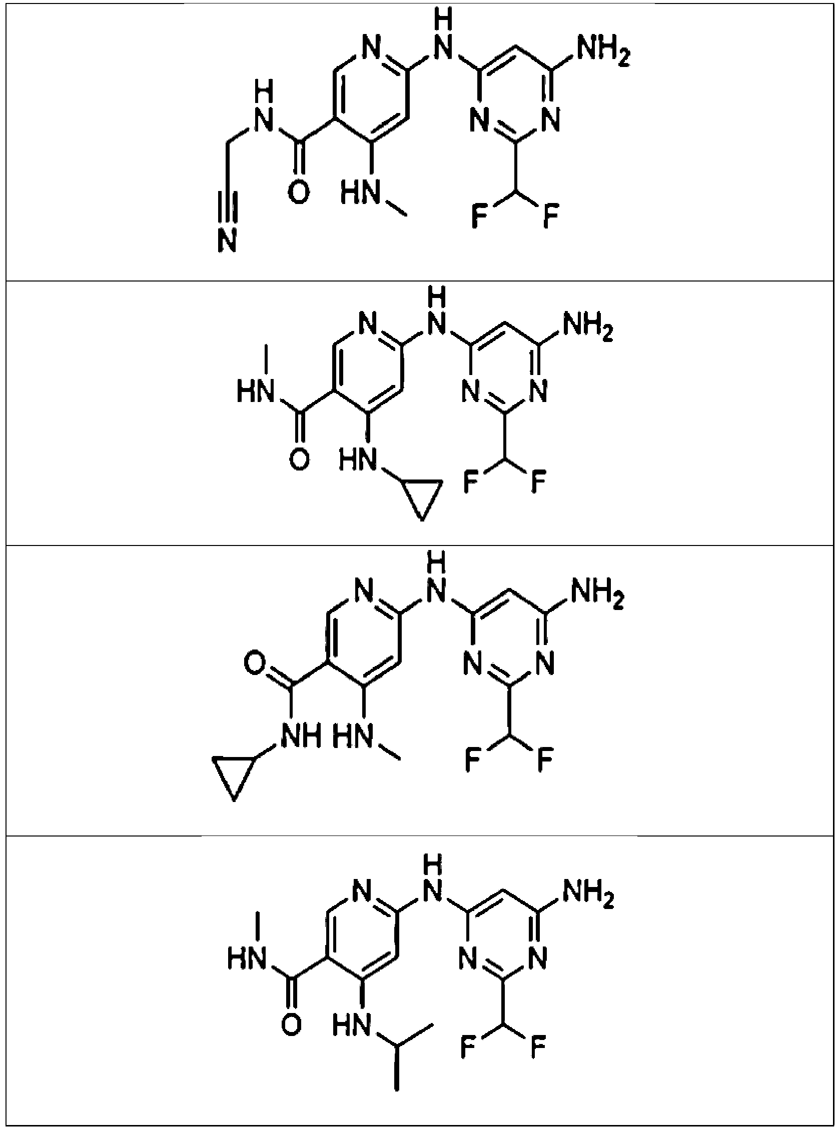

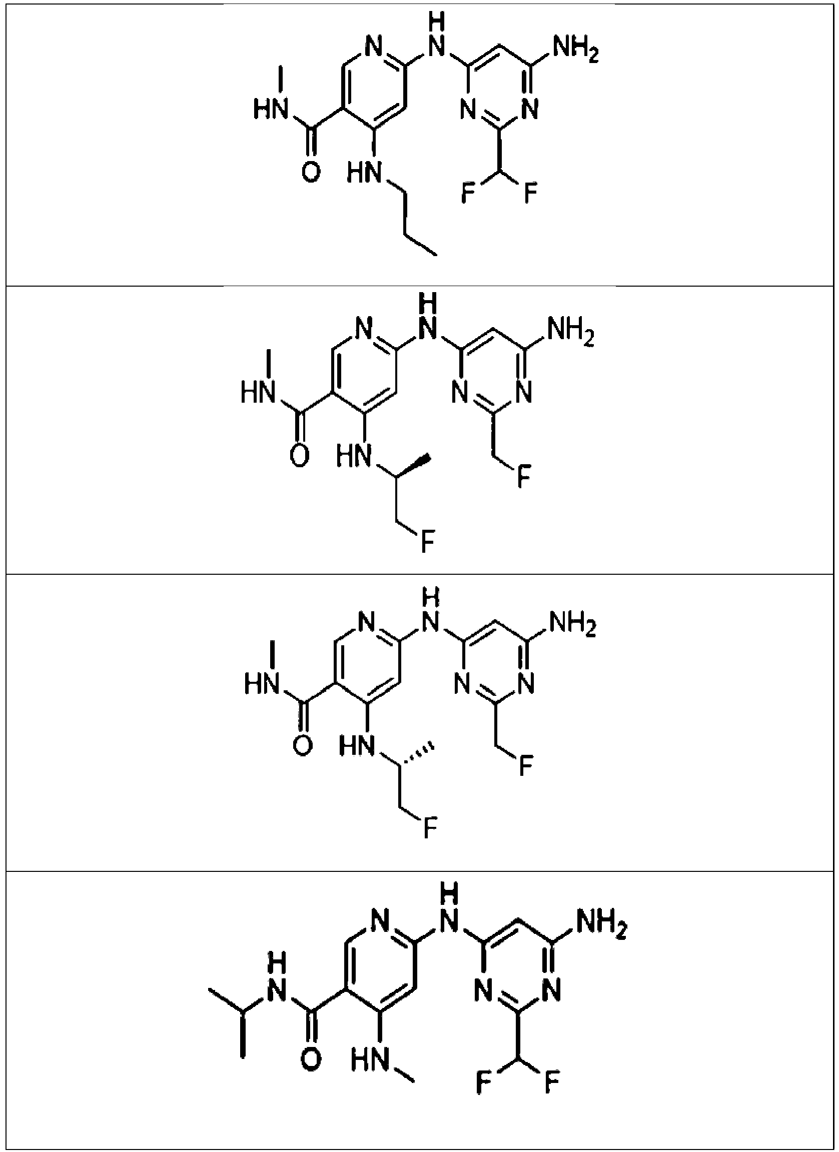

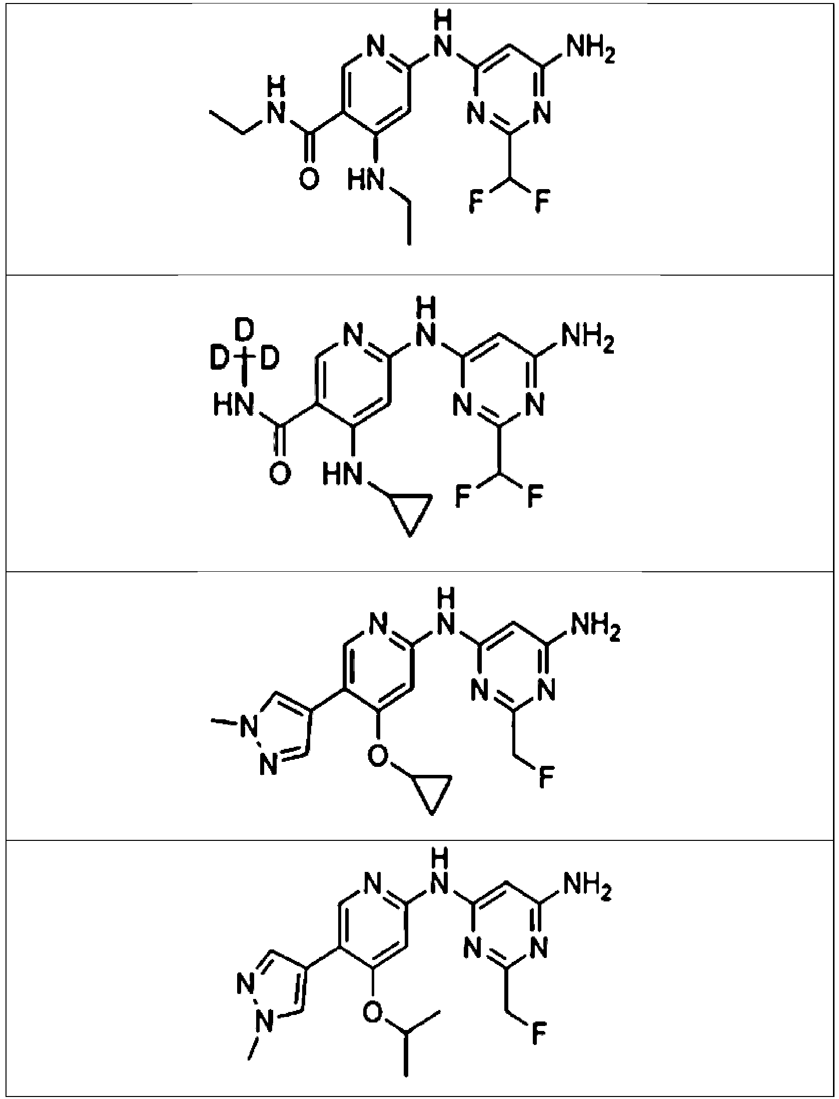

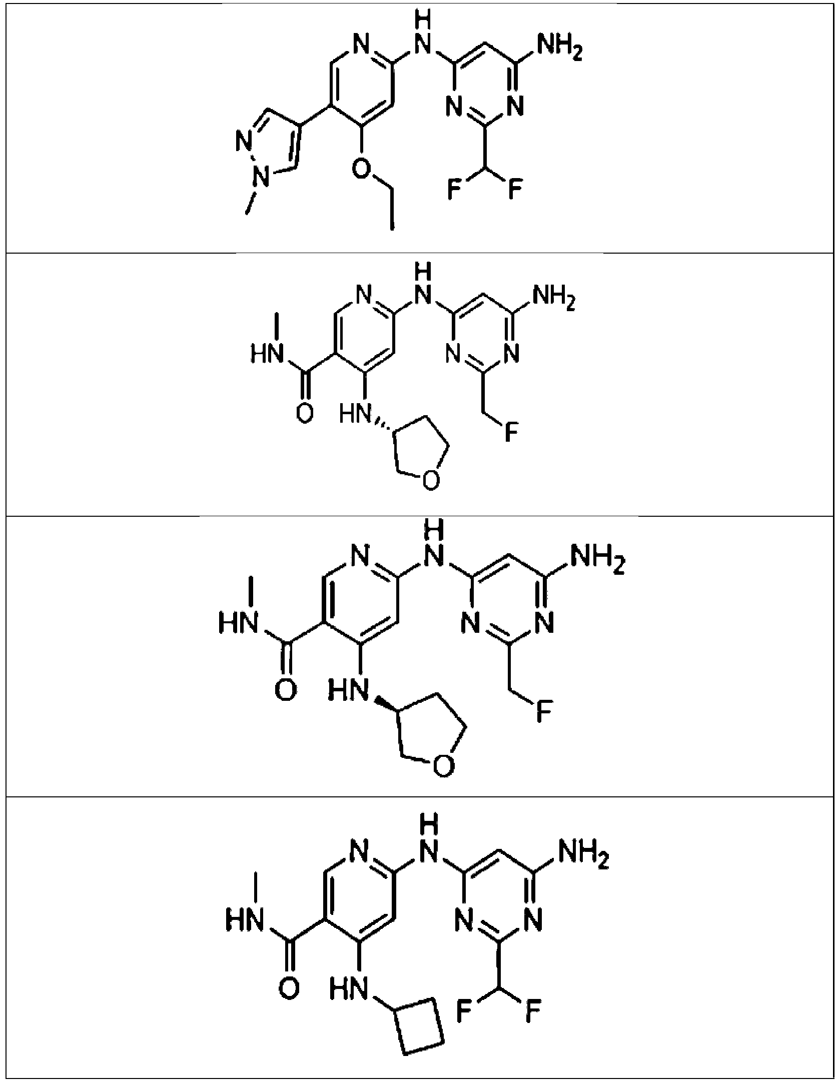

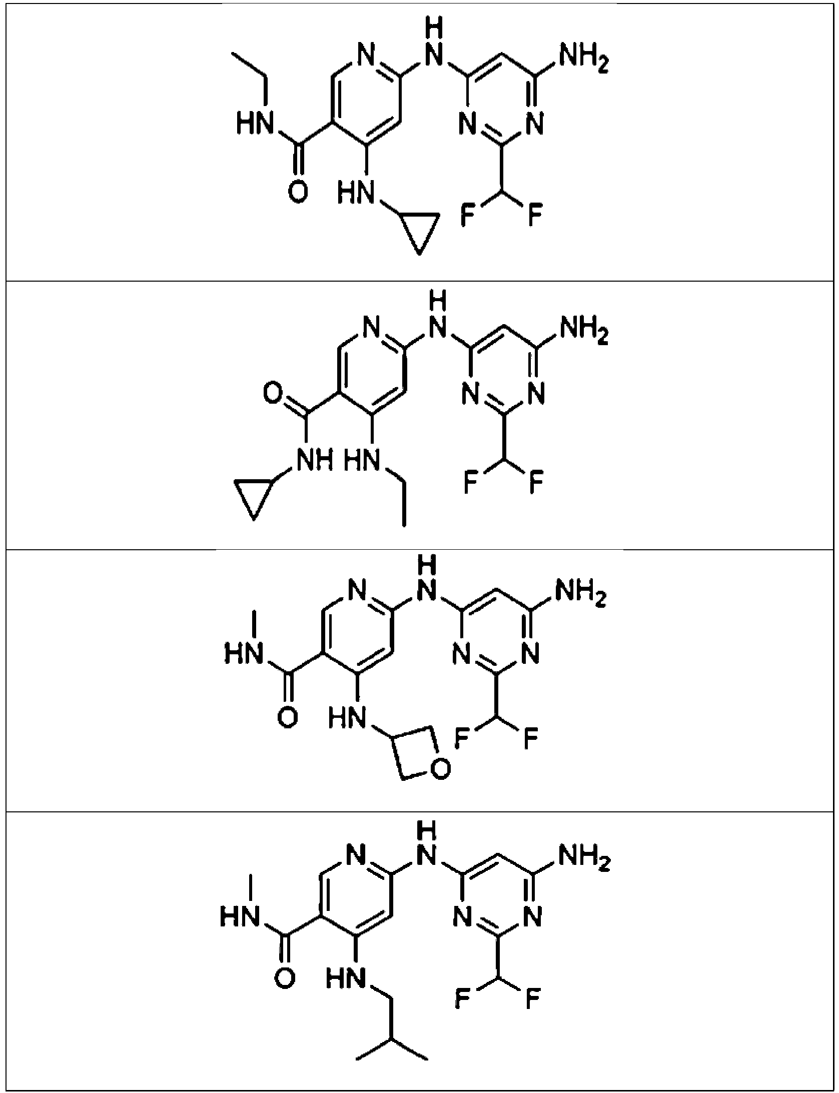

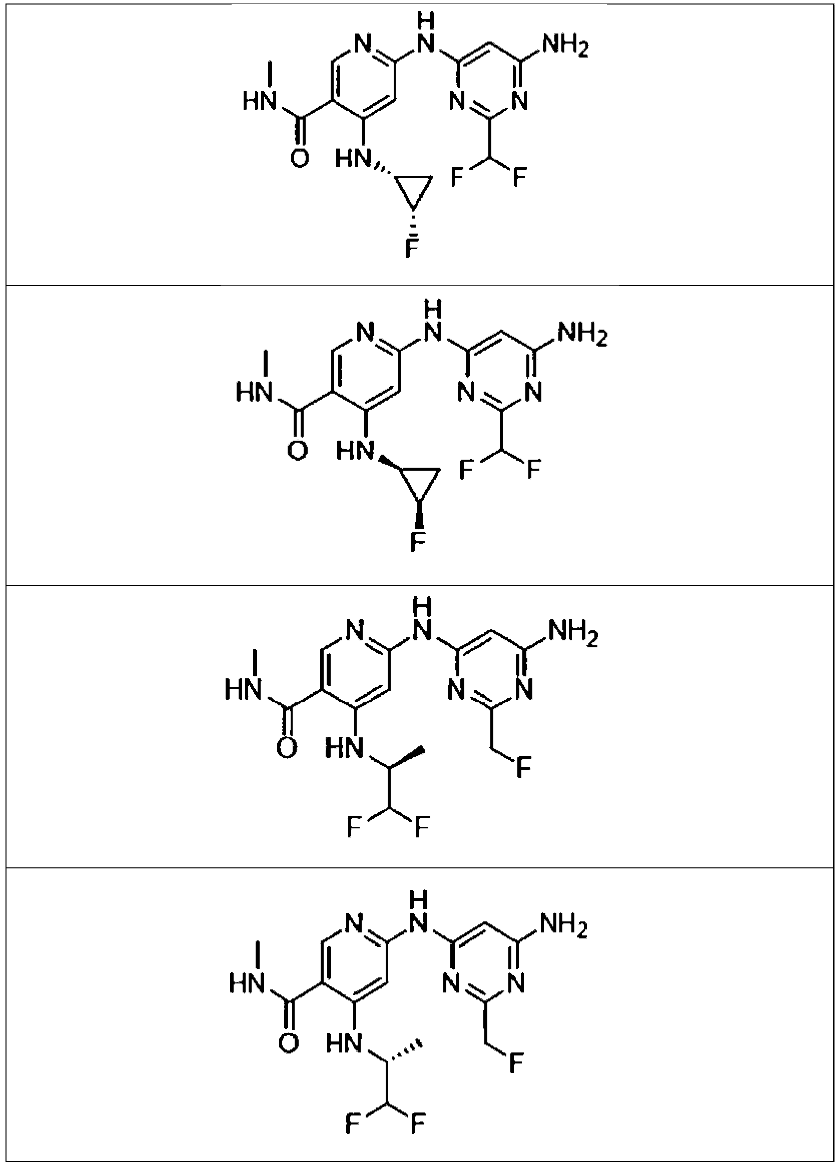

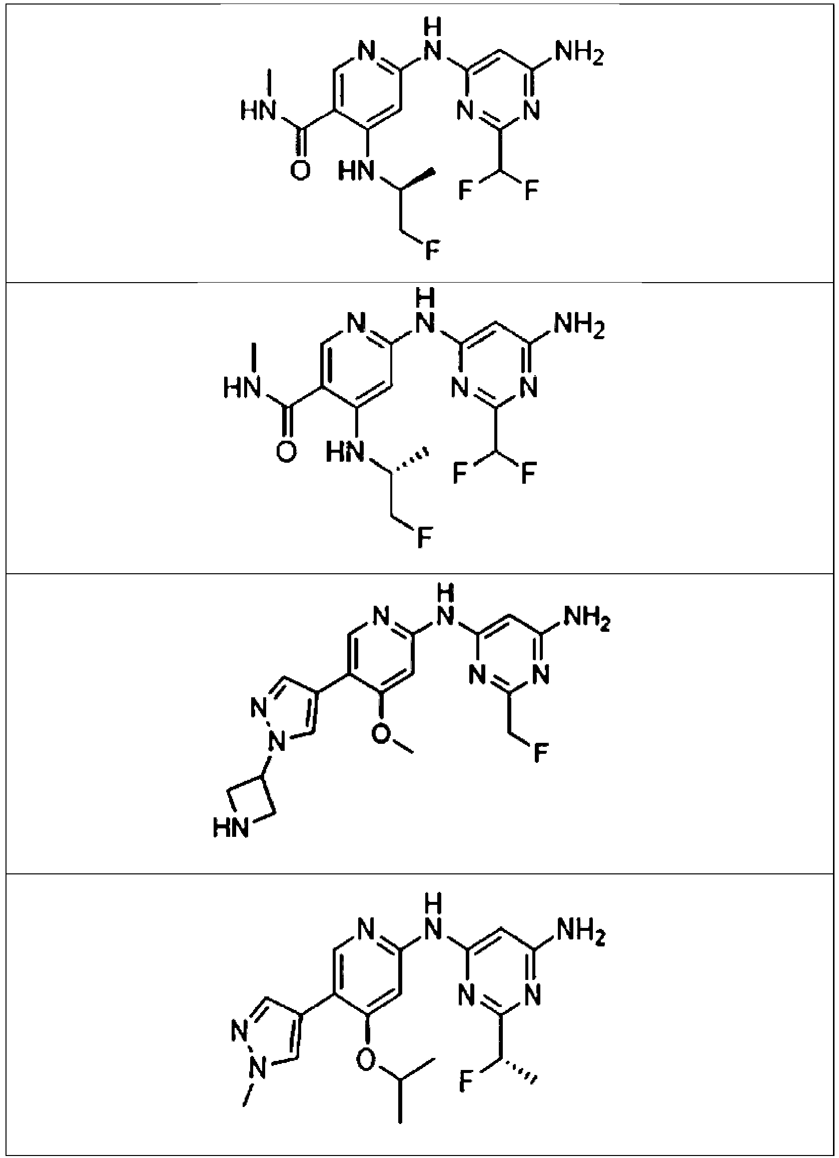

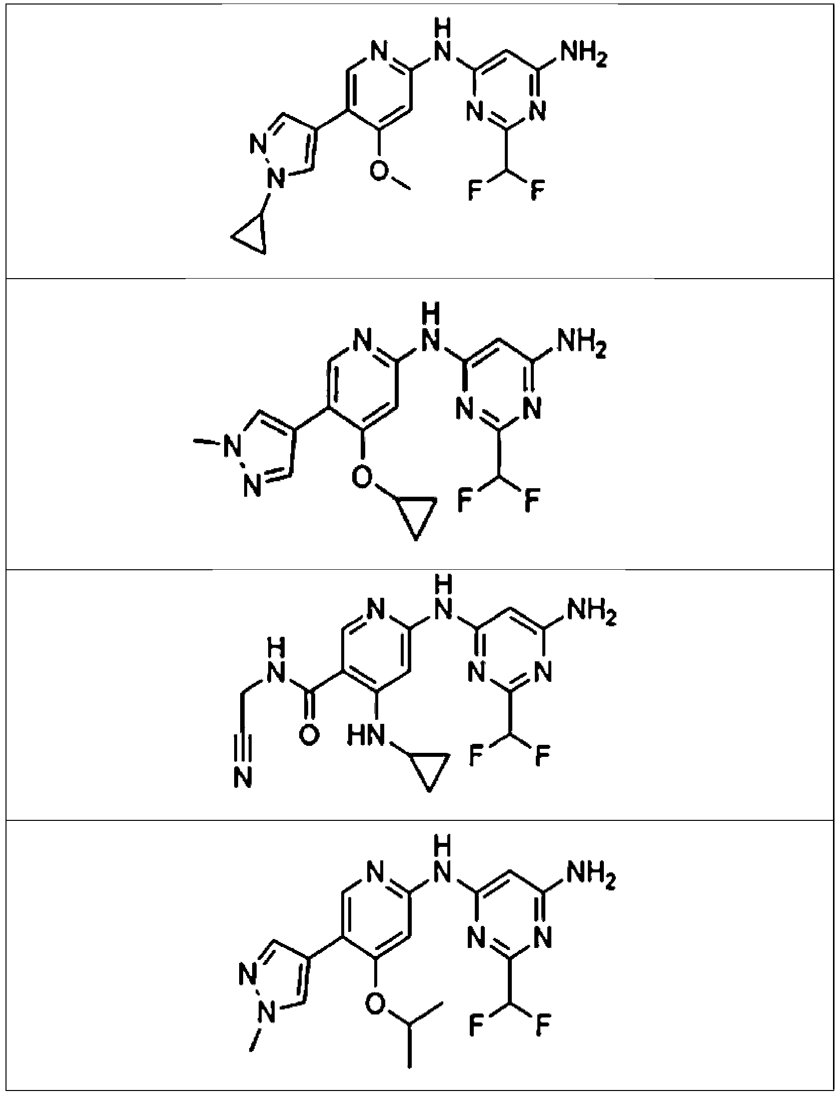

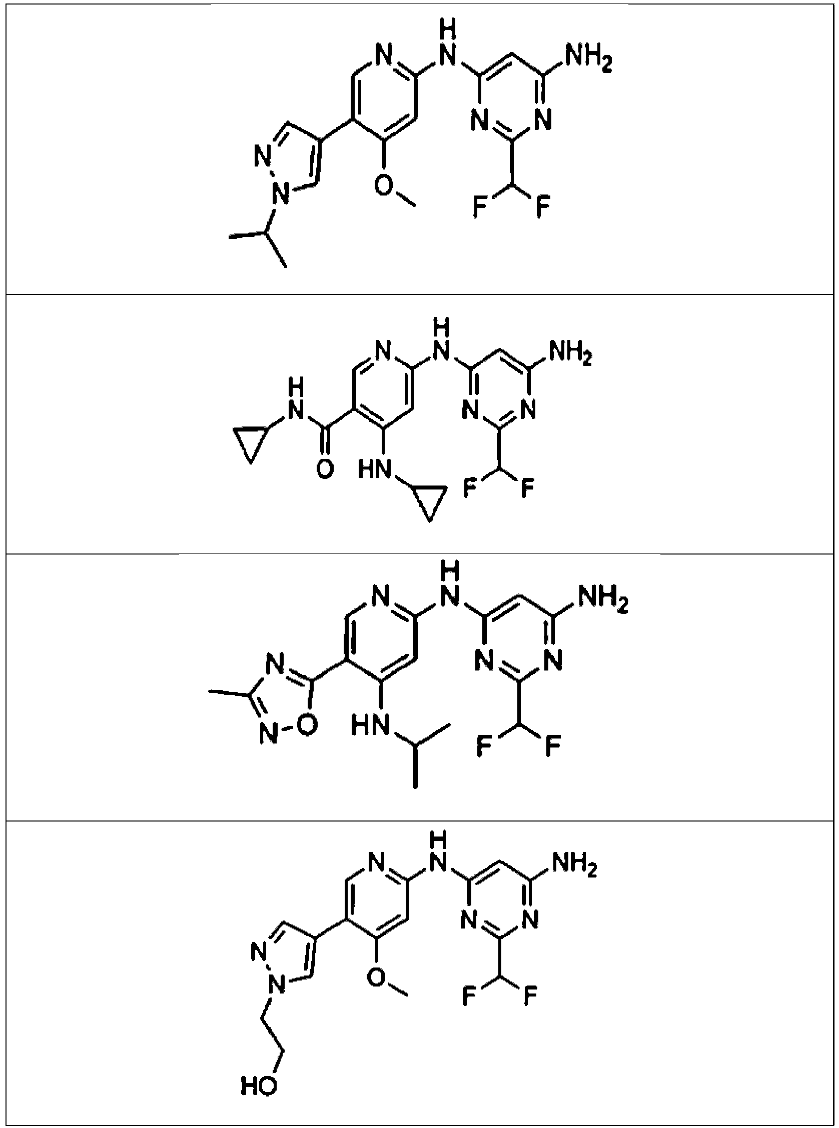

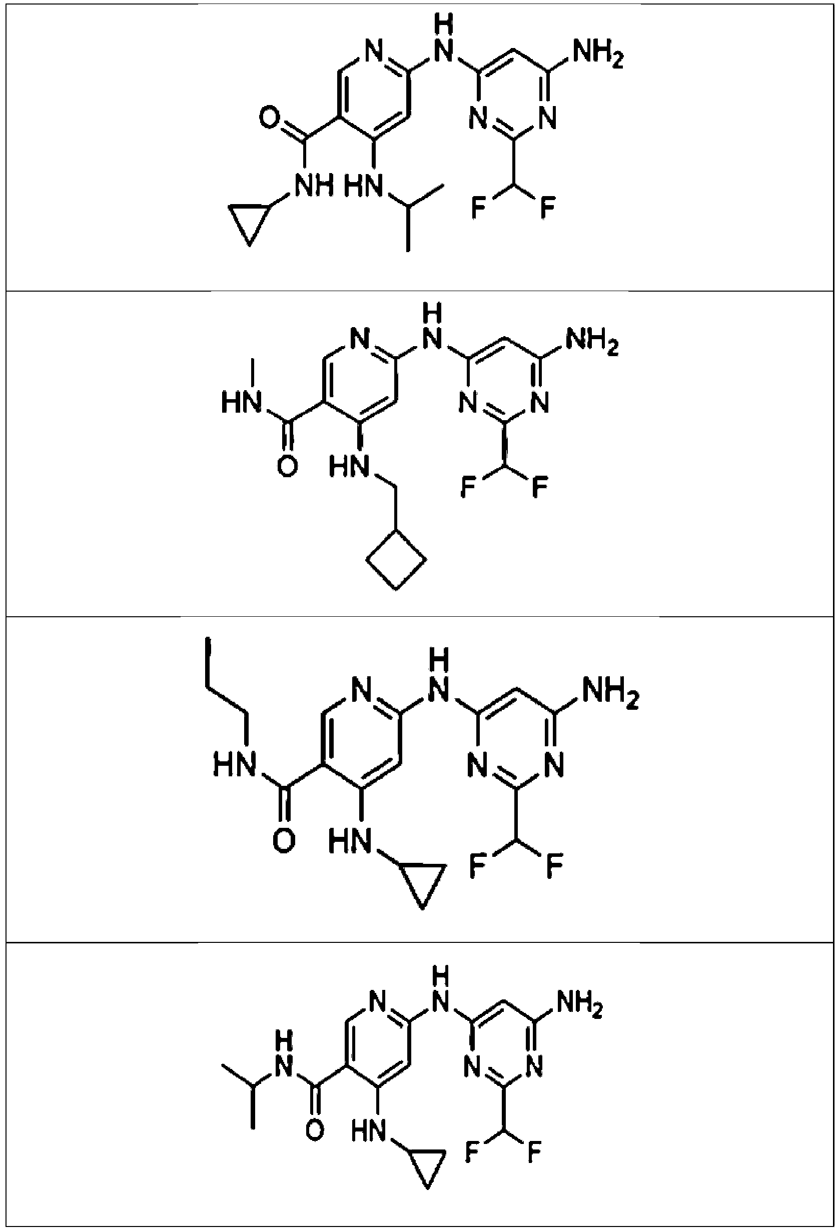

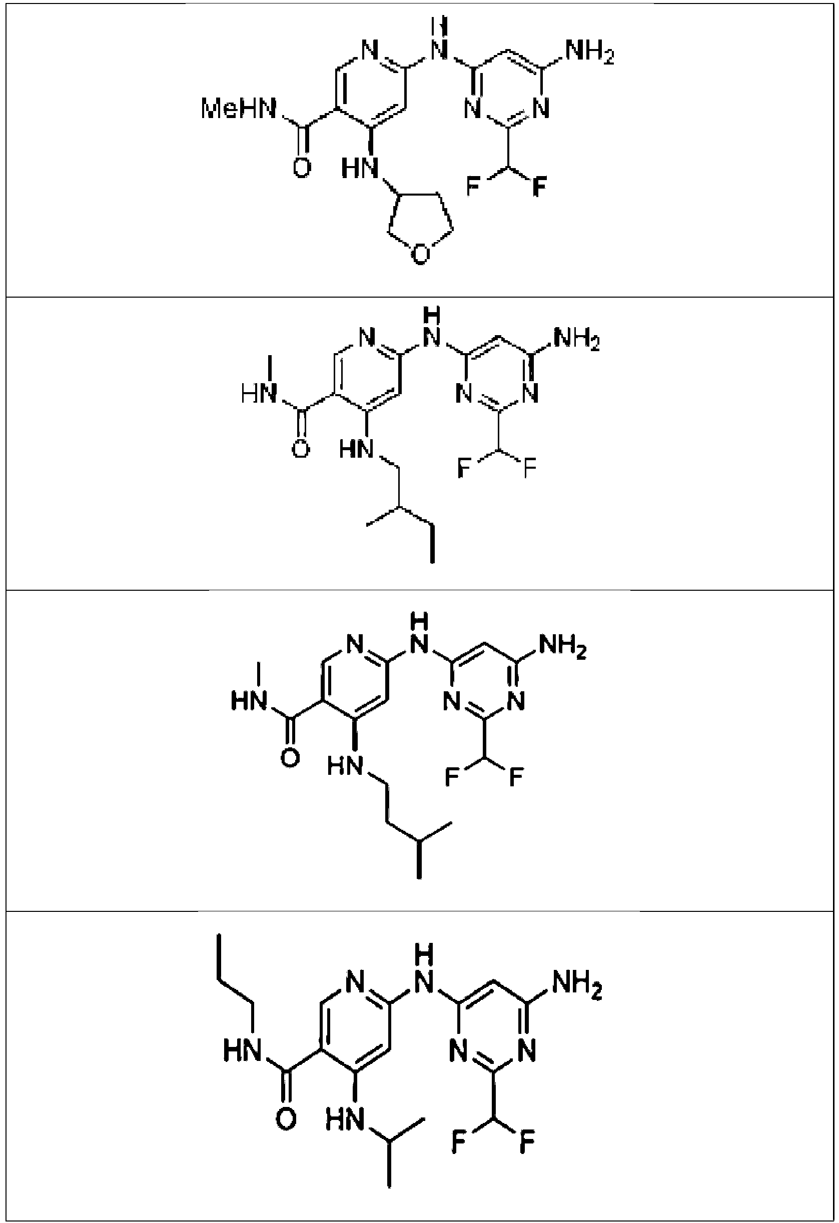

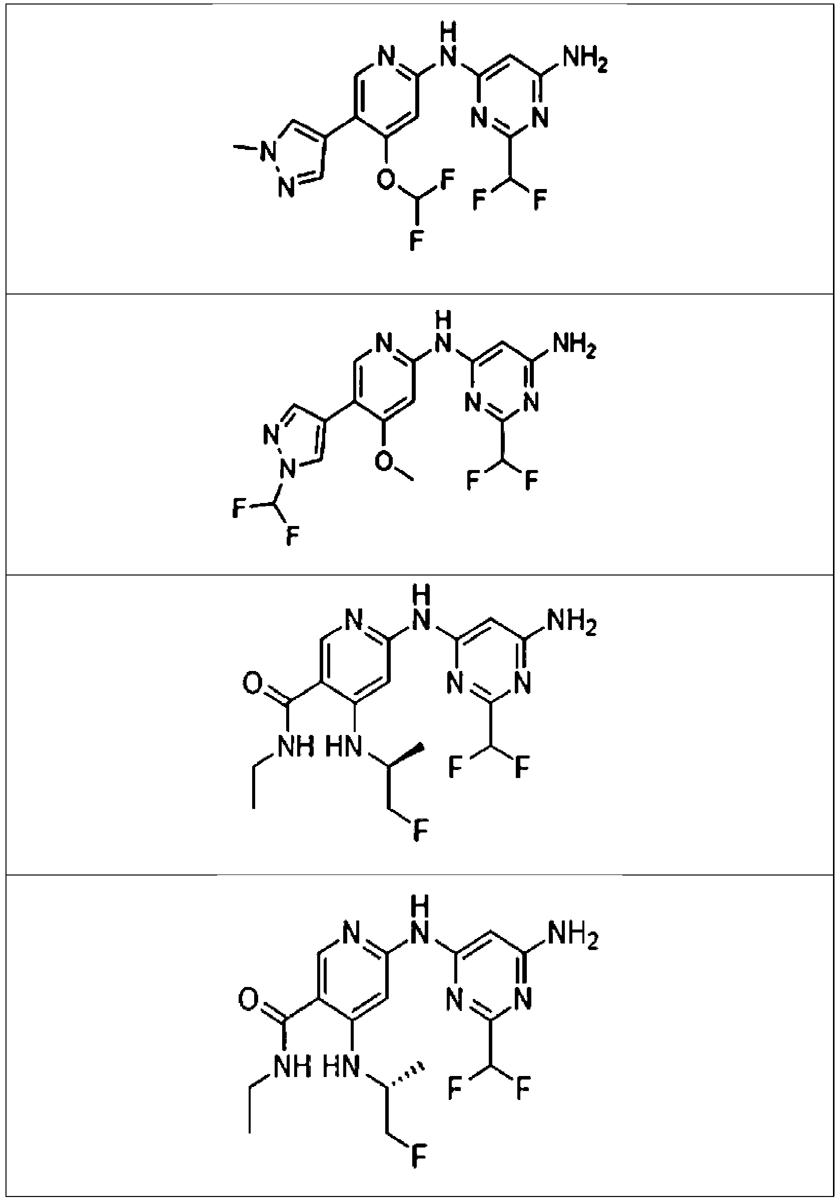

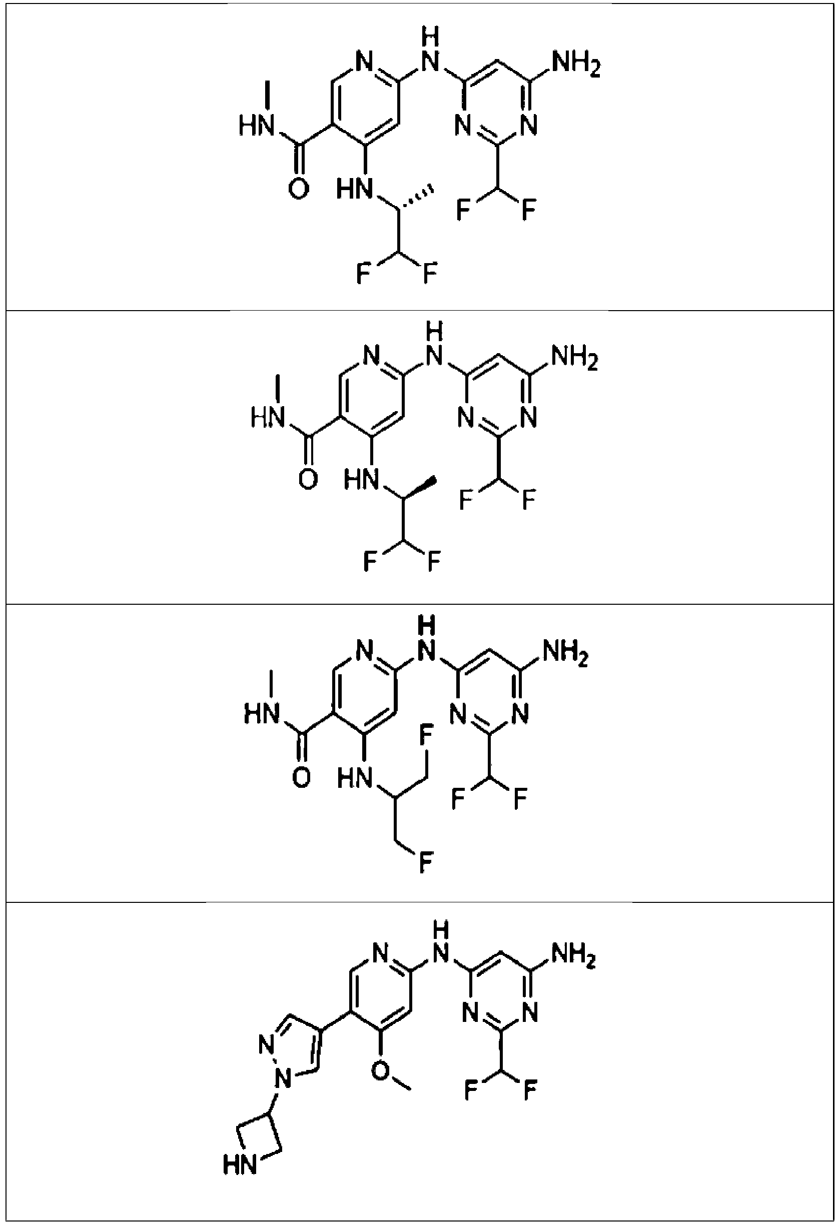

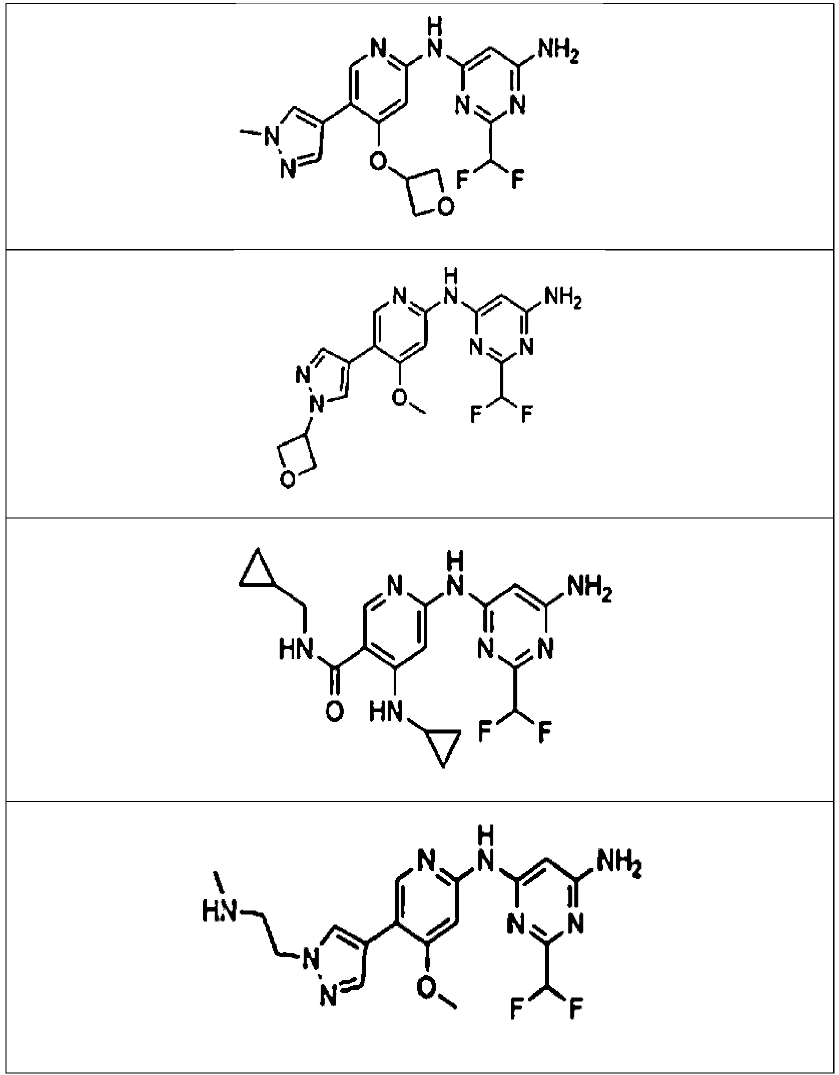

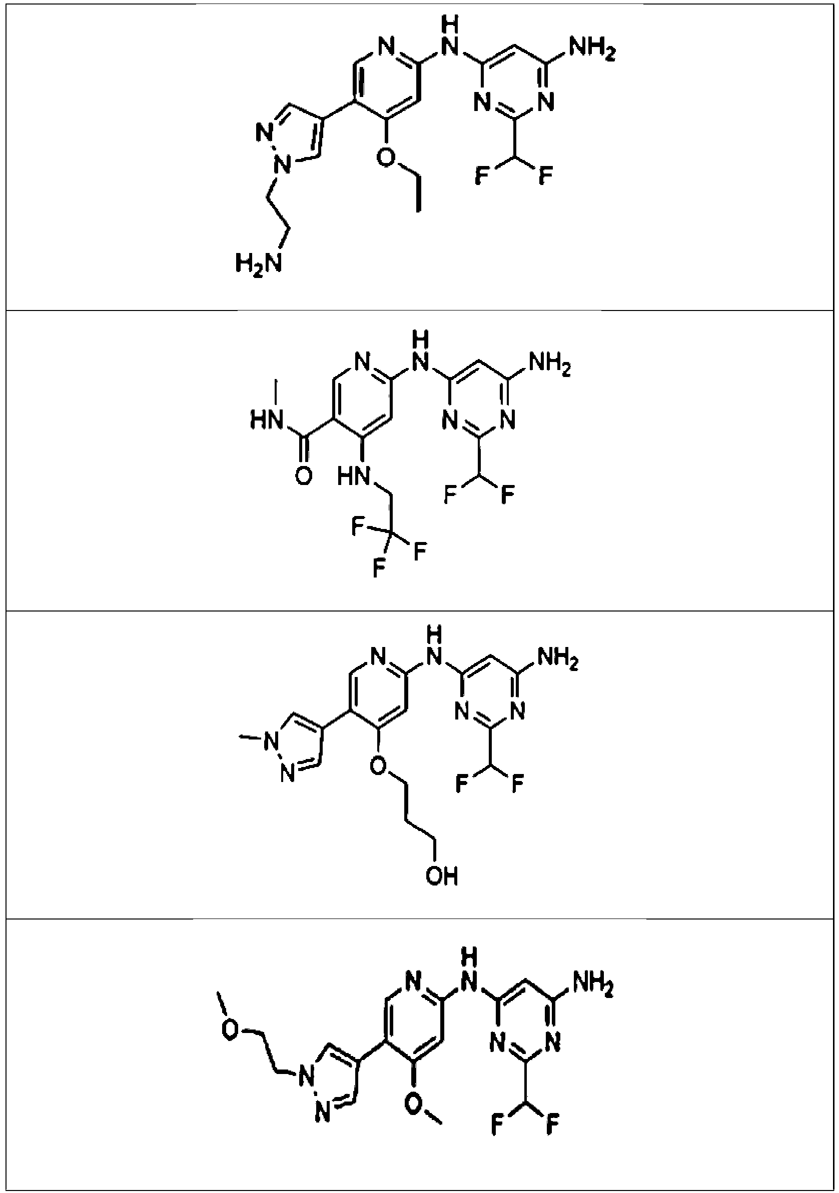

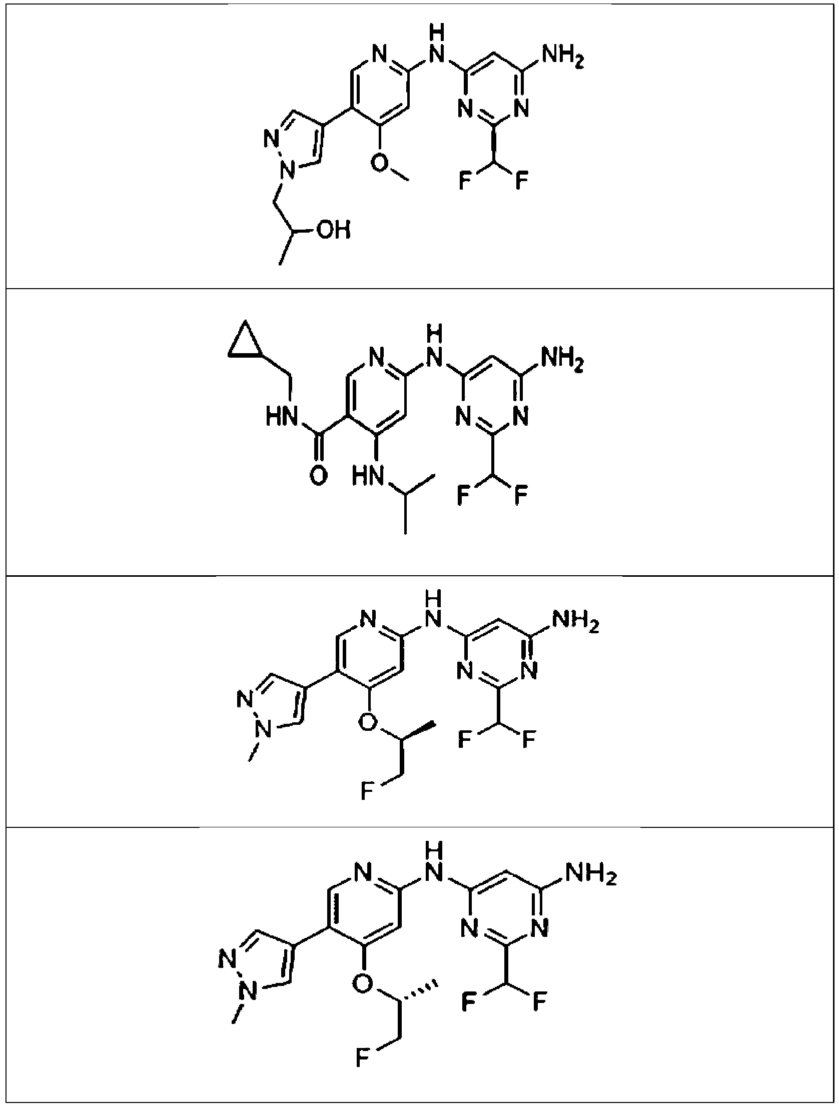

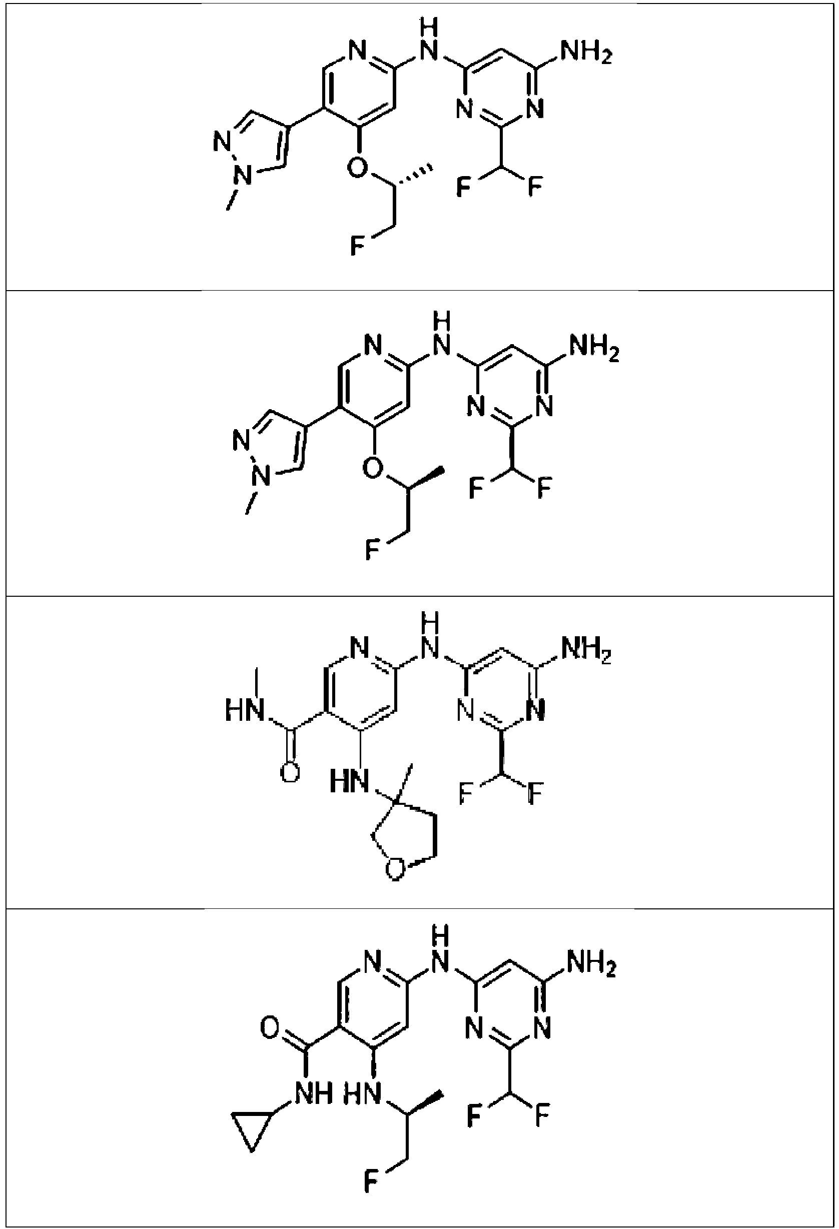

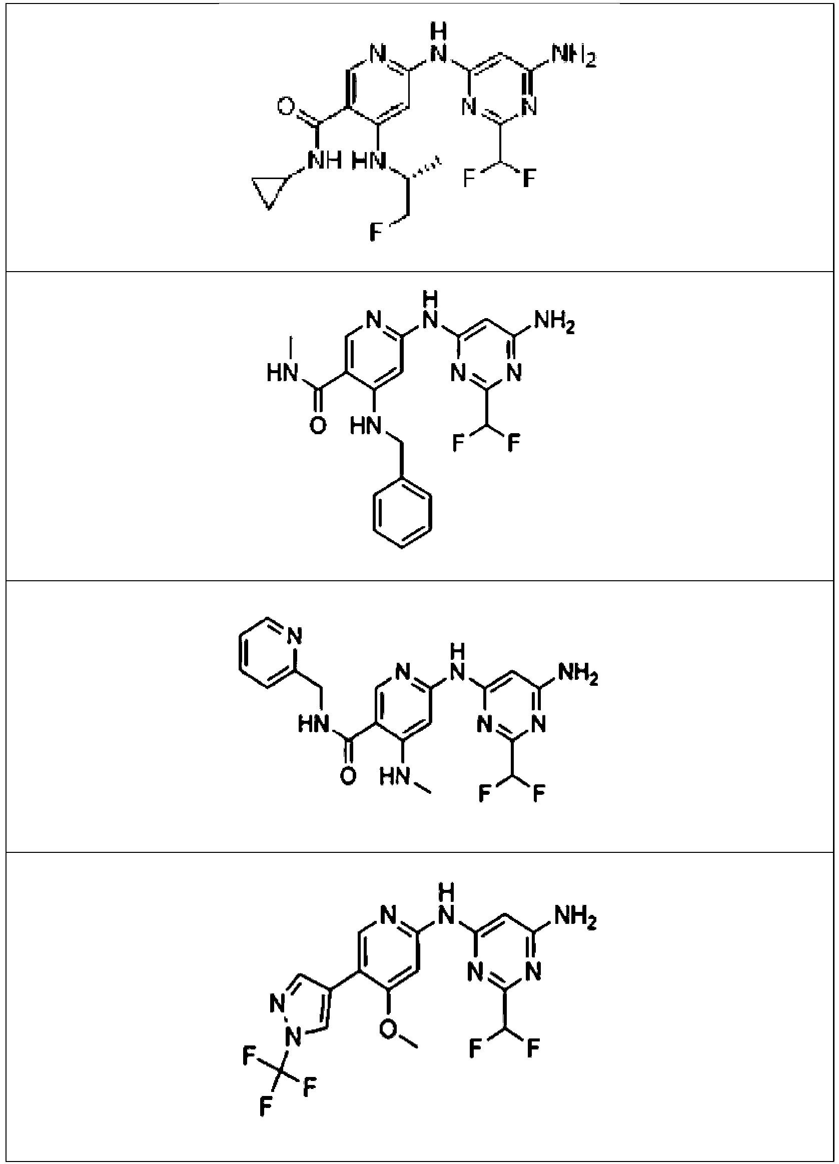

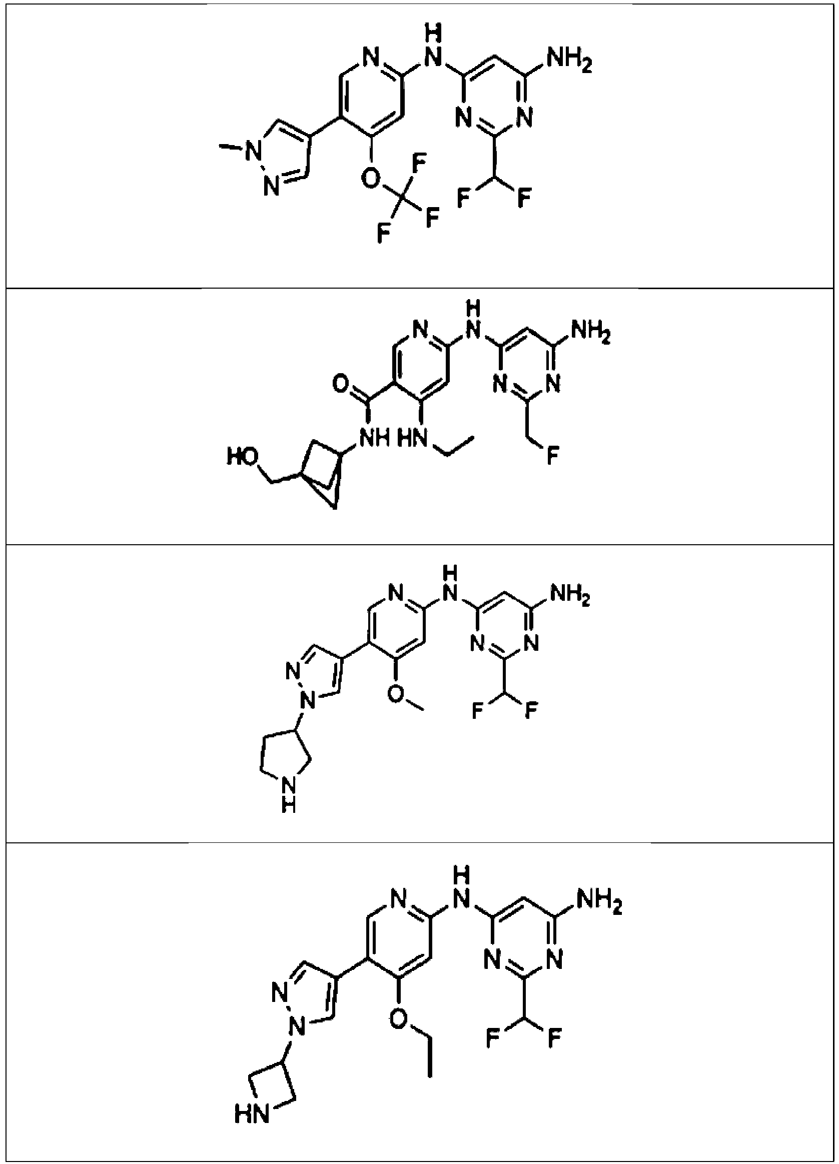

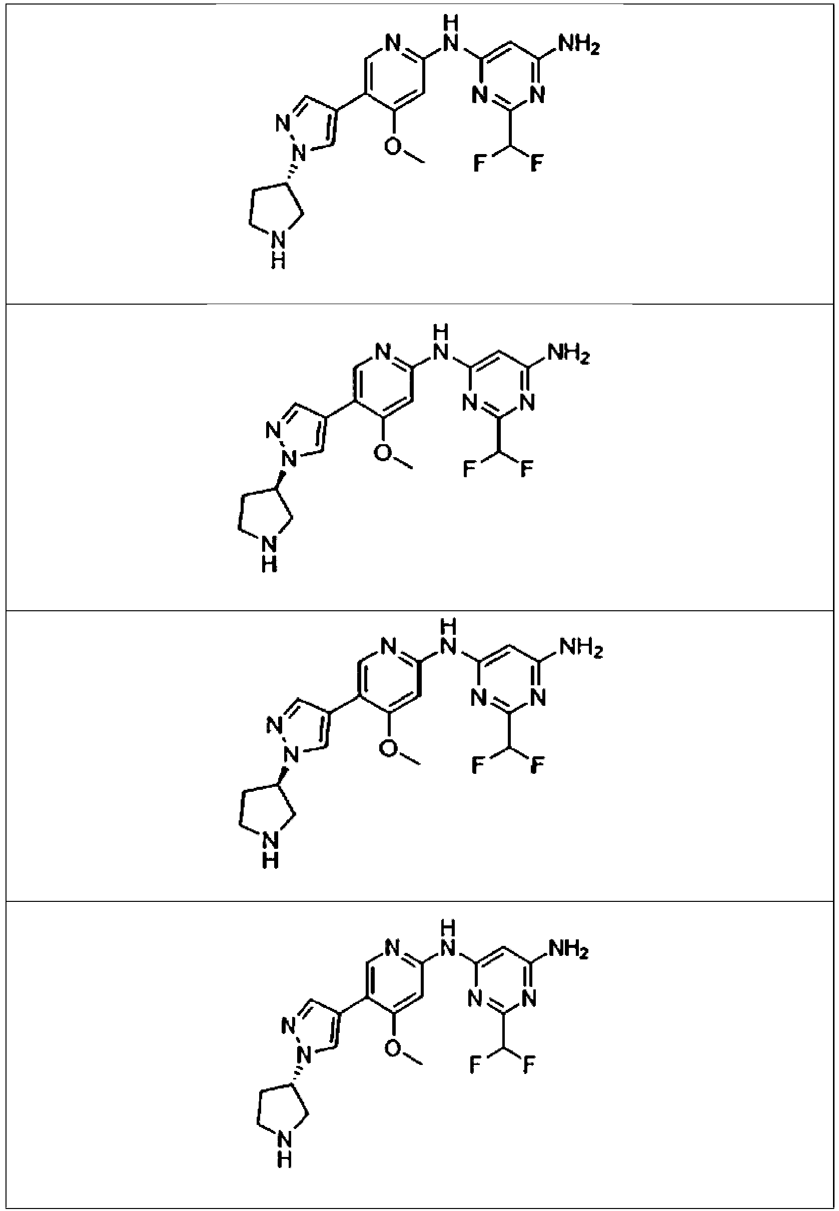

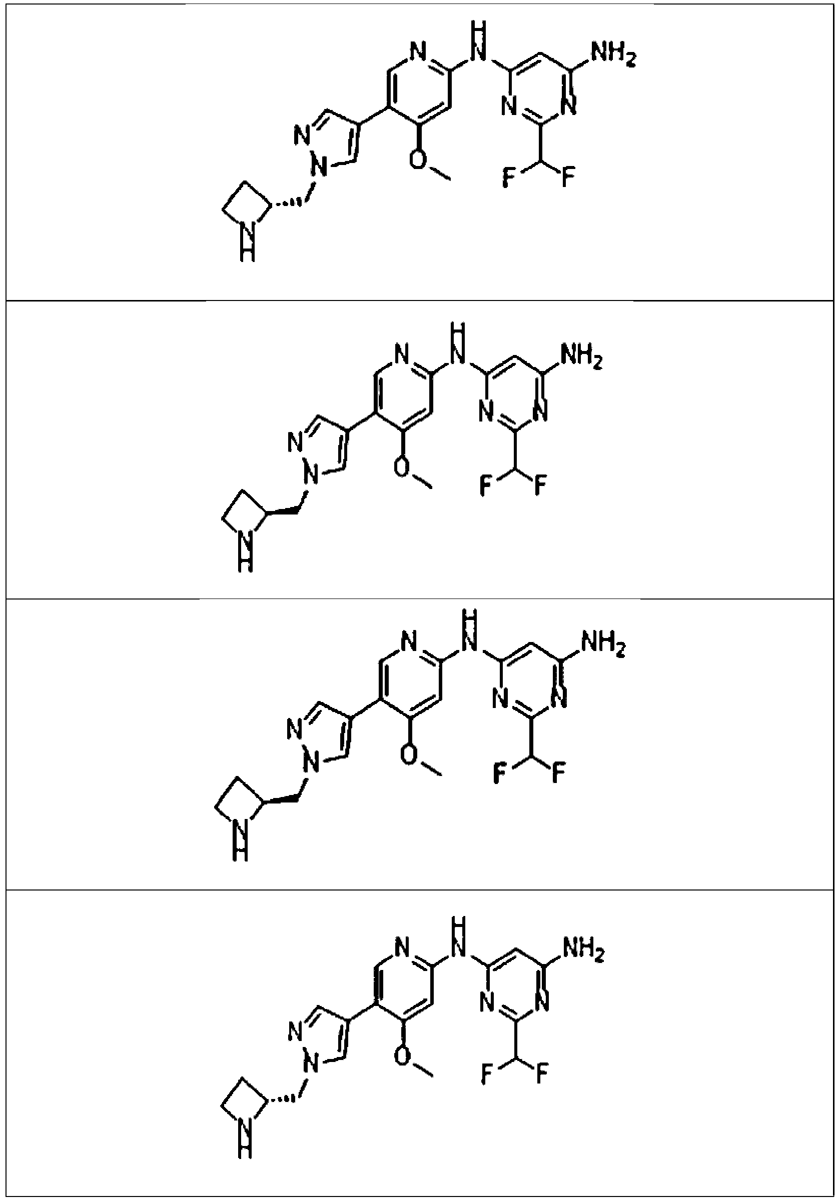

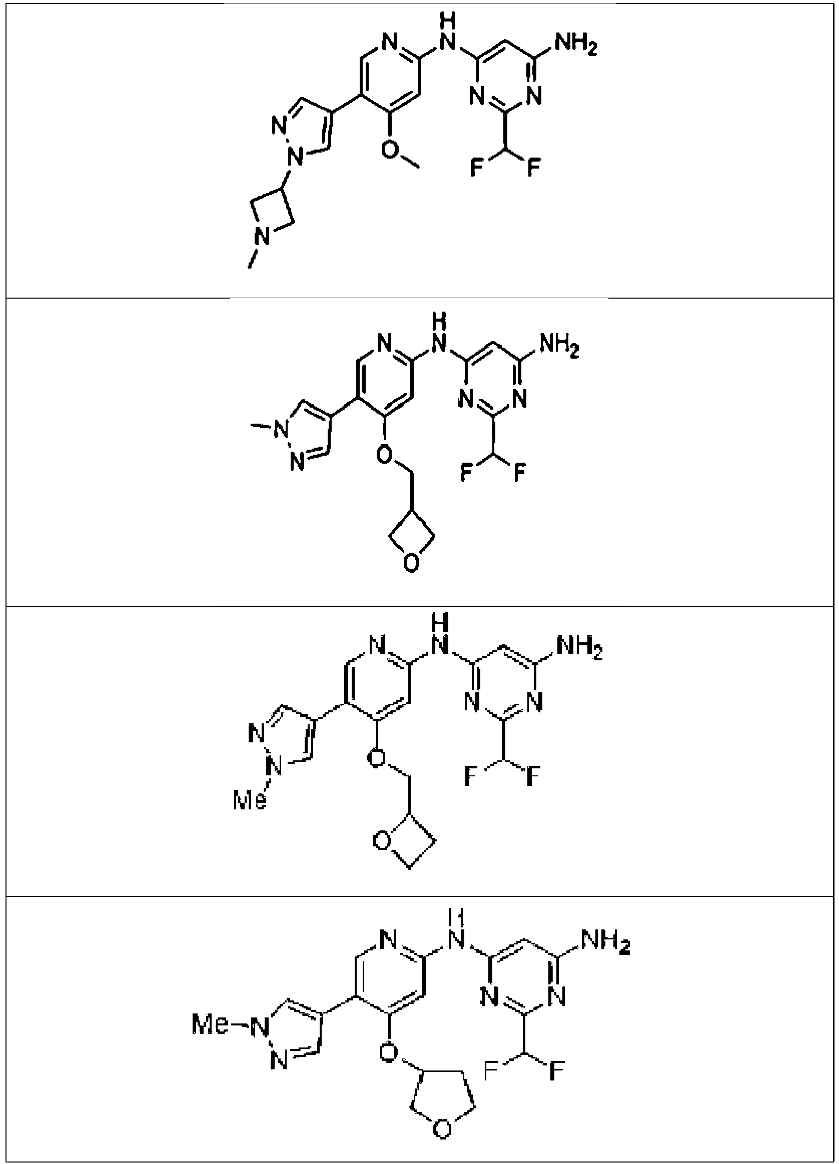

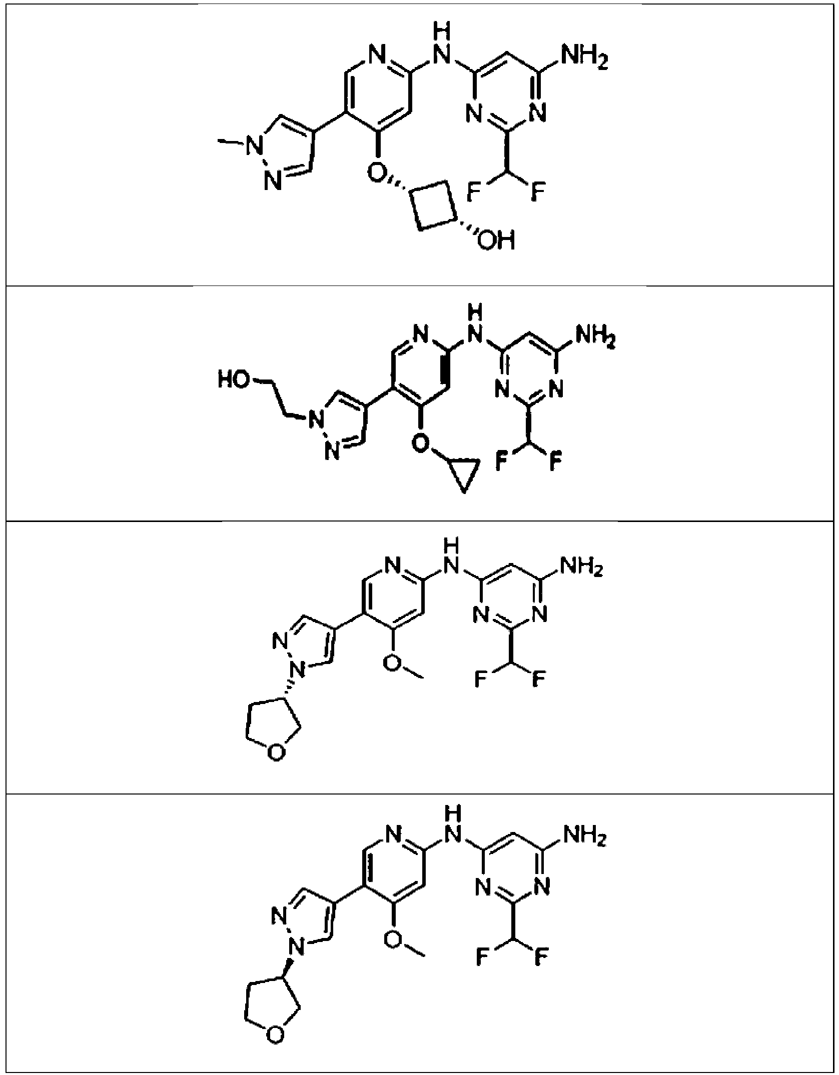

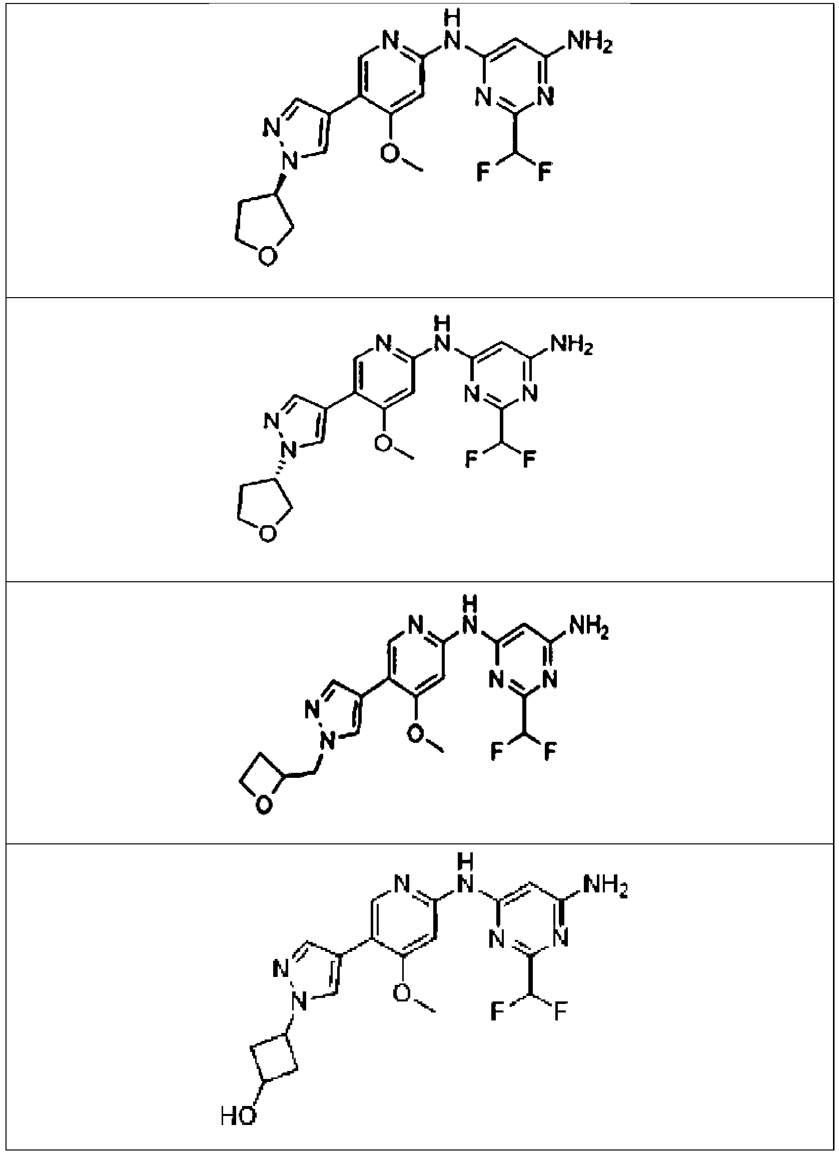

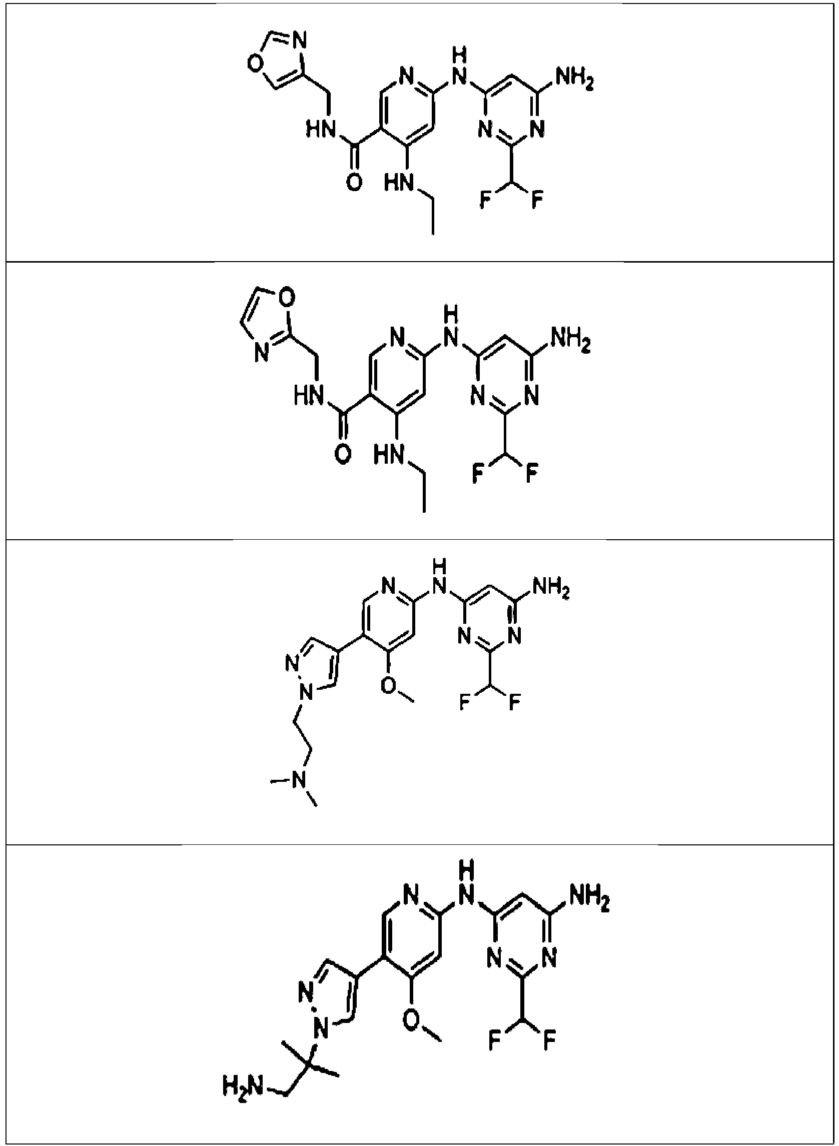

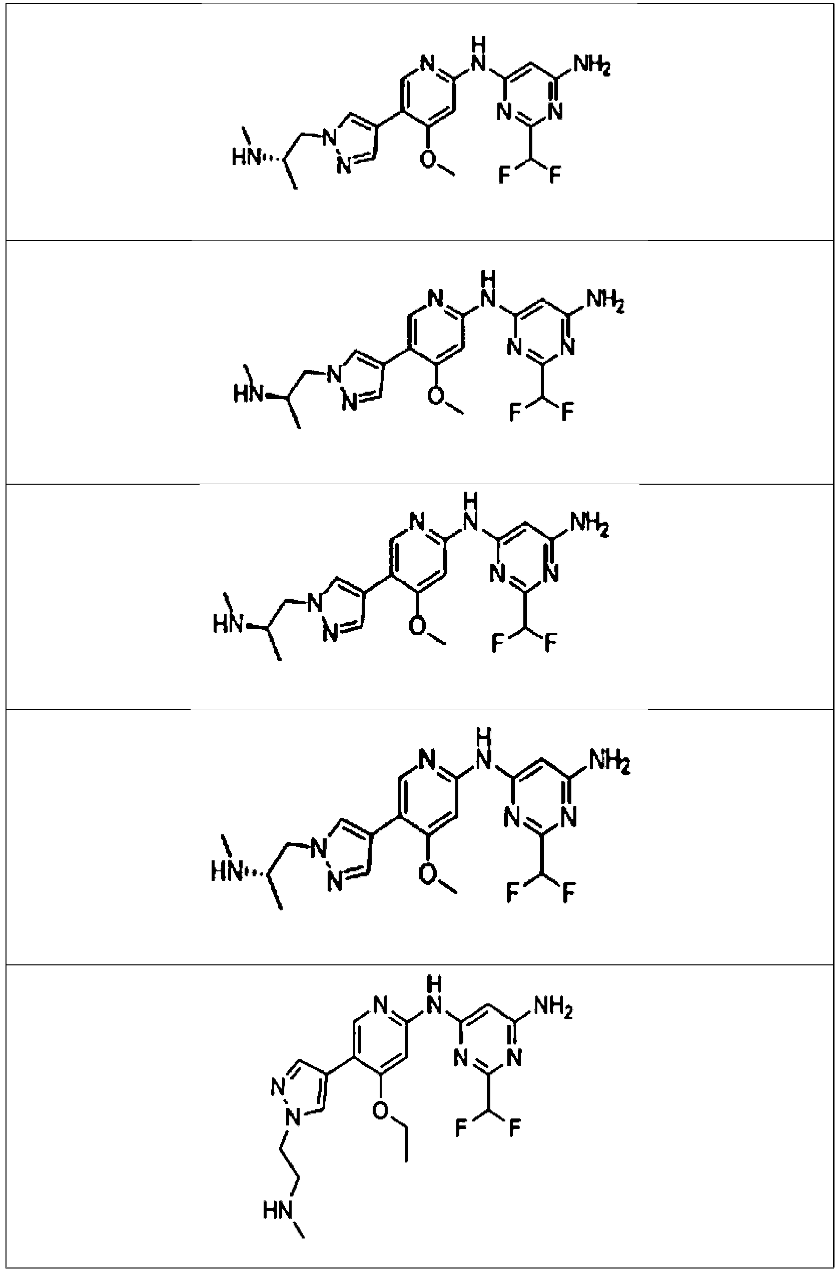

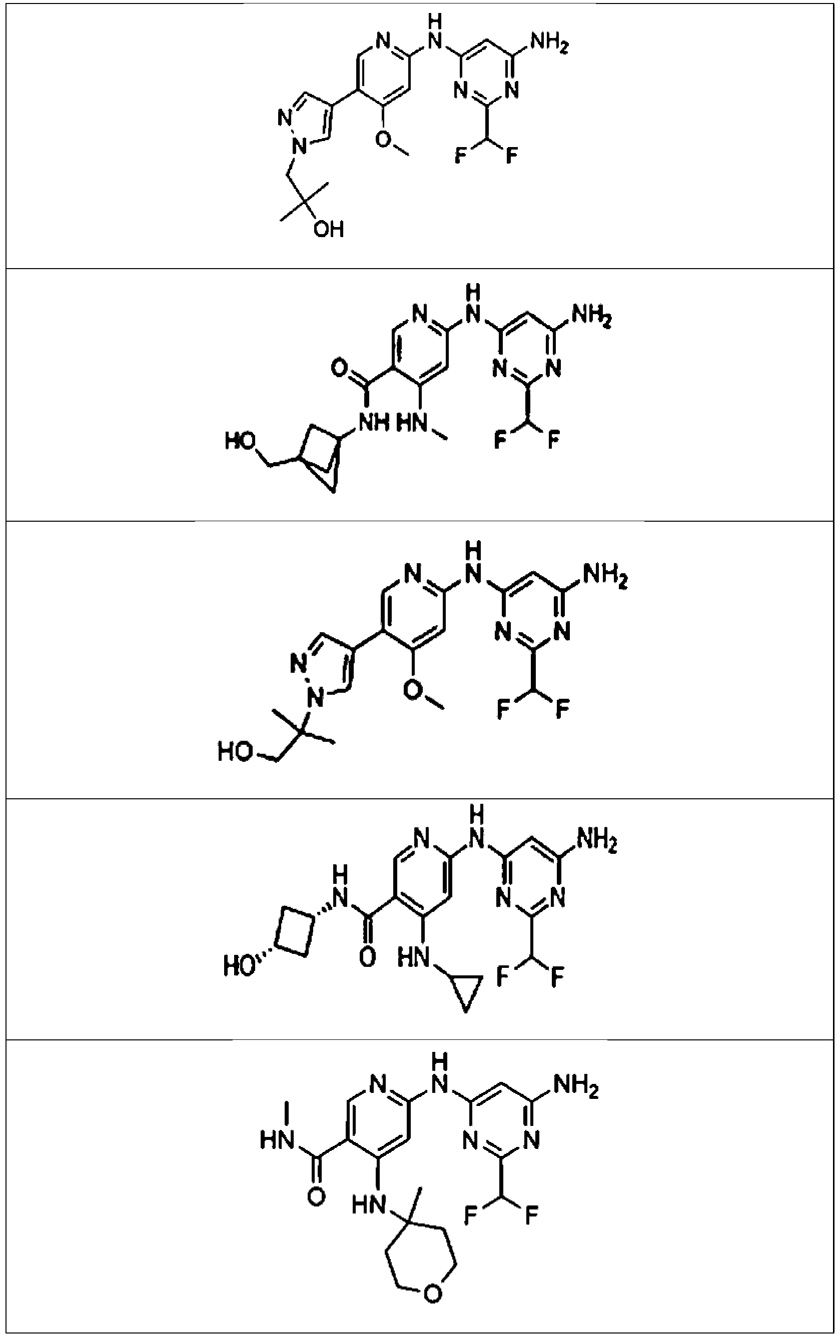

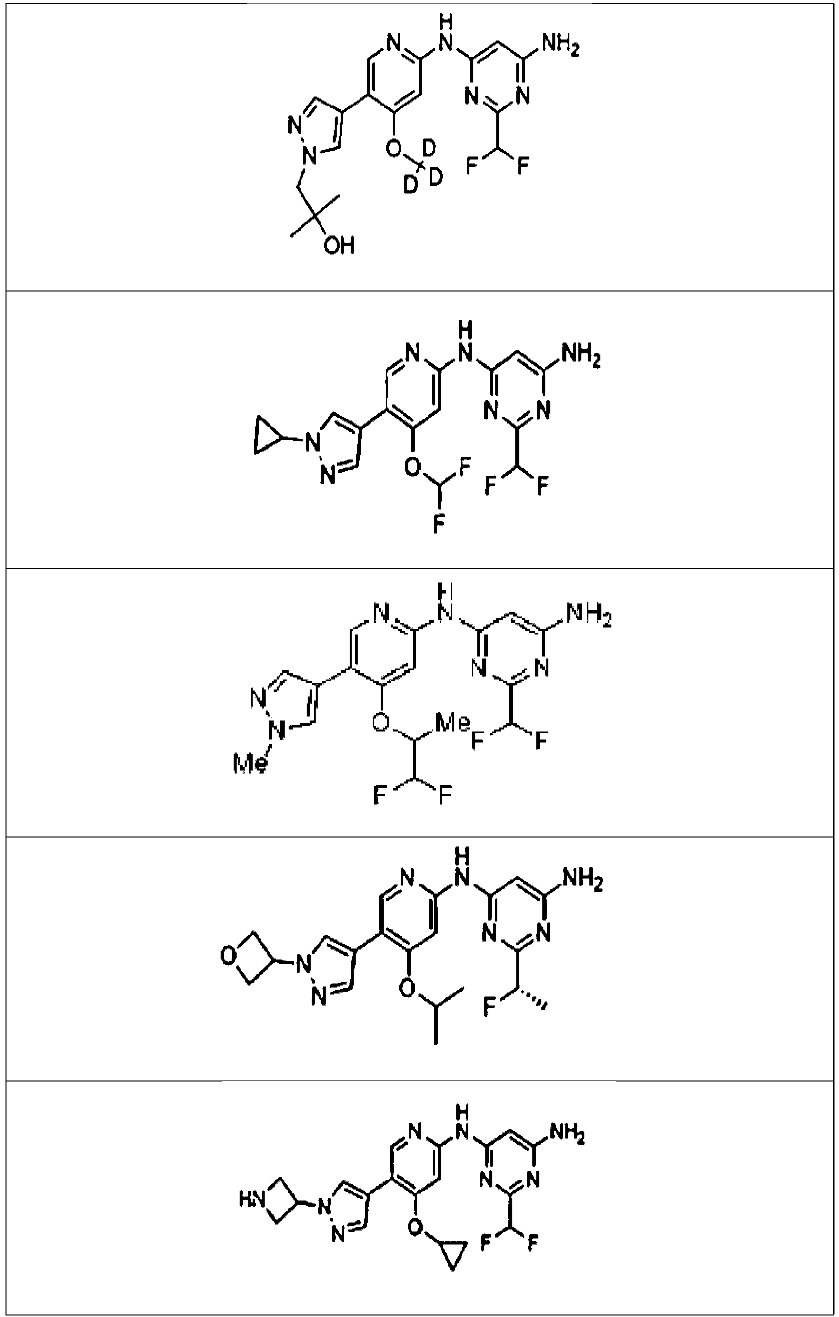

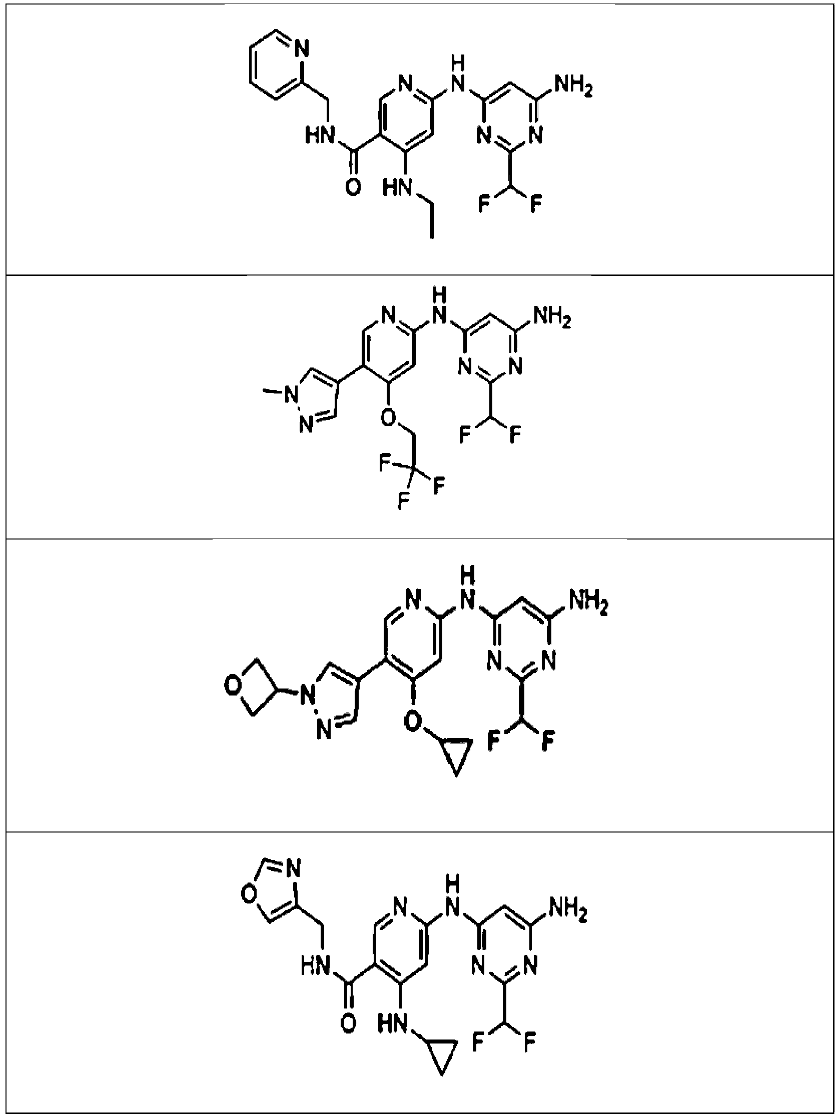

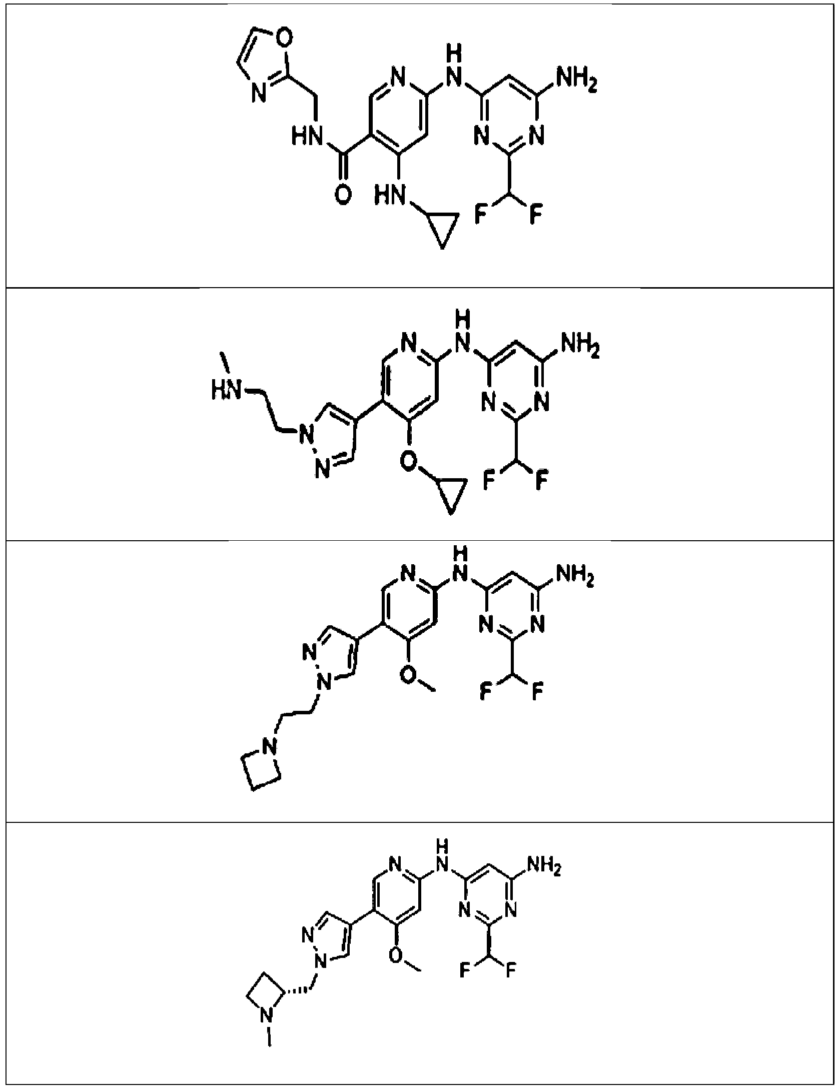

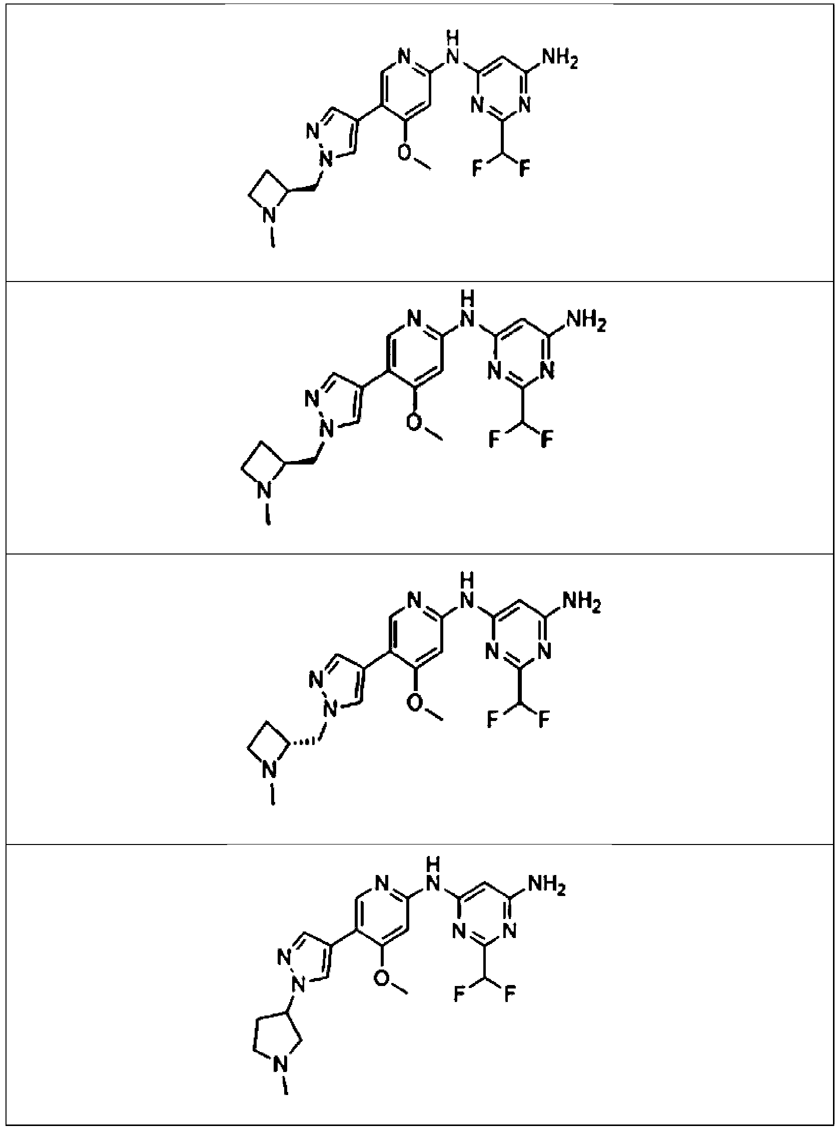

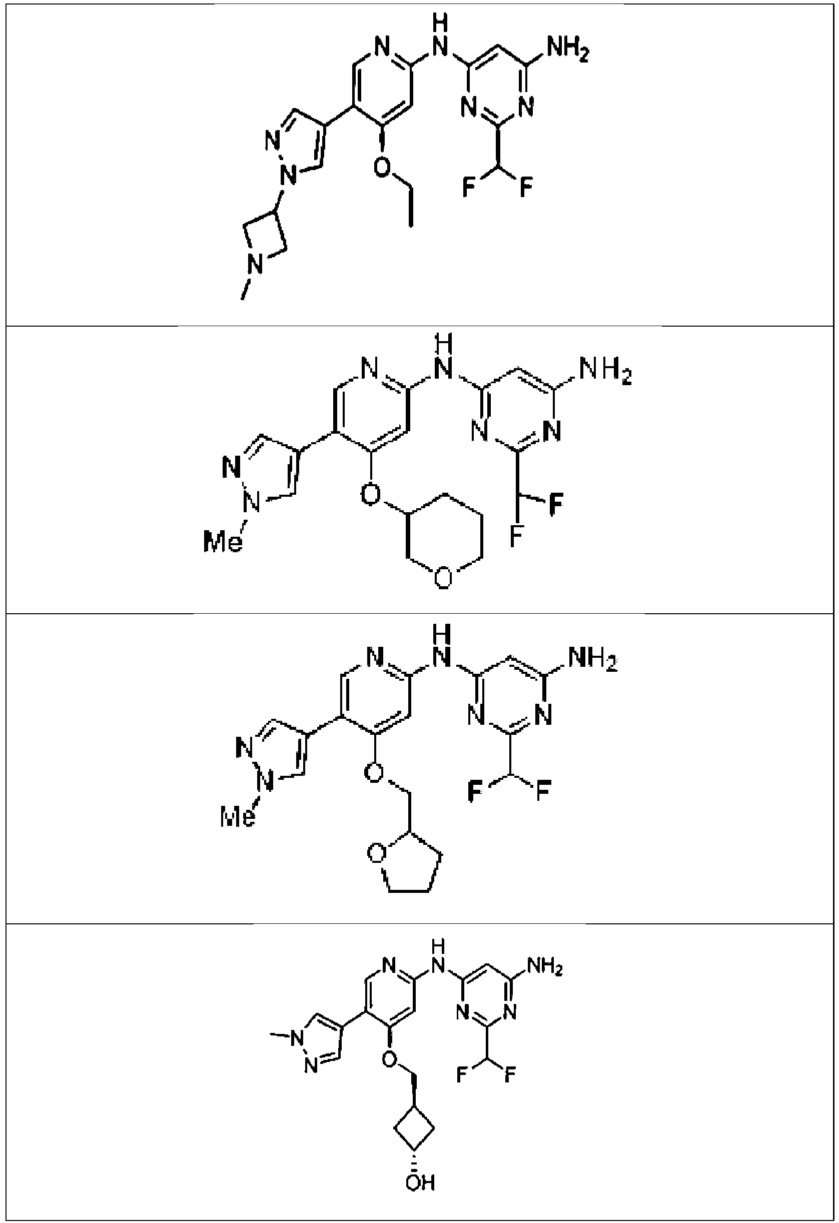

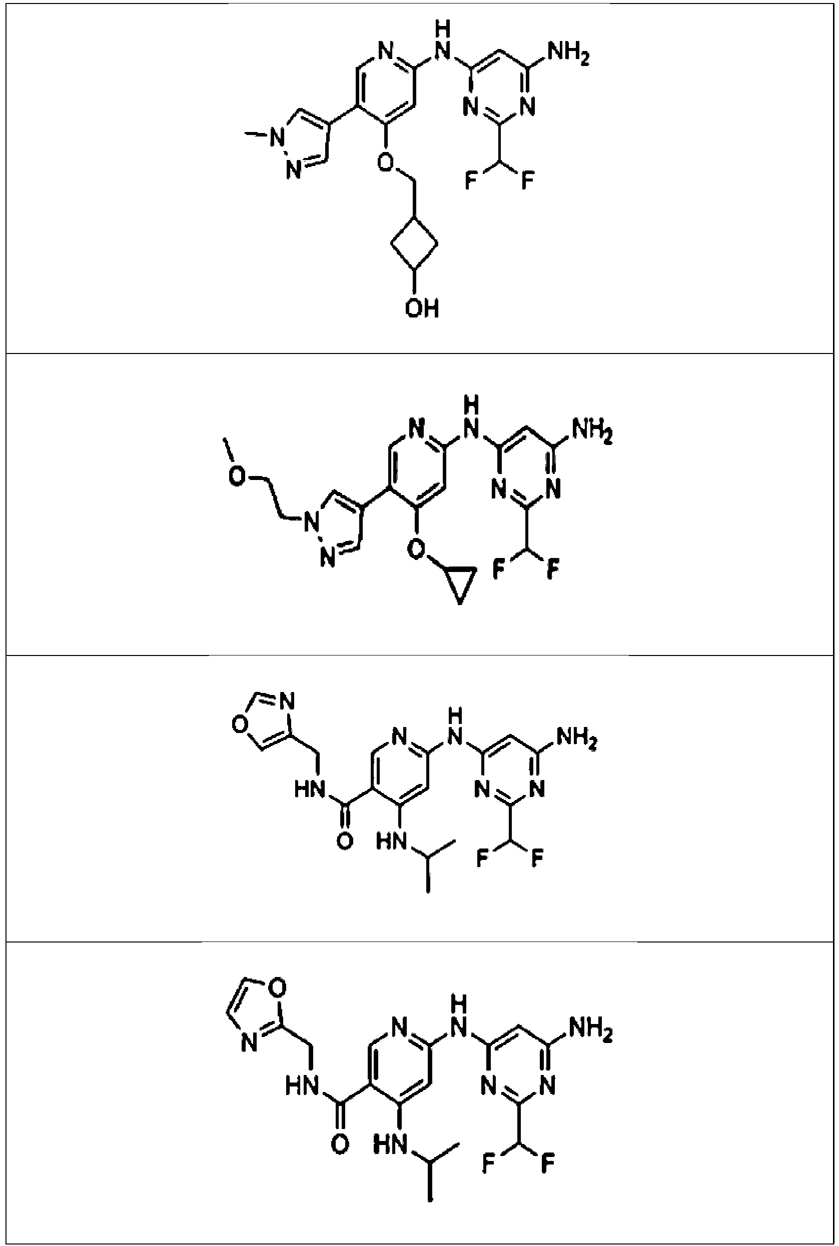

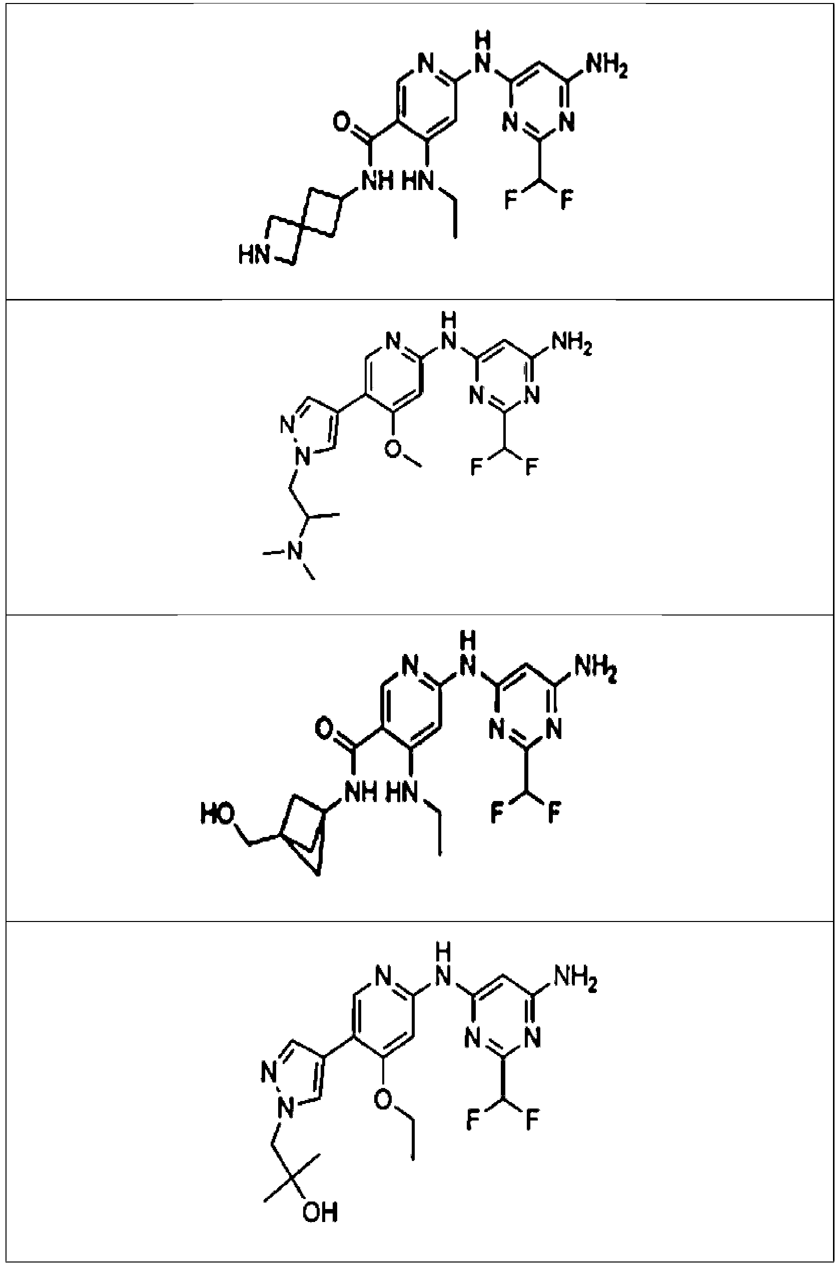

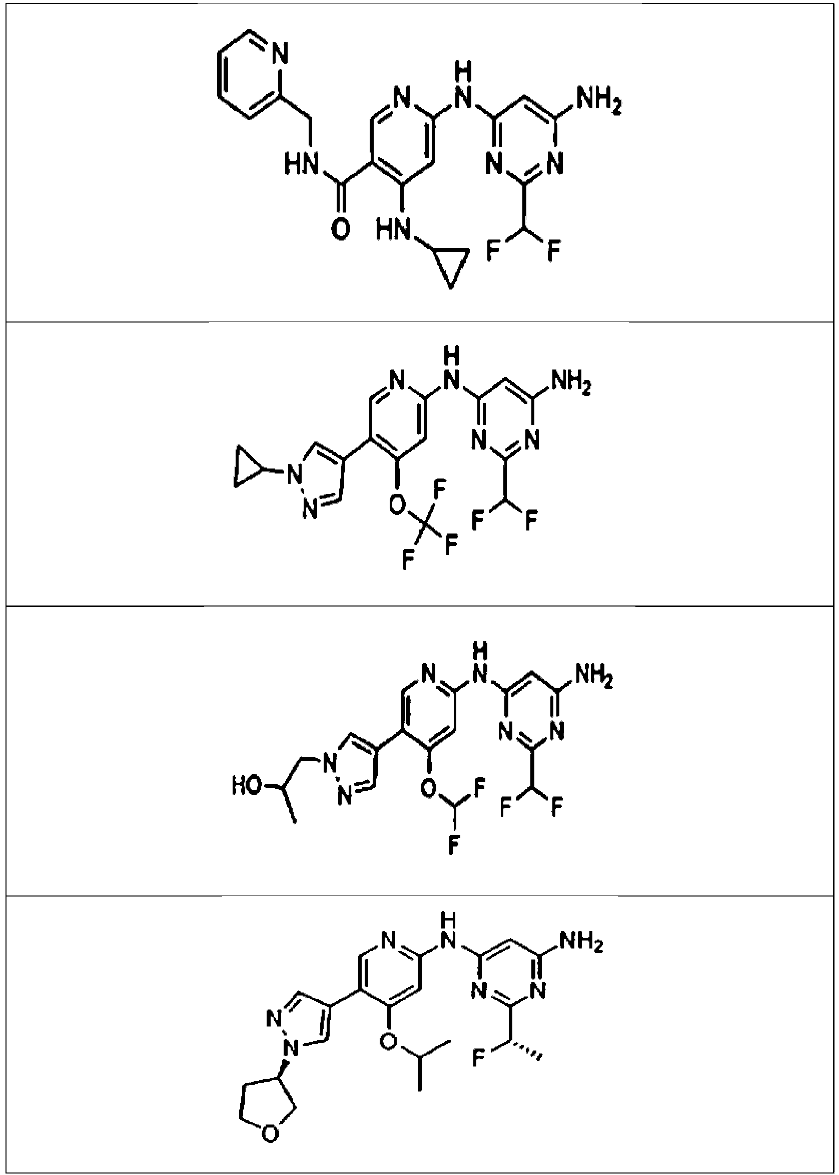

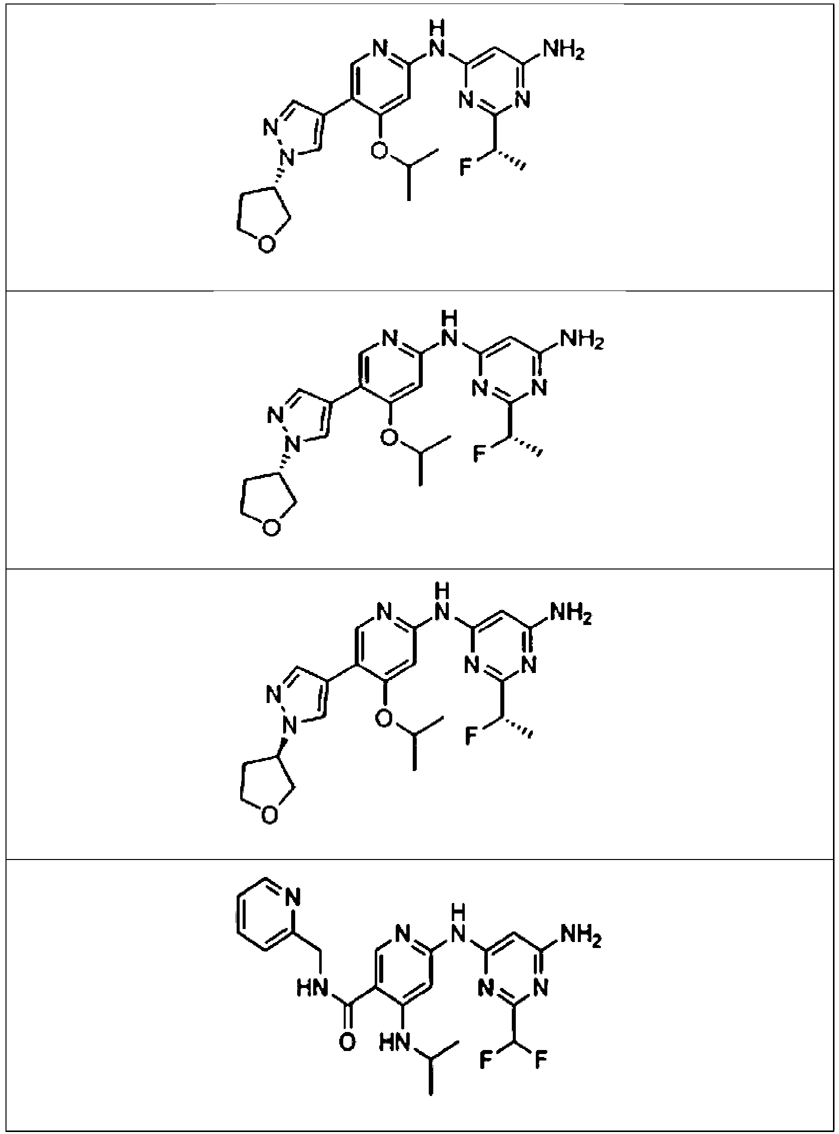

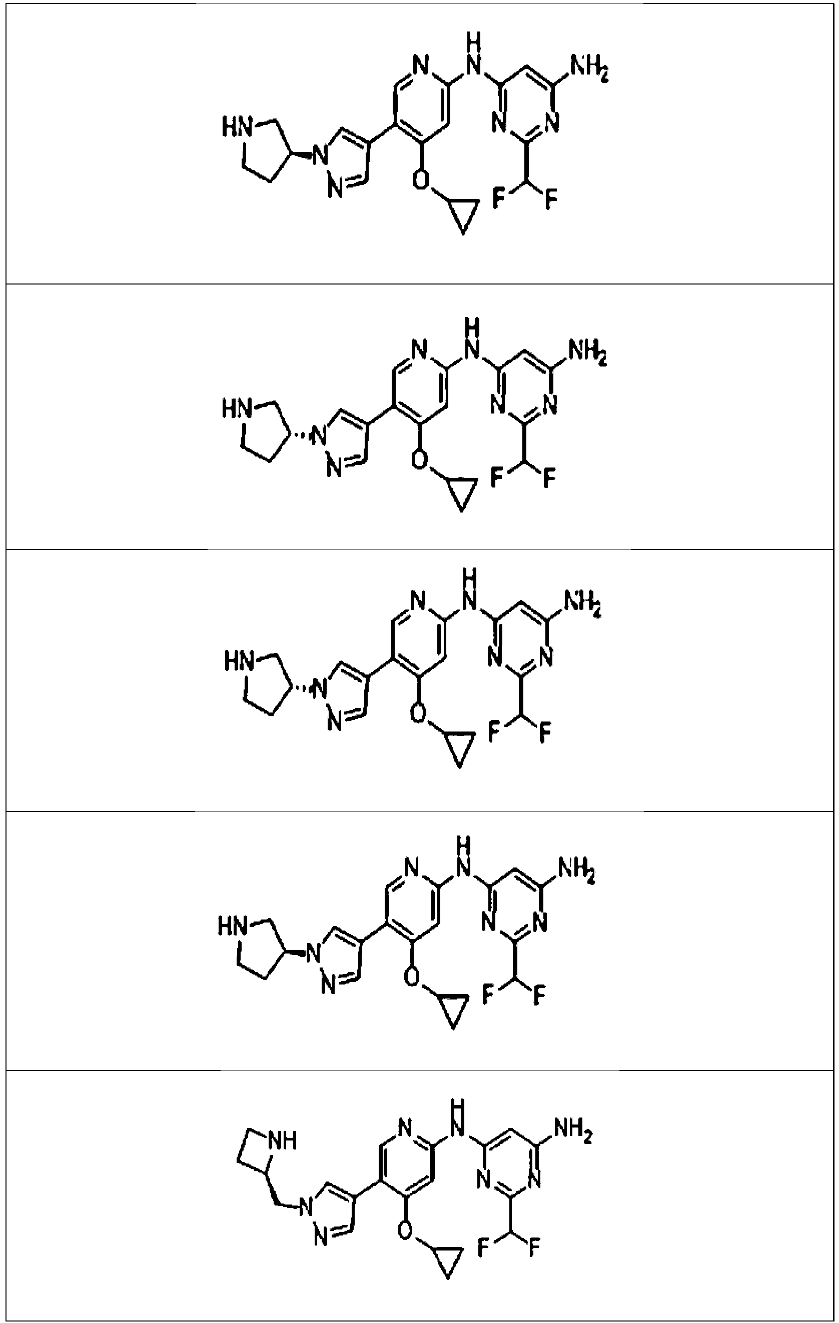

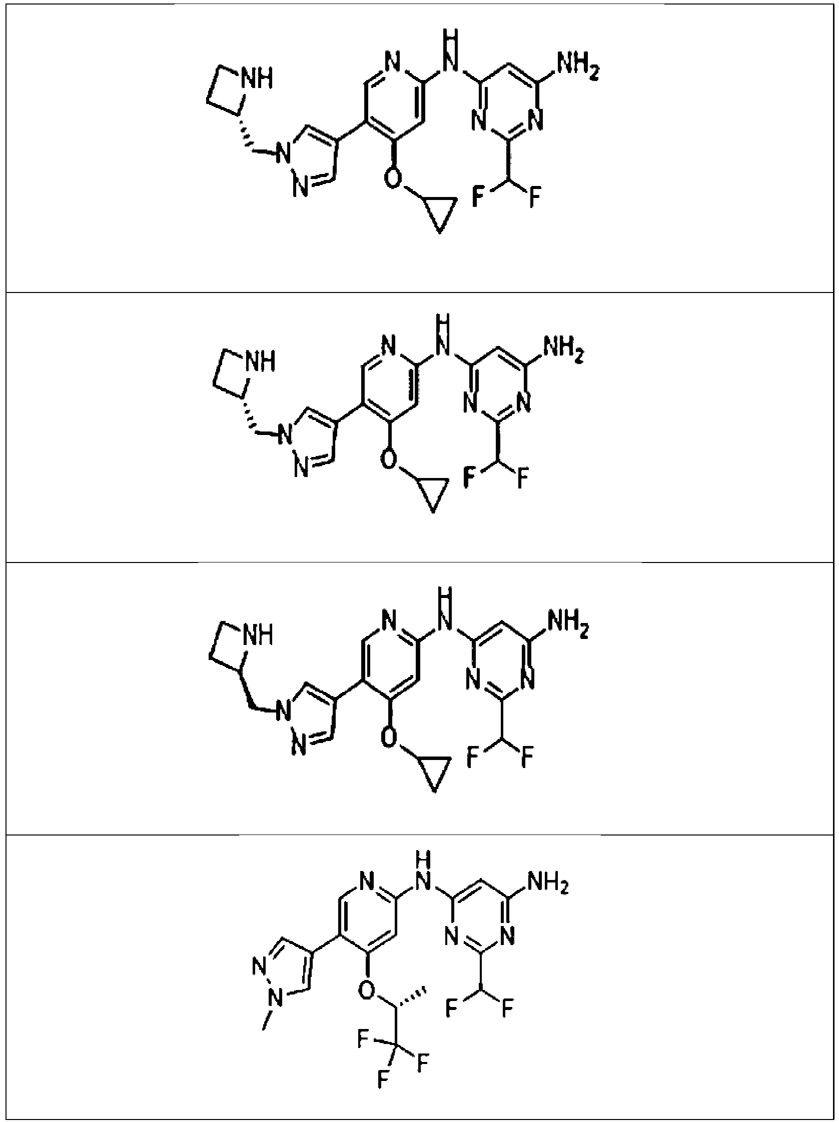

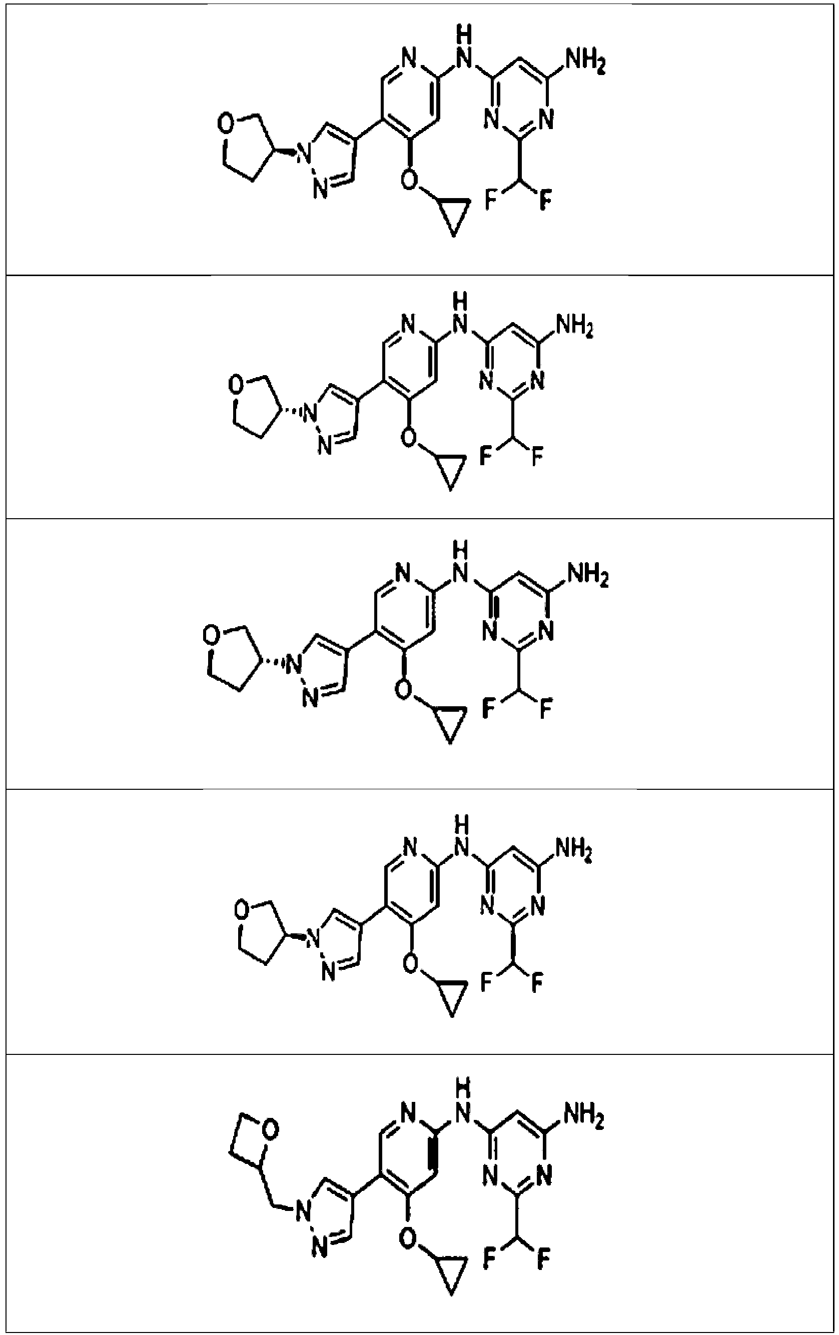

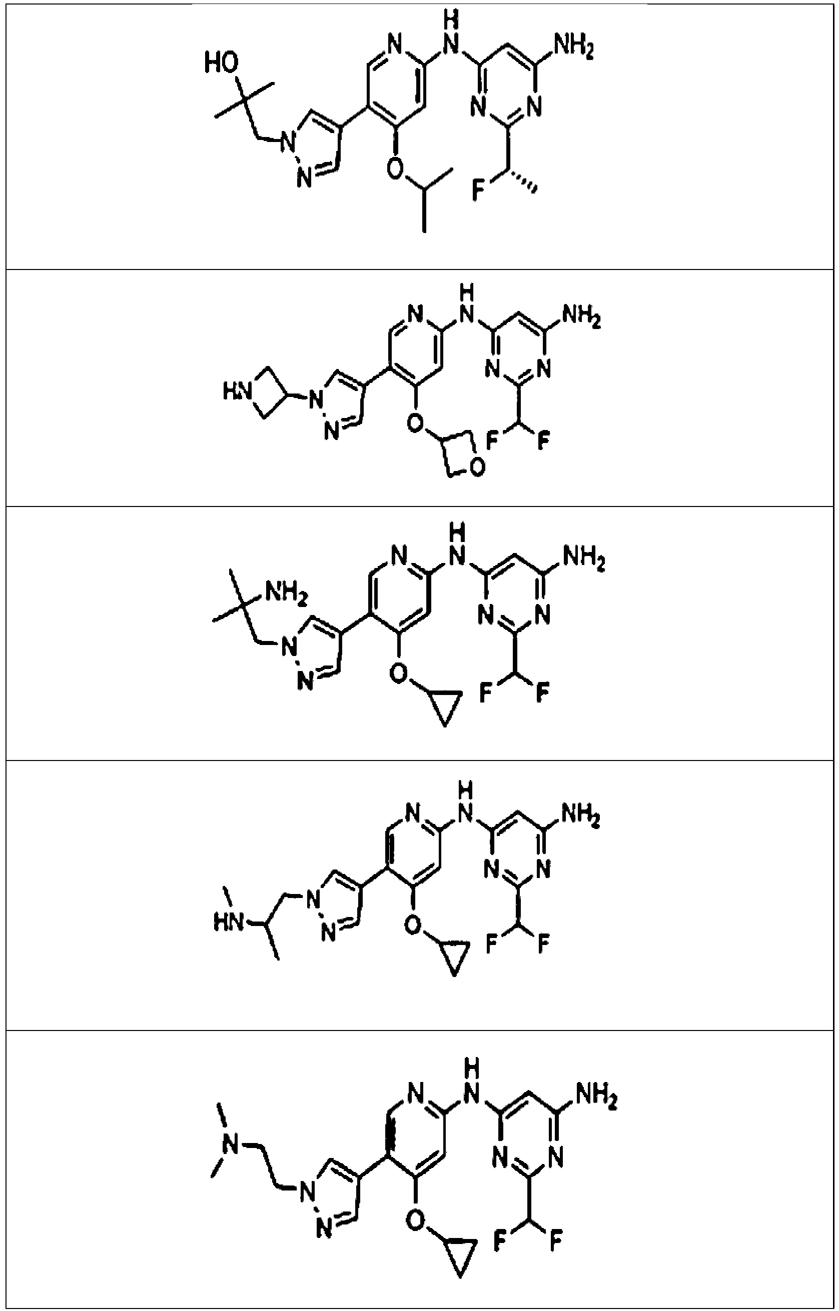

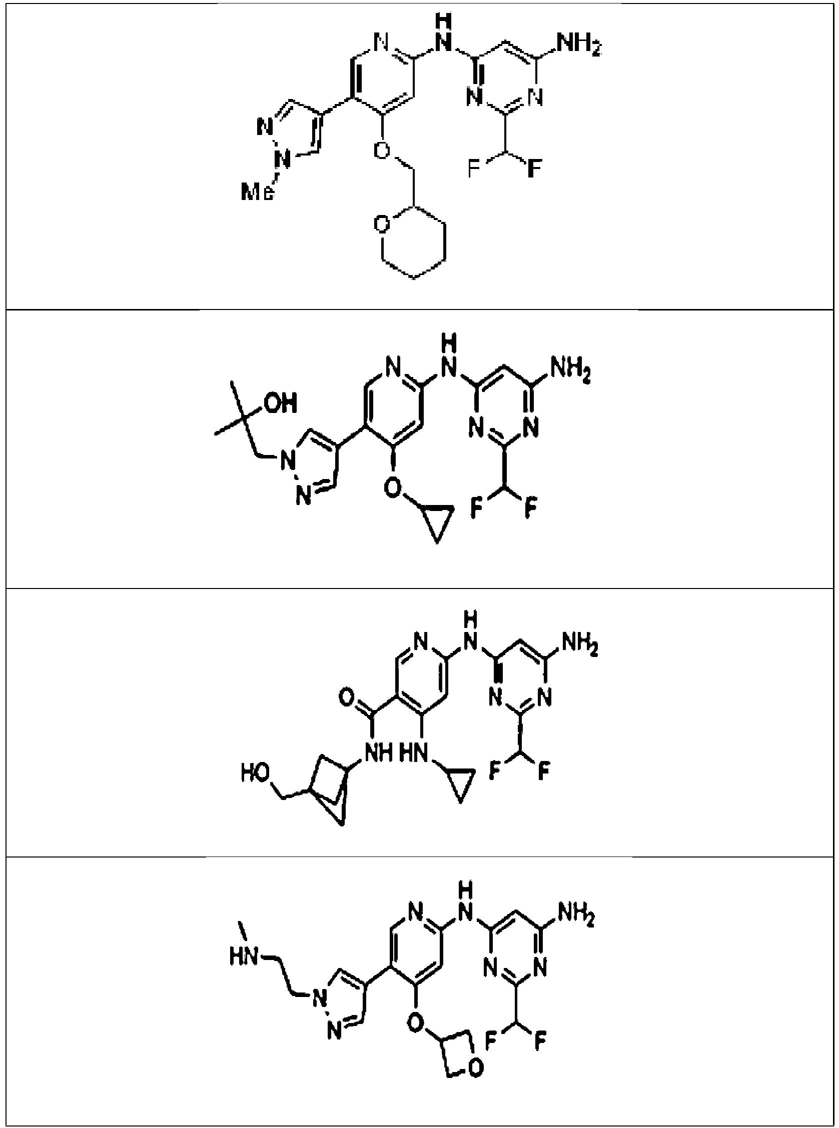

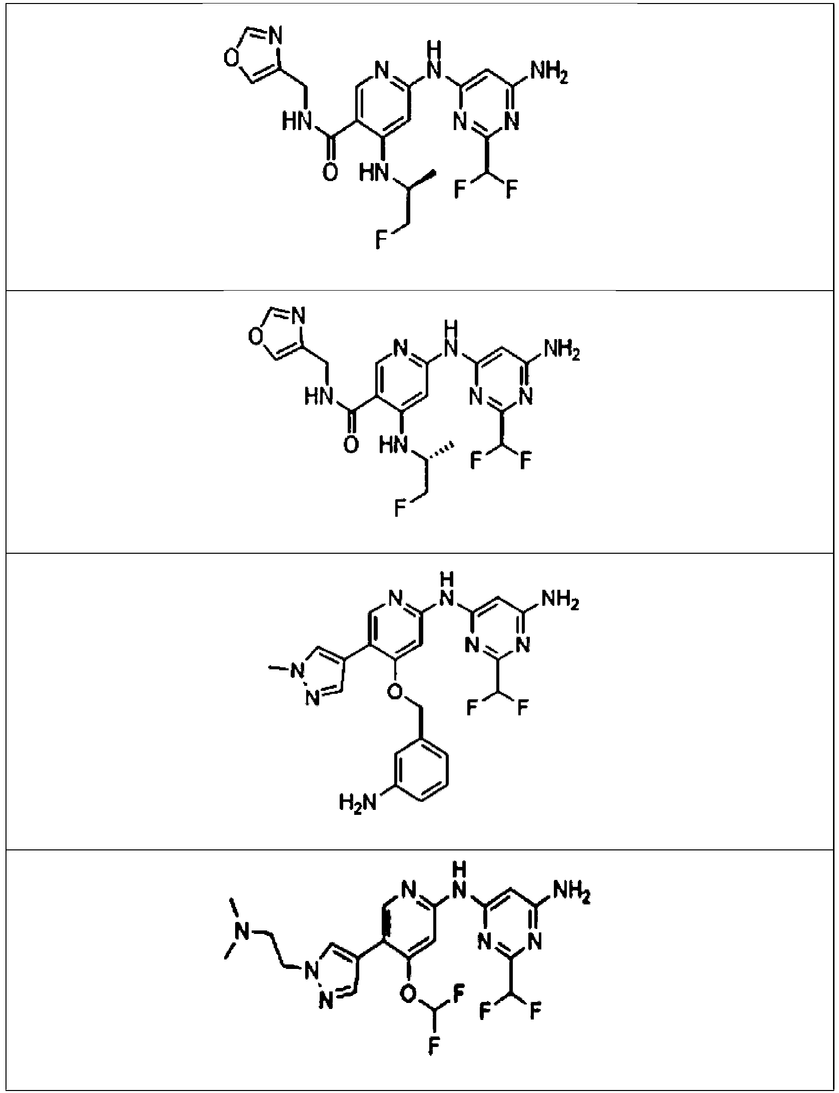

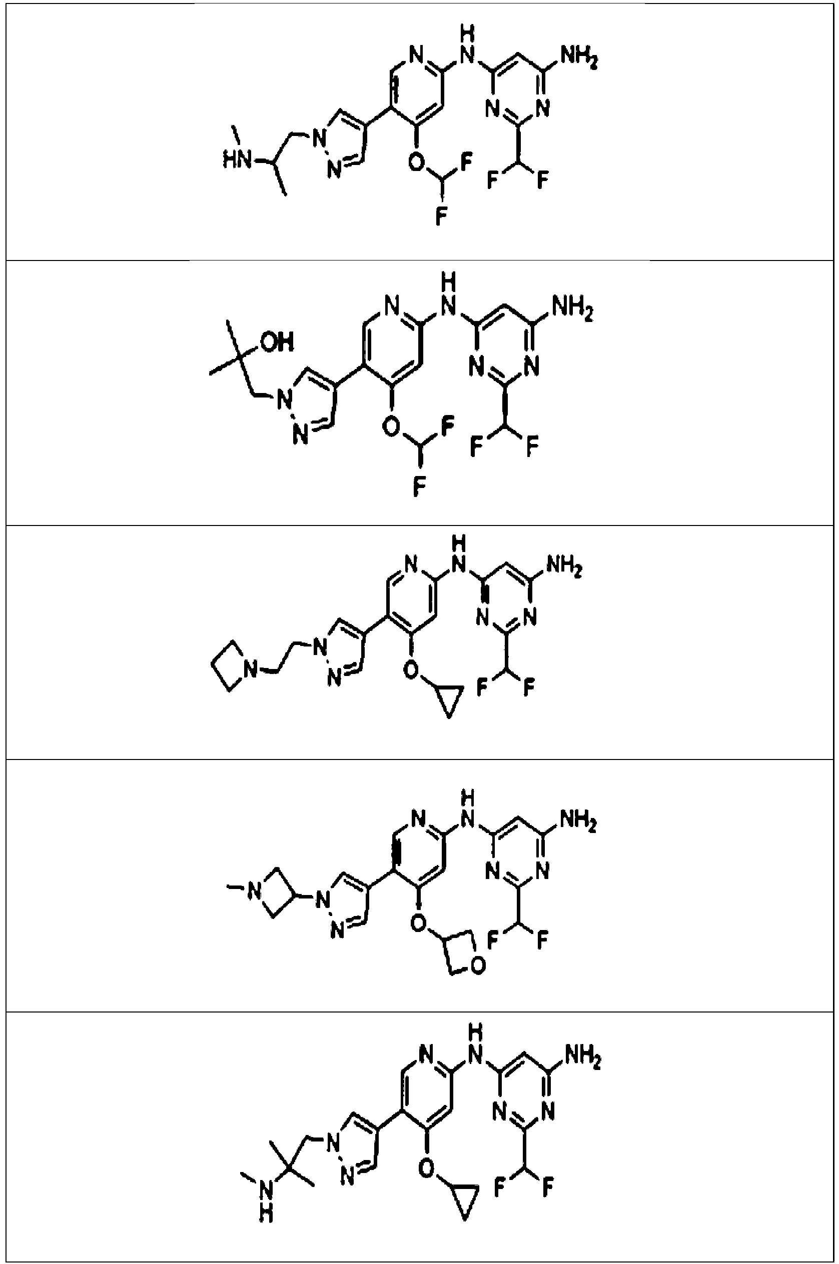

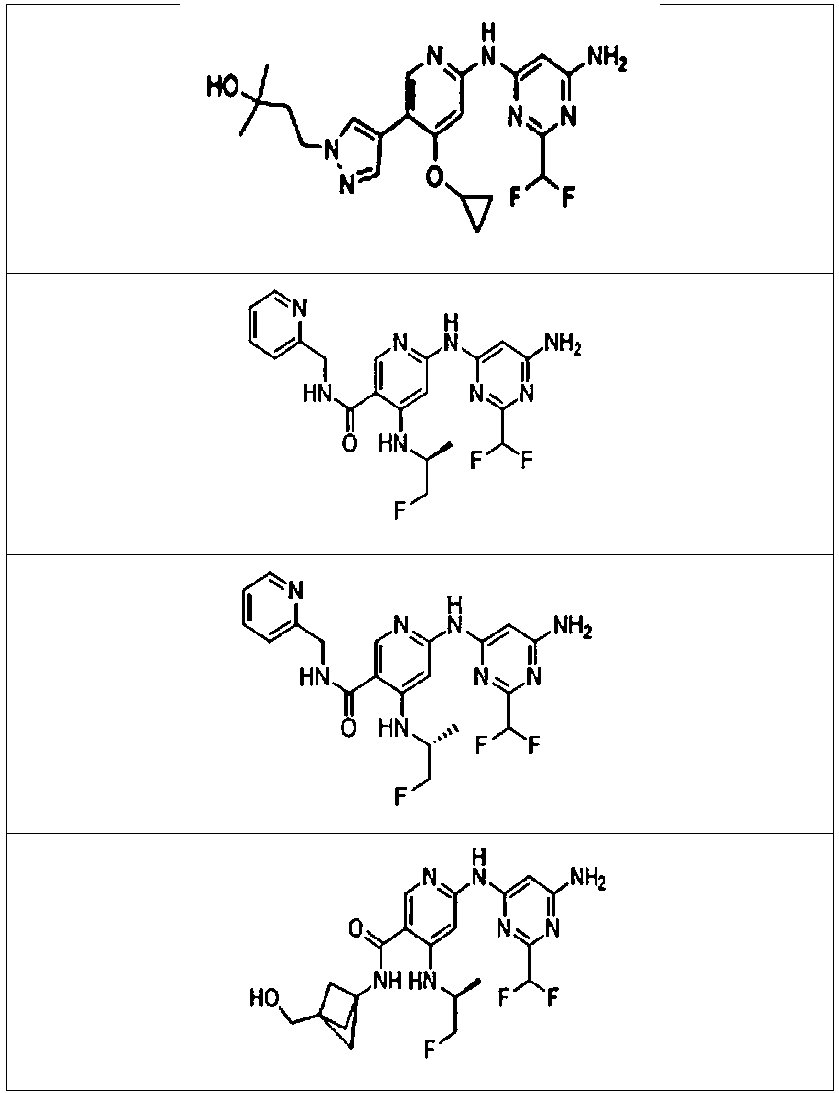

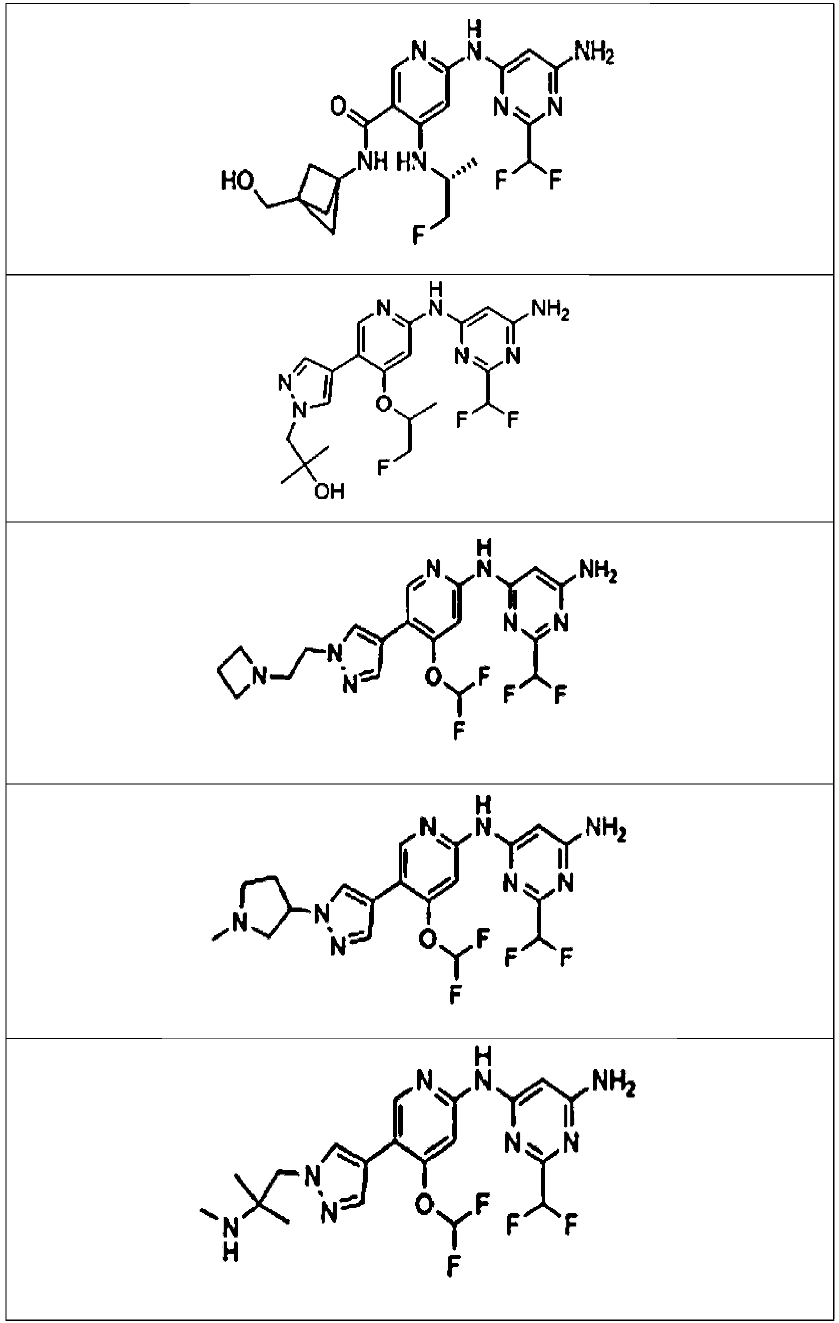

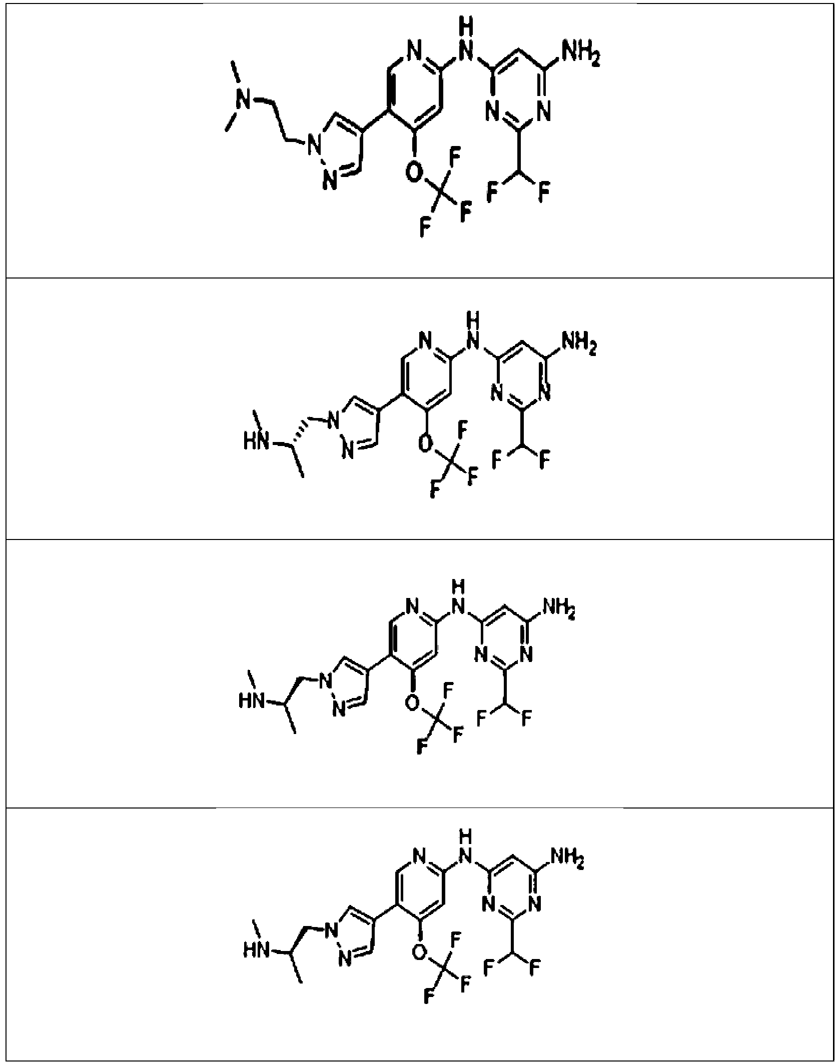

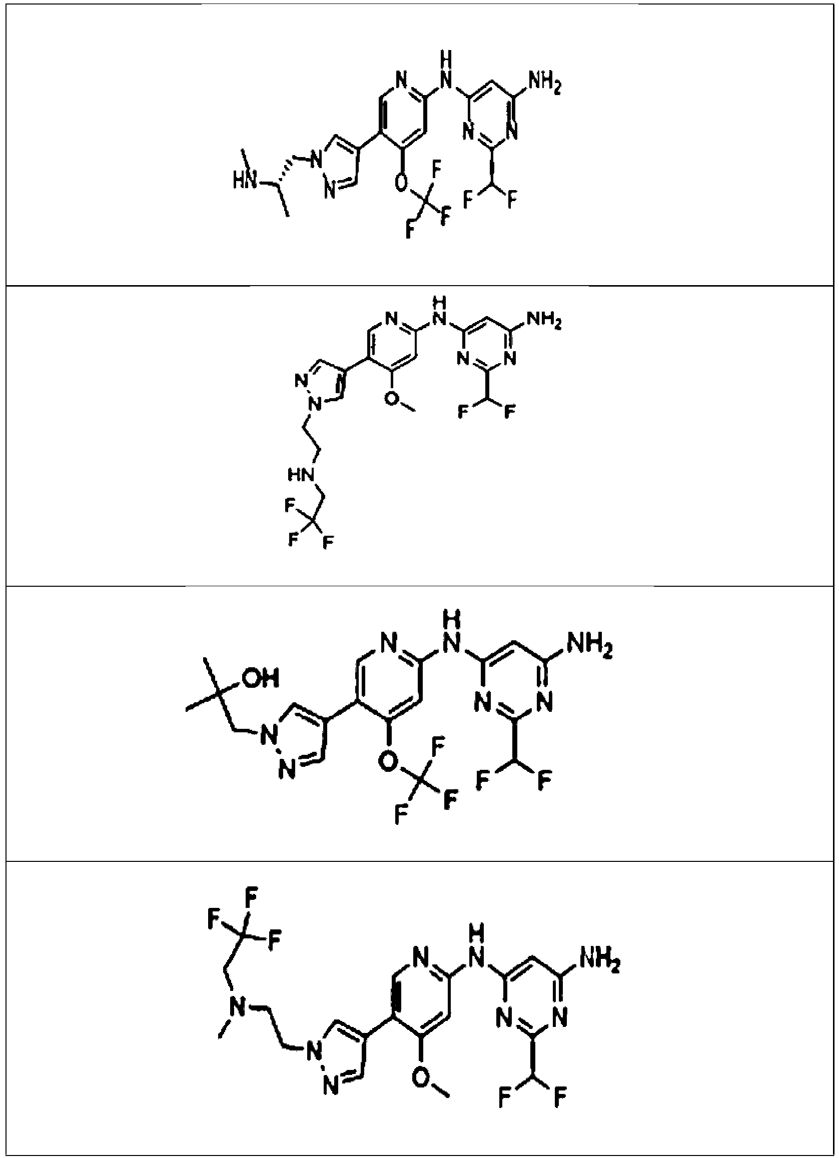

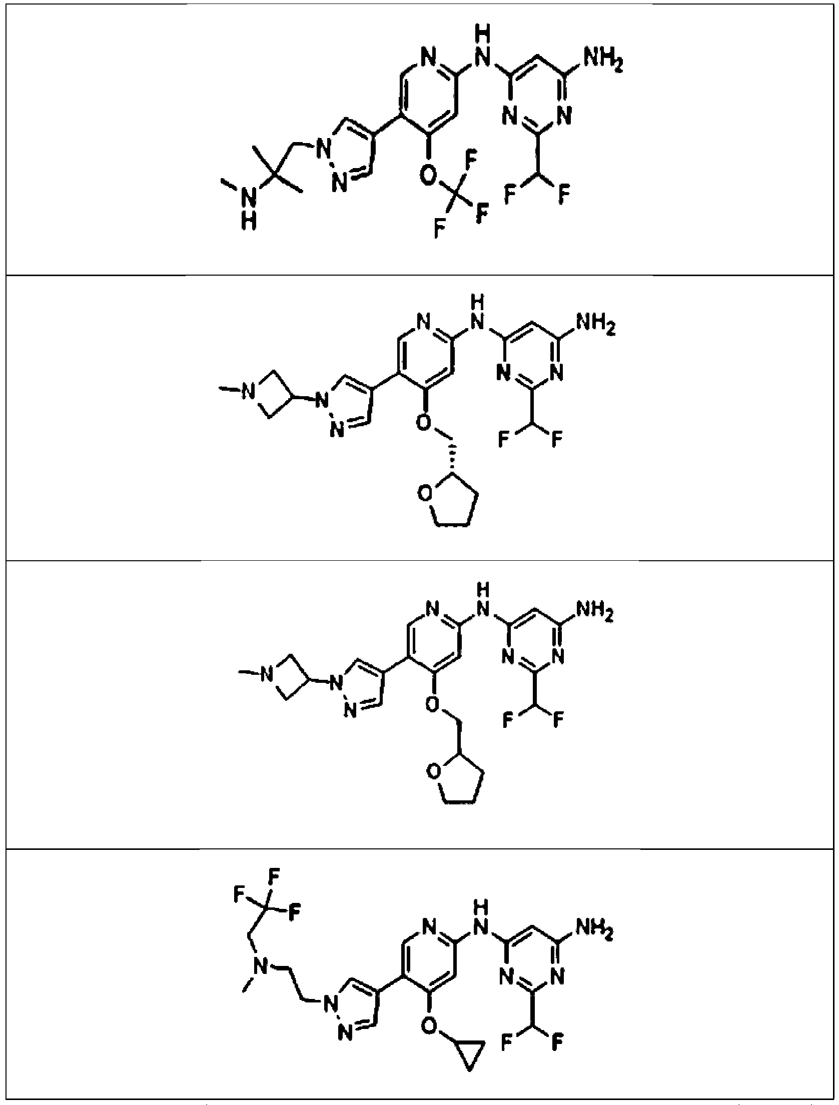

10. Verbindung nach Anspruch 9, wobei die Verbindung aus der Gruppe, bestehend aus 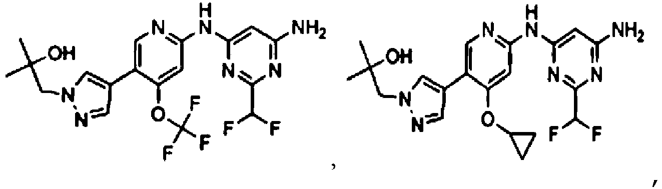 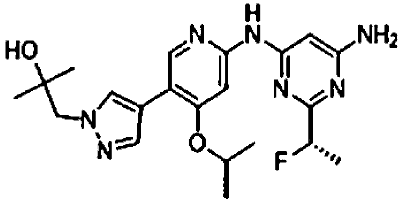 und ausgewählt ist;
oder ein pharmazeutisch akzeptables Salz davon.

11. Pharmazeutische Zusammensetzung, umfassend die Verbindung nach einem der Ansprüche 1-10 oder ein pharmazeutisch akzeptables Salz davon, und einen pharmazeutisch akzeptablen Träger oder Hilfsstoff.

12. Verbindung nach einem der Ansprüche 1 bis 10 oder pharmazeutische Zusammensetzung nach Anspruch 11 zur Verwendung bei der Behandlung eines Patienten, der an einem gastrointestinalen Stromatumor (GIST) leidet, umfassend die Verabreichung einer effektiven Menge der Verbindung nach einem der Ansprüche 1-10 oder eines pharmazeutisch akzeptablen Salzes davon oder der pharmazeutischen Zusammensetzung nach Anspruch 11 an den Patienten.

13. Verbindung oder pharmazeutische Zusammensetzung zur Verwendung nach Anspruch 12, wobei der Patient eine oder mehrere vorherige Behandlungen für den GIST erhielt; optional wobei der GIST nach der vorherigen Behandlung fortschritt und optional wobei die vorherige Behandlung Verabreichung eines oder mehrerer Wirkstoffe umfasst, optional wobei die vorherige Behandlung die Verabreichung eines oder mehrerer Wirkstoffe umfasst, die jeweils unabhängig aus: Imatinib, Sunitinib, Regorafenib, Nilotinib, Cabozantinib, Pazopanib, Ponatinib, Dasatinib, Binimetinib, Vandetanib, Famitinib, Anlotinib, Axitinib, Alvocidib, Ribocliclib, Sorafenib, Pexidartinib, Olaratumab, Crenolanib, Avapritinib, Ripretinib, Bezuclastinib, AZD3229, BLU-263, Everolimus und Larotrectinib ausgewählt werden, und pharmazeutisch akzeptable Salze davon; oder
wobei der GIST durch einen Tumor mit einer oder mehreren KIT-Mutationen gekennzeichnet ist, optional wobei der Tumor eine primär aktivierende KIT-Mutation aufweist und/oder wobei der GIST durch einen Tumor mit einer oder mehreren KIT-Mutationen gekennzeichnet ist, jeweils unabhängig ausgewählt aus einer Exon-9-KIT-Mutation, einer Exon-11-KIT-Mutation, einer Exon-13-KIT-Mutation und eine Exon-17-KIT-Mutation und Kombinationen davon, wobei optional jede der einen oder mehreren KIT-Mutationen unabhängig aus N822K, D816V, D816E, D816F, D816H, D816I, D816Y, D820E, D820Y, D823D, A829P, K642E, V654A und N655K, und Kombinationen davon ausgewählt ist; oder
wobei jede der einen oder mehreren KIT-Mutationen unabhängig aus N822K, D816V, D816E, D816F, D816H, D816I, D816Y, D820E, D820Y, D823D, A829P und K642E und Kombinationen davon ausgewählt ist; oder
wobei der Tumor eine Exon-9-KIT-Mutation aufweist; oder
wobei der Tumor eine Exon-11-KIT-Mutation aufweist; oder
wobei der Tumor eine Exon-17-KIT-Mutation aufweist, optional wobei die Exon-17-KIT-Mutation aus N822K, D816V, D816E, D816F, D816H, D816I, D816Y, D820E, D820Y, D823D und A829P ausgewählt ist; oder
wobei der Tumor eine Exon-13-KIT-Mutation aufweist, optional wobei die Exon-13-KIT-Mutation aus K642E, V654A und N655K und Kombinationen davon ausgewählt ist; und/oder
wobei der Tumor resistent gegen die eine oder mehreren vorherigen Behandlungen ist, optional wobei der Tumor eine Imatinib-resistente Mutation aufweist, optional wobei die Imatinib-resistente Mutation aus einer Exon-13-KIT-Mutation, einer Exon-17-KIT-Mutation und einer Exon-14-KIT-Mutation und Kombinationen davon ausgewählt ist.

14. Verbindung nach einem der Ansprüche 1 bis 10 oder pharmazeutische Zusammensetzung nach Anspruch 11 zur Verwendung in einem Verfahren zur Behandlung eines Patienten, der an einer bösartigen Erkrankung leidet, die durch eine Exon-9-KIT-Mutation, eine Exon-11-KIT-Mutation, eine Exon-13-KIT-Mutation oder eine Exon-17-KIT-Mutation oder eine Kombination davon gekennzeichnet ist, umfassend Verabreichen einer effektiven Menge der Verbindung nach einem der Ansprüche 1-10 oder eines pharmazeutisch akzeptablen Salzes davon oder der pharmazeutischen Zusammensetzung nach Anspruch 12 an den Patienten;
optional, wobei die bösartige Erkrankung aus gastrointestinalem Stromatumor (GIST), AML (akute myeloische Leukämie), Melanom, Lungenkrebs, Gebärmutterkrebs, Astrozytom, Leberkrebs, Seminom, Nierenzellkarzinom, intrakraniellem Keimzelltumor, Pankreaskarzinom und mediastinalem B-Zell-Lymphom ausgewählt ist.

15. Kombination zur Verwendung in einem Verfahren zur Behandlung eines Patienten, der an einem gastrointestinalen Stromatumor (GIST) leidet, umfassend die Verabreichung einer wirksamen Kombination an den Patienten, umfassend:
eine Verbindung nach einem der Ansprüche 1-10 oder ein pharmazeutisch akzeptables Salz davon oder eine pharmazeutische Zusammensetzung nach Anspruch 11, und
mindestens einen zusätzlichen Wirkstoff,
optional, wobei der zusätzliche Wirkstoff ein Exon-9-KIT-Inhibitor oder Exon-11-KIT-Inhibitor ist; oder
wobei der zusätzliche Wirkstoff Imatinib oder ein pharmazeutisch akzeptables Salz davon ist oder
wobei der zusätzliche Wirkstoff ein Exon-17-KIT-Inhibitor ist, optional wobei der zusätzliche Wirkstoff aus Avapritinib, BLU-263, Ripretinib, AZD3229 und Bezuclastinib und pharmazeutisch akzeptablen Salzen davon ausgewählt ist.

16. Verbindung nach einem der Ansprüche 1 bis 10 oder pharmazeutische Zusammensetzung nach Anspruch 11 zur Verwendung in einem Verfahren zur Behandlung eines Patienten, der an einer bösartigen Erkrankung leidet, die durch einen Tumor mit einer Exon-9-KIT-Mutation, einer Exon-11-KIT-Mutation oder einer Exon-17-KIT-Mutation oder einer Kombination davon gekennzeichnet ist, umfassend:
(a) Entnahme einer biologischen Probe vom Patienten;
(b) Erkennen des Vorhandenseins oder Fehlens einer Exon-13-KIT-Mutation oder Exon-14-KIT-Mutation, optional wobei die Exon-13-KIT-Mutation V654A oder N655K ist oder wobei die Exon-14-KIT-Mutation N680K ist; und
(c) Verabreichen einer wirksamen Menge einer Verbindung nach einem der Ansprüche 1-10 oder eines pharmazeutisch akzeptablen Salzes davon oder einer pharmazeutischen Zusammensetzung nach Anspruch 11 an den Patienten, wenn die Mutation erkannt wird.

17. Verbindung nach einem der Ansprüche 1 bis 10 oder pharmazeutische Zusammensetzung nach Anspruch 11 zur Verwendung in der Therapie.

## Revendications

1. Composé de formule (I) : 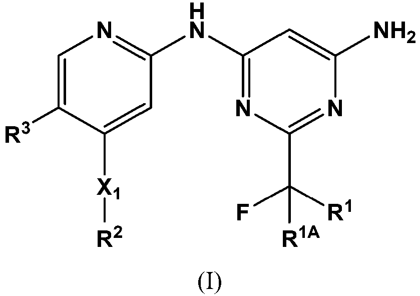
ou un de ses sels pharmaceutiquement acceptables, dans laquelle :
R¹ et R^{1A} sont choisis chacun indépendamment parmi H, un halogène et CH₃,
ou bien R¹ et R^{1A}, pris conjointement avec l'atome de carbone auquel ils sont liés, forment un groupe cyclopropyle ;
R² est choisi parmi un groupe alkyle en C₁₋₅, CD₃, cycloalkyle en C₃₋₆, bicyclo[1.1.1]pentane et un hétérocycle de 4 à 6 chaînons contenant de l'oxygène, ledit groupe alkyle, cycloalkyle ou hétérocycle étant éventuellement substitué par 1 à 3 groupes R⁴ ;
chaque R⁴ est choisi indépendamment parmi un halogène, un groupe CH₃, un groupe alcényle en C₂₋₃, OH, CH₂ OH, un groupe cycloalkyle en C₄₋₆, un hétérocycle de 4 à 6 chaînons contenant de l'oxygène, et un groupe phényle, ledit groupe alkyle, cycloalkyle, hétérocycle ou phényle étant éventuellement substitué par un groupe OH ou NH₂, un groupe alkyle en C₁₋₂, CH₂ NH₂, ou un atome d'halogène ;
R³ est C(O)NHR⁵ , 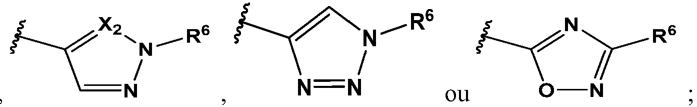
X₁ est NH ou O ;
X₂ est N ou CH ;
X₃ est N ou CH ;
R⁵ est choisi parmi H, un groupe alkyle en C₁₋₃, CD₃, cycloalkyle en C₃₋₄et bicyclo[1.1.1]pentane, ledit groupe alkyle, cycloalkyle ou bicyclo[1.1.1]pentane étant éventuellement substitué par 1 à 2 groupes R⁷;
chaque R⁷ est choisi indépendamment parmi CN, NH₂, OH, CH₂ OH, un groupe cyclopropyle, pyridinyle et oxazolyle, ou, pris ensemble, deux R⁷ liés au même atome de carbone forment un hétérocycle à 4 chaînons contenant de l'azote ;
R⁶ est choisi parmi un groupe alkyle en C₁₋₅, CHF₂, CF₃, un hétérocycle à 4 ou 5 chaînons contenant N ou O, et un groupe cycloalkyle en C₃₋₄, ledit groupe alkyle ou hétérocycle étant éventuellement substitué par un R⁸ ;
R⁸ est choisi parmi OH, NR⁹R⁹, OCH₃ , CH₃ et un hétérocycle à 4 chaînons contenant N ou O, ledit groupe alkyle ou hétérocycle étant éventuellement substitué par un groupe R¹⁰ ;
chaque R⁹ est choisi indépendamment parmi H, CH₃ et CH₂ CF₃ ; et
R¹⁰ est choisi parmi CH₃ et CF₃.

2. Composé selon la revendication 1, ou un de ses sels pharmaceutiquement acceptables, dans lequel :
R¹ et R^{1A} sont chacun choisis indépendamment parmi H, un halogène et CH₃ ;
R² est choisi parmi un groupe alkyle en C₁₋₅, CD₃, cycloalkyle en C₃₋₆ et un hétérocycle de 4 à 6 chaînons contenant O, ledit groupe alkyle, cycloalkyle ou hétérocycle étant éventuellement substitué par 1 à 3 groupes R⁴ ;
chaque R⁴ est choisi indépendamment parmi un halogène, CH₃, OH, un groupe cycloalkyle en C₄₋₆, un hétérocycle de 4 à 6 chaînons contenant de l'oxygène et un groupe phényle, ledit groupe cycloalkyle ou phényle étant éventuellement substitué par OH ou NH₂ ;
chaque R⁷ est choisi indépendamment parmi CN, OH, CH₂ OH, un groupe cyclopropyle, pyridinyle et oxazolyle, ou bien, pris ensemble, deux R⁷ liés au même atome de carbone forment un hétérocycle à 4 chaînons contenant N ; et
R¹⁰ est CH₃.

3. Composé selon la revendication 1 ou 2, **caractérisé en ce que** le composé est représenté par la formule (II) : 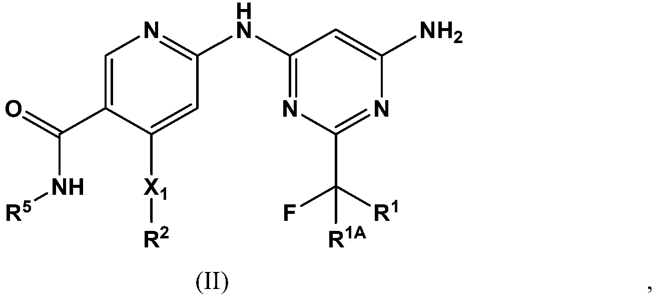
ou un de ses sels pharmaceutiquement acceptables ; ou
dans lequel le composé est représenté par la formule (III) : 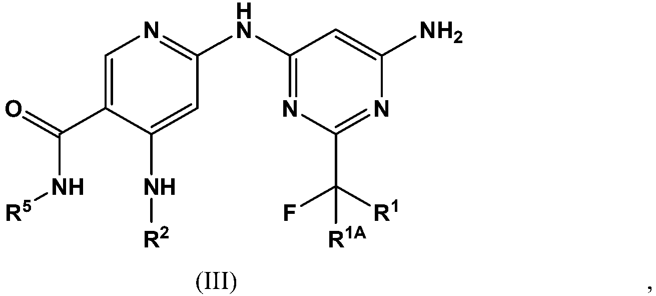
ou un sel pharmaceutiquement acceptable de celui-ci.

4. Composé selon la revendication 3, ou un de ses sels pharmaceutiquement acceptables, dans lequel :
R¹ et R^{1A} sont choisis chacun indépendamment parmi H, un halogène et CH₃ ;
R² est choisi parmi un groupe alkyle en C₁₋₅, CD₃, cycloalkyle en C₃₋₄ et un hétérocycle de 4 à 6 chaînons contenant O, ledit groupe alkyle, cycloalkyle ou hétérocycle étant éventuellement substitué par 1 à 3 R⁴ ;
chaque R⁴ est choisi indépendamment parmi un halogène, un groupe CH₃ et un groupe phényle ;
R⁵ est choisi parmi H, un groupe alkyle en C₁₋₃, CD₃, un groupe cycloalkyle en C₃₋₄ et le bicyclo[1.1.1]pentane, ledit groupe alkyle, cycloalkyle ou bicyclo[1.1.1]pentane étant éventuellement substitué par 1 à 2 R⁷; et
chaque R⁷est choisi indépendamment parmi CN, OH, CH₂OH, un groupe cyclopropyle, pyridinyle et oxazolyle, ou bien, pris ensemble, deux R⁷ liés au même atome de carbone forment un hétérocycle à 4 chaînons contenant N ; et/ou
dans lequel R² est choisi parmi CH₃, CD₃, CH₂CH₃, CH₂CH₂CH₃, CH(CH₃)₂, CH₂CH(CH₃)₂, CH(CH₃)CH₂CH₃, le cyclobutyle, le cyclopropyle, l'oxétanyle, le tétrahydrofuranyle et le tétrahydropyranyle, chacun d'entre eux étant éventuellement substitué par 1 à 3 R⁴ ; et/ou
dans lequel R² est choisi parmi , 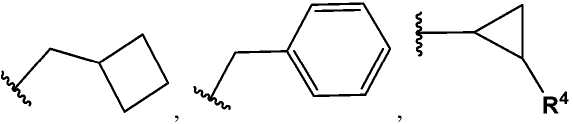 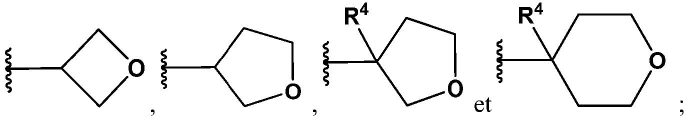 et/ou
dans lequel chaque R⁴ est choisi indépendamment parmi F, CH₃, le cyclobutyle et le phényle ; et/ou
dans lequel R⁵ est choisi parmi H, CH₃, CD₃, CH₂CH₃, CH₂CH₂CH₃, CH(CH₃)₂, le cyclobutyle, le cyclopropyle et le bicyclo[1.1.1]pentanyle, dans lequel lesdits CH₃, CH₂CH₃, CH₂CH₂CH₃ , CH(CH₃)₂, cyclobutyle, cyclopropyle ou bicyclo[1.1.1]pentanyle sont éventuellement substitués par 1 à 2 R⁷ ; et/ou
dans lequel R⁵est choisi parmi 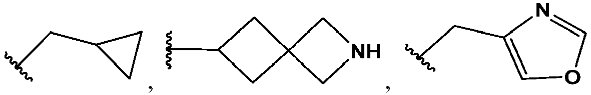 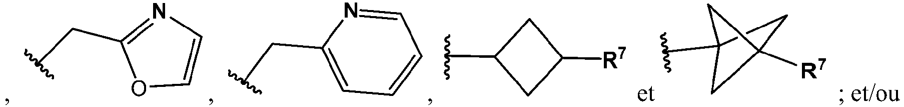
dans lequel chaque R⁷est choisi indépendamment parmi CN, CH₂OH, le cyclopropyle, 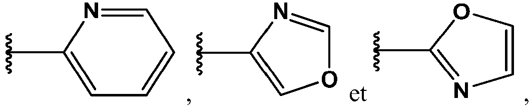 ou bien, pris ensemble, deux R⁷ liés au même atome de carbone forment 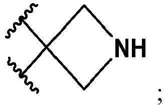 et/ou
dans lequel R¹ et R^{1A} sont chacun choisis indépendamment parmi H, F et CH₃.

5. Composé selon la revendication 1 ou 2, **caractérisé en ce que** le composé est représenté par la formule (IV), (V) ou (VI) : 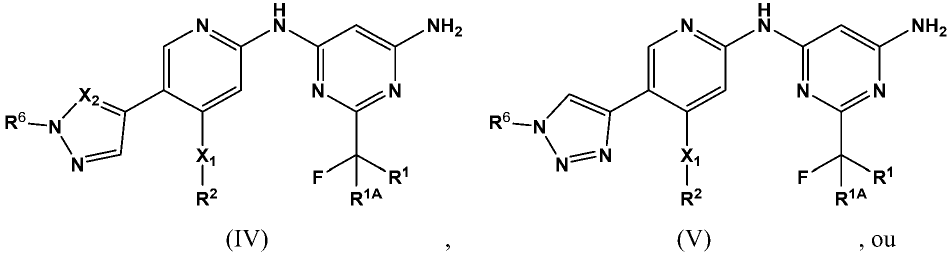 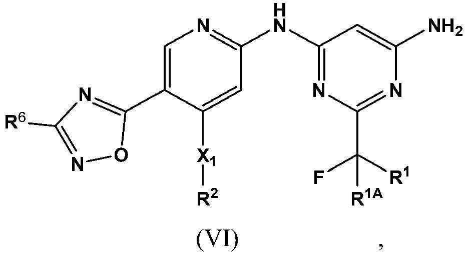
ou un de ses sels pharmaceutiquement acceptables ; ou
dans lequel le composé est représenté par la formule (VII), (VIII), (IX) ou (X) : 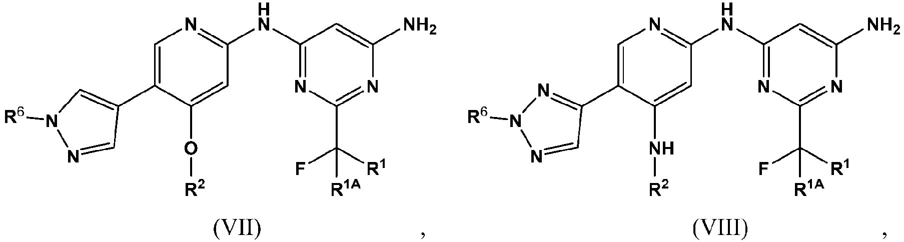 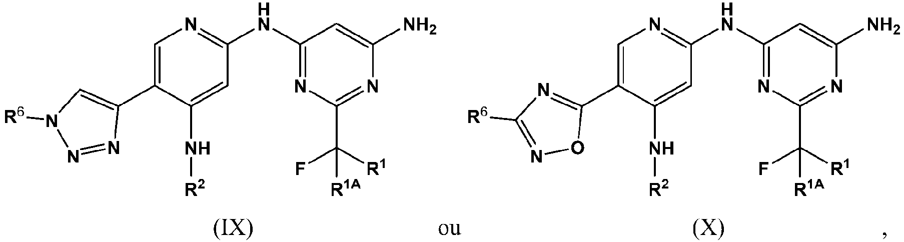
ou un de ses sels pharmaceutiquement acceptables.

6. Le composé selon la revendication 5, ou un de ses sels pharmaceutiquement acceptables, dans lequel :
R¹ et R^{1A} sont chacun choisis indépendamment parmi H, un halogène et CH₃ ;
R² est choisi parmi un groupe alkyle en C₁₋₃, CD₃, cycloalkyle en C₃₋₄ et un hétérocycle de 4 à 6 chaînons contenant un atome d'oxygène, ledit groupe alkyle, cycloalkyle ou hétérocycle étant éventuellement substitué par 1 à 3 groupes R⁴ ;
chaque R⁴ est choisi indépendamment parmi un halogène, un groupe OH, un groupe cyclopropyle, un hétérocycle de 4 à 6 chaînons contenant un atome d'oxygène et un groupe phényle, ledit groupe cyclopropyle ou phényle étant éventuellement substitué par un groupe OH ou NH₂ ;
R⁶ est choisi parmi un groupe alkyle en C₁₋₄, CHF₂, CF₃, un hétérocycle à 4 ou 5 chaînons contenant N ou O, et un groupe cycloalkyle en C₃₋₄, ledit groupe alkyle, hétérocycle ou cycloalkyle étant éventuellement substitué par R⁸;
R⁸ est choisi parmi OH, NR⁹R⁹, OCH₃, CH₃ et un hétérocycle à 4 chaînons contenant N ou O, ledit hétérocycle étant éventuellement substitué par CH₃ ; et
chaque R⁹ est choisi indépendamment parmi H, CH₃ et CH₂CF₃ ; et/ou
dans lequel R² est choisi parmi CH₃, CD₃, CH₂CH₃, CH₂CH₂CH₃, CH(CH₃)₂, cyclobutyle, cyclopropyle, oxétanyle, tétrahydrofuranyle et tétrahydropyranyle, chacun d'entre eux étant éventuellement substitué par 1 à 3 R⁴ ; et/ou
dans lequel R² est choisi parmi 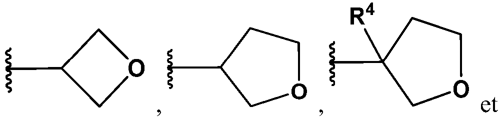 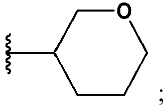 et/ou
dans lequel chaque R⁴ est choisi indépendamment parmi F, OH, un groupe cyclobutyle, oxétanyle, oxétanyle, phényle, tétrahydrofuranyle et tétrahydropyranyle, lesdits groupes cyclobutyle, oxétanyle, oxétanyle, phényle, tétrahydrofuranyle ou tétrahydropyranyle étant éventuellement substitués par un groupe OH ou NH₂ ; et/ou
dans lequel chaque R⁴ est choisi indépendamment parmi F, OH, 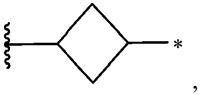 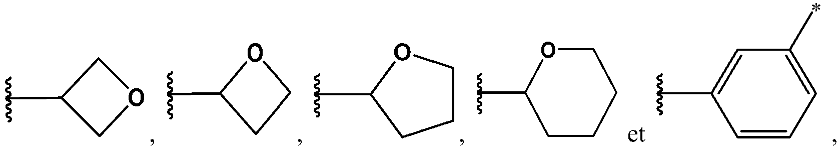 dans lequel *- représente OH ou NH₂ ; et/ou
dans lequel R⁶ est choisi parmi CH₃, CH₂CH₃, CH₂CH₂CH₃, CH(CH₃)₂, CH₂CH(CH₃)₂, C(CH₃)₃, CH₂CH₂CH(CH₃)₂, CHF₂, CF₃, cyclopropyle, cyclobutyle, azétidinyle, oxétanyle, tétrahydrofuranyle et pyrrolidinyle, chacun d'entre eux étant éventuellement substitué par R⁸ ; ou
dans lequel R⁶ est choisi parmi 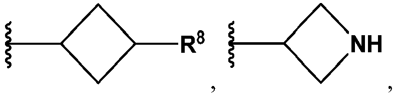 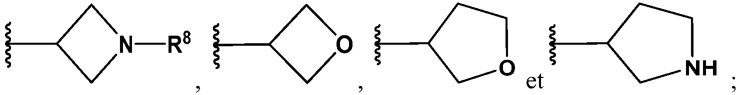 et/ou
dans lequel R⁸ est choisi parmi OH, OCH₃, CH₃, NH₂, NHCH₃, N(CH₃)₂, NHCH₂CF₃, N(CH₃)CH₂CF₃ , azétidinyle, azétidinyle et oxétanyle ; et/ou
dans lequel R⁸ est choisi parmi 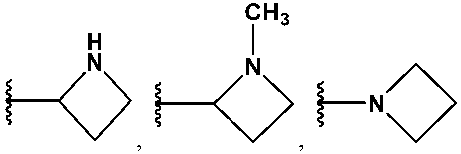 et 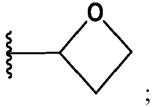 et/ou
dans lequel R¹et R^{1A} sont chacun choisis indépendamment parmi H, F et CH₃.

7. Composé selon la revendication 1 ou 2, **caractérisé en ce que** le composé est représenté par la formule (III) ou (VII) : 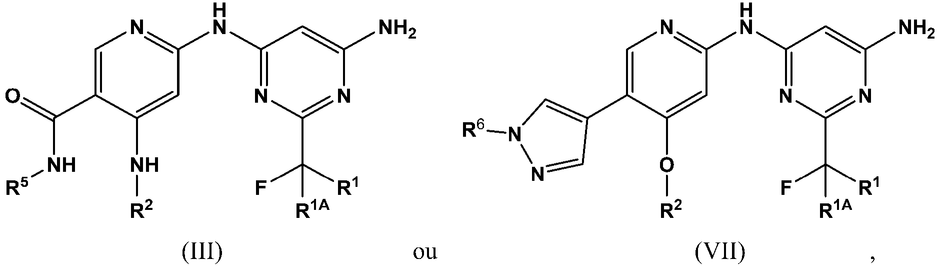 ou un de ses sels pharmaceutiquement acceptables, dans lequel :
R¹ et R^{1A} sont choisis chacun indépendamment parmi H, F et CH₃ ;
R² est choisi parmi un groupe alkyle en C₁₋₃, CD₃, cycloalkyle en C₃₋₄ et un hétérocycle à 4 chaînons contenant O, ledit groupe alkyle étant éventuellement substitué par 1 à 3 halo ;
R⁵ est choisi parmi CH₃, CH₂CH₃ et un groupe cyclopropyle ;
R⁶ est choisi parmi un groupe alkyle en C₁₋₅, CHF₂, CF₃et un hétérocycle à 4 ou 5 chaînons contenant de l'oxygène ou de l'azote, ledit groupe alkyle ou hétérocycle étant éventuellement substitué par R⁸;
R⁸ est choisi parmi OH, NR⁹R⁹, OCH₃, CH₃ et un hétérocycle à 4 chaînons contenant O ou N ; et
chaque R⁹ est choisi indépendamment parmi H et CH₃ ;
éventuellement R² étant choisi parmi CH₃, CHF₂, CF₃, CD₃, CH₂CH₃, CH₂CF₃, CH₂CH₂CH₃, CH(CH₃)₂, CH(CH₃)CH₂F, CH(CH₃)CHF₂, le cyclobutyle, le cyclopropyle et

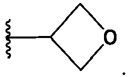

8. Composé selon la revendication 7, ou un de ses sels pharmaceutiquement acceptables, dans lequel R⁶ est choisi parmi CH₃, CH₂CH₃, CH₂CH₂CH₃, CH₂CH(CH₃)₂, CH₂CH₂CH(CH₃)₂, CHF₂, azétidinyle, oxétanyle, tétrahydrofuranyle et pyrrolidinyle, chacun d'entre eux étant éventuellement substitué par un R⁸ ; et/ou
dans lequel R⁶ est choisi parmi 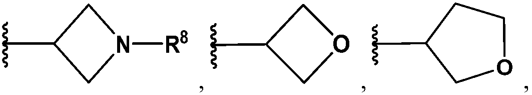 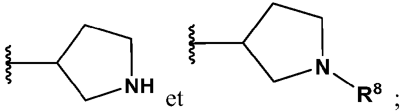 et/ou
dans lequel R⁸ est choisi parmi OH, OCH₃, NH₂, NHCH₃, N(CH₃)₂, CH₃, 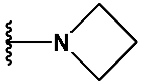 et

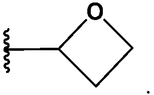

9. Composé selon la revendication 1, choisi parmi le groupe constitué des composés figurant dans le tableau ci-dessous, ou un de ses sels pharmaceutiquement acceptables.

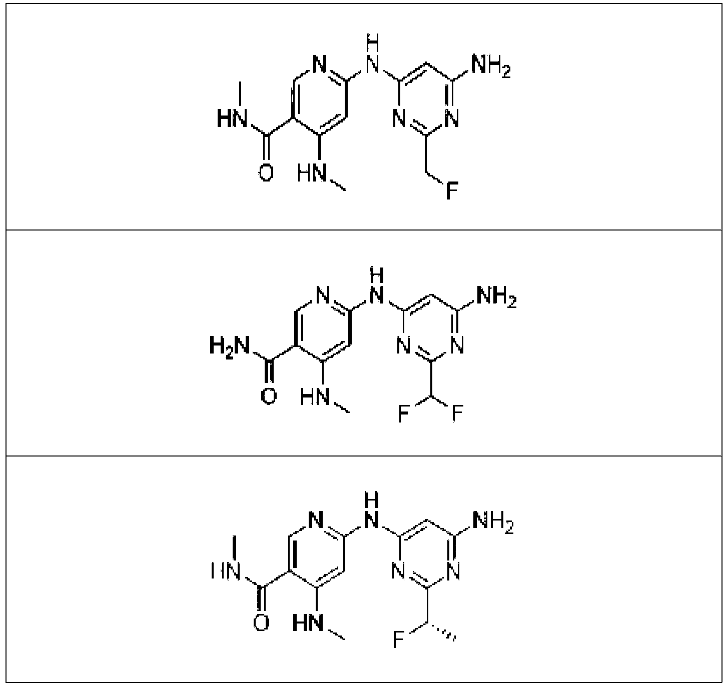

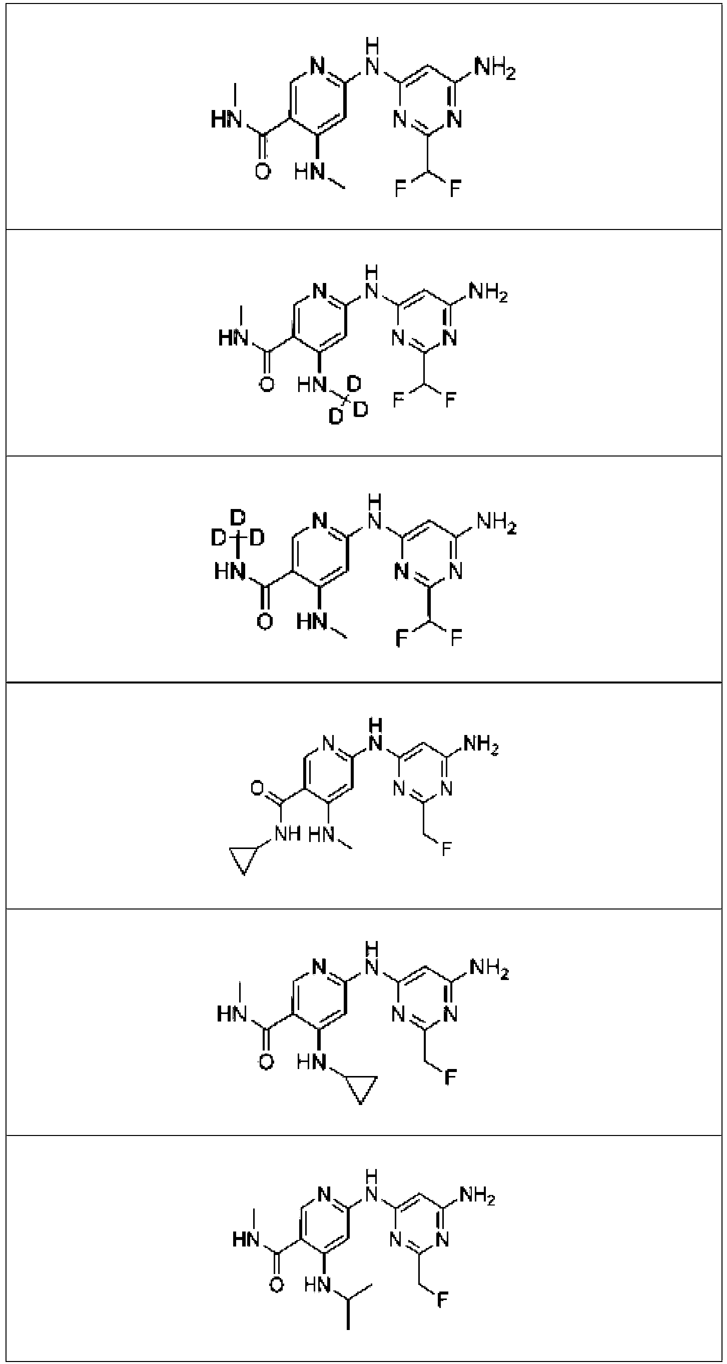

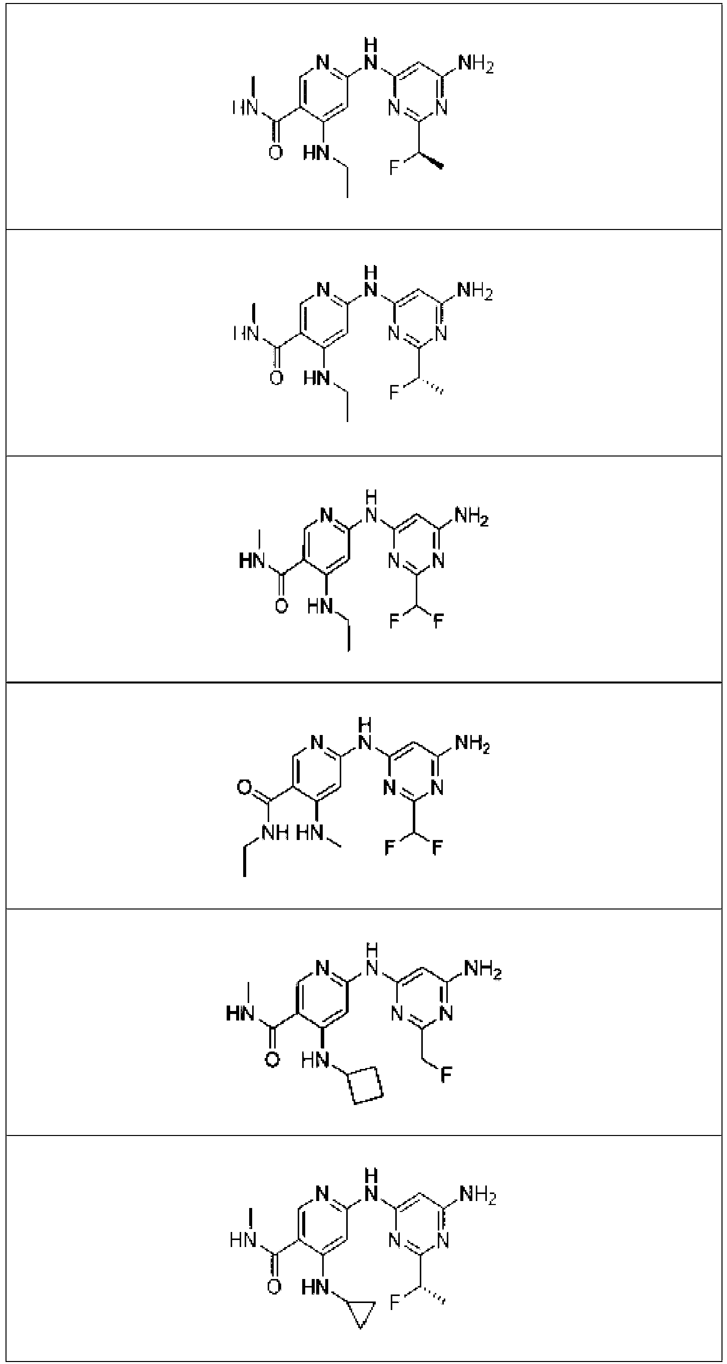

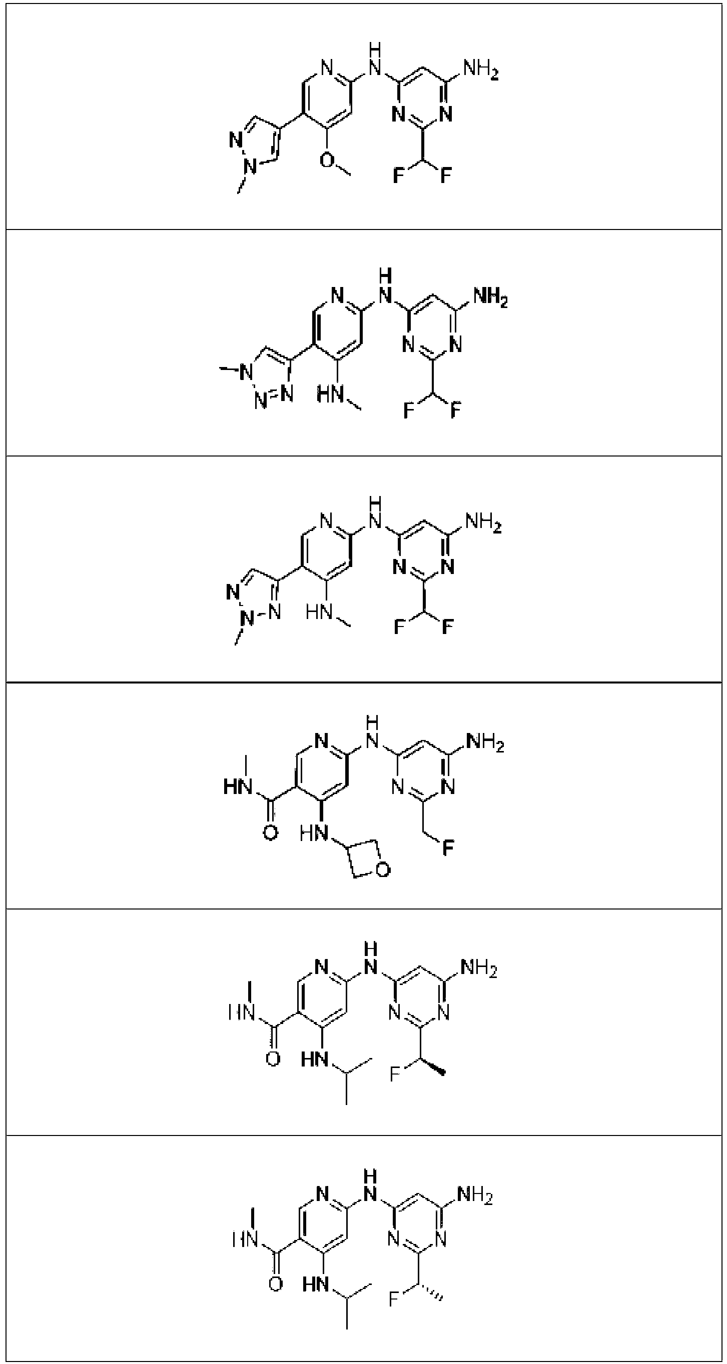

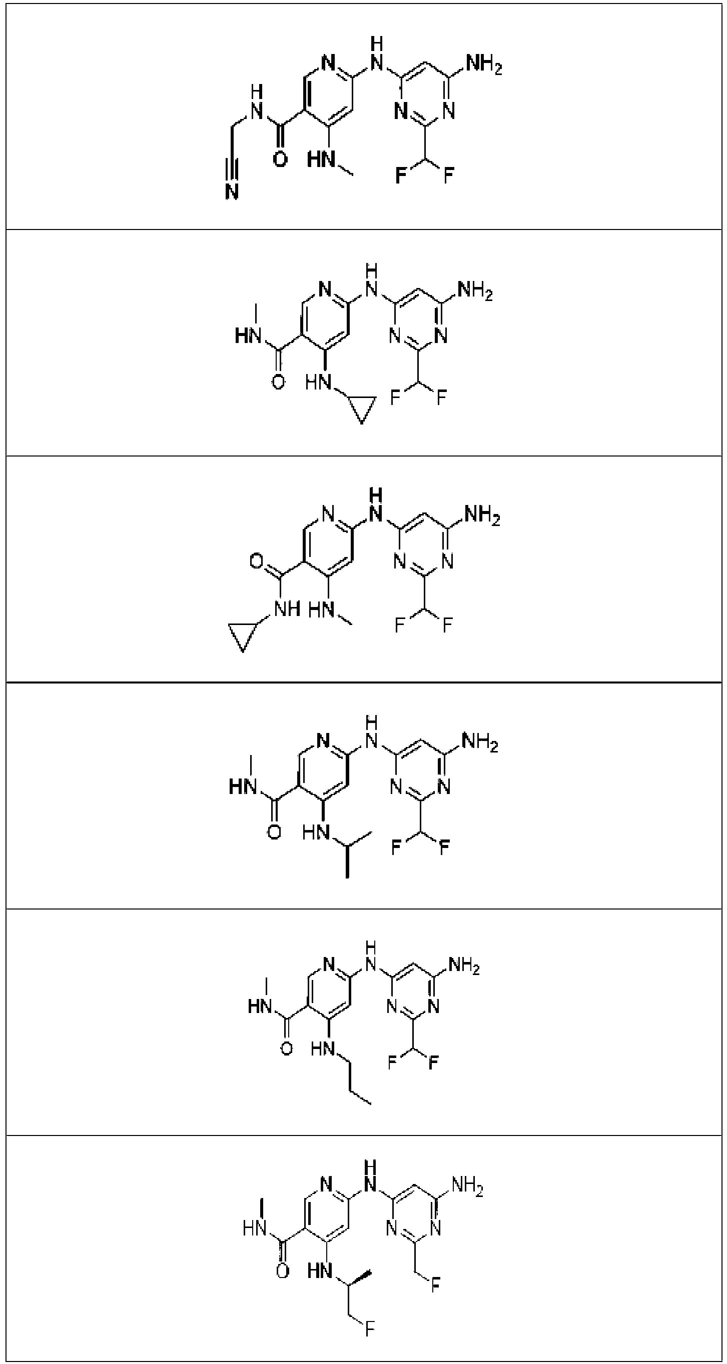

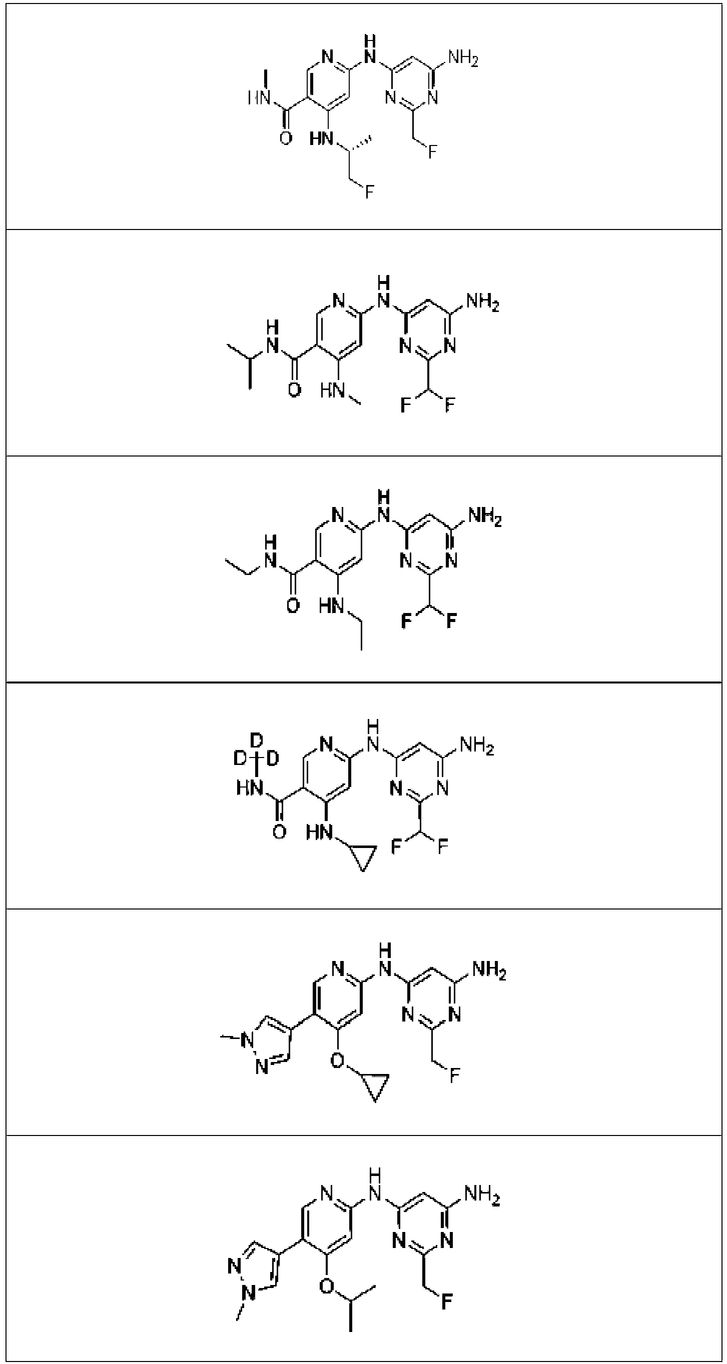

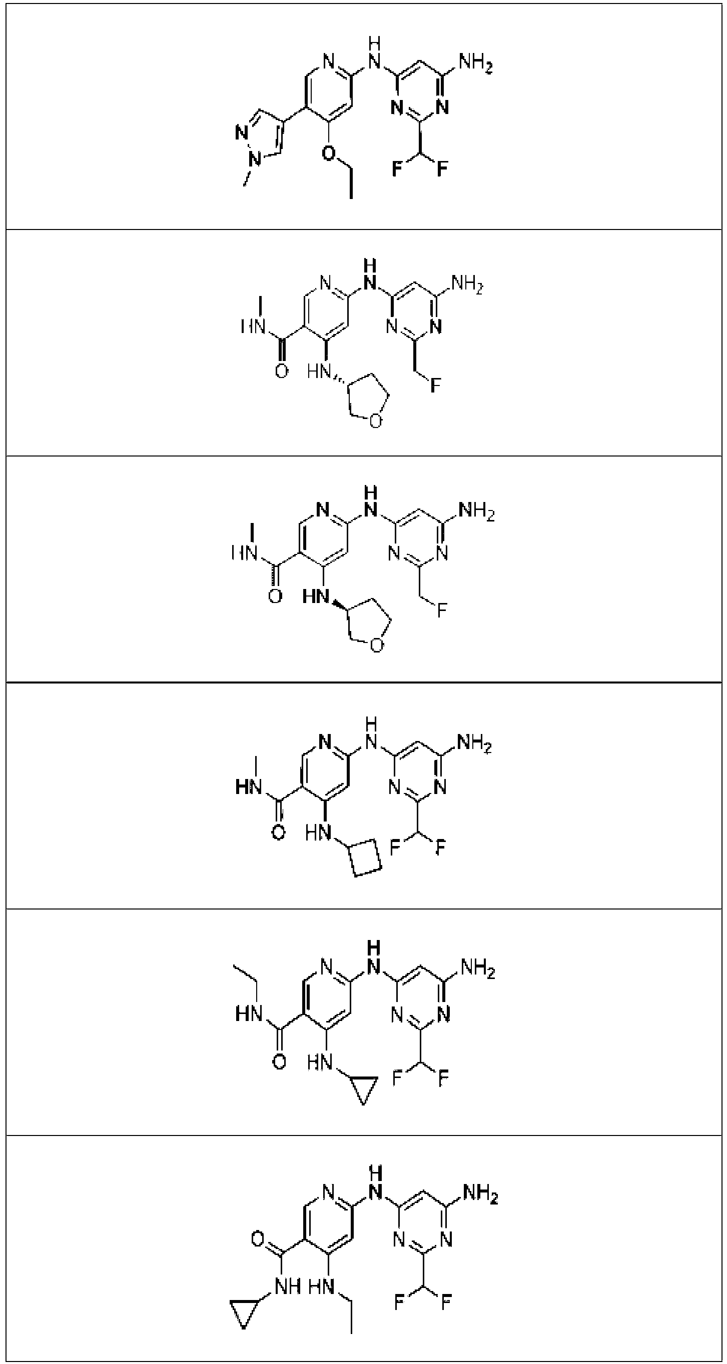

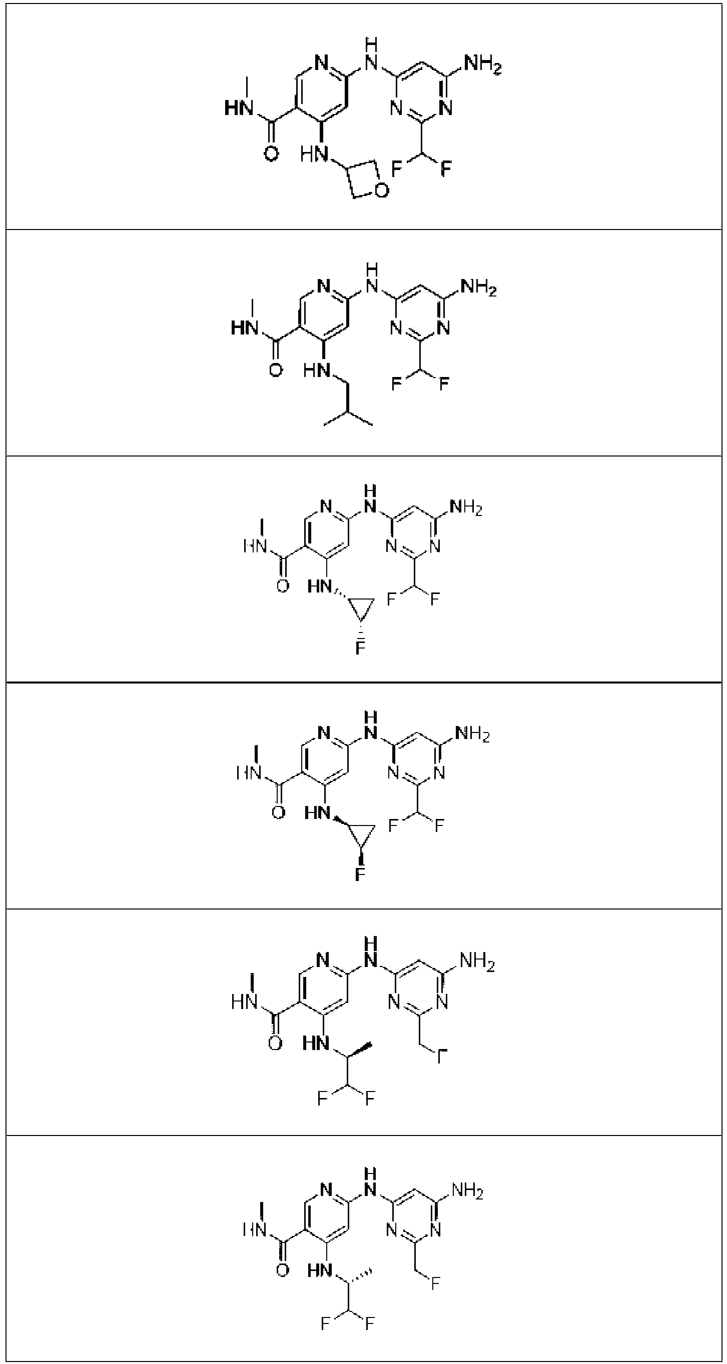

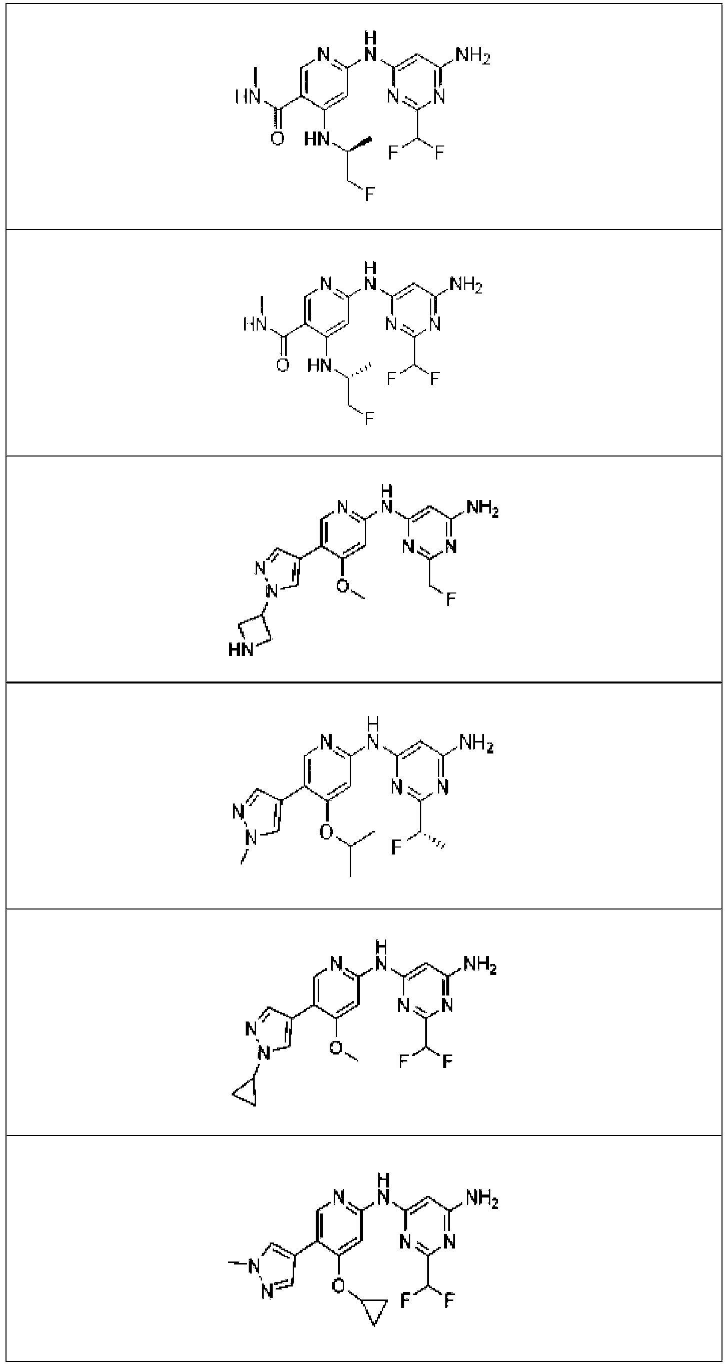

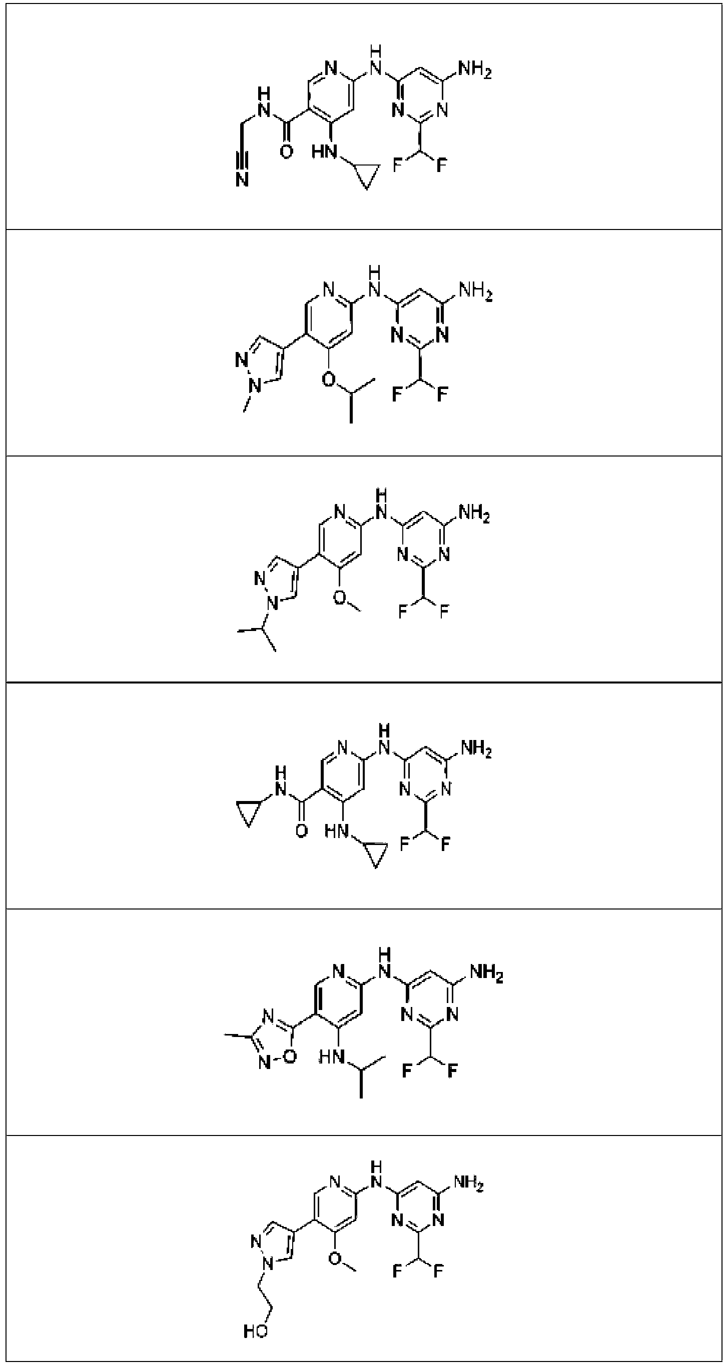

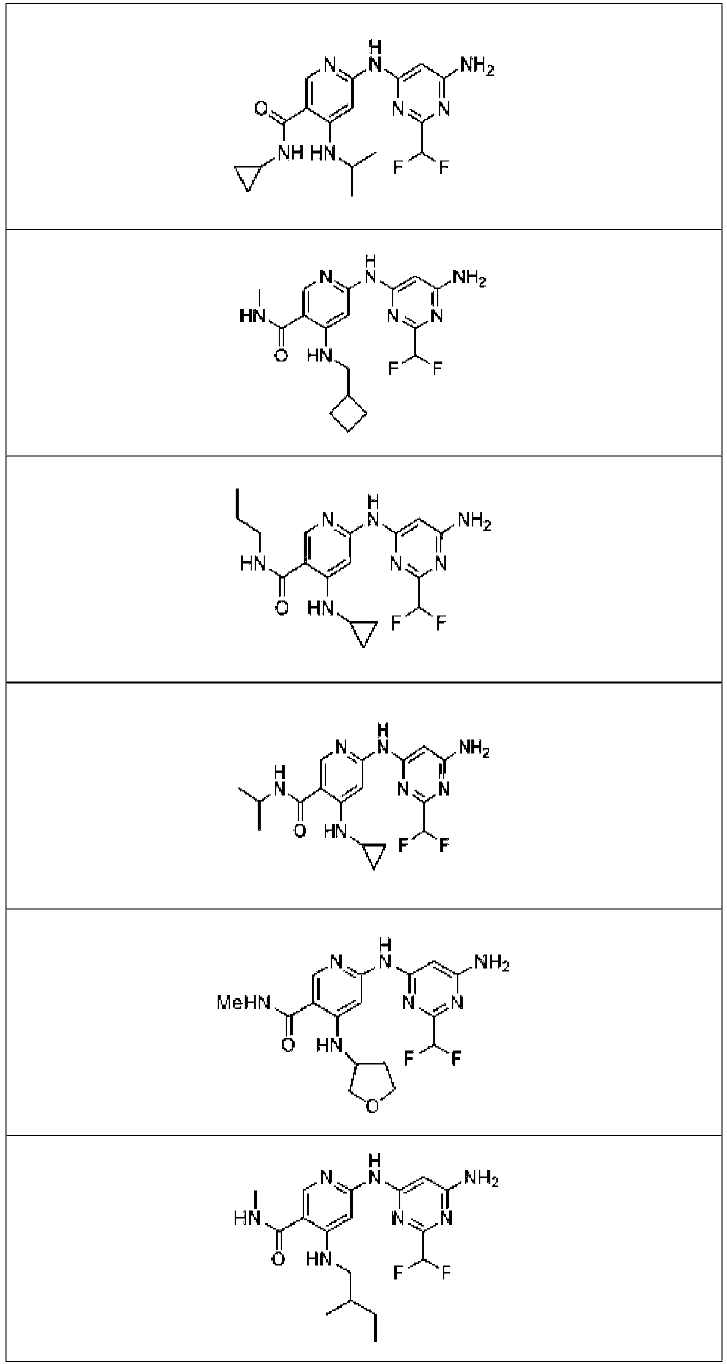

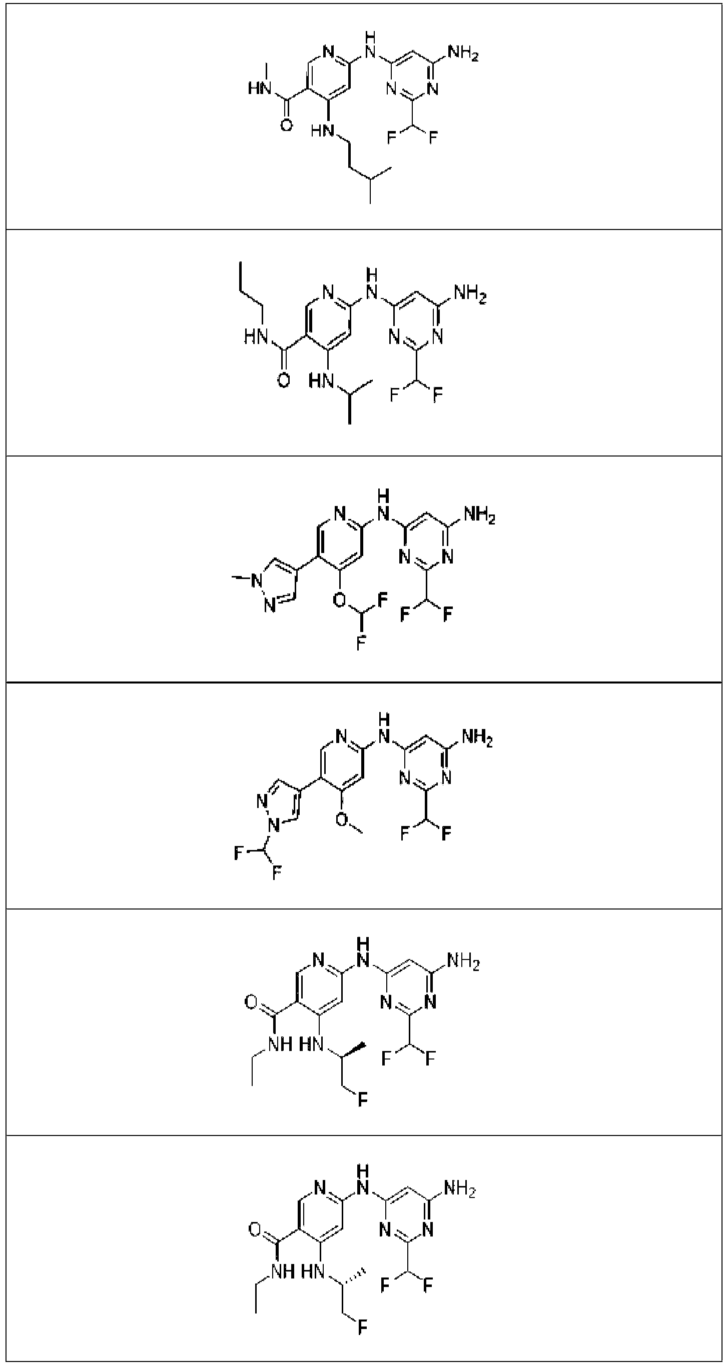

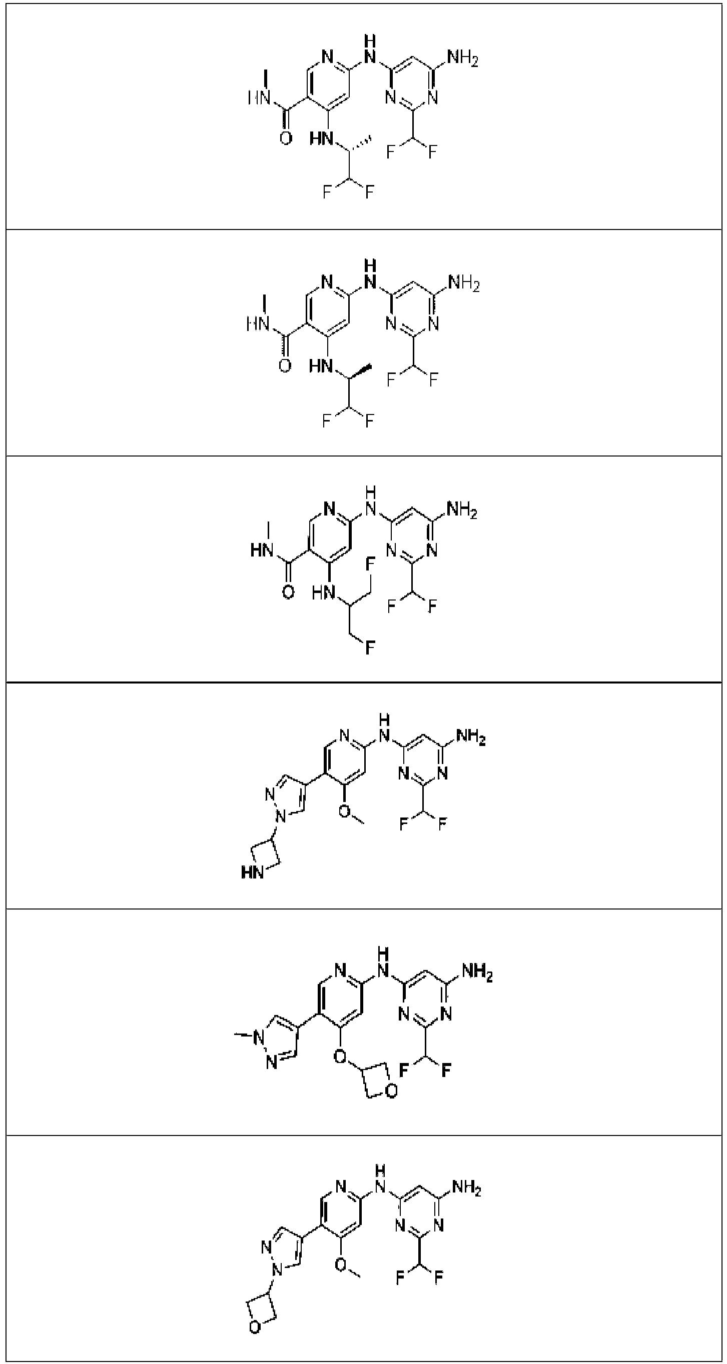

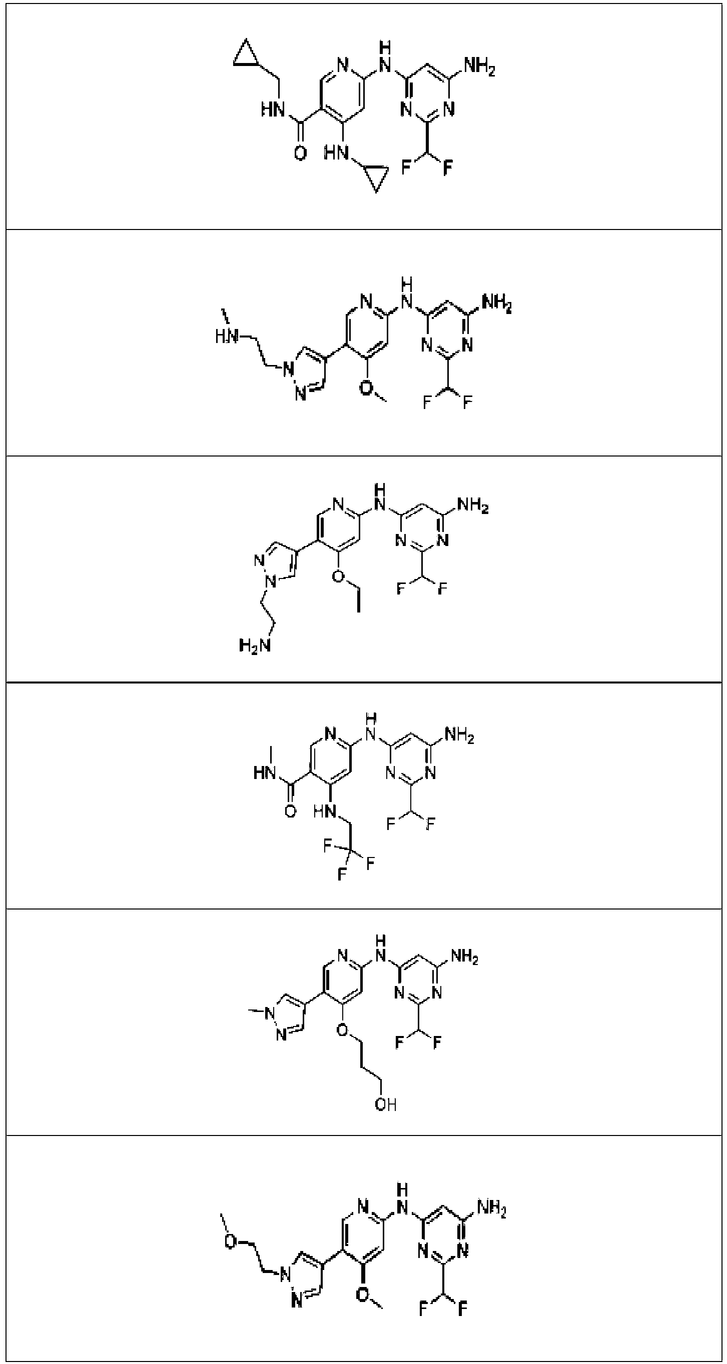

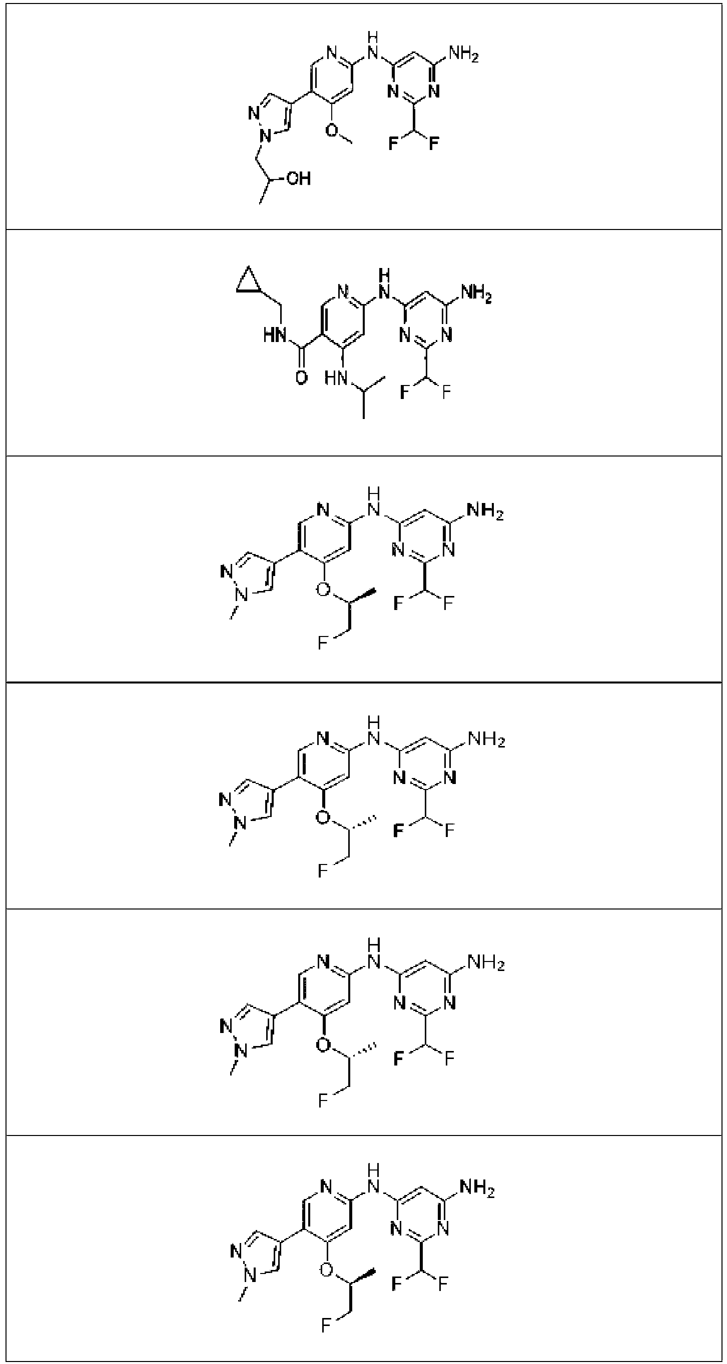

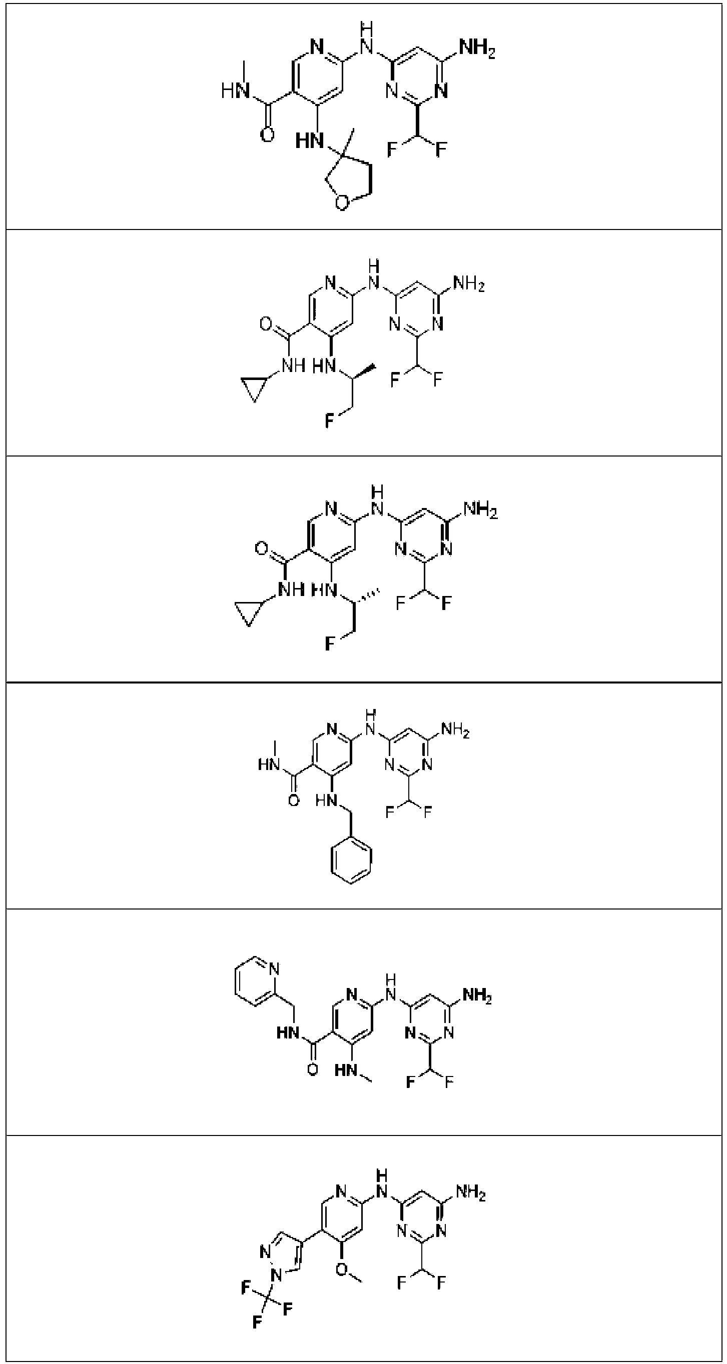

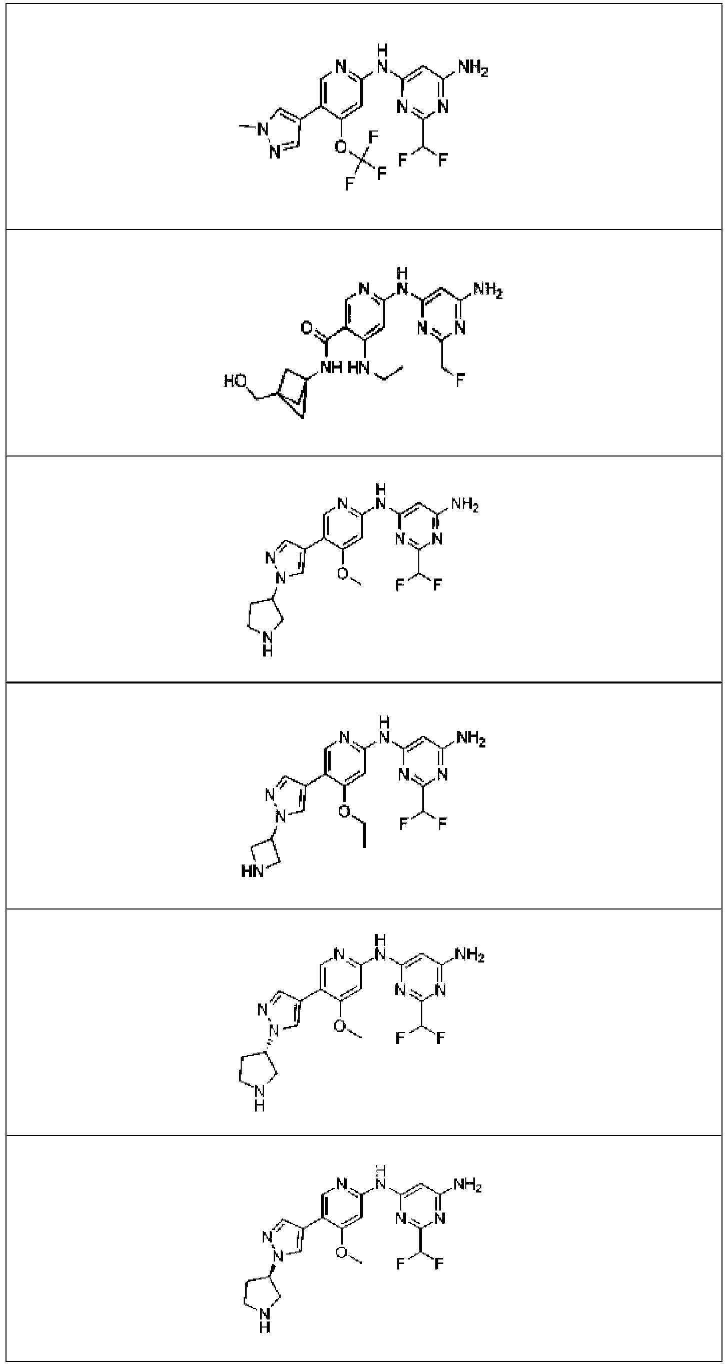

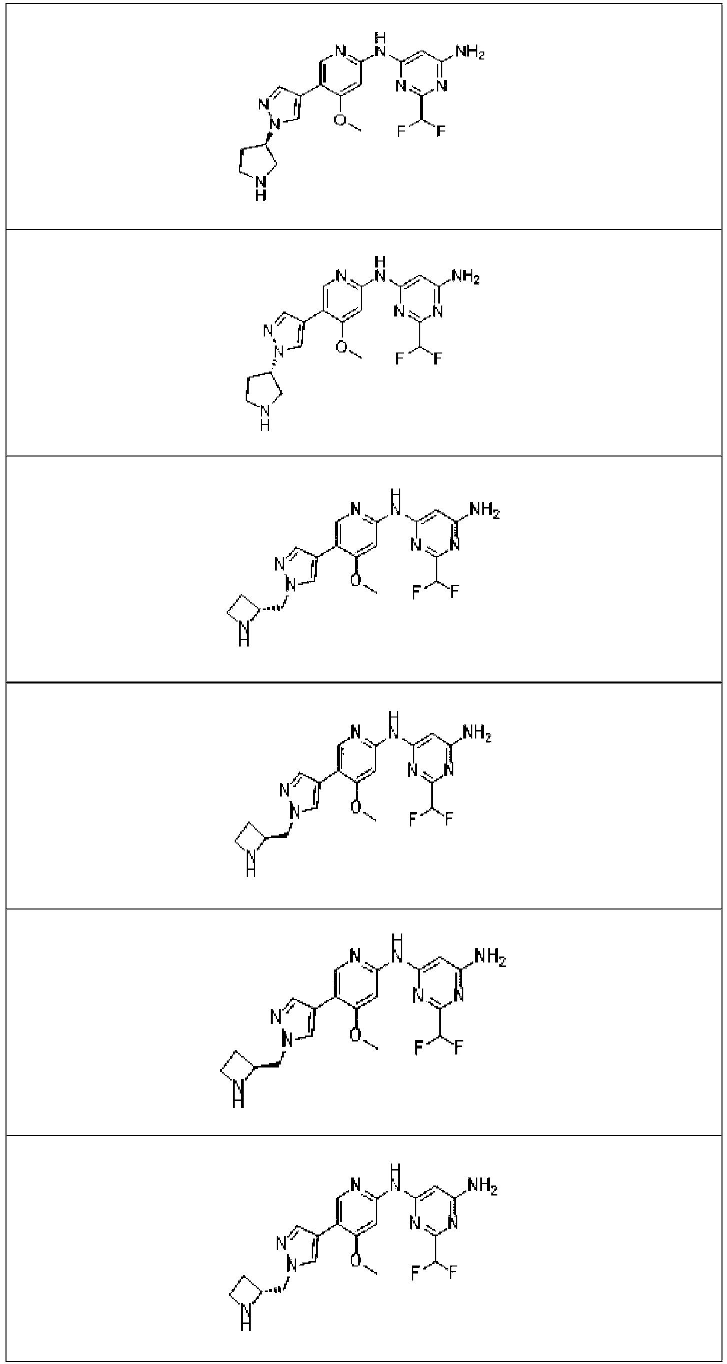

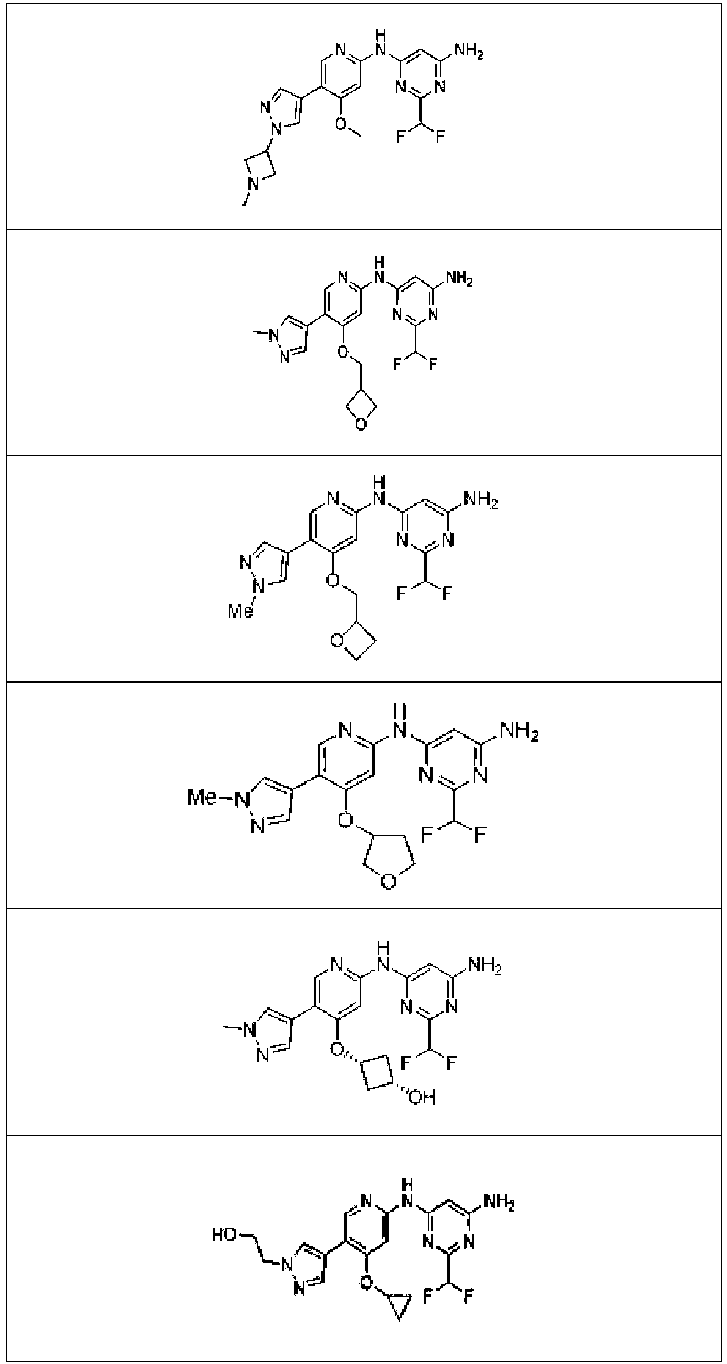

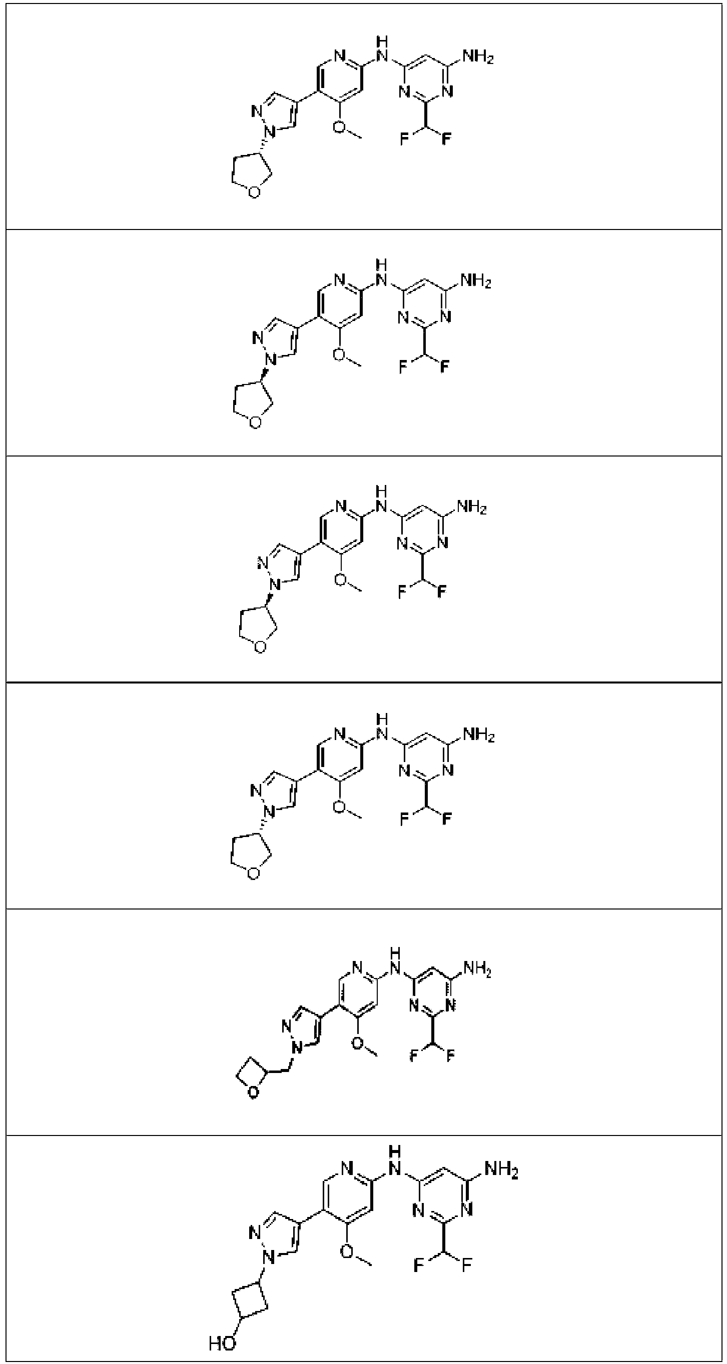

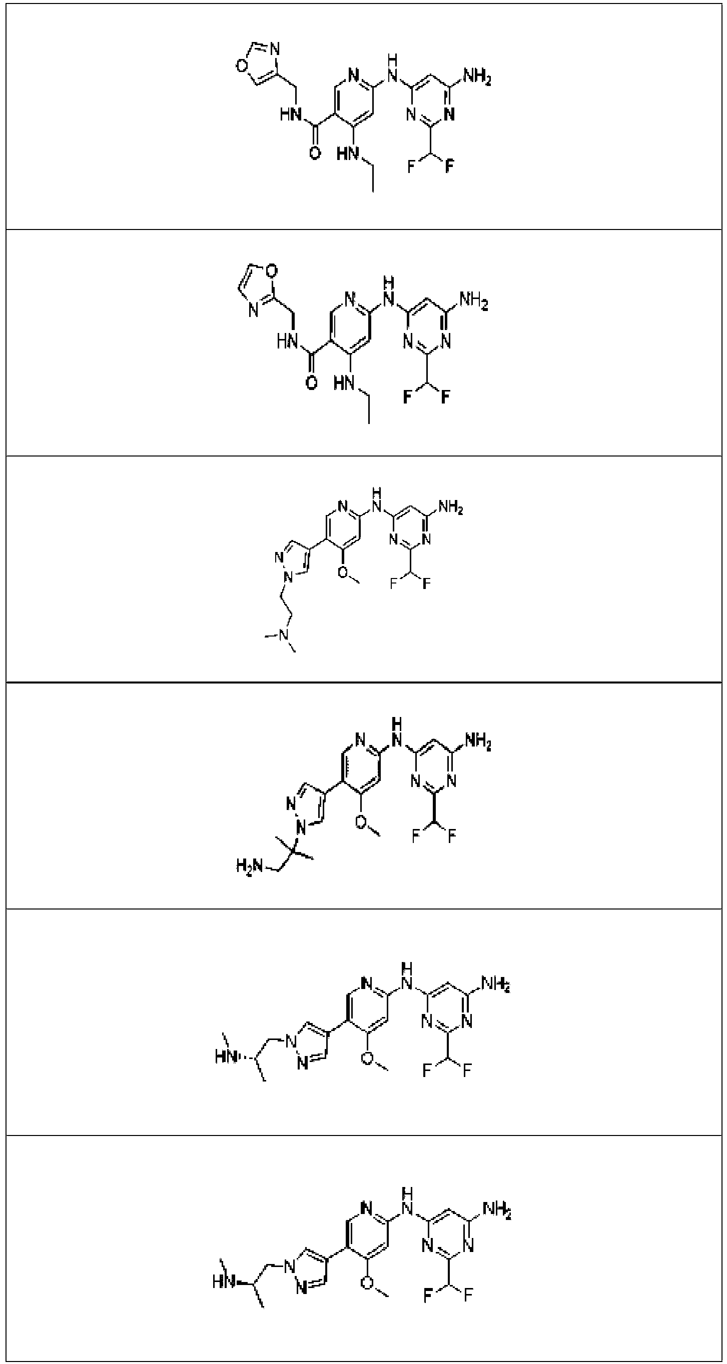

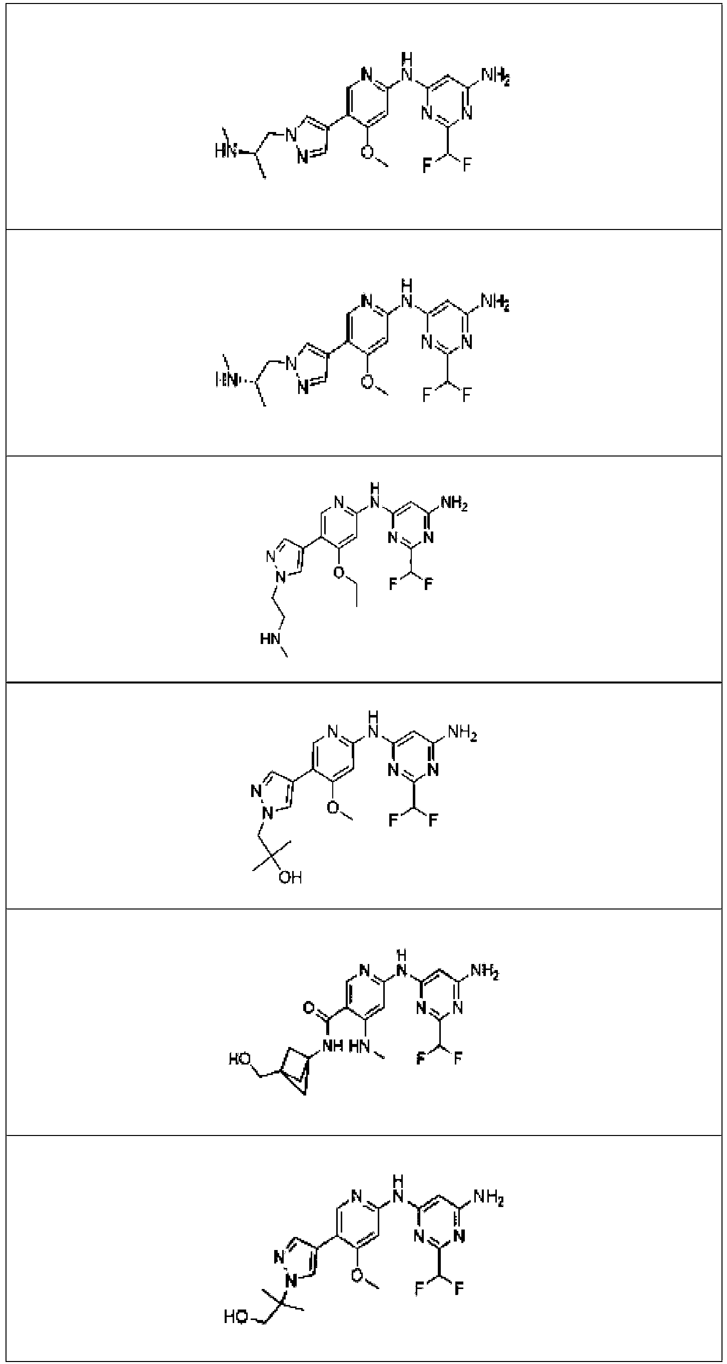

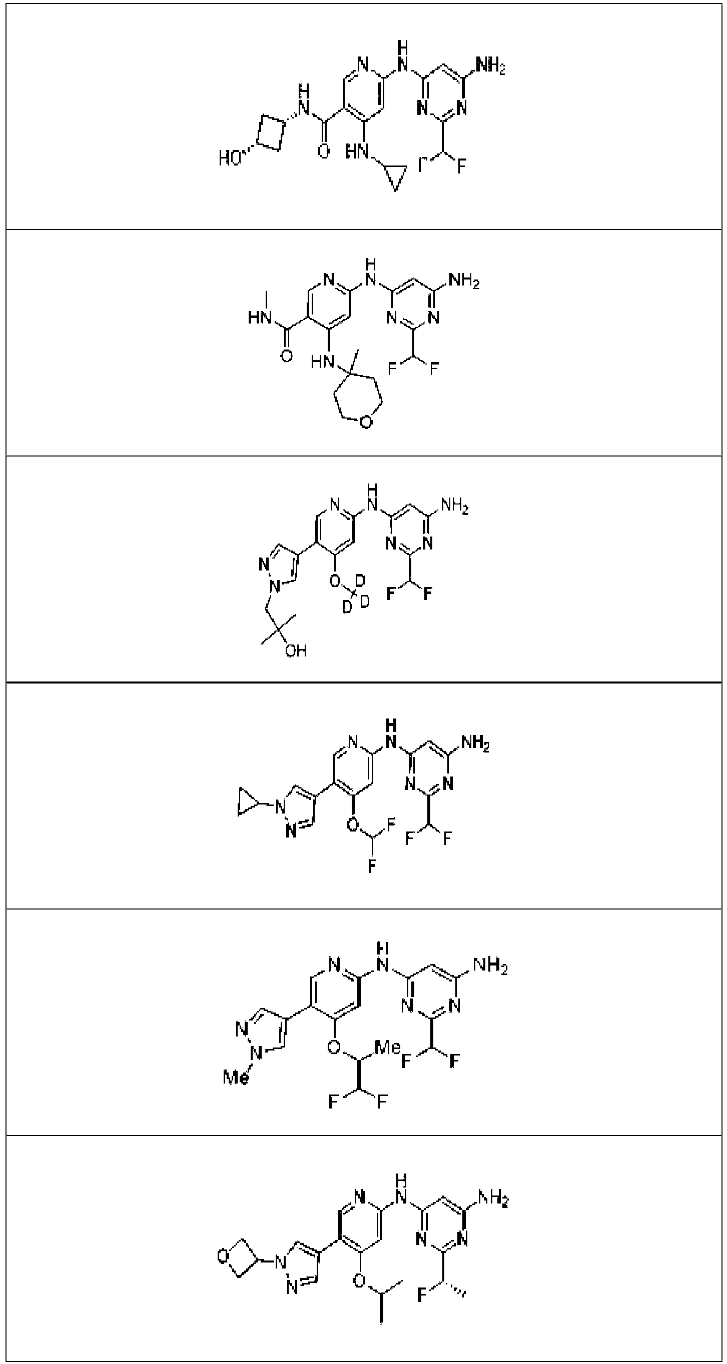

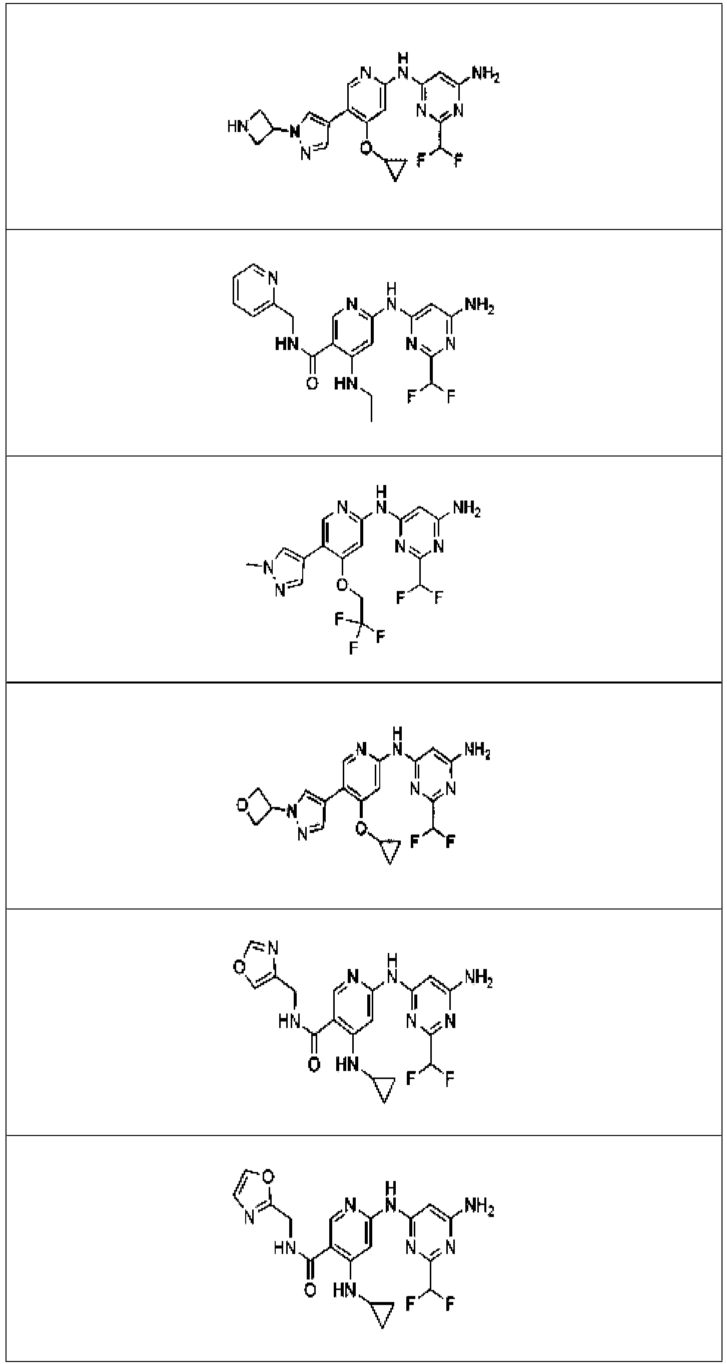

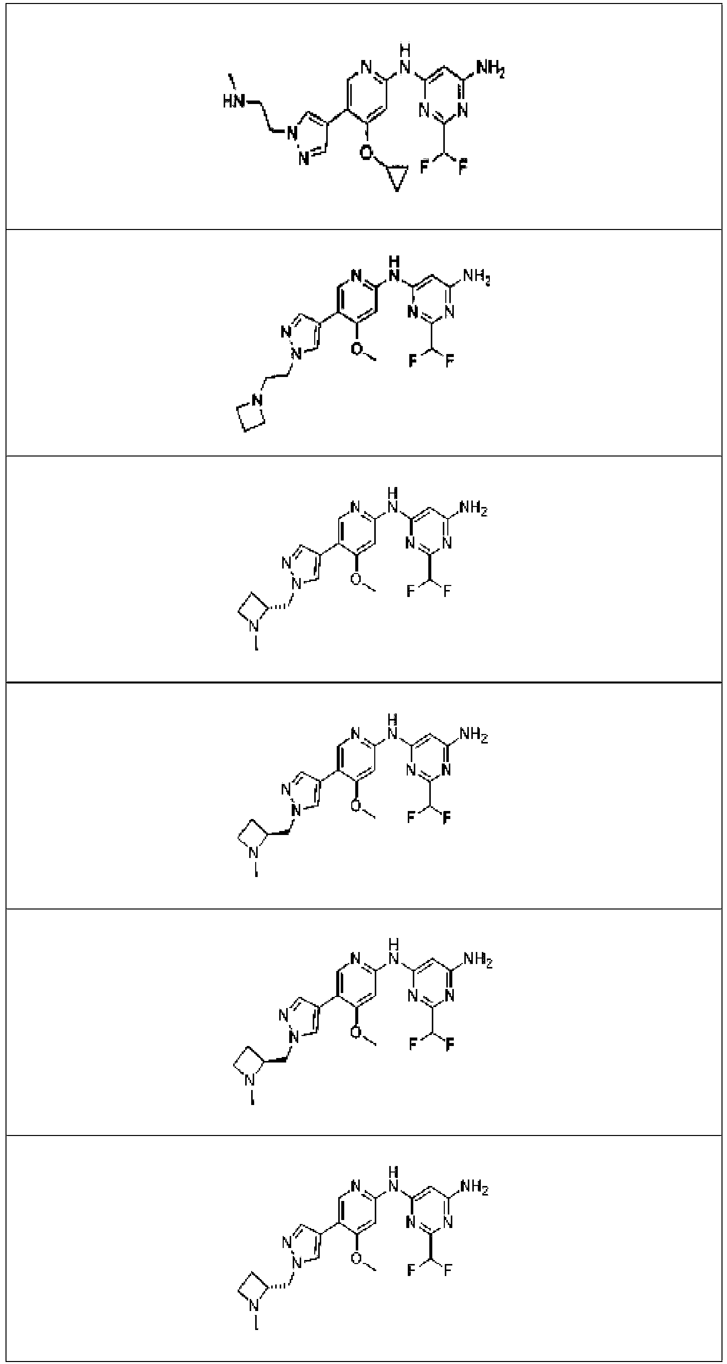

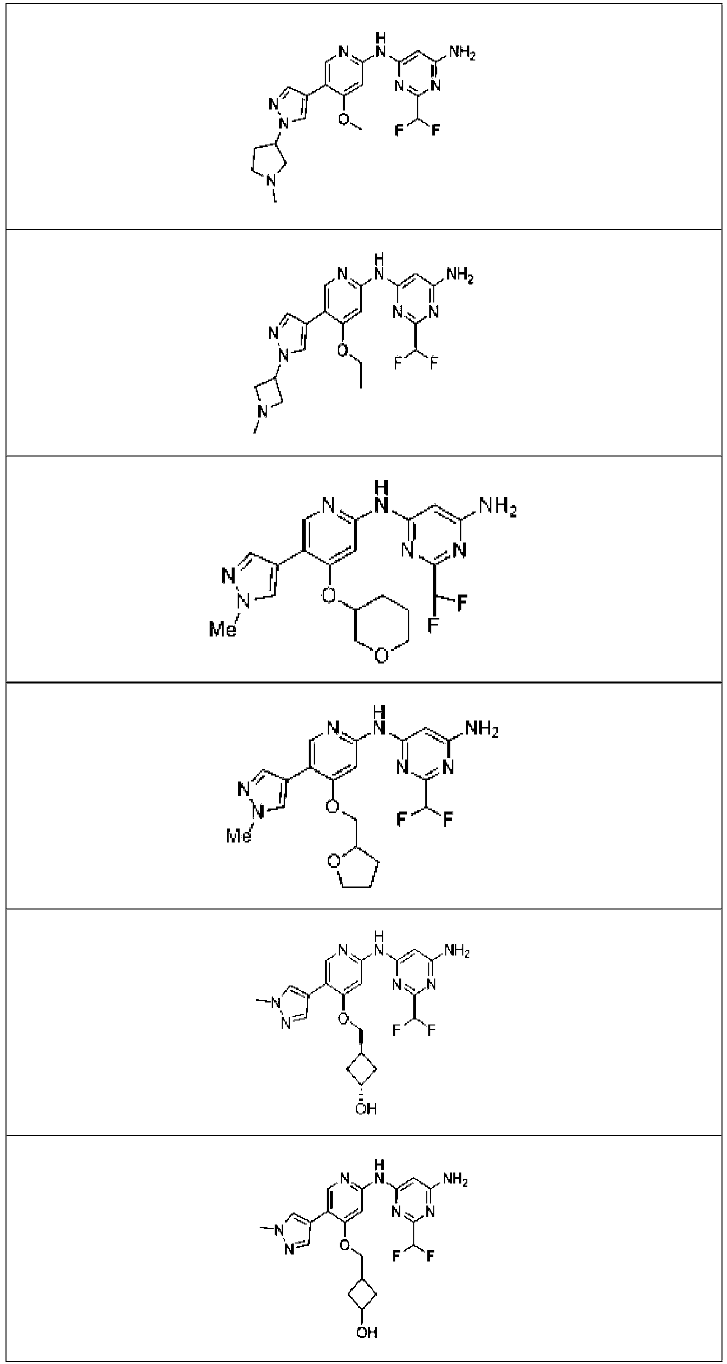

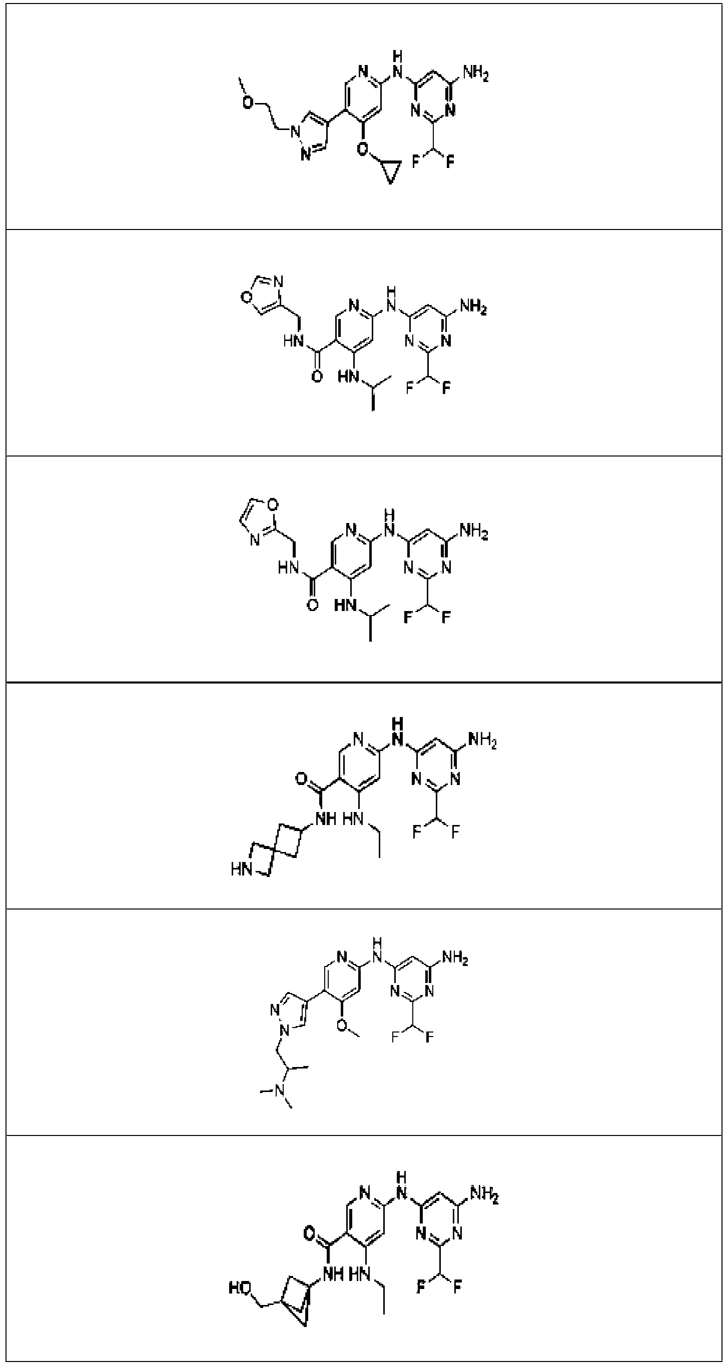

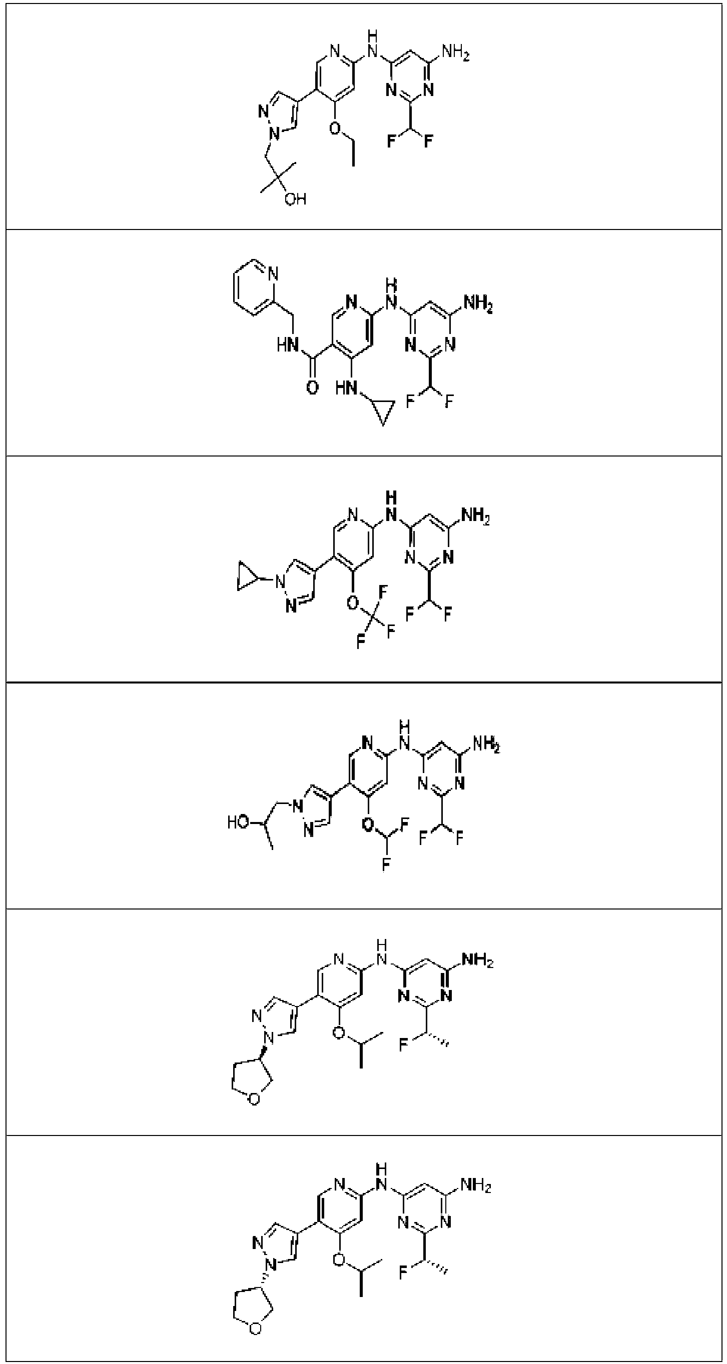

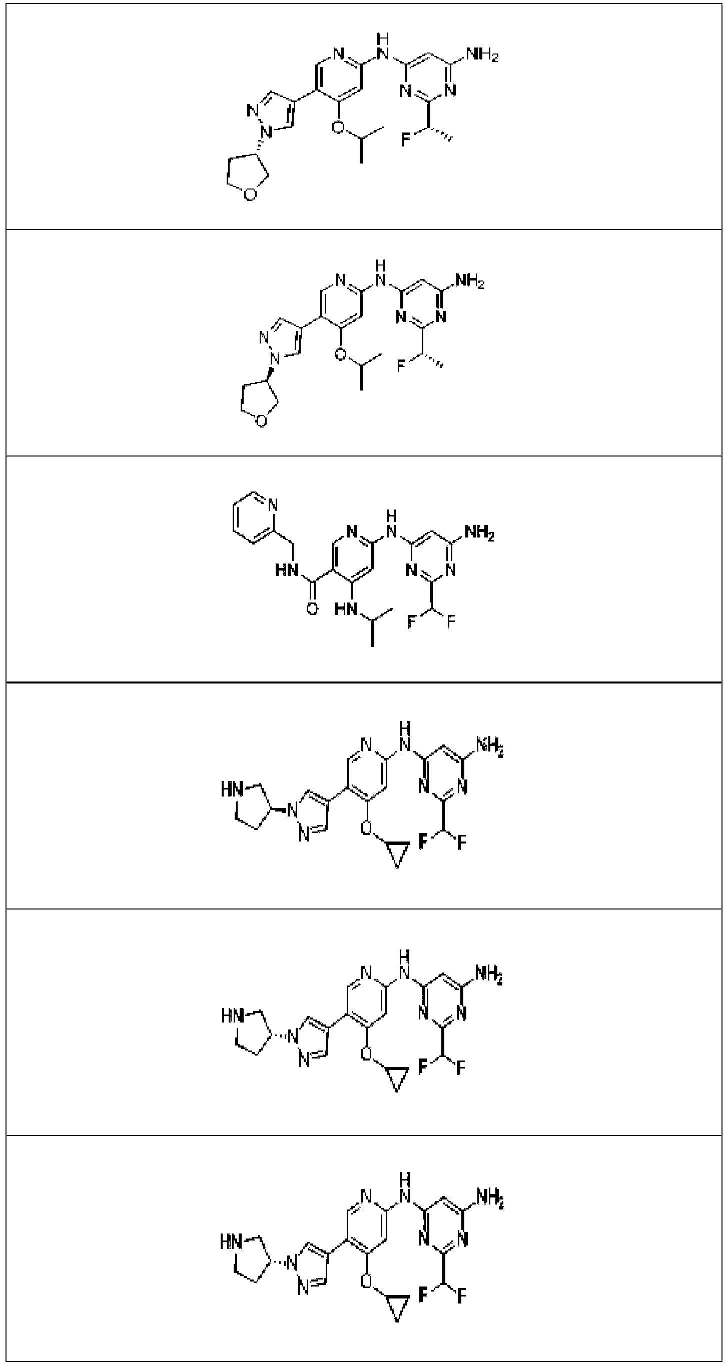

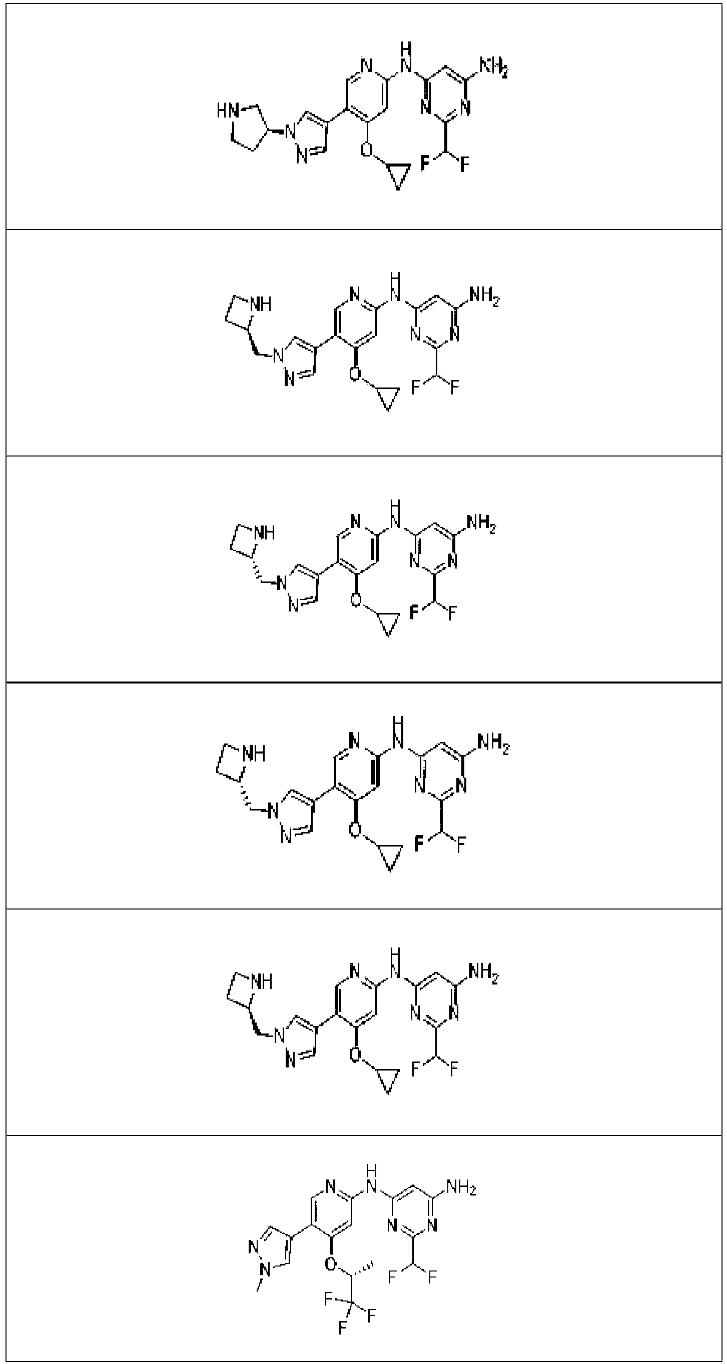

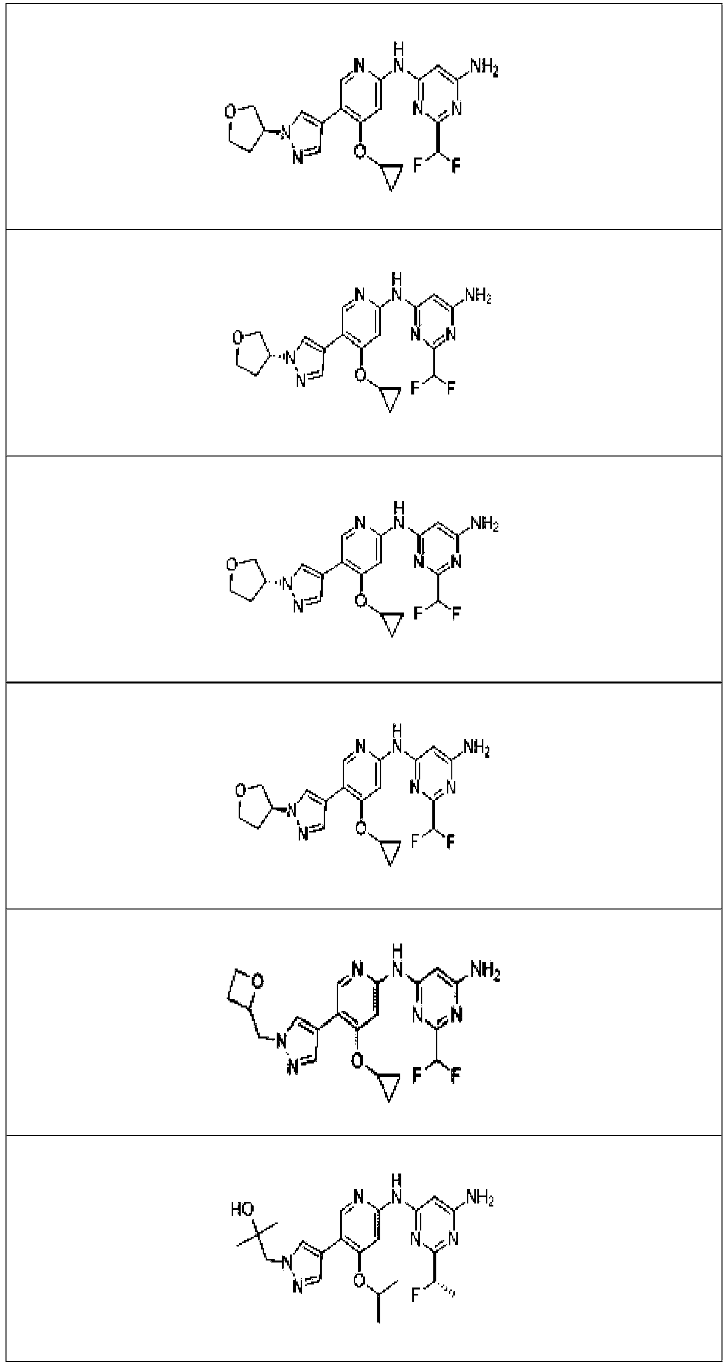

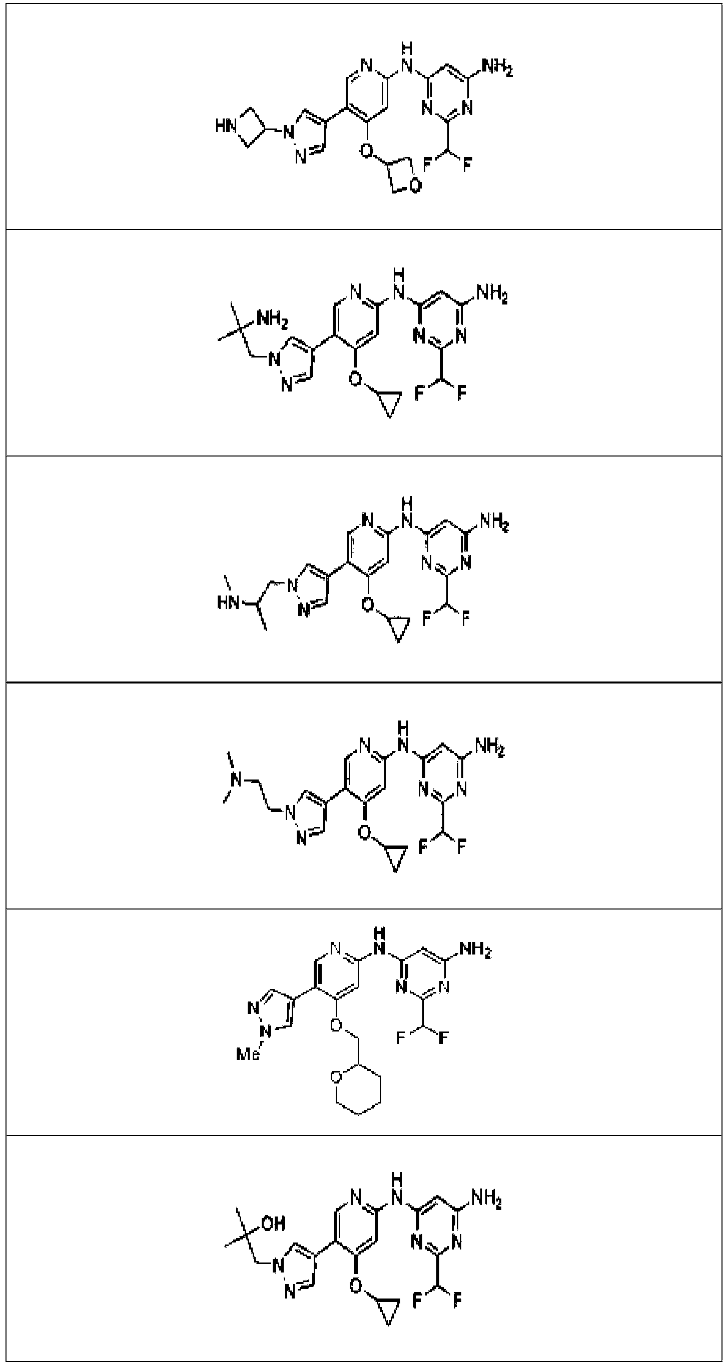

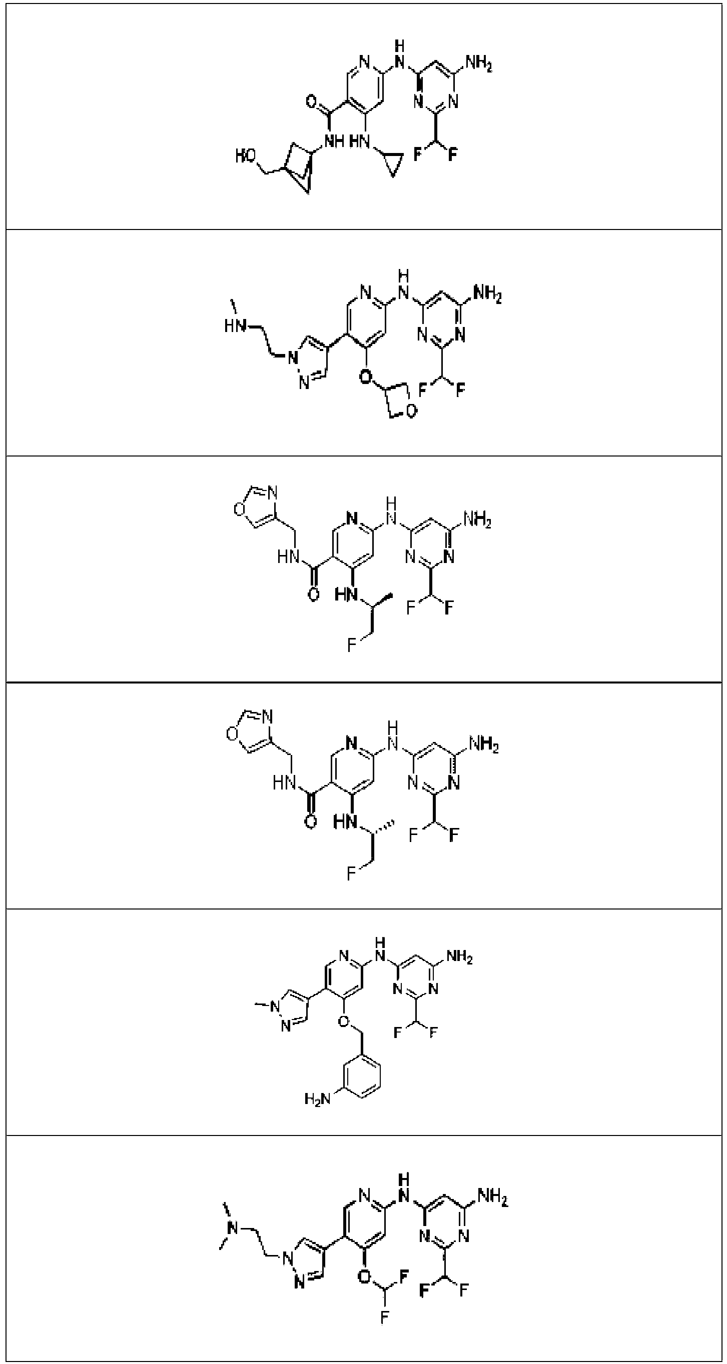

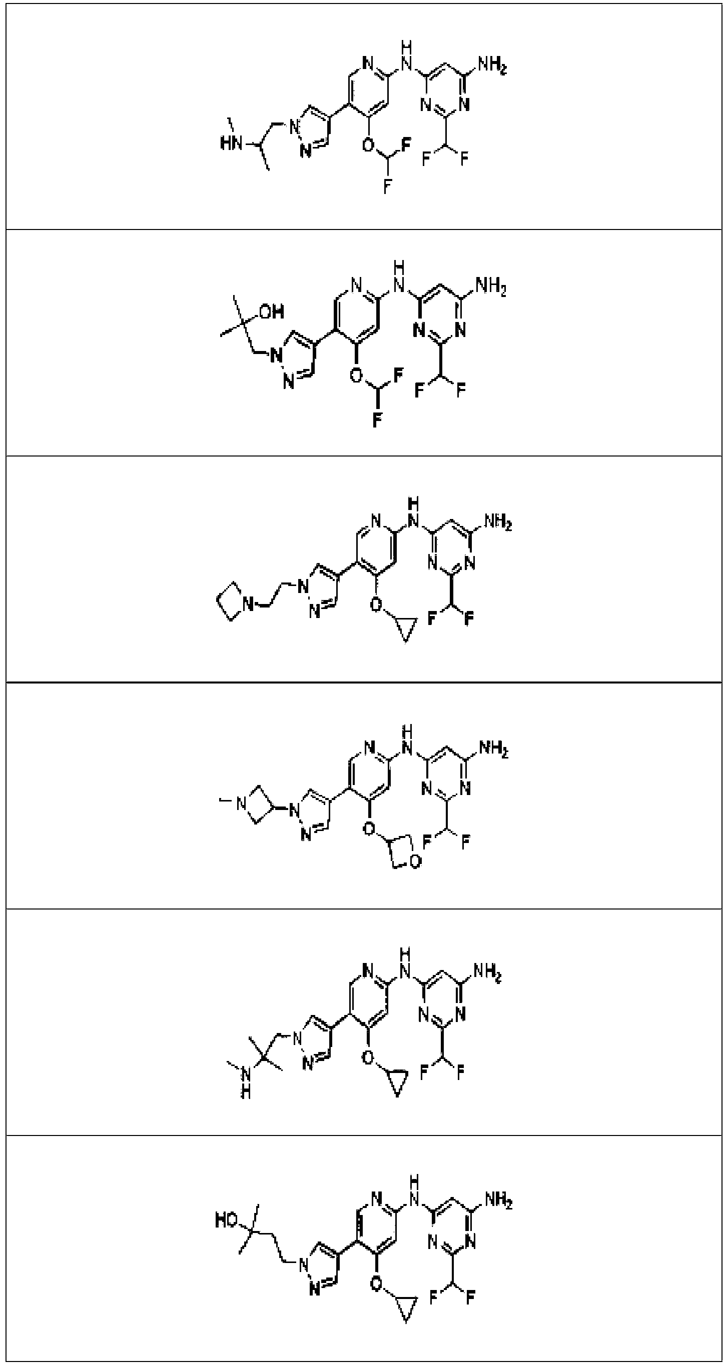

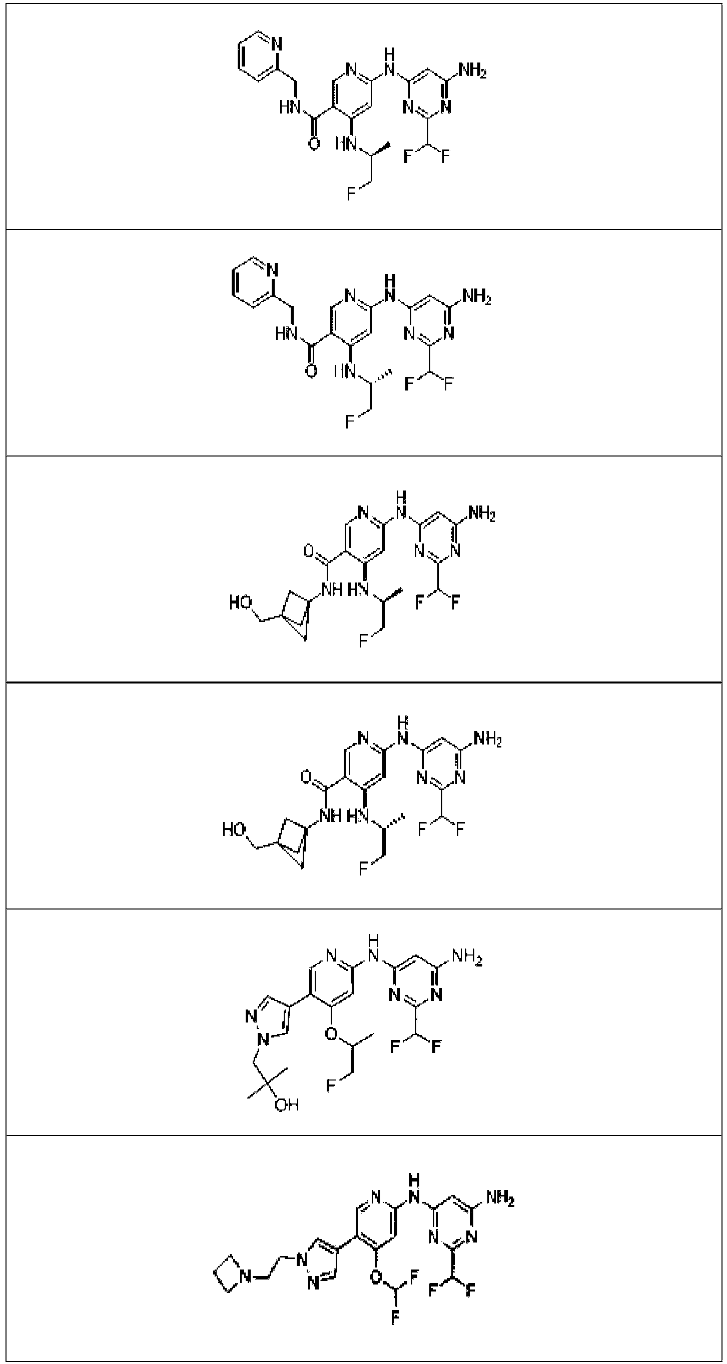

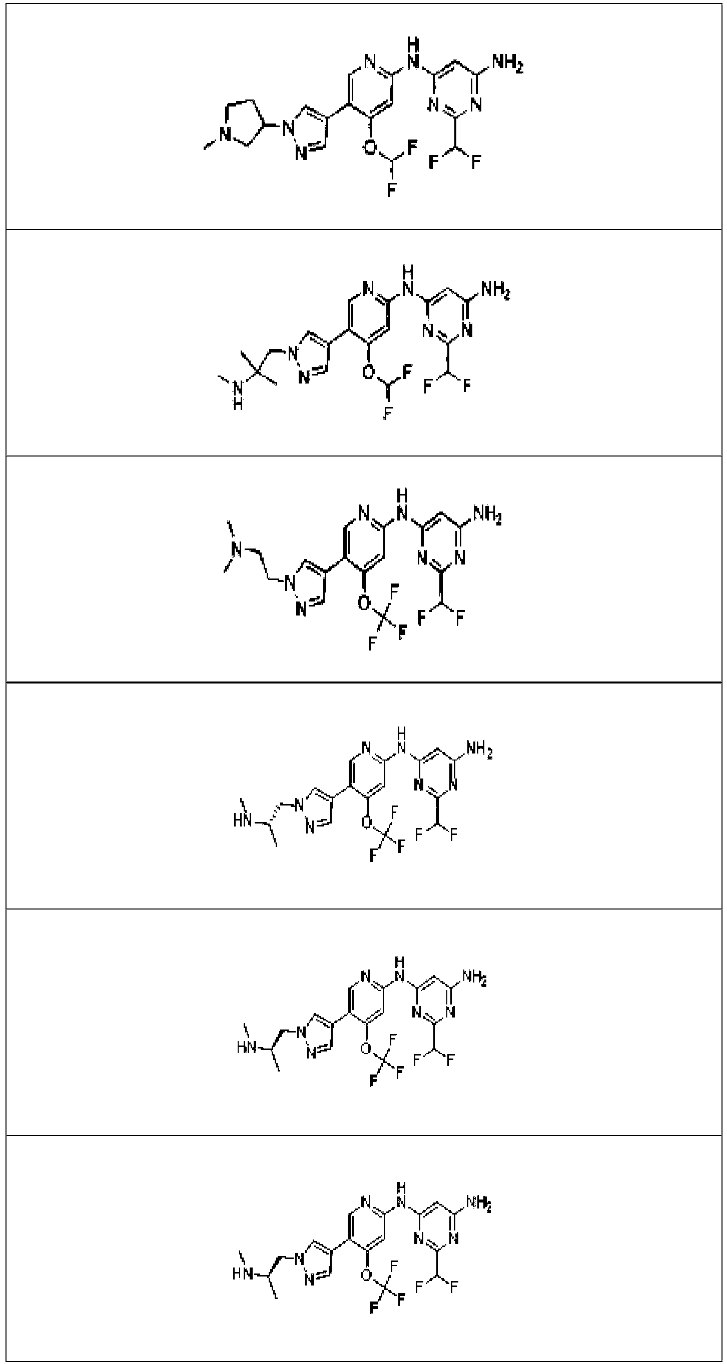

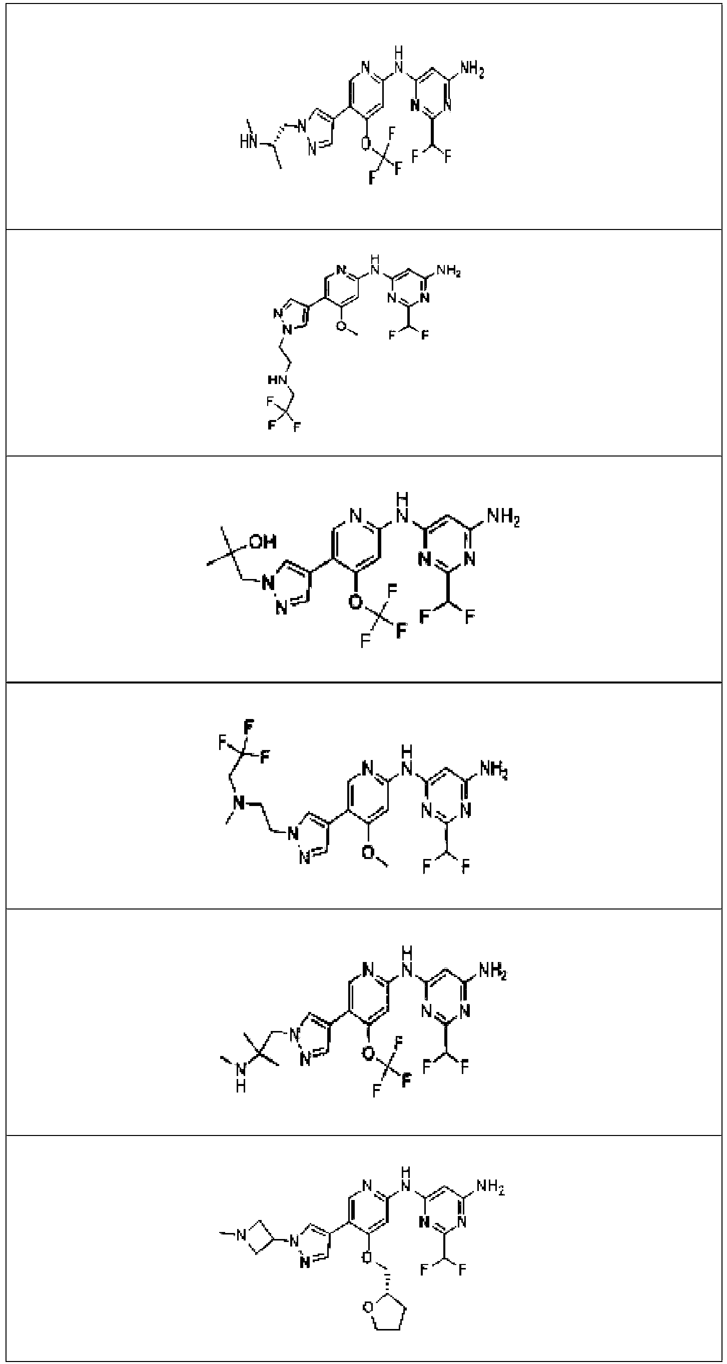

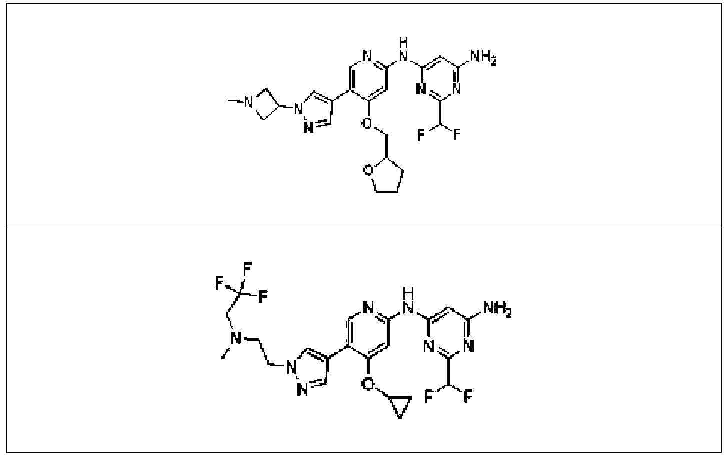

10. Composé selon la revendication 9, **caractérisé en ce que** ledit composé est choisi parmi le groupe constitué de 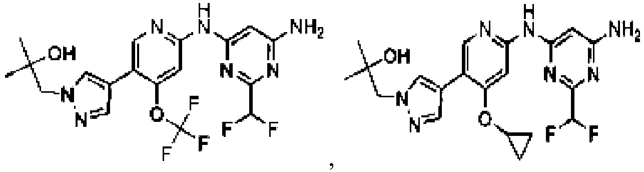 et 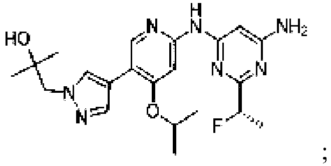 ou un sel pharmaceutiquement acceptable de celui-ci.

11. Composition pharmaceutique comprenant le composé selon l'une quelconque des revendications 1 à 10 ou un sel pharmaceutiquement acceptable de celui-ci, et un support ou excipient pharmaceutiquement acceptable.

12. Le composé selon l'une quelconque des revendications 1 à 10 ou la composition pharmaceutique selon la revendication 11, pour son utilisation dans le traitement d'un patient souffrant d'une tumeur stromale gastro-intestinale (GIST), comprenant l'administration au patient d'une quantité efficace du composé selon l'une quelconque des revendications 1 à 10, ou d'un sel pharmaceutiquement acceptable de celui-ci, ou de la composition pharmaceutique selon la revendication 11.

13. Le composé ou la composition pharmaceutique pour son utilisation selon la revendication 12, dans lequel le patient a reçu un ou plusieurs traitements antérieurs pour la GIST ; éventuellement dans lequel la GIST a progressé après le traitement antérieur et éventuellement dans lequel le traitement antérieur consiste à administrer un ou plusieurs agents, éventuellement dans lequel le traitement antérieur consiste à administrer un ou plusieurs agents, chacun étant choisi indépendamment parmi : l'imatinib, le sunitinib, le regorafenib, le nilotinib, le cabozantinib, le pazopanib, le ponatinib, le dasatinib, le binimetinib, le vandétanib, famitinib, anlotinib, axitinib, alvocidib, ribocliclib, sorafenib, pexidartinib, olaratumab, crenolanib, avapritinib, ripretinib, bezuclastinib, AZD3229, BLU-263, everolimus et larotrectinib, et leurs sels pharmaceutiquement acceptables ; ou
dans lequel le GIST est **caractérisé par** une tumeur présentant une ou plusieurs mutations KIT, éventuellement dans lequel la tumeur présente une mutation activatrice primaire KIT et/ou dans lequel le GIST est **caractérisé par** une tumeur présentant une ou plusieurs mutations KIT, chacune étant choisie indépendamment parmi une mutation KIT au niveau de l'exon 9, une mutation KIT au niveau de l'exon 11, une mutation KIT au niveau de l'exon 13 et une mutation KIT au niveau de l'exon 17, ainsi que des combinaisons de celles-ci, dans lequel, éventuellement, chacune desdites une ou plusieurs mutations KIT est choisie indépendamment parmi N822K, D816V, D816E, D816F, D816H, D816I, D816Y, D820E, D820Y, D823D, A829P, K642E, V654A et N655K, et leurs combinaisons ; ou
dans laquelle chacune des une ou plusieurs mutations KIT est choisie indépendamment parmi N822K, D816V, D816E, D816F, D816H, D8161, D816Y, D820E, D820Y, D823D, A829P et K642E, et leurs combinaisons ; ou
dans laquelle la tumeur présente une mutation KIT au niveau de l'exon 9 ; ou
dans laquelle la tumeur présente une mutation KIT au niveau de l'exon 11 ; ou
dans laquelle la tumeur présente une mutation KIT au niveau de l'exon 17, éventuellement dans laquelle la mutation KIT au niveau de l'exon 17 est choisie parmi N822K, D816V, D816E, D816F, D816H, D816I, D816Y, D820E, D820Y, D823D et A829P ; ou
dans lequel la tumeur présente une mutation KIT au niveau de l'exon 13, éventuellement dans lequel la mutation KIT au niveau de l'exon 13 est choisie parmi K642E, V654A et N655K, et leurs combinaisons ; et/ou
dans laquelle la tumeur présente une résistance par mutation à un ou plusieurs traitements antérieurs, éventuellement lorsque la tumeur présente une mutation de résistance à l'imatinib, éventuellement dans laquelle la mutation de résistance à l'imatinib est choisie parmi une mutation KIT au niveau de l'exon 13, une mutation KIT au niveau de l'exon 17 et une mutation KIT au niveau de l'exon 14, ainsi que leurs combinaisons.

14. Le composé selon l'une quelconque des revendications 1 à 10 ou la composition pharmaceutique selon la revendication 11 pour son utilisation dans un procédé de traitement d'un patient souffrant d'une maladie maligne **caractérisée par** une mutation KIT au niveau de l'exon 9, une mutation KIT au niveau de l'exon 11, une mutation KIT au niveau de l'exon 13 ou une mutation KIT au niveau de l'exon 17, ou une combinaison de celles-ci, comprenant l'administration au patient d'une quantité efficace du composé selon l'une quelconque des revendications 1 à 10, ou d'un sel pharmaceutiquement acceptable de celui-ci, ou de la composition pharmaceutique selon la revendication 12 ;
éventuellement, la maladie maligne étant choisie parmi la tumeur stromale gastro-intestinale (GIST), la LAM (leucémie aiguë myéloïde), le mélanome, le cancer du poumon, le cancer de l'utérus, l'astrocytome, le cancer du foie, le séminome, le carcinome à cellules rénales, la tumeur intracrânienne des cellules germinales, le cancer du pancréas et le lymphome médiastinal à cellules B.

15. Association pour son utilisation dans une méthode de traitement d'un patient souffrant d'une tumeur stromale gastro-intestinale (GIST), comprenant l'administration au patient d'une association efficace comprenant :
un composé selon l'une quelconque des revendications 1 à 10, ou un sel pharmaceutiquement acceptable de celui-ci, ou une composition pharmaceutique selon la revendication 11, et
au moins un agent supplémentaire ;
dans laquelle, éventuellement, l'agent supplémentaire est un inhibiteur de KIT de l'exon 9 ou un inhibiteur de KIT de l'exon 11 ; ou
dans laquelle l'agent supplémentaire est l'imatinib ou un de ses sels pharmaceutiquement acceptables, ou
dans laquelle l'agent supplémentaire est un inhibiteur de l'exon 17 de KIT, l'agent supplémentaire pouvant éventuellement être choisi parmi l'avapritinib, le BLU-263, le ripretinib, l'AZD3229 et le bezuclastinib, ainsi que leurs sels pharmaceutiquement acceptables.

16. Le composé selon l'une quelconque des revendications 1 à 10 ou la composition pharmaceutique selon la revendication 11, pour son utilisation dans une méthode de traitement d'un patient souffrant d'une maladie maligne **caractérisée par** une tumeur présentant une mutation KIT de l'exon 9, une mutation KIT de l'exon 11 ou une mutation KIT de l'exon 17, ou une combinaison de celles-ci, comprenant :
(a) obtenir un échantillon biologique sur le patient ;
(b) détecter la présence ou l'absence d'une mutation KIT au niveau de l'exon 13 ou d'une mutation KIT au niveau de l'exon 14, dans laquelle, éventuellement, la mutation KIT au niveau de l'exon 13 est V654A ou N655K, ou dans laquelle la mutation KIT au niveau de l'exon 14 est N680K ; et
(c) admnistrer au patient une quantité efficace d'un composé selon l'une quelconque des revendications 1 à 10, ou d'un sel pharmaceutiquement acceptable de celui-ci, ou d'une composition pharmaceutique selon la revendication 11, si la mutation est détectée.

17. Le composé selon l'une quelconque des revendications 1 à 10, ou la composition pharmaceutique selon la revendication 11, pour son utilisation en thérapie.
